(19) Europäisches Patentamt
European Patent Office
Office européen des brevets

(11) EP 1 873 257 A2

(12) **EUROPEAN PATENT APPLICATION**

(43) Date of publication:
**02.01.2008 Bulletin 2008/01**

(51) Int Cl.:
***C12Q 1/68*** *(2006.01)* ***C07H 21/02*** *(2006.01)*
***C07H 21/04*** *(2006.01)*

(21) Application number: **06022589.3**

(22) Date of filing: **28.05.2002**

(84) Designated Contracting States:
**AT BE CH CY DE DK ES FI FR GB GR IE IT LI LU MC NL PT SE TR**

(30) Priority:
**25.05.2001 US 293560 P**
**21.06.2001 US 300187 P**
**07.08.2001 US 310781 P**
**17.09.2001 US 323662 P**
**26.10.2001 US 344418 P**
**15.11.2001 US 334674 P**
**02.01.2002 US 346303 P**

(62) Document number(s) of the earlier application(s) in accordance with Art. 76 EPC:
**02739467.5 / 1 423 531**

(71) Applicant: **DNAPrint Genomics, Inc.**
**Sarasota, FL 34243 (US)**

(72) Inventor: **Frudakis, Tony N.**
**Bradenton**
**Florida 34210 (US)**

(74) Representative: **Cornish, Kristina Victoria Joy et al**
**Kilburn & Strode**
**20 Red Lion Street**
**London WC1R 4PJ (GB)**

Remarks:
This application was filed on 30 - 10 - 2006 as a divisional application to the application mentioned under INID code 62.

# (54) Compositions and methods for the inference of pigmentation traits

(57) The invention relates to methods for inferring a genetic pigmentation trait of a human subject from a nucleic acid sample or a polypeptide sample of the subject, and compositions for practicing such methods. The methods of the invention are based, in part, on the identification of single nucleotide polymorphisms (SNPs) that, alone or in combination, allow an inference to be drawn as to a genetic pigmentation trait such as hair shade, hair color, eye shade, or eye colour, and further allow an inference to be drawn as to race. A method of the invention can be performed, for example, by identifying in a nucleic acid sample at least one pigmentation-related haplotype allele of at least one pigmentation gene, and preferably a combination of pigmentation-related haplotypes alleles.

EP 1 873 257 A2

**Description**

## BACKGROUND OF THE INVENTION

### FIELD OF THE INVENTION

The invention relates generally to methods for inferring a genetic pigmentation trait or race of an individual; and more specifically to methods of detecting single nucleotide polymorphisms and combinations thereof in a nucleic acid sample that provide an inference as to hair color or shade or to eye color or shade, or to race.

### BACKGROUND INFORMATION

Biotechnology has revolutionized the field of forensics. More specifically, the identification of polymorphic regions in human genomic DNA has provided a means to distinguish individuals based on the occurrence of a particular nucleotide at each of several positions in the genomic DNA that are known to contain polymorphisms. As such, analysis of DNA from an individual allows a genetic fingerprint or "bar code" to be constructed that, with the possible exception of identical twins, essentially is unique to one particular individual in the entire human population.

In combination with DNA amplification methods, which allow a large amount of DNA to be prepared from a sample as small as a spot of blood or semen or a hair follicle, DNA analysis has become a routine tool in criminal cases as evidence that can free or, in some cases, convict a suspect. Indeed, criminal courts, which do not yet allow the results of a lie detector test into evidence, admit DNA evidence into trial. In addition, DNA extracted from evidence that, in some cases, has been preserved for years after the crime was committed, has resulted in the convictions of many people being overturned.

Although DNA fingerprint analysis has greatly advanced the field of forensics, and has resulted in freedom of people, who, in some cases, were erroneously imprisoned for years, current DNA analysis methods are limited. In particular, DNA fingerprinting analysis only provides confirmatory evidence that a particular person is, or is not, the person from which the sample was derived For example, while DNA in a semen sample can be used to obtain a specific "bar code", it provides no information about the person that left the sample. Instead, the bar code can only be compared to the bar code of a suspect in the crime. If the bar codes match, then it can reasonably be concluded that the person likely is the source of the semen. However, if there is not a match, the investigation must continue.

An effort has begun to accumulate a database of bar codes, particularly of convicted criminals. Such a database allows prospective use of a bar code obtained from a biological sample left at a crime scene; i.e., the bar code of the sample can be compared, using computerized methods, to the bar codes in the database and, where the sample is that of a person whose bar code is in the database, a match can be obtained, thus identifying the person as the likely source of the sample from the crime scene. While the availability of such a database provides a significant advance in forensic analysis, the potential of DNA analysis is still limited by the requirement that the database must include information relating to the person who left the biological sample at the crime scene, and it likely will be a long time, if ever, that such a database will provide information of an entire population. Thus, there is a need for methods that can provide prospective information about a subject from a nucleic acid sample of the subject. The invention satisfies this need, and provides additional advantages.

## SUMMARY OF THE INVENTION

The present invention relates to methods for inferring a genetic pigmentation trait of a human subject from a nucleic acid sample or a polypeptide sample of the subject, and compositions for practicing such methods. The methods of the invention are based, in part, on the identification of single nucleotide polymorphisms (SNPs) that, alone or in combination, allow an inference to be drawn as to a genetic pigmentation trait such as hair shade, hair color, eye shade, or eye color, and further allow an inference to be drawn as to race. As such, the compositions and methods of the invention arc useful, for example, as forensic tools for obtaining information relating to physical characteristics of a potential crime victim or a perpetrator of a crime from a nucleic acid sample present at a crime scene, and as tools to assist in breeding domesticated animals, livestock, and the like to contain a pigmentation trait as desired.

A method of the invention can be performed, for example, by identifying in a nucleic acid sample at least one pigmentation-related haplotype allele of at least one pigmentation gene, wherein the pigmentation gene is oculocutaneous albinism II (OCA2), agouti signaling protein (ASIP), tyrosinase-related protein 1 (TYRP1), tyrosinase (TYR), adaptor-related protein complex 3, beta 1 subunit (AP3B 1) (also known as adaptin B1 protein (ADP1)), adaptin 3 D subunit 1 (AP3D1), dopachrome tautomerase (DCT), silver homolog (SILV), AIM-1 protein (LOC51151), proopiomelanocortin (POMC), ocular albinism 1 (OA1), microphthalmia-associated transcription factor (MITF), myosin VA (MYO5A), RAB27A, coagulation factor II (thrombin) receptor-like 1 (F2RL1), or Adaptin 3 D subunit 1 (AP3D1) whereby the haplotype allele is associated with the pigmentation trait, thereby inferring the genetic pigmentation trait of the subject. In one embodiment, the pigmentation gene includes at least one of OCA2, ASIP, TYRP1, TYR, SILV AP3B1, AP3D1, AP3D1., or DCT, and the pigmentation-related haplotype allele is a penetrant pigmentation-related haplotype allele, which allows an inference to be drawn as to a pigmentation trait of a subject from which the nucleic acid sample was obtained. For example, where the genetic pigmentation trait is eye shade, a pigmentation-related haplotype allele can be identified in at least one of the OCA2, TYRP1, or DCT gene.

A genetic pigmentation trait that can be inferred according to a method of the invention can be hair color, hair shade, eye color, or eye shade, or can be race. A pigmentation-related haplotype allele includes specific nucleotide occurrences of two or more SNPs in a sequence of a pigmentation gene, particularly specific nucleotide occurrences of SNPs, which can be present and the same or different in one or both alleles of the pigmentation gene. A penetrant pigmentation-related haplotype allele is one that, by itself, allows an inference to be drawn that a genetic pigmentation trait of a human subject is more likely than random. A latent pigmentation-related haplotype allele is one that, in the context of one or more penetrant, or other latent haplotypes, allows a stronger inference to be drawn than the inference due to the penetrant or other latent haplotype allele(s), alone.

A sample useful for practicing a method of the invention can be any biological sample of a subject that contains nucleic acid molecules, including portions of the gene sequences to be examined, or corresponding encoded polypeptides, depending on the particular method. As such, the sample can be a cell, tissue or organ sample, or can be a sample of a biological fluid such as semen, saliva, blood, and the like. A nucleic acid sample useful for practicing a method of the invention will depend, in part, on whether the SNPs of the haplotype to be identified are in coding regions or in non-coding regions. Thus, where at least one of the SNPs to be identified is in a non-coding region, the nucleic acid sample generally is a deoxyribonucleic acid (DNA) sample, particularly genomic DNA or an amplification product thereof. However, where heteronuclear ribonucleic acid (RNA), which includes unspliced mRNA precursor RNA molecules, is available, a cDNA or amplification product thereof can be used. Where the each of the SNPs of the haplotype is present in a coding region of the pigmentation gene(s), the nucleic acid sample can be DNA or RNA, or products derived therefrom, for example, amplification products. Furthermore, while the methods of the invention generally are exemplified with respect to a nucleic acid sample, it will be recognized that particular haplotype alleles can be in coding regions of a gene and can result in polypeptides containing different amino acids at the positions corresponding to the SNPs due to non-degenerate codon changes. As such, in another aspect, the methods of the invention can be practiced using a sample containing polypeptides of the subject.

As disclosed herein, the identification of at least one penetrant pigmentation-related haplotype allele of at least one pigmentation gene allows an inference to be drawn as to a genetic pigmentation trait of a human subject. An inference drawn according to a method of the invention can be strengthened by identifying a second, third, fourth or more penetrant pigmentation related haplotype alleles and/or one or more latent pigmentation related haplotype alleles in the same pigmentation gene or in one or more other pigmentation genes. Accordingly, in another embodiment, a method of the invention can further include identifying in the nucleic acid sample at least a second penetrant pigmentation related haplotype allele of the first pigmentation gene and/or at least one penetrant pigmentation-related haplotype allele of at least a second pigmentation gene, for example, of an OCA2, ASIP, TYRP1, TYR, AP3B1, AP3DI, DCT, SILV, LOC51151, AIM1, POMC, OA1, MITF, MYO5A, RAB27A, F2RL1, AP3D1, or melanocortin-1 receptor (MC1R) gene.

By way of example, a method of the invention allows an inference to be drawn that a nucleic acid sample is that of a human Caucasian having a particular eye color or eye shade. In one aspect, a method of inferring that a sample is that of a Caucasian having a particular eye color or eye shade is performed by identifying a penetrant pigmentation-related haplotype allele, including at least one of a) nucleotides of the DCT gene corresponding to a DCT-A haplotype, which includes nucleotide 609 of SEQ ID NO:1 [702], nucleotide 501 of SEQ ID NO:2 [650], and nucleotide 256 of SEQ ID NO:3 [marker 675]; b) nucleotides of the MC1R gene corresponding to a melanocortin-1 receptor (MC1R)-A haplotype, which includes nucleotide 442 of SEQ ID NO:4 [217438]" nucleotide 619 of SEQ ID NO:5 [217439], and nucleotide 646 of SEQ ID NO:6 [217441]; c) nucleotides of the OCA2 gene, corresponding to an OCA2-A haplotype, which includes nucleotide 135 of SEQ ID NO:7 [217458], nucleotide 193 of SEQ ID NO:8 [886894], nucleotide 228 of SEQ ID NO:9 [marker 886895], and nucleotide 245 of SEQ ID NO:10 [marker 886896]; d) nucleotides of the OCA2 gene, corresponding to an OCA2-B haplotype, which includes nucleotide 189 of SEQ ID NO:11 [marker 217452]], nucleotide 573 of SEQ ID NO:12 [marker 712052], and nucleotide 245 of SEQ ID NO:13 [marker 886994]; e) nucleotides of the OCA2 gene, corresponding to an OCA2-C haplotype, which includes nucleotide 643 of SEQ ID NO:14 [712057], nucleotide 539 of SEQ ID NO:15 [712058], nucleotide 418 of SEQ ID NO:16 [712060], and nucleotide 795 of SEQ ID NO: 17, [712064]; f) nucleotides of the OCA2 gene, corresponding to an OCA2-D haplotype, which includes nucleotide 535 of SEQ ID NO: 18, [712054], nucleotide 554 of SEQ ID NO:19, [712056], and nucleotide 210 of SEQ ID NO:20, [886892]; g) nucleotides of the OCA2 gene, corresponding to an OCA2-E haplotype, which includes nucleotide 225 of SEQ ID NO:21, [217455], nucleotide 170 of SEQ ID NO:22, [712061], and nucleotide 210 of SEQ ID NO:20, [886892]; h) nucleotides of the TYRP1 gene corresponding to a TYRP1-B haplotype which includes nucleotide 172 of SEQ ID NO:33, [886938], or nucleotide 216 of SEQ ID NO:24; [886943], or any combination of the above listed penetrant haplotypcs. For example, the pigmentation-related haplotype allele of MCIR-A can be CCC; the pigmentation-related haplotype allele of OCA2-A can be TTA, CCAG, or TTAG; the pigmentation-related haplotype allele of OCA2-B can be CAA, CGA, CAC, or CGC; the pigmentation-related haplotype allele of OCA2-C can be GGAA, TGAA, or TAAA; the pigmentation-related haplotype allele of OCA2-D can be AGG or GGG; the pigmentation-related haplotype allele of OCA2-E can be GCA; the pigmentation-related haplotype allele of TYRP1-B can be TC; and the pigmentation-related haplotype allele of DCT-A can be CTG or GTG.

An inference that a nucleic acid sample is that of a human Caucasian having a particular eye color or eye shade can be strengthened by further identifying in the nucleic acid sample at least one nucleotide occurrence of a latent pigmentation-related SNP of a pigmentation gene, wherein the latent pigmentation-related SNP is nucleotide 61 of SEQ ID NO:25 [marker 560], nucleotide 201 of SEQ ID NO:26 [marker 552], nucleotide 201 of SEQ ID NO:27 [marker 559], nucleotide 201 of SEQ ID NO:28 [marker 468], nucleotide 657 of SEQ ID NO:29 [marker 657], nucleotide 599 of SEQ ID NO:30 [marker 674], nucleotide 267 of SEQ ID NO:31 [marker 632], nucleotide 61 of SEQ ID NO:32 [marker 701], nucleotide 451 of SEQ ID NO:33 [marker 710]; nucleotide 326 of SEQ ID NO:34 [marker 217456], nucleotide 61 of SEQ ID NO:35 [marker 656], nucleotide 61 of SEQ ID NO:36, nucleotide 61 of SEQ ID NO:37 [marker 637], nucleotide 93 of SEQ ID NO:38 [marker 278], nucleotide 114 of SEQ ID NO:39 [marker 386], nucleotide 558 of SEQ ID NO:40 [marker 217480], nucleotide 221 of SEQ ID NO:41 [marker 951497], nucleotide 660 of SEQ ID NO:42 [marker 217468], nucleotide 163 of SEQ ID NO:43 [marker 217473], nucleotide 364 of SEQ ID NO:44 [marker 217485], nucleotide 473 of SEQ ID NO:45 [marker 217486], nucleotide 314 of SEQ ID NO:46 [marker 869787], nucleotide 224 of SEQ ID NO:47 [marker 869745], nucleotide 169 of SEQ ID NO:48 [marker 886933], nucleotide 214 of SEQ ID NO:49 [marker 886937], or nucleotide 903 of SEQ ID NO:50; [856942], or a combination of the above listed pigmentation-related SNPs. Similarly, the inference can be strengthened by further identifying in the nucleic acid sample at least one latent pigmentation-related haplotype allele of a pigmentation gene, wherein the latent pigmentation-related haplotype allele includes a) nucleotides of the ASIP gene corresponding to an ASIP-A haplotype, which include nucleotide 201 of SEQ ID NO:26 [marker 552], and nucleotide 201 of SEQ ID NO:28 [marker 468]; b) nucleotides of the DCT gene corresponding to a DCT-B haplotype, which include nucleotide 451 of SEQ ID NO:33 [marker 710], and nucleotide 657 of SEQ ID NO:29 [marker 657]; c) nucleotides of the SILV gene corresponding to a SILV-A haplotype, which includes nucleotide 61 of SEQ ID NO:35 [marker 656], and nucleotide 61 of SEQ ID NO:36 ; d) nucleotides of the TYR gene corresponding to a TYR-A haplotype, which includes nucleotide 93 of SEQ ID NO:38 [marker 278], and nucleotide 114 of SEQ ID NO:39 [marker 386]; e) nucleotides of the TYRP1 gene corresponding to a TYRP1-A haplotype, which include nucleotide 364 of SEQ ID NO:44 [marker 217485], nucleotide 169 of SEQ ID NO:48 [marker 886933], or nucleotide 214 of SEQ ID NO: 49 [marker 886937], or any combination of the above listed latent pigmentation-related haplotypes. For example, the latent pigmentation-related haplotype allele of ASIP-A can be GT, AT; the latent pigmentation-related haplotype allele of DCT-B can be TA, TG; the latent pigmentation-related haplotype allele of SILV-A can be TC, TT; or CC the latent pigmentation-related haplotype allele of TYR-A can be GA, AA, or GG; and the latcnt pigmentation-related haplotype allele of TYRP1-A can be GTG, TTG, or GTT.

A method of identifying a pigmentation related SNP, including a pigmentation related haplotype allele can be performed using any method useful for identifying a particular nucleotide at a specific position in a nucleotide sequence or, where the nucleotide sequence encodes an amino acid sequence, by identifying an amino acid encoded by a codon of the nucleotide sequence, provided the nucleotide occurrences of the SNP result in a codons that encode different amino acids. Particularly useful methods include those that are readily adaptable to a high throughput format, to a multiplex format, or to both. In addition, a method of the invention can further include applying information relating to the pigment-related haplotype alleles to a matrix created using a feature modeling algorithm. For example, the feature modeling algorithm can be quadratic classifier or can perform a correspondence analysis.

Methods for detecting a nucleotide change can utilize one or more oligonucleotide probes or primers, including, for example, an amplification primer pair, that selectively hybridize to a target polynucleotide, which contains one or more pigmentation-related SNP positions. Oligonucleotide probes useful in practicing a method of the invention can include, for example, an oligonucleotide that is complementary to and spans a portion of the target polynucleotide, including the position of the SNP, wherein the presence of a specific nucleotide at the position (i.e., the SNP) is detected by the presence or absence of selective hybridization of the probe. Such a method can further include contacting the target polynucleotide and hybridized oligonucleotide with an endonuclease, and detecting the presence or absence of a cleavage product of the probe, depending on whether the nucleotide occurrence at the SNP site is complementary to the corresponding nucleotide of the probe. A pair of probes that specifically hybridize upstream and adjacent and downstream and adjacent to the site of the SNP, wherein one of the probes includes a nucleotide complementary to a nucleotide occurrence of the SNP, also can be used in an oligonucleotide ligation assay, wherein the presence or absence of a ligation product is indicative of the nucleotide occurrence at the SNP site. An oligonucleotide also can be useful as a primer, for example, for a primer extension reaction, wherein the product (or absence of a product) of the extension reaction is indicative of the nucleotide occurrence. In addition, a primer pair useful for amplifying a portion of the target polynucleotide including the SNP site can be useful, wherein the amplification product is examined to determine the nucleotide occurrence at the SNP site.

Where the particular nucleotide occurrence of a SNP, or nucleotide occurrences of a pigmentation-related haplotype, is such that the nucleotide occurrence results in an amino acid change in an encoded polypeptide, the nucleotide occurrence can be identified indirectly by detecting the particular amino acid in the polypeptide. The method for determining the amino acid will depend, for example, on the structure of the polypeptide or on the position of the amino acid in the polypeptide. Where the polypeptide contains only a single occurrence of an amino acid encoded by the particular SNP, the polypeptide can be examined for the presence or absence of the amino acid. For example, where the amino acid is at or near the amino terminus or the carboxy terminus of the polypeptide, simple sequencing of the terminal amino acids can be performed. Alternatively, the polypeptide can be treated with one or more enzymes and a peptide fragment containing the amino acid position of interest can be examined, for example, by sequencing the peptide, or by detecting a particular migration of the peptide following electrophoresis. Where the particular amino acid comprises an epitope of the polypeptide, the specific binding, or absence thereof, of an antibody specific for the epitope can be detected. Other methods for detecting a particular amino acid in a polypeptide or peptide fragment thereof arc well known and can be selected based, for example, on convenience or availability of equipment such as a mass spectrometer, capillary electrophoresis system, magnetic resonance imaging equipment, and the like.

In another embodiment, a method of the invention allows an inference to be drawn as to hair color or hair shade of a human subject by identifying in a nucleic acid sample of the subject a penetrant pigmentation-related haplotype allele in at least one pigmentation gene, for example, in at least one of OCA2, ASIP, TYRP1, or MC1R. For example, an inference can be drawn as to the hair color or hair shade of a human by identifying in a nucleic sample from the human a penetrant pigmentation-related haplotype allele, including in at least one of a) nucleotides of the ASIP gene corresponding to an ASIP-B haplotype, which include nucleotide 202 of SEQ ID NO:27, [559], and nucleotide 61 of SEQ ID NO:25, [560]; b) nucleotides of the MC1R gene corresponding to an MC1R-A haplotype, which include nucleotide 442 of SEQ ID NO:4 [217438],, nucleotide 619 of SEQ ID NO:5 [217439], and, nucleotide 646 of SEQ ID NO:6 [217441]; c) nucleotides of the OCA2 gene corresponding to an OCA2-G haplotype, which include nucleotide 418 of SEQ ID NO: 16 [712060], nucleotide 210 of SEQ ID NO:20, [886892], and nucleotide 245 of SEQ ID NO:10 [marker 886896]; d) nucleotides of the OCA2 gene corresponding to a OCA2-H haplotype, which include nucleotide 225 of SEQ ID NO:21, [217455], nucleotide 643 of SEQ ID NO:14 [712057], and nucleotide 193 of SEQ ID NO:8 [886894]; e) nucleotides of the OCA2 gene corresponding to a OCA2-I haplotype, which include nucleotide 135 of SEQ ID NO:7 [217458], and nucleotide 554 of SEQ ID NO:19, [712056]; e) nucleotides of the OCA2 gene corresponding to a OCA2-J haplotype, which include nucleotide 535 of SEQ ID NO:18, [712054], and nucleotide 228 of SEQ ID NO:9 [marker 886895]; or f) nucleotides of the TYRP1 gene corresponding to a TYRP1-C haplotype, which include nucleotide 473 of SEQ ID NO: 45, [217486], or, nucleotide 214 of SEQ ID NO:49; [886937], or any combination of the above-listed penetrant pigmentation-related haplotypes.

For example, the penetrant pigmentation-related haplotype allele can be a) the ASIP-B haplotype allele GA or AA; b) the MC1R-A haplotype allele CCC, CTC, TCC or CCT; c) the OCA2-G haplotype allele AGG or AGA; d) the OCA2-H haplotype allele AGT or ATT; c) the OCA2-I haplotype allele TG;f) the OCA2-J haplotype allele GA or AA; or g) the TYRP 1-C haplotype allele AA or TA; or a combination thereof, including, for example, the ASIP-B haplotype, the MC1R-A haplotype, the OCA2-G haplotype, the OCA2-H haplotype, the OCA2-I haplotype, the OCA2-J haplotype, and the TYRP1-C haplotype. Furthermore, as disclosed herein, an inference as to hair color or hair shade can be strengthened by further identifying, in addition to the at least one penetrant pigmentation related haplotype, in the nucleic acid sample, at least one latent pigmentation-related SNP of a pigmentation gene or at least one latent pigmentation-related haplotype allele, or a combination thereof.

In still another embodiment, a method of the invention allows an inference to be drawn as to the race of a human subject from a nucleic acid sample of the subject. Such a method can be performed, for example, by identifying in the nucleic acid sample, the nucleotide occurrence of at least one race-related single nucleotide polymorphism (SNP) of a race-related gene, whereby the nucleotide occurrence of the race-related SNP is associated with race. The race-related gene can include at least one of OCA2, ASIP, CYP2D6, TYRP1, CYP2C9, CYP3A4, TYR, MC1R, AP3B1, AP3D1, AP3D1, DCT, SILV, AIM-1 protein (LOC51151), POMC, OA1, MITF, MYO5A, RAB27A, F2RL1, HMGCR, FDPS, AHR, or CYP1A1, or can be a combination of nucleotide occurrence of a race-related SNP in any two or more of the above-listed genes, including in all of the genes.

A method of inferring the race of a human subject can be strengthened, for example, by identifying a nucleotide occurrence in each of at least two race-related SNPs, and grouping the identified nucleotide occurrences of the race-related SNPs into one or more race-related haplotype alleles, wherein the relationship of the haplotype allele(s) to race is known. For example, the race-related haplotype can be a race-related haplotype such as a) nucleotides of the DCT gene corresponding to a DCT-A haplotype, which includes nucleotide 609 of SEQ ID NO:1 [702], nucleotide 501 of SEQ ID NO:2 [650], and nucleotide 256 of SEQ ID NO:3 [marker 675]; b) nucleotides of the MC1R gene corresponding to an MC1R-A haplotype, which includes nucleotide 442 of SEQ ID NO:4 [217438], nucleotide 619 of SEQ ID NO:5 [217439], and nucleotide 646 of SEQ ID NO:6 [217441]; c) nucleotides of the OCA2 gene corresponding to an OCA2-A haplotype, which includes nucleotide 135 of SEQ ID NO:7 [217458], nucleotide 193 of SEQ ID NO:8 [886894], nucleotide 228 of SEQ ID NO:9 [marker 886895], and nucleotide 245 of SEQ ID NO:10 [marker 886896]; d) nucleotides of the OCA2 gene corresponding to an OCA2-B haplotype, which includes nucleotide 189 of SEQ ID NO:11 [marker 217452]], nucleotide 573 of SEQ ID NO:12 [marker 712052], and nucleotide 245 of SEQ ID NO:13 [marker 886994]; e) nucleotides of the OCA2 gene corresponding to an OCA2-C haplotype, which includes nucleotide 643 of SEQ ID NO:14 [712057], nucleotide 539 of SEQ ID NO:15 [712058], nucleotide 418 of SEQ ID NO:16 [712060], and nucleotide 795 of SEQ ID NO:17, [712064]; f) nucleotides of the OCA2 gene, corresponding to an OCA2-D haplotype, which includes nucleotide 535 of SEQ ID NO:18, [712054], nucleotide 554 of SEQ ID NO:19, [712056], or nucleotide 210 of SEQ ID NO:20, [886892]; g) nucleotides of the OCA2 gene, corresponding to an OCA2-E haplotype, which includes nucleotide 225 of SEQ ID NO: 21, [217455], nucleotide 170 of SEQ ID NO:22, [712061], and nucleotide 210 of SEQ ID NO:20, [886892]; or h) nucleotides of the TYRP1 gene corresponding to a TYRP1-B haplotype which includes nucleotide 172 of SEQ ID NO:23, [886938], nucleotide 216 of SEQ ID NO:24; [886943], or any combination of the above listed race-related haplotypes.

The inference also can be strengthened by identifying in the nucleic acid sample at least one race-related haplotype allele of a race-related gene. For example, a race-related haplotype allele can include nucleotide occurrences for a) nucleotides of the ASIP gene corresponding to a ASIP-A haplotype, which includes nucleotide 201 of SEQ ID NO: 26 [marker 552], and nucleotide 201 of SEQ ID NO:28 [marker 468]; b) nucleotides of the DCT gene corresponding to a DCT-B haplotype, which includes nucleotide 451 of SEQ ID NO:33 [marker 710], and nucleotide 657 of SEQ ID NO: 29 [marker 657]; c) nucleotides of the SILV gene corresponding to a SILV-A haplotype, which includes nucleotide 61 of SEQ ID NO:35 [marker 656], and nucleotide 61 of SEQ ID NO:36 ; d) nucleotides of the TYR gene corresponding to a TYR-A haplotype, which includes nucleotide 93 of SEQ ID NO:38 [marker 278], and nucleotide 114 of SEQ ID NO:39 [marker 386]; e) nucleotides of the TYR-B gene corresponding to a TYRP-B haplotype, which include nucleotide 364 of SEQ ID NO:44 [marker 217485], nucleotide 169 of SEQ ID NO:48 [marker 886933], or nucleotide 214 of SEQ ID NO: 49 [marker 886937], or any combination of the above listed race-related haplotype alleles.

As such, it will be recognized that a very strong inference as to race can be drawn by identifying combinations of race-related haplotype alleles, which include genotype alleles (i.e., alleles of diploid pairs of haplotypes), including, for example, a combination of the MC1R-A haplotype, the OCA2-A haplotype, the OCA2-B haplotype, the OCA2-C haplotype, the OCA2-D haplotype, the OCA2-E haplotype, the TYRP1-B haplotype, and the DCT-B haplotype; and the ASEP-A haplotype, the DCT-B haplotype, the SILV-A haplotype, the TYR-A haplotype, and the TYRP1-A haplotype. For example, the combination can include MC1R-A haplotype allele CCC; OCA2-A haplotype allele TTAA, CCAG, or TTAG; OCA2-B haplotype allele CAA, CGA, CAC, or CGC; OCA2-C haplotype allele GGAA, TGAA, or TAAA; OCA2-D haplotype allele AGG or GGG; OCA2-E haplotype allele GCA; TYRP1-B haplotype allele TC; and DCT-B haplotype allele CTG, or GTG; and ASIP-A haplotype allele GT or AT; DCT-B haplotype allele TA or TG; SILV-A haplotype allele TT, TC, or CC; TYR-A haplotype allele GA, AA, GG; and TYRP1-A haplotype allele GTG, TTG, or GTT.

In another embodiment, a method for inferring race of a human subject can be performed by identifying a nucleotide occurrence in the sample for at least one race-related SNP from a race-related gene such as OCA2, ASIP, CYP2D6, TYRP1, CYP2C9, CYP3A4, TYR, MC1R, AP3B1, AP3D1, AP3D1, DCT, SILV, AIM-1 (LOC51151) POMC, OA1, MITF, MYO5A, RAB27A, F2RL1, HMGCR, FDPS, AHR, or CYP1A1, whereby the nucleotide occurrence is associated with the race of the human subject. In addition, as disclosed herein, the inference can be strengthened by further identifying in the nucleic acid sample at least one nucleotide occurrence for at least a second race-related SNP of at least a second race-related gene such as the OCA2, ASIP, TYRP1, TYR, AP3B1, AP301, AP3D1, DCT, SILV. LOC51151, POMC, OA1, MITF, MYO5A, RAB27A, F2RL1, MCIR, CYP2D6, CYP2C9, CYP3A4, AP3B1, HMGCR, FDPS, AHR, or CYP1A1 gene. For example, the position of the nucleotide occurrence can be nucleotide 609 of SEQ ID NO:1 [marker 702], nucleotide 501 of SEQ ID NO:2 [marker 650], nucleotide 256 of SEQ ID NO:3 [marker 675], nucleotide 442 of SEQ ID NO:4 [marker 217438], nucleotide 619 of SEQ ID NO:5 [marker 217439], nucleotide 646 of SEQ ID NO:6 [marker 217441]; nucleotide 135 of SEQ ID NO:7 [marker 217458], nucleotide 193 of SEQ ID NO:8 [marker 886894], nucleotide 228 of SEQ ID NO:9 [marker 886895], nucleotide 245 of SEQ ID NO:10 [marker 886896], nucleotide 189 of SEQ ID NO:11 [217452], nucleotide 573 of SEQ ID NO:12 [712052], nucleotide 245 of SEQ ID NO:13 [marker 886994], nucleotide 643 of SEQ ID NO:14 [marker 712057], nucleotide 539 of SEQ ID NO:15 [marker 712058], nucleotide 418 of SEQ ID NO:16 [marker 712060], nucleotide 795 of SEQ ID NO:17 [marker 712064], nucleotide 535 of SEQ ID NO:18 [marker 712054], nucleotide 554 of SEQ ID NO:19 [marker 712056], nucleotide 210 of SEQ ID NO:20 [marker 886892], nucleotide 225 of SEQ ID NO:21 [marker 217455], nucleotide 170 of SEQ ID NO:22 [marker 712061], nucleotide 210 of SEQ ID NO:20 [marker 886892], nucleotide 172 of SEQ ID NO:23 [marker 886938], nucleotide 216 of SEQ ID NO:24 [marker 886943], nucleotide 61 of SEQ ID NO:25 [marker 560], nucleotide 201 of SEQ ID NO:26 [marker 552], nucleotide 201 of SEQ ID NO:27 [marker 559], nucleotide 201 of SEQ ID NO:28 [marker 468], nucleotide 657 of SEQ ID NO:29 [marker 657], nucleotide 599 of SEQ ID NO:30 [marker 674], nucleotide 267 of SEQ ID NO:31 [marker 632], nucleotide 61 of SEQ ID NO:32 [marker 701], nucleotide 451 of SEQ ID NO:33 [marker 710]; nucleotide 326 of SEQ ID NO:34 [marker 217456], nucleotide 61 of SEQ ID NO:35 [marker 656], nucleotide 61 of SEQ ID NO:36, nucleotide 61 of SEQ ID NO: 37 [marker 637], nucleotide 93 of SEQ ID NO:38 [marker 278], nucleotide 114 of SEQ ID NO:39 [marker 386], nucleotide 558 of SEQ ID NO:40 [marker 217480], nucleotide 221 of SEQ ID NO:41 [marker 951497], nucleotide 660 of SEQ ID NO:42 [marker 217468], nucleotide 163 of SEQ ID NO:43 [marker 217473], nucleotide 364 of SEQ ID NO:44 [marker 217485], nucleotide 473 of SEQ ID NO:45 [marker 217486], nucleotide 314 of SEQ ID NO:46 [marker 869787], nucleotide 224 of SEQ ID NO:47 [marker 869745], nucleotide 169 of SEQ ID NO:48 [marker 886933], nucleotide 214 of SEQ ID NO:49 [marker 886937], or nucleotide 903 of SEQ ID NO:50 [marker 886942], nucleotide 207 of SEQ ID NO:51 [marker 217459], nucleotide 428 of SEQ ID NO:52 [marker 217460], nucleotide 422 of SEQ ID NO:48 [marker 217487], nucleotide 459 of SEQ ID NO:54 [marker 217489], nucleotide 1528 of SEQ ID NO:55 [marker 554353], nucleotide 1093 of SEQ ID NO:56 [marker 554363], nucleotide 1274 of SEQ ID NO:57 [marker 554368], nucleotide 1024 of SEQ ID NO:58 [marker 554370], nucleotide 1159 of SEQ ID NO:59 [marker 554371], nucleotide 484 of SEQ ID NO:60 [marker 615921], nucleotide 619 of SEQ ID NO:61 [marker 615925], nucleotide 551 of SEQ ID NO:62 [marker 615926], nucleotide 1177 of SEQ ID NO:63 [marker 664784], nucleotide 1185 of SEQ ID NO:64 [marker 664785], nucleotide 1421 ofSEQ ID NO: 65 [664793], nucleotide 1466 of SEQ ID NO:66 [marker 664802], nucleotide 1311 of SEQ ID NO:67 [marker 664803], nucleotide 808 of SEQ ID NO:68 [marker 712037], nucleotide 1005 of SEQ ID NO:69 [marker 712047], nucleotide 743 of SEQ ID NO:70 [marker 712051], nucleotide 418 of SEQ ID NO:71 [marker 712055], nucleotide 884 of SEQ ID NO: 72 [marker 712059], nucleotide 744 of SEQ ID NO:73 [marker 712043], nucleotide 360 of SEQ ID NO:74 [marker 756239], nucleotide 455 of SEQ ID NO:75 [marker 756251], nucleotide 519 of SEQ ID NO:76 [marker 809125], nucleotide 277 of SEQ ID NO:77 [marker 869769], nucleotide 227 of SEQ ID NO:78 [marker 869772], nucleotide 270 of SEQ ID NO: 79 [marker 869777], nucleotide 216 of SEQ ID NO:80 [marker 869784], nucleotide 172 of SEQ ID NO:81 [marker 869785], nucleotide 176 of SEQ ID NO:82 [marker 869794], nucleotide 145 of SEQ ID NO:83 [marker 869797], nucleotide 164 of SEQ ID NO:84 [marker 869798], nucleotide 166 of SEQ ID NO:85 [marker 869802], nucleotide 213 of SEQ ID NO: 86 [marker 869809], nucleotide 218 of SEQ ID NO:87 [marker 869810], nucleotide 157 of SEQ ID NO:88 [marker 869813], nucleotide 837 of SEQ ID NO:89 [marker 886934], nucleotide 229 of SEQ ID NO:90 [marker 886993], nucleotide 160 of SEQ ID NO:91 [marker 951526], or any combination thereof.

The invention also relates to a method for inferring a genetic pigmentation trait of a human subject from a nucleic acid sample of the human subject by identifying a nucleotide occurrence in the sample for a pigmentation-related SNP from a pigmentation gene, provided the pigmentation gene is not the melanocortin-1 receptor (MC1 R) gene. For example, the method can be practiced by identifying a nucleotide occurrence in the sample for at least one pigmentation-related SNP from a pigmentation gene such as OCA2, ASIP, CYP2D6, TYRP1, CYP2C9, CYP3A4, TYR, MC1R, AP3B1, AP3D1, AP3D1, DCT, SILV, AIM-1 protein (LOC51151), POMC, OA1, MITF, MYO5A, RAB27A, F2RL1, HMGCR, FDPS, AHR, or CYP1A1, whereby the nucleotide occurrence is associated with the pigmentation trait of the human subject. In addition, the method can further include identifying in the nucleic acid sample at least one nucleotide occurrence for at least a second pigmentation-related SNP of at least a second pigmentation gene such as OCA2, ASIP, TYRP1, TYR, AP3B1, AP3D1, AP3D1, DCT, SILV, LOC51151, POMC, OA1, MITF, MYO5A, RAB27A, F2RL1, or MC1R.

The genetic pigmentation trait inferred according to a method of the invention can be hair color, hair shade, eye color, or eye shade, and further can be race. Where the pigmentation trait is eye shade or eye color, pigmentation gene can be the OCA2 gene, DCT gene, MC1R gene, or TYRP1 gene, or any combination thereof. A SNP identified according to a method of the invention can be a SNP of a penetrant haplotype associated with eye color or eye shade, for example, a nucleotide occurrence such as nucleotide 609 of SEQ ID NO: [marker 702], nucleotide 501 of SEQ ID NO:2 [marker 650], nucleotide 256 of SEQ ID NO:3 [marker 675], nucleotide 442 of SEQ ID NO:4 [marker 217438], nucleotide 619 of SEQ ID NO:5 [marker 217439], nucleotide 646 of SEQ ID NO:6 [marker 217441]; nucleotide 135 of SEQ ID NO:7 [marker 217458], nucleotide 193 of SEQ ID NO:8 [niarker 886894], nucleotide 228 of SEQ ID NO:9 [marker 886895], nucleotide 245 of SEQ ID NO:10 [marker 886896], nucleotide 189 of SEQ ID NO:11 [217452], nucleotide 573 of SEQ ID NO:12 [712052], nucleotide 245 of SEQ ID NO:13 [marker 886994], nucleotide 643 of SEQ ID NO:14 [marker 712057], nucleotide 539 of SEQ ID NO: 15 [marker 712058], nucleotide 418 of SEQ ID NO:16 [marker 712060], nucleotide 795 of SEQ ID NO:17 [marker 712064], nucleotide 535 of SEQ ID NO:18 [marker 712054], nucleotide 554 of SEQ ID NO:19 [marker 712056], nucleotide 210 of SEQ ID NO:20 [marker 886892], nucleotide 225 of SEQ ID NO:21 [marker 217455], nucleotide 170 of SEQ ID NO:22 [marker 712061], nucleotide 210 of SEQ ID NO:20 [marker 886892], nucleotide 172 of SEQ ID NO:23 [marker 886938], or nucleotide 216 of SEQ ID NO:24 [marker 886943], or any combination thereof. The SNP also can be a SNP of a latent haplotype associated with eye color or eye shade, for example, a nucleotide occurrence such as nucleotide 61 of SEQ ID NO:25 [marker 560], nucleotide 201 of SEQ ID NO:26 [marker 552], nucleotide 201 of SEQ ID NO:27 [marker 559], nucleotide 201 of SEQ ID NO:28 [marker 468], nucleotide 657 of SEQ ID NO:29 [marker 657], nucleotide 599 of SEQ ID NO:30 [marker 674], nucleotide 267 of SEQ ID NO:31 [marker 632], nucleotide 61 of SEQ ID NO:32 [marker 701], nucleotide 451 of SEQ ID NO:33 [marker 710]; nucleotide 326 of SEQ ID NO:34 [marker 217456], nucleotide 61 of SEQ ID NO:35 [marker 656], nucleotide 61 of SEQ ID NO:36, nucleotide 61 of SEQ ID NO:37 [marker 637], nucleotide 93 of SEQ ID NO:38 [marker 278], nucleotide 114 of SEQ ID NO:39 [marker 386], nucleotide 558 of SEQ ID NO:40 [marker 217480], nucleotide 221 of SEQ ID NO:41 [marker 951497], nucleotide 660 of SEQ ID NO:42 [marker 217468], nucleotide 163 of SEQ ID NO:43 [marker 217473], nucleotide 364 of SEQ ID NO:44 [marker 217485], nucleotide 473 of SEQ ID.NO:45 [marker 217486], nucleotide 314 of SEQ ID NO:46 [marker 869787], nucleotide 224 of SEQ ID NO:47 [marker 869745], nucleotide 169 of SEQ ID NO:48 [marker 886933], nucleotide 214 of SEQ ID NO:49 [marker 886937J, or nucleotide 903 of SEQ ID NO:50 [marker 886942], or any combination thereof.

Where the pigmentation trait is hair color or hair shade, a SNP identified according to a method of the invention can be a SNP of a penetrant haplotype associated with hair color or hair shade, for example, a nucleotide occurrence such as nucleotide 201 of SEQ ID NO:27 [marker 559], nucleotide 61 of SEQ ID NO:25 [marker 560], nucleotide 442 of SEQ ID NO:4 [marker 217438], nucleotide 619 of SEQ ID NO:5 [marker 217439], nucleotide 646 of SEQ ID NO:6 [marker 217441], nucleotide 418 of SEQ ID NO: 16 [marker 712060], nucleotide 210 of SEQ ID NO:20 [marker 886892], nucleotide 245 of SEQ ID NO:10 [marker 886896], nucleotide 225 of SEQ ID NO:21 [marker 217455], nucleotide 643 of SEQ ID NO:14 [marker 712057], nucleotide 193 of SEQ ID NO:8 [marker 886894], nucleotide 135 of SEQ ID NO:7 [marker 217458], nucleotide 554 of SEQ ID NO: 19 [marker 712056], nucleotide 535 of SEQ ID NO:18 [marker 712054], nucleotide 228 of SEQ ID NO:9 [marker 886895], nucleotide 473 of SEQ ID NO:45, [217486], or nucleotide 214 of SEQ ID NO:49; [886937], or any combination thereof.

A method for inferring a genetic pigmentation trait of a human subject from a nucleic acid sample of the human subject by identifying a nucleotide occurrence in the sample for a pigmentation-related SNP from a pigmentation gene can further include grouping the nucleotide occurrences of the pigmentation-related SNPs for a gene into one or more haplotype alleles. The identified haplotype alleles then can be compared to known haplotype alleles such that, when the relationship of the known haplotype alleles to the genetic pigmentation trait is known, an inference can be drawn as to the genetic pigmentation trait of the subject providing the nucleic acid sample. Identification of the nucleotide occurrence can be performed using any method suitable for examining the particular sample. For example, wherein the sample contains nucleic acid molecules, the identification can be performed by contacting polynucleotides in (or derived from) the sample with a specific binding pair member that selectively hybridizes to a region of the polynucleotide that includes the pigmentation-related SNP, under conditions wherein the binding pair member specifically binds at or near the pigmentation-related SNP. The binding pair member can be any molecule that specifically binds or associates with the target polynucleotide, including, for example, an antibody or an oligonucleotide.

The invention also relates to a method for classifying an individual as being a member of a group sharing a common characteristic. Such a method can be performed, for example, by identifying a nucleotide occurrence of a SNP in a polynucleotide of the individual, wherein the SNP corresponds to nucleotide 473 of SEQ ID NO:45 [marker 217456], nucleotide 224 of SEQ ID NO:47 [marker 869745], nucleotide 314 of SEQ ID NO:46 [marker 869787], nucleotide 210 of.SEQ ID NO:20 [marker 886892], nucleotide 228 of SEQ ID NO:9 [marker 886895], nucleotide 245 of SEQ ID NO:10 [marker 886896], nucleotide 169 of SEQ ID NO:48 [marker 886933], nucleotide 214 of SEQ ID NO:49 [marker 886937], nucleotide 245 of SEQ ID NO:13 [marker 886994], nucleotide 193 of SEQ ID NO:8 [marker 886894], nucleotide 172 of SEQ ID NO:23 [marker 886938], nucleotide 216 of SEQ ID NO:24 [marker 886943], or nucleotide 903 of SEQ ID NO: 50 [marker 886942], or any combination thereof. Such a method can be performed, for example, using an amplification reaction or a primer extension reaction.

The invention further relates to a method for detecting a nucleotide occurrence for a SNP of a human pigmentation gene. Such a method can be performed, for example, by contacting a sample containing a polynucleotide with a specific binding pair member, which can specifically bind at or near a sequence of the polynucleotide suspected of being polymorphic, including a nucleotide occurrence corresponding to nucleotide 473 of SEQ ID NO:45 [marker 217486], nucleotide 224 of SEQ ID NO:47 [marker 869745], nucleotide 314 of SEQ ID NO:46 [marker 869787], nucleotide 210 of SEQ ID NO:20 [marker 886892], nucleotide 228 of SEQ ID NO:9 [marker 886895], nucleotide 245 of SEQ ID NO:10 [marker 886896], nucleotide 169 of SEQ ID NO:48 [marker 886933], nucleotide 214 of SEQ ID NO:49 [marker 886937], nucleotide 245 of SEQ ID NO:13 [marker 886994], nucleotide 193 of SEQ ID NO:8 [marker 886894], nucleotide 172 of SEQ ID NO:23 [marker 886938], nucleotide 216 of SEQ ID NO:24 [marker 886943], or nucleotide 903 of SEQ ID NO: 50 [marker 886942], or any combination thereof; and detecting selective binding of the specific binding pair member, wherein selective binding is indicative of the presence of the nucleotide occurrence.

The invention also relates to an isolated primer pair, which can be useful for determining a nucleotide occurrence of a SNP in a polynucleotide, wherein the primer pair includes a forward primer that can selectively bind to the polynucleotide upstream of the SNP position on one strand, and a reverse primer that can selectively bind to the polynucleotide upstream of the SNP position on a complementary strand, wherein the SNP position corresponds to nucleotide 473 of SEQ ID NO:45 [marker 217486], nucleotide 224 of SEQ ID NO:47 [marker 869745], nucleotide 314 of SEQ ID NO:46 [marker 869787], nucleotide 210 of SEQ ID NO:20 [marker 886892], nucleotide 228 of SEQ ID NO:9 [marker 886895], nucleotide 245 of SEQ ID NO:10 [marker 886896], nucleotide 169 of SEQ ID NO:48 [marker 886933], nucleotide 214 of SEQ ID NO:49 [marker 886937], nucleotide 245 of SEQ ID NO:13 [marker 886994], nucleotide 193 of SEQ ID NO:8 [marker 886894], nucleotide 172 of SEQ ID NO:23 [marker 886938], nucleotide 216 of SEQ ID NO:24 [marker 886943], or nucleotide 903 of SEQ ID NO:50 [marker 886942].

In addition, the invention relates to an isolated specific binding pair member, which can be useful for determining a nucleotide occurrence of a SNP in a target polynucleotide, particularly a region of a pigmentation gene or xenobiotic gene including a SNP, as disclosed herein. For example, a specific binding pair member of the invention can be an oligonucleotide or an antibody that, under the appropriate conditions, selectively binds to a target polynucleotide at or near nucleotide 473 of SEQ ID NO:45. [marker 217486], nucleotide 224 of SEQ ID NO:47 [marker 869745], nucleotide 314 of SEQ ID NO:46 [marker 869787], nucleotide 210 of SEQ ID NO:20 [marker 886892], nucleotide 228 of SEQ ID NO:9 [marker 886895], nucleotide 245 of SEQ ID NO:10 [marker 886896], nucleotide 169 of SEQ ID NO:48 [marker 886933], nucleotide 214 of SEQ ID NO:49 [marker 886937], nucleotide 245 of SEQ ID NO: 13 [marker 886994], nucleotide 193 of SEQ ID NO:8 [marker 886894], nucleotide 172 of SEQ ID NO:23 [marker 886938], nucleotide 216 of SEQ ID NO:24 [marker 886943], or nucleotide 903 of SEQ ID NO:50 [marker 886942]. As such, a specific binding pair member of the invention can be an oligonucleotide probe, which can selectively hybridize to a target polynucleotide and can, but need not, be a substrate for a primer extension reaction, or an anti-nucleic acid antibody. The specific binding pair member can be selected such that it selectively binds to any portion of a target polynucleotide, as desired, for example, to a portion of a target polynucleotide containing a SNP as the terminal nucleotide.

The invention also relates isolated polynucleotides comprising a portion of a gene including a SNP associated with a genetic pigmentation trait, wherein the isolated polynucleotide is at least about 30 nucleotides in length (for example, about 40, 50, 100, 200, 250, or 500 nucleotides in length). Polynucleotides of the invention are exemplified by a polynucleotide of at least about 30 nucleotides of the human OCA2 gene, and including at least a thymidine residue at a nucleotide corresponding to nucleotide 193 of SEQ ID NO:8 [marker 886894], a guanidine residue at a nucleotide corresponding to nucleotide 228 of SEQ ID NO:9 [marker 886895], a cytidine residue at a nucleotide corresponding to nucleotide 210 of SEQ ID NO:20 [marker 886892], a thymidine residue at a nucleotide corresponding to nucleotide 245 of SEQ ID NO:10 [marker 886896], an adenosine residue at a nucleotide corresponding to nucleotide 245 of SEQ ID NO:13 [marker 586994], or a combination of such residues; and by a polynucleotide of at least about 30 nucleotides of the human TYRP gene, and including at least a thymidine residue at a nucleotide corresponding to nucleotide 172 of SEQ ID NO:23 [marker 886938], a thymidine residue at a nucleotide corresponding to nucleotide 216 of SEQ ID NO:24 [marker 886943], a thymidine residue at a nucleotide corresponding to nucleotide 473 of SEQ ID NO:45 [marker 217486], a cytidine residue at a nucleotide corresponding to nucleotide 224 of SEQ ID NO:47 [marker 869745], a guanidine residue at a nucleotide corresponding to nucleotide 314 of SEQ ID NO:46 [marker 869787], a cytidine residue at a nucleotide corresponding to nucleotide 169 of SEQ ID NO:48 [marker 886933], a thymidine residue at a nucleotide corresponding to nucleotide 214 of SEQ ID NO:49 [marker 886937], a adenosine residue at a nucleotide corresponding to nucleotide 903 of SEQ ID NO:50 [marker 886942], or a combination of such residues.

An isolated polynucleotide of the invention, which generally is at least about 30 nucleotides in length, also can be, for example, an isolated segment of an DCT gene, wherein nucleotides CTG or GTG occur at positions corresponding to nucleotide 609 of SEQ ID NO:1 [702], nucleotide 501 of SEQ ID NO:2 [marker 650], and nucleotide 256 of SEQ ID NO:3 [675], respectively; or an isolated segment of an MC1R gene, wherein nucleotides CCC occur at positions corresponding to nucleotide 442 of SEQ ID NO:4 [217438], nucleotide 619 of SEQ ID NO:5 [217439], and nucleotide 646 of SEQ ID NO:6 [217441], respectively; or an isolated segment of an OCA2 gene, wherein nucleotides TTAA, CCAG, or TTAG occur at positions corresponding to nucleotide 135 of SEQ ID NO:7 [217458], nucleotide 193 of SEQ DD NO:8 [886894], nucleotide 228 of SEQ ID NO:9 [886895], and nucleotide 245 of SEQ ID NO:10 [886896], respectively; or an isolated segment of the OCA2 gene, wherein nucleotides CAA, CGA, CAC, or CGC occur at positions corresponding to position 189 of SEQ ID NO:11 [217452], position 573 of SEQ ID NO:12 [712052], and position 245 of SEQ ID NO:13 [886994], respectively, or an isolated segment of the OCA2 gene, wherein nucleotides GGAA, TGAA, and TAAA occur at positions corresponding to nucleotide 643 of SEQ ID NO:14 [712057], nucleotide 539 of SEQ ID NO:15 [712058], nucleotide 418 of SEQ ID NO:16 [712060], and nucleotide 795 of SEQ ID NO:17 [712064], respectively, or an isolated segment of the OCA2 gene, wherein nucleotides AGG or GGG occur at positions corresponding to nucleotide 535 of SEQ ID NO:18 [712054], nucleotide 554 of SEQ ID NO:19 [712056], and nucleotide 210 of SEQ ID NO:20 [886892], respectively, or an isolated segment of the OCA2 gene, wherein nucleotides GCA occur at positions corresponding to nucleotide 225 of SEQ ID NO:21 [217455], nucleotide 170 of SEQ ID NO:22 [712061], and nucleotide 210 of SEQ ID NO:20 [886892], respectively; or an isolated segment of a TYRP1 gene, wherein nucleotides TC occur at positions corresponding to nucleotide 172 of SEQ ID NO:23 [886938], and nucleotide 216 of SEQ ID NO:24 [886943], respectively. In one embodiment, an isolated polynucleotide of the invention is derived from the OCA2 gene and includes comprises any combination of the nucleotides TTAA, CCAG, or TTAG at positions corresponding to nucleotide 135 of SEQ ID NO: 7 [217458], nucleotide 193 of SEQ ID NO:8 [886894], nucleotide 228 of SEQ ID NO:9 [886895], and nucleotide 245 of SEQ ID NO: 10 [886896], respectively; nucleotides CAA, CGA, CAC, or CGC at positions corresponding to position Y of SEQ ID NO:11 [217452], position 573 of SEQ ID NO:12 [712052], and position 245 of SEQ ID NO:13 [886994], respectively; nucleotides GGAA, TGAA, and TAAA at positions corresponding to nucleotide 643 of SEQ ID NO:14 [712057], nucleotide 539 of SEQ ID NO:15 [712058], nucleotide 418 of SEQ ID NO:16 [712060], and nucleotide 795 of SEQ ID NO:17 [712064], respectively; nucleotides AGG or GGG at positions corresponding to nucleotide 535 of SEQ ID NO:18 [712054], nucleotide 554 of SEQ ID NO:19 [712056], and nucleotide 210 of SEQ ID NO:20 [886892], respectively; and nucleotides GCA at positions corresponding to nucleotide 225 of SEQ ID NO:21 [217455], nucleotide 170 of SEQ ID NO:22 [712061], and nucleotide 210 of SEQ ID NO:20 [886892], respectively.

An isolated polynucleotide of the invention also can be, for example, an isolated segment of an ASIP gene, wherein nucleotides GT or AT occur at positions corresponding to nucleotide 201 of SEQ ID NO:26 [552], and nucleotide 201 of SEQ ID NO:28 [468], respectively; an isolated segment of a DCT gene, wherein nucleotides TA or TG occur at positions corresponding to nucleotide 451 of SEQ ID NO:33 [710], and nucleotide 356 of SEQ ID NO:29 [657], respectively, an isolated segment of a SILV gene wherein nucleotides TC, TT, or CC occur at positions corresponding to nucleotide 61 of SEQ ID NO:35 [656], and nucleotide 61 of SEQ ID NO:36 [662], respectively; an isolated segment of a TYR gene, wherein nucleotides GA, AA, or GG occur at positions corresponding to nucleotide 93 of SEQ ID NO:38 [278], and nucleotide 114 of SEQ ID NO:39 [386], respectively; or an isolated segment of a TYRP1 gene, wherein nucleotides GTG, TTG, GTT occur at positions corresponding to nucleotide 442 of SEQ ID NO:44 [217485], nucleotide 442 of SEQ ID NO:44 [886933], and nucleotide 442 of SEQ ID NO:49 [886937], respectively.

In addition, an isolated polynucleotide of the invention can be, for example, an isolated segment of an ASIP gene, wherein nucleotides GA or AA occur at positions corresponding to nucleotide 201 of SEQ ID NO:27 [559], and nucleotide 61 of SEQ ID NO:25 [560], respectively; an isolated segment of a MC1R gene, wherein nucleotides CCC, CTC, TCC, or CCT occur at positions corresponding to nucleotide 442 of SEQ ID NO:4[217438], nucleotide 619 of SEQ ID NO:5 [217439], and nucleotide 646 of SEQ ID NO:6 [217441], respectively; an isolated segment of an OCA2 gene, wherein nucleotides AGG or AGA occur at positions corresponding to nucleotide 418 ofSEQ ID NO:16 [712060], nucleotide 210 of SEQ ID NO:20 [886892], and nucleotide 245 of SEQ ID NO:10 [886896], respectively; an isolated segment of an OCA2 gene, wherein nucleotides AGT or ATT occur at positions corresponding to nucleotide 225 of SEQ ID NO: 21 [217455], nucleotide 643 of SEQ ID NO:14 [712057], and nucleotide 193 of SEQ ID NO:8 [886894], respectively; an isolated segment of an OCA2 gene, wherein nucleotides TG occur at positions corresponding to nucleotide 135 of SEQ ID NO:7 [217458], and nucleotide 554 of SEQ ID NO:19 [712056], respectively; an isolated segment of an OCA2 gene, wherein nucleotides AGG or ATT occur at positions corresponding to nucleotide 535 of SEQ ID NO:18 [712054], and nucleotide 228 of SEQ ID NO:9 [886895], respectively; or an isolated segment of a TYRP1 gene, wherein nucleotides AA or TA occur at positions corresponding to nucleotide 442 of SEQ ID NO:45 [217486], and nucleotide 442 of SEQ ID NO:49 [886937], respectively.

In one embodiment, an isolated polynucleotide of the invention is derived from the OCA2 gene and includes comprises any combination of the nucleotides AGG or AGA occur at positions corresponding to nucleotide 418 of SEQ ID NO: 16 [712060], nucleotide 210 of SEQ ID NO:20 [886892], and nucleotide 245 of SEQ ID NO:10 [886896], respectively; an isolated segment of an OCA2 gene, wherein nucleotides AGT or ATT occur at positions corresponding to nucleotide 225 of SEQ ID NO:21 [217455], nucleotide 643 of SEQ ID NO:14 [712057], and nucleotide 193 of SEQ ID NO:8 [886894], respectively; an isolated segment of an OCA2 gene, wherein nucleotides TG occur at positions corresponding to nucleotide 135 of SEQ ID NO:7 [217458], and nucleotide 554 of SEQ ID NO:19 [712056], respectively; an isolated segment of an OCA2 gene, wherein nucleotides GA or AA occur at positions corresponding to nucleotide 535 of SEQ ID NO:18 [712054], and nucleotide 228 of SEQ IDNO:9 [886895], respectively.

The invention also relates to kits, which can be used, for example, to perform a method of the invention. Thus, in one embodiment, the invention provides a kit for identifying haplotype alleles of pigmentation-related SNPs. Such a kit can contain, for example, an oligonucleotide probe, primer, or primer pair of the invention, such oligonucleotides being useful, for example, to identify a SNP or haplotype allele as disclosed herein; or can contain one or more polynucleotides corresponding to a portion of a pigmentation, xenobiotic, or other relevant gene containing one or more nucleotide occurrences associated with a genetic pigmentation trait, with race, or with a combination thereof, such polynucleotide being useful, for example, as a standard (control) that can be examined in parallel with a test sample. In addition, a kit of the invention can contain, for example, reagents for performing a method of the invention, including, for example, one or more detectable labels, which can be used to label a probe or primer or can be incorporated into a product generated using the probe or primer (e.g., an amplification product); one or more polymerases, which can be useful for a method that includes a primer extension or amplification procedure, or other enzyme or enzymes (e.g., a ligase or an endonuclease), which can be useful for performing an oligonucleotide ligation assay or a mismatch cleavage assay; and/or one or more buffers or other reagents that are necessary to or can facilitate performing a method of the invention.

In one embodiment, a kit of the invention includes one or more primer pairs of the invention, such a kit being useful for performing an amplification reaction such as a polymerase chain reaction (PCR). Such a kit also can contain, for example, one or reagents for amplifying a polynucleotide using a primer pair of the kit. The primer pair(s) can be selected, for example, such that they can be used to determine the nucleotide occurrence of a pigmentation-related SNP, wherein a forward primer of a primer pair selectively hybridizes to a sequence of the target polynucleotide upstream of the SNP position on one strand, and the reverse primer of the primer pair selectively hybridizes to a sequence of the target polynucleotide upstream of the SNP position on a complementary strand.

In another embodiment, a kit of the invention provides a plurality of oligonucleotides of the invention, including one or more oligonucleotide probes or one or more primers, including forward and/or reverse primers, or a combination of such probes and primers or primer pairs. Such a kit provides a convenient source for selecting probe(s) and/or primer (s) useful for identifying one or more SNPs or haplotype alleles as desired. Such a kit also can contain probes and/or primers that conveniently allow a method of the invention to be performed in a multiplex format.

The invention also relates to a method for identifying a pigmentation-related SNP. Such a method can be performed, for example, by identifying a candidate SNP of a pigmentation gene or a xenobiotic metabolism gene; determining that the candidate SNP has a genotype class comprising alleles exhibiting a coherent inheritance pattern, and a minor allele frequency that is greater than 0.01 in at least one race, thereby identifying a validated SNP; and determining that the validated SNP exhibits significantly different genotype distributions and allele frequencies between individuals of different pigmentation phenotypes or racial classes, thereby identifying a pigmentation-related SNP. In addition, the invention relates to a method for identifying a race-related SNP. Such a method can be performed, for example, by identifying a candidate SNP of a pigmentation gene or a xenobiotic metabolism gene; determining that the SNP has a genotype class, a coherent pattern, and a minor allele frequency that is greater than 0.01 in at least one race, thereby identifying a validated SNP; and determining that the validated SNP exhibits significantly different genotype distributions and allele frequencies between racial classes, thereby identifying a race-related SNP. Either of such methods can further include, for example, using linear, quadratic, correspondence analysis or classification tree multivariate modeling to develop an abstract classifier incorporating one or more validated SNP or set of validated SNP that blindly generalizes to other individuals of known pigmentation or of known race, respectively.

The power of the inference drawn according to the methods of the invention is increased by using a complex classifier function. Accordingly, the invention also relates to methods that draw an inference regarding a pigmentation trait or race of a subject using a classification function. A classification function applies nucleotide occurrence information identified for a SNP or set of SNPs such as one or preferably a combination of haplotype alleles, to a set of rules to draw an inference regarding a pigmentation trait or a subject's race. In certain examples, the classifier function includes applying the pigment-related haplotype alleles to a matrix created using a feature modeling algorithm. In certain examples, classification function is a linear or quadratic classifier or performs correspondence analysis.

In one embodiment, the invention includes a method for identifying a classifier function for inferring a pigmentation-trait of a subject. The method includes: i) identifying one or more candidate SNPs of one or more pigmentation genes that have a genotype class comprising alleles exhibiting a coherent inheritance pattern, and a minor allele frequency that is greater than 0.01 in at least one race, thereby identifying one or more validated SNPs; ii) determining that the one or more validated SNPs exhibits significantly different genotype distributions and allele frequencies between individuals of different pigmentation phenotypes or racial classes, and iii) Using linear, quadratic, correspondence analysis or classification tree multivariate modeling to develop an abstract classifier function incorporating one or more validated SNPs or combinations of validated SNPs that blindly generalizes to other individuals of known pigmentation, thereby identifying a pigmentation-related classification strategy.

In another embodiment, the invention includes a method for identifying a classifier function for inferring the race of a subject. The method includes: i) identifying one or more candidate SNPs of one or more race-related genes that have a genotype class comprising alleles exhibiting a coherent inheritance pattern, and a minor allele frequency that is greater than 0.01 in at least one race, thereby identifying one or more validated SNPs; ii) determining that the one or more validated SNPs exhibits significantly different genotype distributions and allele frequencies between individuals of different pigmentation phenotypes or racial classes, and iii) Using linear, quadratic, correspondence analysis or classification tree multivariate modeling to develop an abstract classifier function incorporating one or more validated SNPs or combinations of validated SNPs that blindly generalizes to other individuals of known race, thereby identifying a classifier function for inferring the race of a subject.

In another embodiment, the invention provides a method for classifying a sample. The method includes: a) computing a variance/covariance matrix for all possible trait class pairs; b) creating a combination of class mean vectors, wherein vector components arc binary encodings, correspondence analysis principal coordinates, correspondence analysis factor scores or correspondence analysis standard coordinates; c) representing a sample as an n-dimensional sample vector; and d) classifying a sample by identifying a class mean vector from the combination of class mean vectors, that is the shortest distance from the sample.

## BRIEF DESCRIPTION OF THE DRAWINGS

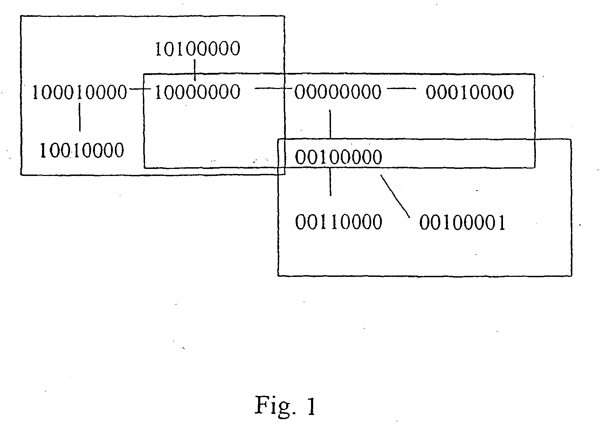

Figure 1 is a cladogram or a parsimony tree showing that haplotypes observed in the human population can be expressed such that the evolutionary relationships between the haplotypes are discernable. In the diagram, lines separate haplotypes that are one mutational step from another and biallelic positions within a gene are represented in binary form (1 and 0).

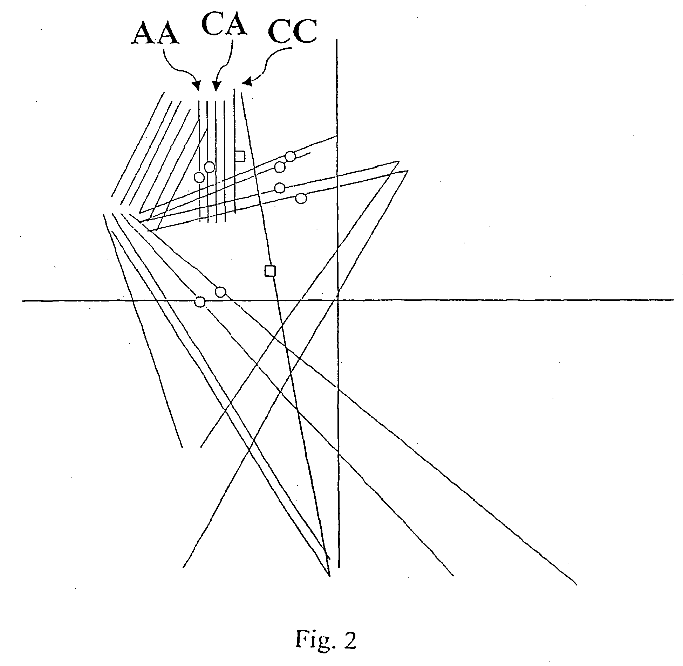

Figure 2 is a graph of the OCA2 8 haplotypes described in Example 6 herein. For simplicity the plot is in two dimensions, with a third dimension, that of the TYR_3 genotype (for three classes of OCA2 haplotype pairs) shown in bold print. Each line represents a diploid set of haplotypes encoded as described in the text. Where the origin of two or more lines is located at the same coordinate position, the lines were placed next to one another to simplify presentation. For example, the 6 lines without a square or circle attached, at the upper left-hand region of the plot placed next to one another represent the same combination of OCA2 haplotypes in different individuals of brown hair color. A third dimension in the grid is the TYR_3 genotype of the individuals, and this genotype is shown for three individual types in the plot (only 3 to keep the figure manageable.)

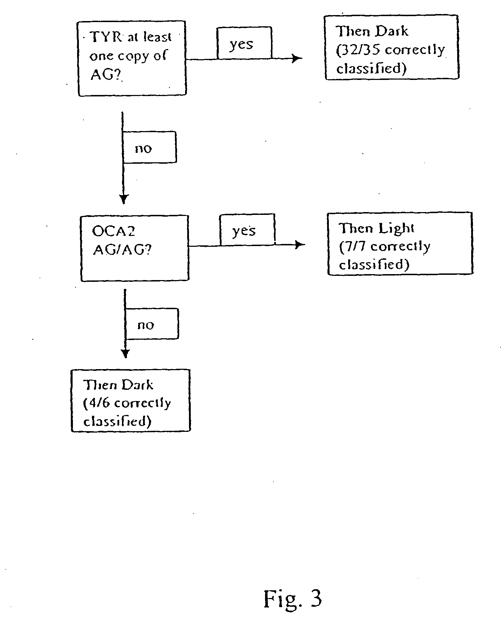

Figure 3 shows the composite solution for predicting the natural hair color from an unknown DNA specimen (see Example 7). This particular solution correctly classified dark haired Caucasian individuals 95% of the time and light haired individuals 70% of the time.

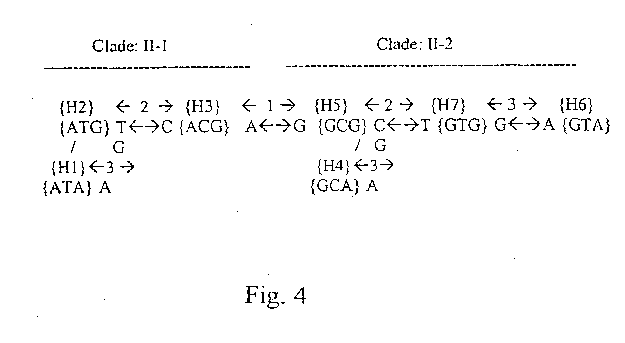

Figure 4 is a cladogram and clade designations for OCA3LOCI09 haplotypes as described in Example 8. The haplotype is shown as a trinucleotide sequence, and the name of the haplotype appears above the sequence. Haplotypcs are related to one another in the cladogram by step-wise mutations indicated by the altered nucleotide on either side of the bi-directional arrows. Two-step clade designations (II=1, II=2) are shown above the dashed line at the top of the figure.

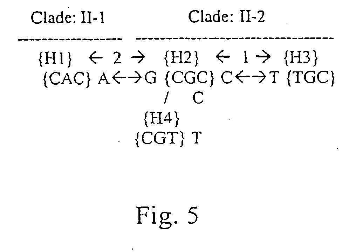

Figure 5 is a cladogram and clade designations for OCA3LOC920 haplotypes as indicated in Example 8. The haplotype is shown as a trinucleotide sequence, and the name of the haplotype appears above the sequence. Haplotypes are related to one another in the cladogram by step-wise mutations indicated by the altered nucleotide on either side of the bi-directional arrows. Two-step clade designations (II=1, II=2) are shown above the dashed line at the top of the figure.

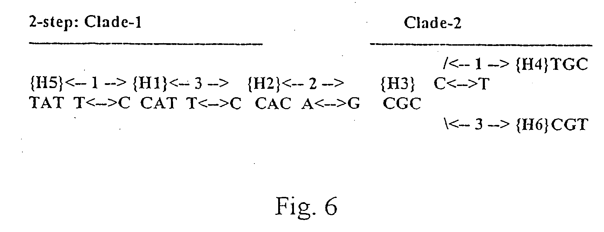

Figure 6 is a cladogram for OCA2 haplotypes, as described in Example 11.

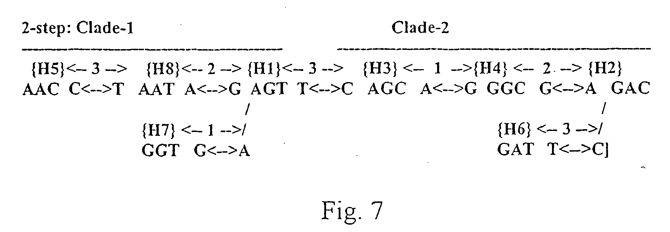

Figure 7 is cladogram for OCA3LOC922, as described in Example 11.

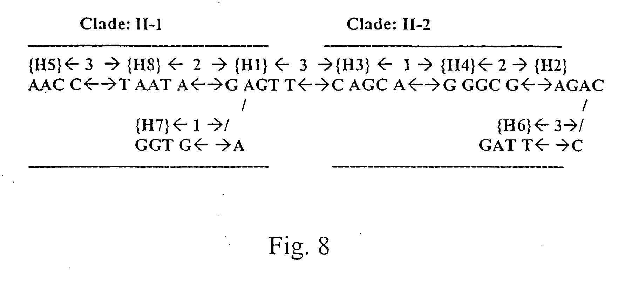

Figure 8 is cladogram for OCA3LOC922, as described in Example 11.

## DETAILED DESCRIPTION OF THE INVENTION

The invention relates to methods for inferring a genetic pigmentation trait of a mammalian subject from a nucleic acid sample or a polypeptide sample of the subject, and compositions for practicing such methods. The methods of the invention are based, in part, on the identification of single nucleotide polymorphisms (SNPs) that, alone or in combination, allow an inference to be drawn as to a genetic pigmentation trait such as hair shade, hair color, eye shade, or eye color, and further allow an inference to be drawn as to race. As such, the compositions and methods of the invention are useful, for example, as forensic tools for obtaining information relating to physical characteristics of a potential crime victim or a perpetrator of a crime from a nucleic acid sample present at a crime scene, and as tools to assist in breeding domesticated animals, livestock, and the like to contain a pigmentation trait as desired.

In one aspect, the invention provides a method for inferring a genetic pigmentation trait of a mammalian subject from a biological sample of the subject by identifying in the biological sample at least one pigmentation-related haplotype allele of at least one pigmentation gene. The pigmentation gene can be oculocutaneous albinism II (OCA2), agouti signaling protein (ASIP), tyrosinase-related protein 1 (TYRP1), tyrosinase (TYR), adaptor-related protein complex 3, beta I subunit (AP3B1) (also known as adaptin B I protein (ADP1)), adaptin 3 D subunit 1(AP3D1), dopachrome tautomerase (DCT), silver homolog (SILV), AIM-1 protein (LOC51151), proopiomelanocortin (POMC), ocular albinism 1 (OA1), microphthalmia-associated transcription factor (MITF), myosin VA (MYO5A), RAB27A, or coagulation factor II (thrombin) receptor-like 1 (F2RL1. The haplotype allele of the penetrant pigmentation-related haplotype is associated with the pigmentation trait, thereby allowing an inference to be drawn regarding the genetic pigmentation trait of the subject.

As disclosed herein, the identification of at least one penetrant pigmentation-related haplotype allele of at least one pigmentation gene allows an inference to be drawn as to a genetic pigmentation trait of a mammalian subject. An inference drawn according to a method of the invention can be strengthened by identifying a second, third, fourth or more penetrant pigmentation related haplotype alleles and/or one or more latent pigmentation related haplotype alleles in the same pigmentation gene or in one or more other genes. Accordingly, the method can further include identifying in the nucleic acid sample at least one pigmentation-related haplotype allele of at least a second pigmentation gene. The second pigmentation gene can be OCA2, ASIP, TYRP1, TYR, AP3B1, AP3D1, DCT, SILV, LOC51151, POMC, OA1, MITF, MYO5A, RAB27A, F2RL1, or melanocortin-1 receptor (MC1R), or any combination of these genes.

By way of example, the pigmentation gene for this aspect of the invention can include at least one ofOCA2, ASIP, TYRP1, TYR, SILV AP3B1, AP3D1, or DCT. As disclosed in the Examples included herein, such as Examples 17 and 18, penetrant and/or latent haplotypes and haplotype alleles for these genes are provided. In certain embodiments, the pigmentation-related haplotype allele is a penetrant pigmentation-related haplotype allele. By way of example, where the pigmentation-related haplotype allele is a penetrant pigmentation-related haplotype allele, the pigmentation trait can be eye shade, eye color, hair shade, or hair color. Furthermore, where the pigmentation trait is eye shade or eye color the pigmentation-related haplotype allele can occur in at least one of OCA2, TYRP1, or DCT. Penetrant haplotypes for eye color inference from these genes are identified herein (see Example 17).

As used herein, the term "at least one", when used in reference to a gene, SNP, haplotype, or the like, means 1, 2, 3, 4, 5, 6, 7, 8, 9, 10, etc., up to and including all of the exemplified pigmentation-related haplotype alleles, pigmentation genes, or pigmentation-related SNPs. Reference to "at least a second" gene, SNP, or the like, for example, a pigmentation gene, means two or more, i.e., 2, 3, 4, 5, 6, 7, 8, 9, 10, etc., pigmentation genes.

The term "haplotypes" as used herein refers to groupings of two or more nucleotide SNPs present in a gene. The term "haplotype alleles" as used herein refers to a non-random combination of nucleotide occurrences of SNPs that make up a haplotype. Haplotype alleles are much like a string of contiguous sequence bases, except the SNPs are not adjacent to one another on a chromosome. For example, the SNPs OCA2_5 and OCA2_8 can be included as part of the same haplotype, but they are about 60,000 base pairs apart from one another.

"Penetrant pigmentation-related haplotype alleles" are haplotype alleles whose association with a pigmentation trait is strong enough that it can be detected using simple genetics approaches. Corresponding haplotypes of penetrant pigmentation-related haplotype alleles, are referred to herein as "penetrant pigmentation-related haplotypes." Similarly, individual nucleotide occurrences of SNPs are referred to herein as "penetrant pigmentation-related SNP nucleotide occurrences" if the association of the nucleotide occurrence with a pigmentation trait is strong enough on its own to be detected using simple genetics approaches, or if the SNP loci for the nucleotide occurrence make up part of a penetrant haplotype. The corresponding SNP loci are referred to herein as "penetrant pigmentation-related SNPs." Haplotype alleles of penetrant haplotypes are also referred to herein as "penetrant haplotype alleles" or "penetrant genetic features." Penetrant haplotypes arc also referred to herein as "penetrant genetic feature SNP combinations."

Latent pigmentation-related haplotype alleles arc haplotype alleles that, in the context of one or more penetrant haplotypes, strengthen the inference of the genetic pigmentation trait. Latent pigmentation-related haplotype alleles arc typically alleles whose association with a pigmentation trait is not strong enough to be detected with simple genetics approaches. Latent pigmentation-related SNPs are individual SNPs that make up latent pigmentation-related haplotypes. As disclosed in Example 17, latent pigmentation-related SNPs show unusual minor allele frequency differences between Caucasians and Africans/Asians combined. Therefore, it will be recognized that, based on the teachings disclosed herein, additional latent pigmentation-related SNPs can be identified using routine methods.

Table 1 identifies and provides information regarding SNPs disclosed herein that are preferentially associated with eye pigmentation and/or hair pigmentation. All of the SNPs of the methods and compositions of the invention have nucleotide occurrences that preferentially segregate for hair shade or eye shade. Table 1 sets out the marker number, a SEQ ID NO: for the SNP and surrounding nucleotide sequences in the genome, and the position of the SNP within the sequence listing entry for that SNP and surrounding sequences. From this information, the SNP loci can be identified within the human genome.

**Table 1. Exemplary Race-Related and/or Pigmentation-Related SNPs**

| SEQ ID NO: | MARKER | POSITION OF SNP IN SEQ ID |
|---|---|---|
| 1 | 702 | 609 |
| 2 | 650 | 501 |
| 3 | 675 | 256 |
| 4 | 217438 | 442 |
| 5 | 217439 | 619 |
| 6 | 217441 | 646 |
| 7 | 217458 | 135 |
| 8 | 886894 | 193 |
| 9 | 886895 | 228 |
| 10 | 886896 | 245 |
| 11 | 217452 | 189 |
| 12 | 712052 | 573 |
| 13 | 886994 | 245 |
| 14 | 712057 | 643 |
| 15 | 712058 | 539 |
| 16 | 712060 | 418 |

(continued)

| SEQ ID NO: | MARKER | POSITION OF SNP IN SEQ ID |
|---|---|---|
| 17 | 712064 | 795 |
| 18 | 712054 | 535 |
| 19 | 712056 | 554 |
| 20 | 886892 | 210 |
| 21 | 217455 | 225 |
| 22 | 712061 | 170 |
| 23 | 886938 | 172 |
| 24 | 886943 | 216 |
| 25 | 560 | 61 |
| 26 | 552 | 201 |
| 27 | 559 | 201 |
| 28 | 468 | 201 |
| 29 | 657 | 356 |
| 30 | 674 | 599 |
| 31 | 632 | 267 |
| 32 | 701 | 61 |
| 33 | 710 | 451 |
| 34 | 217456 | 326 |
| 35 | 656 | 61 |
| 36 | 662 | 61 |
| 37 | 637 | 61 |
| 38 | 278 | 93 |
| 39 | 386 | 114 |
| 40 | 217480 | 558 |
| 41 | 951497 | 221 |
| 42 | 217468 | 660 |
| 43 | 217473 | 163 |
| 44 | 217485 | 364 |
| 45 | 217486 | 473 |
| 46 | 869787 | 314 |
| 47 | 869745 | 224 |
| 48 | 886933 | 169 |
| 49 | 886937 | 214 |
| 50 | 886942 | 903 |
| 51 | 217459 | 207 |
| 52 | 217460 | 428 |
| 53 | 217487 | 422 |
| 54 | 217489 | 459 |

(continued)

| SEQ ID NO: | MARKER | POSITION OF SNP IN SEQ ID |
|---|---|---|
| 55 | 554353 | 1528 |
| 56 | 554363 | 1093 |
| 57 | 554368 | 1274 |
| 58 | 554370 | 1024 |
| 59 | 554371 | 1159 |
| 60 | 615921 | 484 |
| 61 | 615925 | 619 |
| 62 | 615926 | 551 |
| 63 | 664784 | 1177 |
| 64 | 664785 | 1185 |
| 65 | 664793 | 1421 |
| 66 | 664802 | 1466 |
| 67 | 664803 | 1311 |
| 68 | 712037 | 808 |
| 69 | 712047 | 1005 |
| 70 | 712051 | 743 |
| 71 | 712055 | 418 |
| 72 | 712059 | 884 |
| 73 | 712043 | 744 |
| 74 | 756239 | 360 |
| 75 | 756251 | 455 |
| 76 | 809125 | 519 |
| 77 | 869769 | 277 |
| 78 | 869772 | 227 |
| 79 | 869777 | 270 |
| 80 | 869784 | 216 |
| 81 | 869785 | 172 |
| 82 | 869794 | 176 |
| 83 | 869797 | 145 |
| 84 | 869798 | 164 |
| 85 | 869802 | 166 |
| 86 | 869809 | 213 |
| 87 | 869810 | 218 |
| 88 | 869813 | 157 |
| 89 | 886934 | 837 |
| 90 | 886993 | 229 |
| 91 | 951526 | 160 |

Data regarding the nucleotide occurrences at many of these SNPs in hair color or eye color can be found in Tables 9-1 and 18-1, for eye shade and hair shade, respectively. Additionally, Tables 9-1 and 18-1 include the name and marker numbers for the SNPs identified as pigmentation-related and/or race-related herein, justifications explaining the association between a SNP and a pigmentation trait, as well as the name and Genbank accession number of the gene from which a SNP occurs.

Polymorphisms are allelic variants that occur in a population. The polymorphism can be a single nucleotide difference present at a locus, or can be an insertion or deletion of one or a few nucleotides. As such, a single nucleotide polymorphism (SNP) is characterized by the presence in a population of one or two, three or four nucleotides (i.e., adenosine, cytosine, guanosine or thymidine) at a particular locus in a genome such as the human genome. Accordingly, it will be recognized that, while the methods of the invention are exemplified primarily by the detection of SNPs, the disclosed methods or others known in the art similarly can be used to identify other polymorphisms in the exemplified or other pigmentation-related and/or race-related genes.

Simple genetic approaches for discovering penetrant pigmentation-related haplotype alleles include analyzing allele frequencies in populations with different phenotypes for a pigmentation trait being analyzed, to discover those haplotypes that occur more or less frequently in individuals with a certain pigmentation trait phenotype, for example, blue eyes. In such simple genetics methods SNP nucleotide occurrences in different pigmentation traits, such as eye shade or hair shade, are scored and distribution frequencies, such as those shown in Tables 9-1 and 18-1 arc analyzed. The Examples provide illustrations of using simple genetics approaches to discover penetrant haplotypes, and disclose methods that can be used to discover other pigmentation-related haplotype and their alleles, and, therefore, pigmentation-related SNPs that make up the pigmentation-related haplotypes.

Haplotypes can be inferred from genotype data corresponding to certain SNPs using the Stephens and Donnelly algorithm (Am. J. Hum. Genet. 68:978-989, 2001). Haplotype phases (i.e., the particular haplotype alleles in an individual) can also be determined using the Stephens and Donnelly algorithm (Am, J. Hum. Genet. 68:978-989, 2001). Software programs are available which perform this algorithm (e.g., The PHASE program, Department of Statistics, University of Oxford).

In one example, called the Haploscope method *(See* U.S. Pat. Appln. No. 10/120,804 entitled "METHOD FOR THE IDENTIFICATION OF GENETIC FEATURES FOR COMPLEX GENETICS CLASSISFIERS," filed April 11, 2002) a candidate SNP combination is selected from a plurality of candidate SNP combinations for a gene associated with a genetic trait. Haplotype data associated with this candidate SNP combination are read for a plurality of individuals and grouped into a positive-responding group and a negative-responding group based on whether predetermined trait criteria for an individual are met. A statistical analysis (as discussed below) on the grouped haplotype data is performed to obtain a statistical measurement associated with the candidate SNP combination. The acts of selecting, reading, grouping, and performing are repeated as necessary to identify the candidate SNP combination having the optimal statistical measurement. In one approach, all possible SNP combinations are selected and statistically analyzed. In another approach, a directed search based on results of previous statistical analysis of SNP combinations is performed until the optimal statistical measurement is obtained. In addition, the number of SNP combinations selected and analyzed may be reduced based on a simultaneous testing procedure.

As used herein, the term "infer" or "inferring", when used in reference to a genetic pigmentation trait or race, means drawing a conclusion about a pigmentation trait or about the race of a subject using a process of analyzing individually or in combination nucleotide occurrence(s) of one or more pigmentation-related or race-related SNP(s) in a nucleic acid sample of the subject, and comparing the individual or combination of nucleotide occurrence(s) of the SNP (s) to known relationships of nucleotide occurrence(s) of the pigmentation-related or race-related SNP(s). As disclosed herein, the nucleotide occurrence(s) can be identified directly by examining nucleic acid molecules, or indirectly by examining a polypeptide encoded by a particular gene, for example, an OCA2 gene, wherein the polymorphism is associated with an amino acid change in the encoded polypeptide.

Methods of performing such a comparison and reaching a conclusion based on that comparison are exemplified herein (see Example 17). The inference typically involves using a complex model that involves using known relationships of known alleles or nucleotide occurrences as classifiers. As illustrated in Example 17, the comparison can be performed by applying the data regarding the subject's pigmentation-related haplotype allele(s) to a complex model that makes a blind, quadratic discriminate classification using a variance-covariance matrix. Various classification models are discussed in more detail herein, and illustrated in the Examples.

To determine whether haplotypes are useful in an inference of a pigmentation trait, numerous statistical analysis can be performed. Allele frequencies can be calculated for haplotypes and pair-wise haplotype frequencies estimated using an EM algorithm (Excoffier and Slatkin, Mol Biol Evol. 1995 Sep; 12(5):921-7). Linkage disequilibrium coefficients can then be calculated. In addition to various parameters such as linkage disequilibrium coefficients, allele and haplotype frequencies (within ethnic, control and case groups), chi-square statistics and other population genetic parameters such as Panmitic indices can be calculated to control for ethnic, ancestral or other systematic variation between the case and control groups.

Markers/haplotypes with value for distinguishing the case matrix from the control, if any, can be presented in mathematical form describing any relationship and accompanied by association (test and effect) statistics. A statistical analysis result which shows an association of a SNP marker or a haplotype with a pigmentation trait with at least 80%, 85%, 90%, 95%, or 99%, most preferably 95% confidence, or alternatively a probability of insignificance less than 0.05, can be used to identify penetrant haplotypes, as illustrated in Example 17. These statistical tools may test for significance related to a null hypothesis that an on-test SNP allele or haplotype allele is not significantly different between the groups. If the significance of this difference is low, it suggests the allele is not related to the a pigmentation trait. The discovery of penetrant haplotype alleles can be verified and validated as genetic features for pigmentation using a nested contingency analysis of haplotype cladograms, as illustrated in Example 17.

It is beneficial to express polymorphisms in terms of multi-locus haplotypes because, as disclosed in the Examples provided herein, far fewer haplotypes exist in the world population than would be predicted based on the expectations from random allele combinations. For example, as disclosed in Example 2, for the three disclosed polymorphic loci within the OCA2 gene, OCA2_5 (G/A), OCA2_8 (T/C), and OCA2_6 (G/A), there would be $2^3 = 8$ possible haplotype combinations observed in the population - ATG, ACG, GCG, GTG, ACA, GCA, ATA and GTA. With the first letter in each haplotype allele corresponding to the first SNP, OCA2_5, the second letter corresponding to the nucleotide occurrence of the second SNP (OCA2_5) in the haplotype, and the third letter corresponding to the nucleotide occurrence of the third SNP (OCA2_8) of the haplotype. The various haplotype alleles exemplified above can be considered possible or potential "flavors" of the OCA2 gene in the population. However, for the OCA2 SNPs listed above, four haplotypes or "flavors" have been observed in real data from people of the world- ATG, ACG, GCG and GCA. The observance of a number of haplotypes in nature that is far fewer than the number of haplotypes possible is common and appreciated as a general principle among those familiar with the state of the art, and it is commonly accepted that haplotypes offer enhanced statistical power for genetic association studies. This phenomenon is caused by systematic genetic forces such as population bottlenecks, random genetic drift, selection, and the like, which have been at work in the population for millions of years, and have created a great deal of genetic "pattern" in the present population. As a result, working in terms of haplotypes offers a geneticist greater statistical power to detect associations, and other genetic phenomena, than working in terms of disjointed genotypes. For larger numbers of polymorphic loci the disparity between the number of observed and expected haplotypes is larger than for smaller numbers of loci. The various haplotype alleles exemplified above can be considered as all possible or potential "flavors" of the OCA2 gene in the population. However, for the OCA2 SNPs listed above, only four haplotypes or "flavors" have been observed thus far in real data from people of the world. For larger numbers of polymorphic loci the disparity between the number of observed and expected haplotypes can be larger. Such a phenomenon is caused, in part, by systematic genetic forces such as population bottlenecks, random genetic drift, selection, and the like, which have been at work in the population for millions of years, and have created a great deal of genetic "pattern" in the present population. As a result, working in terms of haplotypes offers a geneticist greater statistical power to detect associations, and other genetic phenomena, than does working in terms of disjointed genotypes.

In diploid organisms such as humans, somatic cells, which are diploid, include two alleles for each haplotype. As such, in some cases, the two alleles of a haplotype are referred to herein as a genotype, and the analysis of somatic cells, such as skin cells obtained at a crime scene, typically identifies the alleles for each copy of the haplotype. These alleles can be identical (homozygous) or can be different (heterozygous). The haplotypes of a subject can be symbolized by representing alleles on the top and bottom of a slash (e.g., ATG/CTA or GTT/AGA), where the sequence on the top of the slash represents the combination of polymorphic alleles on the maternal chromosome and the other, the paternal (or vice versa). Although the methods of the invention are illustrated using analysis of diploid cells (see Examples), the analysis similarly can be applied to haploid cells, such as sperm cells. When using haploid sequences, the contingency table from a population study that is used to derive the factor scores for quadratic discrimination, becomes a table of haploid sequences versus pigmentation classes. The dimensionality of the problem is lower, and therefore the classifications more simple, accomplished faster, and are slightly more accurate. Thus the variance-covariance matrix takes on a slightly different form, but is generally the same.

As disclosed herein, the power of the inference of a pigmentation trait can be improved using specific combinations of haplotypes, including penetrant and latent haplotypes. As shown, for example, in Example 17, such combinations improve the accuracy of an inference drawn according to a method of the invention. This result is not unreasonable in view, for example, of genetic epistasis, wherein specific combinations of genes have unique impacts on traits.

The methods and compositions of the invention allow complex gcnomics solutions for eye, hair, and skin pigmentation and, therefore, provide numerous utilities. For example, the methods and compositions are useful as forensic tools in human subjects. Pigmentation solutions for eye color also can have relevance for pigmentation related disease research focused, for example, on cataracts (Cumming et al., Am. J. Opthalmol. 130:237-238, 2000), late-onset blindness, and melanoma (Brogelli et al., Br. J. Dermatol. 125: 349-52, 1991; Palmer et al., Am. J. Hum. Genet. 66: 176-86, 2000).

A sample useful for practicing a method of the invention can be any biological sample of a subject that contains nucleic acid molecules, including portions of the gene sequences to be examined, or corresponding encoded polypeptides, depending on the particular method. As such, the sample can be a cell, tissue or organ sample, or can be a sample of a biological fluid such as semen, saliva, blood, and the like. A nucleic acid sample useful for practicing a method of the invention will depend, in part, on whether the SNPs of the haplotype to be identified are in coding regions or in non-coding regions. Thus, where at least one of the SNPs to be identified is in a non-coding region, the nucleic acid sample generally is a deoxyribonucleic acid (DNA) sample, particularly genomic DNA or an amplification product thereof. However, where heteronuclear ribonucleic acid (RNA), which includes unspliced mRNA precursor RNA molecules, is available, a cDNA or amplification product thereof can be used. Where the each of the SNPs of the haplotype is present in a coding region of the pigmentation gene(s), the nucleic acid sample can be DNA or RNA, or products derived therefrom, for example, amplification products. Furthermore, while the methods of the invention generally are exemplified with respect to a nucleic acid sample, it will be recognized that particular haplotype alleles can be in coding regions of a gene and can result in polypeptides containing different amino acids at the positions corresponding to the SNPs due to non-degenerate codon changes. As such, in another aspect, the methods of the invention can be practiced using a sample containing polypeptides of the subject.

Methods of the invention can be practiced with respect to human subjects and, therefore, can be particularly useful for forensic analysis. In a forensic application or a method of the invention, the human nucleic acid sample can be obtained from a crime scene, using well established sampling methods. Thus, the sample can be fluid sample or a swab sample For example, the sample can be a swab sample, blood stain, semen stain, hair follicle, or other biological specimen, taken from a crime scene, or can be a soil sample suspected of containing biological material of a potential crime victim or perpetrator, can be material retrieved from under the finger nails of a potential crime victim, or the like, wherein nucleic acids (or polypeptides) in the sample can be used as a basis for drawing an inference as to a pigmentation trait according to a method of the invention.

A mammalian subject that can be examined according to a method of the invention can be any mammalian species. In particular, the methods are applicable to drawing an inference as to a pigmentation trait of a human subject. The human subject can be from a general population of mixed ethnicity, or the human subject can be of a particular ethnic background or race. For example, the subject can be a Caucasian.

By way of example, a method of the invention can be performed using a biological sample from a human subject, the genetic pigmentation trait to be inferred can be eye color or eye shade, and the penetrant pigmentation-related haplotype allele can be from at least one of the following pigmentation-related haplotypes:

a) nucleotides of the DCT gene corresponding to a DCT-A haplotype, which includes, nucleotide 609 of SEQ ID NO:1 [702], nucleotide 501 of SEQ ID NO:2 [650], and nucleotide 256 of SEQ ID NO:3 [marker 675];
b) nucleotides of the MC1R gene corresponding to a melanocortin-1 receptor (MC1R)-A haplotype, which includes nucleotide 442 of SEQ ID NO:4 [217438], nucleotide 619 of SEQ ID NO:5 [217439], and nucleotide 646 of SEQ ID NO:6 [217441];
c) nucleotides of the OCA2 gene, corresponding to an OCA2-A haplotype, which includes nucleotide 135 of SEQ ID NO:7 [217458], nucleotide 193 of SEQ ID NO:8 [886894], nucleotide 228 of SEQ ID NO:9 [marker 886895], and nucleotide 245 of SEQ ID NO:10 [marker 886896];
d) nucleotides of the OCA2 gene, corresponding to an OCA2-B haplotype, which includes nucleotide 189 of SEQ ID NO:11 [marker 217452]], nucleotide 573 of SEQ ID NO:12 [marker 712052], and nucleotide 245 of SEQ ID NO: 13 [marker 886994];
e) nucleotides of the OCA2 gene, corresponding to an OCA2-C haplotype, which includes nucleotide 643 of SEQ ID NO:14 [712057], nucleotide 539 of SEQ ID NO:15 [712055], nucleotide 418 of SEQ ID NO:16 [712060], and nucleotide 795 of SEQ ID NO:17, [712064];
f) nucleotides of the OCA2 gene, corresponding to an OCA2-D haplotype, which includes nucleotide 535 of SEQ ID NO:18, [712054], nucleotide 554 of SEQ ID NO:19, [712056], and nucleotide 210 of SEQ ID NO:20, [886892];
g) nucleotides of the OCA2 gene, corresponding to an OCA2-E haplotype, which includes nucleotide 225 of SEQ ID NO:21, [217455], nucleotide 170 of SEQ ID NO:22, [712061], and nucleotide 210 of SEQ ID NO:20, [886892]; or
h) nucleotides of the TYRP1 gene corresponding to a TYRP1-B haplotype which includes: nucleotide 172 of SEQ ID NO:23, [886938], and nucleotide 216 of SEQ ID NO:24; [886943], or any combination of a) through h). The above listed haplotypes provide preferred penetrant pigmentation-related haplotypes for eye pigmentation (see Example 17). To improve the power of the inference, the pigmentation-related haplotype can be all of the above listed pigmentation-related haplotypes.

This list of penetrant pigmentation-related SNPs are preferred penetrant pigmentation-related SNPs for eye color, as illustrated in Example 17.

It will be recognized by one skilled in the art that the invention includes any 1 of the pigmentation-related haplotypes, alone, or any combination of 2, 3, 4, or more, including, for example all 8 pigmentation-related haplotypes listed above.

A method of the invention, which can include methods wherein the pigmentation-rotated haplotype alleles are determined for the preferred penetrant pigmentation-rotated haplotypes for eye pigmentation, the subject is a human, and the genetic pigmentation trait is eye color or eye shade, can further include identifying in the nucleic acid sample a nucleotide occurrence of at least one latent pigmentation-related SNP of a pigmentation gene, thereby improving the power of the inference of eye color or eye shade. The latent pigmentation-related SNP can be, for example, one or more of nucleotide 61 of SEQ ID NO:25 [marker 560], nucleotide 201 of SEQ ID NO:26 [marker 552], nucleotide 201 of SEQ ID NO:27 [marker 559], nucleotide 201 of SEQ ID NO:28 [marker 468], nucleotide 657 of SEQ ID NO: 29 [marker 657], nucleotide 599 of SEQ ID NO:30 [marker 674], nucleotide 267 of SEQ ID NO:31 [marker 632], nucleotide 61 of SEQ ID NO:32 [marker 701], nucleotide 451 of SEQ ID NO:33 [marker 710]; nucleotide 326 of SEQ ID NO:34 [marker 217456], nucleotide 61 of SEQ ID NO:35 [marker 656], nucleotide 61 of SEQ ID NO:36, nucleotide 61 of SEQ ID NO:37 [marker 637], nucleotide 93 of SEQ ID NO:38 [marker 278], nucleotide 114 of SEQ ID NO:39 [marker 386], nucleotide 558 of SEQ ID NO:40 [marker 217480], nucleotide 221 of SEQ ID NO:41 [marker 951497], nucleotide 660 of SEQ ID NO:42 [marker 217468], nucleotide 163 of SEQ ID NO:43 [marker 217473], nucleotide 364 of SEQ ID NO:44 [marker 217485], nucleotide 473 of SEQ ID NO:45 [marker 217486], nucleotide 314 of SEQ ID NO:46 [marker 869787], nucleotide 224 of SEQ ID NO:47 [marker 869745], nucleotide 169 of SEQ ID NO:48 [marker 886933], nucleotide 214 of SEQ ID NO:49 [marker 886937], or nucleotide 903 of SEQ ID NO:50; [886942], or any combination thereof. The above-listed latent pigmentation-related SNPs provide preferred latent pigmentation-related SNPs related to eye color (see Example 17). According to this aspect of a method of the invention, latent pigmentation-related haplotype allele can be:

i) nucleotides of the ASIP gene corresponding to an ASIP-A haplotype, which includes nucleotide 201 of SEQ-ID NO:26 [marker 552], and nucleotide 201 of SEQ ID NO:28 [marker 468];

j) nucleotides of the DCT gene corresponding to a DCT-B haplotype, which includes nucleotide 451 of SEQ ID NO: 33 [marker 710], and nucleotide 657 of SEQ ID NO:29 [marker 657];

k) nucleotides of the SILV gene corresponding to a SILV-A haplotype, which includes nucleotide 61 of SEQ ID NO: 35 [marker 656], and nucleotide 61 of SEQ ID NO:36 ;

l) nucleotides of the TYR gene corresponding to a TYR-A haplotype, which includes nucleotide 93 of SEQ ID NO: 38 [marker 278], and nucleotide 114 of SEQ ID NO:39 [marker 386]; or

m) nucleotides of the TYRP1-A gene corresponding to a TYRP1-A haplotype, which includes nucleotide 364 of SEQ ID NO:44 [marker 217485], nucleotide 169 of SEQ ID NO:48 [marker 886933], and nucleotide 214 of SEQ ID NO: 49 [marker 886937], or any combination of i) through m).

Further according to this aspect of a method of the invention, wherein the pigmentation-related haplotype alleles are determined for the preferred penetrant pigmentation-related haplotypes for eye pigmentation, the subject is a human, and the genetic pigmentation trait is eye color or eye shade, the method can further include identifying in the nucleic acid sample all of the above listed following latent haplotypes.

In one embodiment, the penetrant pigmentation-trait related haplotypes for eye color can be one or more of the following:

a) the MC1R-A haplotype allele CCC;
b) the OCA2-A haplotype allele TTAA, CCAG, or TTAG;
c) the OCA2-B haplotype allele CAA, CGA, CAC, or CGC;
d) the OCA2-C haplotype allele GGAA, TGAA, or TAAA;
e) the OCA2-D haplotype allele AGG or GGG;
f) the OCA2-E haplotype allele GCA;
g) the TYRP1-B haplotype allele TC; and
h) the DCT-B haplotype allele CTG, or GTG.

These alleles are preferred penetrant pigmentation-related haplotype alleles for eye color, as illustrated in Example 17.

In a preferred example with high inference power, the method of the invention wherein the pigmentation-related haplotype alleles are determined for the preferred penetrant pigmentation-related haplotypes for eye color or eye shade, the subject is a human, and the genetic pigmentation trait is eye color or eye shade, further include the following penetrant pigmentation-trait related haplotype alleles:

a) the MC1R-A haplotype allele CCC;
b) the OCA2-A haplotype allele TTAA, CCAG, or TTAG;
c) the OCA2-B haplotype allele CAA, CGA, CAC, or CGC;
d) the OCA2-C haplotype allele GGAA, TGAA, or TAAA;
e) the OCA2-D haplotype allele AGG or GGG;

f) the OCA2-E haplotype allele GCA;
g) the TYRP1-B haplotype allele TC; and
h) the DCT-B haplotype allele CTG, or GTG; and the following latent pigmentation-related haplotype alleles:
i) the ASIP-A haplotype allele GT or AT;
j) the DCT-B haplotype allele TA or TG;
k) the SILV-A haplotype allele TC, TT, or CC;
l) the TYR-A haplotype allele GA, AA or GG; and
m) the TYRP1-A haplotype allele GTG, TTG, or GTT.

The alleles listed in the preceding paragraph represent the group of penetrant and latent pigmentation-related haplotypes that are identified in Example 17. This combination of haplotypes when used to infer eye pigmentation using the classification model disclosed in Example 17, inferred eye shade for a group of 225 Caucasians with 99% accuracy for the inference of iris color shade, and 97% accuracy for the inference of actual eye colors.

In another aspect, the invention provides a method for inferring eye shade or color of a human subject from a biological sample of the subject by performing a nested contingency analysis of haplotypes. The method includes performing the steps described in Table 17-4.

In another aspect, the invention provides a method for inferring hair color or hair shade of a mammalian subject from a biological sample of the subject by identifying in the biological sample at least one pigmentation-related haplotype allele of at least one pigmentation gene. The biological sample can be (or contain) a nucleic acid sample. The pigmentation-related haplotype preferably includes a penetrant pigmentation-related haplotype. For example, where the pigmentation-related haplotype allele is a penetrant pigmentation-related haplotype allele, the penetrant pigmentation-related haplotype allele can occur in at least one of the OCA2, ASIP. TYRP1, or MC1R gene. To improve the power of the inference, a combination of penetrant pigmentation-related haplotype alleles from OCA2, ASIP, TYRP1 and MCIR can be identified, with exemplary penetrant haplotypes related to an inference of hair color or hair shade set forth in Example 18.

A method inferring hair color or hair shade can be performed using a biological sample from a human subject, and the penetrant pigmentation-related haplotype allele can occur in at least one pigmentation-related haplotypes, as follows:

a) nucleotides of the ASIP-B haplotype corresponding to:

nucleotide 202 of SEQ ID NO:27, [559], and
nucleotide 61 of SEQ ID NO:25, [560]

b) nucleotides of the MC1R-A haplotype corresponding to:

nucleotide 442 of SEQ ID NO:4, [217438], nucleotide 619 of SEQ ID NO:5 [217439], and
nucleotide 646 of SEQ ID NO:6 [217441];

c) nucleotides of the OCA2-G haplotype corresponding to:

nucleotide 418 of SEQ ID NO:16 [712060],
nucleotide 210 of SEQ ID NO:20, [886892], and
nucleotide 245 of SEQ ID NO:10 [marker 886896];

d) nucleotides of the OCA2-H haplotype corresponding to:

nucleotide 225 of SEQ ID NO:21, [217455], nucleotide 643 of SEQ ID NO:14 [712057], and
nucleotide 193 of SEQ ID NO:8 [886894];

e) nucleotides of the OCA2-I haplotype corresponding to:

nucleotide 135 of SEQ ID NO:7 [217458], and
nucleotide 554 of SEQ ID NO:19, [712056];

e) nucleotides of the OCA2-J haplotype corresponding to:

nucleotide 535 of SEQ ID NO:18, [712054], and

nucleotide 228 of SEQ ID NO:9 [marker 8S6895]; or

g) nucleotides of the TYRPI-C haplotype corresponding to:

nucleotide 473 of SEQ ID NO:45, [217486], and
nucleotide 214 of SEQ ID NO:49; [886937], or any combination thereof.

The haplotypes listed in elements a)-g) above are preferred penetrant pigmentation-related haplotypes for hair pigmentation, as illustrated in Example 18.

To improve the inference power, the method of this aspect of the invention directed at an inference drawn to hair color or hair shade, can be performed using a biological sample from a human subject by identifying a penetrant pigmentation-related haplotype allele in all of the following pigmentation-related haplotypes:

a) nucleotides of the ASIP-B haplotype corresponding to:

nucleotide 202 of SEQ ID NO:27, [559], and
nucleotide 61 of SEQ ID NO:25, [560]

b) nucleotides of the MC1R-A haplotype corresponding to:

nucleotide 442 of SEQ ID NO:4, [217438],
nucleotide 619 of SEQ ID NO:5 [217439], and
nucleotide 646 of SEQ ID NO:6 [217441];

c) nucleotides of the OCA2-G haplotype corresponding to:

nucleotide 418 of SEQ ID NO:16 [712060],
nucleotide 210 of SEQ ID NO:20, [886892], and
nucleotide 245 of SEQ ID NO:10 [marker 886896];

d) nucleotides of the OCA2-H haplotype corresponding to:

nucleotide 225 of SEQ ID NO:21, [217455],
nucleotide 643 of SEQ ID NO:14 [712057], and
nucleotide 193 of SEQ ID NO:8 [886894];

e) nucleotides of the OCA2-I haplotype corresponding to:

nucleotide 135 of SEQ ID NO:7 [217458], and
nucleotide 554 of SEQ ID NO:19, [712056];

e) nucleotides of the OCA2-J haplotype corresponding to:

nucleotide 535 of SEQ ID NO: 18, [712054], and
nucleotide 228 of SEQ ID NO:9 [marker 886895];

g) nucleotides of the TYRP1-C haplotype corresponding to:

nucleotide 473 of SEQ ID NO:45, [217486], and
nucleotide 214 of SEQ ID NO:49; [886937].

A method for inferring hair color or shade, wherein the pigmentation-related haplotype andes are determined for any one combination of the pigmentation-related haplotypes for the haplotypes listed as elements a)-g) above, can further include identifying at least one of the following alleles:

a) the ASIP-B haplotype allele GA or AA;
b) the MCIR-A haplotype allele CCC;
c) the OCA2-G haplotype allele AGG, or AGA;

d) the OCA2-H haplotype allele AGT or ATT;
e) the OCA2-I haplotype allele TG;
f) the OCA2-J haplotype allele GA or AA; and
h) the TYRP1-C haplotype allele AA or TA.

By way of an example with improved inference power, the method of the invention for inferring hair color or shade wherein the pigmentation-related haplotype alleles are determined for all of the alleles listed above.

The method of the invention for this aspect of the invention includes methods wherein the pigmentation-related haplotype alleles are those listed in elements a)-h) above, and wherein the method further includes identifying in the nucleic acid sample, at least one latent pigmentation-related SNP of a pigmentation gene, to improve the power of the inference of hair color or hair shade.

The mammalian subject can also be a livestock species, such as a cow, a sheep, a pig, or a goat, etc., or a cat, a horse, or a dog, or other domestic animal, or a mouse, a rat, or a rabbit, or other laboratory species. The methods of the invention when practiced on a non-human subject, utilize pigmentation genes of the species of the non-human subject. These pigmentation genes include homologs of the human pigmentation genes disclosed herein. For example, in mice such homologs are known to exist, and some studies directed at mutations of pigmentation genes have been performed. Although little is known regarding SNPs of pigmentation genes of non-human species, MC1R SNPs have been described to be associated with chestnut coat coloration in horses (Rieder et al., Mamm Genome. 12(6):450-5 (2001).

In mammalian species, especially non-human subjects, the methods of the invention are valuable in providing predictions of commercially valuable pigmentation phenotypes, for example in breeding. For example, by using the methods of the invention, the methods of the invention can be used to derive homologous methods in other species that can be used to breed a mammalian subject such that offspring will be more likely to have a desired pigmentation trait. Furthermore, early stage embryos can be isolated and analyzed using the methods of the invention to select before implantation, those that will develop into adults with a desired pigmentation trait, whether it be coat color, eye color, or any other trait linked to pigmentation.

The term "genetic pigmentation trait" is used herein to mean a trait involving variation in the degree to which melanin is deposited in a particular tissue. Such deposition generally occurs during development of a mammalian organism, and is a function of the degree to which melanin is synthesized and degraded. As exemplified herein, the pigmentation trait can be the degree of hair pigmentation, which can be analyzed in terms of hair color or hair shade; or the degree of eye pigmentation, which can be analyzed in terms of eye color or eye shade; or the degree of skin pigmentation. Melanin is synthesized, degraded, deposited, and transported by a group of genes referred to herein as pigmentation genes. Pigmentation genes are usually defined as such based on loss of function mutations observed in man as well as model organisms such as mouse or Drosophila.

For hair shade, individuals generally are partitioned into two groups - persons of dark natural hair color (black or brown) and persons of light natural hair color (red, blonde). The term "eye color" is synonymous with the degree to which the iris is pigmented; the term "hair color" is synonymous with the degree to which the hair is pigmented. For eye shade, typically individuals are partitioned into two groups; persons of dark natural eye color (i.e., individuals of brown or black irises) and individuals of light iris shade group (i.e., individuals of blue, green, or hazel irises). Therefore, by way of example, the methods of the invention can determine whether the eye color of a subject is blue, green, hazel, black, or brown.

The first pigmentation gene and, where appropriate, second or other pigmentation genes useful for examination according to a method of the invention can be any gene that is involved in the production, degradation, or transport of melanin. In certain preferred embodiments, the first pigmentation gene examined according to a method of the invention is not MC1R or is not MC1R and ASIP, although in these embodiments the MC1R or AS1P gene can be the second, third, fourth or other pigmentation gene examined, thus strengthening an inference that can be drawn. Pigmentation genes can be identified by performing wet lab experiments, or as illustrated in the Examples, by identifying published reports of studies describing genes for which mutations arc known to cause detectable changes in pigmentation. In humans, genes for which mutations cause severe hypopigmentation are especially attractive candidates as pigmentation genes for use in the disclosed methods.

Pigmentation genes can be identified based on evidence from the literature, and from other sources of information, that implicate them in either the synthesis, degradation and/or the deposition of the human chromatophore melanin. The Physicians Desk Reference, Online Mendelian Inheritance database (available at the National Center for Biotechnology Information web site) and PubMed/Medline are two examples of sources that provide such information.

Examples of pigmentation genes include OCA2, ASIP, OCA2, SILV, TYRP1, DCT, TYR, MC1R, and AP3B1. As disclosed herein, these pigmentation genes comprise loci of penetrant and/or latent SNP haplotypes for hair pigmentation (i.e., color and shade) and/or eye pigmentation (i.e., color and shade). The methods of the invention include the identification of pigmentation-related haplotype alleles for one pigmentation gene, as well as for any combination of two or more pigmentation genes, which can improve the power of the inference drawn. In certain aspects of the invention, the inferred pigmentation trait is eye shade and the pigmentation-related haplotype allele occurs in at least one of OCA2, TYRP1, or DCT. These genes are disclosed herein as including the loci of penetrant haplotypes associated with eye color and/or shade (see Example 17).

Mutations in the TYR, MCIR, TYRP1, and OCA genes have been shown to be deterministic for hereditary oculocutaneous albinism (reviewed in Oetting and King, Hum. Mutat. 13:99-115, 1999). Catastrophic mutations in any of these genes impair the synthesis and deposition of melanin in human epidermis. However, before the present study, relatively little was known about how these genes naturally vary in the non-albino population. For example, the human genome project has resulted in the generation of a publicly available human polymorphism database, which contains the location and identity of potential variants (SNPs) for many of the human genes. However, whether these potential variants are actual SNPs and whether they are associated with traits such as pigmentation-traits have not been reported.

Biochemical information is available regarding the function of pigmentation genes in the synthesis, degradation, and transport of melanin, including cumalanin (brown pigment) and pheomelanin (brown pigment). Eumelanin is a light absorbing polymer synthesized in specialized lysozomes called melanosomes in a specialized cell type called melanocytes. Within the melanosomes, the tyrosinase (TYR) gene product catalyzes the rate-limiting hydroxylation of tyrosine (to 3,4-dihydroxyphenylanine or DOPA) and oxidation of the resulting product (to DOPA quinone) to form the precursor for eumelanin synthesis. Though centrally important, pigmentation in animals is not simply a Mendelian function of TYR (or any other) gene sequences. In fact, study of the transmission genetics for pigmentation traits in man and various model systems suggests that variable pigmentation is a function of multiple, heritable factors whose interactions appear to be quite complex (Akey et al., Hum. Genet. 103:516-520, 2001; Brauer and Chopra, Anthropol. Anz. 36(2):109-120, 1978; Bito et al., Arch Ophthalmol. 115(5):659-663, 1997; Sturm et al., Gene 277:49-62, 2001; Box et al., Hum. Mole. Genet. 6:1891-1897, 1997; Box et al., Am. J. Hum. Genet. 69:765-773, 2001). For example, unlike human hair color (Sturm et al., Gene 277:49-62, 2001), there appears to be no dominance component for mammalian iris color determination (Brauer and Chopra, Anthropol. Anz. 36(2):109-120, 1978), and no correlation between skin, hair and iris color within or between individuals of a given population. In contrast, between-population comparisons show good concordance; populations with darker average iris color also tend to exhibit darker average skin tones and hair colors. These observations suggest that the genetic determinants for pigmentation in the various tissues are distinct, and that these determinants have been subject to a common set of systematic forces that have shaped their distribution in the worlds various populations.

At the cellular level, variable iris color in healthy humans is the result of the differential deposition of melanin pigment granules within a fixed number of stromal melanocytes in the iris (Imesch et al., Surv. Ophthalmol. 41 Suppl 2: S117-S123, 1997). The density of granules appears to reach genetically determined levels by early childhood and usually remains constant throughout later life (but, see Bito et al., Arch Ophthalmol. 115(5):659-663, 1997). Pedigree studies in the mid-scvcnties suggested iris color variation is a function of two loci; a single locus responsible for de-pigmentation of the iris, not affecting skin or hair, and another pleiotropic gene for reduction of pigment in all tissues (Brues, Am. J. Phys. Anthropol. 43(3):387-391, 1975). Most of what we have learned about pigmentation since has been derived from molecular genetics studies of rare pigmentation defects in man and model systems such as mouse and *Drosophila.* For example, dissection of the oculocutaneous albinism (OCA) trait in humans has shown that most pigmentation defects are due to lesions in one gene *(TYR)* resulting in their designation as tyrosinase (*TYR*) negative OCAs (Oetting and King, Hum. Mutat. 13:99-115, 1999; Oetting and King, Hum. Mutat. 2:1-6, 1993; Oetting and King, Hum. Genet. 90: 258-262, 1992; Oetting and King, Clin. Res. 39:267A, 1991. TYR catalyzes the rate-limiting step of melanin biosynthesis and the degree to which human irises are pigmented correlates well with the amplitude of *TYR* message levels (Lindsey et al., Arch. Opthalmol. 119(6):853-860, 2001). Nonetheless, the complexity of OCA phenotypes has illustrated that *TYR* is not the only gene involved in iris pigmentation (Lee et al., Hum. Molec. Genet. 3:2047-2051, 1994). Though most TYR- negative OCA patients are completely de-pigmented, dark-iris albino mice (C44H), and their human type IB oculocutaneous counterparts exhibit a lack of pigment in all tissues except for the iris (Schmidt and Beermann, Proc. Natl. Acad. Sci., U.S.A. 91(11):4756-4760, 1994).

Study of a number of other TYR-positive OCA phenotypes have shown that, in addition to *TYR,* the oculocutaneous 2 *(OCA2;* Durham-Pierre et al., Nature Genet. 7:176-179, 1994; Durham-Pierre et al., Hum. Mutat. 7:370-373, 1996; Gardner et al., Science 257:1121-1124, 1992; Hamabe et al., Am. J. Med. Genet. 4.1:54-63, 1991), tyrosinase like protein *(TYRP1;* Chintamaneni et al., Biochem. Biophys. Res. Commun. 178:227-235, 1991; Abbott et al., Genomics 11:471-473, 1991; Boissy et al., Am J. Hum. Genet. 58:1145-1156, 1996), melanocortin receptor (MC1 R; Robbins et al., Cell 72:827-834, 1993; Smith et al., J. Invest. Derm. 111:119-122; 1998; Flanagan et al., Hum. Molec. Genet. 9: 2531-2537, 2000) and adaptin 3B (AP3B; Ooi et al., EMBO J. 16(15):4508-451 S, 1997) loci, as well as other genes (reviewed by Sturm et al., Gene 277:49-62, 2001) arc necessary for normal human iris pigmentation. In *Drosophila,* iris pigmentation defects have been ascribed to mutations in over 85 loci contributing to a variety of cellular processes in melanocytes (Ooi ct al., EMBOJ. 16(15):4548-4518, 1997; Lloyd et al., Trends Cell Biol. 8(7):257-259, 1998), but mouse studies have suggested that about 14 genes preferentially affect pigmentation in vertebrates (reviewed in Sturm ct al.,

Gene 277:49-62, 2001), and that disparate regions of the *TYR* and other *OCA* genes arc functionally inequivalent for determining the pigmentation in different tissues.

Though research on pigment mutants has made clear that a small subset of genes is largely responsible for catastrophic pigmentation defects in mice and man, until the present disclosure, it remained unclear whether or how common single nucleotide polymorphisms (SNPs) in these genes contribute towards (or are linked to) natural variation in human iris color. A brown-iris locus was localized to an interval containing the MC1R gene (Eiberg and Mohr, Eur. J. Hum. Genet 4(4):237-241, 1996), and specific polymorphisms in the MC1R gene have been associated with red hair and blue iris color in relatively isolated Irish populations (Robbins et al., Cell 72:827-834, 1993; Smith et al., J. Invest. Derm. 111:119-122, 1998; Flanagan et al., Hum. Molec. Genet. 9:2531-2537, 2000; Valverde et al., Nature Genet. 11: 328-330, 1995; Koppula et al., Hum. Mutat. 9:30-36, 1997). An ASIP polymorphism was also recently described that may be associated with both brown iris and hair color (Kanetsky et al., Am J. Hum. Gen. 70:770-775, 2002) However, the penetrance of each of the MC1R and ASIP alleles is low and in general, they appear to explain only a very small amount of the overall variation in iris colors within the human population (Spritz, Nature Cenet. 11:225-226, 1995). Such studies for associating genes and traits are gene-centric in that alleles descriptive of variant gene loci are considered as definitive and focal objects. To date, however, these methods have not worked well because most human traits are complex and genetic wholes are often times greater than the sum of its parts. As such, innovative genomics-based study designs and analytical methods for screening genetic data *in silico,* such as the methods disclosed herein, are needed that are respectful of genetic complexity (for example, the components of dominance and epistatic genetic variance).

Numerous methods for identifying haplotype alleles in nucleic acid samples (also referred to a surveying the genome) are disclosed herein or otherwise known in the art. As disclosed herein, nucleic acid occurrences for the individual SNPs that make up the haplotype alleles are determined, then, the nucleic acid occurrence data for the individual SNPs is combined to identify the haplotype alleles. For example, for the OCA2-A haplotype, both nucleotide occurrences at each SNP loci corresponding to markers 217458, 886894, and 886895 can be combined to determine a the two OCA2-A haplotype alleles of a subject (i.e., OCA2-A genotype; see Example 17). The Stephens and Donnelly algorithm (Am. J. Hum. Genet. 68:978-989, 2001, which is incorporated herein by reference) can be applied to the data generated regarding individual nucleotide occurrences in SNP markers of the subject, in order to determine the alleles for each haplotype in the subject's genotype. Other methods that can be used to determine alleles for each haplotype in the subject's genotype, for example Clarks algorithm, and an EM algorithm described by Raymond and Rousset (Raymond et al. 1994. GenePop. Ver 3.0. Institut des Siences de l'Evolution. Universite de Montpellier, France. 1994)

The attached sequence listing provides flanking nucleotide sequences for the SNPs disclosed herein. These flanking sequence serve to aid in the identification of the precise location of the SNPs in the human genome, and serve as target gene segments useful for performing methods of the invention. A target polynucleotide typically includes a SNP locus and a segment of a corresponding gene that flanks the SNP. Primers and probes that selectively hybridize at or near the target polynucleotide sequence, as well as specific binding pair members that can specifically bind at or near the target polynucleotide sequence, can be designed based on the disclosed gene sequences and information provided herein.

As used herein, the term "selective hybridization" or "selectively hybridize," refers to hybridization under moderately stringent or highly stringent conditions such that a nucleotide sequence preferentially associates with a selected nucleotide sequence over unrelated nucleotide sequences to a large enough extent to be useful in identifying a nucleotide occurrence of a SNP. It will be recognized that some amount of non-specific hybridization is unavoidable, but is acceptable provide that hybridization to a target nucleotide sequence is sufficiently selective such that it can be distinguished over the non-specific cross-hybridization, for example, at least about 2-fold more selective, generally at least about 3-fold more selective, usually at least about 5-fold more selective, and particularly at least about 10-fold more selective, as determined, for example, by an amount of labeled oligonucleotide that binds to target nucleic acid molecule as compared to a nucleic acid molecule other than the target molecule, particularly a substantially similar (i.e., homologous) nucleic acid molecule other than the target nucleic acid molecule. Conditions that allow for selective hybridization can be determined empirically, or can be estimated based, for example, on the relative GC:AT content of the hybridizing oligonucleotide and the sequence to which it is to hybridize, the length of the hybridizing oligonucleotide, and the number, if any, of mismatches between the oligonucleotide and sequence to which it is to hybridize (see, for example, Sambrook et al., "Molecular Cloning: A laboratory manual (Cold Spring Harbor Laboratory Press 1989)).

An example of progressively higher stringency conditions is as follows: 2 x SSC/0.1% SDS at about room temperature (hybridization conditions); 0.2 x SSC/0.1% SDS at about room temperature (low stringency conditions); 0.2 x SSC/0.1% SDS at about 42EC (moderate stringency conditions); and 0.1 x SSC at about 68EC (high stringency conditions). Washing can be carried out using only one of these conditions, e.g., high stringency conditions, or each of the conditions can be used, e.g., for 10-15 minutes each, in the order listed above, repeating any or all of the steps listed. However, as mentioned above, optimal conditions will vary, depending on the particular hybridization reaction involved, and can be determined empirically.

The term "polynucleotide" is used broadly herein to mean a sequence of deoxyribonucleotides or ribonucleotides that are linked together by a phosphodiester bond. For convenience, the term "oligonucleotide" is used herein to refer to a polynucleotide that is used as a primer or a probe. Generally, an oligonucleotide useful as a probe or primer that selectively hybridizes to a selected nucleotide sequence is at least about 15 nucleotides in length, usually at least about 18 nucleotides, and particularly about 21 nucleotides or more in length.

A polynucleotide can be RNA or can be DNA, which can be a gene or a portion thereof, a cDNA, a synthetic polydeoxyribonucleic acid sequence, or the like, and can be single stranded or double stranded, as well as a DNA/RNA hybrid. In various embodiments, a polynucleotide, including an oligonucleotide (e.g., a probe or a primer) can contain nucleoside or nucleotide analogs, or a backbone bond other than a phosphodiester bond. In general, the nucleotides comprising a polynucleotide are naturally occurring deoxyribonucleotides, such as adenine, cytosine, guanine or thymine linked to 2'-deoxyribose, or ribonucleotides such as adenine, cytosine, guanine or uracil linked to ribose. However, a polynucleotide or oligonucleotide also can contain nucleotide analogs, including non-naturally occurring synthetic nucleotides or modified naturally occurring nucleotides. Such nucleotide analogs arc well known in the art and commercially available, as are polynucleotides containing such nucleotide analogs (Lin et al., Nucl. Acids Res. 22:5220-5234 (1994) ; Jellinek et al., Biochemistry 34:11363-11372 (1995); Pagratis et al., Nature Biotechnol. 15:68-73 (1997), each of which is incorporated herein by reference).

The covalent bond linking the nucleotides of a polynucleotide generally is a phosphodiester bond. However, the covalent bond also can be any of numerous other bonds, including a thiodiester bond, a phosphorothioate bond, a peptide-like bond or any other bond known to those in the art as useful for linking nucleotides to produce synthetic polynucleotides (see, for example, Tam et al., Nucl. Acids Res. 22:977-986 (1994); Ecker and Crooke, BioTechnology 13:351360 (1995), each of which is incorporated herein by reference). The incorporation of non-naturally occurring nucleotide analogs or bonds linking the nucleotides or analogs can be particularly useful where the polynucleotide is to be exposed to an environment that can contain a nucleolytic activity, including, for example, a tissue culture medium or upon administration to a living subject, since the modified polynucleotides can be less susceptible to degradation.

A polynucleotide or oligonucleotide comprising naturally occurring nucleotides and phosphodiester bonds can be chemically synthesized or can be produced using recombinant DNA methods, using an appropriate polynucleotide as a template. In comparison, a polynucleotide or oligonucleotide comprising nucleotide analogs or covalent bonds other than phosphodiester bonds generally are chemically synthesized, although an enzyme such as T7 polymerase can incorporate certain types of nucleotide analogs into a polynucleotide and, therefore, can be used to produce such a polynucleotide recombinantly from an appropriate template (Jellinek et al., *supra*, 1995). Thus, the term polynucleotide as used herein includes naturally occurring nucleic acid molecules, which can be isolated from a cell, as well as synthetic molecules, which can be prepared, for example, by methods, of chemical synthesis or by enzymatic methods such as by the polymerase chain reaction (PCR).

In various embodiments, it can be useful to detectably label a polynucleotide or oligonucleotide. Detectable labeling of a polynucleotide or oligonucleotide is well known in the art. Particular non-limiting examples of detectable labels include chemiluminescent labels, radiolabels, enzymes, haptens, or even unique oligonucleotide sequences.

A method of the identifying a SNP also can be performed using a specific binding pair member. As used herein, the term "specific binding pair member" refers to a molecule that specifically binds or selectively hybridizes to another member of a specific binding pair. Specific binding pair member include, for example, probes, primers, polynucleotides, antibodies, etc. For example, a specific binding pair member includes a primer or a probe that selectively hybridizes to a target polynucleotide that includes a SNP loci, or that hybridizes to an amplification product generated using the target polynucleotide as a template.

For example, a specific binding pair member of the invention can be an oligonucleotide or an antibody that, under the appropriate conditions, selectively binds to a target polynucleotide at or near nucleotide 473 of SEQ ID NO: 45 [marker 217486], nucleotide 224 of SEQ ID NO:47 [marker 869745], nucleotide 314 of SEQ ID NO:46 [marker 869787], nucleotide 210 of SEQ ID NO:20 [marker 886892], nucleotide 228 of SEQ ID NO:9 [marker 886895], nucleotide 245 of SEQ ID NO:10 [marker 886896], nucleotide 169 of SEQ ID NO:48 [marker 886933], nucleotide 214 of SEQ ID NO:49 [marker 886937], nucleotide 245 of SEQ ID NO:13 [marker 886994], nucleotide 193 of SEQ ID NO:8 [marker 886894], nucleotide 172 of SEQ ID NO:23 [marker 886938], nucleotide 216 of SEQ ID NO:24 [marker 886943], or nucleotide 903 of SEQ ID NO:50 [marker 886942]. As such, a specific binding pair member of the invention can be an oligonucleotide probe, which can selectively hybridize to a target polynucleotide and can, but need not, be a substrate for a primer extension reaction, or an anti-nucleic acid antibody. The specific binding pair member can be selected such that it selectively binds to any portion of a target polynucleotide, as desired, for example, to a portion of a target polynucleotide containing a SNP as the terminal nucleotide.

As used herein, the term "specific interaction," or "specifically binds" or the like means that two molecules form a complex that is relatively stable under physiologic conditions. The term is used herein in reference to various interactions, including, for example, the interaction of an antibody that binds a polynucleotide that includes a SNP site; or the interaction of an antibody that binds a polypeptide that includes an amino acid that is encoded by a codon that includes a SNP site. According to methods of the invention, an antibody can selectively bind to a polypeptide that includes a particular amino acid encoded by a codon that includes a SNP site. Alternatively, an antibody may preferentially bind a particular modified nucleotide that is incorporated into a SNP site for only certain nucleotide occurrences at the SNP site, for example using a primer extension assay.

A specific interaction can be characterized by a dissociation constant of at least about $1 \times 10^{-6}$ M, generally at least about $1 \times 10^{-7}$ M, usually at least about $1 \times 10^{-8}$ M, and particularly at least about $1 \times 10^{-9}$ M or $1 \times 10^{-10}$ M or greater. A specific interaction generally is stable under physiological conditions, including, for example, conditions that occur in a living individual such as a human or other vertebrate or invertebrate, as well as conditions that occur in a cell culture such as used for maintaining mammalian cells or cells from another vertebrate organism or an invertebrate organism. Methods for determining whether two molecules interact specifically are well known and include, for example, equilibrium dialysis, surface plasmon resonance, and the like.

Numerous methods are known in the art for determining the nucleotide occurrence for a particular SNP in a sample. Such methods can utilize one or more oligonucleotide probes or primers, including, for example, an amplification primer pair, that selectively hybridize to a target polynucleotide, which contains one or more pigmentation-related SNP positions. Oligonucleotide probes useful in practicing a method of the invention can include, for example, an oligonucleotide that is complementary to and spans a portion of the target polynucleotide, including the position of the SNP, wherein the presence of a specific nucleotide at the position (i.e., the SNP) is detected by the presence or absence of selective hybridization of the probe. Such a method can further include contacting the target polynucleotide and hybridized oligonucleotide with an endonuclease, and detecting the presence or absence of a cleavage product of the probe, depending on whether the nucleotide occurrence at the SNP site is complementary to the corresponding nucleotide of the probe.

An oligonucleotide ligation assay also can be used to identify a nucleotide occurrence at a polymorphic position, wherein a pair of probes that selectively hybridize upstream and adjacent to and downstream and adjacent to the site of the SNP, and wherein one of the probes includes a terminal nucleotide complementary to a nucleotide occurrence of the SNP. Where the terminal nucleotide of the probe is complementary to the nucleotide occurrence, selective hybridization includes the terminal nucleotide such that, in the presence of a ligase, the upstream and downstream oligonucleotides are ligated. As such, the presence or absence of a ligation product is indicative of the nucleotide occurrence at the SNP site.

An oligonucleotide also can be useful as a primer, for example, for a primer extension reaction, wherein the product (or absence of a product) of the extension reaction is indicative of the nucleotide occurrence. In addition, a primer pair useful for amplifying a portion of the target polynucleotide including the SNP site can be useful, wherein the amplification product is examined to determine the nucleotide occurrence at the SNP site. Particularly useful methods include those that are readily adaptable to a high throughput format, to a multiplex format, or to both. The primer extension or amplification product can be detected directly or indirectly and/or can be sequenced using various methods known in the art. Amplification products which span a SNP loci can be sequenced using traditional sequence methodologies (e.g., the "dideoxy-mediated chain termination method," also known as the "Sanger Method"(Sanger, F., et al., J. Molec. Biol. 94:441 (1975); Prober et al. Science 238:336-340 (1987)) and the "chemical degradation method," "also known as the "Maxam-Gilbert method"(Maxam, A. M., et al., Proc. Natl. Acad. Sci. (U.S.A.) 74:564 (1977)), both references herein incorporated by reference) to determine the nucleotide occurrence at the SNP loci.

Methods of the invention can identify nucleotide occurrences at SNPs using a "microsequencing" method. Microsequencing methods determine the identity of only a single nucleotide: at a "predetermined" site. Such methods have particular utility in determining the presence and identity of polymorphisms in a target polynucleotide. Such microsequencing methods, as well as other methods for determining the nucleotide occurrence at a SNP loci are discussed in Boyce-Jacino , et al., U.S. Pat. No. 6,294,336, incorporated herein by reference, and summarized herein.

Microsequencing methods include the Genetic Bit Analysis method disclosed by Goelet, P. et al. (WO 92/15712, herein incorporated by reference). Additional, primer-guided, nucleotide incorporation procedures for assaying polymorphic sites in DNA have also been described (Komher, J. S. et al, Nucl. Acids. Res. 17:7779-7784 (1989); Sokolov, B. P., Nucl. Acids Res. 18:3671 (1990); Syvanen, A. -C., et al., Genomics 8:684-692 (1990); Kuppuswamy, M. N. et al., Proc. Natl. Acad. Sci. (U.S.A.) 88:1143-1147 (1991); Prezant, T. R. et al, Hum. Mutat. 1:159-164 (1992); Ugozzoli, L. et al., GATA 9:107-112 (1992); Nyren, P. et al., Anal. Biochem. 208:171-175 (1993); and Wallace, WO89/10414). These methods differ from Genetic Bit™. Analysis in that they all rely on the incorporation of labeled deoxynucleotides to discriminate between bases at a polymorphic site. In such a format, since the signal is proportional to the number of deoxynucleotides incorporated, polymorphisms that occur in runs of the same nucleotide can result in signals that are proportional to the length of the run (Syvanen, A. -C., et al. Amer. J. Hum. Genet. 52:46-59 (1993)).

Alternative microsequencing methods have been provided by Mundy, C.R. (U.S. Pat. No. 4,656,127) and Cohen, D. et al (French Patent 2,650,840; PCT Appln. No. WO91/02087) which discusses a solution-based method for determining the identity of the nucleotide of a polymorphic site. As in the Mundy method of U.S. Pat. No. 4,656,127, a primer is employed that is complementary to allelic sequences immediately 3'-to a polymorphic site.

In response to the difficulties encountered in employing gel electrophoresis to analyze sequences, alternative methods for microsequencing have been developed. Macevicz (U.S. Pat. No. 5,002,867), for example, describes a method for determining nucleic acid sequence via hybridization with multiple mixtures of oligonucleotide probes. In accordance with such method, the sequence of a target polynucleotide is determined by permitting the target to sequentially hybridize with sets of probes having an invariant nucleotide at one position, and a variant nucleotides at other positions. The Macevicz method determines the nucleotide sequence of the target by hybridizing the target with a set of probes, and then determining the number of sites that at least one member of the set is capable of hybridizing to the target (i.e., the number of "matches"). This procedure is repeated until each member of a sets of probes has been tested.

Boyce-Jacino , et al., U.S. Pat. No. 6,294,336 provides a solid phase sequencing method for determining the sequence of nucleic acid molecules (either DNA or RNA) by utilizing a primer that selectively binds a polynucleotide target at a site wherein the SNP is the most 3' nucleotide selectively bound to the target.

In one particular commercial example of a method that can be used to identify a nucleotide occurrence of one or more SNPs, the nucleotide occurrences of pigmentation-related SNPs in a sample can be determined using the SNP-IT™ method (Orchid BioSciences, Inc., Princeton, NJ). In general, SNP-IT™ is a 3-step primer extension reaction. In the first step a target polynucleotide is isolated from a sample by hybridization to a capture primer, which provides a first level of specificity. In a second step the capture primer is extended from a terminating nucleotide trisphosphate at the target SNP site, which provides a second level of specificity. In a third step, the extended nucleotide trisphosphate can be detected using a variety of known formats, including: direct fluorescence, indirect fluorescence, an indirect colorimetric assay, mass spectrometry, fluorescence polarization, etc. Reactions can be processed in 384 well format in an automated format using a SNPstream™ instrument ((Orchid BioSciences, Inc., Princeton, NJ).

In a specific example of a method for identifying marker 217458 of the OCA2-A haplotype, a primer pair is synthesized that comprises a forward primer that hybridizes to a sequence 5' to the SNP of SEQ ID NO:7 (the SEQ ID corresponding to marker 217458 (see Table 1)) and a reverse primer that hybridizes to the opposite strand of a sequence 3' to the SNP of SEQ ID NO:7. This primer pair is used to amplify a target polynucleotide that includes marker 217458, to generate an amplification product. A third primer can then be used as a substrate for a primer extension reaction. The third primer can bind to the amplification product such that the 3' nucleotide of the third primer (e.g., adenosine) binds to the marker 217458 site and is used for a primer extension reaction. The primer can be designed and conditions determined such that the primer extension reaction proceeds only if the 3' nucleotide of the third primer is complementary to the nucleotide occurrence at the SNP, which proceeds if the nucleotide occurrence of marker 217458 is a thymidine, for example, but not if the nucleotide occurrence of the marker is cytidine.

Phase known data can be generated by inputting phase unknown raw data from the SNPstream™ instrument into the Stephens and Donnelly's PHASE program.

Accordingly, using the methods described above, the pigmentation-related haplotype allele or the nucleotide occurrence of the pigmentation-related SNP can be identified using an amplification reaction, a primer extension reaction, or an immunoassay. The pigmentation-related haplotype allele or the pigmentation-related SNP can also be identified by contacting polynucleotides in the sample or polynucleotides derived from the sample, with a specific binding pair member that selectively hybridizes to a polynucleotide region comprising the pigmentation-related SNP, under conditions wherein the binding pair member specifically binds at or near the pigmentation-related SNP. The specific binding pair member can be an antibody or a polynucleotide.

Antibodies that are used in the methods of the invention include antibodies that specifically bind polynucleotides that encompass a pigmentation-related or race-related haplotype. In addition, antibodies of the invention bind polypeptides that include an amino acid encoded by a codon that includes a SNP. These antibodies bind to a polypeptide that includes an amino acid that is encoded in part by the SNP. The antibodies specifically bind a polypeptide that includes a first amino acid encoded by a codon that includes the SNP loci, but do not bind, or bind more weakly to a polypeptide that includes a second amino acid encoded by a codon that includes a different nucleotide occurrence at the SNP.

Antibodies are well-known in the art and discussed, for example, in U.S. Pat. No. 6,391,589. Antibodies of the invention include, but are not limited to, polyclonal, monoclonal, multispecific, human, humanized or chimeric antibodies, single chain antibodies, *Fab* fragments, F(ab') fragments, fragments produced by a *Fab* expression library, anti-idiotypic (anti-Id) antibodies (including, e.g., anti-Id antibodies to antibodies of the invention), and epitope-binding fragments of any of the above. The term *"antibody,"* as used herein, refers to immunoglobulin molecules and immunologically active portions of immunoglobulin molecules, i.e., molecules that contain an antigen binding site that immunospecifically binds an antigen. The immunoglobulin molecules of the invention can be of any type (e.g., IgG. IgE, IgM, IgD, IgA and IgY), class (e.g., IgG1, IgG2, IgG3, IgG4, IgA1 and IgA2) or subclass of immunoglobulin molecule.

Antibodies of the invention include antibody fragments that include, but are not limited to, Fab, Fab' and F(ab') 2. Fd, single-chain Fvs (scFv), single-chain antibodies, disulfide-linked Fvs (sdFv) and fragments comprising either a VL or VH domain. Antigen-binding antibody fragments, including single-chain antibodies, may comprise the variable region(s) alone or in combination with the entirety or a portion of the following: hinge region, CH1, CH2, and CH3 domains. Also included in the invention are antigen-binding fragments also comprising any combination of variable region(s) with a hinge region, CH1, CH2, and CH3 domains. The antibodies of the invention may be from any animal origin including birds and mammals. Preferably, the antibodies are human, murine (e.g., mouse and rat), donkey, ship rabbit, goat, guinea pig, camel, horse, or chicken. The antibodies of the invention may be monospecific, bispecific, trispecific or of greater multispecificity.

The antibodies of the invention may be generated by any suitable method known in the art. Polyclonal antibodies to an antigen-of-interest can be produced by various procedures well known in the art. For example, a polypeptide of the invention can be administered to various host animals including, but not limited to, rabbits, mice, rats, etc. to induce the production of sera containing polyclonal antibodies specific for the antigen. Various adjuvants may be used to increase the immunological response, depending on the host species, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and Corynebacterium parvum. Such adjuvants are also well known in the art.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example; in Harlow et al., Antibodies: A Laboratory Manual, (Cold Spring Harbor Laboratory Press, 2nd ed. 1988); Hammerling, ct al., in: Monoclonal Antibodies and T-Cell Hybridomas 563-681 (Elsevier, N.Y., 1981) (said references incorporated by reference in their entireties): The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Where the particular nucleotide occurrence of a SNP, or nucleotide occurrences of a pigmentation-related haplotype, is such that the nucleotide occurrence results in an amino acid change in an encoded polypeptide, the nucleotide occurrence can be identified indirectly by detecting the particular amino acid in the polypeptide. The method for determining the amino acid will depend, for example, on the structure of the polypeptide or on the position of the amino acid in the polypeptide.

Where the polypeptide contains only a single occurrence of an amino acid encoded by the particular SNP, the polypeptide can be examined for the presence or absence of the amino acid. For example, where the amino acid is at or near the amino terminus or the carboxy terminus of the polypeptide, simple sequencing of the terminal amino acids can be performed. Alternatively, the polypeptide can be treated with one or more enzymes and a peptide fragment containing the amino acid position of interest can be examined, for example, by sequencing the peptide, or by detecting a particular migration of the peptide following electrophoresis. Where the particular amino acid comprises an epitope of the polypeptide, the specific binding, or absence thereof, of an antibody specific for the epitope can be detected. Other methods for detecting a particular amino acid in a polypeptide or peptide fragment thereof are well known and can be selected based, for example, on convenience or availability of equipment such as a mass spectrometer, capillary electrophoresis system, magnetic resonance imaging equipment, and the like.

In another aspect, the invention is a method for inferring a genetic pigmentation trait of a mammalian subject from a nucleic acid sample of the mammalian subject, wherein the method includes identifying a nucleotide occurrence in the sample for at least one pigmentation-related single nucleotide polymorphism (SNP) from a pigmentation gene. The pigmentation gene can be oculocutaneous albinism 11 (OCA2), agouti signaling protein (ASIP), tyrosinase-related protein 1 (TYRP1), tyrosinase (TYR), adaptor-related protein complex 3, beta 1 subunit (AP3B1). AP3D1, dopachrome tautomerase (DCT), silver homolog (SILV), AIM-1 protein (LOC51151), proopiomelanocortin (POMC), ocular albinism 1 (OA 1), microphthalmia-associatcd transcription factor (MITF), myosin VA (MYO5A), RAB27A, or coagulation factor II (thrombin) receptor-like 1 (F2RL1). The nucleotide occurrence is associated with the pigmentation trait of the mammalian subject, thereby inferring the pigmentation trait of the mammalian subject. The method can further include identifying in the nucleic acid sample at least one nucleotide occurrence for at least a second pigmentation-related SNP of at least a second pigmentation gene. In certain preferred embodiments where the method involves only a single pigmentation-related SNP or involves pigmentation-related SNPs in a single gene, the pigmentation-related SNP(s) are not the ASIP SNPs disclosed in Kenetsky et al., Am. J. Hum. Genet., 70:770 (2002).

The method can further comprise identifying in the nucleic acid sample a nucleotide occurrence for at least a second pigmentation-related SNP of at least a second pigmentation gene. The second pigmentation gene can be OCA2, ASIP, TYRP1, TYR, AP3B1, AP3D1,DCT, SILV, LOC51151, POMC, OA1, MITF, MYO5A, RAB27A, F2RL1, or melanocortin-1 receptor (MC1R), or any combination of these genes.

In certain embodiments of methods according to this aspect of the invention, the first pigmentation gene does not include the MC1R gene.

A method according to this aspect of the invention infers eye color or eye shade as the pigmentation trait, and identifies the nucleotide occurrence for at least one of:

nucleotide 609 of SEQ ID NO:1 [marker 702], nucleotide 501 of SEQ ID NO:2 [marker 650], nucleotide 256 of SEQ ID NO:3 [marker 675], nucleotide, 442 of SEQ ID NO:4 [marker 217438], nucleotide 619 of SEQ ID NO:5 [marker 217439], nucleotide 646 of SEQ ID NO:6 [marker 217441]; nucleotide 135 of SEQ ID NO:7 [marker 217458], nucleotide 193 of SEQ ID NO:8 [marker 886894], nucleotide 228 of SEQ ID NO:9 [marker 886895], nucleotide 245 of SEQ ID NO:10 [marker 886896], nucleotide 189 of SEQ ID NO:11 [217452], nucleotide 573 of SEQ ID NO:12 [712052], nucleotide 245 of SEQ ID NO:13 [marker 886994], nucleotide 643 of SEQ ID NO:14 [marker 712057], nucleotide 539 of SEQ ID NO:15 [marker 712058], nucleotide 418 of SEQ ID NO:16 [marker 712060], nucleotide 795 of SEQ ID NO:17 [marker 712064], nucleotide 535 of SEQ ID NO:18 [marker 712054], nucleotide 554 of SEQ ID NO:19 [marker 712056], nucleotide 210 of SEQ ID NO:20 [marker 886892], nucleotide: 225 of SEQ ID NO:21 [marker 217455], nucleotide 170 of SEQ ID NO:22 [marker 712061], nucleotide 210 of SEQ ID NO:20 [marker 886892], nucleotide 172 of SEQ ID NO:23 [marker 886938], or nucleotide 216 of SEQ ID NO:24 [marker 886943], or any combination thereof. These SNPs listed in this example are penetrant SNPs in that they make up penetrant haplotypes as illustrated in Example 17.

Furthermore, in methods of this aspect of the invention involving the penetrant SNPs listed above, a method of the invention identifies nucleotide occurrences for at least one of: nucleotide 61 of SEQ ID NO:25 [marker 560], nucleotide 201 of SEQ ID NO:26 [marker 552], nucleotide 201 of SEQ ID NO:27 [marker 559], nucleotide 201 of SEQ ID NO:28 [marker 468], nucleotide 657 of SEQ ID NO:29 [marker 657], nucleotide 599 of SEQ ID NO:30 [marker 674], nucleotide 267 of SEQ ID NO:31 [marker 632], nucleotide 61 of SEQ ID NO:32 [marker 701], nucleotide 451 of SEQ ID NO:33 [marker 710]; nucleotide 326 of SEQ ID NO:34 [marker 217456], nucleotide 61 of SEQ ID NO:35 [marker 656], nucleotide 61 of SEQ ID NO:36 , nucleotide 61 of SEQ ID NO:37 [marker 637], nucleotide 93 of SEQ ID NO:38 [marker 278], nucleotide 114 of SEQ ID NO:39 [marker 386], nucleotide 558 of SEQ ID NO:40 [marker 217480], nucleotide 221 of SEQ ID NO:41 [marker 951497], nucleotide 660 of SEQ ID NO:42 [marker 217468], nucleotide 163 of SEQ ID NO: 43 [marker 217473], nucleotide 364 of SEQ ID NO:44 [marker 217485], nucleotide 473 of SEQ ID NO:45 [marker 217486], nucleotide 314 of SEQ ID NO:46 [marker 869787], nucleotide 224 of SEQ ID NO:47 [marker 869745], nucleotide 169 of SEQ ID NO:48 [marker 886933], nucleotide 214 of SEQ ID NO:49 [marker 886937], or nucleotide 903 of SEQ ID NO: 50 [marker 886942], or any combination thereof. These SNPs are latent SNPs for eye pigmentation in that they make up the latcnt haplotypes identified in Example 17.

A method according to this aspect of the invention can infer hair color or hair shade as the pigmentation trait, and can identify the nucleotide occurrence for at least one of: nucleotide 201 of SEQ ID NO:27 [marker 559], nucleotide 61 of SEQ ID NO:25 [marker 560], nucleotide 442 of SEQ ID NO:4 [marker 217438], nucleotide 619 of SEQ ID NO:5 [marker 217439], nucleotide 646 of SEQ ID NO:6 [marker 217441], nucleotide 418 of SEQ ID NO:16 [marker 712060], nucleotide 210 of SEQ ID NO:20 [marker 886892], nucleotide 245 of SEQ ID NO:10 [marker 886896], nucleotide 225 of SEQ ID NO:21 [marker 217455], nucleotide 643 of SEQ ID NO:14 [marker 712057], nucleotide 193 of SEQ ID NO:8 [marker 886894], nucleotide 135 of SEQ ID NO:7 [marker 217458], nucleotide 554 of SEQ ID NO: 19 [marker 712056], nucleotide 535 of SEQ ID N0:18 [marker 712054], nucleotide 228 of SEQ ID NO:9 [marker 586895], nucleotide 473 of SEQ ID NO:45, [2174861, nucleotide 214 of SEQ ID NO:49; [886937], or any combination thereof. These SNPs are penetrant SNPs for hair pigmentation in that they make up the penetrant haplotypes identified in Example 18.

The method of the invention that include identifying a nucleotide occurrence in the sample for at least one pigmentation-related SNP from a pigmentation gene, as discussed above, in preferred embodiments can include grouping the nucleotide occurrences of the pigmentation-related SNPs for a pigmentation gene into one or more identified haplotype alleles of a pigmentation-related haplotype. To infer the pigmentation trait of the subject, the identified haplotype alleles are then compared to known haplotype alleles of the pigmentation-related haplotype, wherein the relationship of the known haplotype alleles to the genetic pigmentation trait is known.

In another aspect, the present method provides a method for inferring a genetic pigmentation trait of a mammalian subject from a biological sample of the mammalian subject. The method includes identifying a nucleotide occurrence in the sample for a pigmentation-related single nucleotide polymorphism (SNP) from a pigmentation gene, wherein the pigmentation gene is other than melanocortin-1 receptor (MC1 R). The nucleotide occurrence is associated with the pigmentation trait of the mammalian subject, thereby allowing an inference to be drawn related to pigmentation trait of the mammalian subject.

In another aspect, the invention provides a method for inferring race of a human subject from a biological sample of the human subject. The method includes identifying in the nucleic acid sample, the nucleotide occurrence of at least one race-related single nucleotide polymorphism (SNP) of a race-related gene. The nucleotide occurrence of the race-related SNP is associated with race, thereby allowing an inference to be drawn regarding the race of the subject.

Human identity testing relies on the fact that binned alleles from polymorphic loci segregate into unique combinations in individual human beings. The allele combinations serve as "bar-codes" by which to unambiguously identify individual human beings. Because systematic genetic forces have shaped the genetic structure of modem day humanity, most human polymorphisms, including STRs and SNPs, are characterized by alleles that are unevenly distributed among the various populations of the world. In the case of STR markers, inter-population differences in allele frequencies are so great that knowledge of the individuals racial background is required to formally qualify STR alleles for exclusion calculations (Budowle et al., J. Forensic Sci. 46(3):453-489, 2001; Levadokou et al., J. Forensic Sci. 46(3):736-761, 2001; Budowle et al., Clin. Chim. Acta 228(1):3-18, 1994; Kersting et al., Croat Med. J. 42(3):310-314, 2001; Meyer et al., Int. Int. J. Legal Med. 107(6):314-322, 1995).

Use of a database for the wrong population can result in errors of several orders of magnitude (Monson et al., J. Forensic Sci. 43(3):483-488, 1998). Though these exclusion calculations can be performed retrospectively, once the perpetrator has been identified, there is a great need for racial profiling tools that function in a retrospective (suspect already in hand) as well as a prospective (suspect not yet identified) capacity. Racial classifiers can assist retrospective case work because, for various reasons, including within-individual mixture, race is not always easily discernable in certain individuals. A good racial classification tool that genetically defines a person's racial and ethnic background (including mixture) can legally justify the choice of reference database(s) used for calculating exclusion probabilities. In a prospective sense, racial classification markers can be (and are) used to guide criminal investigations towards individuals that cannot be racially excluded. In some cases, a racial classification result can provide just cause for legally requesting a DNA specimen from a suspect, and in so doing, create a leverage crux for maximizing the efficacy of our criminal justice system.

Various probabilistic methods have been proposed to take advantage of inter-population frequency differences for inferring the racial origin of DNA specimens (Brenner, Am. J. Hum. Genet., 62(6):1558-1560, 1998; Lowe et al., Forensic Sci. Int. 119(1):17-22, 2001; Brenner, Proceedings 7th Intl. Symposium on Hum. Identification 4892, 1997). For example, Bayesian statistical schemes have been employed to use allele frequencies in given populations (class conditional probabilities) for the calculation of the posterior probability that a DNA sample was derived from an individual of that population. Most STR markers currently in use (i.e., F13A, TH01, FES/FPS and VWA) offer little power to resolve between the possible racial groups that a specimen can belong. Resolution values for distinguishing individuals of African from Caucasian descent average about r = 1.7 (log10r = 0.4) per locus, which means that, assuming a prior probability of 50% classification in alternative, wrong decisions would be made 20% of the time. Though a collection of such markers may effectively resolve racial origin in most cases, the statistical distributions are such that 5-10% of classifications are ambiguous (Brenner, Proceedings 7th Intl. Symposium on Hum. Identification 4892, 1997). Clearly, given the scrutiny afforded to forensic statistical calculations in the courtroom (particularly when speaking of court orders for requesting DNA specimens from suspects), greater performance is necessary. Either markers that show more dramatic racial bias (log10r values 2 or greater) need to be found, or a very large collection of modest markers need to be identified.

In fact, screens for STR markers of dramatic racial bias have been conducted, and resulted in the discovery of 10 loci capable of resolving Caucasian Americans from African Americans (Shriver et al, Am. J. Hum. Genet. 60: 957-964,1997). Though Bayesian racial inference methods using these STR markers appear to be fairly robust, there is considerable debate on their rigor. Some of this debate focuses on general problems of what race really is (Goodman, Am. J. Public Health 90(11): 1699-1702, 2000), which apply to any test, but the most compelling arguments against the STR methods are technical and statistical in nature (Brenner, Proceedings 7th Intl. Symposium on Hum. Identification 4892, 1997, Erickson and Svensmark, Int. J. Legal Med. 106:254-257, 1994, Evett et al., J. Forensic Sci. Soc. 32: 301-306, 1992, Shriver et al, Am. J. Hum. Genet. 60:957-964, 1997). For example, population-specific allele frequency determination is often biased for STR markers due to inequalities and bias in reference database resources. STR markers have a relatively large number of alleles (often 20 or more), and this complexity can cause sampling bias in the estimation of allele frequencies in certain populations. Sampling bias can cause estimated frequencies to appear smaller or greater than they really are, artificially inflating or deflating (sometimes dramatically) the log likelihood ratios of racial classification ( Brenner, Proceedings 7th Intl. Symposium on Hum. Identification 4892, 1997). Problems such as these are unique to multi-allelic markers such as STRs.

A positive by-product of STR allelic complexity is that relatively few loci need be measured for each test to identify a human, or infer his or her ethnic origin. Indeed, because this reduces the number of assays that need to be executed for each sample, this is one reason they are used. A negative by-product of this complexity, however, is that very large databases are required in order to estimate allele frequencies, which are necessary for identity or racial exclusion calculations. For this reason, loci of complex allelic structure impose unique statistical problems for both identity testing and racial inference. In contrast, bi-allelic tests (i.e., SNPs) involve the measurement of larger numbers of loci of simpler allelic structure to obtain the same statistical power as STR markers, because there are only two alleles for each locus in the population. However, because of the small number of alleles, fewer individuals from each population are necessary for accurate minor allele frequency determinations in reference databases. Since so many SNPs are available, those with reasonable minor allele frequencies can be selected so that the minor allele frequencies are relatively high compared to STR alleles. This potentially renders sampling bias issues mute and allows for the use of smaller reference databases in identity and racial exclusion calculation. Reference database sizes being equal, the statistical power of SNP-based identity determination and racial inference is likely to be greater due to the sheer number of SNPs that can be used.

On top of these statistical advantages, recent advances in high-throughput genotyping technologies have made SNPs technically and economically more attractive for use in identity testing. Until recently, small numbers of complex alleles have been preferred over large numbers of less complex loci due to the expense and technical difficulty in running multiple tests on single specimens. Given the recent technological advancements that reduce the expense of typing multiple markers in individual samples, the current rate limiting step in forensic molecular biology is no longer the number of sites that can be economically typed in each sample, but the number of individuals that can be tested. With STR markers, several thousand specimens are required in each population to accurately estimate allele frequencies (and other parameters), and this problem is greater the larger the number of possible alleles per locus, and the rarer the minor allele(s) in a given population. With SNP markers, this is less of an issue because so many SNPs are available for typing that batteries of SNPs with reasonable pan-racial minor allele frequencies can be pre-selected. For these reasons, it is likely that identity determination of the future, at some level, will involve SNP typing. Probably the most significant barrier remaining for the use of SNPs in forensic identity testing is not scientific or technical, but commercial inertia; new equipment will have to be purchased, new databases constructed and new assays validated. However, none of these factors is significant enough to justify the use of an inferior methodology, particularly when human lives arc in the balance.

Though SNP based identity testing appears to the wave of the future, relatively few SNP based human identity testing products have yet been developed and/or published. Further, no SNP based tests have yet been described that are capable of accurately inferring the racial origin of a DNA specimen. The invention provides a panel of 64 "Significant markers of race," which are SNPs whose association with a particular race of a subject is strong enough to be detected using simple genetics approaches. As illustrated in Example 14, significant markers of race show a race-biased frequency distribution. Significant markers of race can also be referred to as "race-related SNPs."

A method according to this aspect of the invention that relates to an inference of race includes methods wherein the nucleotide occurrence of at least 2 race-related SNPs arc identified. In these embodiments, to increase the power of the inference, the method can further comprise grouping the identified nucleotide occurrences of the race-related SNPs into one or more race-related haplotype alleles, which exhibit a race-biased frequency distribution.

To determine whether SNPs or haplotypes are race-related, numerous statistical analysis can be performed, similar to those described above related to pigmentation-related haplotypes. Allele frequencies can be calculated for haplotypes and pair-wise haplotype frequencies estimated using an EM algorithm (Excoffier and Slatkin 1995). Linkage disequilibrium coefficients can then be calculated. In addition to various parameters such as linkage disequilibrium coefficients, allele and haplotype frequencies (within ethnic, control and case groups), chi-square statistics and other population genetic parameters such as Panmitic indices can be calculated to control for ethnic, ancestral or other systematic variation between the case and control groups.

Markers/haplotypes with value for distinguishing the case matrix from the control, if any, can be presented in mathematical form describing any relationship and accompanied by association (test and effect) statistics. A statistical analysis result which shows an association of a SNP marker or a haplotype with a pigmentation trait with at least 80%, 85%, 90%, 95%, or 99%, most preferably 95% confidence, or alternatively a probability of insignificance less than 0.05. These statistical tools may test for significance related to a null hypothesis that an on-test SNP allele or haplotype allele is not significantly different between individuals of different races.

The panel of significant markers of race provided herein in Example 14, are SNP markers in the major human pigmentation and xenobiotic metabolism genes, as well as other genes, that can be used to infer the ethnic origin of a DNA specimen with near perfect accuracy in a sample of Asian, African, and Caucasian descent. We also present herein in Example 17, a series of penetrant haplotypes and a series of latent haplotypes for eye color. The SNPs of these penetrant and latent haplotypes are also significant markers of race, and can be used to infer the race of a subject with near perfect accuracy. To improve the power of the inference even further, the combination of haplotypes of Example 17, which includes these SNPs, can be used to infer race.

The race-related gene of the methods of this aspect of the invention can include a pigmentation gene or a xenobiotic gene, or any other gene in which a statistically significant association with a particular race or group of races (e.g., Asian and African populations) for a nucleotide occurrence of a SNP or a haplotype occurring within the gene, is observed. Race-related SNPs are SNPs with genotype distributions and allele frequencies that are statistically different between the three ethnic groups (See e.g., Example 14). Minor alleles for each of these 68 SNP markers were preferentially represented in one of the three major racial groups tested (Asians, African Americans or Caucasians) and many of these SNPs showed dramatic differences between the groups. All three of the possible preference categories are observed; preferentially present in the Caucasian population, preferentially present in the Asian population, and preferentially present in the African American population.

The race-related gene can include at least one of oculocutaneous albinism II (OCA2), agouti signaling protein (ASIP), CYP2D6, tyrosinase-related protein 1 (TYRP1), cytochrome p450-2 (CYP2C9), cytochrome p450-3 (CYP3A4), tyrosinase (TYR), melanocortin-1 receptor (MC1R), adaptor-related protein complex 3, beta 1 subunit (AP3B1), AP3D1, dopachrome tautomerase (DCT), silver homolog (SILV), AIM-1 protein (LOC51151), proopiomelanocortin (POMC), ocular albinism 1 (OA1), microphthalmia-associated transcription factor (MITF), myosin VA (MYO5A), RAB27A, coagulation factor II (thrombin) receptor-like 1 (F2RL1), HMG CoA reductase (HMGCR), farnesyl diphosphate synthase (FDPS), aryl hydrocarbon reductase (AHR), or cytochrome p450-1 (CYP1A1), or any combination thereof.

This method can further include in the nucleic acid sample at least one nucleotide occurrence for at least a second race-related SNP of at least a second race-related gene. The second race-related gene can be OCA2, ASIP, TYRP1, TYR, AP3B1, AP3D1, DCT, SILV, LOC51151, POMC, OA1, MITF, MYO5A, RAB27A, F2RL1, melanocortin-1 receptor (MC1R), CYP2D6, CYP2C9, CYP3A4, AP3B1, HMGCR, FDPS, AHR, or CYP1A1, or any combination thereof.

Of these race-related genes listed above OCA2, SILV, ASIP, TYRP1, DCT, TYR, MC1R, and AP3B1 are pigmentation genes; AHR and CYP1A1 are xenobiotic genes; and CYP2D6, CYP2C9, CYP3A4, HMGCR, and FDPS, are neither pigmentation nor xenobiotic genes.

Though SNPs and/or haplotypes in many genes could reasonably be expected to be associated with a particular race or group of races, the present disclosure reveals that pigmentation genes and xenobiotic genes appear to include an unusually large number of significant markers of race, and these markers arc strong indicators of race, as illustrated in Example 14. That is, the present disclosure reveals that the pigmentation and xenobiotic genes appear to be sinks for accumulating these kinds of SNPs over evolutionary time. Therefore, the race-related gene in this aspect of the invention can include one or more pigmentation gene and/or one or more xenobiotic genes.

The race-related SNPs disclosed herein not only can be useful for inferring race but can be useful for inferring pigmentation traits through correlation.

The attached Examples such as Example 14, illustrate methods of inferring an individual's race. Methods of Examples, such as Example 17, which infer a pigmentation-trait can be used to infer race by substituting known race relationships for known pigmentation-trait relationships. The inference typically involves using a complex model that involves using known relationships of known alleles or nucleotide occurrences as classifiers. As illustrated in Example 17, the inference can be drawn by applying data regarding the subject's race-related haplotype allele(s) to a complex model that makes a blind, quadratic discriminate classification using a variance-covariance matrix. Various classification models are discussed in more detail herein, and illustrated in the Examples.

A method according to this aspect of the invention that relates to an inference of race includes methods wherein the nucleotide occurrence of at least 2 race-related SNPs are identified, In these embodiments, to increase the power of the inference, the method can further comprise grouping the identified nucleotide occurrences of the race-related SNPs into one or more race-related haplotype alleles, wherein the relationship of the haplotype alleles to race is known.

In this aspect of the invention, the race-related haplotype can be at least one of the following race-related haplotypes:

a) nucleotides of the DCT gene corresponding to a DCT-A haplotype, which includes: nucleotide 609 of SEQ ID NO:1 [702], nucleotide 501 of SEQ ID NO:2 [650], and nucleotide 256 of SEQ ID NO:3 [marker 675];

b) nucleotides of the MC1R gene corresponding to an MC1R-A haplotype, which includes: nucleotide 442 of SEQ ID NO:4 [217438], nucleotide 619 of SEQ ID NO:5 [217439], and nucleotide 646 of SEQ ID NO:6 [217441]; or

c) nucleotides of the OCA2 gene corresponding to an OCA2-A haplotype, which includes: nucleotide 135 of SEQ ID NO:7 [217458], nucleotide 193 of SEQ ID NO:8 [886894], nucleotide 228 of SEQ ID NO:9 [marker 886895], and nucleotide 245 of SEQ ID NO:10 [marker 886896];

d) nucleotides of the OCA2 gene corresponding to an OCA2-B haplotype, which includes: nucleotide 189 of SEQ ID NO:11 [marker 217452]], nucleotide 573 of SEQ ID NO:12 [marker 712052], and nucleotide 245 of SEQ ID NO: 13 [marker 886994];

e) nucleotides of the OCA2 gene corresponding to an OCA2-C haplotype, which includes: nucleotide 643 of SEQ ID NO:14 [712057], nucleotide 539 of SEQ ID NO:15 [712058], nucleotide 418 of SEQ ID NO:16 [712060], and nucleotide 795 of SEQ ID NO: 17, [712064]

f) nucleotides of the OCA2 gene, corresponding to an OCA2-D haplotype, which includes: nucleotide 535 of SEQ ID NO: 18, [712054], nucleotide 554 of SEQ ID NO:19, [712056], and nucleotide 210 of SEQ ID NO:20, [886892];

g) nucleotides of the OCA2 gene, corresponding to an OCA2-E haplotype, which includes: nucleotide 225 of SEQ ID NO:21, [217455], nucleotide 170 of SEQ ID NO:22, [712061], and nucleotide 210 of SEQ ID NO:20, [886892]; or

h) nucleotides of the TYRP1 gene corresponding to a TYRP1-B haplotype which includes: nucleotide 172 of SEQ ID NO:23, [886938], nucleotide 216 of SEQ ID NO:24; [886943], or any combination of a) through h).

To improve the power of the inference, in methods of this aspect of the invention involving the race-related haplotypes above, these race-related haplotype can further include at least one of the following haplotypes:

i) nucleotides of the ASIP gene corresponding to a ASIP-A haplotype, which comprises: nucleotide 201 of SEQ ID NO:26 [marker 552], and nucleotide 201 of SEQ ID NO:28 [marker 468];

j) nucleotides of the DCT gene corresponding to a DCT-B haplotype, which comprises: nucleotide 451 of SEQ ID NO:33 [marker 710], and nucleotide 657 of SEQ ID NO:29 [marker 657];

k) nucleotides of the SILV gene corresponding to a SILV-A haplotype, which comprises: nucleotide 61 of SEQ ID NO:35 [marker 656], and nucleotide 61 of SEQ ID NO:36;

l) nucleotides of the TYR gene corresponding to a TYR-A haplotype, which comprises: nucleotide 93 of SEQ ID NO:38 [marker 278], and nucleotide 114 of SEQ ID NO:39 [marker 336]; or

m) nucleotides of the TYRP1 gene corresponding to a TYRP1-A haplotype, which comprises: nucleotide 364 of SEQ ID NO:44 [marker 217485], nucleotide 169 of SEQ ID NO:48 [marker 886933], and nucleotide 214 of SEQ ID NO:49 [marker 886937], or any combination of i) through m).

In methods of this aspect of the invention involving the preferred race-related haplotypes and preferred race-related haplotypes, at least one race-related haplotype allele includes a combination of haplotype alleles of the MC1R-A haplotype, the OCA2-A haplotype, the OCA2-B haplotype, the OCA2-C haplotype, the OCA2-D haplotype, the OCA2-E haplotype, the TYRP1-B haplotype, and the DCT-B haplotype. By way of a preferred example, in these methods the at least one haplotype allele of a)- m) above can include at least one haplotype allele in each of the ASIP-A haplotype, the DCT-B haplotype, the SILV-A haplotype, the TYR-A haplotype, and the TYRP1-A haplotype.

In certain methods involving the race-related haplotypes disclosed above, the race-related haplotype allele is a combination of haplotype alleles that includes:

a) the MC1R-A haplotype allele CCC;
b) the OCA2-A haplotype allele TTAA, CCAG, or TTAG;
c) the OCA2-B haplotype allele CAA, CGA, CAC, or CGC;
d) the OCA2-C haplotype allele GGAA, TGAA, or TAAA;
e) the OCA2-D haplotype allele AGG or GGG;
f) the OCA2-E haplotype allele GCA;
g) the TYRP1-B haplotype allele TC; and
h) the DCTB gene haplotype allele CTG or GTG
Furthermore, to further improve the inference power, this method that includes all the haplotypes for race, can further include a combination of haplotype alleles that includes,
i) the ASIP-A haplotype allele 'GT' or 'AT';
j) the DCT-B haplotype allele 'TA' or 'TG';
k) the SILV-A haplotype allele 'TC' or 'CC';
l) the TYR-A haplotype allele 'GA', 'AA' or 'GG'; and
m) the TYRP1-B haplotype allele 'GTG', 'GTT' or 'TTT'.

By way of another example, a method according to this aspect of the invention can include determining the nucleotide occurrence for at least one of the SNPs disclosed herein as segregating preferentially with eye shade or hair shade. These SNPs include:

nucleotide 609 of SEQ ID NO:1 [marker 702], nucleotide 501 of SEQ ID NO:2 [marker 650], nucleotide 256 of SEQ ID NO:3 [marker 675], nucleotide 442 of SEQ ID NO:4 [marker 217438], nucleotide 619 of SEQ ID NO:5 [marker 217439], nucleotide 646 of SEQ ID NO:6 [marker 217441]; nucleotide 135 of SEQ ID NO:7 [marker 217458], nucleotide 193 of SEQ ID NO:8 [marker 886894], nucleotide 228 of SEQ ID NO:9 [marker 886895], nucleotide 245 of SEQ ID NO:10 [marker 886896], nucleotide 189 of SEQ ID NO:11 [217452], nucleotide 573 of SEQ ID NO:12 [712052], nucleotide 245 of SEQ ID NO:13 [marker 886994], nucleotide 643 of SEQ ID NO:14 [marker 712057], nucleotide 539 of SEQ ID NO:15 [marker 712058], nucleotide 418 of SEQ ID NO:16 [marker 712060], nucleotide 795 of SEQ ID NO:17 [marker 712064], nucleotide 535 of SEQ ID NO:18 [marker 712054], nucleotide 554 of SEQ ID NO:19 [marker 712056], nucleotide 210 of SEQ ID NO:20 [marker 886892], nucleotide 225 of SEQ ID NO:21 [marker217455], nucleotide 170 of SEQ ID NO:22 [marker 712061], nucleotide 210 of SEQ ID NO:20 [marker 886892], nucleotide 172 of SEQ ID NO:23 [marker 886938], nucleotide 216 of SEQ ID NO:24 [marker 886943], nucleotide 61 of SEQ ID NO:25 [marker 560], nucleotide 201 of SEQ ID NO:26 [marker 552], nucleotide 201 of SEQ ID NO:27 [marker 559], nucleotide 201 of SEQ ID NO:25 [marker 468], nucleotide 657 of SEQ ID NO:29 [marker 657], nucleotide 599 of SEQ ID NO:30 [marker 674], nucleotide 267 of SEQ ID NO:31 [marker 632], nucleotide 61 of SEQ ID NO:32 [marker 701], nucleotide 451 of SEQ ID NO:33 [marker 710]; nucleotide 326 of SEQ ID NO:34 [marker 217456], nucleotide 61 of SEQ ID NO:35 [marker 656], nucleotide 61 of SEQ ID NO:36, nucleotide 61 of SEQ ID NO:37 [marker 637], nucleotide 93 of SEQ ID NO:38 [marker 278], nucleotide 114 of SEQ ID NO:39 [marker 386], nucleotide 558 of SEQ ID NO:40 [marker 217480], nucleotide 221 of SEQ ID NO:41 [marker 951497], nucleotide 660 of SEQ ID NO:42 [marker 217468], nucleotide 163 of SEQ ID NO:43 [marker 217473], nucleotide 364 of SEQ ID NO:44 [marker 217485], nucleotide 473 of SEQ ID NO:45 [marker 217486], nucleotide 314 of SEQ ID NO:46 [marker 869787], nucleotide 224 of SEQ ID NO:47 [marker 869745], nucleotide 169 of SEQ ID NO:48 [marker 886933], nucleotide 214 of SEQ ID NO:49 [marker 886937], or nucleotide 903 of SEQ ID NO:50 [marker 886942], nucleotide 207 of SEQ ID NO:51 [marker 217459], nucleotide 428 of SEQ ID NO:52 [marker 217460], nucleotide 422 of SEQ ID NO:48 [marker 217487], nucleotide 459 of SEQ ID NO:54 [marker 217489], nucleotide 1528 of SEQ ID NO:55 [marker 554353], nucleotide 1093 of SEQ ID NO:56 [marker 554363], nucleotide 1274 of SEQ ID NO:57 [marker 554368], nucleotide 1024 of SEQ ID NO:58 [marker 554370], nucleotide 1159 of SEQ ID NO:59 [marker 554371], nucleotide 484 of SEQ ID NO:60 [marker 615921], nucleotide 619 of SEQ ID NO:61 [marker 615925], nucleotide 551 of SEQ ID NO:62 [marker 615926], nucleotide 1177 of SEQ ID NO:63 [marker 664784], nucleotide 1185 of SEQ ID NO:64 [marker 664785], nucleotide 1421 of SEQ ID NO:65 [664793], nucleotide 1466 of SEQ ID NO:66 [marker 664802], nucleotide 1311 of SEQ ID NO:67 [marker 664803], nucleotide 808 of SEQ ID NO:68 [marker 712037], nucleotide 1005 of SEQ ID NO:69 [marker 712047], nucleotide 743 of SEQ ID NO:70 [marker 712051], nucleotide 418 of SEQ ID NO:71 [marker 712055], nucleotide 884 of SEQ ID NO:72 [marker 712059], nucleotide 744 of SEQ ID NO:73 [marker 712043], nucleotide 360 of SEQ ID NO:74 [marker 756239], nucleotide 455 of SEQ ID NO:75 [marker 756251], nucleotide 519 of SEQ ID NO:76 [marker 809125], nucleotide 277 of SEQ ID NO:77 [marker 869769], nucleotide 227 of SEQ ID NO:78 [marker 869772], nucleotide 270 of SEQ ID NO:79 [marker 869777], nucleotide 216 of SEQ ID NO:80 [marker 869784], nucleotide 172 of SEQ ID NO:81 [marker 869785], nucleotide 176 of SEQ ID NO:82 [marker 869794], nucleotide 145 of SEQ ID NO:83 [marker 869797], nucleotide 164 of SEQ ID NO:84 [marker 869798], nucleotide 166 of SEQ ID NO:85 [marker 869802], nucleotide 213 of SEQ ID NO:86 [marker 869809], nucleotide 218 of SEQ ID NO:87 [marker 869810], nucleotide 157 of SEQ ID NO:88 [marker 869813], nucleotide 837 of SEQ ID NO:89 [marker 886934], nucleotide 229 of SEQ ID NO:90 [marker 886993], nucleotide 160 of SEQ ID NO:91 [marker 951526], or any combination thereof.

By way of another example, a method according to this aspect of the invention can include determining the nucleotide occurrence for at least one of:

nucleotide 442 of SEQ ID NO:4 [marker 217438], nucleotide 619 of SEQ ID NO:5 [marker 217439], nucleotide 646 of SEQ ID NO:6 [marker 217441]; nucleotide 193 of SEQ ID NO:8 [marker 886894], nucleotide 228 of SEQ ID NO: 9 [marker 886895], nucleotide 245 of SEQ ID NO:10 [marker 886896], nucleotide 189 of SEQ ID NO:11 [217452], nucleotide 573 of SEQ ID NO:12 [712052], nucleotide 245 of SEQ ID NO:13 [marker 886994], nucleotide 643 of SEQ ID NO:14 [marker 712057], nucleotide 539 of SEQ ID NO:15 [marker 712058], nucleotide 795 of SEQ ID NO: 17 [marker 712064], nucleotide 535 of SEQ ID NO:18 [marker 712054], nucleotide 210 of SEQ ID NO:20 [marker 886892], nucleotide 225 of SEQ ID NO:21 [marker 217455], nucleotide 558 of SEQ ID NO:40 [marker 217450], nucleotide 221 of SEQ ID NO:41 [marker 951497], nucleotide 660 of SEQ ID NO:42 [marker 217468], nucleotide 163 of SEQ ID NO:43 [marker 217473], nucleotide 364 of SEQ ID NO:44 [marker 217485], nucleotide 473 of SEQ ID NO:45 [marker 217486], nucleotide 314 of SEQ ID NO:46 [marker 869787], nucleotide 224 of SEQ ID NO:47 [marker 869745], nucleotide 169 of SEQ ID NO:48 [marker 886933], nucleotide 214 of SEQ ID NO:49 [marker 886937], nucleotide 207 of SEQ ID NO:51 [marker 217459], nucleotide 428 of SEQ ID NO:52 [marker 217460], nucleotide 422 of SEQ ID NO:48 [marker 217487], nucleotide 459 of SEQ ID NO:54 [marker 217489], nucleotide 1528 of SEQ ID NO:55 [marker 554353], nucleotide 1093 of SEQ ID NO:56 [marker 554363], nucleotide 1274 of SEQ ID NO:57 [marker 554368], nucleotide 1024 of SEQ ID NO:58 [marker 554370], nucleotide 1159 of SEQ ID NO:59 [marker 554371], nucleotide 484 of SEQ ID NO:60 [marker 615921], nucleotide 619 of SEQ ID NO:61 [marker 615925], nucleotide 551 of SEQ ID NO:62 [marker 615926]. nucleotide 1177 of SEQ ID NO:63 [marker 664784], nucleotide 1185 of SEQ ID NO.64 [marker 664785], nucleotide 1421 of SEQ ID NO:65 [664793], nucleotide 1466 of SEQ ID NO:66 [marker 664802], nucleotide 1311 of SEQ ID NO:67 [marker 664803], nucleotide 808 of SEQ ID NO:68 [marker 712037], nucleotide 1005 of SEQ ID NO:69 [marker 712047], nucleotide 743 of SEQ ID NO:70 [marker 712051]. nucleotide 418 of SEQ ID NO:71 [marker 712055], nucleotide 884 of SEQ ID NO:72 [marker 712059], nucleotide 744 of SEQ ID NO:73 [marker 712043], nucleotide 360 of SEQ ID NO:74 [marker 756239], nucleotide 455 of SEQ ID NO:75 [marker 756251], nucleotide 519 oC SEQ ID NO:76 [marker 809125], nucleotide 277 of SEQ ID NO:77 [marker 869769], nucleotide 227 of SEQ ID NO:78 [marker 869772], nucleotide 270 of SEQ ID NO:79 [marker 869777], nucleotide 216 of SEQ ID NO:80 [marker 869784], nucleotide 172 of SEQ ID NO:81 [marker 869785], nucleotide 176 of SEQ ID NO:S2 [marker 869794], nucleotide 145 of SEQ ID NO:83 [marker 869797], nucleotide 164 of SEQ ID NO:84 [marker 869798], nucleotide 166 of SEQ ID NO:85 [marker 869802], nucleotide 213 of SEQ ID NO:86 [marker 869809], nucleotide 218 of SEQ ID NO:87 [marker 869810], nucleotide 157 of SEQ ID NO:88 [marker 869813], nucleotide 837 of SEQ ID NO:89 [marker 886934], nucleotide 229 of SEQ ID NO:90 [marker 886993], nucleotide 160 of SEQ ID NO:91 [marker 951526], or any combination thereof. Example 14 discloses that the panel of 64 SNPs listed above can be used to infer the ethnic origin of a DNA specimen with near perfect accuracy in a sample of Asian, African, and Caucasian descent.

The invention also relates to a method for classifying an individual as being a member of a group sharing a common characteristic. Such a method can be performed, for example, by identifying a nucleotide occurrence of a SNP in a polynucleotide of the individual, wherein the SNP corresponds to nucleotide 473 of SEQ ID NO:45 [marker 217486], nucleotide 224 of SEQ ID NO:47 [marker 869745], nucleotide 314 of SEQ ID NO:46 [marker 869787], nucleotide 210 of SEQ ID NO:20 [marker 886892], nucleotide 228 of SEQ ID NO:9 [marker 886895], nucleotide 245 of SEQ ID NO:10 [marker 886896], nucleotide 169 of SEQ ID NO:48 [marker 886933], nucleotide 214 of SEQ ID NO:49 [marker 886937], nucleotide 245 of SEQ ID NO:13 [marker 886994], nucleotide 193 of SEQ ID NO:8 [marker 886894], nucleotide 172 of SEQ ID NO:23 [marker 886938], nucleotide 216 of SEQ ID NO:24 [marker 886943], or nucleotide 903 of SEQ ID NO: 50 [marker 886942], or any combination thereof.

Methods described above for identifying a SNP can be used to identify an occurrence of a polynucleotide in a SNP for this aspect of the invention. For example, a method according to this aspect of the invention can include an amplification reaction, a primer extension reaction, or an immunoassay to identify the nucleotide occurrence of the SNP.

In another aspect the invention provides a method for detecting a nucleotide occurrence for a single nucleotide polymorphism (SNP) of a human pigmentation gene. The method includes:

i) incubating a sample that includes a polynucleotide with a specific binding pair member, wherein the specific binding pair member specifically binds at or near a polynucleotide suspected of being polymorphic, wherein the polynucleotide comprises one of the nucleotide occurrences corresponding to at least one of nucleotide 473 of SEQ ID NO:45 [marker 217486], nucleotide 224 of SEQ ID NO:47 [marker 869745], nucleotide 314 of SEQ ID NO:46 [marker 869787], nucleotide 210 of SEQ ID NO:20 [marker 886892], nucleotide 228 of SEQ ID NO:9 [marker 886895], nucleotide 245 of SEQ ID NO:10 [marker 886896], nucleotide 169 of SEQ ID NO:48 [marker 886933], nucleotide 214 of SEQ ID NO:49 [marker 886937], nucleotide 245 of SEQ ID NO:13 [marker 886994], nucleotide 193 of SEQ ID NO:8 [marker 886894], nucleotide 172 of SEQ ID NO:23 [marker 886938], nucleotide 216. of SEQ ID NO:24 [marker 886943], or nucleotide 903 of SEQ ID NO:50 [marker 886942], or any combination thereof; and
ii) detecting selective binding of the specific binding pair member.

Selective binding is indicative of the presence of the nucleotide occurrence. The nucleotide occurrence for the polymorphism can be detected. In another aspect the invention provides an isolated primer pair for determining a nucleotide occurrence of a single nucleotide polymorphism (SNP) in a polynucleotide. A forward primer of the primer pair binds the polynucleotide upstream of the SNP position on one strand and a reverse primer binds the polynucleotide upstream of the SNP position on a complementary strand. For this aspect of the invention the SNP position corresponds to nucleotide 473 of SEQ ID NO:45 [marker 217486], nucleotide 224 of SEQ ID NO:47 [marker 869745], nucleotide 314 of SEQ ID NO:46 [marker 869787], nucleotide 210 of SEQ ID NO:20 [marker 886892], nucleotide 228 of SEQ ID NO:9 [marker 886895], nucleotide 245 of SEQ ID NO:10 [marker 886896], nucleotide 169 of SEQ ID NO:48 [marker 886933], nucleotide 214 of SEQ ID NO:49 [marker 886937], nucleotide 245 of SEQ ID NO:13 [marker 886994], nucleotide 193 of SEQ ID NO:8 [marker 886894], nucleotide 172 of SEQ ID NO:23 [marker 886938], nucleotide 216 of SEQ ID NO:24 [marker 886943], or nucleotide 903 of SEQ ID NO:50 [marker 886942]. The primer pair can be used in an amplification reaction as described above, as is well known in the art.

In another aspect, the invention provides an isolated specific binding pair member for determining a nucleotide occurrence of a single-nucleotide polymorphism (SNP) in a polynucleotide. The specific binding pair member for this aspect of the invention specifically binds to the polynucleotide at or near nucleotide 473 of SEQ ID NO:45 [marker 217456], nucleotide 224 of SEQ ID NO:47 [marker 869745], nucleotide 314 of SEQ ID NO:46 [marker 869787], nucleotide 210 of SEQ ID NO:20 [marker 886892], nucleotide 228 of SEQ ID NO:9 [marker 886895], nucleotide 245 of SEQ ID NO:10 [marker 886896], nucleotide 169 of SEQ ID NO:48 [marker 886933], nucleotide 214 of SEQ ID NO:49 [marker 886937], nucleotide 245 of SEQ ID NO:13 [marker 886994], nucleotide 193 of SEQ ID NO:8 [marker 886894], nucleotide 172 of SEQ ID NO:23 [marker 886938], nucleotide 216 of SEQ ID NO:24 [marker 886943], or nucleotide 903 of SEQ ID NO:50 [marker 886942].

The specific binding pair member can be used to identify the nucleotide occurrence at a SNP, for example a pigmentation-related SNP using methods described above for identifying SNPs. Many types of specific binding pair members are known in the art. The specific binding pair member can be a polynucleotide probe, an antibody, or a substrate for a primer extension reaction. For methods wherein the specific binding pair member is a substrate for a primer extension reaction, the specific binding pair member is a primer that binds to a polynucleotide at a sequence comprising the SNP as the terminal nucleotide. As discussed above, methods such as SNP-IT (Orchid BioSciences), utilize primer extension reactions using a primer whose terminal nucleotide binds selectively to certain nucleotides at a SNP loci, to identify a nucleotide occurrence at the SNP loci..

In another aspect, the invention provides an isolated polynucleotide that includes at least 30 nucleotides of the human OCA2 gene, where the polynucleotide includes one or more of a thymidine residue at a nucleotide corresponding to nucleotide 193 of SEQ ID NO:8 [marker 886894], a guanidine residue at a nucleotide corresponding to nucleotide 228 of SEQ ID NO:9 [marker 886895], a cytidine residue at a nucleotide corresponding to nucleotide 210 of SEQ ID NO: 23 [marker 886892], a thymidine residue at a nucleotide corresponding to nucleotide 245 of SEQ ID NO:10 [marker 886896], a adenosine residue at a nucleotide corresponding to nucleotide 245 of SEQ ID NO:13 [marker 886994], or a combination thereof. In certain embodiments of this aspect of the invention, the isolated polynucleotide can be 50, 100, 150, 200, 250, 500, 1000, etc. nucleotides in length. In certain embodiments of this aspect of the invention, the isolated polynucleotide can be at least 50, at least 100, at least 150, at least 200, at least 250, at least 500, at least 1000, etc. nucleotides in length. In another aspect, the invention provides an isolated polynucleotide comprising at least 30 nucleotides of the human TYRP gene, wherein the polynucleotide includes one or more of a thymidine residue at a nucleotide corresponding to nucleotide 172 of SEQ ID NO:23 [marker 886938], a thymidine residue at a nucleotide corresponding to nucleotide 216 of SEQ ID NO:24 [marker 886943], a thymidine residue at a nucleotide corresponding to nucleotide 473 of SEQ ID NO:45 [marker 217486], a cytidine residue at a nucleotide corresponding to nucleotide 224 of SEQ ID NO:47 [marker 869745], a guanidine residue at a nucleotide corresponding to nucleotide 314 of SEQ ID NO:46 [marker 869787], a cytidine residue at a nucleotide corresponding to nucleotide 169 of SEQ ID NO:48 [marker 886933], a thymidine residue at a nucleotide corresponding to nucleotide 214 of SEQ ID NO:49 [marker 886937], a adenosine residue at a nucleotide corresponding to nucleotide 903 of SEQ ID NO:50 [marker 886942], or a combination thereof. In certain embodiments of this aspect of the invention, the isolated polynucleotide can be 50, 100, 150, 200, 250, 500, 1000, etc. nucleotides in length. In certain embodiments of this aspect of the invention, the isolated polynucleotide can be at least 50, at least 100, at least 150, at least 200, at least 250, at least 500, at least 1000, etc. nucleotides in length.

In another aspect, the invention provides an isolated polynucleotide at least 30 nucleotides in length, wherein the isolated polynucleotide includes:

a) a segment of the DCT gene wherein nucleotides CTG or GTG occur at positions corresponding to nucleotide 609 of SEQ ID NO:1 [marker 702], nucleotide 501 of SEQ ID NO:2 [marker 650], and nucleotide 256 of SEQ ID NO: 3 [marker 675] [marker 675], respectively;
b) a segment of the MC1R gene wherein nucleotides CCC, CTC, TCC or CCT occur at positions corresponding to nucleotide 442 of SEQ ID NO:4 [217438], nucleotide 619 of SEQ ID NO:5 [217439], and nucleotide 646 of SEQ ID NO:6 [217441], respectively;
c) a segment of the OCA2 gene wherein nucleotides TTAA, CCAG, or TTAG occur at positions corresponding to nucleotide 135 of SEQ ID NO:7 [217458], nucleotide 193 of SEQ ID NO:8 [886894], nucleotide 228 of SEQ ID NO: 9 [886895], and nucleotide 245 of SEQ ID NO:10 [886896], respectively;
d) a segment of the OCA2 gene wherein nucleotides CAA, CGA, CAC, or CGC occur at positions corresponding to nucleotide 189 of SEQ ID NO: 11 [217452], nucleotide 573 of SEQ ID NO: 12 [712052], and nucleotide 245 of SEQ ID NO:I3 [886994], respectively;
e) a segment of the OCA2 gene wherein nucleotides GGAA, TGAA, and TAAA occur at positions corresponding to nucleotide 643 of SEQ ID NO:14 [712057], nucleotide 539 of SEQ ID NO:15 [712058], nucleotide 418 of SEQ ID NO:16 [712060], and nucleotide 795 of SEQ ID NO:17 [712064], respectively;
f) a segment of the OCA2 gene wherein nucleotides AGG or GGG occur at positions corresponding to nucleotide 535 of SEQ ID NO:18 [712054], nucleotide 554 of SEQ ID NO:19 [712056], and nucleotide 210 of SEQ ID NO:20 [886892], respectively;
g) a segment of the OCA2 gene wherein nucleotides GCA occur at positions corresponding to nucleotide 225 of SEQ ID NO:21 [217455], nucleotide 170 of SEQ ID NO:22 [712061], and nucleotide 210 of SEQ ID NO:20 [886892], respectively; or
h) a segment of the TYRP1 gene wherein nucleotides TC occur at positions corresponding to nucleotide 172 of SEQ ID NO:23 [886938], and nucleotide 216 of SEQ ID NO:24 [886943], respectively. This isolated nucleotide includes the alleles for penetrant eye color or eye shade haplotypcs. In certain examples, the isolated polynucleotide is derived from the OCA2 gene and includes any combination of c-g.

In another aspect, the invention provides an isolated polynucleotide at least 30 positions in length, wherein the isolated polynucleotide includes:

a) a segment of the ASIP gene wherein nucleotides GT or AT occur at positions corresponding to nucleotide 201 of SEQ ID NO:26 [552], and nucleotide 201 of SEQ ID NO:28 [468], respectively;
b) a segment of the DCT gene wherein nucleotides TA or TG occur at positions corresponding to nucleotide 451 of SEQ ID NO:33 [710], and nucleotide 356 of SEQ ID NO:29 [657], respectively;
c) a segment of the SILV gene wherein nucleotides TC, TT, or CC occur at positions corresponding to nucleotide 61 of SEQ ID NO:35 [656], and nucleotide 61 of SEQ ID NO:36 [662], respectively;
d) a segment of the TYR gene wherein nucleotides GA, AA, or GG occur at positions corresponding to nucleotide 93 of SEQ ID NO:38 [278], and nucleotide 114 of SEQ ID NO:39 [386], respectively; or
e) a segment of the TYRP1 gene wherein nucleotides GTG, TTG, or GTT occur at positions corresponding to nucleotide 442 of SEQ ID NO:44 [217485], nucleotide 442 of SEQ ID NO:48 [886933], and nucleotide 442 of SEQ ID NO:49 [886937], respectively.

This isolated polynucleotide includes the alleles for latent eye color or eye shade haplotypes. In certain embodiments of this aspect of the invention, the isolated polynucleotide can be 50, 100, 150, 200, 250, 500, 1000, etc. nucleotides in length.

In another aspect, the invention provides an isolated polynucleotide at least 30 positions in length, which includes:

a) a segment of the ASIP gene wherein nucleotides GA or AA occur at positions corresponding to nucleotide 201 of SEQ ID NO:27 [559], and nucleotide 61 of SEQ ID NO:25 [560], respectively;
b) a segment of the MC1 R gene wherein nucleotides CCC, CTC, TCC or CCT occur at positions corresponding to nucleotide 442 of SEQ ID NO:4 [217438], nucleotide 619 of SEQ ID NO:5 [217439], and nucleotide 646 of SEQ ID NO:6 [217441], respectively;
c) a segment of the OCA2 gene wherein nucleotides AGG or AGA occur at positions corresponding to nucleotide 418 of SEQ ID NO:16 [712060], nucleotide 210 of SEQ ID NO:20 [886892], and nucleotide 245 of SEQ ID NO:10 [886896], respectively;
d) a segment of the OCA2 gene wherein nucleotides AGT or ATT occur at positions corresponding to nucleotide 225 of SEQ ID NO:21 [217455], nucleotide 643 of SEQ ID NO:14 [712057], and nucleotide 193 of SEQ ID NO:8 [886894], respectively;
e) a segment of the OCA2 gene wherein nucleotides TG occur at positions corresponding to nucleotide 135 of SEQ ID NO:7 [217458], and nucleotide 554 of SEQ ID NO:19 [712056], respectively;
f) a segment of the OCA2 gene wherein nucleotides GA or AA occur at positions corresponding to nucleotide 535 of SEQ ID NO:18 [712054], and nucleotide 228 of SEQ ID NO:9 [886895], respectively; or
g) a segment of the TYRP gene wherein nucleotides AA or TA occur at positions corresponding to nucleotide 442 of SEQ ID NO:45 [217486], and nucleotide 442 of SEQ ID NO:49 [886937], respectively, or any combination thereof.

This isolated nucleotide includes one or any combination of alleles for penetrant eye color or eye shade haplotypes. In certain examples, the isolated polynucleotide is derived from the OCA2 gene and includes any combination of c-f. In certain embodiments of this aspect of the invention, the isolated polynucleotide can be 50, 100, 150, 200, 250, 500, 1000, etc. nucleotides in length. In certain embodiments of this aspect of the invention, the isolated polynucleotide can be at least 50, at least 100, at least 150, at least 200, at least 250, at least 500, at least 1000, etc. nucleotides in length.

In another aspect, the invention provides a method for identifying genes, including pigmentation genes, SNPs, SNP alleles, haplotypes, and haplotype alleles that are statistically associated with a pigmentation trait. This aspect of the invention provides commercially valuable research tools, for example. The approach can be performed generally as follows:

1) Select genes from the human genome database that arc likely to be involved in the synthesis, degradation and deposition of melanin;
2) Identify the common genetic variations in the selected genes by designing primers to flank each promoter, exon and 3' UTR for each of the genes; amplifying and sequencing the DNA corresponding to each of these regions in enough donors of varying ethnic backgrounds to provide a statistically significant sample (e.g., approximately 500 multi-ethnic donors); and utilizing an algorithm to compare the sequences to one another in order to identify the positions within each region of each gene that are variable in the population, to produce a gene map for each of the relevant genes;
3) Use the gene maps to design and execute large-scale genotyping experiments, whereby a-significant number of individuals, typically at least one hundred, more preferably at least two hundred individuals, of known hair, eye and skin color (and ethnicity) are scored for the polymorphisms; and
4) Use the results obtained in step 3) to identify genes, polymorphisms, and sets of polymorphisms, including haplotypes, that are quantitatively and statistically associated with pigmentation.

Examples 4, 14, and 17, illustrate general approaches for discovering pigmentation-related SNPs and SNP alleles as provided above. For example, pigmentation-related SNPs and SNP alleles can be discovered using DNA from blood samples of patients exhibiting variable eye, skin and skin pigmentation levels (colors). Data on eye color, hair color, skin color, and race can also be collected and analyzed for patients providing the blood samples. Assays for identifying the alleles of a SNP or a SNP candidate can be performed using, for example, an Orchid SNPstream 25K instrument (Orchid BioSciences, Inc., Princeton, NJ) for high throughput genotyping. Other assays known in the art, as described above for identifying nucleic acid occurrences at SNPs can be used for this step, as will be readily apparent to a skilled artisan.

Specimens from patient samples can be used as a template for amplification using a polymerase, such as of Pfu turbo thermostable DNA polymerase, Taq polymerase, or a combination thereof. Amplification can be performed using standard conditions. For example, amplification can be performed in the presence of 1.5 mM $MgCl_2$, 5 mM KCl,

1 mM Tris, pH 9.0, and 0.1% Triton X-100 nonionic detergent. Amplification products can be cloned into a T-vector using the Clontech (Palo Alto CA) PCR Cloning Kit, transformed into Calcium Chloride Competent cells (Stratagene; La Jolla CA), plated on LB-ampicillin plates, and grown overnight.

Clones can be selected from each plate, isolated by mini-prep using the Promega Wizard or Qiagen Plasmid Purification Kit, and sequenced using standard methods, such as using PE Applied Biosystems Big Dye Terminator Sequencing Chemistry. Sequences can be trimmed of vector sequence and quality trimmed, and deposited into an Internet based relational database system.

Candidate SNPs can be also discovered from pigmentation-related or race-related (see below) genes ("data mining") using, for example, the NCBI SNP database, the Human Genome Unique Gene database (Unigene; NCBI). Sequence files for the genes can be downloaded from proprietary and public databases and input into a SNP/HAPLOTYPE automated pipeline discovery software system such as (SNiPDOC$^{SM}$ system; DNAPrint genomics, Inc.; Sarasota FL). This system finds candidate SNPs among the sequences, and documents haplotypes for the sequences with respect to these SNPs. The software uses a variety of quality control metrics when selecting candidate SNPs including the use of user specified stringency variables, the use of PHRED quality control scores and others *(See* U.S. Pat. App. No. Serial No.: 09/964,059, filed September 26, 2001).

As illustrated in the Examples herein, and as described in more detail therein, the invention provides methods for discovering penetrant haplotype alleles. For example, the method can use an iterative, empirical approach to test haplotype alleles of all possible SNP combination within a gene, for the ability to statistically resolve individuals of various trait values. Alternatively, preferred haplotype alleles discovered in a population can be analyzed.

In another aspect, the invention provides a method for identifying a pigmentation-related or a race-related single nucleotide polymorphism (SNP). The method includes:

i) identifying a candidate SNP of a pigmentation-related gene or a race-related gene;
ii) determining that the SNP has a genotype class comprising alleles exhibiting a coherent inheritance pattern, and a minor allele frequency that is greater than 0.01 in at least one race, thereby identifying a validated SNP;
iii) determining that the validated SNP exhibits significantly different genotype distributions and allele frequencies between individuals of different pigmentation phenotypes or racial classes, thereby identifying a pigmentation-related or race-related SNP.

The invention also relates to kits, which can be used, for example, to perform a method of the invention. Thus, in one embodiment, the invention provides a kit for identifying haplotype alleles of pigmentation-related SNPs. Such a kit can contain, for example, an oligonucleotide probe, primer, or primer pair, or combinations thereof, of the invention, such oligonucleotides being useful, for example, to identify a SNP or haplotype allele as disclosed herein; or can contain one or more polynucleotides corresponding to a portion of a pigmentation, xenobiotic, or other relevant gene containing one or more nucleotide occurrences associated with a genetic pigmentation trait, with race, or with a combination thereof, such polynucleotide being useful, for example, as a standard (control) that can be examined in parallel with a test sample. In addition, a kit of the invention can contain, for example, reagents for performing a method of the invention, including, for example, one or more detectable labels, which can be used to label a probe or primer or can be incorporated into a product generated using the probe or primer (e.g., an amplification product); one or more polymerases, which can be useful for a method that includes a primer extension or amplification procedure, or other enzyme or enzymes (e.g., a ligase or an endonuclease), which can be useful for performing an oligonucleotide ligation assay or a mismatch cleavage assay; and/or one or more buffers or other reagents that are necessary to or can facilitate performing a method of the invention.

In one embodiment, a kit of the invention includes one or more primer pairs of the invention, such a kit being useful for performing an amplification reaction such as a polymerase chain reaction (PCR). Such a kit also can contain, for example, one or reagents for amplifying a polynucleotide using a primer pair of the kit. The primer pair(s) can be selected, for example, such that they can be used to determine the nucleotide occurrence of a pigmentation-related SNP, wherein a forward primer of a primer pair selectively hybridizes to a sequence of the target polynucleotide upstream of the SNP position on one strand, and the reverse primer of the primer pair selectively hybridizes to a sequence of the target polynucleotide upstream of the SNP position on a complementary strand. When used together in an amplification reaction an amplification product is formed that includes the SNP loci.

In addition to primer pairs, in this embodiment the kit can further include a probe that selectively hybridizes to the amplification product of one of the nucleotide occurrences of a SNP, but not the other nucleotide occurrence. Also in this embodiment, the kit can include a third primer which can be used for a primer extension reaction across the SNP loci using the amplification product as a template. In this embodiment the third primer preferably binds to the SNP loci such that the nucleotide at the 3' terminus of the primer is complementary to one of the nucleotide occurrences at the SNP loci. The primer can then be used in a primer extension reaction to synthesize a polynucleotide using the amplification product as a template, preferably only where the nucleotide occurrence is complementary to the 3' nucleotide of the primer. The kit can further include the components of the primer extension reaction.

In another embodiment, a kit of the invention provides a plurality of oligonucleotides of the invention, including one or more oligonucleotide probes or one or more primers, including forward and/or reverse primers, or a combination of such probes and primers or primer pairs. Such a kit provides a convenient source for selecting probe(s) and/or primer (s) useful for identifying one or more SNPs or haplotype alleles as desired. Such a kit also can contain probes and/or primers that conveniently allow a method of the invention to be performed in a multiplex format.

The kit can also include instructions for using the probes or primers to identify a pigmentation-related haplotype allele.

The power of the inference drawn according to the methods of the invention is increased by using a complex classifier function. Accordingly, preferred examples of the methods of the invention draw an inference regarding a pigmentation trait or race of a subject using a classification function. A classification function applies nucleotide occurrence information identified for a SNP or set of SNPs such as one or preferably a combination of haplotype alleles, to a set of rules to draw an inference regarding a pigmentation trait or a subject's race. The Examples included herein provide numerous strategies for developing and implementing a classifier function.

Example 7 shows that a classification scheme may be identified by performing statistical analysis on various combinations of SNPs and haplotypes until maximum accuracy is achieved.. In order to use these SNPs or haplotypes to develop a genetic solution that explains the maximum amount of variation of a pigmentation trait in the population, haplotypes incorporating each of these positions in individuals of a known pigmentation trait can be scored, and the results can be combined in various combinations in order to obtain the optimum solution for resolving individuals for that pigmentation trait, for example individuals with dark versus light hair color. Example 7 illustrates a composite, nested solution for classifying an unknown individual as belonging to the dark versus light hair colored groups.

In certain examples, genotype/biographical data matrices for two groups of pigmentation traits, for example, dark versus light eye color, can be used for a pattern detection algorithm such as the SNiPDOCS$^{SM}$ algorithm (DNAPrint genomics, Inc., Sarasota, FL). The purpose of pattern detections algorithms is to fit quantitative (or Mendelian) genetic data with continuous trait distributions (or discrete trait distributions, as the case may be).

One specific approach that can be used, as illustrated in Example 9, is a Bayesian method, using the frequencies of, for example eye color classes, as the prior probabilities and the frequency of a haplotype based genotype in the eye color class as the class conditional density functions. The posterior probability that a subject belongs to a given class of eye color shade is simply the product of the posterior probabilities derived for each of the four genes, and the eye color class with the highest probability is selected. The power of the inference drawn by this method can be increased by assigning weights to the posterior probabilities for each haplotype system, based on the amount of variance each explains on its own.

Furthermore, a nested statistical scheme can be developed, as illustrated in Example 9, by which to construct classification rules using complex, compound genotypes. A Bayesian classifier can also be used for this task. However, a routine can be chosen that resembles a genetic algorithm. Within the scheme, a compound genotype contains elements (haplotype pairs = genotypes) from multiple genes. The scheme builds a classification tree in a step-wise manner. The roots of the tree are genotypes of a randomly selected haplotype system. Nodes arc randomly selected genotype classes, within which there are numerous different constituent genotypes. Compound genotype classes contain more than one compound genotype, the constituents of which are derived from a discrete combination of haplotype systems. In these classification function strategies resembling a genetic classifier, edges connect roots and nodes to comprise compound genotype classes. The tree can be built by first selecting a set of roots and growing the edges to nodes based on the genetic distinction between individuals of light (blue, green) and dark (black, brown) eye color shade within the new compound genotype class defined by the connection (hazel is always assigned to the eye color shade with the most members). Within a compound genotype class, a pair-wise F statistic and associated p-value is used to measure the genetic structure differences between individuals of the various shade of eye colors, though an exact test p-value has also been used with similar results. Individuals of ambiguous haplotype class (less than 75% certainty) are discarded and classified as "not classifiable". All possible nodes not yet incorporated in the path from the root are tested during each new branching step. The branch that results in the most distinctive partition (i.e., the lowest p-value) among the classes of eye color shade is selected.

If there is no genetic structure within the new compound genotype class, another node (haplotype) is selected for possible branching, unless there are no more haplotype systems to consider or unless the sample size for the compound genotype is below a certain pre-selected threshold (in which case a "no-decision" is specified). If the lowest p-value for the new compound genotype class is significant, rules are made from its constituent compound genotypes exhibiting significant chi-square residuals. In this case, genotypes within the compound genotype class which are not explainable (for whom chi-square residuals are not significant) are segregated from the rest of the compound genotypes within the class to form new nested node(s), from which further branching is accomplished. Nested nodes always represent new compound genotype classes at first. If branching from this nested node does not result in the ability to create classification rules, the algorithm returns to the compound genotype class from which the nested node was derived and recreates N nested nodes of N constituent compound genotypes. In either case, nested nodes are only created from nodes with statistically significant population structure differences among the shade of eye color classes. In effect, this algorithm allows for the maximum amount of genetic variance contributed by the various combinations of haplotype systems to be learned within specific genetic backgrounds. Once the tree has been completed, the rules produced from it are used to predict the race or pigmentation trait, for example eye shade, of each individual. If the prediction rate is good (e.g., 95% or greater) the process ends, and if it is not, the process is begun again starting with a new haplotype system for the root.

The classification function can also be performed using other classification methods, such as those disclosed in "Classification and Regression Trees" by Leo Brieman Charles J. Stone Richard A. Olshen Jerome H. Friedman, (Wadsworth International Group, Belmont, CA, 1984) or those provided in the following computer programs (Available from StatSoft (STATISTICA brand)) for classification analysis: QUEST (Loh & Shih, 1997) and C&RT (Breiman et. al., 1954) programs as well as FACT (Loh & Vanichestalcul, 1988) and THAID (Morgan & Messenger, 1973).

Classification trees can be applied to individual haplotypes, or to improve the accuracy of the inference drawn using the classification trees, can be applied to combinations of haplotypes.

Example 6 discusses a general method for qualifying a genetic association between a haplotype and a phenotype using a cladogram or a parsimony tree. In the parsimony tree, lines separate haplotypes that are one mutational step from another and biallelic positions within a gene are represented in binary form (1 and 0): Haplotypes residing at similar regions of a cladogram or tree tend to share common phenotypic attributes. This assumption is reasonable since haplotypes situated in proximity to one another share more sequence in common than randomly selected haplotypes, and it is the sequence of a gene that largely determines its function. As such, haplotype analysis using the cladogram provides a useful means for representing genetic data in such a way as to facilitate multivariate analyses for the determination of the biological relevance of the haplotype, as discussed in further detail in Example 6.

By way of a preferred example typically performed using computer software, the classification function can be developed using linear, quadratic, or correspondence analysis or classification tree multivariate modeling to develop a classifier function incorporating one or more SNPs or sets of SNPs that blindly generalizes to other individuals having a known pigmentation trait. For an example of a combined correspondence analysis and linear/quadratic analysis for constructing complex genetic classifiers see U.S. Pat. No. 60/377,164, filed May 2, 2002. In a preferred example, correspondence analysis is used to encode genotypes for creating the vectors. This overcomes a problem associated with dimensionality, and then the vector components are weighted using a heuristic algorithm to optimize the classifier.

In one embodiment, the invention includes a method for identifying a classifier function for inferring a pigmentation-trait of a subject. The method includes: i) identifying one or more candidate SNPs of one or more pigmentation genes that have a alleles exhibiting a coherent inheritance pattern (i.e., they are in Hardy-Wienberg equilibrium), and a minor allele frequency that is greater than 0.01 in at least one race, thereby identifying one or more validated SNPs; ii) determining that the one or more validated SNPs exhibits significantly different genotype distributions and allele frequencies between individuals of different pigmentation phenotypes or racial classes, and iii) Using linear, quadratic, correspondence analysis or classification tree multivariate modeling to develop an abstract classifier function incorporating one or more validated SNPs or combinations of validated SNPs that blindly generalizes to other individuals of known pigmentation, thereby identifying a pigmentation-related classification strategy.

In another embodiment, the invention includes a method for identifying a classifier function for inferring the race of a subject. The method includes:

i) identifying one or more candidate SNPs of one or more race-related genes that have a genotype class comprising alleles exhibiting a coherent inheritance pattern, and a minor allele frequency that is greater than 0.01 in at least one race, thereby identifying one or more validated SNPs; ii) determining that the one or more validated SNPs exhibits significantly different genotype distributions and allele frequencies between individuals of different pigmentation phenotypes or racial classes, and iii) Using linear, quadratic, correspondence analysis or classification tree multivariate modeling to develop an abstract classifier function incorporating one or more validated SNPs or combinations of validated SNPs that blindly generalizes to other individuals of known race, thereby identifying a classifier function for inferring the race of a subject.

In another embodiment, the invention provides a method for classifying a sample. The method includes: a) computing a genetic variance/covariance matrix for all possible trait class pairs; b) creating a combination of class mean vectors, wherein vector components are binary encodings, correspondence analysis principal coordinates, correspondence analysis factor scores or correspondence analysis standard coordinates; c) representing a sample as an n-dimensional sample vector; and d) classifying a sample by identifying a class mean vector from the combination of class mean vectors, that is the shortest distance from the sample. Such a method is illustrated in Example 14.

Example 17 illustrates the use of a classification function that uses a parametric, multivariate Quadratic classification technique with modifications for gcnomics data. Under the assumption that samples are taken from multivariate normal distributions with different mean vectors, with a common variance covariance matrix, a classification procedures introduced previously by Fisher, R.A. (Annals of Eugenics 1936. 7:179-188), Rao (1947,1948a,1948b) and Smith (Smith, C.A.B., et al., Annals of Eugenics 1948; 13:272-282), can be applied.

Under the assumption of normality, the sample mean vector and the sample covariance matrix constitute minimally sufficient statistics, in the sense that any inference based of them carries with it all the information available in the sample. Thus, any classification rule based on these summary statistics ought to be optimal from the point of view of sample information used for their analysis. However, with complex systems, the data often provide additional information not reflected by these statistics, and this additional information can often be used for improving the results based on these statistics. With genetics, sequences may contribute towards phenotype variation through dominance or additivity, wherein their associations with trait values from independent analyses are of varying degrees of strength, but statistically significant. Alternatively, sequences may contribute through epistasis, wherein their association with trait values from independent analyses is weak or non-existent.

To produce a quadratic classifier sensitive for the epistatic contributions, we devised a weighting scheme for producing unequal variance-covariance matrices for each of the iris color groups used in quadratic analysis. First the most strongly associated genotypes were identified. Next, genotypes of weaker association were randomly selected. Normally when constructing the covariance matrix, M for each factor was calculated using the Z-scores and binary values; a value of 0 within the individual vector if the genotype was absent in an individual, and a I if present. Using the weighting scheme, instead of using a binary x when calculating M for each factor, 1+x was used for randomly selected weakly/non-associated sequences, where x is the number of strongly associated genotypes also present in that individual.

By successively selecting random combinations of weakly/non-associated pigmentation gene features for weighting and testing how well the model derived from these combinations generalizes to the test sample for iris color classification, an optimal weighting strategy can be obtained. Recoding in this manner generally increases the variability of the scores of weakly/non-associated sequences and hence it improves the discriminating power of the model. Although the coding procedure may seem arbitrary, it is important from a practical point of view. For example, there are instances in the areas of statistical forecasting of time series or economics, wherein a data supported methods are recommended, as long as they lead to relatively more accurate inferences. In this case, once the optimal model has been identified, the weighting used for its generation can provide clues on the non-linear relationships between genotypes of different genes towards complex trait variation (i.e., epistasis).

To test the accuracy of a classification function a Monte Carlo simulation study can be used. A computer program can be written to use a random number generator to select a significant number of individuals on the basis of observed allele frequencies from two pigmentation-trait groups to calculate a multivariate linear classification probability matrix. This experiment can be repeated many times (e.g., 10000 times) to get the summary statistics of Classification and misclassification rates and their Confidence Intervals.

Example 16, further discusses the recording method used in Example 17 for improving a classification analysis, especially those involving a sample mean vector and sample covariance matrix. This method utilizes additional information that is not reflected by these statistics.

This procedure recodes weaker genotypes whenever they appear along with 'best' genotypes in an individual sample unit.

Specifically the procedure can include the following:

Step 1. Identify a small number of 'best' genotypes for cross-coding the weak genotypes. This can be done by selecting a subset of the 'best' genotype in each gene according to their range of variation in their relative frequencies. Various combinations can be attempted to arrive at an optimal selection. The study reported in Example 16 revealed an optimal choice of the three genotypes g (1,1) (OCA2A), g (3,1) (OCA2C) and g (4,1) (OCA2D). (Note: the first number in parenthesis denotes the haplotype and the second number the allele of that haplotype. G(1,1) would means genoytpe 1 for feature combination 1. For example ATTA/ATTA may be genotype 1, ATTA/ATTG, genotype 2 etc for the OCA2-A SNP combination which is combination number 1.

Step 2: Recode second best genotypes:

Assign Code 0 if the genotype is absent

Code 1+ n, where n is the number of selected 'best' genotypes that occur together in an individual.

Such recoding generally increases the variability of scores across the colors (while carrying out the usual discriminant analysis), and hence one can expect a marginal improvement over the results obtained before incorporating such a recoding procedure in them.

The following examples are intended to illustrate but not limit the invention.

## EXAMPLE 1 IDENTIFICATION OF TYRP1 and OCA POLYMORPHISMS ASSOCIATED WITH PIGMENTATION IN HUMANS

A multi-step approach was designed to identify genes and gene variants in the population that arc statistically associated with hair, eye and skin color. The approach was performed generally as follows:

1) Select genes from the human genome database that are likely to be involved in the synthesis, degradation and deposition of melanin, the chemical that causes pigmentation.
2) Identify the common genetic variations in the selected genes by designing primers to flank each promoter, exon and 3' UTR for each of the genes; amplifying and sequencing the DNA corresponding to each of these regions in approximately 500 multi-ethnic donors; and utilizing an algorithm to compare the sequences to one another in order to identify the positions within each region of each gene that are variable in the population. This process results in a gene map for each of the relevant genes.
3) Use the gene maps to design and execute large-scale genotyping experiments, whereby several hundred individuals, of known hair, eye and skin color (and ethnicity) are scored for the polymorphisms.
4) Use the results obtained in step 3) to identify polymorphisms, and sets of polymorphisms, that are quantitatively and statistically associated with pigmentation.

No relationship to human pigmentation for any of the originally reported 3 SNPs for the TYRP 1 gene and 5 SNPs for the OCA gene has previously been reported. Accordingly, the polymorphisms were scored in hundreds of individuals of known hair, eye and skin color, and statistical analysis was performed on the results (see below). As disclosed herein, an SNP in the TYRP1 gene (TYRP1_3), which appears to be statistically associated with eye color, and an SNP in the OCA gene (OCA2_5), which appears to be statistically associated with eye color and hair color, were identified.

### A. METHODS:

Polymorphisms were scored using a single-nucleotide sequencing protocol and equipment purchased and licensed from Orchid Biosciences (Orchid SNPstream 25K instrument, (Orchid BioSciences, Inc., Princeton, NJ)). Briefly, primers were designed to flank the polymorphism (see Tables 1 to 4), whereby one primer of each pair contained 5' polythiophosphonate groups. Amplification products were physically attached to a solid substrate via the polythiophosphonate groups and washed using TNT buffer. Washed amplification products were subject to exonuclease III in order to produce single stranded, polythiophosphonatc strands. A primer was attached via hybridization to the single stranded molecule, such that the primer could be extended by a single labeled nucleotide.
The primers used for the OCA2_5 genotyping were:

CAATCACAGCCAGTGCTGC (SEQ ID NO: 97); and
GCGGTAATITCCTGTGCTTCT (SEQ ID NO: 98).
The primers used for the TYRP1_3 genotyping were
AAAGGGTCTTCCCAGCTTTG (SEQ ID NO: 99); and
GTGGTCTAACAAATGCCCTACTCTC (SEQ ID NO: 100).

For the TYRP1 polymorphism, if the incorporated nucleotide was a G, a monoclonal antibody was bound in the first step and read via secondary antibody hybridization and conjugate catalyzed reaction in a colorimeter. If the incorporated nucleotide was a T, the antibody did not bind and no color was read. In the second round of hybridization, an antibody that recognizes the modified "T" was used. If the amplification product for an individual contained a "T" at the position, the antibody bound, and was read via secondary binding and conjugate activity in the colorimeter. Individuals of the "GG" genotype showed a dark blue color in the first reaction, which did not change during the second reaction. Individuals of the "GT" genotype showed a light blue color in the first reaction, which became dark blue during the second reaction. Individuals of the "TT" genotype showed no color in the first reaction, and a dark blue color in the second reaction. For the OCA genotypes the letters read were GG, GA and AA, in the same manner.

### B. RESULTS:

The SNPs for TYRP1_3 (marker 217485) and OCA2_5 (marker 217455) are shown in Table 1 which provides information regarding a marker number for each SNP, the name of the gene in which the SNP is found on the chromosome, a public sequence database accession number for a sequence that includes at least one allele of the SNP (where appropriate), the variant IUB code for the SNP, as well as additional information such as the type of polymorphism (coding or non-coding).

The results, which were obtained from the same runs over a course of 2 days, demonstrate that some of the markers showed no relationship between genotype and pigmentation, whether it be eye, hair or skin (see below; see also, Table 1-1). These results (Table 1-1) provide an additional negative control to include with the "no template", "template, but no detection materials", and "water" controls run with each plate in each assay.

Results in Table 1-1 are segregated based on pigmentation, as well, as on the ethnicity of the donor. If a SNP allele is a genetic determinant, or is linked to a genetic determinant of pigmentation, then it should be enriched in African Americans as compared to Caucasians because the average African American generally tends to have darker average skin, eye and hair color than the average Caucasian. However, the reverse is not true; i.e., if an SNP allele is enriched in African Americans compared to Caucasians, it is not necessarily involved in pigmentation, because a) most alleles in almost all human genes show ethnic frequencies differences, which are sometimes quite large, and most of these human genes have nothing to do with pigmentation; and b) any SNP allele that is involved in human pigmentation must show the relationship within any one ethnic group as well as between ethnic groups; i.e., the validity of an SNP allele as a marker for pigmentation (or any trait) must be based on association between individuals of any one ethnic group as well as individuals between ethnic groups, and using race differences to qualify a SNP allele only addresses the latter.

The results in this Example indicate that the TYRP1_3 SNP and OCA2_5 can have predictive value for human eye color, and that the G allele may be part of a multi-SNP haplotype that is deterministic of, or related to, haplotypes that are deterministic to darker eye color. In addition, the OCA2_5 SNP can have a predictive value for human hair color, and the G allele again can be part of a multi-SNP haplotype that is deterministic of, or related to haplotypes that are deterministic for dark hair color.

## EYE COLOR

No quantitative no qualitative relationship was detected between the zygosity or specific genotype of the TYR_2 SNP (SEQ ID NO:217467) in Caucasians and eye color. The frequency of the G allele was lower in Caucasians than in African Americans or Asians, though the sample size for Asians was low.

With respect to the TYRP1_3 SNP (SEQ ID NO:217485), whereas the ratio of GG, GT and GA genotypes for Caucasians having light eye color was 1:4:4, the ratio for Caucasians having dark eye color is 1:1:1. Further, the ratio of these genotypes in African Americans was 7:2:1, whereas it was 1:2.5:3 in Caucasians, supporting the assertion that the G allele is associated with dark eye color in human beings (since African Americans tend to have darker eye color on average than Caucasians). Furthermore, the ratio in persons of light brown eye color (brown) was lower than the ratio of persons with medium (brown2) or dark (brown3) eye color, thus indicating a potential quantitative relationship among persons of brown eye color. The results for light versus dark eye color were statistically significant. ($p=0.01$). These results indicate that genotype, alone, is useful for explaining some percent of variation in the population of eye color (greater than zero), although it does not explain 100% of the variation. As such, the G allele can be part of a multi-SNP haplotype that is deterministic or related to haplotypes that are deterministic to eye color.

Regarding the OCA2_5 genotype, whereas the ratio of GG:GA:AA genotypes in Caucasians of light (blue, hazel or green) eye color was approximately 0:1:2, the ratio in Caucasians of dark eye color was approximately 0:1:1. Comparing ethnic groups, the ratio of GG:GA:AA genotypes in Caucasians is 0:1:2 and in African Americans, the ratio was approximately 2:1:0, supporting the assertion that the frequency of the G allele is higher in persons of dark eye color than in persons of lighter eye color (again following from the fact that the average African American has darker eye color than the average Caucasian). These results suggest that genotype, alone, cannot explain 100% of the variation in the population of eye color, but that they explain some percent of variation greater than zero, and that the G allele may be part of a multi-SNP haplotype that is deterministic or related to haplotypes that are deterministic to eye color.

Regarding OCA2_6 genotype, no quantitative nor qualitative relationship existed between the zygosity or specific genotype and eye color within the Caucasian ethnic group. The ratio of the GG:GA:AA genotypes was about the same in Caucasians as in African Americans or Asians (though the sample size for Asians is low), supporting the assertion that this SNP is not deterministic for, nor related to haplotypes that are deterministic for human eye color.

## HAIR COLOR

With respect to the TYR_2 genotype, no quantitative or qualitative relationship existed between the zygosity or specific genotype in Caucasians and hair color. The ratio of the GG:GA:AA genotypes in persons of light hair color was 1:1:0, the same as the ratio in persons of dark hair color. Nevertheless, the frequency of the G allele was lower in Caucasians than in African Americans or Asians (though the sample size for Asians is low).

With respect to the TYRP1_3 genotype, whereas the ratio of GG:GT:TT genotypes in Caucasian persons of light (blond, auburn) hair color was approximately 1:1:1, the ratio in Caucasian persons of dark hair color (brown or black) was approximately 1:3:2. However, the ratio of these genotypes in the three ethnic groups does not support the assertion that the G allele is associated with lighter hair color; the frequency of the G allele was lower in Caucasians than African Americans, which contradicts the postulate that the frequency of the G allele is higher in persons of light hair color than in persons of dark hair color.

With respect to the OCA2_5 genotype, whereas the ratio of GG:GA:AA genotypes was 0:0:1 in Caucasian persons of lighter hair color, the ratio in Caucasian persons of darker hair color was 0:1:1, indicating that the frequency of the G allele is higher in Caucasian persons of lighter hair color. Comparing ethnic groups, the ratio of GG:GA:AA genotypes in Caucasians was 0:1:2, and was approximately 2:1:0 in African Americans, supporting the assertion that the frequency of the G allele is higher in persons of dark hair color than in persons of lighter hair color (which follows from the fact that the average African American has darker hair color than the average Caucasian). These results suggest that genotype, alone, cannot explain 100% of the variation in the population of hair color, but that they explain some percent of variation greater than zero; the G allele may be part of a multi-SNP haplotype that is deterministic to, or related to haplotypes that are deterministic for dark hair color.

With respect to the OCA2_6 genotype, no quantitative or qualitative relationship existed between the zygosity or specific genotype and hair color within the Caucasian ethnic group. The ratio of the GG:GA:AA genotypes was about the same in Caucasians as in African Americans or Asians (though the sample size for Asians is low), supporting the assertion that this SNP is not deterministic for, nor related to haplotypes that are deterministic for human eye color.

## SKIN PIGMENTATION

With respect to the TYR_2 genotype, the ratio of the GG:GA:AA genotypes in persons of light skin color was 1:1:0; the same as the ratio in Caucasian persons of medium skin color, though the ratio is higher in Caucasian persons of dark skin color (2:0:0). However, the sample size for Caucasian persons of dark skin color was too low to draw a conclusion from this result. Nevertheless, the frequency of the G allele was lower in Caucasians than in African Americans or Asians (though the sample size for Asians is low), suggesting that this allele can be involved in human skin color, though confirmation of this result must await further results with a larger sample size of Caucasian persons of dark skin color.

With respect to the TYRP1_3 genotype, No statistically significant difference in GG:GT:TT ratios was detected, given the sample size.

With respect to OCA2_5, no statistically significant difference in GG:GA:AA ratios was detected, given the sample size.

With respect to OCA2_5, no statistically significant difference in GG:GA:AA ratios was detected, given the sample size.

**TABLE 1-1**

| TYR 2 | | GG | GA | AA | | | GG | GA | AA |
|---|---|---|---|---|---|---|---|---|---|
| EYE (Caucasians) | BLUE | 8 | 9 | 0 | | CAUC | 69 | 45 | 0 |
| | GREEN | 5 | 5 | 0 | | AFRICAM | 59 | 7 | 0 |
| | HAZEL | 7 | 6 | 0 | | ASIAN | 4 | 0 | 0 |
| | BROWN1 | 2 | 1 | 0 | | | | | |
| | BROWN2 | 2 | 5 | 0 | | | | | |
| | BROWN3 | 1 | 1 | 0 | | | | | |
| | NONBRN | 20 | 20 | 0 | | | | | |
| | BRN | 5 | 7 | 0 | | | | | |
| | | | | | | | | | |
| HAIR(Caucasians) | BLOND | 4 | 4 | 0 | | | | | |
| | AUBURN | 1 | 1 | 0 | | | | | |
| | BROWN | 13 | 17 | 0 | | | | | |
| | BLACK | 1 | 2 | 0 | | | | | |
| | LT | 5 | 5 | 0 | | | | | |
| | DRK | 14 | 19 | 0 | | | | | |

(continued)

| TYR 2 | | GG | GA | AA | | | GG | GA | AA |
|---|---|---|---|---|---|---|---|---|---|
| | | | | | | | | | |
| SKIN(Caucasians) | FAIR | 6 | 10 | 0 | | | | | |
| | MED | 10 | 14 | 0 | | | | | |
| | DRK | 2 | 0 | 0 | | | | | |
| | | | | | | | | | |
| | | | | | | | | | |
| | | | | | | | | | |
| TYRP1 3 GG | | GG | TT | GT | | | GG | GT | TT |
| EYE(Caucasians) | BLUE | 3 | 10 | 9 | | CAUC | 25 | 63 | 72 |
| | GREEN | 2 | 4 | 5 | | AFRICAM | 71 | 19 | 8 |
| | HAZEL | 1 | 9 | 9 | | ASIAN | 28 | 0 | 0 |
| | BROWN1 | 0 | 3 | 0 | | | | | |
| | BROWN2 | 4 | 2 | 5 | | | | | |
| | BROWN3 | 1 | 2 | 0 | | | | | |
| | NONBRN | 6 | 23 | 23 | | | | | |
| | BRN | 5 | 4 | 5 | | | | | |
| | | | | | | | | | |
| HAIR(Caucasians) | BLOND | 3 | 3 | 2 | | | | | |
| | AUBURN | 0 | 1 | 1 | | | | | |
| | BROWN | 7 | 16 | 12 | | | | | |
| | BLACK | 0 | 2 | 1 | | | | | |
| | LT | 3 | 4 | 3 | | | | | |
| | DRK | 7 | 18 | 13 | | | | | |
| SKIN(Caucasians) | FAIR | 3 | 9 | 7 | | | | | |
| | MED | 6 | 12 | 9 | | | | | |
| | DRK | 1 | 0 | 1 | | | | | |
| | | | | | | | | | |
| OCA2_5 | | GG | GA | AA | | | GG | GA | AA |
| EYE(Caucasians) | BLUE | 0 | 9 | 16 | | CAUC | 9 | 58 | 106 |
| | GREEN | 0 | 2 | 8 | | AFRICAM | 61 | 26 | 8 |
| | HAZEL | 1 | 7 | 15 | | ASIAN | 14 | 47 | 58 |
| | BROWN1 | 0 | 3 | 3 | | | | | |
| | BROWN2 | 0 | 2 | 2 | | | | | |
| | BROWN3 | 0 | 3 | 6 | | | | | |
| | NONBRN | 1 | 18 | 39 | | | | | |
| | BRN | 0 | 10 | 12 | | | | | |

(continued)

| OCA2_5 | | GG | GA | AA | | | GG | GA | AA |
|---|---|---|---|---|---|---|---|---|---|
| | | | | | | | | | |
| HAIR(Caucasians) | BLOND | 0 | 1 | 9 | | | | | |
| | AUBURN | 0 | 0 | 3 | | | | | |
| | BROWN | 0 | 17 | 19 | | | | | |
| | BLACK | 0 | 2 | 1 | | | | | |
| | LT | 0 | 1 | 12 | | | | | |
| | DRK | 0 | 19 | 20 | | | | | |
| | | | | | | | | | |
| SKIN(Caucasians) | FAIR | 0 | 6 | 15 | | | | | |
| | MED | 0 | 11 | 17 | | | | | |
| | DRK | 0 | 1 | 0 | | | | | |
| | | | | | | | | | |
| OCA2_6 | | GG | GA | AA | | | GG | GA | AA |
| EYE(Caucasians) | BLUE | 22 | 3 | 0 | | CAUC | 151 | 26 | 0 |
| | GREEN | 11 | 0 | 0 | | AFRICAM | 92 | 3 | 0 |
| | HAZEL | 22 | 4 | 0 | | ASIAN | 103 | 17 | 0 |
| | BROWN1 | 3 | 1 | 0 | | | | | |
| | BROWN2 | 8 | 1 | 0 | | | | | |
| | BROWN3 | 3 | 0 | 0 | | | | | |
| | NONBRN | 55 | 7 | 0 | | | | | |
| | BRN | 20 | 4 | 0 | | | | | |
| | | | | | | | | | |
| HAIR(Caucasians) | BLOND | 11 | 0 | 0 | | | | | |
| | AUBURN | 3 | 0 | 0 | | | | | |
| | BROWN | 32 | 5 | 0 | | | | | |
| | BLACK | 2 | 1 | 0 | | | | | |
| | LT | 14 | 0 | 0 | | | | | |
| | DRK | 34 | 6 | 0 | | | | | |
| | | | | | | | | | |
| SKIN(Caucasians) | FAIR | 20 | 2 | 0 | | | | | |
| | MED | 25 | 3 | 0 | | | | | |
| | DRK | 2 | 0 | 0 | | | | | |

Brown is light brown eye color;
Brown 2 is medium brown; and
Brown 3 is dark brown.
All phenotype data(color) is self-reported by blood donor subjects on a questionnaire filled out at the time of blood donation.

**EXAMPLE 2**

**OCA2 8 POLYMORPHISM**

This example describes an additional OCA polymorphism, thus confirming and extending the results disclosed in Example 1. Methods for detecting the nucleotide occurrence at a SNP position are described in Example 1.

Further analysis of the OCA2 gene also identified another marker, OCA2_8, which is associated with the degree to which human eyes and hair are pigmented. The OCA2_8 polymorphism is a Y (T or C) change and is present at position 86326 within the GenBank Accession No. 13651545 genomic sequence file (see Table 1 for information regarding OCA2_8 as well as all of the SNP markers disclosed herein).

With respect to OCA2_8, the counts for Caucasian persons of various eye, hair and skin color are shown in Table 2-1. The number of CC and CT genotypes, relative to TT genotypes, was greater in persons of darker eye and hair color than in persons of darker hair color, demonstrating that the frequency of the C allele was greater in persons of darker hair and eye color than in persons of lighter hair and eye color. Since these results were from Caucasians, if the C allele at this locus is associated with eye pigmentation, it was expected to be enriched in racial groups that tend to show darker pigmentation than Caucasians. The data for the ethnic groups showed that, indeed, the frequency of the C allele was significantly higher in African American and Asian persons than in Caucasians (Table 2-1). These results seemed to confirm that the C allele at this locus is predictive for human eye and hair color. Although the results for skin color were inconclusive due to the low sample size, there appeared to be a similar, though less impressive, trend. In addition to the OCA2_8 locus, two other markers in the OCA2 gene showed a similar trend, OCA2_5, which, as disclosed in Example 1, showed strong predictive value for eye/hair pigmentation, and OCA2_6, which showed a weaker predictive value.

Haplotype analysis was performed involving three potentially valuable markers in the OCA2 gene - OCA2_5, OCA2_6, and OCA2-8. The haplotypes of the subjects were documented with respect to the three markers (e.g., ATG/CTA or GTT/AGA; see Table 2-2), where the sequence on the top of the line represents the combination of polymorphic alleles on the maternal chromosome and the other, the paternal (or vice versa). Haplotypes are strings of polymorphic alleles, much like a string of contiguous sequence bases, except they are not adjacent to one another on a chromosome. In fact, OCA2_5 and OCA2_8 are about 60,000 base pairs apart from one another. It is beneficial to express polymorphisms in terms of multi-locus haplotypes because far fewer haplotypes exist in the world population than would be predicted based on the expectations from random allele combinations. For example, for the three disclosed polymorphic loci within this gene, (G/A), (T/C) and (G/A), there would be $2^3 = 8$ possible haplotype combinations observed in the population - ATG, ACG, GCG, GTG, ACA, GCA, ATA and GTA. These can be considered possible or potential "flavors" of the OCA2 gene in the population. However, only four haplotypes or "flavors" have been observed in the real data from peoples of the world. For larger numbers of polymorphic loci the disparity between the number of observed and expected haplotypes is larger. This well known phenomenon is caused by systematic genetic forces such as population bottlenecks, random genetic drift, selection, and the like, which have been at work in the population for millions of years, and have created a great deal of genetic "pattern" in the present population. As a result, working in terms of haplotypes offers a geneticist greater statistical power to detect associations, and other genetic phenomena, than working in terms of disjointed genotypes.

OCA2_5 - OCA2_6 - OCA2_8 haplotype counts for patients, counted with respect to hair color are shown in Table 2-2. Similar results were obtained when counted with respect to eye color. Though OCA2_6 only showed weak association, it was included in this analysis because its value as part of the haplotype is greater than its value on its own. (The same is true for the other two markers).

From this data, it is clear that the ATG haplotype was the most frequent haplotype, and was disproportionately present in persons of lighter hair color. Haplotypes other than ATG (such as ACG, GCG and GCA) tended to occur in the DNA of persons of darker hair color. Another way to look at this data is to look at haplotype pairs, or compound genotypes (see Table 2-3). This view of the data, which is the most biologically relevant view, shows that persons of lighter hair color (blond and red) are almost always ATG/ATG, whereas persons of darker hair color are more likely to be of another combination including ATG and some other haplotype (see, also; Table 2-3).

These results demonstrate that persons of light hair color (red or blond) are almost always ATG/ATG genotypes (12 out of 15 cases). In contrast, persons of dark hair color usually harbor an ATG haplotype in combination with some other haplotype (26 out of 40 cases). A specimen of one ATG haplotype in combination with some other haplotype (ATG/OTHER), is almost always a person of darker hair color. A person of two ATG haplotypes (ATG/ATG) could be either a person of light hair color or a person of dark hair color, but is more likely to be a person of light hair color.

These results also demonstrate that the OCA2_5 - OCA2_6 - OCA2_8 multilocus genotype of a person provides a predictive value for their hair (and eye) color. The certainty of assignment of an unknown human specimen to the dark or light hair color class, using their compound genotype (haplotype pair) for these three loci can be calculated using well known statistical methods.

**TABLE 2-1**

| OCA2_8 | | TT | CT | CC | Ethnic Group | TT | CT | CC |
|---|---|---|---|---|---|---|---|---|
| EYE | BLUE | 14 | 9 | 2 | CAUC | 39 | 42 | 14 |
| | GREEN | 7 | 3 | 0 | AFRICAM | 11 | 31 | 56 |
| | HAZEL | 11 | 9 | 3 | ASIAN | 1 | 7 | 11 |
| | BROWN | 7 | 11 | 7 | | | | |
| | B/G (LIGHTER) | 21 | 12 | 2 | | | | |
| | H/BR (DARKER) | 18 | 20 | 4 | | | | |
| | | | | | | | | |
| HAIR | BLOND | 8 | 3 | 0 | | | | |
| | RED/AUBURN | 4 | 0 | 0 | | | | |
| | BROWN | 12 | 15 | 3 | | | | |
| | BLACK | 1 | 2 | 0 | | | | |
| | BL/RD (LIGHT) | 12 | 3 | 0 | | | | |
| | BR/BL (DARK) | 13 | 17 | 3 | | | | |
| | | | | | | | | |
| SKIN | FAIR | 13 | 8 | 1 | | | | |
| | MED | 10 | 11 | 2 | | | | |
| | DRK | 0 | 1 | 0 | | | | |

**TABLE 2-2**

| | | OCA2_5 | OCA2_8 | OCA2_6 | HAPLO-TYPES | | | |
|---|---|---|---|---|---|---|---|---|
| | | | | | | | | |
| HAIR | ATG | ACG | GCG | GTG | ACA | GCA | ATA | GTA |
| BLOND | 19 | 2 | 1 | 0 | 0 | 0 | 0 | 0 |
| RED | 8 | 0 | 0 | 0 | 0 | 0 | 0 | 0 |
| BROWN | 39 | 8 | 12 | 0 | 0 | 4 | 0 | 0 |
| BLACK | 4 | 0 | 1 | 0 | 0 | 1 | 0 | 0 |
| LIGHT (BL+RD) | 27 | 2 | 1 | 0 | 0 | 0 | 0 | 0 |
| DARK (BRN + BLK) | 43 | 8 | 13 | 0 | 0 | 5 | 0 | 0 |

**TABLE 2-3**

| | ATG/ ATG | ATG/ GCG | ATG/ ACG | ACG/ ACG | GCA/ ATG | GCA/ ACG | ACG/ ATG |
|---|---|---|---|---|---|---|---|
| | | | | | | | |
| BLOND | 8 | 1 | 0 | 0 | 0 | 0 | 2 |
| RED | 4 | 0 | 0 | 0 | 0 | 0 | 0 |
| BROWN | 13 | 11 | 4 | 1 | 3 | 1 | 4 |
| BLACK | 1 | 1 | 0 | 0 | 1 | 0 | 0 |

(continued)

| | ATG/ ATG | ATG/ GCG | ATG/ ACG | ACG/ ACG | GCA/ ATG | GCA/ ACG | ACG/ ATG |
|---|---|---|---|---|---|---|---|
| LIGHT | 12 | 1 | 0 | 0 | 0 | 0 | 2 |
| DARK | 14 | 12 | 4 | 1 | 4 | 1 | 4 |

**TABLE 2-4**

| | Two copies of ATG | One copy of ATG | No copies of ATG |
|---|---|---|---|
| | ATG/ATG | ATG/OTHER | OTHER/OTHER |
| LIGHT | 12 | 3 | 0 |
| DARK | 14 | 20 | 6 |

**EXAMPLE 3**

**IDENTIFICATION OF TYROSINASE (TYR) GENE POLYMORPHISM ASSOCIATED WITH PIGMENTATION**

This example demonstrates that a SNP in a third gene, encoding tyrosinase, is associated with pigmentation in humans. Methods for detecting the nucleotide occurrence at a SNP position are described in Example 1.

A SNP, designated TYR_3, that was associated with pigmentation was identified in the tyrosinase gene. The TYR_3 SNP is shown in Table 1. The gene, the polymorphism name, its location, and the reference sequence identifier (NCBI:Genbank) are indicated in Table 1. In addition, the variant IUB code, its source of discovery, and the type of polymorphisms (a scrine to a tyrosine amino acid change in the coding amino acid sequence of the expression product, are also shown; "Poly" indicates that it was verified as a polymorphic position).

TYR_3 is one of the SNPs disclosed herein as being associated with the degree to which human tissues are pigmented. Of a very large number of different genes, the TYR gene is the third gene found to harbor SNPs so associated. Each of the three genes, OCA2, TYRP 1 and, now, TYR, was discovered based on the observation that loss-of-function mutants in mice and humans exhibited a condition called oculocutaneous albinism. Individuals afflicted with this disease lack any pigment in their skin, hair or eyes, and arc victims of numerous physiological and social challenges. Oculocutaneous mutants arc quite rare in the human population and, until now, it was not known whether or how natural polymorphic variants in these genes were related to the normal variation in human skin, eye and hair color exhibited by the various peoples of the world.

The TYR_3 SNP, which is the first SNP found in the tyrosinase gene to be associated with human pigmentation, is a C to an A change (IUB symbol = M) at nucleotide position 657 in the NCBI reference sequence accession number NM000372. The TYR_3 polymorphism also is present in the publicly available NCBI SNP database (dbSNP), but it was not previously associated with the degree to which human tissues are pigmented.

TYR_3 is a unique polymorphism that meets the requirements for a SNP associated with pigmentation as disclosed herein. The data showing the association, as well as an interpretation of the data, are presented in Table 3-1 and Table 3-2. The presented results are statistically significant for hair color.

## Hair Color

The ratio of CC:CA:AA genotypes in persons of dark hair (black or brown) was 24:14:3, and in persons of light colored hair was 1:5:3. These ratios are sufficiently different from one another to conclude that the frequency of the A allele at the TYR_3 locus was significantly higher in persons of light colored hair. For example, the frequency of the C allele in persons with dark hair color was (24+(0.5)(14))/41=0.75, whereas the frequency of the C allele in persons of lighter hair color was (1+(0.5)(5))/9=0.39; the values, 0.75 and 0.39, are quite distinct.

## Eve Color

Although the results are provocative for eye color, they are not conclusive. The ratio of CC:CA:AA genotypes in persons of dark eye color was 27:12:5, and the ratio in persons of light hair color was 12:20:4, which is significantly distinct. Nonetheless, the number of AA genotypes in the two classes of individuals was not significantly different (5 for dark, 4 for light). If the C allele was associated with darker eye color, as is indicated by the number of relative homozygous CC to heterozygous CA genotypes between these two goups, the number of AA homozygotes of lighter eye color would exceed that of darker eye color. However, this was not the case, and as a result, the results are less impressive (though not negative) for eye color.

**Skin Color** In comparing persons of fair and medium skin tone, there were no obvious differences in the ratio of CC:CA:AA genotypes. The frequency of the C allele in persons of dark skin tone may have been greater than in persons of light or medium skin tone, however the sample size was not adequate to draw a conclusion.

**Ethnic Differences**

If the C allele is associated with darker hair color, and functionally related to the degree to which humans in the world are pigmented, as indicated by the data, the C allele should be enriched in persons of average darker hair, eye and skin color. African Americans are one such group. The ratio of CC:CA:AA genotypes in randomly selected African Americans was 84:13:1, and the ratio in randomly selected Caucasians (a distinct population from that for which eye, hair and skin pigmentation results are presented above) was 37:49:13 (Table 3-2). Indeed, the frequency of the C allele at this polymorphic locus was enriched in persons of darker average eye, hair and skin color (African Americans), extending the results observed within the Caucasian group, and supporting the assertion that the C allele was associated with darker hair color in human beings. No polymorphism has been found to be apparently associated with darker eye, hair, or skin color that was not also enriched in ethnic groups of average darker eye, hair or skin color.

**TABLE 3-1**

| TYR 3 | DNAPRINT SNP NUMBER 217468 | | |
|---|---|---|---|
| EYE (Caucasians) | CC | CA | AA |
| BROWN | 10 | 8 | 3 |
| HAZEL | 17 | 4 | 2 |
| GREEN | 2 | 8 | 1 |
| BLUE | 10 | 12 | 3 |
| | | | |
| HAIR(Caucasians) | CC | CA | AA |
| BLACK | 3 | 0 | 0 |
| BROWN | 21 | 14 | 0 |
| RED/AUBURN | 0 | 3 | 0 |
| BLOND | 1 | 5 | 3 |
| | | | |
| SKIN(Caucasians) | CC' | CA | AA |
| FAIR | 9 | 9 | 2 |
| MEDIUM | 12 | 12 | 4 |
| DARK | 2 | 0 | 0 |

**TABLE3-2**

| | CC | CA | AA |
|---|---|---|---|
| Caucasian | 37 | 49 | 13 |
| African American | 84 | 13 | 1 |

**EXAMPLE 4**

**IDENTIFICATION OF POLYMORPHISMS ASSOCIATED WITH PIGMENTATION**

The study sample consisted of several hundred patients exhibiting variable eye, skin and skin pigmentation levels (colors). Subjects provided a blood sample after providing informed consent and completing a biographical questionnaire. Samples were processed immediately into DNA, which will be stored at -80 degrees for the duration of the study. Samples were used only as per the study design and project protocol. Biographical data was entered into an Oracle relational database system run on a Sun Enterprise 420R server.

Gene markers were selected based on evidence from the body of literature, and from other sources of information, that implicate them in either the synthesis, degradation and/or the deposition of the human chromatophore melanin. The Physicians Desk Reference, Online Mendelian Inheritance database (NCBI) and PubMed/Medline are two examples for sources of this type of information.

Candidate SNPs were discovered from marker genes ("data mining") using, for example, the NCBI SNP database or the Human Genome Unique Gene database (Unigene; NCBI). Sequence files for the genes were downloaded from proprietary and public databases and saved as a text file in FASTA format and analyzed using a multiple sequence alignment tool. The text file that was obtained from this analysis served as the input for a SNP/HAPLOTYPE automated pipeline discovery software system. This system finds candidate SNPs among the sequences, and documents haplotypes for the sequences with respect to these SNPs. The software uses a variety of quality control metrics when selecting candidate SNPs including the use of user specified stringency variables; the use of PHRED quality control scores and others (*See* U.S. Pat. App. No. Serial No.: 09/964,059, filed September 26, 2001).

Assays using SNP-specific kits were performed using an Orchid SNPstream 25K instrument for high throughput genotyping (Orchid BioSciences, Inc., Princeton, NJ). This instrument, which is based on Beckman-Coulter robotics and operates as a completely automated platform, carrying out the entire process from DNA specimen to called allele, can read 25,000 genotypes in a day. An automated ABI310 and an ABI3700 capillary electrophoresis genetic analyzer are used for SNP discovery. Amplification reactions are set up using a Beckman Automated liquid handling system, and amplified in an MJ research Thermal Cyclers or using a PE Applied Biosystems 9700 thermal cycler. Data analysis is performed using a SUN Enterprise 460 Unix server, which includes 6 PC terminals networked with the server.

The public genome database was constructed from donors for which eye, skin and hair color information is absent. Further, it was constructed from only 5 donors. In order to discover new SNPs that may be under-represented or biased against in the public human SNP and Unigene databases, a larger pool (n=500) of DNA specimens obtained from the Cornell Institute were seeded with certain of the specimens collected using the disclosed methods. Specimens from this combined pool were used as a template for amplification using a combination of Pfu turbo thermostable DNA polymerase and Taq polymerase. Amplification was performed in the presence of 1.5 mM $MgCl_2$, 5 mM KCl, 1 mM Tris, pH 9.0, and 0.1% Triton X-100 nonionic detergent. Amplification products were cloned into a T-vector using the Clontech (Palo Alto CA) PCR Cloning Kit, transformed into Calcium Chloride Competent cells (Stratagcne; La Jolla CA), plated on LB-ampicillin plates, and grown overnight.

Clones were selected from each plate, isolated by mini-prep using the Promega Wizard or Qiagcn Plasmid Purification Kit, and sequenced using standard PE Applied Biosystems Big Dye Terminator Sequencing Chemistry. Sequences were trimmed of vector sequence and quality trimmed, and deposited into an Internet based relational database system.

Genotypes were surveyed within the specimen cohorts by sequencing using Klenow fragment-based single base primer extension and an automated Orchid Biosciences SNPstream instrument (Orchid BioSciences, Inc., Princeton, NJ). Orchid technology is based on dye-linked immunochemical recognition of base incorporated during extension. Reactions are processed in 384 well format and stored into a temporary database application until transferred to the UNIX based SQL database.

The data produced corresponds to SNPs that are informative for distinguishing common genetic haplotypes identified from public and private databases. Using algorithms to infer haplotypes as described in the detail description section *(See* U.S. Pat. App. No. Serial No.: 09/964,059, filed September 26, 2001) the data was be used to infer haplotypes from genotype data corresponding to these SNPs. In addition to this, raw genotypes were considered empirically, without respect to predefined haplotypes.

Allele frequencies were calculated and pair-wise haplotype frequencies estimated using an EM algorithm (Excoffier and Slatkin 1995). Linkage disequilibrium coefficients was then calculated. The analytical approach was always based on the case-control study design. Genotype/biographical data matrices for both groups, for example, dark versus light eye color, were used for a pattern detection algorithm such as the SNiPDOCS$^{SM}$ algorithm (See U.S. Pat. App. No. Serial No.: 09/964,059, filed September 26, 2001). The purpose of these algorithms is to fit quantitative (or Mendelian) genetic data with continuous trait distributions (or discrete, as the case may be). In addition to various parameters such as linkage disequilibrium coefficients, allele and haplotype frequencies (within ethnic, control and case groups), chi-square statistics and other population genetic parameters such as Panmitic indices were calculated to control for ethnic, ancestral or other systematic variation between the case and control groups. Markers/haplotypes with value for distinguishing the case matrix from the control, if any, were presented in mathematical form describing any relationship and accompanied by association (test and effect) statistics.

## EXAMPLE 5

## SINGLE NUCLEOTIDE POLYMORPHISMS PREDICTIVE OF RETINA PIGMENTATION AND HAIR PIGMENTATION

This example identifies SNPs with predictive value for the degree of iris or hair pigmentation, or both, in humans. The following results were obtained for the disclosed SNPs from Caucasians of various eye and hair colors. All phenotype data (color) is self-reported by blood donor subjects on a questionnaire filled out at the time of blood donation.

In Table 5-1, below, "DARK" for eyes means brown and hazel; "LIGHT" for eyes means blue and green. "DARK" for hair means black and brown; "LIGHT" for hair means blond and red/aubum. Methods for detecting the nucleotide occurrence at a SNP position are described in Example 4.

The results shown below are segregated based on pigmentation of each group of individuals. In the following results, eye color is synonymous with the degree to which the retina is pigmented. The same is true for skin pigmentation and hair color. Numerous studies have shown that the variation in human skin, eye and hair color is caused by variation in the degree to which melanin is deposited in the appropriate tissues during development, which in turn is a function of the degree to which melanin is synthesized and degraded. Until now, it has not been known which, or whether, polymorphic variation in the melanin synthesis genes determines natural variation in human eye and hair color.

### Results for each SNP surveyed in these experiments

### EYE COLOR:

OCA2DBSNP 52401: The association of this marker with eye color can be seen by comparing the brown versus non-brown groups. Whereas the brown group shows an AA:GA:GG genotype ratio of 14:14:1, the non-brown group shows a 53:25:2 ratio. Thus, the ratio of the brown group reduces to a 1:1:0 ratio, that of the non-brown group reduces to an approximate 2:1:0 ratio and the AA genotype is twice as common in persons of an eye color other than brown. The results comparing dark versus light eye color for this marker do not appear to be as strong. This may be because the AA genotype is carried more frequently in persons of hazel versus brown eye color, and looking at the ratios for the specific eye colors supports this idea. Thus the frequency of the A allele is greater in persons of lighter or non-brow eye color.

QCA1DBSNP 165011: The association of this marker with eye color can be seen by comparing the dark (brown plus hazel) versus light (green plus blue) groups. The ratio of AA:GA:GG genotypes for the dark eye group is 34:17:1, but is higher in the light eye group - 42:10:0. This reduces to an approximate ratio of 2:1:0 for dark and 4: 1:0 for light. The ratio of brown versus non brown arc similar - 20:9:0 for brown versus 56:18:1 for non brown. This reduces to 2:1: 0 for brown and 3:1:0 for non-brown. Thus, the frequency of the A allele is higher in persons of lighter or non-brown eye color.

OCA2DBSNP 146405: The association of this marker with eye color can be seen by comparing the dark (brown plus hazel) versus light (green plus blue) groups. The ratio of AA:GA:GG genotypes for the dark eye group is 24:16:9 but only 16:29:6 for the light eye group. This reduces to an approximate ratio of 3:2:1 for dark and 2:3:1 for light. The ratio of brown versus non brown are less compelling. In total, the frequency of the A allele is higher in persons of darker or brownish eye color, and may be especially predictive of the HAZEL group.

OCA2DBSNP 8321: The association of this marker with eye color can be seen by comparing the dark (brown plus hazel) versus light (green plus blue) groups. The ratio of GG:G:TT genotypes for the dark eye group is 32:20:2 but 44:11:0 for the light eye group. This reduces to an approximate ratio of 1.5:1:0 for dark and 4:0:0 which is significantly different. The ratio of brown versus non brown are less compelling. In total, the frequency of the G allele is higher in persons of lighter or bluish/green eye color.

**PIGMENT:** None of the markers appeared to be predictive for the darkness of Caucasian skin color.

### HAIR COLOR:

OCA2DBSNP 52401: The association of the G allele with lighter hair color can be seen by comparing the ratios of blond persons versus persons of non-blond colored hair. The ratio of persons of blond hair is 8:6:0 versus a ratio of 42:23:2 for persons of non-blond hair. This reduces to an approximate ratio of 1:1:0 for blonds and 2:1:0 for non-blonds. Thus the frequency of the G allele is greater by two-fold in persons of blond hair versus persons of non-blond hair color.

OCA2DBSNP 165011: The association of the A allele with darker hair color can be seen by comparing the ratios of blond persons versus persons of non-blond colored hair. The ratio of persons of blond hair is 8:4:0 versus a ratio of 55:9:1 for persons of non-blond hair. This reduces to an approximate ratio of 2:1:0 for blonds and 5:1:0 for non-blonds. The results for persons of dark versus light hair color are similar in ratios.

Thus the frequency of the A allele is greater by 2.5-fold in persons of blond hair versus persons of non-blond hair color.

OCA2DBSNP 146405: The association of the G allele with lighter hair color can be seen by comparing the ratios of blond persons versus persons of non-blond colored hair as well as the ratio of persons of dark versus light hair color. The ratio of persons of blond hair is 0:6:6 versus a ratio of 29:28:8 for persons of non-blond hair. This reduces to an approximate ratio of 0:6:6 for blonds and 4:4:1 for non-blonds. The results for persons of dark versus light hair color are similar in ratios. Dark hair persons show a 26:26:8 ratio but persons of lighter hair color show a ratio of 3:8:6 reducing to 4:4:1 and 1:2:2 respectively. These ratios are dramatically different. Thus the frequency of the G allele is greater in persons of blond or light hair versus persons of non-blond or dark hair color.

OCA2DBSNP 8321: The sample size for the comparison of persons of lighter colored hair versus persons of darker colored hair is not adequate in this particular experiment. These results demonstrate that each of the SNPs described above has predictive value for the degree of retina or hair pigmentation, or both, in humans.

**TABLE 5-1**

| **OCA2DBSNP 52401** | | AA | GA | GG |
|---|---|---|---|---|
| EYE (Caucasians) | BLUE | 26 | 12 | 2 |
| | GREEN | 11 | 5 | 0 |
| | HAZEL | 16 | 8 | 1 |
| | BROWN | 14 | 14 | 1 |
| | | | | |
| | DARK | 30 | 22 | 2 |
| | LIGHT | 37 | 17 | 2 |
| | | | | |
| | BROWN | 14 | 14 | 1 |
| | NON-BROWN | 53 | 25 | 2 |
| | | | | |
| HAIR(Caucasians) | BLOND | 8 | 6 | 0 |
| | RED/AUBURN | 3 | 3 | 0 |
| | BROWN | 37 | 19 | 2 |
| | BLACK | 2 | 1 | 0 |
| | | | | |
| | LT | 11 | 9 | 0 |
| | DRK | 39 | 20 | 2 |
| | | | | |
| | BLOND | 8 | 6 | 0 |
| | NONBLOND | 42 | 23 | 2 |
| | | | | |
| SKIN (Caucasians) | FAIR | 23 | 11 | 1 |
| | MED | 24 | 18 | 0 |
| | DRK | 1 | 0 | 0 |
| | | | | |
| **OCA2DBSNP_165011** | | AA | GA | GG |
| EYE(Caucasians) | BLUE | 29 | 9 | 0 |
| | GREEN | 13 | 1 | 0 |
| | HAZEL | 14 | 8 | 1 |

(continued)

| **OCA2DBSNP 52401** | | AA | GA | GG |
|---|---|---|---|---|
| | BROWN | 20 | 9 | 0 |
| | | | | |
| | NONBRN | 56 | 18 | 1 |
| | BRN | 20 | 9 | 0 |
| | | | | |
| | DARK | 34 | 17 | 1 |
| | LIGHT | 42 | 10 | 0 |
| | | | | |
| HAIR (Caucasians) | BLOND | 8 | 4 | 0 |
| | RED/AUBURN | 5 | 1 | 0 |
| | BROWN | 47 | 8 | 1 |
| | BLACK | 3 | 0 | 0 |
| | | | | |
| HAIR (Caucasians) | BLOND | 8 | 4 | 0 |
| | RED/AUBURN | 5 | 1 | 0 |
| | BROWN | 47 | 8 | 1 |
| | BLACK | 3 | 0 | 0 |
| | | | | |
| | LT | 3 | 3 | 3 |
| | DRK | 7 | 18 | 13 |
| | | | | |
| | NON BLOND | 55 | 9 | 1 |
| | BLOND | 8 | 4 | 0 |
| | | | | |
| SKIN (Caucasians) | FAIR | 24 | 8 | 1 |
| | MED | 37 | 5 | 0 |
| | DRK | 1 | 0 | 0 |
| | | | | |
| **OCA2DBSNP_146405** | | AA | GA | GG |
| EYE (Caucasians) | BLUE | 13 | 20 | 2 |
| | GREEN | 3 | 9 | 4 |
| | HAZEL | 13 | 5 | 4 |
| | BROWN1 | 11 | 11 | 5 |
| | | | | |
| | NONBRN | 11 | 11 | 5 |
| | BRN | 29 | 34 | 6 |
| | | | | |
| | DARK | 24 | 16 | 9 |

(continued)

| **OCA2DBSNP 52401** | | AA | GA | GG |
|---|---|---|---|---|
| | LIGHT | 16 | 29 | 6 |
| | | | | |
| | BROWN | 11 | 11 | 5 |
| | NON BROWN | 29 | 34 | 6 |
| | | | | |
| HAIR (Caucasians) | BLOND | 0 | 6 | 6 |
| | RED/AUBURN | 3 | 2 | 0 |
| | BROWN | 25 | 25 | 7 |
| | BLACK | 1 | 1 | 1 |
| | | | | |
| | LT | 3 | 8 | 6 |
| | DRK | 26 | 19 | 20 |
| | | | | |
| | NON BLOND | 29 | 28 | 8 |
| | BLOND | 0 | 6 | 6 |
| | | | | |
| SKIN (Caucasians) | FAIR | 12 | 14 | 6 |
| | MED | 15 | 19 | 0 |
| | DRK | 0 | 1 | 0 |
| | | | | |
| **OCA2DBSNP_8321** | | GG | GT | TT |
| EYE (Caucasians) | BLUE | 31 | 9 | 0 |
| | GREEN | 13 | 3 | 0 |
| | HAZEL | 15 | 10 | 0 |
| | BROWN | 17 | 10 | 2 |
| | | | | |
| | NONBRN | 59 | 22 | 0 |
| | BRN | 17 | 10 | 2 |
| | | | | |
| | LIGHT | 44 | 11 | 0 |
| | DARK | 32 | 20 | 2 |
| | | | | |
| HAIR (Caucasians) | BLOND | 8 | 6 | 0 |
| | RED/AUBURN | 5 | 1 | 0 |
| | BROWN | 40 | 17 | 1 |
| | BLACK | 3 | 0 | 0 |
| | | | | |
| | LT | 13 | 7 | 0 |

(continued)

| OCA2DBSNP 52401 | | AA | GA | GG |
|---|---|---|---|---|
| | DRK | 43 | 17 | 1 |
| | | | | |
| | NON BLOND | 48 | 18 | 1 |
| | BLOND | 8 | 6 | |
| | | | | |
| SKIN (Caucasians) | FAIR | 23 | 12 | 0 |
| | MED | 29 | 13 | 1 |
| | DRK | 1 | 0 | 0 |

## EXAMPLE 6

## METHOD FOR RELATING OCA2 GENE VARIANTS TO HUMAN EYE AND HAIR COLOR: SNP ANALYSIS IN THE CONTEXT OF THE HAPLOTYPE

The results in this Example provides a general method for qualifying a genetic association between a haplotype and a phenotype. Methods for detecting the nucleotide occurrence at a SNP position are described in Example 4.

The results described below demonstrate that the OCA2 SNPs disclosed herein are intimately involved in the degree to which human eye and hair is pigmented. The method relies on the generally known principle that haplotypes observed in the human population can be expressed in a cladogram or a parsimony tree such that the evolutionary relationships between the haplotypes are discernable. In such a cladogram, haplotypes derived from common haplotype ancestors will be present in similar regions of the tree. Furthermore, haplotypes that arc similar in sequence content will be more closely proximated in the tree to one another than to dissimilar haplotypes. One such tree is shown in FIG. 1, where lines separate haplotypes that are one mutational step from another and biallelic positions within a gene are represented in binary form (1 and 0):

The present method is based on the fact that this type of haplotype tree can be used as the starting point for a novel method of drawing associations between gene variants and physical traits in the human population because haplotypes that arc similar to one another in sequence content are more likely to share common, or similar phenotypic values than randomly selected haplotypes. Thus, haplotypes residing at similar regions of a cladogram or tree will tend to share common phenotypic attributes. For example, the biological effect of haplotype 00100001 at the lower right hand side of the cladogram in the above figure is more likely to be similar to that of 00110000 next to it in the cladogram than to 100010000 at the upper left hand side of the cladogram. This assumption is reasonable since haplotypes situated in proximity to one another share more sequence in common than randomly selected haplotypes, and it is the sequence of a gene that largely determines its function. As such, haplotype analysis using the cladogram provides a useful means for representing genetic data in such a way as to .facilitate multivariate analyses for the determination of the biological relevance of the haplotype.

The two main features of the presently disclosed approach are that a simple haplotype encoding scheme can be used to graphically project haplotypes in a manner that is sensitive to their position in the haplotype cladogram, and therefore their interrelations (see below); and that both haplotypes present in an individual are encoded, and the diploid combinations of haplotypes are actually plotted. When the analysis is performed in this manner for many individuals, and plotted (in the case of a univariate or bivariate analysis), patterns are easily recognized (or not recognized, depending on the experiment).

Each diploid pair of haplotypes was projected in n-dimensional space, in such a manner as to be true to the relative position of the haplotypes in the cladogram or tree. Thus, vectors for two individuals with "similar" haplotype combinations are closer to one another in the plot than to others that have a dissimilar haplotype combination (just like in the cladogram). The method can be used to plot n-dimensional vectors for individuals of various haplotype combinations, in n-dimensional feature space. Plots in n-dimensional feature space allow for the recognition of complex genetic pattern that results from dominance effects, additivity or other complex or quantitative genetic phenomena such epistatic effects. This method of genetic data representation offers a new power to detect and quantify the degree to which haplotypes determine various human traits because it allows data traditionally considered in discrete, discontinuously distributed terms, to be considered in a more useful continuous format.

The method used to encode the haplotypes for plotting was as follows: The haploids are represented as points in a multidimensional haploid space. For example, an 8 locus haplotype can be plotted in an 8 dimensional haploid space of ($4^8$) possible locations. A heterozygote pair of haplotypes can be represented by a line joining the two points. In the case of homozygotes, a loop is formed to join the point with itself. To represent the association between haplotype and phenotype, or genotype and phenotype, for characters like eye color or hair color, the line representing the corresponding haplotypes in a pair is colored for visual ease, or assigned a value for computational convenience. This analysis helps reveal the relationship between haplotype and phenotypes. For interpretation, or to visualize a complex multidimensional plot, the dimension of the plot can be reduced by considering a variety of mathematical methods. Doing this, the multidimensional plot can be projected into a two or three dimensional real space ($R^2$ or $R^3$), for making relationships visible.

The value in the method is its ability to express discrete genetics combinations in terms of a continuum of values. Though it is counter-intuitive to considering genetic values such as genotypes or haplotypes in terms of continuous distributions (after all, genes are discrete entities), there is value in doing so. This can be appreciated when one considers that it is often times difficult to produce data that is representative of all the world's population. It is not practical, nor feasible to sequence every person in the world. Genetic data sets are therefore samples of the larger world populations, and parameters derived from these data are estimates of true parameter values. Because it is not practical to generate genetic data sets completely representative of the world's peoples, classifying individuals based on estimates of genetic parameters or features is a common problem with genetic studies. For example, if a study using 1000 individuals produces a "solution" such that all 1000 people can be properly classified based on their genetic constitution, it is difficult to know how to classify an individual containing a haplotype or haplotype combination not observed in this study. The present approach helps to solve this problem.

By representing genetic data in continuous terms (i.e., in a feature space), continuous partitions in that space can be defined that effectively resolve between discrete haplotype-trait events that have been observed and scored, and have not yet been observed and scored Thus, a solution developed through application of the present method can be more comprehensive than one developed based on standard multivariate analyses.

Geometric modeling of OCA2 haplotypes reveals the power of the individual SNP markers as predictive markers for human hair and eye color. The method is exemplified using the OCA2 gene subject as disclosed herein. Eight SNPs, alleles of which, individually, are associated with the degree to which human hair and eyes are pigmented, were used. These SNPs are, in order, OCA2_5, OCA2_6, OCA2_8, OCA2_RS1800414, OCA2DBSNP_52401, OCA2DBSNP_146405, OCA2DBSNP_165011 and OCA2DBSNP_8321.

Each of these (except OCA2_RS1800414 due to low minor allele frequency) showed an ostensible association with eye or hair color on their own. A haplotype of these 8 markers would be expressed as ATGAAAAG. The first A represents the allele on a person's chromosome at the OCA2_5 locus, the second T the allele at the persons OCA2_6 locus, etc. Each person would have two haplotypes to make a haplotype pair, such as ATGAAAAG/ATGAAAAT. Applying the Stephens and Donnelly algorithm (Am. J. Hum. Genet. 68:978-989, 2001, which is incorporated herein by reference) to the genotype data for Caucasians resulted in the list of haplotypes shown in Table 6-1, below.

The phase of the 8 SNPs in the OCA2 gene were determined for a group of 47 individuals by computationally inferring haplotypes using an algorithm originally proposed by Stephens and Donnelly (2001). From genotype data, the algorithm used a Bayesian Likelihood estimation scheme to predict that there are 19 OCA2 haplotypes present in the 47 person Caucasian population, and predicted the particular pair of haplotypes for each of these individuals. It is from point that the present approach operates.

To encode the haplotypes in a manner that is visually appreciated, a simpler approach than that described above was used. Rather than plot the haplotype cladogram in the 8 dimensional space, assign numerical values to the individual haplotypes and plot the haplotype value pairs for each individual in n-dimensional space (where n is the number of genes or haplotype systems), the haplotype cladogram in 2-dimensional space is plotted and assigned Cartesian coordinates to the individual haplotypes for plotting of haplotype pairs in the n-dimensional space.

Haplotypes were used to construct a cladogram, or an evolutionary tree similar to that shown above. The tree was constructed using a maximum parsimony technique and is not shown because it is essentially represented in Table 6-2. The first step was to use the cladogram to recode the haplotypes into a form that is amenable for plotting in multidimensional space. The method could work as effectively for haplotype-haplotype combinations as for haplotype-genotype combinations.

The algorithm was as follows for the two dimensional approach used in this study:

1) Construct a haplotype cladogram for the haplotype systems of interest.
2) For any one haplotype system (i.e., gene), transpose the cladogram onto a two dimensional grid (see the grid in Table 16-2).
3) Assign values from -n to n to the grid columns and rows such that {n-(-n)}<2.
4) Recode each individual haplotype into its new (x,y) coordinates within this graph. For example, haplotype 2 gets the value (-1,2). Each individual in the haplotype list will now have two pair of coordinates. For example, a person with one copy of haplotype 2 and one copy of haplotype 4 would have the values (-1,2) and (-2,4). This creates a 2X2 matrix for each individual (i.e., {-1,2/-2,4}).

5) Repeat the process starting at step 2 for other haplotype systems (genes) or environmental variables (i.e., biographical or medical data) part of the analysis. If only genotype data is available for a marker, the matrix for each person would be a 1X2 matrix rather than 2X2. Non-genetic data can be encoded for by building a 1XN matrix v= (v1,v2...vn) where N is the number of variables, and v represents a numerical value for the data that is derived by considering a scaled range of possible values.

6) Calculate a vector p = ($p_l$,...,$p_m$) as follows; p1 is the 2X2 or 1X2 matrix of coordinate values for haplotype or genotype one, p2 is the matrix of coordinate values for haplotype or genotype pair two etc; and

7) Plot the vectors in m-dimensional space.

**TABLE 6-1**

List of haplotypes of OCA2

| | |
|---|---|
| 1: | AGTAAAAT (5) |
| 2: | AGTAAAGG (8) |
| 3: | AGTAGGAG (13) |
| 4: | AGTAAAAG (43) |
| 5: | GGCAAAGG (7) |
| 6: | AGTAAGAG (30) |
| 7: | GGCAAAAG (17) |
| 8: | GACAAAAG (9) |
| 9: | AGTAGGAT (10) |
| 10: | AGTAGAAG (5) |
| 11. | GGCAGAGT (2) |
| 12. | AGCAAGAG (13) |
| 13: | AFTAGGGG (1) |
| 14: | GGTAGGAG (2) |
| 15: | AGCAAAAG (3) |
| 16: | AGCAAAAT (4) |
| 17: | AGCAGAAG (3) |
| 18: | AGTAGAAT (2) |
| 19: | AGTAAGAT (1) |

Table 6-1 shows a list of haplotypes for the OCA2 gene obtained by applying the Stephens and Donnelly algorithm to the genotype data set for the markers, in order, to form a haplotype. The grid in Figure 2 was used to encode individual haplotype pairs. For example, a person with the 2,3 haplotype combination would be represented with the values (-1,4) and (-2,1) in the matrix {(-1,4)/(-2,1)}. Once the haplotype pair of each individual was re-coded as a vector, they were plotted in m-dimensional feature space (Figure 2).

In Figure 3, the haplotype pairs for each individual was plotted by drawing a line between the first pair of coordinates (encoded from the first haplotype for that person) to the second pair of coordinates (encoded from the second haplotype for that person). Figure 3 shows that the diploid pair of haplotypes in individuals is non-randomly distributed with respect to hair color. The block arrow indicates that one haplotype combination was only seen in persons of brown hair color. Only persons of blond hair color contain haplotype pairs that arc represented in the plot as lines extending from the bottom left part of the upper left quadrant to the upper right quadrant. Only persons of brown hair color contain haplotype pairs that are represented in the plot as lines extending from the upper right quadrant to the lower left quadrant. Further, only persons of brown hair color contain haplotype pairs that are represented by lines extending from the lower region of the upper left quadrant to the lower left quadrant, and only blonds contain haplotype pairs represented by lines extending from the lower region of the upper left quadrant to the lower right quadrant or upper right quadrant. This pattern was apparent because 1) OCA2 haplotypes are determinative for variable hair color in the human population; 2) individuals with the same, or related haplotypes tend to exhibit a similar hair color trait; and 3) OCA2 haplotypes are associated with hair color in terms of haplotype combinations. The last point provides a reasonable conclusion in view of commonly known genetics principles (i.e., genetic dominance).

The curved arrows indicate that another haplotype combination was seen in persons of black, brown and blond hair color, but that the TYR_3 genotype in persons of black hair color is CC, that in persons of brown hair color is CA and that in persons of blond hair color is AA. This is an example of a second dimension (a second variable) helping to resolve the data and facilitating concept formation. This results is reasonable in terms of genetic epistasis, wherein specific combinations of genes have unique impacts on traits.

From the plot, a series of patterns are discemable, and from these patterns, rules can be constructed that can enable the classification of the posterior probability of correctly classifying a person as belonging to a particular hair color group. If the plot was presented in three dimensions, rather than two, partitions in the space can be drawn to segregate the various hair color groups (which would then be planes), and these partitions can be used as a decision plane against which to make such a classification decision. Additional haplotypes also can be present in the population not represented in this analysis. However, using the present method, routine statistical tests can be used to measure the reliability of the classification of such unknown haplotypes. Assuming that members of a given hair color class contain previously identified haplotypes associated in this analysis with a given class, or related to such haplotypcs evolutionarily, then the present method will provide that they would be positioned in the plot in the same neighborhood as others found in persons of that same hair color. As such, they would fall on the same side of the decision plane as the known haplotype combinations for that group, and their classification would be made accurately because of this. This is true even though the specific haplotypes, or haplotype combination, was not observed in our study.

This data presented herein is a representative sampling of a much larger data set, and only part of the data is shown to keep the figure manageable in terms of complexity. The results of this analysis of 8 locus OCA2 haplotypes and one TYR SNP, allows the following determination:

1) Individuals containing the OCA2 haplotype combination AGTAAGAG/AGTAAAAG (haplotypes 6,4 encoded as (-3,1)(-2,3)) are always (6/6) brown haired individuals. These two haplotypes differ by only one position, hence their proximity on the plot.

2) Individuals containing the OCA2 haplotype combination AGTAGGAG/AGTAAAAG (6/6) (haplotypes 3,4 encoded as (-2,1)(-2,3)) are dark (brown or black) haired individuals if their TYR_3 genotype is CC or CA, but blond or auburn (light brown) haired individuals if their TYR_3 genotype is AA (allele A was linked with the light hair color phenotype on its own).

3) Individuals containing the OCA2 haplotype pair AGTAAAAG/AGTAGGAT (haplotypes (4,9) encoded as (-2,3) (1,3)) are always brown haired individuals (2/2). Any individual with haplotype AGTAGGAT (haplotype 9) and a haplotype other than AGTAAAAG is brown haired individuals (4/4 individuals).

4) Individuals containing the OCA2 haplotype pair AGCAAGAG/AGTAGGAT (haplotypes 9,12 encoded as (-3,-1) (1,3)) are always blond haired individuals (2/2).

5) Individuals with the haplotype 12 AGCAAGAG 6 (-3,-1) and another haplotype not 9 (1,3) arc brown haired individuals (5/5 individuals).

6) Individuals with the haplotype AGTAAAGG (haplotype 2 encoded as (-1,4)), and any other haplotype, are always brown haired individuals (3/3 individuals). Evidently haplotype AGTAAAGG is dominant for brown hair.

7) Individuals with the haplotype AGTAAGAG/GACAAAAG (haplotype combination (6,8) encoded as (-3,1)(0,-4)) are always brown hair (2/2 individuals).

8) Individuals with the haplotype GGCAAAAG (haplotype 7 encoded as (1,-4)) is always brown unless it is accompanied by a haplotype 7 (-3,1) (3/3 individuals). The same is true for haplotype 5 (2,-4) - brown unless paired with (-3,1) (3/3 individuals)

The value of the geometric modeling scheme can be seen in result 8. The same result was obtained with haplotypes 5 and 7, and these two are juxtaposed in the haplotype cladogram which shows that they are highly related to one another. Though the sample size is low for haplotype 5 or haplotype 7, the sample size for haplotype 5+7 is greater, and the result may show statistical significance. By grouping related haplotypes that show similar average genetic effects, one can overcome the limitations inherent to multivariate analyses (mainly, the larger the number of variables, the smaller your sample size for each class of variable combination).

The value of plotting in multiple dimensions can be seen from result 2). Without the TYR_3 genotype to resolve the individuals in the haplotype 3,4 combination group, these individuals would be confounders.

Several other haplotype pairs are present in only one individual used in this experiment. There are some confounders for this study. For example, the haplotype AGTAAAAG/AGTAAAAG(haplotype (4,4), encoded as (-1,3)(-1,3)) appears for persons of brown, red and auburn hair individuals, and the TYR_3 genotype does not help resolve these three groups (not shown in figure). A brown haired person with this pair has the AA genotype and another the CC genotype although the C allele is most frequent in /persons of dark hair. This apparent discrepancy can be explained by assuming that the OCA2 haplotype + TYR_3 genotype does not explain all of the hair color variation in the population; there may be other TYR alleles involved, or other genotypes/haplotypes in other genes that may need to be measured to resolve persons with this haplotype pair. This is an important observation: hair color in humans is not determined by one gene, or by one gene and an allele of a second. It is more complex than a biallelic trait, and there are probably 4-5 genes involved in the coloration of human hair. The results presented in the present two gene analysis identify two of these genes. These may be genes that are analyze later, or they may be genes that have not yet been analyzed.

Although the present analysis does not explain 100% of the variability in human hair color, and indeed, one would not expect a two gene solution to explain all of the variability in human hair color because there are 4-5 genes involved in melanin synthesis for which mutations have been identified to impact human pigmentation, the results obtained for the OCA2 8 locus haplotype + TYR_3 genotype plot explained all but 5/42 of the individuals, and 22/24 haplotype pair classes. The results indicate that human hair color is largely explainable through consideration of the diploid OCA2 haplotype and TYR-3 genotype combination present in any Caucasian individual.

**Table 16-2**

| | -3 | -2 | -1 | 0 | 1 | 2 | 3 |
|---|---|---|---|---|---|---|---|
| 4 | | | 2 | 18 | | | |
| 3 | | 4 | 1 | 19 | 9 | NOTOBS | |
| 2 | 10 | NOTOBS | | NOTOBS | NOTOBS | | |
| 1 | 6 | 3 | | 14 | 13 | | |
| 0 | | | | | | | |
| -1 | 12 | | | | | | |
| -2 | NOTOBS | 17 | 15 | 16 | | | |
| -3 | | | | | | | |
| -4 | | | | 8 | 7 | 5 | NOTOBS |
| -5 | | | | | | | 11 |

Table 16-2 provides a grid of OCA2 haplotypes obtained by overlaying the cladogram of haplotypes onto a two dimensional grid. The number of the haplotype corresponds to the number of the haplotype sequence show in Table 16-1 (i.e., haplotype 2 is AGTAAAAT).

**EXAMPLE 7**

**HAIR COLOR HAPLOTYPE IDENTIFICATION AND MODEL DEVELOPMENT**

The single nucleotide polymorphisms (SNPs) disclosed in this example each, on their own, show an association with the degree to which human hair is pigmented, that is they are penetrant SNPs. In addition, these SNPs can be combined in different combinations to explain variable hair color in the human population.

A "vertical" re-sequencing effort was performed in order to identify the common SNP variants at each of three genes known to be deterministically involved in melanin synthesis; the Tyrosinase (TYR), Tyrosinase like protein (TYRPI) and the Oculocutaneous albinism 2 gene (OCA2). Methods for detecting the nucleotide occurrence at a SNP position are described in Example 4. Of 23 SNP positions surveyed for these three genes, three SNPs were identified at the TYR locus, and four SNPs were identified at the OCA2 locus that contain predictive value for the degree to which human hair is pigmented (see Table 16). All of the SNPs have been disclosed except for the TYRSNP_8 SNP.

TYRSNP_8 is a polymorphism in the tyrosinase gene that was discovered through several mechanisms. Initially, it was identified using software as disclosed above to compare EST sequences to one another from the NCBI Unigene database. It was subsequently identified again from an in-house re-sequencing effort. The TYRSNP_8 SNP is one of the few TYR SNPs present in the public SNP database (dbSNP, NCBI). The data for the TYRSNP_8 marker are shown in Table 1. On its own, this marker appeared to have little value as a predictive tool for hair coloration in humans (Table 7-1). However, when combined into haplotypcs with other TYR markers presented herein, TYRSNP_8 reveals its influence, which is significant.

Unphased genotypes were scored at seven loci (Table 7-2) for 189 individuals. Of these, 46 individuals were Caucasians, for whom there were no missing data for any of the seven loci and for whom hair color was known. Haplotypes within the TYR and OCA2 genes were inferred using the algorithm of Stephens and Donnelly (2001). A program was developed to store these inferred haplotypes into an Oracle schema containing phenotype information for each individual, and phenotype and genotype date for the individuals were then partitioned into two groups; persons of dark natural hair color (black or brown) and persons of light natural hair color (red, blonde).

Table 1 and Table 7-2 show the polymorphisms used for constructing composite solution A. The gene within which the SNP resides is shown in column 1. The name of the SNP is shown in column 2, and the marker number (identification number) is shown in column 3. The IUB code for the nucleotide change imposed by the SNP is shown in column 4, and the amino acid change (if any) is shown in column 5. Nucleotides in brackets indicate deletions. All of these markers are disclosed herein and Table 1 provides additional information regarding the markers used in this study.

In order to test for population level differences in genetic structure between these two groups, pair-wise difference estimations, Slatkin linearized F-statistic estimations and exact tests for non-differentiation assuming the null hypothesis (that no difference between the groups exists) were performed. The results are summarized for three different whole gene haplotype systems in table 7-3.

Table 7-3 shows the population level structure differences between haplotyped individuals (Column 3) at three genes (Column 1) in two different groups (Column 2). The first group contained individuals with dark hair color (brown and black) and the second contained individuals with light hair color (red and blond). The exact test for non-differentiation (Column 4) performs several thousand randomly generated permutations to randomly generate haplotype constituencies for the two groups, and tests the frequency with which these virtual groups show a greater difference between them than the observed groups. A low number indicates that the data actually observed in the study was not due to chance.

The corrected pair-wise differences (CORR. PW, Column 5) measures the average number of differences between randomly chosen sites within haplotypes selected from the two groups, corrected against the average number of differences observed within each group. A higher number indicates that the haplotype constituency of the two groups is significantly different. The P-value for this measurement which is an effect statistic, is shown in Column 6 (PW FST P); a value below 0.05 indicates that the value present in Column 5 is statistically significant. A third measurement of the difference between the colored hair groups is presented in Column 7., the Slatkin F-statistic (SLATKIN); a number higher than 0.05 indicates that the difference between the two groups is statistically significant. The results of these tests show that there is significant difference in the TYR haplotype constituency between the dark and light hair color groups (row 1, Table 7-3). In contrast, little difference in the TYRP1 haplotype constituency exists (row 2, Table 7-3) and borderline difference in the OCA2 haplotype constituency exists (row 3, Table 7-3).

In order to elaborate on the significant population level difference in TYR haplotype constitution, an automated software application was used to score TYR haplotype pairs within each of the two groups. Four different TYR haplotypes (ACG, ACA, AAG, and AGC) and five different haplotype combinations were observed in this analysis (AGC/ACA, ACG/AAG, ACG/ACG, AAG/AAG, AAG/ACA; Table 18). The results of this analysis showed a clear distinction in the average effect on hair color for the four observed TYR haplotypes. Of the persons found to have at least one ACG haplotype (n=32), 96.8% of these individuals had either brown or black hair. Of the remaining individuals (n=15), roughly half were of dark (black or brown) hair color and half were of red or blond (light) hair color. Of persons with two copies of the ACG TYR haplotype (row 3, Table 7-4), 30% had black hair, whereas 9.5% of persons with only one copy of ACG had black hair.

Table 7-4 shows the TYR haplotype pair frequencies for individuals of each of the four hair color classes. The haplotype pair is shown in columns 1 and 2, and the frequency of individuals exhibiting a given hair color within this group is shown in columns 3-6. The haplotype associated with darker hair color is shown in bold print (ACG). Frequencies were tabulated from simple counts of individuals for each diploid pair class.

Though the presence of the ACG TYR haplotype was a good predictive marker for dark hair color, there were a small number (n=8) of confounding dark haired (brown) individuals without the ACG haplotype. In an attempt to explain these confounders, OCA2 haplotypes were compared for the light and dark haired individuals, whom did not have an-AGC TYR haplotype. In addition to lacking an AGC haplotype at the TYR gene, each blond hair individual also haplotyped as a CACG homozygote at the OCA2 locus. Half of the dark haired confounders also had a homozygote pair of CACG haplotypes, but half did not, and grouping the individuals based on the criteria of a homozygous CACG OCA2 haplotype partitioned the data most effectively; no other SNP combinations within the OCA2 gene resolved dark and light haired individuals not containing the AGC TYR haplotype.

In total, using the TYR AGC haplotype and the homozygous condition of the CACG OCA2 haplotype, the combined results explained 100% of the blond individuals and 90% of the brown hair colored individuals in our study (Table 7-5). The two gene solution also explains 91.3% of the total number of individuals in our study with regard to their natural hair color (Table 7-5). Table 7-5 shows a composite solution for variable human hair color in the Caucasian population. The constraints on gene haplotype sequences for our SNPs are boxed in columns 2 and 3, and the line between the columns indicate the operator "AND". For example, row one shows that 100% of the individuals with the non-AGC TYR haplotype AND the CACG homozygous haplotype pair were correctly classified as light haired individuals. The percent of individuals explained by these constraints for the two hair color classes is indicated (rows 1 and 3) in column 4. The total number of individuals explained by the composite solution are indicated in the fourth row of column 4.

The logic of the solution is shown in Figure 3. The accuracy of predictions for the solution is shown in Table 7-6a and Table 7-6b. The solution is capable of predicting the proper natural hair color (Light = blond or red or Dark = black or brown) in Caucasians with over 90% accuracy. Part of the 10% not correctly classified are Auburn haired individuals who were not scored in this study (since it is not clear which group to assign them to). When the test is performed on a multi-ethnic group of individuals the accuracy improves to 98%. The reason for this improvement is due to dramatic differences in allele frequencies for each of these markers in the various ethnic groups, and for each of the seven SNPs part of this solution, the frequency of the allele associated with darker hair color in Caucasians is dramatically enriched in the ethnic groups which tend to have darker hair color (African Americans). Because of this, the haplotype solution applies better to the general world population than to Caucasians alone; including African Americans and Asians improves the performance of the solution.

In the experiment discussed in this Example, SNPs within the TYR, TYRP1 and OCA2 genes were identified that are individually associated with the degree to which human hair is pigmented. In order to use these SNPs to develop a genetic solution that explains the maximum amount of hair color variation in the population, haplotypes incorporating each of these positions in individuals of known hair color were scored, and the results were combined in various combinations in order to obtain the optimum solution for resolving individuals with dark versus light hair color. The results revealed a composite, nested solution for classifying an unknown individual as belonging to the dark versus light hair colored groups.

The solution employs haplotypes at two of these genes (TYR and OCA2). The first step of the solution determines the diploid pair of TYR_3, TYR_5 and TYRSNP_8 haplotypes in an individual. Individuals with one or two copies of the AGC haplotype are classified as belonging to the dark hair color group with 81% accuracy in Caucasians and 98% accuracy when applied to individuals irrespective of race. This step results in two groups - a correctly classified dark hair color group (AGC haplotype containing), and a mixed group of dark and light hair colored individuals (non-AGC haplotype containing). The second step uses the individuals without the TYR-AGC haplotype. The diploid pair of OCA2_2, OCA2_5, OCA2_RS 1800405 and OCA2_6 haplotypes were determined for each individual. If an individual had a homozygous CACG haplotype pair, they were classified in the light hair group with 100% accuracy. If not, they were classified in the dark hair group with only 50% accuracy. The final accuracy of the solution was 90% within the Caucasian group and 98% when applied to individuals irrespective of race.

This solution appears to be the first method capable of using a DNA specimen to classify an unknown individual with regard to natural hair color. If the ethnicity of the individual is known from other tests such as an STR test, then the accuracy of the determination can be precisely determined. For example, if the race of the individual is African American, the dark hair answer from our solution would be correct 98% of the time. If the race of the individual is Caucasian, the dark hair answer would have a likelihood of being correct of 90%, and a light hair answer would have a likelihood of correctness of nearly 100%.

The results also indicate that there is a dose response effect for the ACG haplotype, as individuals with the ACG/ACG haplotype pair are significantly more likely to have black hair than brown hair. Individuals with only one copy of ACG arc more likely to have brown hair than black. Interestingly, the ACG/ACG haplotype pair is the most frequent haplotype found in the African American group, which is mainly comprised of black haired individuals. By noting the number of ACG haplotypes an individual harbors, the posterior probability that the specimen belongs to a black versus a brown haired individual can be calculated. Thus, the solution disclosed herein can resolve hair colored individuals on terms that are more subtle than dark versus light.

**TABLE 7-1**

| | TYRSNP_8 GENOTYPE | | |
|---|---|---|---|
| | AA | GA | GG |
| EYE | | | |
| BROWN | 0 | 6 | 5 |
| HAZEL | 0 | 5 | 5 |
| GREEN | 0 | 5 | 4 |
| BLUE | 0 | 7 | 8 |
| | | | |
| HAIR | | | |
| BLACK | 0 | 2 | 0 |
| BROWN | 0 | 14 | 12 |
| RED/AUB | 0 | 2 | 2 |
| BLOND | 0 | 3 | 3 |

**TABLE 7-2**

| Gene | SNP name | Marker | Nucleotide Change | AA change |
|---|---|---|---|---|
| TYR | TYR 2 | 217467 | [ATA] | Ile deletion |
| TYR | TYR 3 | 217468 | M | Ser to Tyr |
| TYR | TYRSNP 8 | 217473 | R | Arg to Gln |
| OCA2 | OCA2 2 | 217452 | Y | Arg to Trp |
| OCA2 | OCA2 5 | 217455 | R | Silent |
| OCA2 | OCA2 RS1800405 | 712061 | Y | Intron |
| OCA1 | OCA2_6 | 217456 | R | Arg to Gln |

**TABLE 7-3**

| GENE | GROUPS | N | EXACT P VALUE | CORR. PW | PW FST P | SLATKIN |
|---|---|---|---|---|---|---|
| TYR | DARK/LIGHT hair | 48 | 0.00000 +- 0.00000 | 0.27053 | <0.0001+- 0.0000 | 0.376 |
| TYRP 1 | DARK/LIGHT hair | 48 | 0.41130+- 0.00663 | 0.01013 | 0.4775+- 0.0237 | 0 |
| OCA2 | DARK/LIGHT hair | 48 | 0.98720 +- 0.00289 | 0.11463 | 0.0360+- 0.0201 | 0.042 |

**TABLE 7-4**

| | | NUMBER OF HAIR COLORED INDIVIDUALS | | | |
|---|---|---|---|---|---|
| HAP1 | HAP2 | BLACK | BROWN | RED | BLOND |
| ACG | ACA | 0.14 | 0.86 | 0 | 0 |
| ACG | AAG | 0.53 | 0.41 | 0 | 0.06 |
| ACG | ACG | 0.30 | 0.70 | 0 | 0 |
| AAG | AAG | 0 | 0.40 | 0 | 0.60 |
| AAG | ACA | 0 | 0.60 | 0.10 | 0.30 |

**TABLE 7-5**

| HAIR | TYR | OCA2 | CORRECT CLASSIF. |
|---|---|---|---|
| LIGHT | NON AGC | CACG HOMO | 100% |
| DARK | NON AGC | NOT CACG HOMO | 50% |
| DARK | AGC | | 97% |
| ALL | | | 91.3% |

**Table 7-6a Total Caucasians Correctly Classified:**

| Group | Individuals correctly classified | Total individuals in group | Percent accuracy of classification |
|---|---|---|---|
| Light | 7 | 7 | 100% |
| Dark | 36 | 41 | 88% |
| Total | 43 | 48 | 90% |

**Table 7-6b Total Caucasians, African Americans and Asians Correctly Classified:**

| Group | Individuals correctly classified | Total individuals in group | Percent accuracy of classification |
|---|---|---|---|
| Light | 7 | 7 | 100% |
| Dark | 228 | 233 | 98% |
| Total | 235 | 240 | 98% |

## EXAMPLES 8

## EYE COLOR HAPLOTYPE IDENTIFICATION AND INFERENCE MODEL DEVELOPMENT

Having identified several haplotype systems whose constituents were associated with eye color shade, a nested statistical approach was developed for assembling these component pieces into a complex genetics mosaic for explaining variable human eye color shade. A classification tree solution developed using these systems was 96.3% accurate for genetically predicting the degree to which human retinas are pigmented in Caucasians.

In this example, which is not the optimal solution, the tyrosinase (TYR), oculocutaneous 2 (OCA2), tyrosinase like protein 1 (TYRP1), melanocortin receptor (MC1R) and adaptin B1 protein (ADP1), adaptin 3 D subunit 1 (AP3D1) loci were selected as candidate genes for the study of variable human eye color because they are known to be involved in pigmentation and from mutant OCA phenotypes it is known that they play a role in retinal pigmentation. Except for the OCA2 gene, relatively few SNPs have been documented in public database resources (NCBI:dbSNP), and those SNPs that are present are not evenly distributed across the coding sequence of the genes. Because comprehensive SNP maps (both in a horizontal sense from 5' to 3' and in a vertical sense from large numbers of individuals) are required in order to thoroughly survey the contribution of common haplotypes towards variable human traits, first a detailed SNP map was built for each of these genes. Methods for detecting the nucleotide occurrence at a SNP position are described in Example 4. Forty, 20, 15, 25 and 10 candidate SNPs were identified in the OCA2, TYRP1, MC1R, TYR and APB3 genes, respectively. Using a group of 133 Caucasian, 133 African American and 40 Asian individuals of unknown pigmentation, about 80% of these SNPs were validated as polymorphisms, 60% of these had a minor allele frequency of 1% or greater in this multi-ethnic group and half of these 60% were bi-allelic in the Caucasian population (data not shown, and accumulated with the assistance of Orchid Biosciences of Princeton, NJ). These SNPs were passed to phase 2 of the study.

Next approximately 300 Caucasian individuals were scored for self-reported eye color at each of these SNPs. From this data, the SNPs were prioritized by calculating the allele and genotype frequencies in groups of individuals of different races and varying eye colors and eye color shades. For the latter classification, light ) eyes were defined as either blue or green and dark eyes as black, brown or hazel. SNPs were passed to the third round of analysis if their bi-allelic genotypes, or one of their alleles, were preferentially represented within an eye color or eye color shade group as determined using chi-square tests. If a SNP passed this test, and the dark allele was preferred in, or monomorphically present in races of average darker eye color than Caucasians (such as African Americans and Asians), it was passed to the third phase of the analysis. In fact, this latter constraint proved to not be necessary, as all of the alleles associated with darker eye colors in Caucasians were over-represented in races with darker average eye color (data not shown). SNPs passing all three tests were passed to the next step of the analysis where they were randomly condensed into various overlapping, and non-overlapping haplotype systems and tested for association to shade of eye color. To maximize the statistical power of our analysis, we focused on 2 and 3 locus haplotype systems.

### TYR2LOC920

Fifteen novel (validated) SNPs within the TYR gene were identified. Five of these SNPs passed the three selection criteria. Using these five SNPs, five haplotype systems were constructed and identified one that appeared to be especially predictive for Caucasian eye color (TYR2LOC920, incorporating 2 SNPs in the seventh exon of the TYR gene). To test whether individual TYR2LOC920 haplotypes are associated with shade of eye color, individual haplotypes were counted in each of two classes of eye color shade (dark= black, brown or hazel; light= blue or green). The null hypothesis that eye colors are not associated with specific TYR2LOC920 haplotypes was tested by performing a Pearson's Chi-square and Fisher's exact test on haplotype counts (Table 8-1).

The Pearson's chi-square test value was 6.56 (df=3j, p=0.087), and the Fisher's exact test resulted in a p=0.079. Both of these are significant at the p<0.10 level, but not at the p<0.05 level. Constructing conditional probability statements from the data, where p=prob(light|haplotype), we observed that the probability that a TYR2LOC920 individual with a CA haplotype is light eyed is p=0.39, (95%Cl is [0.32, 0.44]), which is almost one half that of an individual with a CG haplotype (p=0.51, 95% CI [0.43, 0.58]). Taken together, the results suggest that there may be a statistical association between individual TYR2LOC920 haplotypes and shade of eye color. Analysis at the level of the genotype (diploid pair of haplotypes) revealed more convincing results. To test the null hypothesis that there is no association between genotypes and eye colors we calculated Chi-square test and effect statistics for each of the haplotype systems. Table 8-2 shows the counts of the observed TYR2LOC920 genotypes. The results suggested a clear relationship between TYR2LOC920 genotypes and eye color; a greater number of individuals with G23 genotype (AG/CA) are light eyed than not, but the reverse is true for individuals with the G11 genotype (CG/CG). Pearson's chi-square test without Yates' continuity correction for counts of the 6 observed genotypes yielded a value of 21.31, with 5 degrees of freedom (p = 0.0007). A Fishers exact test statistic was significant at the P= 0.0003 level. These results allow a rejection of the null hypothesis in favor of the hypothesis that eye colors (defined as light = blue and green, and dark - hazel, brown and black) are associated with specific TYR2LOC920 genotypes. To more specifically identify and quantify the associations we computed the adjusted residuals (AR, data not shown), which follow an N(0,1) distribution as per large sample theory. The values of AR clearly showed that genotypes G11:CG/CG and G22: AG/AG are significantly and positively associated with dark eye colors (p<0.05) and genotype G23:AG/CA is associated with light eye color (p<0.05)(data not shown).

OCA3LOC109

Nineteen novel SNPs were identified within the OCA2 gene that met the three selection criteria. Using these SNPs, we constructed and tested 10 haplotype systems and identified five that appeared to be predictive for Caucasian eye color. Two of these haplotype systems (OCA3LOC109, incorporating 3 SNPs (markers 217458, 712054, and 886896) distributed evenly within the region from exon 11 to the 3'UTR within the OCA2 gene; OCA3LOC920, incorporating 3 SNPs (217452, 217455, and 712061) spread more or less evenly within the $9^{th}$ and $10^{th}$ exons of the OCA2 gene) gave especially strong results.

To test the null hypothesis that there is no association between OCA3LOC109 haplotypes and shade of eye color, we performed chi-square and adjusted residual tests on the OCA3LOC109 haplotype counts for individuals of the various eye color shades (Table 8-3).

This analysis indicated that specific OCA3LOC109 haplotypes were associated with shade of eye color (chi-square = 29.47, d.f.=6, p<0.0001). Adjusted residuals were calculated for the haplotypes and haplotype H1:ATA was found to be significantly associated with light eye color (p<0.05). In contrast, haplotypes H4:GCA, H5:GCG, H6:GTA and H7:GTG were found to be significantly associated with dark eye color (p<0.05 for each haplotype). We next extended the analysis to OCA3LOC109 genotypes (diploid pairs of haplotypes) (Table 8-4). We tested the null hypothesis that there is no association between OCA3LOC109 genotypes and eye color shade. The result of this analysis revealed that certain OCA3LOC109 genotypes were associated with shade of eye color (chi-square value=42.5478, d.f.=17, p=0.0006). These results allowed a rejection of the null hypothesis in favor of the hypothesis that eye colors (defined as light = blue and green, and dark - hazel, brown and black) are associated with specific OCA3LOC109 genotypes. To more specifically identify and quantify the associations, we computed the AR for the genotype counts (data not shown). This analysis revealed that genotype G12:ATA/ATG is statistically associated with light eye color (p<0.05 level), and that genotypes G25:ATG/GCG and G27:ATG/GTG are found to be associated with dark eye color (p<0.05 for each).

Due to the unusual strength of these associations, a site-by- site analysis of allelic contribution towards variance of eye color was conducted. To test the null hypothesis that mutation at the first locus of the system contributed any variation in eye color, chi-square tests were conducted on sub-cladogram groups of OCA3LOC109 haplotypes that isolated the variation at locus one within three locus haplotype system. Testing the significance of difference between individual haplotypes within this context revealed chi-square values that were highly significant; comparison of eye colors for individuals of the H2:CGC versus the H3:TGC genotypes gave a Chi-square value=8.0115, d.f.=1, P=0.0046 and Fisher's exact test P-value=0.0049. Similar results obtained when mutations at site 2 and site 3 of this haplotype system were tested (Chi-square value=4.3544 d.f.=1, P=0.0369/Fisher's exact test P-value=0.0571 and Chi-square value=4.4399 , d.f.=1, P=0.035/Fisher's exact test P-value=0.0363, respectively). The conclusion from these combined results was that mutations at each of the three sites within the OCA3LOC109 haplotype system contribute to variation in eye color shade. A nested contingency analysis between haplotypes and eye colors confirmed these findings. In this case, we have seven haplotypcs: 0-step clades are represented by: H1:ATA, H2:ATG, H3: ACG, H4:GCA, H5:GCG, H6:GTA, H7:GTG. 1-step clades are represented by: I-1:(H1, H2), I-2:(H3), I-3:(H4, H5), I-4:(H6, H7) and 2-step clades: II-1:(I1, I2)=(H1,H2, H3), II-2:(I3,I4)=(H4, H5, H6, H7) (Figure 4).

The nested contingency analysis (using light=blue, green and not-light=black, brown and hazel eye colors) revealed a significant chi-square value between 2-step clades ((H1+H2+H3) vs. (H4+H5+H6+H7) (chi-square=20.75, p=<0.0001, Fishers P=0.000017). The results showed that Haplotypes H1:ATA, H2:ATC and H3:ACG are significantly and positively associated with light eye colors, where as haplotypes H4;GCA, H5:GCG, H6:GTA and H7:GTG are significantly associated with not-light eye colors. Odds ratio for (H1+H2+H3) presence in individuals of light eye color shade were 3.134 and its 95% C.I. is [1.8871, 5.2051]. Analysis of the results showed that most of the significant variations in eye colors can be traced back to the mutation at site-1.

OCA3LOC920

The results from analysis of the OCA3LOC920 haplotype system revealed similar phenomena to that described for the OCA3LOC109 system. From the haplotype counts, we observed that the individual OCA3LOC920 haplotypes were associated with the shade of human eye color (chi-square value=15.0293, d.f.=3, p =0.0018; Fisher's exact p= 0.0021) (Table 8-5).

Adjusted residuals for the OCA3LOC920 system revealed that haplotype H1:CAC is found to be significantly associated with light eye color, and haplotypes H2:CGC, and H3:TGC are found to be significantly associated with dark eye color at the p<0.05 level. To isolate the deterministic mutations within the haplotype system we tested the null hypothesis that mutation at site-1, site-2 and site-3 within the system did not contribute any variation in shade of eye color (data not shown). Mutation at site-1 (C←→T, H2: CGC←1→H3:TGC) was found to be marginally associated with eye color shade (Chi-square value=2.8265, d.f.=1, P=0.0927 and Fisher's exact test P-value=0.1414), but mutation at site-2 (A←→G H1: CAC←2→H2:CGC) was found to be significantly associated with the shade of eye color (chi-square value=6.0122, d.f.=1, P=0.0142 and Fisher's exact test P-value=0.0185). Odds ratio for H2: CGC for dark eye color was 1.8677 and its 95% C.L is [1.1275, 3.0941]. Mutation at site-3 (C←→T H2: CGC←3→H4:CGT) revealed insignificant results. From these results it was inferred that mutation at site-2 contributes toward most of the variation in shade of eye color.

To determine whether and which specific OCA3LOC920 genotypes (diploid pairs of haplotypes) were associated with eye color shade, the null hypothesis that there was no association between OCA3LOC920 haplotypes and shade of eye color, was tested (Table 8-6). The results revealed that there were indeed associations between OCA3LOC920 genotypes and eye color shade (chi-square value=19.5808, d.f.=6 and P-value=0.0033; Fisher's exact test P-value=0.0027).

Because these results were significant, wen next performed a nested contingency analysis between haplotypes and eye colors, with 0-step clades: H1:CAC, H2:CGC, H3: TGC, H4:CGT, 1-step clades: I-1:(H1), I-2:(H2, H4), I-3:(H3) and 2-step clades: II-1:(I1)=(H1), II-2:(I2, I3)=(H2, H4, H3). The results revealed a significant difference in eye color shade between two step clades (chi-square=14.9709, d.f.= 1, p= 0.0001, exact p=0.0003) (Fig. 5). The odds ratio that individuals with haplotypes among the cladogram sub-group (H2+H3+H4) are dark eye shade individuals is 2.4903 and its 95% C.I. = [1.5534, 3.9924]. This analysis reveals that haplotype H1:CAC is positively and significantly associated with light eye color shade, whereas haplotypes, H2:CGC and H3: TGC are positively significantly associated with dark eye color shade. From inspection of the haplotype subgroups, we inferred that the variation in eye color shade can be traced back to the primary mutation at site-2 within the OCA3LOC920.

<u>MCR3LOC AND TYRP3L105</u>

A similar analyses was performed for SNPs in 6 other genes (AP3B1, CYP3A4, CYP3A5, CYP2D6, CYP2C9, HMGCR, FDPS among others)(Table 8-7). Within these 6 genes, an average of 30 SNPs were discovered per gene, but only two of the genes (MC1R and TYRP1) had SNPs that passed each of our three eye color selection criteria (data not shown). Three haplotype systems were tested in each gene (average number of loci = 2.5) for association with specific classes of eye color shade. For each of the systems, the results were statistically insignificant at the p<0.05 level. The best MC1R haplotype system was the MCR3LOC105 haplotype system comprised of 3 SNPs (markers 217438,217439, and 217441) distributed more or less evenly across the coding region of the gene (p>0.20). The best TYRP1 haplotype system was TYRP3LOC105, which contained 3 SNPs (markers 886937, 217458, and 217486) distributed more or less evenly across the region between the fourth exon and the 3'UTR (p = 0.144). Because the SNPs comprising these haplotype systems passed the three SNP selection criteria, suggesting that they are capable of explaining at least a small amount of the variation in human eye color, they were incorporated in the analyses described below. The haplotypes were used for these genes rather than their component SNPs because of the enhanced statistical power haplotypes offer for genetic association studies.

Next, an attempt was made to develop a classification strategy for using the four haplotypes systems to predict eye color. The first approach attempted was a Bayesian method, using the frequencies of the eye color classes as the prior probabilities and the frequency of a (haplotype based) genotype in the eye color class as the class conditional density functions. The posterior probability that an individual belongs to a given class of eye color shade is simply the product of the posterior probabilities derived for each of the four genes, and the eye color class with the highest probability is selected. When applied to our study sample, this method resulted in a classification solution of poor accuracy (about 84%, data not shown) and low utility (less than 80%). By assigning weights to the posterior probabilities for each haplotype system, based on the amount of variance each explains on its own, the accuracy could be improved slightly to 89%, but the utility of the classifier was still low (less than 85%).

As an alternative to these methods, a nested statistical scheme was developed by which to construct classification rules using complex, compound genotypes. Though a Bayesian classifier could have been used for this task, instead a routine was chosen that resembles a genetic algorithm. Within the scheme, a compound genotype contains elements (haplotype pairs = genotypes) from multiple genes. The scheme builds a classification tree in a step-wise manner. The roots of the tree are genotypes of a randomly selected haplotype system. Nodes arc randomly selected genotype classes, within which there are numerous different constituent genotypes. Compound genotype classes contain more than one compound genotype, the constituents of which are derived from a discrete combination of haplotype systems. Edges connect roots and nodes to comprise compound genotype classes. The tree is built by first selecting a set of roots and growing the edges to nodes based on the genetic distinction between individuals of light (blue, green) and dark (black, brown) eye color shade within the new compound genotype class defined by the connection (hazel is always assigned to the eye color shade with the most members). Within a compound genotype class, a pair-wise F statistic and associated p-value is used to measure the genetic structure differences between individuals of the various shade of eye colors, though an exact test p-value has also been used with similar results. Individuals of ambiguous haplotype class (less than 75% certainty) are discarded and classified as "not classifiable". All possible nodes not yet incorporated in the path from the root are tested during each new branching step, and the branch that results in the most distinctive partition (i.e., the lowest p-value) among the classes of eye color shade is selected. If there is no genetic structure within the new compound genotype class, the branching continues to another node (haplotype system), unless there are no more haplotype systems to consider or unless the sample size for the compound genotype is below a certain pre-selected threshold (in which case a "no-decision" is specified). If the lowest p-value for the new compound genotype class is significant, rules are made from its constituent compound genotypes exhibiting significant chi-square residuals. In this case, genotypes within the compound genotype class which are not explainable (for whom chi-square residuals are not significant) are segregated from the rest of the compound genotypes within the class to form new nested node(s), from which further branching is accomplished. Nested nodes always represent new compound genotype classes at first. If branching from this nested node does not result in the ability to create classification rules, the algorithm returns to the compound genotype class from which the nested node was derived and recreates N nested nodes of N constituent compound genotypes. In either case, nested nodes are only created from nodes with statistically significant population structure differences among the shade of eye color classes. In effect, this algorithm allows for the maximum amount of genetic variance contributed by the various combinations of haplotype systems to be learned within specific genetic backgrounds. Once the tree has been completed, the rules produced from it arc used to predict the eye color shade of each individual. If the prediction rate is good (say 95% or greater) the process ends, and if it is not, the process is begun again starting with a new haplotype system for the root.

A classification tree was generated using this approach with the TYR2LOC920 (markers 217468 and 217473), OCA3LOC920 (markers 217452, 217455, and 712061), OCA3LOC109 (markers 217458, 712054, and 886896), TYRP3L105 (markers 886937, 217485, and 217586) and MCR3LOC105 (markers 886937, 217485, and 217486) haplotype systems (Table 8-8). The roots for the optimal tree selected were genotypes of the TYR2LOC920 haplotype system. The identity and order of the subsequent nodes originating from the various TYR2LOC920 genotype classes were distinct for each particular root. For example, the first node (second haplotype system) selected for TYR2LOC920 AG/CA individuals (rows 1-12, Table 8-8) was the OCA3LOC920 system, though the MCR3LOC105 system was selected as the second node for TYR2LOC920 AG/AG individuals (rows 15-22, Table 8-8). The effect statistics for the branching process are shown in Table 8-9. Comparing this Table with the specific rules in Table 8-8, it is clear that all decisions to formulate classification rules for a compound genotype were justified by the existence of population level genetic structure differences within the compound genotype class from which it was derived. A number of rules were formed from compound genotype classes for which measures of population level genetic structure differences were not calculable. Usually, this was because there was only one compound genotype class for one or both of the hair color shade groups (the test requires genetic diversity within each population). In these cases, chi-square residuals on the compound genotypes justified the construction of classification rules incorporating them (requiring a $p<0.05$, data not shown). Sometimes, rules could be constructed for compound genotypes derived from compound genotype classes of small sample size (i.e., $n<15$), because the distribution of genotypes among the eye color shades were clearly partitioned as measured using the chi-square residuals. For example, only 9 individuals were part of the TYR2LOC920 AG/AG: MCR3LOC106 OTHER (not CCC/CYC) compound genotype class , but these 9 individuals partitioned nicely among the eye color groups with a F-statistic P=0.027 +/- 0.014. In some cases, significant chi-square residuals were obtained for compound genotypes of quite low sample size because individuals with these genotypes were all of darker eye color shade which were under-represented in our study by a ratio of about 1:2.

Tabulating the number of correct and incorrect classifications that result from application of the optimal classification tree (Table 8-8), it was observed that 208 individuals were correctly classified, whereas only 8 were misclassified. Thus, the accuracy rate of the solution was 96.3% (Table 8-10). Thirty three individuals were not classified. In rare cases, these inconclusive determinations were the result of small sample sizes within the compound genotype class that negatively impacted the p-values even if there was a good segregation of compound genotypes among the hair color shade classes. In most cases, the chi-square statistic residuals for the compound genotype classes for these individuals were statistically insignificant because the compound genotype class simply did not allow an explanation of the individual's eye color shade. For these individuals, the four gene, five haplotype system model that was employed simply did not "work". The (computationally derived) haplotype phase of 27 individuals were not certain at the 75% level, and thus no classification could be made for them. Combining the inconclusive determinations with the un-haplotypable, a total of 60 individuals were not classifiable in our study. Thus, the solution exhibited a utility for 81% of Caucasians tested. However, within haplotype-certain Caucasians (a more relevant group for the determination since haplotype uncertainty can be easily eliminated by a user of the test) the solution exhibited a utility for 87% of Caucasians. We also tested the solution on individuals of other races (Asians and African Americans). When applied to African Americans, Caucasians and Asians, the accuracy of our solution improved to 99.9%, with 98% of the individuals classifiable.

The tree in Table 8-9 follows the same format shown in Table 8-8, and shows the pair-wise F-statistic P values used within a compound genotype class to infer genetic structure differences between groups of individuals of different eye colors. The ability to partition individuals within a compound genotype class in a manner that is statistically significant using this test imparts justification by which to formulate classification rules for particular genotypes within the compound system (see text and Table 8-8). The rules are constructed from chi-square residuals as described in the text. The haplotype system used to construct compound genotypes within each row (compound genotype) is indicated in each column. If a genotype is provided with the haplotype designation (ex. OCA3LOC109 ATA/ATR), the node comprises individuals of only these genotypes. Degenerate nucleotide positions are indicated with IUB codes. The tree is read from left to right starting with the operator ***if***. The first column contains the root (see text) of a compound genotype class. Progressing to the next column to the right, the operator ***and*** is used to include the first node (if any), and then the second (if any) and so on until a statistically significant partition can be made within the new compound genotype class. If individuals of different eye color shades within this new compound genotype class can be partitioned into subgroups of statistically significant genetic structure (described in the text, using a pair-wise F-statistic test), the process terminates along a row at the relevant P value for the test. If not, this process continues to the next haplotype system to the right. When (or if) statistical significance is achieved, the compound genotypes are used to construct classification rules (shown in Figure 4 and discussed in text) for the pertinent individuals. For example, considering rows one through three, there is no statistical association between OCA3LOC920 genotypes and eye color within the class of individuals with a TYR2LOC920 AG/CA genotype. Thus, the path leads to the MCR3LOC106 haplotype system in the second column. Individuals of the compound genotype class TYR2LOC920 AG/CA:OCA3LOC109 CAC/CAC (rows 1 and 2) thus comprised a new compound genotype class. Members of this class are partitionable along eye color classes using the MCR3LOC106 haplotype system in column 3. For example, TYR2LOC920 AG/CA:OCA3LOC109 CAC/CAC individuals with the MCR3LOC106 OTHER (not CCC/CYC) genotype were partitionable into the various eye color shade classes as indicated by statistically significant differences in the MCR3LOC106 haplotype composition between light (blue, green) and dark eye (brown or black) individuals within the compound genotype class (P<0.001 +/- 0.001, n=33). Thus, classification rules were constructed for individuals of particular compound TYR2LOC920:OCA3LOC920:MCR3LOC106 genotypes. P=INCALC means that the P value was not calculable. The most common reason for this is genetic homogeneity within one or both of the eye color classes for the compound genotype in question. The pair-wise method measures the average number of differences within groups compared to that number between groups, and this genetic homogeneity within the final haplotype system of a compound class makes the calculation of the within group difference technically impossible. In this case, chi-square residuals were used to justify the formulation of classification rules.

## DISCUSSION

A four gene five haplotype system model for genetically predicting human eye color, is described in this Example. To our knowledge this is the first such model described. The solution derived from this model is capable of correct classification 96.3% of the time, conditional on the race of the DNA donor being Caucasian. If there is equal probability that the race of the donor is Caucasian, African or Asian, the accuracy of the solution improves to 99.9%, and the utility (the ability to make a decision) improves from 81 % to 98%. Most non-Caucasian ethnic groups exhibit low variability in eye color, so this improvement may not seem surprising. However, though the variability of eye color is relatively low in these ethnic groups, an incorrect solution would not necessarily be more accurate when applied unconditionally to individuals of the various world populations. Notwithstanding genetic heterogeneity, a correct solution would be more accurate when so applied. The reason for this is that if alleles associated with darker eye color in Caucasians are deterministic, or linked to deterministic alleles for melanin production and eye color, and if we assume genetic heterogeneity in eye color determination is low, the frequencies of these alleles should be greater in populations of average darker eye color. In fact, the accuracy of the solution increases when applied pan-ethnically because all of the dark-eye associated haplotypes that are part of the solution, as well as each of their component SNPs individually, were found in greater frequencies in non-Caucasian ethnic groups. Therefore, the fact that the accuracy of the complex solution improves when applied pan-ethnically confirms the validity of the solution and suggests that genetic heterogeneity in eye color determination is low in the world population.

Though our solution is 96.3% accurate in "classifiable" individuals, 18% of the total number of Caucasians we tested were not classifiable with our solution. About half of these individuals were individuals of rare compound haplotype classes, which are problematic because: 1) their haplotype phase determination is uncertain using computational (i.e., probabilistic) methods and 2) the sample size for the compound genotype classes within which they fall is too small for statistically significant rules to be constructed (which was rarely the case). Biochemical, rather than computational haplotyping would eliminate group 1) individuals and larger sample sizes (and additional work) may eliminate group 2) individuals. In both cases, the solution disclosed in this Example will have to be augmented to accommodate these rare haplotypes (if they are even classifiable). However, the other half of the not-classifiable group of individuals were simply not explained by our solution at all. These represent individuals within compound genotype classes that do not neatly segregate into (i.e., were not statistically associated with) the various eye color shades. For these individuals, it seems that either: 1) other SNPs within the genes we surveyed are deterministic for eye color shade, and therefore, our solution does not explain all of the variability that these four genes contribute towards variability in the trait and/or 2) other loci altogether are deterministic for eye color shade within certain genetic backgrounds derived from the model. The likelihood of the former of these possibilities seems low since our approach for discovering SNPs was comprehensive. The latter possibility seems more likely, but invoking it would require the assumption that the contribution of a genotype at a particular locus is dependent on the genetic background within which it is found. Indeed, inspection of the solution we have generated confirms that this is the case for almost all genotypes part of the solution. We therefore assert that the utility of our solution is about 87% in Caucasians of known TYR, OCA2, MC1R and TYRP haplotypes, and that the amount of eye color shade variance our model could explain is likely to be somewhat higher, though limited by the as of yet unqualified involvement of other loci that we have not part of this study.

Though ours is a four gene model, it is not inconsistent with Brue's assertion that retinal pigmentation is predominantly controlled by the activity of two loci. The best classification tree (i.e., solution) derived from our algorithm incorporated the haplotype system from the TYR gene as the root. Four of the five first nodes were genotypes of the haplotype system from the OCA2 gene. It is interesting to note that, of the four genes we used for classification rule construction, these two were by far the most significantly associated with eye color. Even though two thirds of Caucasians required haplotype systems in other genes (MC1R and TYRP1) to be correctly classified, about a third of the individuals (68) were correctly classifiable based on TYR and OCA genotype alone and virtually none of the eye color variation in our study was explainable with compound genotypes not including the TYR and OCA2 systems. These observations combine to strongly suggest that the TYR and OCA2 genotypes combine to explain most of the variability in Caucasian eye color, and that other genes (mainly MC1R, TYRP, and perhaps others) contribute to explain a small amount of this variation. These observations are not inconsistent with Brues' model. Nonetheless, the complexity of our model illustrates a crucial point for developing classifier tests. Though most of the variation in human eye color can be explained by two genes, and reasonable classifier tests can be constructed based on them alone, we have shown that the tests so developed perform with an accuracy that is unacceptable for use in the field or clinic. Results of the studies discussed in this Example indicate that the simple approach of using individual haplotypes as discrete objects rather than components of complex objects leads to classification solutions that perform poorly (although they still perform, to a certain extent). Not to be limited by theory, this may be because eye color is a complex genetic trait, and complex genetic "wholes" are often times greater than the sum of their component "parts". Measuring classification probabilities as a function of individual haplotype frequencies does not allow for the capture all of the trait variation the genes combine to explain. Our results illustrate a seemingly obvious but interesting concept: simple genetics approaches are useful for ascribing trait associations for individual genes and haplotypes within them, but because most human traits are complex, complex genetics tools arc required to use these genes and haplotypes for the development of accurate classification tests. In our case, we had to consider individuals in terms of compound genotypes (i.e., analogous to n-dimensional feature vectors plotted in the n-dimensional feature space) in order to develop an accurate classifier. This idea has precedence from studies in *Drosophila*, where allelic penetrance for a large number of complex traits has been shown to be a function of genetic background.

Interestingly, the solution generated as discussed in this example docs not appear to explain variable hair or skin color (data not shown). In fact, this is what one would expect from a good eye color solution for Caucasians since eye, skin and hair color are independently inherited and distributed within this racial group. Our solution is also usually not sensitive enough to predict the precise eye color of an individual. Rather, it can only be used to classify a biological specimen as having been derived from an individual of a given shade of eye color. This also portends the involvement of other genes and/or variant(s) in the determination of this complex trait. The accuracy of the solution for explaining variable eye color in members of other ethnic groups is not yet known with precision due to the low number of minor eye colors in these groups (which are difficult to obtain). Nonetheless, as the first genetic solution capable of ascribing qualitative characteristics from anonymously donated DNA, our results represent a potentially important achievement. First, they illustrate one method for dissecting complex human traits using high-throughput genomics techniques. Second, as a forensics tool, our solution could be used to guide criminal or other forensics investigations. Third, as a research tool, the common haplotypes we have identified may help researchers more accurately define risks for pigmentation related diseases such as cataracts and melanoma.

**Table 8-1**

| Haplotypes | H1:CG | H2:AG | H3:CA | and | H4:AA |
|---|---|---|---|---|---|
| | | | | | |
| Eye colors | | Haplotypes | | | |
| | H1 | H2 | H3 | H4 | Total |
| | | | | | |
| Light | 86 | 86 | 74 | 0 | 246 |
| Not-Light | 135 | 107 | 72 | 2 | 316 |
| TOTAL | 221 | 193 | 146 | 2 | 562 |

Table 8-1. Individual TYR2LOC920 haplotype classes in the various shade of eye color classes. Dark - black, brow or hazel and Light - blue or green. The total number of individuals counted within each class is shown on the bottom row, and the total number of individuals of each haplotype are shown in the last column.

**Table 8-2**

| Genotypes | G11=CG/CG | G12=CG/AG | G13=CG/CA | | | | |
|---|---|---|---|---|---|---|---|
| | G22=AG/AG | G23=AG/CA | G24=AG/AA | | | | |
| Eye colors | Genotypes | | | | | | |
| | G11 | G12 | G13 | G22 | G23 | G24 | Total |
| Light | 4 | 36 | 42 | 9 | 32 | 0 | 123 |
| Not-Light | 25 | 36 | 49 | 23 | 23 | 2 | 158 |
| Total | 29 | 72 | 91 | 32 | 55 | 2 | 281 |
| | | | | | | | |

Table 8-2. TYR2LOC920 genotype counts for the various classes of eye color shade. The genotype designations are shown at the top of the table. Not-light - black, brown or hazel and Light - blue or green. The total number of individuals counted within each class is shown on the bottom row, and the total number of individuals of each genotype are shown in the last column.

**Table 8-3**

| Haplotype\Eye color | Light | Not-light | Total |
|---|---|---|---|
| H1:ATA | 201 | 53 | 254 |
| H2:ATG | 106 | 43 | 149 |
| H3:ACG | 2 | 0 | 2 |
| H4:GCA | 51 | 31 | 82 |
| H5:GCG | 31 | 25 | 56 |
| H6:GTA | 3 | 6 | 9 |
| H7:GTG | 4 | 6 | 10 |
| Total | 398 | 164 | 562 |

Table 8-3. Individual OCA3LOC109 haplotype counts in the various classes of eye color shade. Dark - black, brown or hazel and Light - blue or green. The total number of individuals counted within each class is shown on the bottom row, and the total number of individuals of each haplotype are shown in the last column.

**Table 8-4**

| Genotype\Eye color | Light | Not-light | Total |
|---|---|---|---|
| G11:(ATA, ATA) | 47 | 11 | 58 |
| G12: (ATA, ATG) | 55 | 10 | 65 |
| G13: (ATA, ACG) | 1 | 0 | 1 |
| G14: (ATA, GCA) | 29 | 7 | 36 |
| G15: (ATA, GCG) | 16 | 6 | 22 |
| G16: (ATA, GTA) | 3 | 4 | 7 |
| G17: (ATA, GTG) | 3 | 4 | 7 |
| G22:(ATG, ATG) | 16 | 6 | 22 |
| G23: (ATG, ACG) | 1 | 0 | 1 |
| G24: (ATG, GCA) | 8 | 8 | 16 |
| G25: (ATG, GCG) | 10 | 10 | 20 |
| G26: (ATG, GTA) | 0 | 1 | 1 |
| G27: (ATG, GTG) | 0 | 2 | 2 |
| G44: (GCA, GCA) | 5 | 6 | 11 |
| G45: (GCA, GCG) | 3 | 4 | 7 |
| G47: (GCA, GTG) | 1 | 0 | 1 |
| G55: (GCG, GCG) | 1 | 2 | 3 |
| G56: (GCG, GTA) | 0 | 1 | 1 |
| Total | 199 | 82 | 281 |

Table 8-4. OCA3LOC109 genotype (diploid haplotype pair) classes in the various shade of eye color classes. Dark - black, brown or hazel and Light - blue or green. The total number of individuals counted within each class is shown on the bottom row, and the total number of individuals of each haplotype are shown in the last column.

**Table 8-5**

| Haplotype\Eye color | Dark | Light | Total |
|---|---|---|---|
| H1:CAC | 126 | 353 | 479 |
| H2: CGC | 30 | 45 | 75 |
| H3: TGC | 9 | 5 | 14 |
| H4: CGT | 1 | 5 | 6 |
| Total | 166 | 408 | 574 |

Table 8-5. Individual OCA3LOC920 haplotype classes in the various shade of eye color classes. Dark - black, brown or hazel and Light - blue or green. The total number of individuals counted within each class is shown on the bottom row, and the total number of individuals of each haplotype are shown in the last column.

**Table 8-6**

| Genotype\Eye color | Dark | Light | Total |
|---|---|---|---|
| G11:(CAC, CAC) | 50 | 151 | 201 |

(continued)

| Genotype\Eye color | Dark | Light | Total |
|---|---|---|---|
| G12:(CAC, CGC) | 19 | 42 | 61 |
| G 13:(CAC, TGC) | 6 | 5 | 11 |
| G14:(CAC, CGT) | 1 | 4 | 5 |
| G22:(CGC, CGC) | 4 | 1 | 5 |
| G23:(CGC, TGC) | 3 | 0 | 3 |
| G24:(CGC.CGT) | 0 | 1 | 1 |
| Total | 83 | 204 | 287 |

Table 8-6. OCA3LOC109 genotype (diploid haplotype pair) classes in the various shade of eye color classes. Dark - black, brown or hazel and Light - blue or green. The total number of individuals counted within each class is shown on the bottom row, and the total number of individuals of each haplotype are shown in the last column.

**Table 8-7**

| GENE | PARTITION | HAPLOTYPE SYSTEM | TEST STATISTICS |
|---|---|---|---|
| TYR | DARK+HAZ/LIGHT | TYR2LOC920 | HAPLOTYPE |
| OCA2 | DARK/LIGHT+HAZ | OCA3LOC109 | HAPLOTYPE |
| OCA2 | DARK/LIGHT+HAZ | OCA3LOC920 | HAPLOTYPE |
| TYRP | DARK/LIGHT+HAZ | TYRP3L05 | SNP |
| MC1R | DARK/LIGHT+HAZ | MCR3LOC106 | SNP |

Table 8-7. Summary of analyses at the level of the single gene haplotype system. The gene within which the haplotype system is found is shown in column one (GENE). The distinction of light and dark classes of eye color shade is shown in column 2 (PARTITION). The haplotype system is shown in column 3, and the level of complexity for which the statistically significant results were obtained is shown in column 4.

**Table 8-8**

| TYR2LOC920 | OCA3LOC 920 | OCA3LOC 109 | MCR3LOC105 | OCA3LOC 109 | TYRP3L106 | CLASS | COR R | INCLA SS | INCOR R |
|---|---|---|---|---|---|---|---|---|---|
| 1. AG/CA | CAC/CAC | | CCC/CYC | | GTT/GTT | DK/HAZ | 7 | 0 | 2 |
| 2. AG/CA | CAC/CAC | | CCC/CYC | | GTT/TTT | LT/HAZ/B1 | 6 | 0 | 0 |
| 3. AG/CA | CAC/CAC | | CCC/CYC | | GGA/GGT | INCONCL | 0 | 4 | 0 |
| 4. AG/CA | CAC/CAC | | CCC/CYC | | GGA/GTT | BLOND | 8 | 0 | 0 |
| 5. AG/CA | CAC/CAC | | CCC/CYC | | GGA/GGA | DK | 2 | 0 | 0 |
| 6. AG/CA | CAC/CAC | | CCC/CYC | | GGT/TGA | LT/HAZ | 4 | 0 | 0 |
| 7. AG/CA | CAC/CAC | | NOT CCC/CYC | | | LT/HAZ | 14 | 0 | 1 |
| 8. AG/CA | NGC/NNN | | CCC/CCY | | | LT/HAZ | 9 | 0 | 0 |
| 9. AG/CA | NGC/NNN | | CCC/CTC | | | DK/HAZ | 3 | 0 | 0 |
| 10. AG/CA | NGC/NNN | | OTHER | | | NOT OBS | 0 | 0 | 0 |
| 11. AG/CA | TNC/CNC | | | | | DK | 2 | 0 | 0 |
| 12. AG/CA | OTHER | | | | | INSUFF | 0 | 1 | 0 |
| | | | | | | TOTAL | 55 | 5 | 3 |
| | | | | | | | | | |
| 13. AG/AG | | | CCGCYC | ATA/ATR | GTT/KT7 | DK/HAZ | 3 | 0 | 0 |
| 14 AG/AG | | | CCC/CYC | ATA/ATR | GGNGKY | LT/HAZ | 5 | 0 | 0 |
| 15. ACJAG | | | CCCICYC | ATGIATG | | INCONCL | 0 | 4 | 0 |
| 16. AG/AG | | | CCC/CYC | GYR/ATR | | DK/HAZ | 7 | 0 | 1 |
| 17. AG/AG | | | CCG/CYC | OTHER | | LT/HAZ | 4 | 0 | 0 |
| 18 AG/AG | | | CCC/TCC | | | LT/HAZ | 5 | 0 | 0 |
| 19 AUAG | | | CCC/CCT | | | HAZ | 4 | 0 | 0 |
| 20 AG/AG | | | OTHER | | | NOT OBS | 0 | 0 | 0 |
| | | | | | | TOTAL | 28 | 4 | 1 |
| | | | | | | | | | |
| 21 CG/CG | CAC/YRC | | CCC/CCC | | | DK/HAZ | 13 | 0 | 0 |

(continued)

| TYR2LOC920 | OCA3LOC 920 | OCA3LOC 109 | MCR3LOC105 | OCA3LOC 109 | TYRP3L106 | CLASS | COR R | INCLA SS | INCOR R |
|---|---|---|---|---|---|---|---|---|---|
| 22 CGCG | CAC/YRC | | CCC/CTC | | | LT/HAZ | 4 | 0 | 0 |
| 23 CGCG | CAC/YRC | | OTHER | | | DK | 3 | 0 | 0 |
| 24 CG/CG | OTHER | | | | | DK | 3 | 0 | 0 |
| | | | | | | TOTAL | 23 | 0 | 0 |
| | | | | | | | | | |
| 25 CG/AG | | ATA/ATG | | | | LT/HAZ | 16 | 0 | 2 |
| 26 CG/AG | | ATG/GCG | | | | LT | 4 | 0 | 0 |
| 27 CG/AG | | ATA/ATA | CCC/CCC | | | LT/HAZ | 6 | 0 | 1 |
| 28 CG/AG | | ATA/ATA | OTHER | | | DK/HAZ | 5 | 0 | 0 |
| 29 CG/AG | | ATG/ATG | | | | INCONCL | 0 | 6 | 0 |
| 30 CG/AG | | GTA/ATA | | | | DK | 2 | 0 | 0 |
| 31 CG/AG | | GCG/GCG | | | | DK/HAZ | 1 | 0 | 0 |
| 32 CG/AG | | GCA/GCA | CCC/CCC | | | LT | 3 | 0 | 0 |
| 33 CG/AG | | GCA/GCA | OTHER | | | DK | 1 | 0 | 0 |
| 34 CG/AG | | GCA/ATA | CCC/CCC | | | DK | 4 | 0 | 0 |
| 35 CG/AG | | GCA/ATA | CCC/CTC | | | INCONCL | 0 | 3 | 0 |
| 36 CG/AG | | GCA/ATA | CCC/CCT | | | LT | 1 | 0 | 0 |
| 37. CG/AG | | OTHER | | | | NOT OBS | 0 | 0 | 0 |
| | | | | | | TOTAL | 43 | 9 | 3 |
| | | | | | | | | | |
| 38. CG/CA | | ATA/ATA | CCC/YYC | | | LT/HAZ | 15 | 0 | 0 |
| 39. CG/CA | | ATA/ATA | OTHER | | | INCONCL | 0 | 4 | 0 |
| 40. CG/CA | | ATA/ATG | CCC/YYC | | | LT/HAZ | 13 | 0 | 1 |
| 41. CG/CA | | ATA/ATG | CCC/CCT | | | INCONCL | 0 | 4 | 0 |
| 42. CG/CA | | ATA/ATG | OTHER | | | NOT OBS | 0 | 0 | 0 |

(continued)

| TYR2LOC920 | OCA3LOC 920 | OCA3LOC 109 | MCR3LOC105 | OCA3LOC 109 | TYRP3L106 | CLASS | COR R | INCLA SS | INCOR R |
|---|---|---|---|---|---|---|---|---|---|
| 43. CG/CA | | ATG/ATG | | | | LT/HAZ | 7 | 0 | 0 |
| 44. CG/CA | | ATA/GCA | | | | LT/HAZ | 20 | 0 | 0 |
| 45. CG/CA | | GCA/GCA | | | | INCONCL | 0 | 2 | 0 |
| 46. CG/CA | | ATG/GCG | | | | INCONCL | 0 | 4 | 0 |
| 47 CG/CA | | ATGIACG | | | | INCONCL | 0 | 1 | 0 |
| 48 CG/CA | | GCA/GCG | | | | DK/HAZ | 4 | 0 | 0 |
| 49. CG/CA | | OTHER | | | | NOT OBS | 0 | 0 | 0 |
| | | | | | | TOTAL | 59 | 15 | 1 |
| | | | | | | | | | |
| ALL CLASSES | | | | | | TOTAL | 208 | 33 | 8 |
| | | | | | | | | | |
| | | | | | | TOTAL* | 96% | | 3% |

Table 8-8. Classification tree incorporating haplotype systems described herein to categorize individuals as dark or light eye individuals.

**Table 8-9**

| CONDITION I | CONDITION 2 | CONDITION 3 | CONDITION 4 | P VALUE | N |
|---|---|---|---|---|---|
| | | | | | |
| 1) TYR2LOC920 AG/CA | OCA3LOC920 CAC/CAC | MCR3LOC106 CCC/CYC | TYRP3L105 | P<0.00 +/-0.001 | 33 |
| 2) TYR2LOC920 AG/CA | OCA3LOC920 CAC/CAC | MCR3LOC106 OTHER | | P=0.027 +/- 0.014 | 14 |
| 3) TYR2LOC920 AG/CA | OCA3LOC920 YGC/CRC | MCR3LOC106 | | P<0.001 +/-0.001 | 14 |
| | | | | | |
| 4) TYR2LOC920 AG/AG | MCR3LOC106 CCC/CYC | OCA3LOC109 ATA/ATR | TYRY3L105 | P=0.045+/-0.024 | 8 |
| 5) TYR2LOC920 AG/AG | MCR3LOC106 CCC/CYC | OCA3LOC109 OTHER | | P=INCALC | 13 |
| 6) TYR2LOC920 AG/AG | MCR3LOC106 OTHER | | | P=0.027+/-0.014 | 9 |
| | | | | | |
| 7) TYR2LOC920 CG/CG | OCA3LOC920 YRC/CAC | MCR3LOC106 | | P<0.001+/-0.001 | 20 |
| 8) TYR2LOC920 CG/CG | OCA3LOC920 OTHER | | | P=INCALC | 3 |
| | | | | | |
| 9) TYR2LOC920 CG/AG | OCA3LOC109 ATA/ATA | MCR3LOC106 | | P-INCALC | 13 |
| 10) TYR2LOC920 CG/AG | OCA3LOC109 GCA/GCA | MCR3LOC106 | | P-INCALC | 4 |
| 11) TYR2LOC920 CG/AG | OCA3LOC109 GCA/ATA | MCR3LOC106 | | P-INCALC | 8 |
| 12) TYR2LOC920 CG/AG | OCA3LOC109OTHER | | | P-0.045 +/- 0.015 | 58 |
| | | | | | |
| 13) TYR2LOC920 CG/CA | OCA3LOC109 ATA/ATA | MCR3LOC106 | | P-INCALC | 19 |
| 14) TYR2LOC920 CG/CA | OCA3LOC109 ATA/ATG | MCR3LOC106 | | P-INCALC | 18 |
| 15) TYR2LOC920 CG/CA | OTHER | | | P-0.018+-0.018 | 42 |
| | | | | | |
| TOTAL | | | | | 276 |

Table 8-9. Effect statistics for the formulation of classification tree rules shown in Table 8-8.

**Table 8-10**

| | SOLUTION RESULTS | |
|---|---|---|
| | COUNT | PERCENT |
| CORRECT | 208 | 96.30% |
| INCORRECT | 8 | 3.70% |

Table 8-10. Final counts from the classification solution of Table 8-8.

## EXAMPLE 9

## CLASSIFICATION MODEL EYE COLOR ANALYSIS

The following example further discusses the classification model presented in Example 8, that generated the preferred eye color solution involving optimal haplotype systems for four different genes, described therein. Our goal was to develop a classification solution for human eye color. About 300 Caucasians of variable eye color were genotyped for an average of 30 SNP markers in 5 genes known to be involved in melanin production. The results showed that alleles of SNPs in the TYR, TYRP1, OCA2 and MC1R genes showed statistical associations with certain human eye colors and/or shades, as discussed in Example 8. However, the relationship between allele and eye color/shade was one of bias. Though the associations between SNP alleles and eye color/shade were statistically significant, on their own, the markers make for poor predictive tools because the error rate of classification is too high. This suggested that the discovered SNPs were component pieces of a larger, more complex puzzle.

Given what is known about the inheritance of eye color, this is not an unreasonable hypothesis. Specifically, eye color is a complex trait, not a simple Mendelian trait. Although there is an element of dominance for darker eye colors, knowing the eye color of a mother and father do not allow one to predict with accuracy the eye color of the children. This is because eye color is a function of multiple genes interacting among themselves, rather than a single gene. Given that a collection of SNPs that were informative for human eye color had been identified, the SNPs were considered in terms of both inter and intra-genic complexity.

To perform this, the best combination of markers within each of the genes for explaining eye color, were identified. In the next step (see below) these optimal haplotype systems for each of the four genes were combined in an inter-genic analysis to develop the final solution.

Step 1. Intra-genic complexity.

For each of these four genes, random SNP (marker) combinations were selected to constitute a haplotype system. For each haplotype system, raw genotypes were converted into haplotypes using computational inference (Stephens and Donnelly, 2000), and individuals were grouped into one of two groups of eye shade; light (blue, green, gray or hazel eyes) or dark (light brown, medium brown, dark brown or black eyes). To test for population structure differences between these groups, a pair-wise F-statistic (or in some cases, a Fishers exact test of sample differentiation) was calculated . The F statistic is based on genetic distances for short divergence time. The Exact test of population differentiation tests the non-random distribution of haplotypes into population samples under the hypothesis of panmixia. P-values calculated from these tests were stored. The process was repeated until all of the possible haplotype systems for the gene were tested. At this point, the haplotype systems showing the lowest P-values were selected for further analysis.

For example, the OCA2 gene had 19 SNPs with alleles that were biased for one of the two classes of eye shade (for a list of the SNPs identified in this Example as having predictive value for human eye color, see Table 9-1). Using this approach several haplotype systems were identified that each had predictive value for human eye color. The haplotype systems used for this work are defined, in order from left to right, as follows:

| | |
|---|---|
| TYR2LOC920 | Markers 217468, 217473 |
| OCA3LOC920 | Markers 217452, 217455, 712061 |
| OCA3LOC109 | Markers 217458, 712054, 886896 |
| MCR3LOC106 | Markers 217438, 217439, 217441 |

For a description of each of these SNPs (Markers), please see Example 10 below. The markers arc also included in the comprehensive list of claimed SNPs in Table I.

As discussed in Example 10, the TYR2LOC920 and OCA3LOC109 haplotype I systems arc especially informative. Persons of dark eye color tend to have different haplotypes, and diploid combinations of haplotypes (haplotype pairs) than persons of lighter eye color as measured by the pair-wise F statistic. The P value for these statistics is shown below in Table 9-2. For the TYRP and MCIR systems which did not have p values that indicated statistical significance, analysis was continued despite this because their component alleles, found to be associated with darker eye colors, were more frequently found in (indeed, they were practically monomorphic in) persons of African American or Asian descent. Because the average eye color of these ethnic groups is darker than Caucasians, and due to the nature of the gene in which the SNPs occur, the markers may be useful eye color markers *on a complex genetic level.* Indeed, this turned out to be the case (see Table 8-8).

Step 2. Inter-genic complexity.

Once the interesting haplotype systems had been defined for each gene, Classification rules based on these haplotype systems were then developed using a nested statistical approach (see Example 12). First, individuals were stratified based on their genotype at the TYR2LOC920 haplotype system. For example, individuals with CG/CA genotype were segregated from the rest. If all or most of these individuals were blue, green, hazel, brown, light (blue or green) or dark (brown or hazel) eye individuals (as measured using a pair-wise F statistic), a rule was formulated stating that if an individual had the TYR2LOC920 CG/CA genotype, they belonged to the appropriate eye color class. It so happens, that this rule was not possible to make. Therefore, individuals within the TYR2LOC920 CG/CA class were partitioned based on their genotypes for several other haplotype systems (randomly selected) and a pair-wise F statistic test was used to determine whether there was population structure differences between individuals of the various new compound genotypes and the various eye color classes. The haplotype system that showed the best ability to partition the subjects based on eye color was selected. For the OCA3 gene, this haplotype system happened to be the OCA3LOC109 system (P=0.018 +/-0.018). For many OCA3LOC109 genotypes within the TYR2LOC106 CG/CA class it was possible to construct classification rules. For example, 7 of 7 individuals with the TYR2LOC106 CG/CA genotype and OCA3LOC109 ATG/ATG genotype (see Table 8-8) were of light eyes. This number is statistically significant. Therefore, we constructed a rule stating that if a person is found to have this compound genotype, they can be classified into the light eye group. For other OCA3LOC109 genotypes within this TYR2LOC920 class, it was not possible to make rules, so a third term was added to the model in the same manner as was the second term. It so happens that the best haplotype system for resolving TYR2LOC920 CG/CA: OCA3LOC109 ATA/ATA individuals, based on eye color, was the MCR3LOC105 haplotype system; 15 of 15 individuals with the TYR2LOC920 CG/CA : OCA3LOC109 ATA/ATA : MCR3LOC105 CCC/YYC compound genotype class were of light or hazel eyes. Thus, a rule was formed form this observation.

All of the rules, formulated in the above manner, appear in the classification tree presented as Table 8-8. Each classification results from a statistical decision. The effect statistics for these decisions are presented in the classification tree that is presented as Table 8-10. The tree follows the same format shown in Table 8-8, and shows the pair-wise F-statistic P values used within a compound genotype class to infer genetic structure differences between groups of individuals of different eye colors. The ability to partition individuals within a compound genotype class in a manner that is statistically significant is used as justification by which to formulate classification rules for particular genotypes within the compound system (see Table 8-8).

The tree in Table 8-10 is read from left to right. Within a column, the haplotype system is listed and the genotype class for that system appears to the immediate right. Individuals of a given class within the haplotype system identified in a column are partitioned into genotype classes for the next haplotype system to the right (if any). If individuals within this new compound genotype class can be partitioned into subgroups, based on eye color shade (described in the text), that arc statistically distinct with regard to haplotype composition (using a pair-wise F-statistic test), the process terminates along a row at the relevant P value for the test. If not, this process continues to the next haplotype system to the right. When (or if) statistical significance is achieved, the compound genotypes arc used to construct classification rules (shown in Table 8-8) for the pertinent individuals.

For example, considering rows one through three, there is no statistical association between OCA3LOC920 genotypes and eye color within the class of individuals with a TYR2LOC920 AG/CA genotype. Thus, the path leads to the MCR3LOC106 haplotype system for individuals of each compound TYRP2LOC920:OCA2LOC920 class. For the example shown in row two, there were statistically significant differences in the MCR3LOC106 haplotype composition between light (blue, green) and dark eye (brown or black) individuals within the compound TYR2LOC920 AG/CA, OCA2LOC920 CAC/CAC genotype class (P<0.001 +/- 0.001, n=33). Thus, classification rules were constructed for individuals of particular compound TYR2LOC920:OCA3LOC920:MCR3LOC106 genotypes.

For some of the haplotypes (listed as "P=INCALC") the P value was not calculable. The most common reason for this is genetic homogeneity within one or both of the eye color classes for the compound genotype in question. The pair-wise method measures the average number of differences within groups compared to that number between groups, and this genetic homogeneity within the final haplotype system of a compound class makes the calculation of the within group difference impossible.

The combined solution tree described in Table 8-8 and Table 8-10 results in the correct classification of 208 individuals, the incorrect classification of 8 individuals, and an inconclusive result for 33 individuals (see Table 8-9). Thus, the solution has an accuracy rate of 96%, which makes it a useful tool for predicting human eye color from DNA.

**TABLE 9-1**

**SNPS WITH ALLELES THAT SEGREGATE PREFERENTIALLY IN EITHER DARK OR LIGHT EYE COLOORED CAUCASIANS:**

**1.**

| GENE | SNPNAME | MARKER | LOCATION | GENBANK | INTEGRITY |
|---|---|---|---|---|---|
| OCA2 | OCA2_2 | 217452 | 17264 | 13651545 | POLY |

217452 OCA2_2

| | CC | CT | TT |
|---|---|---|---|
| BRN | 28 | 0 | 0 |
| HAZL | 25 | 0 | 0 |
| GRN | 17 | 0 | 0 |
| BLUE | 39 | 0 | 2 |

JUSTIFICATION: This SNP is part of the OCA3LOC920 haplotype system, the utility of which has been demonstrated in the text elsewhere in this patent. It can be seen from this distribution that only blue eyed individuals carry the T allele.

**2.**

| GENE | SNPNAME | MARKER | LOCATION | GENBANK | INTEGRITY |
|---|---|---|---|---|---|
| OCA2 | OCA2_5 | 217455 | 21103 | 13651545 | POLY |

217455 OCA2_5

| | AA | GA | GG |
|---|---|---|---|
| BRN | 19 | 9 | 0 |
| HAZL | 18 | 7 | 1 |
| GRN | 13 | 4 | 0 |
| BLUE | 23 | 11 | 0 |

JUSTIFICATION: This SNP is part of the OCA3LOC109 and OCA3LOC920 haplotype systems, the utility of which has been demonstrated in the text elsewhere in this patent. As can be seen from this distribution, the G allele is enriched for individuals of darker (brown and hazel) eye color. In particular, green eyed individuals rarely carry the G allele.

**3.**

| GENE | SNPNAME | MARKER | LOCATION | GENBANK | INTEGRITY |
|---|---|---|---|---|---|
| OCA2 | OCA2_6 | 217456 | 26558 | 13651545 | POLY |

217456 OCA2_6

| | AA | GA | GG |
|---|---|---|---|
| BRN | 0 | 4 | 22 |
| HAZL | 0 | 4 | 19 |
| GRN | 0 | 1 | 14 |
| BLUE | 0 | 2 | 27 |

JUSTIFICATION: As can be seen from this distribution, the frequency of the A allele is greater in individuals with darker eye colors than lighter (blue and green). The ratio of genotypes AA:GA:GG in dark eyed individuals (Brown and Hazel) is 0:8:41, but only):3:41 for light (blue and green) individuals.

**4.**

| GENE | SNPNAME | MARKER | LOCATION | GENBANK | INTEGRITY |
|---|---|---|---|---|---|
| *OCA2* | *OCA2_8* | *217458* | *86316* | *13651545* | *POLY* |

217458 OCA2_8

| | CC | CT | TT |
|---|---|---|---|
| BRN | 2 | 14 | 13 |
| HAZL | 2 | 10 | 13 |
| GRN | 1 | 7 | 10 |
| BLUE | 3 | 14 | 24 |

JUSTIFICATION: The C allele is enriched in individuals of darker (brown and hazel) eye color relative to light. The ratio of CC:CT:TT genotypes in the former group is 4:24:26 but only 4:21:34 in the latter group.

**5.**

| GENE | SNPNAME | MARKER | LOCATION | GENBANK | INTEGRITY |
|---|---|---|---|---|---|
| *OCA2* | *OCA2_RS1800405* | *712061* | *21161* | *13651545* | *POLY* |

JUSTIFICATION: This SNP is part of the OCA3LOC920 haplotype system, the utility of which was demonstrated in the text.

**6.**

| GENE | SNPNAME | MARKER | LOCATION | GENBANK | INTEGRITY |
|---|---|---|---|---|---|
| OCA2 | OCA2_RS1800414 | 712064 | 101492 | 13651545 | POLY |

712064 OCA2_RS1800414

| | AA | GA | GG |
|---|---|---|---|
| BRN | 26 | 1 | 0 |
| HAZL | 23 | 0 | 0 |
| GRN | 15 | 0 | 0 |
| BLUE | 40 | 0 | 0 |

JUSTIFICATION: Only individuals of brown eye color carry the G allele, which appears to be quite rare.

**7.**

| GENE | SNPNAME | MARKER | LOCATION | GENBANK | INTEGRITY |
|---|---|---|---|---|---|
| OCA2 | OCA2DBSNP_52401 | 712052 | 52401 | 13651545 | POLY |

712052 OCA2DBSNP_52401

| | AA | GA | GG |
|---|---|---|---|
| BRN | 17 | 15 | 1 |
| HAZL | 17 | 10 | 2 |
| GRN | 12 | 5 | 0 |
| BLUE | 28 | 14 | 2 |

JUSTIFICATION: The G allele is more frequently found in individuals of darker (brown and hazel) eye color than lighter eye color. The ratio of AA:GA:GG genotypes in the dark group is 34:25:3, but only 40:19:2 in the light group.

**8.**

| GENE | SNPNAME | MARKER | LOCATION | GENBANK | INTEGRITY |
|---|---|---|---|---|---|
| OCA2 | OCA2DBSNP_98488 | 712058 | 98488 | 13651545 | POLY |

712058 OCA2DBSNP_98488

(continued)

| | AA | GA | GG |
|---|---|---|---|
| BRN | 0 | 8 | 14 |
| HAZL | 0 | 6 | 20 |
| GRN | 0 | 4 | 10 |
| BLUE | 1 | 3 | 37 |

JUSTIFICATION: The ratio of AA:GA:GG genotypes in dark eyed individuals (brown and hazel) is 0:14:34, but 1:7:47 in lights showing that the A allele is more frequent in the dark group. This SNP is part of the OCA3LOC109 haplotype system described in the text.

**9.**

| GENE | SNPNAME | MARKER | LOCATION | GENBANK | INTEGRITY |
|---|---|---|---|---|---|
| OCA2 | OCA2DBSNP_146405 | 712054 | 146405 | 13651545 | POLY |

712054 OCA2DBSNP_146405

| | AA | GA | GG |
|---|---|---|---|
| BRN | 12 | 12 | 7 |
| HAZL | 15 | 6 | 5 |
| GRN | 4 | 9 | 4 |
| BLUE | 15 | 22 | 2 |

JUSTIFICATION: The ratio of AA:GA:GG genotypes in the dark (brown and hazel) group is 27:18:12 but is 19:31:6 in the light group showing that the G allele is more frequently found in the light eye group.

**10.**

| GENE | SNPNAME | MARKER | LOCATION | GENBANK | INTEGRITY |
|---|---|---|---|---|---|
| OCA2 | OCA2DBSNP_8321 | 712057 | 8321 | 13651545 | POLY |

712057 OCA2DBSNP_8321

| | GG | GT | TT |
|---|---|---|---|
| BRN | 19 | 11 | 3 |
| HAZL | 16 | 13 | 0 |
| GRN | 14 | 3 | 0 |
| BLUE | 34 | 10 | 0 |

JUSTIFICATION: The GG:GT:TT genotype ratio in the dark group is 35:24:3, but 48:13:0 showing that the T allele is much more frequently found in the dark group. This SNP is part of the OCA3LOC109 haplotype system described in the text of the application.

**11.**

| GENE | SNPNAME | MARKER | LOCATION | GENBANK | INTEGRITY |
|---|---|---|---|---|---|
| OCA2 | OCA2E11_263 | 886895 | 26692 | 1365145 | POLY |

886895 OCA2E 11_263

| | AA | AG | GG |
|---|---|---|---|
| BRN | 19 | 8 | 0 |
| HAZL | 23 | 7 | 0 |
| GRN | 11 | 4 | 0 |
| BLUE | 40 | 5 | 2 |

JUSTIFICATION: The ratio of AA:AG:GG genotypes in the dark eye group is 42:15:0 and 51:9:2 in the light group. Though this docs not seem to be too different, this SNP is part of the OCA3LOC109 haplotype system, the utility of which was described in the text.

**12.**

| **GENE** | **SNPNAME** | **MARKER** | **LOCATION** | **GENBANK** | **INTEGRITY** |
|---|---|---|---|---|---|
| *OCA2* | *OCA2E11_350* | *886896* | *26779* | *1365145* | *POLY* |

886896 OCA2E11_350

| | AA | AG | GG |
|---|---|---|---|
| BRN | 6 | 20 | 2 |
| HAZL | 16 | 12 | 2 |
| GRN | 10 | 4 | 1 |
| BLUE | 31 | 13 | 3 |

JUSTIFICATION: The ratio of AA:AG:GG genotypes is 22:32:4 for dark hair individuals but only 41:17:4 for the light group. The frequency of the G allele is therefore greater in the dark eye group. This SNP is part of the OCA3LOC109 haplotype system, the utility of which was demonstrated in the text.

**13.**

| **GENE** | **SNPNAME** | **MARKER** | **LOCATION** | **GENBANK** | **INTEGRITY** |
|---|---|---|---|---|---|
| OCA2 | OCA2E14_447 | 886894 | 95957 | 1365145 | POLY |

886894 OCA2E14_447

| | CC | CT | TT |
|---|---|---|---|
| BRN | 1 | 16 | 11 |
| HAZL | 2 | 13 | 16 |
| GRN | 0 | 5 | 10 |
| BLUE | 3 | 11 | 13 |

JUSTIFICATION: The ratio of CC:CT:TT genotypes in dark eye individuals (brown and hazel) is 3:34:27 but only 3: 11:13 in light eye individuals. The frequency of the C allele is therefore greater in the dark eye group (more heterozygotes relative to TT homozygotes).

**14.**

| **GENE** | **SNPNAME** | **MARKER** | **LOCATION** | **GENBANK** | **INTEGRITY** |
|---|---|---|---|---|---|
| OCA2 | OCA2E16_300 | 886892 | 101644 | 1365145 | POLY |

886892 OCA2E 16_300

| | GG | GC | CC |
|---|---|---|---|
| BRN | 28 | 0 | 0 |
| HAZL | 30 | 0 | 0 |
| GRN | 14 | 0 | 0 |
| BLUE | 43 | 0 | 1 |

JUSTIFICATION: The C allele is only found in persons of blue eye color.

**15.**

| **GENE** | **SNPNAME** | **MARKER** | **LOCATION** | **GENBANK** | **INTEGRITY** |
|---|---|---|---|---|---|
| OCA2 | OCA2E10_102 | 886993 | 25083 | 1365145 | POLY |

886993 OCA2E10_102

| | AA | AG | GG |
|---|---|---|---|
| BRN | 0 | 7 | 13 |
| HAZL | 2 | 4 | 17 |
| GRN | 0 | 1 | 13 |
| BLUE | 0 | 6 | 33 |

(continued)

| | AA | AG | GG |
|---|---|---|---|

JUSTIFICATON: The ratio of AA:AG:GG genotypes in individuals of dark eye color is 2:11:30, but only 0:7:46 in persons of light eye color. Therefore the frequency of the A allele is greater in persons of darker eye color.

**16.**

| GENE | SNPNAME | MARKER | LOCATION | GENBANK | INTEGRITY |
|---|---|---|---|---|---|
| OCA2 | OCA2E10_549 | 886994 | 25519 | 1365145 | POLY |

886994 OCA2E10_549

| | CC | CA | AA |
|---|---|---|---|
| BRN | 0 | 11 | 16 |
| HAZL | 2 | 5 | 22 |
| GRN | 0 | 1 | 14 |
| BLUE | 0 | 8 | 37 |

JUSTIFICATION: The ratio of CC:CA:AA genotypes in persons of darker eye color is 2:16:38 but only 0:9:51 in persons of lighter eye color. Therefore, the C allele is more frequently found in persons of darker eye color.

**17.**

| GENE | SNPNAME | MARKER | LOCATION | GENBANK | INTEGRITY |
|---|---|---|---|---|---|
| TYR | TYR_3 | 217468 | 656 | AP000720 | POLY |

217468 TYR_3

| | CC | CA | AA |
|---|---|---|---|
| BRN | 10 | 13 | 7 |
| HAZL | 14 | 9 | 2 |
| GRN | 3 | 12 | 2 |
| BLUE | 16 | 21 | 2 |

JUSTIFICATION: The ratio of CC:CA:AA genotypes is 24:21:9 in persons of darker eye color, but 19:33:4 in persons of lighter eye color. Therefore, the frequency of the A allele is greater in persons of lighter eye color.

**18.**

| GENE | SNPNAME | MARKER | LOCATION | GENBANK | INTEGRITY |
|---|---|---|---|---|---|
| TYR | TYRSNP_7 | 217472 | 37266 | AP000720 | POLY |

19.

| GENE | SNPNAME | MARKER | LOCATION | GENBANK | INTEGRITY |
|---|---|---|---|---|---|
| TYR | TYRSNP_8 | 217473 | 77771 | AP000720 | POLY |

217473TYRSNP_8

| | AA | GA | GG |
|---|---|---|---|
| BRN | 0 | 18 | 20 |
| HAZL | 0 | 19 | 21 |
| GRN | 0 | 13 | 12 |
| BLUE | 0 | 33 | 29 |

JUSTIFICATION: The frequency of AA:GA:GG genotypes in persons of dark eye color (brown and hazel) is 0:37:41, but 0:46:41 in persons of light eye color. Thus, the frequency of the A allele is slightly higher in persons of light eye color.

**20.**

| GENE | SNPNAME | MARKER | LOCATION | GENBANK | INTEGRITY |
|---|---|---|---|---|---|
| TYR | TYRE3_358 | 951497 | 37434 | AP000720 | POLY |

951497 TYRE3_358

| | AA | GA | GG |
|---|---|---|---|
| BRN | 0 | 6 | 21 |
| HAZL | 0 | 10 | 20 |
| GRN | 0 | 2 | 13 |
| BLUE | 2 | 3 | 41 |

JUSTIFICATION: The ratio of AA:GA:GG genotypes in persons of darker eye color (brown and hazel) is 0:16:41 but 2:5:54 in persons of lighter eye color. The heterozygous GA state is more frequently found in persons of darker eye color.

**21.**

| GENE | SNPNAME | MARKER | LOCATION | GENBANK | INTEGRITY |
|---|---|---|---|---|---|
| MC1R | MC1R_4 | 217438 | 442 | X67594 | POLY |

217438 MCIR_4

| | CC | CT | TT |
|---|---|---|---|
| BRN | 28 | 4 | 0 |
| HAZL | 26 | 2 | 0 |
| GRN | 16 | 1 | 0 |
| BLUE | 37 | 4 | 0 |

JUSTIFICATION: The ratio of CC:CT:TT genotypes in persons of darker eye color is 54:6:0 and 53:5:0 in persons of lighter eye color, which is not significantly different. However, this SNP is part of the MCR3LOC105 haplotype system, the utility of which was discussed in the text.

**22.**

| GENE | SNPNAME | MARKER | LOCATION | GENBANK | INTEGRITY |
|---|---|---|---|---|---|
| MC1R | MC1R_5 | 217439 | 619 | X67594 | POLY |

217439 MC1R_5

| | CC | CT | TT |
|---|---|---|---|
| BRN | 28 | 4 | 0 |
| HAZL | 24 | 4 | 0 |
| GRN | 16 | 0 | 0 |
| BLUE | 35 | 6 | 0 |

JUSTIFICATION: This SNP is part of the MCR3LOC105 haplotype system, the utility of which was discussed in the text.

**23.**

| GENE | SNPNAME | MARKER | LOCATION | GENBANK | INTEGRITY |
|---|---|---|---|---|---|
| MC1R | MC1R_6 | 217440 | 632 | X67594 | POLY |

JUSTIFICATION: This SNP is only found to be a variant in African Americans, and absent in Caucasians, and the former have darker mean eye color than the latter.

**24.**

| GENE | SNPNAME | MARKER | LOCATION | GENBANK | INTEGRITY |
|---|---|---|---|---|---|
| MC1R | MC1R_7 | 217441 | 646 | X67594 | POLY |

217441 MC1R_5

(continued)

| | CC | CT | TT |
|---|---|---|---|
| BRN | 27 | 4 | 0 |
| HAZL | 24 | 4 | 0 |
| GRN | 11 | 6 | 0 |
| BLUE | 36 | 5 | 0 |

JUSTIFICATION: This SNP is part of the MCR3LOC105 haplotype system, the utility of which was described in the text.

**25.**

| GENE | SURNAME | MARKER | LOCATION | GENBANK | INTEGRITY |
|---|---|---|---|---|---|
| MCIR | MCIR_14 | NULL | 1048 | X67594 | POLY |

JUSTIFICATION: This SNP is only found to be a variant in African Americans, and absent in Caucasians, and the former have darker mean eye color than the latter.

**26.**

| GENE | SNPNAME | MARKER | LOCATION | GENBANK | INTEGRITY |
|---|---|---|---|---|---|
| MC1R | MCIR_15 | 217450 | 1272 | X67594 | POLY |

JUSTIFICATION: This SNP is only found to be a variant in African Americans, and absent in Caucasians, and the former have darker mean eye color than the latter.

**27.**

| GENE | SNPNAME | MARKER | LOCATION | GENBANK | INTEGRITY |
|---|---|---|---|---|---|
| TYRP | TYRP_3 | 217485 | 21693 | AF001295 | POLY |

217485 TYRP_3

| | GG | GT | TT |
|---|---|---|---|
| BRN | 6 | 7 | 7 |
| HAZL | 1 | 11 | 9 |
| GRN | 1 | 5 | 4 |
| BLUE | 2 | 10 | 11 |

JUSTIFICATION: The ratio of GG:GT:TT genotypes is 7:18:16 in persons of darker eye color (brown and hazel) but 3:15:15 in persons of lighter eye color. The GG genotype is therefore more frequently found in persons of darker eye color.

**28.**

| GENE | SNPNAME | MARKER | LOCATION | GENBANK | INTEGRITY |
|---|---|---|---|---|---|
| TYRP | TYRP_4 | 217486 | 21970 | AF001295 | POLY |

217486 TYRP_4

| | AA | AT | TT |
|---|---|---|---|
| BRN | 4 | 12 | 6 |
| HAZL | 1 | 12 | 10 |
| GRN | 2 | 10 | 4 |
| BLUE | 0 | 16 | 18 |

JUSTIFICATION: The ratio of AA:AT:TT genotypes is 5:24:16 in persons of darker eye color (brown and hazel) but 2:26:22 in person of lighter eye color. Thus, the frequency of the A allele is greater in persons of darker eye color.

**29.**

| GENE | SNPNAME | MARKER | LOCATION | GENBANK | INTEGRITY |
|---|---|---|---|---|---|
| TYRP | TYRP1_7 | 217489 | 22470 | AF001295 | POLY |

217489 TYRP_7

| | CC | CT | TT |
|---|---|---|---|
| BRN | 7 | 5 | 0 |
| HAZL | 6 | 0 | 0 |
| GRN | 2 | 2 | 2 |
| BLUE | 12 | 4 | 0 |

JUSTIFICATION: The ratio of CC:CT:TT genotypes in persons of darker eye color (brown and hazel) is 13:5:0 but 14:6:2 in light eye persons. Thus, the frequency of the T allele is greater in persons of lighter eyes.

**30.**

| GENE | SNPNAME | MARKER | LOCATION | GENBANK | INTEGRITY |
|---|---|---|---|---|---|
| TYRP | TYRPIEIE2_357 | 869787 | 6824 | AF001295 | POLY |

JUSTIFICATION: This SNP is only found to be a variant in African Americans, and absent in Caucasians, and the former have darker mean eye color than the latter.

**31.**

| GENE | SNPNAME | MARKER | LOCATION | GENBANK | INTEGRITY |
|---|---|---|---|---|---|
| TYRP | TYRPIEIE2-5_38 | 869743 | 5695 | AF001295 | POLY |

JUSTIFICATION: This SNP is only found to be a variant in African Americans, and absent in Caucasians, and the former have darker mean eye color than the latter.

**32.**

| GENE | SNPNAME | MARKER | LOCATION | GENBANK | INTEGRITY |
|---|---|---|---|---|---|
| TYRP | TYRP1E1E2-5_307 | 869745 | 5964 | AF001295 | POLY |

JUSTIFICATION: This SNP is only found to be a variant in African Americans, and absent in Caucasians, and the former have darker mean eye color than the latter.

**33.**

| GENE | SNPNAME | MARKER | LOCATION | GENBANK | INTEGRITY |
|---|---|---|---|---|---|
| TYRP | TYRP1 E4_32 | 886933 | 10739 | AF001295 | POLY |

886933 TYRP1E4_32

| | CC | CT | TT |
|---|---|---|---|
| BRN | 0 | 2 | 26 |
| HAZL | 0 | 3 | 28 |
| GRN | 0 | 0 | 15 |
| BLUE | 0 | 2 | 45 |

JUSTIFICATION: The ratio of CC:CT:TT genotypes in persons of darker eye color is 0:5:54 but 0:2:60 in lighter eye persons, demonstrating that the C allele is slightly more frequent in persons of darker eye color.

**34.**

| GENE | SNPNAME | MARKER | LOCATION | GENBANK | INTEGRITY |
|---|---|---|---|---|---|
| TYRP | TYRP1E4_499 | 886937 | 11204 | AF001295 | POLY |

886937 TYRP1E4_499

| | GG | GT | TT |
|---|---|---|---|
| BRN | 26 | 2 | 0 |
| HAZL | 27 | 4 | 0 |

(continued)

| | GG | GT | TT |
|---|---|---|---|
| GRN | 12 | 3 | 0 |
| BLUE | 43 | 4 | 0 |

JUSTIFICATION: The ratio of GG:GT:TT genotypes in persons of darker eye color is 53:6:0 but 55:7:0 in lighter eye persons. Though not significantly different, this SNP is part of the TYR3L105 haplotype system, the utility of which was described in the text.

**35.**

| GENE | SNPNAME | MARKER | LOCATION | GENBANK | INTEGRITY |
|---|---|---|---|---|---|
| TYRP | TYRPIE6-354 | 886938 | 17112 | AF001295 | POLY |

**Table 9-2**

| GENE | DMSION | HAPLOTYPE SYSTEM | FST P VALUE |
|---|---|---|---|
| TYR | DARK+HAZ/LIGHT | TYR2LOC920 | P=0.064 |
| OCA2 | DARK/LIGHT+HAZ | OCA3LOC109 | P<0.001 |
| OCA2 | DARK/LIGHT+HAZ | OCA3LOC920 | P=0.001 |
| TYRP | DARK/LIGHT+HAZ | TYRP3L05 | P=HNSIG |
| MC1R | DARK/LIGHT+HAZ | MCR3LOC106 | P=INSIG |

A lower P value indicates the haplotype system is especially useful for predicting eye color. INSIG means the P value was not statistically significant, but in the case of TYRP3L105 and MCR3LOC106 systems, it was close.

**EXAMPLE 10**

**FURTHER ANALYSIS OF HAPLOTYPES**

This example provides further analysis of the single haplotype systems discussed in Examples 8 and 9, and analysis of new combinations of these haplotypes using classification approaches other than the nested statistical approach. The data in Table 9-1 provides the relative value of each individual haplotype system for resolving individuals of the two main eye color classes (light = blue or green and dark = brown or black). These were the best haplotype systems that were identified in our analysis of Examples 8-9, within each of the four genes, as measured using the F-statistic P value for haplotypic differentiation between the two groups (DIVISION in Table 9-1), and as indicated by their contribution towards the best compound/complex genetic solution for human eye color (Table 8-8). For some genes, such as OCA2, we observed several other haplotype systems that are almost as good as that which contributes to the optimal solution (see Single Haplotype Systems below for the OCA3LOC908, OCA3LOC922 systems).

We used a classification tree generating software package to define rules for classifying individuals into the various eye color groups using these haplotype systems according to methods described herein (See Frudakis, Serial No. 60/338,734, CLASSIFICATION TREE METHODS FOR CONSTRUCTING COMPLEX GENETICS CLASSIFIERS. Filed December 3, 2001). The rules were generated for each of the haplotype systems alone - MCR3LOC105, OCA3LOC109, TYRP3L105 and TYR2LOC920, and are shown in Table 10-1.

From the analysis of the data, it is clear that classification rules made using each of the four haplotype systems lead to a reasonable classification success rate; each of these four haplotype systems has a success rate greater than 85% and the average is 87%. The best results were obtained from OCA3LOC109 and TYR2LOC920 - the two haplotype systems with the lowest P values in Table 9-1. Although the average success rate of 87% seems good, it is probably not good enough for use in the field.

In order to improve this success rate (in ways other than the nested statistical approach we used to construct the optimal solution in Table 8-8), one can construct conditional rules from combinations of classification decisions derived from the four haplotype systems. Using the haplotype systems shown in Table 10-1, the classification from each of the four rule trees (one for each haplotype system) can be combined within one person. For example, one could classify individuals as dark eyed if at least 3 of the 4 classifications were dark, or if only 1 of 4 was dark etc . By using the latter rule (that only one dark classification is needed to classify a person as dark - which is consistent with genetic dominance suspected to play a role in human eye color inheritance), the conditional approach allows us to improve the accuracy of the solution to 88.5%. This is still far below the 96% the nested approach obtained.

Table 10-1

| | MCR3LOC105 | OCA3LOC109 | TYRP3L105 | TYR2LOC920 |
|---|---|---|---|---|
| | | | | |
| CORRECT | 140 | 146 | 144 | 146 |
| INCORRECT | 25 | 19 | 21 | 19 |

Table 10-1. Classification success rates for the single-haplotype system classification rules discussed in the text.

**EXAMPLE 11**

**ADDITIONAL OCA2 HAPLOTYPES ASSOCIATED WITH EYE COLOR**

This example provides additional haplotypes from the OCA2 gene that are associated with eye color. Methods for detecting the nucleotide occurrence at a SNP position are described in Example 4. The OCA3LOC90S haplotype system is comprised of markers 217452, 217455, and 217458 (See Table 1 for a description of the markers). Table 11-1 contains data on haplotype alleles and eye color for these haplotypes. Various statistical analyses are included below, that prove that the OCA3LOC90S haplotype system, and its constituent SNPs, are associated with (and possibly deterministic for) human eye color. Statistically significant P values are in bold print. The results of successful as well as unsuccessful tests arc presented.

Statistical Analysis for OCA-Gene, Association Between Haplotyes & Eye Colors

Haplotypes: H1:CAT, H2:CAC, H3:CGC, H4:TGC, H5:TAT, H6:CGT
Eye Colors: Brown & Not Brown
HYPOTHESES: H0: Eye Colors are not Associated with specific Haplotypes.
H1: Eye Colors arc Associated with specific Haplotypes.
Pearson's Chi-Square & Fisher's Exact Test were used to test H0.

**Table 11-1**

| Eye Color | Haplotypes | | | | | | Total |
|---|---|---|---|---|---|---|---|
| | H1:CAT | H2:CAC | H3:CGC | H4:TGC | H5:TAT | H6:CGT | |
| Brown | 35 | 8 | 9 | 6 | 2 | 0 | 60 |
| Not Brown | 94 | 17 | 22 | 0 | 0 | 1 | 134 |
| Total | 129 | 25 | 31 | 6 | 2 | 1 | 194 |

RESULTS:

Pearson's chi-square test without Yates' continuity correction:
Chi-square = 19.2502, df= 5, p-value = 0.0017
Fisher's exact test p-value = 0.0014, alternative hypothesis: two-sided
These tests lead to the Rejection of H0 in favor of H1.
To determine and quantify the Association between Haplotypes & Eye Colors the Adjusted Residuals(Rij) are worked out, where

$$Rij =( nij-Mij) / \{SQRT[Mij (1-Pi+) (1-P+j)]\} \ \& \ Mij =E (nij)$$

Rij follows N(0,1) as per Large Sample theory .In this case we have
R11 =-1.885, R21 = 1.885,R12 =0.124, R22 =-0.124, R13 =-0.249, R23 =0.249
R14 = 3.718, R24 =-3.718,R15 =-2.124,R25 =-2.124,R16 =-0.670,R26 =0.670

It is clear from the values of Adjusted Residuals that Haplotype H1:CAT is more associated with Not-Brown Eye Color than with Brown Eye Color,

Whereas Haplotypes H4 :TGC & H5 :TAT are Significantly & positively associated with Brown Eye Color.

Odds Ratio(OR) can also be used to infer the Association between Haplotypes & Eye Colors, by considering Haplotypes in pairs. ICwe consider Haplotypes H4 & H 1 the sample OR for H4 for Brown(OR for H 1 for Not-Brown)= 34.61 ,CI (2.05,583.47)

In the case of H 1 & 115, the OR for H5 for Brown = 13.31 ,95% CI (0.62 , ''84.29)
In the case of H3 & H4,OR for H3 for Not-Brown = 30.79, 95% CI (1.57 , 603.05)

The sample OR also confirms that Haplotypes H4 & H5 arc more associated with Brown Eye color & Haplotypes H1 & H3 arc more associated with Not-Brown Eye Color.

Next effect of Mutations was next studied.

Site-1: Mutation

Mutation at site-1: C <--> T H1:CAT <--> TAT:H5, H3: CGC <--> TGC:H4

Data regarding these mutations and their effect on eye color is shown in Table 11-2.

HYPOTHESES:

H0: Mutation at site-1 has not contributed to variations in Eye colors.
H1: Mutation at site-1 has contributed to variations in Eye colors.
Let us consider Haplotypes H1 and H5.
We use Pearson's Chi-Square & Fisher's Exact Tests.

**Table 11-2**

| | Eye Color | | |
|---|---|---|---|
| Haplotypes | Brown | Not Brown | Total |
| H1 | 35 | 94 | 129 |
| H5 | 2 | 0 | 2 |
| Total | 37 | 94 | 131 |

RESULTS:

Pearson's Chi-square with out Yate's correction = 5.1599,
P value = 0.0231 and with Yate's correction = 2.1908,P value = 0.1388
Fisher's Exact test P-value = 0.0782
Result: Significant at 10% level

Let us consider Haplotypes H3 & H4

**Table 11-3**

| | Eye Color | | |
|---|---|---|---|
| Haplotypes | Brown | Not Brown | Total |
| H3 | 9 | 22 | 31 |
| H4 | 6 | 0 | 6 |
| Total | 15 | 22 | 37 |

RESULTS:

Pearson's chi-square test with Yates' continuity correction,
Chi-square value = 7.7654, df= 1, p-value = 0.0053.
Fisher's exact test, p-value = 0.0022, alternative hypothesis: two-sided
Result: Significant.

The Observations of Haplotypes H1 with H3 and H5 were pooled with H4 and the effect of Mutation at site-1 on Eye Color variations was studied.

Results of correlations between haplotype and eye color are shown in Table 11-4. Pearson's Chi-square & Fishers Exact tests were used to test H0.

**Table 11-4**

| | Eye Color | | |
|---|---|---|---|
| Haplotypes | Brown | Not Brown | Total |
| H1 + H3 | 44 | 116 | 160 |
| H4 + H5 | 8 | 0 | 8 |
| Total | 52 | 116 | 168 |

RESULTS:

Pearson's chi-square test with Yates' continuity correction
Chi-square = 15.4997, df = 1, p-value = 0.0001
Fisher's exact test, p-value = 0.0001, alternative hypothesis: two-sided

Reject H0 at 0.01 % Level in favor of H1 & Infer that Mutation at site-1 has produced Haplotypes which are strongly associated with Brown EYE COLOR.

We also computed the Sample Odds Ratio, after adding 0.5 to each cell, n22 = 0 & 95% Confidence Interval (CI) to quantify the associations for Tables 3,4,5 considering H1 Vs H5 the sample OR for H5 for Brown(H1 for Not-Brown) OR = 13.31, CI = (0.624, 284.291).Considering H3 Vs H4 the sample OR = 30.789, CI = (1.737, 603.05). These OR values show that H5 &H4 are strongly associated with Brown Eye Color and H1 & H3 are strongly associated with Not-Brown Eye Color.

Considering (H1 + H3) Vs (H4 +H5)in table-5, the sample OR for (H4 + H5 )for Brown = 44.506, CI : (2.517, 787.607).

This shows that Haplotypes (H1 + H3) are strongly associated with Not Brown Eye Colors and Haplotypes (H4 + H5) are strongly associated with Brown Eye Color.

We have also computed the Adjusted Residuals for the above table-5.
R11 = -4.329, R12 = 4.329, R21 = 4.329 & R22 = -4.329

As per Large sample theory Rij are distributed as N(0,1), the values of the Residuals clearly show that Haplotypes (H4 + H5 ) are significantly Positively associated with Brown Eye Color and Haplotypes (H1 + H5) are significantly & Positively associated with Not-Brown Eye Colors. Thus, mutation at site-1, has produced Significant variations in eye colors, through haplotypes H4 &H5. In other words the phenotypic variation in eye colors can be traced back to the mutation at site-1.

**Nested contingency analysis:**

Association between haplotypes and eye colors (Brown vs. Not Brown):

According to Templeton et al. *supra* (1987) haplotypes form 0-step clades, haplotypes connected by single mutation constitutes the 1-step clades and haplotypes connected (including the inferred {.} ones) by 2 or less mutations constitute the -step clades and so on and carry out nested contingency analysis.

In this case there are six haplotypes:
H1:(CAT), H2:CAC), H3:(CGC), H4:(TGC), H5:(TAT), H6:(CGT).

The following cladogram has been obtained by using PAUP version 4.0b8 software (Sinauer Associates, Inc. Publishers, Sunderland, Massachusetts. Downloadable from http://paup.csit.fsu.edu/index.html) with maximum parsimony as an optimality criterion.

1-step clades are: I1:(H1,H5), I2:H2,13:(H3,H4), I4:H6.

2-step clades are:
Clade-1: (I1,I2)=(H1, H5, H2), Clade-2:(I3,I4)=(H3, H4, H6).

See Figure 6 for diagram of 2 step clade.

Hypotheses:H0: Eye colors are not associated with various levels of clades. H1: Eye colors arc associated with various levels of clades, which represents certain mutations.

We used Pearson's chi-square and Fisher's exact tests, to test H0, as shown in Table 11-5.

**Table 11-5**

| Source | Chi-Square | d.f. | P-value | Fishers P-Value | Significance |
|---|---|---|---|---|---|
| With in 1-step (H1 Vs H5) | 2.1908 | 1 | 0.1388 | 0.0782 | <.10 |
| With in 1-step (H3 Vs H4) | 7.7654 | 1 | 0.0053 | 0.0022 | <.01 |
| With in 2-step ((H1+H5) Vs H2)) | 0.1443 | 1 | 0.7041 | 0.7041 | NS |
| With in 2-step ((H3+H4) Vs H6)) | 0.0000 | 1 | 1.0000 | 1.0000 | NS |
| Between 2-step ((H1+H2+H5)Vs (H3+H4+H6)) | 1.6155 | 1 | 0.2037 | 0.2409 | NS |
| Note: H1 Vs H5 and H3 vs. H4 represents mutations at site I and H1+H2+H5 vs. H3+H4+H6 represents mutation at site-2. | | | | | |

**Inference:**

Statistical Analysis shows that the mutation at site-1 is the source for significant variations in Eye Colors. In other words the variations in Eye Colors can be traced back to mutation in OCA2908 Gene at site-1.
Details of computations are provided below, based on the data shown Table 11-6 to 11-10:

**Table 11-6: H1 vs. H5**

| | Eye Color | | |
|---|---|---|---|
| Within 1-step clade Haplotypes | Brown | Not Brown | Total |
| H1 | 35 | 94 | 129 |

(continued)

| | Eye Color | | |
|---|---|---|---|
| Within 1-step clade Haplotypes | Brown | Not Brown | Total |
| H5 | 2 | 0 | 2 |
| Total | 37 | 94 | 131 |
| Chi-square statistic value=2, P=value=0 and Fisher's exact test, P=value=0.0782. | | | |

**Table 11-7: H3 Vs H4**

| | Eye Color | | |
|---|---|---|---|
| Within 1-step clade Haplotypes | Brown | Not Brown | Total |
| H3 | 9 | 22 | 31 |
| H4 | 6 | 0 | 6 |
| Total | 15 | 22 | 37 |
| Chi-square statistic value=7.7654, P-value=0.0053 and Fisher's exact test, P-value=0.0022. | | | |

**Table 11-8: (H1+H5) Vs H2**

| | Eye Color | | |
|---|---|---|---|
| Between 1-step clade Haplotypes | Brown | Not Brown | Total |
| H1+H5 | 37 | 94 | 131 |
| H2 | 8 | 17 | 25 |
| Total | 45 | 111 | 156 |
| Chi-square statistic value=0.1443, P-value=0.7041 and Fisher's exact test, P-value=0.8100. | | | |

**Table 11-9: (H3+H4) Vs H6**

| | Eye Color | | |
|---|---|---|---|
| Between 1-step clade Haplotypes | Brown | Not Brown | Total |
| H3+H4 | 15 | 22 | 37 |
| H6 | 0 | 1 | 1 |
| Total | 15 | 23 | 38 |
| Chi-square statistic value=0.0000, P-value=1.0000 and Fisher's exact test, P-value=1.0000. | | | |

**Table 11-10 (H1+H2+H5) vs. (H3+H4+H6)**

| | Eye Color | | |
|---|---|---|---|
| Between 2-step clades Haplotypes | Brown | Not Brown | Total |
| H1+H2+H5 | 45 | 111 | 156 |
| H3+H4+H6 | 15 | 23 | 38 |

(continued)

| | Eye Color | | |
|---|---|---|---|
| Between 2-step clades Haplotypes | Brown | Not Brown | Total |
| Total | 60 | 134 | 194 |
| Chi-square statistic value=1.6155, P-value=0.2037 and Fisher's exact test, P-value=0.2409. | | | |

## Single haplotype system OCA3LOC908

The OCA3LOC922 haplotype system is comprised of markers 217455, 886993, and 217458 (See Table 1 for a description of the markers). What follows below arc various statistical analyses that prove that the OCA3LOC922 haplotype system, and its constituent SNPs, are associated with (and possibly deterministic for) human eye color. Statistically significant P values arc in bold print. The results of successful as well as unsuccessful tests arc presented.

## STATSTICAL ANALYSIS FOR OCA3LOC922 HAPLOTYPE SYSTEM

ASSOCIATION BETWEEN GENOTYPES AND EYE COLORS (Dark, Not-Dark) Hypotheses: H0: Eye Colors are not Associated with specific Genotypes. H1: Eye Colors are Associated with specific Genotypes.

We use Pearson's Chi-square & Fisher's exact tests to test H0.

Data on Genotype and eye color are shown in Table 11-11.

**Table 11-11**

| Genotypes | Eye Color | | |
|---|---|---|---|
| | Dark | Not Dark | Total |
| G11: (H1,H1) : (AGT,AGT) | 31 | 103 | 134 |
| G12: (H1,h2) : (AGT,GAC) | 10 | 18 | 28 |
| G13: (H1,H3) : (AGT,AGC) | 4 | 9 | 13 |
| G 14: (H1,H4) : (AGT,GGC) | 8 | 16 | 24 |
| G15: (H1,H5) : (AGT,AAC) | 4 | 16 | 20 |
| G16: (H1,H6) : (AGT,GAT) | 1 | 1 | 2 |
| G17: (H1,H7) : (AGT,GGT) | 1 | 5 | 6 |
| G18: (H1,H8) : (AGT,AAT) | 0 | 1 | 1 |
| G22: (H2,H2): (GAC,GAC) | 2 | 1 | 3 |
| G23: (H2,H3) : (GAC,AGC) | 1 | 1 | 2 |
| G24: (H2,H4) : (GAC,GGC) | 4 | 1 | 5 |
| G25: (H2,H5) : (GAC,AAC) | 1 | 2 | 3 |
| G26: (H2,H6) : (GAC,GAT) | 1 | 0 | 1 |
| G34: (H3,H4) : (AGC,GGC) | 0 | 1 | 1 |
| G35: (H3,H5) : (AGC,AAC) | 3 | 0 | 3 |
| G45: (H4,H5) : (GGC,AAC) | 0 | 2 | 2 |
| G55: (H5,H5) : (AAC,AAC) | 0 | 1 | 1 |
| Total | 71 | 178 | 249 |

Results:

Pearson's chi-square test without Yates' continuity correction:

Chi-square = 25.6524, df= 16, p-value = 0.0591

These results are not significant at a 5% level of Significance. However at a 10% level of significance the Results are significant. At this level the data show that specific association between Eye colors and Genotypes exists. To determine and quantify the association we computed the Odds Ratio(OR)& 95% Confidence Interval(C1) by considering two Genotypes at a time.

Considering the Genotypes G11 & G 12, OR for G11 for Not Dark Eye colors = OR for G 12 for Dark Eye colors = 1.846 CI = (0.772, 4.410).

In the case of G11, G22 OR for G22 for Dark Eye = 6.645 CI = (0.553,75.77)

In the case of G11,G24 OR for G24 for dark Eye = 13.29 CI = (1.432,123.32)

We also computed the Adjusted Residuals(AR) Rij, which follow SND N(0,1)to quantify the associations. Presented below are a few ARs of interest. R11 = -2.0297, R12 = 2.0297, R91 = 1.473 ,R92 = -1.473 ,R111= 2.576 & R112 = -2.576.

The values of OR & AR clearly reveal that Genotype G11 :(AGT,AGT)more significantly associated with Not-Dark Eye colors, than with Dark eye colors.

Genotypes G12 :(AGT,GAC),G22 :(GAC,GAC) & G24 :(GAC,GGC) are strongly associated with Dark Eye colors than with Not-dark eye colors.

Next we examined the Haplotypes, individually, as to whether they are associated with Eye colors

STATISTICAL ANALYSIS for OCA3LOC922 Gene Association between

Haplotypes & Eye Colors.

The haplotypes analyzed included:
H1:AGT,H2:GAC,H3:AGC,H4:GGC,H5:AAC,H6:GAT,H7:GGT & H8:AAT.

Eye Colors scored included: dark (Brown, Brown, Brown2, Brown3, and Black) and "Not-Dark"(Green, Blue, Hazel).

HYPOTHESES :

H0:Eye colors are not associated with specific Haplotypes.
H1:Eye Colors are associated with specific Haplotypes.
Pearson's Chi-square test was used to test H0.

In the methods used, if a test showed significance, the sample Odds Ratio was computed along with 95% Confidence Interval(CI) by considering two Haplotypes at a time. Also Computed were the Adjusted Residuals, Rij which are distributed as Standard Normal Deviates as per Large sample theory, to determine and quantify the association between Haplotypes and Eye colors. Data on eye color and haplotype are shown in Table 11-12.

**Table 11-12**

| | Eye color | | |
|---|---|---|---|
| Haplotypes | Dark | Not Dark | Total |
| H1:AGT | 90 | 272 | 362 |
| H2:GAC | 21 | 24 | 45 |
| H3:AGC | 8 | 11 | 19 |
| H4:GGC | 12 | 20 | 32 |
| H5:AAC | 8 | 22 | 30 |
| H6:GAT | 2 | 1 | 3 |
| H7:GGT | 1 | 5 | 6 |
| H8:AAT | 0 | 1 | 1 |
| Total | 142 | 356 | 498 |

Results:

The Pearson's chi-square test without Yates' continuity correction yielded significant results:
Chi-square = 15.6375, df= 7, p-valuc = 0.0286

Therefore, H0 is rejected in favor of H1 and infer that Eye colors are associated with specific Haplotypes.

Considering H1 & H2, the Odds Ratio(OR) for H1 Not-Dark Eye colors = OR for H2 for Dark Eye colors & CI arc: OR = 2.664 ,CI = (1.405,4.976)
Considering H1 & H3 OR for H3 for Dark Eye colors = 2.198 , CI = (0.857,5.634)
Considering H1 & H4 OR for H4 for Dark Eye Colors = 1.813 , CI = (0.853.3.855)
Adjusted Residuals: R11 = -2.945, R12 = 2.945, R21 = 2.828, R22 = -2.828 R31 = 1.338 ,R32 = -1.338 R41 =1.164 , R42=-1.164,R51 = -0.231 R52= 0.231
R61 = 1.468 .R62 = -1.468 ,R71 =-0.647 ,R72 = 0.647, R81 = -0.632,R82 = 0.632

The values of OR along with CI and the values of Adjusted Residuals Clearly show that Haplotypes H1:AGT is significantly and positively associated with Not-Dark Eye colors, whereas haplotypes H2, H3 & H4 arc more strongly associated with Dark Eye colors than Not-Dark Eye colors.

Next we studied whether any mutations are responsible for this associations, by carrying out nested contingency analysis.

**STATISTICAL ANALYSIS OCA3LOC922: Nested contingency analysis**

We studied the association between OCA3LOC922 haplotypes and eye colors (Dark vs. Not-dark). According to Templeton et al., *supra* (1987) haplotypes form 0-step clades, haplotypes connected by single mutation constitutes the 1-step clades and haplotypes connected (including the inferred {.} ones) by 2 or less mutations constitutes the 2-step clades and so on and carry out nested contingency analysis.

Eye Colors analyzed included: Dark (Brown, Brown, Brown2, Brown3 and Black)and Not-Dark(Blue, Green, Hazel).

For OCA3LOC922 there are eight haplotypes {0-step Clades}:

111: AGT, H2:GAC, H3:AGC, H4:GGC, H5:AAC, H6:GAT, H7:GGT, H8:AAT

The following cladogram has been obtained :

1-step clades:I1:(H5,H8), I2:(H7,H1),I3:(H3,H4), I4:(H2,H6).
2-step clades are:
Clade-1:{I1,I2}={(H5,H8),(H7,H1)}, Clade-2:{I3,I4}={(H3,H4),(H2,H6)}.
See figure 7 for 2-step cladogram: Clade-1 Clade-2.

The hypotheses tested included the following:

H0:Eye colors are not associated with various levels of clades.
H1:Eye colors are associated with various levels of clades, which represents certain mutations.
Pearson's chi-square and Fisher's exact tests were used to test H0.

Results of nested contingency analysis for Brown vs. not-brown eye colors are presented in Table 11-13:

**Table 11-13**

| Source | Chi-Square | d.f. | P-value | Fisher's | P-Value Significance |
|---|---|---|---|---|---|
| NS | Within 1-step Clades (H5 vs: H8) | 0.3159 | 1 | 0.5741 | 1.0000 |
| NS | (H1 vs. H7) | 0.0002 | 1 | 0.9876 | ---- |
| NS | (h2 vs. h6) | 0.0056 | 1 | 0.9405 | 0.6011 |
| NS | (h3 vs. h4) | 0.0008 | 1 | 0.9768 | 0.7743 |

(continued)

| Source | Chi-Square | d.f. | P-value | Fisher's | P-Value Significance |
|---|---|---|---|---|---|
| NS | Within 2-step Clades {(H1+H7) vs. (HS+H8)} | 0.0069 | 1 | 0.9338 | ----- |
| NS | {(H3+H4) vs. (H2+H6)} | 0.4219 | 1 | 0.5028 | 0.4219 |
| <.01 | Between 2-step Clades {(H1+H7+H5+H8) vs. (H3+H4+H2+H6)} | 12.5967 | 1 | 0.0004 | --- |
| Note: {(H1+H7+H5+H8) Vs (H3+H4+H2+H6)} represents mutation at site 3, which has resulted in significant variations in Eye colors. | | | | | |

Details of analysis between Two level Clades:

The hypothesis tested included:

H0 : There is no association between two level clades and Eye colors.
H1 : The Two level Clades are associated with specific eye colors.

Data for this analysis of eye color and 2-step clades are shown in 11-14.

**Table 11-14**

| | Eye Color | | |
|---|---|---|---|
| Two step Clades | Brown | Not Brown | Total |
| Clade-2 H2+H3+H4+H6 | 43 | 56 | 99 |
| Clade-1 H1+H5+H7+H8 | 99 | 300 | 399 |

**RESULT:**

Pearson's chi-square test with Yates' continuity correction yielded the following values:
Chi-square = 12.5967, df = 1, p-value = 0.0004

Hypothesis Ho was rejected and an inference was made that the Two-Step Clades are associated with specific Eye colors.

To quantify the association the Odds Ratio (OR) was computed along with

95% Confidence Interval (CI) and the Adjusted Residuals { Rij}, which follow N(0,1) as per large sample theory.
OR for (H2+H3+H4+H6) for Dark eye colors = 2.327 ,CI = (1.478, 3.693),R11=3.674 =R22
OR for (H1+H5+H7+H8) for Not-Dark Eye = 2.327 ,CI = (1.478, 3.693),R21 =-3.674 =R12

The values of OR and Adjusted Residuals clearly show that haplotypes H2,H3,H4,&H6 arc significantly positively associated with Dark Eye colors, and Haplotypes H1,H5,H7&H8 are significantly and positively associated with Not-Dark Eye colors. The mutation at site-3 is responsible for this association. In other words the variations in eye colors can be traced back to the mutation at site-3.

**Statistical Analysis for OCA3LOC922 eye color: Association between genotypes and eye colors**

The hypothesis tested in this analysis included the following:

H0: There is no association between genotypes and eye colors.
H1: There is an association between genotypes and eye colors.

Chi-square and Fisher's exact test's P- value were calculated. Data on Genotype and eye color for this analysis is presented in Table 11-15. Data was calculated in terms of light (blue+gre and not-light (brown+dark+hazel) eye color.

**Table 11-15**

| | Eye Color | | |
|---|---|---|---|
| Genotype | Light | Not Light | Total |
| G11 : (H1,H1) : (AGT,AGT) | 67 | 67 | 134 |
| G12 : (H1,H2: (AGT,GAC) | 11 | 17 | 28 |
| G13: (H1,H3): (AGC,AGT) | 3 | 10 | 13 |
| G14 : (H1,H4):(AGT,GGC) | 12 | 12 | 24 |
| G15 : (H1,H5):(AGT,AGT) | 12 | 8 | 20 |
| G16 : (H1,H6):(AGT,GAT) | 1 | 1 | 2 |
| G17 : (H1,H7): (AGT,GGT) | 5 | 1 | 6 |
| G18 : (H1,H8):(AAT,AGT) | 0 | 1 | 1 |
| G22 : (H2,H2):(GAC,GAC) | 0 | 3 | 3 |
| G23 : (H2,H3) : (AGC,GAC) | 0 | 2 | 2 |
| G24 : (H2,H4) : (GAC,GGC) | 0 | 5 | 5 |
| G25 : (H2,H5) : (AAC,GAC) | 1 | 2 | 3 |
| G26 : (H2,H6) : (GAC,GAT) | 0 | 1 | 1 |
| G34 : (H3,H4) : (AGC,CGC) | 1 | 0 | 1 |
| G35 : (H3,H5) : (AAC,AGC) | 0 | 3 | 3 |
| G45 : (H4,H5) : (AAC,GGC) | 1 | 1 | 2 |
| G55 : (H5,H5) : (AAC,AAC) | 0 | 1 | 1 |
| Total | | | |

Result: Chi-square Statistic values (24.2564, d.f.=16 and P-value=0.0841) were not significant.
Inference: There was no significant difference between genotypes and eye colors at a 5% level.

**Association between haplotypes and eye colors light (blue+green) and not-light (brown+dark+hazel)].**

The hypothesis tested in this analysis included the following:

H0: There is no association between haplotypes and eye colors.
H1: There is an association between haplotypes and eye colors.

Chi-square and Fisher's exact test's P- value were calculated. Data of geneotype and eye color are shown in Table 11-16.

**Table 11-16**

| | Eye Color | | |
|---|---|---|---|
| Genotype | Light | Not Light | Total |
| H1: AGT | 178 | 184 | 362 |
| H2: GAC | 12 | 33 | 45 |

(continued)

| | Eye Color | | |
|---|---|---|---|
| Genotype | Light | Not Light | Total |
| H3: AGC | 14 | 15 | 19 |
| H4: GGC | 14 | 18 | 32 |
| H5: AAC | 14 | 16 | 30 |
| H6: GAT | 1 | 2 | 3 |
| H7: GGT | 5 | 1 | 6 |
| H8: AAT | 0 | 1 | 1 |
| Total | 228 | 270 | 498 |

Result: The results for this analysis were significant (Chi-square Statistic value=17.4834, d.f.=7 and P-value = 0.0145). The haplotypes were found to be associated with specific eye colors

**Table 11-17**

| Haplotype Chi-Square | Fisher's Odd Pair (Hi,Hj) S | d.f. | P-value | P-value Hi fo | 95% C.I. |
|---|---|---|---|---|---|
| (H1, H2) | 8.1441 | 1 | 0.0043 | 0.00432.6603 | [1.3316,5.31 |
| (H1, H3) | 4.6492 | 1 | 0.0311 | 0.01853.6277 | [1.1813, 11.1 |
| (H2, H7) | 5.3125 | 1 | 0.0212 | 0.01240.0727 | [0.0077, 0.68 |

**Nested contingency analysis between haplotypes and eye colors**

Haplotypes form 0-step clades, haplotypes connected by single mutation constitutes 1-step clades and haplotypes connected by 2 or less mutations constitute 2-step clades and so on for carrying out nested analysis (Templeton et al. ,1987).

In this case, we have eight haplotypes and they form 0-step clades which are given below:

0-step clades: H1: AGT, H2: GAC, H3:AGC, H4:GGC, H5:AAC, H6:GAT, H7: GGT and H8: AAT.

The following two clades were obtained by using PAUP Ver. 4.0b8 software.

1-step clades: I-1:(H5,H8), I-2:(H7,H1), I-3:(H3,H4), I-4:(H2,H6)
2-step clades: II-1:(I1,I2)=(H8,H8,H7,H1), II-2:(I3,I4)=(H3,H4,H2,H6)

See FIG. 8 for 2-step cladogram: Clade-1 Clade-2.

The hypotheses that were tested included:

H0: Eye colors are not associated with various steps of clades.
H1: Eye colors are associated with various steps of clades.

Test Statistic: Chi-square test and Fisher's exact test P-value were determined.

The nested contingency analysis for blue vs green eye colors is shown in Table 11-18:

**Table 11-18**

| Source | Chi-square | d.f. | P-value | P-value | Fisher's Significance |
|---|---|---|---|---|---|
| Within 1-step | | | | | |
| (H5 vs H8) | 0.0000 | 1 | 1.0000 | 1.0000 | Not-significant |
| (H1 vs H7) | 1.5582 | 1 | 0.2119 | 0.1204 | Not-significant |
| (H2 vs H6) | 0.0000 | 1 | 1.0000 | 1.0000 | Not-significant |
| (H3 vs H4) | 1.7872 | 1 | 0.1819 | 0.1350 | Not-significant |
| Within 2-step | | | | | |

(continued)

| Source | Chi-square | d.f. | P-value | P-value | Fisher's Significance |
|---|---|---|---|---|---|
| ((H1+ H7) vs (H5+H8)) | 0.4210 | 1 | 0.5155 | 0.5524 | Not-significant |
| ((H3+ H4) vs (H2+H6)) | 0.7751 | 1 | 0.3787 | 0.3959 | Not-significant |
| Between 2-step ((H1+H5+H7+H8) vs (H2+H3+H4+H6)) | 10.4229 | 1 | 0.0012 | 0.0015 | <0.001 |

Result: The results of this analysis indicated that two level clades are associated with eye colors (Table 11-19). Odds ratio for (H1+H5+H7+H8) for light eye color = Odds ratio for (H2+H3+H4+H6) for not-light eye color is 2.1398 and 95% C.I. is [1.3399, 3.4156].

**Table 11-19**

| Source | Chi-square | d.f. | P-value | Fisher's P-value | Significance |
|---|---|---|---|---|---|
| Within 1-step (H5 vs. H8) | 0.0000 | 1 | 1.0000 | 1.0000 | Not significant |
| (H1 vs. H7) | 1.5582 | 1 | 0.2119 | 0.1204 | Not significant |
| (H2 vs. H6) | 0.0000 | 1 | 1.0000 | 1.0000 | Not significant |
| (H3 vs. H4) | 1.7872 | 1 | 0.1819 | 0.1350 | Not significant |
| Within 2-step ((H1+H7)vs. (H5 + H8)) | 0.4210 | 1 | 0.5165 | 0.5824 | Not significant |
| ((H3+H4) vs. (H2+H6)) | 0.7751 | 1 | 0.3787 | 0.3959 | Not significant |
| Between 2-stcp ((H1+H5+H7+H8) vs. (H2+H3+H4+H6)) | 10.4229 | 1 | 0.0012 | 0.0015 | <0.001 |

EXAMPLE 12

**CLASSIFICATION TREE ALGORITHM**

This Example presents a classification tree algorithm used for solution development. Classification trees are used to predict membership of dependent/ response variables from one or more independent /predictor variables in a set of data. Classification trees are mainly used in data mining. Classification trees present results in the from of trees. Every basic tree structure has a root, decision nodes, leafs and edges. Classification trees are built by asking a serious of questions and a decision is taken depending on the answer to that question, the final answer depends on all the previous answers.

The root of the tree is the starting point of the tree, it asks the first question. Each decision node asks a question and depending on the answer the tree keeps growing (goes to the next decision node) or terminates with a leaf node which gives the final answer. The edges connect the root to the nodes and leafs.

In classification trees the value at the leaf is categorical (NOT NUMBERS)

In regression trees the value at the leaf is numeric.

The following are important in building the trees.

1. What attribute to select at a particular decision node.
2. What value should be selected as threshold for the attribute, in order to split the tree and continue growing.
3. What is the stopping criterion

**C4.5 tree construction algorithm**

The tree is empty initially and the algorithm starts building it from the root and adds decision nodes or leaf nodes as it goes down each branch of the tree. The following steps are carried out recursively

1. Calculating the information gain of each attribute.

2. The attribute with the highest information gain is selected for test at the node.
3. If the attribute selected is discrete, node is branched with all possible values. If the attribute is continuous, a cut point is selected that yields highest information gain. The cut-point splits the node into two sets: those with the value less than or equal to the cut point and those with value greater than the cut point.
4. Assigning the data items into corresponding branches
5. Repeating all the above steps in each branch of the tree.

This recursive method is a greedy approach, as the algorithm never backtracks to reconsider previous decision to modify the learnt tree. The algorithm stops when a stopping criterion is met. The C4.5 grows a large tree and the over fitting problem is solved at the pruning stage, we can see that the following four elements form the core of C4.5 tree building algorithm:

**Choosing the attribute for the decision node**

The central choice in building a tree is selecting which attribute to test at each node in the tree. The selected attribute must be most useful for classifying dataset. C4.5 uses either information gain or information gain ratio. The information gained by partitioning training set T using the test X is defined as the following:

$$gain(X) = info(T) - info_x(T),$$

$$info_x(T) = \sum_{i=1}^{n} \frac{|T_i|}{|T|} \times info(T),$$

$$info(T) = -\sum_{j=1}^{k} \frac{freq(C_j,T)}{|T|} \times log_2\left(\frac{freq(C_j,T)}{|T|}\right) \text{bits},$$

Where *info* (*T*) is the average amount of information needed to identify the class of an example in T. *info, (T)* is the expected information requirement after T is partitioned into n subsets {Ti} in accordance with the outcomes of the test X;

Information gain criterion has a strong bias in favor of tests with many outcomes, so C4.5 uses gain ratio as a default split criterion, the gain ratio is defined as

$$gain\,ratio(X) = \frac{gain(X)}{split\ info(X)},$$

$$split\ info(X) = -\sum_{i=1}^{n} \frac{|T_i|}{|T|} \times log_2\left(\frac{|T_i|}{|T|}\right),$$

where *split info(X),* is the potential information generated by splitting T into n subsets.

Notations

| Symbol | Description |
|---|---|
| T | Training data set |

(continued)

| Symbol | Description |
|---|---|
| X | Test formed using attribute A |
| Freq ($C_j$ ,T) | Number of cases in T that belongs to class $C_j$ |
| K | Number of classes in data set T |

Choosing the threshold value for the split

Once the attribute is selected a value of the attribute should be assigned to the node. For discrete attribute A, node is branched with all possible values. For continues attribute A, a binary test with outcomes $A \leq \Gamma$ and $A > \Gamma$ is done. The best threshold $\Gamma$ is found for an attribute A by: first, sorting the training examples and thresholds are selected buy finding the mid points of two adjacent values in the sorted list. The threshold that yields the best value of the splitting criterion is then selected.

**Stop splitting condition and class assignment**

The C4.5 stops splitting if all the cases at the node belong to the same class $C_j$, the node becomes a leaf node with associated class $C_j$. If number of cases at the node is less than minimum required and cases belong to more than on one class, the node becomes a leaf node with associated class $C_j$ (the most frequent class). The classification error of the leaf is the number of cases in T whose class is not Cj.

From Trees to rules.

1. Every path from the root of a tree to a leaf gives one initial rule
2. Each rule is simplified by removing conditions that does not help in discriminating the predicted class.
3. Rules that do not contribute to accuracy is removed
4. The sets of rules for the classes are then ordered to minimize misclassification rates and a default class is chosen.

**EXAMPLE 13**

**CORRESPONDENCE ANALYSIS FOR COMPLEX GENETIC ANALYSIS**

The following example discusses correspondence analysis for complex genetic analysis. Correspondence Analysis is a powerful multivariate graphical procedure to study the association between variables and attributes, and can be considered a scaling method linked to principal component analysis and canonical correlation analysis (Kishino and Waddel, Genome Informatics 11:83-95, 2000; Benzecri, in "Correspondence Analysis Handbook" (Dekker, New York 1992); Benzecri, in "L'Analyse des donnees" Vol. 2: L'Analyse des Correspondence (Dunod, Paris 1973); Greenacre, in "Theory and Application of Correspondence Analyses" (London, Academic Press 1984), each of which is incorporated herein by reference). Values and attributes are represented within a contingency table of "i" rows (the observed haplotype pairs for the TYR2LOC920, OCA3LOC920, MCR3LOC105, OCA3LOC109 and TYRP3L106 haplotype systems) and "j" columns (eye color classes). From this table, an orthogonal system of axes is constructed through Principal Components, where row and column attributes are jointly displayed in a k dimensional space, preserving the distance between the row (i) attributes and the distance between the column (j) attributes, where k = min {i-1, j-1}, is preserved. Two row points that are close to each other in the k dimensional space indicate that the two rows have similar profiles (conditional distributions) across the columns. Similarly two column points close to one another in the space indicate that the column attributes share similar profiles (conditional distributions) down the rows.

As disclosed herein, proximity between row and column points indicated that particular row-column (haplotype pair, eye color) combinations occurred more frequently than would have been expected based on the assumption of independence, and thereby indicated a strong association between the row (haplotype pairs) and column (eye color) attributes. The usual output from correspondence analysis includes the "best" two-dimensional representation of the data with the coordinates of the plotted points (i, row points; j, column points) along with a measure (called the inertia) of the amount of information retained in each dimension. ,Multidimensional space is represented with multiple two-dimensional plots. The display coordinates $x_i^{(g)}$, g (genotype or haplotype system) ($i=1,2,...n_g$) and eye color $x_j^{(c)}$ ($j=1,2,..n_c$) were obtained by minimizing:

$$L = \sum^{n_g} \sum^{n_e} f_{ij} [x_i^{(g)} - x_j^{(c)}]^2 \quad (1)$$

under the constraints that the mean coordinates are zero with variance=1, and where $f_{ij}$ is ≥ 0. The cost function (1) relates genotypes (haplotypes) to eye color in a more direct way than the classification tree method.

The classification tree analysis was limited by its own complexity, which caused the sample size within certain compound genotype classes to be low. Because of the statistical limitations of the classification tree approach, a Correspondence Analysis was applied to study the association between genotypes and eye colors. Correspondence analysis is primarily a graphical technique designed to represent complex associations in a low-dimensional space. Eigenvalues of the 3 (traits minus 1) X 49 (haplotype pairs) contingency table were used to collapse the data into three dimensions represented by the scatter plots of genotypes (diploid haplotype pairs) and trait values (eye colors).

) Good scatter of genotypes and trait values was observed in all three dimensions. Dimensions 1 and 2 combined to explain 86.5% of the genotypic and phenotypic variation, whereas dimensions 1+3 and 2+3 combined to explain 72.5%, and 41 % of the variation, respectively. Aside from explaining the variance in eye color contributed by genotypes of these haplotype systems, the plot of row and 5 column attributes within the k-dimensional space allows for the construction of a graphical classifier that is less sensitive to compound genotype class sizes. In this case, the genetic attributes for haplotype phase-certain individuals of known but concealed eye color were identified and plotted. Connecting the within-individual attributes to one another with edges creates a k-dimensional object, the moment of 0 which is offset from the j column attribute (eye color class) coordinates by j Euclidian distances. The likelihood that the individual falls within each class was inferred from these Euclidian distances and used to formulate a prediction that is compared against the actual eye color. This technique allowed the correct classification of 97% of Caucasian individuals tested as belonging to a particular eye color shade (n=254; Light = Blue, Green; Dark = Brown, Hazel). In contrast to the classification tree method, where the particular eye color was almost never predictable, the correspondence analysis allowed for the correct prediction of specific eye color 45% of the time. Whereas the classification tree method could not be applied to 14% of Caucasians, only 4% of Caucasians tested were inconclusive using the Correspondence Analysis method.

These results demonstrate that correspondence analysis provides a means to perform complex genetic analyses such as an analysis of eye color. As such, correspondence analysis can be used to identify genetic risk factors associated such as a predisposition to cataracts or melanoma or the like with a complex genetic trait such as eye color, skin pigmentation, or hair color. For example, persons with a haplotype associated with a certain light eye color can be compared to persons with a haplotype associated with a different light eye color to determine whether there is a correlation with incidence of melanoma. The identification of specific haplotypes as predictive markers for a disease such as melanoma also provides a means to develop targets for drugs that can modulate the susceptibility to a disorder of an individual having a haplotype associated with the disorder.

## EXAMPLE 14

## GENETIC CLASSIFIER FOR RACIAL INFERENCE

The following example presents a genetic classifier for SNP-based racial inference DNA based human identity testing is dependent on accurate and impartial determinations of racial and/or ethnic affiliation. STR markers have been described to be capable of racial classification, but the multi-allelic nature of STRs impose unique statistical and technical problems. In an effort to identify bi-allelic markers that could be used to infer racial affiliation from DNA, common single nucleotide polymorphisms were surveyed in the human pigmentation and xenobiotic metabolism genes. Sixty SNPs were identified, as discussed in further detail in this Example, with significant minor allele frequency differences between groups of unrelated Asians, African Americans and Caucasians (n=230), and used both linear and quadratic methods to incorporate these SNPs into a classifier model. Generalization of a quadratic model revealed perfect accuracy and sensitivity in a group of 505 unrelated individuals (403 Caucasians, 114 African Americans and 15 Asians). These results indicate that the human pigmentation and xenobiotic metabolism genes are an unusually rich source for racially informative SNPs, and suggest that powerful systematic genetic forces that have shaped the distribution of these gene sequences throughout human evolution. The racial classifier disclosed herein has the potential to expand the utility of forensic DNA identity testing by offering a novel method for qualifying reference population databases used for calculating exclusion probabilities, as well by ascribing physical characteristics to anonymous DNA samples.

## METHODS

### Data Collection

Specimens and basic biographical data were obtained from randomly selected individuals of self-reported African, Asian and Caucasian descent within the state of Florida, under informed consent guidelines (each participant approved of the use of their specimen for forensic DNA research with the aims outlined in this manuscript). We extracted DNA from circulating lymphocytes using commercial (Qiagen and Promega) preparation kits, and used a novel nested PCR approach to front-end a primer extension protocol employing a 25K SNPstream genotyping system (Orchid Bio-Sciences; Princeton NJ).

### Resequencing

Vertical resequencing for the various genes was performed by amplifying gene sequences from a multiethnic panel of 670 unrelated individuals for whom only race was known. For each gene used in our study, we amplified the proximal promoter, each of the exons with flanking intron, and 3'UTR. PCR amplification was accomplished using *pfu* Turbo, according to the manufacture's guidelines (Stratagene). We developed a program to design re-sequencing primers to insure that only the region of interest was amplified, and no cross-over from pseudo genes or other homologous genes would occur. This was accomplished by analyzing the sequence file of interest in tandem with all other flat-files identified through BLAST searches to have homology with this sequence. The program also insured that the maximum number of relevant regions were included in the fewest possible number of amplicons. Amplification products were subcloned into the pTOPO (Invitrogen) sequencing vector. 96 insert positive colonies were grown and Plasmid DNA was isolated and sequenced using PE Applied Biosystems BDT chemistry and an ABI3700 sequencer. Sequences were deposited into a commercial relational database system (iFINCH, Geospiza, Seattle, WA). The resulting sequences were aligned and analyzed using another program developed to align sequences (using Clustal X) within each amplification region, identify discrepancies between these sequences, and qualify the discrepancies as candidate SNPs using PHRED quality metrics.

### Genotyping

A first round of PCR was performed on these samples using the high-fidelity DNA polymerase *pfu* turbo. Because the primers for this step were the same primers that were used for resequencing, they were known to not cross-react with other competing sequences in the genome. The resulting PCR products were checked on an agarose gel, diluted, and then used as template for a second round of PCR incorporating phosphothionated primers. We observed a higher specificity when using this nested genotyping approach than when using a single amplification protocol, presumably because most of the genes we targeted were members of multi-gene families and because of BLAST algorithm deficiencies and public sequence database limitations (incompleteness). Genotyping was performed on individual DNA specimens using a single base primer extension protocol and an Orchid SNPstream 25K platform (Orchid BioSciences, Inc., Princeton, NJ).

## RESULTS

In order to identify SNP markers useful for racial classification, SNPs were targeted in the human pigmentation and xenobiotic metabolism genes (TYR, TYRP1, $OCA_2$, MC1R, DCT, AP3B, CYP3A4, CYP2C8, CYP2D6, CYP2C9, CYP1A1 and AHR) as well as the HMGCR gene. To identify SNP candidates, we rc-sequenced the promoter, exon and 3' UTR regions for each gene using a racially diverse pool of 200 individuals and supplemented these by mining the public database resources (NCBI:dbSNP). Combining the resources, an average of 44 candidate SNPs were identified per gene (a total of 484 SNPs). The two methods of SNP discovery produced significant overlap, and we observed that most of the informative SNPs (those with minor alleles of higher-frequency) were already present in the public database (NCBI:dbSNP), presumably because the public database was constructed from few donors and, therefore, is biased towards these types of SNPs. Nonetheless, resequencing identified several novel SNPs per gene, and many of them are part of the classifier disclosed herein.

One hundred unrelated Caucasians were genotyped, as were 100 unrelated African Americans and 30 unrelated Asians (different individuals than those used for resequencing) at 188 of the 484 SNPs (roughly 15 per gene for each of the 11 genes). Five of the SNP markers were genotyped in sample sizes that were roughly double these numbers. Minor allele frequencies spanned from zero (unvalidated SNPs) to 48%. 96 of the 188 SNPs revealed clear genotype classes in the assay, had coherent patterns (i.e., no co-amplification of competing sequences evident) and had minor allele frequencies that were greater than 0.01 in at least one of the three races (validation rate = 51%). Most of the SNPs that dropped out at this step had coherent genotype patterns but had minor allele frequencies less than 0.01. Of these 96 SNPs, many revealed genotype distributions and allele frequencies that were not significantly different between the racial classes (for example, see Table 14-1). These SNP markers were discarded from our analysis.

Others revealed genotype distributions and allele frequencies which were not necessarily the same between the three racial groups, but which were not significantly different using a chi-square test. Usually, the frequency of the minor allele for these SNPs was exceedingly low (though at least 1% in one of the racial groups; Table 14-2), and we discarded these SNPs from further analysis as well.

Sixty-seven (67) of the 96 validated SNP markers revealed genotype distributions and allele frequencies that were statistically different between the three ethnic groups (Table 3). Minor alleles for each of these 68 SNP markers were preferentially represented in one of the three major racial groups tested (Asians, African Americans or Caucasians) and many of these SNPs showed dramatic differences between the groups. All three of the possible preference categories were observed; preferentially present in the Caucasian population (n=25), preferentially present in the Asian population (n=10) and preferentially present in the African American population (n=32). Most of the SNP markers had alleles that were in Hardy-Wicnbcrg Equilibrium (HWE) (data not shown). Three of the 67 SNPs were not in HWE, likely because the assay for these SNPs co-amplified competing sequences, but because there were discrete classes of alleles (i.e., XX, XY and YY), because the results were reproducible, and, because there were racial differences in genotypes, we included them in this analysis. Table 14-3 shows SNP markers for which genotype distributions and allele frequencies were significantly different between the racial classes. Nucleotide composition for the SNP markers listed in Table 14-3 are shown in Table 1 (three were discarded due to high failure rates).

The breakdown of the number of SNPs per gene, with minor allele frequencies that were different between the three racial groups, reveal that most of the useful SNPs were in the OCA2 gene (n=18; Table 14-4). OCA2 is an oculocutaneous albinism gene that plays a role in the synthesis of eumelanin. The second most number of racially informative SNPs was found in the CYP2D6 gene (n=12). By gene type, 85% of the pigmentation gene SNPs (TYR, TYRP1, MC1R and OCA2) were racially informative (33/39) and the variance of the ratio of racially informative/total SNPs tested within this class of genes was remarkably low (i.e., each of the genes had a similar ratio). In contrast, only 61% of the xenobiotic metabolism SNPs were racially informative (28/46). As with the pigmentation gene class, the variance of the ratio of racially informative SNPs to uninformative was very low. Lastly, SNPs from two non-pigmentation or xenobiotic metabolism genes were also tested, and 28% of these SNPs were racially informative (6/21). Because the minor alleles for most of the SNPs in these two genes were relatively rare, when adjusted for frequency, the percentage of the total number of racially informative alleles counted is closer to 1%. Corrected by the number of SNPs tested per gene, the OCA2, TYR, TYRP1 genes, all pigmentation genes, minor alleles with frequencies that were most often distinct between the racial groups.

To develop a classifier using these SNPs, a linear classification algorithm was developed and implemented. The algorithm computes a varianee/covariance matrix for all possible trait class pairs, represents individual samples as n-dimensional vectors (n=number of markers), measures average distances between these vectors and class (race) mean vectors and then classifies the sample into the class for which the distance is lowest (See Example 15 for more details). Using an iterative sampling scheme, the sample mean vectors are rendered unbiased estimates. Missing data complicated the analysis using this scheme, so we discarded markers 217487, 217439, 664784, 217460, 217473, 615925 and 664785, which had high failure rates in at least one of the three racial groups. Using the sixty SNP markers that were left after this subtraction, individual differences from the mean of each class were calculated for the 230 individuals of African (AA), Asian (AI) and Caucasian (CA) descent (the same individuals genotyped in Table 3, no racial mixtures) and each was classified into one of the racial groups to produce an exclusion probability matrix (Table 14-5).

From the resulting class (race) exclusion probability matrix, we observed extremely low corrected probabilities (See Example 15 for more details) of excluding an AA individual from the AA group (pr=0.0016), an AI individual from the AI group (pr=0.0001) and a CA individual from the CA group (pr<0.0001; Table 14-5). Uncorrected probabilities were equally impressive (Table 14-5). These probabilities exceeded those produced by Shriver et al. (1997) using STR markers, which were claimed to be log likelihood about 3, or about 1 in 1,000 (though see discussion for criticisms). Corrected probabilities for excluding individuals from incorrect racial groups were generally very high - the lowest less than 1 in 10,000 (AA misclassified as CA, row one, column 3, Table 14-5).

Because genotyping expense for a sample is in direct proportion to the number of markers tested, the exclusion probabilities for a smaller group of SNPs were calculated. A subset of 15 of the 60 markers were randomly selected and classified them using the linear classifier (Example 15), a similar number as that required for the production of log10=3 exclusion probabilities using selected STR markers (17; Shriver et al., 1997). Exclusion probabilities were poor, the probability of excluding an AA individual from the AA group (pr=0.143), an AI individual from the AI group (pr=0.148) and a CA individual from the CA group (pr<0.096) were generally not suitable for forensics purposes (Table 14-6). Given that bi-allelic markers possess less information than multi-allelic markers, this result was not unexpected.

To determine whether the 60-SNP classifier model generalized well, the classifier was used to categorize an additional 275 unrelated Caucasians and 12 unrelated African Americans (none of the individuals were racial mixtures).

These individuals were not included in the resequencing group or the group of 230 individuals used to generate the classifier model. The accuracy for Caucasian classification was 100% (275/275 classified as Caucasian) and the accuracy for classifying the 12 individuals of African descent was also 100% (12/12). Given the previously described results, 505/505 individuals were classified with perfect results.

## DISCUSSION

A battery of 60 SNPs within the human pigmentation and xenobiotic metabolism genes were identified that can be used to reliably classify an individual DNA specimen into one of three major racial groups. Using a sample of 275 individuals, the estimated exclusion probabilities for cognate classifications was very low (less than 1 in 10,000). Applied for the classification of 505 individuals, the classifier showed perfect accuracy. In order to guide a criminal investigation based on DNA sequence, or to justify the use a specific reference population for statistical calculations, the power of racial exclusion must be extremely high, and the classifier we have described appears to be quite promising in light of this requirement. Though the estimates disclosed herein are believed to be unbiased, the next step is to validate the estimates of exclusion in larger populations of African, Caucasian and Asian individuals, as well as in other racial groups (Latinos, Middle Eastern, etc.). Further, the classifier disclosed in this Example needs to be tested for its ability to resolve between ethnic groups within races (i.e., Japanese, Korean, and Chinese, within the Asian group). Nonetheless, until Shriver et al. (1997) described how STR markers could be used for racial profiling, DNA testing was merely a quantitative tool capable of producing numeric "bar-codes" for matching specimens and individuals. The classifier disclosed herein is the third qualitative forensics tool (Shriver et al., 1997) and second racial classifier yet discovered.

To find good SNP markers of race, the human pigmentation and xenobiotic genes were targeted with the assumption that these genes had been subject to unusually strong systematic genetic forces over the course of human evolution. For the pigmentation genes, a prediction was made that sexual selection and geographical isolation had affected gene sequence distributions between the worlds various racial groups. For the xenobiotic genes, it was reasoned that unique diets in the various regions of the world had imposed unique and powerful constraints on sequence diversity within and between racial groups (i.e., geographical isolation and possibly, selection). Previous screens for racially informative STR markers have proven difficult due to their rarity. In one screen of 1,000 STR loci (Shriver et al., 1997), racial allele distributions were found for only 17 (1.7%, though this is likely to be a low estimate of their frequency in the genome due to the sample sizes used for each STR).

Single nucleotide polymorphisms (SNPs) were surveyed from two non-pigmentation and non-xenobiotic metabolism genes (HMGCR, FDPS), and disclosed a somewhat higher percentage of SNPs to be of value for predicting race (about 28%). How typical these two genes are is not clear, but many of the SNPs in these genes were not frequent so their racial value is subject to more debate. In fact, when adjusted for allelic frequency, the percentage of racially informative minor alleles counted in these genes, with respect to the total number counted for all genes, is closer to 1%. In contrast, the frequency of racially informative SNPs in the human pigmentation and xenobiotic metabolism genes was significantly higher; 85% (33/39) of the pigmentation gene SNPs and 61 % (28/46) of the xenobiotic metabolism gene SNPs were racially informative. The total number of counted minor alleles from these genes included over 99% the total number counted, though they represented only 80% (85/106) the total number of validated SNPs studied. These results confirm that systematic forces shape pigmentation and xenobiotic metabolism gene allelic variance between these three racial groups, and that the disclosed strategy can be used for identifying racially informative markers by targeting these genes. Further, these results indicate that the model generated herein can be extended well to other racial groups.

The racial classifier disclosed herein was developed from 230 individuals of African, Asian and Caucasian descent. Its performance was confirmed in another group of 287 individuals. Though 505 individuals were used to develop and test the classifier, larger sample sizes will almost certainly drop the exclusion probabilities because many of the racially informative markers were monomorphic in one or more of the racial groups. This situation precludes their use with the quadratic classifier (See Example 15), which generally produces a superior result. Nonetheless, the statistical problems associated with monomorphism are less influential than with STR markers because a) we used a linear classification approach rather than log likelihood, and b) with STR markers, monomorphism is more likely to exist for several alleles at a given locus, whereas with SNP markers it can exist with only one. By increasing the sample sizes by a factor of only 2, we are likely to be able to apply the geometric classifier for all 60 SNPs. Further, by increasing the number of racially mixed individuals in future studies, the disclosed linear classifier, or a quadratic one, can be one of the first classifiers capable of resolving racially mixed individuals. It is anticipated that, because our classifier relies on individual vector differences from the mean, and because mixed individuals are likely to be evenly mixed for a majority of alleles, the probabilities of exclusion from homogeneous racial groups is likely to be greater than for mixed groups made of them. Previous methods with STR markers did not test racial mixes (within individuals), though because they rely on log likelihood ratios and their alleles are heterogeneous, it is unlikely that they would be powerful enough to resolve them satisfactorily without invoking a number of significant digits illegitimate for the sample sizes used in their generation.

The accuracy of correctly classifying individuals of African descent was the lowest of the three racial groups (misclassification 2 in 1000). This result is interesting because the age of the African lines, and the genetic complexity of Africans, in general, is the greatest among the worlds various racial groups (Tishkoff et al., 2000; Mateu et al., 2001).

Previous STR methods described alleles with log10 = 1.858 (r = 72) in power for discriminating between individuals of African versus European origin. Other statistical measures of the same data gave lower values (log 1 0 = 1.59; Erikson and Svensmark, Int. J. Legal Med. 106:254-257, 1994). It would appear that "by all accounts, the FY- locus is a powerful marker for discriminating between individuals of African versus Caucasian origin" and that "in 96% of the cases in which an unknown stain donor is African American, this locus alone will answer the question of ethnic origin" (Brenner, Proceedings 7th Intl. Symposium on Hum. Identification 4892, 1997). However, Brenner performed Monte Carlo computer simulations which suggested that the 17 markers were discovered from the approximately 1,000 canvassed due to sampling bias rather than due to their true value as markers of race. Brenner thus proposed that the procedure used could be successful in identifying "a set of 10 loci that differentiate the 9-year-old children from the 10-year-olds in the local playground". He also further criticized the STR methods by posing an interesting question about the confounding affects of allelic association between STR loci.

Herein lie the main deficiencies of the STR based approach for racial classification. Because small number of complex loci are used, low sample sizes are for STR allele classes are invoked. As a result, estimated parameters can be (and are often) distorted. Further, because of the small numbers of loci, linkage effects between loci that muddle the data are magnified. SNP based methodologies, such as that disclosed herein, offer an alternative for overcoming these deficiencies. Due to higher minor allele frequencies, which can actually be crafted from very large numbers of candidate SNPs, estimated parameters such as allele frequency estimation are more likely to be unbiased and, therefore, useful for their intended purpose. Due to the larger number of loci used (60 in our battery versus 14 in Shriver's 1997 STR battery), linkage problems that bias the sample size towards one or another conclusion are minimized. The allele frequencies of the SNPs as disclosed herein are higher, the sample sizes used to estimate these frequencies greater, and the reliability of our frequency estimates superior. As a result, the discriminatory power of the SNP battery disclosed herein is significantly greater than this STR method (exclusion probabilities exceeding 1 in 10,000 versus 1 in less than 1000). Thus, the classifier not only is the first SNP base method for reliably distinguishing between the world's major racial groups, but also can be the best method for this purpose *de facto.*

Even if the inertia for changing from STR to SNP based methods is great, the SNP battery also is useful as a complement to current testing approaches. In particular, the battery disclosed herein can be useful for both racial classification and human identification in cases where sample integrity is a problem. STR tests require DNA that is generally intact because STR regions arc amplified from the DNA in a manner that is effectively sensitive to the concentration of intact DNA sequence between the primers used. For a given level of DNA degradation, the chance of successful amplification (and typing) of lengthy targets is lower than for shorter targets because the probability of discontinuity between PCR primers increases as the length between the primers increases. Because the probability that a polymorphic site is successfully amplified for genetic typing is a function of the length of the amplification product, the amount of DNA used and the degree of DNA degradation, all other things being equal, the disclosed battery of 60 SNPs provides advantages where there is a small amount of DNA available and/or the DNA is degraded. Because the amount and integrity of DNA is often suboptimal for forensic investigations, the disclosed battery can provide a useful adjunct to current STR based methods. In cases of extreme sample limitation, mitochondrial DNA approaches are preferred, though no mitochondrial method has, to our knowledge, yet been described for racial classification.

**TABLE 14-1**

| Marker | XX ASIAN | XY ASIAN | YY ASIAN | XX AFRICAN AMERICAN | XY AFRICAN AMERICAN | YY AFRICAN AMERICAN | XX CA | XY CA | YY CA |
|---|---|---|---|---|---|---|---|---|---|
| 809123 | 25 | 5 | 0 | 77 | 12 | 0 | 71 | 16 | 0 |
| 809126 | 0 | 2 | 28 | 1 | 8 | 81 | 0 | 5 | 83 |
| 869756 | 26 | 0 | 0 | 60 | 2 | 0 | 69 | 0 | 0 |
| 869766 | 30 | 0 | 0 | 87 | 3 | 0 | 87 | 0 | 0 |
| 869806 | 0 | 11 | 19 | 6 | 34 | 50 | 5 | 32 | 51 |
| 971872 | 30 | 0 | 0 | 86 | 3 | 0 | 83 | 3 | 0 |

Table 14-1 provides examples of SNP markers for which genotype distributions and allele frequencies were not significantly different between the racial classes. Only a few of the SNP markers of this class are shown. Each row shows the data for a single SNP, which is referred to as a "marker". Individual counts for these markers are shown. Within each racial group (shown at the top of the table), counts for the allele 1 homozygote class (XX): the heterozygote class (XY): and the allele 2 (YY) homozygote class arc shown.

**TABLE 14-2**

| Marker | XX ASIAN | XY ASIAN | YY ASIAN | XX AFRICAN AMERICAN | XY AFRICAN AMERICAN | YY AFRICAN AMERICAN | XX CA | XYCA | YYCA |
|---|---|---|---|---|---|---|---|---|---|
| 869780 | 25 | 0 | 0 | 87 | 1 | 0 | 75 | 0 | 0 |
| 951520 | 29 | 1 | 0 | 90 | 0 | 0 | 87 | 1 | 0 |

Table 14-2 shows SNP markers for which genotype distributions and allele frequencies were not significantly different between the racial classes. Only a few of the SNP markers of this class arc shown. Each row shows the data for a single SNP (''marker"). Individual counts for these markers arc shown. Within each racial group (shown at the top of the table), counts for the allele 1 homozygote class (XX): the heterozygote class (XY): and the allele 2 (YY) homozygote class are shown.

**TABLE 14-3**

| Marker | XX ASIAN | XY ASIAN | YY ASIAN | XX AFRICAN AMERICAN | XY AFRICAN AMERICAN | YY AFRICAN AMERICAN | XX CA | XY CA | YY CA | SEQ ID NO: |
|---|---|---|---|---|---|---|---|---|---|---|
| 217438 | 15 | 15 | 0 | 88 | 2 | 0 | 73 | 14 | 0 | 4 |
| 217439 | 30 | 0 | 0 | 85 | 0 | 0 | 73 | 2 | 0 | 5 |
| 217441 | 29 | 0 | 0 | 86 | 2 | 0 | 74 | 13 | 0 | 6 |
| 217452 | 30 | 0 | 0 | 61 | 2 | 27 | 74 | 0 | 14 | 11 |
| 217455 | 10 | 16 | 4 | 61 | 23 | 5 | 7 | 32 | 49 | 21 |
| 217456 | 29 | 1 | 0 | 87 | 3 | 0 | 73 | 15 | 0 | 35 |
| 217459 | 59 | 0 | 0 | 166 | 10 | 0 | 175 | 0 | 0 | 52 |
| 217460 | 0 | 0 | 27 | 2 | 31 | 46 | 0 | 0 | 86 | 53 |
| 217468 | 26 | 0 | 0 | 81 | 8 | 1 | 32 | 46 | 10 | 43 |
| 217473 | 40 | 1 | 0 | 138 | 17 | 0 | 103 | 55 | 0 | 44 |
| 217480 | 30 | 0 | 0 | 86 | 0 | 0 | 83 | 5 | 0 | 41 |
| 217485 | 28 | 0 | 0 | 71 | 19 | 7 | 14 | 46 | 39 | 45 |
| 217486 | 28 | 1 | 0 | 64 | 20 | 5 | 12 | 42 | 34 | 46 |
| 217487 | 0 | 10 | 0 | 23 | 18 | 0 | 59 | 7 | 0 | 54 |
| 217489 | 0 | 3 | 53 | 16 | 53 | 100 | 70 | 76 | 18 | 55 |
| 554353 | 29 | 0 | 0 | 89 | 0 | 0 | 83 | 3 | 0 | 56 |
| 554363 | 0 | 3 | 27 | 1 | 5 | 84 | 0 | 0 | 88 | 57 |
| 554368 | 28 | 0 | 0 | 88 | 1 | 0 | 83 | 5 | 0 | 58 |
| 554370 | 29 | 0 | 0 | 64 | 14 | 10 | 78 | 0 | 0 | 59 |
| 554371 | 7 | 12 | 9 | 67 | 7 | 14 | 54 | 12 | 19 | 60 |
| 615921 | 30 | 0 | 0 | 81 | 6 | 2 | 86 | 1 | 0 | 61 |
| 615925 | 29 | 0 | 0 | 44 | 9 | 15 | 44 | 15 | 10 | 62 |
| 615926 | 51 | 6 | 0 | 118 | 62 | 0 | 130 | 45 | 0 | 63 |
| 664784 | 0 | 30 | 29 | 0 | 90 | 65 | 0 | 88 | 62 | 64 |

(continued)

| Marker | XX ASIAN | XY ASIAN | YY ASIAN | XX AFRICAN AMERICAN | XY AFRICAN AMERICAN | YY AFRICAN AMERICAN | XX CA | XY CA | YY CA | SEQ ID NO: |
|---|---|---|---|---|---|---|---|---|---|---|
| 664785 | 0 | 34 | 17 | 0 | 213 | 6 | 0 | 193 | 7 | 65 |
| 664793 | 0 | 0 | 30 | 0 | 12 | 78 | 0 | 5 | 83 | 66 |
| 664802 | 30 | 0 | 0 | 81 | 9 | 0 | 88 | 0 | 0 | 67 |
| 664803 | 0 | 0 | 30 | 0 | 35 | 15 | 0 | 5 | 81 | 68 |
| 712037 | 16 | 12 | 2 | 11 | 53 | 11 | 76 | 12 | 0 | 69 |
| 712047 | 8 | 22 | 0 | 62 | 28 | 0 | 12 | 76 | 0 | 70 |
| 712051 | 30 | 0 | 0 | 78 | 12 | 0 | 88 | 0 | 0 | 71 |
| 712052 | 14 | 10 | 6 | 2 | 15 | 73 | 5 | 32 | 51 | 12 |
| 712054 | 4 | 17 | 9 | 12 | 45 | 33 | 17 | 45 | 26 | 18 |
| 712055 | 0 | 2 | 28 | 7 | 37 | 45 | 0 | 9 | 78 | 72 |
| 712057 | 0 | 10 | 20 | 49 | 36 | 5 | 64 | 20 | 4 | 14 |
| 712058 | 3 | 6 | 21 | 55 | 29 | 4 | 75 | 12 | 0 | 15 |
| 712059 | 4 | 17 | 9 | 12 | 45 | 33 | 17 | 45 | 26 | 73 |
| 712064 | 9 | 15 | 6 | 0 | 1 | 89 | 0 | 0 | 88 | 17 |
| 712043 | 29 | 0 | 1 | 84 | 6 | 0 | 69 | 18 | 1 | 74 |
| 756239 | 0 | 0 | 30 | 0 | 18 | 72 | 0 | 0 | 88 | 75 |
| 756251 | 30 | 0 | 0 | 74 | 15 | 1 | 56 | 30 | 2 | 76 |
| 809125 | 29 | 0 | 1 | 83 | 6 | 0 | 69 | 18 | 1 | 77 |
| 869745 | 0 | 0 | 30 | 1 | 5 | 84 | 0 | 0 | 88 | 48 |
| 569769 | 8 | 15 | 6 | 11 | 33 | 45 | 5 | 31 | 52 | 78 |
| 869772 | 29 | 1 | 0 | 48 | 32 | 10 | 87 | 1 | 0 | 79 |
| 869777 | 4 | 16 | 10 | 16 | 31 | 43 | 22 | 33 | 33 | 80 |
| 869784 | 7 | 23 | 0 | 3 | 87 | 0 | 4 | 83 | 1 | 81 |
| 869785 | 30 | 0 | 0 | 70 | 12 | 8 | 88 | 0 | 0 | 82 |

(continued)

| Marker | XX ASIAN | XY ASIAN | YY ASIAN | XX AFRICAN AMERICAN | XY AFRICAN AMERICAN | YY AFRICAN AMERICAN | XX CA | XY CA | YY CA | SEQ ID NO: |
|---|---|---|---|---|---|---|---|---|---|---|
| 869787 | 0 | 0 | 30 | 1 | 5 | 84 | 0 | 0 | 88 | 47 |
| 869794 | 0 | 1 | 27 | 0 | 2 | 87 | 1 | 28 | 59 | 83 |
| 869797 | 0 | 0 | 30 | 14 | 17 | 59 | 10 | 19 | 59 | 84 |
| 869798 | 0 | 0 | 30 | 0 | 20 | 70 | 0 | 0 | 87 | 85 |
| 869802 | 0 | 5 | 25 | 0 | 20 | 70 | 0 | 0 | 83 | 86 |
| 869809 | 0 | 0 | 30 | 0 | 3 | 87 | 1 | 9 | 77 | 87 |
| 869810 | 0 | 5 | 25 | 0 | 2 | 88 | 1 | 10 | 77 | 88 |
| 869813 | 0 | 0 | 30 | 2 | 17 | 71 | 0 | 0 | 87 | 89 |
| 886892 | 0 | 0 | 30 | 0 | 4 | 86 | 0 | 17 | 71 | 23 |
| 886894 | 18 | 9 | 2 | 64 | 22 | 4 | 11 | 44 | 33 | 8 |
| 886895 | 19 | 8 | 3 | 10 | 36 | 44 | 1 | 22 | 65 | 9 |
| 886896 | 27 | 3 | 0 | 64 | 21 | 4 | 11 | 45 | 32 | 10 |
| 886933 | 1 | 6 | 23 | 4 | 33 | 53 | 0 | 13 | 75 | 49 |
| 886934 | 0 | 0 | 30 | 0 | 2 | 88 | 0 | 14 | 74 | 90 |
| 886937 | 30 | 0 | 0 | 81 | 8 | 1 | 74 | 14 | 0 | 50 |
| 886993 | 29 | 1 | 0 | 22 | 41 | 27 | 47 | 37 | 2 | 91 |
| 886994 | 0 | 1 | 29 | 28 | 40 | 22 | 2 | 38 | 47 | 13 |
| 951497 | 19 | 11 | 0 | 47 | 37 | 6 | 67 | 21 | 0 | 42 |
| 951526 | 0 | 0 | 30 | 2 | 13 | 73 | 0 | 0 | 85 | 92 |

Table 14-3 shows SNP markers for which genotype distributions and allele frequencies were significantly different between the racial classes. The results show genotype counts in 30 Asians, 100 Africans and 100 Caucasians, though five of the SNP markers were genotyped in sample sizes that were roughly double these numbers. SNP unique identifiers are shown in column 1, and the XX, XY and YY allele counts are shown for each of the three racial groups listed at the top of the table.

**TABLE 14-4**

| GENE | NO. SNPS | TOTAL TESTED |
|---|---|---|
| OCA2 | 18 | 19 |
| CYP2D6 | 12 | 21 |
| TYRP1 | 8 | 9 |
| CYP2C9 | 7 | 14 |
| CYP3A4 | 4 | 8 |
| TYR | 4 | 5 |
| HMGCR | 4 | 13 |
| MC1R | 3 | 6 |
| FDPS | 2 | 8 |
| AHR | 1 | 3 |
| CYP1A1 | 1 | 2 |
| TOTAL | 64 | 108 |

**TABLE 14-5**

| | AA | | AI | | CA | |
|---|---|---|---|---|---|---|
| | Correction | No Correction | Correction | No Correction | Correction | No Correction |
| AA | 0.00189 | 0.00161 | 0.99998 | 0.99998 | 0.99974 | 0.99976 |
| AI | 0.99999 | 0.99999 | 0.00013 | 0.00011 | 0.99999 | 0.99999 |
| CA | 0.99999 | 0.99999 | 0.99999 | 0.99999 | 0.00006 | 0.00005 |

Table 5 shows a racial exclusion probability matrix derived from the linear classifier for individuals of African (AA), Asian (AI) and Caucasian (CA) descent using the 60 SNP markers described in the text. Because the number of Asians in this analysis (15) was lower than the number of markers, we broke the analysis into 4 groups of 15 markers, calculated the variance covariance matrix using all 230 individuals for each group of SNPs and generated an exclusion matrix for each. These were then combined into one matrix by calculating the exclusion probability as Γlx, from x-SNP group 1 to SNP group 4 for each cell. Though perfect classification results were obtained with our sample of 505 individuals, the exclusion probability matrix is composed of non-zero values due to the implementation of this particular sampling method. To generate the composite classifier, zero probabilities present in a group were arbitrarily adjusted to 0.01 to avoid multiplication by zero (this occurred only for A1 cells, due to the low AI sample size of 15). The matrix is square because of asymmetry in ordinate metrics; the X ordinate represents class means and the Y ordinate represents classification frequencies.

**TABLE 14-6**

| | AA | | AI | | CA | |
|---|---|---|---|---|---|---|
| | Correction | No Correction | Correction | No Correction | Correction | No Correction |
| AA | 0.14290 | 0.14290 | 0.98700 | 0.98700 | 0.87010 | 0.87010 |
| AI | 0.96300 | 0.96300 | 0.18520 | 0.14810 | 0.85190 | 0.88890 |
| CA | 0.97590 | 0.97590 | 0.91570 | 0.92770 | 0.10840 | 0.09640 |

Table 6 shows a racial exclusion probability matrix derived from the linear classifier for individuals of African (AA), Asian (AI) and Caucasian (CA) descent using a randomly selected set of 15 SNP markers of the 60 described in the text.

## EXAMPLE 15

## CLASSIFIER TOOL

This example discloses an innovative linear and quadratic classifier construction tool for multivariate trait classification using multi-locus genotypes. A software-based method was developed for incorporating multiple genetic attributes into a linear and/or quadratic classifier. This method has certain strengths and weaknesses over other approaches such as Correspondence analysis method and the Classification Tree method. The latter method is best suited for situations where the trait is subject to genetic dominance. The disclosed linear and quadratic methods, which use sample means as a basis for classification, are superior in cases where the trait is subject of additive effects but not genetic dominance. The method is as easily applied for haplotype or phase-unknown analysis and performs well whatever the marker type (RFLP, STR, SNP etc.).

The problem of classifying a given individual as a member of one of several populations or groups to which that particular individual can possibly belong is of interest to many types of scientists, including, for example, statisticians, geneticists, anthropologists, taxonomists, psychologists and sociologists. There are mainly 3 approaches in the classification analysis, namely, 1) parametric, 2) semi-parametric, and 3) non-parametric and their robust (Balakrishnan, et al., Handbook of Statistics 1991; 8:145-202.) versions. In each approach, many contributions have been made by various authors (McLachlan, G.J., Wiley, New York, 1992.). Though linear and quadratic classification procedures have been well documented in the literature, few algorithms have been generated for their implementation as software tools within the field of complex genetics. Disclosed herein is the implementation of a parametric multivariate linear classification (Fisher, 1936) and Quadratic classification (Anderson, T.W., Introductin to Multivariate Statistical Analysis. Wiley, New York 1958; Srivastava et al., Mykosen. 1979 Sep;22(9):311-3; Srivastava, M.S. ct al., "An introduction to multivariate statistics," North Holland, Amsterdam: 1979) with their modifications for genomics data (Spilman et al., 1976, Smouse, P.E., et al., Genetics 1977; 85:733-752):

Under the assumption that the samples have been taken from multivariate normal distributions with different mean vectors with common variance covariance matrix, linear classification procedure introduced by Fisher (1936), Rao (1947, 1948a, 1948b), or Smith (1947) can be applied. However, if the populations have different variance covariance matrices, quadratic classification should be used. For the linear method, the pooled within-population variance-covariance matrix can be computed from the formula:

$$S = \Sigma^{p}_{i=1} \Sigma^{N_i}_{j=1} (Y_{ij} - \mu_i)(Y_{ij} - \mu_i)' / \Sigma(N_i - 1) \quad (1)$$

Where $Y_{ij}$ is the vector of character measurements for the j'th individual in the i'th trait value. $\mu_i$ and $N_i$ are the vector of means and sample size for the i'th trait value. The components for these vectors could be surrogate values for SNP alleles, each dimension of the vector representing a different locus. The components may or may not be linked to one another in gametic disequilibrium (i.e., it may or may not be part of a haplotype system). Indeed, this is a strength of the method - it is equally applicable to SNPs on different chromosomes as to those within a particular gene. The generalized distance of the ij'th individual from the mean of the k'th trait value can be computed from the formula:

$$D^2_{ij,k} = (Y_{ij} - \mu_k)' S^{-1} (Y_{ij} - \mu_k) \qquad \text{for } k \neq i \quad (2)$$

The vector $Y_{ij}$ is used to calculate $\mu_k$, the mean of it's own trait value. To avoid circularity caused by this, Smouse, *supra,* (1977) (*see also* Spielman, R.S.et al., Am. J Hum Genet. 1976; 28:317-331) used correction when comparing an element with its own class. In the case of complex genetics, we use this to correct for circularity caused by comparing an individual with the mean of its own trait value:

$$D^2_{ij,i} = (N_i/(N_i - 1))^2 (Y_{ij} - \mu_i)' S^{-1} (Y_{ij} - \mu_i) \quad (3)$$

The usual procedure is to allocate the ij'th individual to that trait value for which (2)/(3) is minimum. Large between class distances, relative to within class differences, provide justification for using the mean vector values for each class as a classifier tool. In this case, an unknown vector is compared to the mean vectors for the various classes, and the class that minimizes (2) and (3) is selected. Depending on the magnitude of (2) for the various classes, there may be ambiguity for some individual vectors, in which case the classifier can either produce a hybrid classification (a prediction of "mixture") or offer an inconclusive result. The result of applying (2) and (3) is a inclusion or exclusion probability matrix for the various trait classes.

A quadratic classification procedure for genetic classification can also be implemented. The quadratic discriminant score for the i'th trait value is:

$$D^2_{ij,k} = \ln|S_k| + (Y_{ij} - \mu_k)' S^{i}_{k}(Y_{ij} - \mu_k) \qquad \textit{for } k = 1,2,\ldots g(\textit{trait values}) \ (4)$$

Classification is then simply the allocation of the ij'th individual to that trait value for which (4) is minimum.

**EXAMPLE 16**

**RECORDING METHOD FOR IMPROVED CLASSIFICATION**

This example discloses a recording method for improving the classification analysis. Under the assumption of normality, the sample mean vector and the sample covariance matrix constitute minimally sufficient statistics, in the sense that any inference based of them carries with it all the information available in the sample.

Thus any classification rule based on these summary statistics ought to be optimal from the point of view of sample information used for their analysis. However it appears that the data can provide some additional information which are not reflected by these statistics. The question, therefore, is: Can this additional information be used for improving the results that were based on these statistics?

A closer scrutiny of the frequency distributions of gene-wise genotypes, based on the given sample data (for the 10 genes), reveal that some genotypes exhibit larger (relative) variations in their frequency of occurrences across colors than others (Table 16-1).

It is well known that those with larger variations in their (relative) frequencies, across the colors, have better discriminating ability for colors. From that context the genotypes g(1,1), g(2,3), g(3,1), g(4,1), g(5,1), g(6,2), g(7,2), g (8,2), g(9,2) and g(10.3) can be useful (and, therefore, stronger) for discrimination, both in terms of their frequencies as well as their ranges of variation, than the others, with the g(1,1), g(3,1) and g(4,1) being the relatively stronger among them (See Table 16-3 for coding key). Obviously, the next ranked genotypes within each gene have lesser strength for discrimination among colors. In the given data, keeping in view the total frequencies of their occurrences one can identify the following second ranked genotypes within each gene.

g(1,2),g(2,1),g(3,2),g(4,2),g(5,4),g(6,1),g(7,5), g(8,1), g(9,1) and g(10.103)

It can be noted that these genotypes have fairly large frequencies (≥ 5 in each color) and have weaker (than those that were ranked as 'best') discriminating power, (as their relative frequencies are almost equal across colors). One method of extracting more useful information from these genotypes could be to incorporate a 'measure of their association' with any or all of the above mentioned 'best' genotypes.

The procedure used in the present analysis is to recode the weaker genotypes whenever they appear along with the 'best' ones in a an individual sample unit. Specifically the procedure used is as follows:

Step 1. Identify a small number of 'best' genotypes for cross-coding the weak genotypes. This can be done by selecting a subset of the 'best' in each gene according to their range of variation in their relative frequencies. One can try various combinations and arrive at the optimal selection. Our study revealed an optimal choice of the three genotypes g(1,1) (OCA2A), g(3,1) (OCA2C) and g(4,1) (OCA2D).

Step 2: Recoding of second best genotypes:

Assign Code 0 if the genotype are absent

Assign Code 1+ (the number of selected 'best' genotypes it occurs together in an individual): For example if two of the best genotypes occurs in an individual, a weaker genotype score would be its value plus 1. Such recoding will generally increase the variability of scores across the colors (while carrying out the usual discriminant analysis), and hence one can expect a marginal improvement over the results obtained before incorporating such a recoding procedure in them.

There are some advantages and warning signals that go with the proposed methodology. Regarding advantages of the methodology, first, statistically, any attempt to increase the variability of the scores of genotypes across colors, should lead to a better classification since it increases the discriminating ability of the genotype. Second, if the result turns out to be relatively better, the method can provide clues or a source of hypotheses of the relationships between genotypes of different genes in relation to the phenotype, such as a pigmentation trait under study. Third, although the coding procedure may seem arbitrary, encouraging improvements, if any, may be important from a practical point of view , especially in the context of reducing the classification errors. Fourth, there are instances, especially in the area of statistical forecasting of time series, wherein data supported methods are recommended, as long as they lead to relatively more accurate inferences.

Regarding warning signals of the methodology, first, the arbitrary nature of the coding has to be justified from a theoretical point of view. Second, the sample size should be large enough for the recoded genotypes, so that the exercise does not become data specific.

The method was tried for the data involving 286 individuals with reference to the following 10 genes. OCA2A, OCA2B, OCA2C, OCA2D, OCA2E, MICRA, TYRA, TYRPA, TYRPB, AND DCT B.

Towards exploring the possibility of successive application of the method, the recoding exercise was carried out on the data set obtained after recoding the genotypes g(2,1),g(5,4),g(6,1),g(7,5), g(8,1), g(9,1) and g(10,103) with reference to the three 'best' genotypes selected, namely g(1,1) (OCA2A), g(3,1) (OCA2C) and g(4,1) (OCA1D). In this case relative frequencies were not obtained but the average scores for each genotypes (since some codes are larger than unity) .(Table 16-2 is the reflection of Table 16-1 at this stage).

Using these averages three 'best' genotype were identified as g(2,1), g(3,1) and g(4,1). At this stage the genotypes g(1,2),g(4,5),g(5,1),g(7,1),g(8,1),g(9,2) and g(10,1) were recoded with reference to the genotypes g(2,1), g (3,1) and g(4,1) using the same recoding procedure.

**TABLE 16-1**

| genotype | | Blue | green | hazel | brown | | range |
|---|---|---|---|---|---|---|---|
| | | | | | | | |
| G(1,1) | | 0.56701 | 0.386667 | 0.366667 | 0.511905 | | 0.200344 |
| G(1,2) | | 0.14433 | 0.226667 | 0.233333 | 0.166667 | | 0.089003 |
| G(1,3) | | 0.041237 | 0.013333 | 0 | 0 | | 0.041237 |
| G(1.4) | | 0.051546 | 0.013333 | 0.033333 | 0.02381 | | 0.038213 |
| G(1,5) | | 0.103093 | 0.12 | 0.166667 | 0.166667 | | 0.063574 |
| G(1,6) | | 0 | 0.026667 | 0 | 0 | | 0.026667 |
| g(1,7) | | 0.010309 | 0 | 0 | 0 | | 0.010309 |
| g(1,8) | | 0.010309 | 0.026667 | 0 | 0 | | 0.026667 |
| g(1,9) | | 0.020619 | 0.066667 | 0.133333 | 0 | | 0.133333 |
| g(1,10) | | 0.010309 | 0.053333 | 0 | 0.02381 | | 0.053333 |
| g(1,11) | | 0 | 0 | 0 | 0.011905 | | 0.011905 |
| g(1,12) | | 0.010309 | 0.026667 | 0.1 | 0.059524 | | 0.089691 |
| g(1,13) | | 0.010309 | 0 | 0.033333 | 0 | | 0.033333 |
| g(1,14) | | 0 | 0.013333 | 0 | 0 | | 0.013333 |
| g(1,15) | | 0.010309 | 0 | 0 | 0 | | 0.010309 |
| g(1,16) | | 0 | 0.013333 | 0 | 0 | | 0.013333 |
| g(1,17) | | 0 | 0 | 0 | 0.011905 | | 0.011905 |
| g(1,18) | | 0.010309 | 0 | 0 | 0 | | 0.010309 |
| | | | | | | | |
| g(2,1) | | 0.371134 | 0.306667 | 0.3 | 0.416667 | | 0.116667 |
| g(2,2) | | 0.164948 | 0.186667 | 0.133333 | 0.154762 | | 0.053333 |

(continued)

| genotype | | Blue | green | hazel | brown | | range |
|---|---|---|---|---|---|---|---|
| g(2,3) | | 0.319588 | 0.226667 | 0.233333 | 0.154762 | | 0.164826 |
| g(2,4) | | 0.030928 | 0.026667 | 0.033333 | 0.071429 | | 0.044762 |
| g(2,5) | | 0 | 0 | 0 | 0.02381 | | 0.02381 |
| g(2,6) | | 0.051546 | 0.106667 | 0.166667 | 0.047619 | | 0.119048 |
| g(2,7) | | 0.010309 | 0.04 | 0 | 0.02381 | | 0.04 |
| g(2,8) | | 0 | 0 | 0.033333 | 0 | | 0.033333 |
| g(2,9) | | 0.041237 | 0.026667 | 0.066667 | 0.035714 | | 0.04 |
| g(2,10) | | 0.010309 | 0.04 | 0.033333 | 0 | | 0.04 |
| g(2,11) | | 0 | 0.04 | 0.033333 | 0.011905 | | 0.04 |
| g(2,12) | | 0 | 0 | 0.033333 | 0.011905 | | 0.033333 |
| | | | | | | | |
| g(3.1) | | 0.701031 | 0.6 | 0.4 | 0.52381 | | 0.301031 |
| g(3,2) | | 0.154639 | 0.12 | 0.266667 | 0.202381 | | 0.146667 |
| g(3.3) | | 0.041237 | 0.053333 | 0.033333 | 0.011905 | | 0.041429 |
| g(3.4) | | 0.092784 | 0.106667 | 0.166667 | 0.059524 | | 0.107143 |
| g(3,5) | | 0 | 0 | 0.033333 | 0 | | 0.033333 |
| g(3.6) | | 0 | 0.066667 | 0.033333 | 0.083333 | | 0.083333 |
| g(3,7) | | 0 | 0.026667 | 0 | 0.02381 | | 0.026667 |
| g(3,8) | | 0 | 0 | 0.066667 | 0.02381 | | 0.066667 |
| g(3.9) | | 0 | 0.026667 | 0 | 0 | | 0.026667 |
| g(3,10) | | 0 | 0 | 0.033333 | 0 | | 0.033333 |
| g(3,11) | | 0 | 0 | 0 | 0.035714 | | 0.035714 |
| g(3,12) | | 0 | 0 | 0 | 0.011905 | | 0.011905 |
| g(3,13) | | 0.010309 | 0 | 0 | 0 | | 0.010309 |
| g(4,1) | | 0.371134 | 0.253333 | 0.433333 | 0.404762 | | 0.18 |
| g(4,2) | | 0.453608 | 0.44 | 0.366667 | 0.369048 | | 0.086942 |
| g(4,3) | | 0.010309 | 0.013333 | 0.033333 | 0.035714 | | 0.025405 |
| g(4,4) | | 0.010309 | 0.013333 | 0 | 0 | | 0.013333 |
| g(4,5) | | 0.113402 | 0.226667 | 0.166667 | 0.095238 | | 0.131429 |
| g(4,6) | | 0.041237 | 0.026667 | 0.033333 | 0.059524 | | 0.032857 |
| g(4,7) | | 0 | 0.013333 | 0 | 0 | | 0.013333 |
| g(4,8) | | 0 | 0 | 0 | 0.011905 | | 0.011905 |
| | | | | | | | |
| g(5,1) | | 0.608247 | 0.453333 | 0.533333 | 0.547619 | | 0.154914 |
| g(5,2) | | 0.092784 | 0.186667 | 0.166667 | 0.107143 | | 0.093883 |
| g(5,3) | | 0.134021 | 0.173333 | 0.066667 | 0.142857 | | 0.106667 |
| g(5,4) | | 0.14433 | 0.12 | 0.2 | 0.083333 | | 0.116667 |
| g(5.5) | | 0.010309 | 0 | 0.033333 | 0.011905 | | 0.033333 |

(continued)

| genotype | | Blue | green | hazel | brown | | range |
|---|---|---|---|---|---|---|---|
| g(5,6) | | 0 | 0.013333 | 0 | 0 | | 0.013333 |
| g(5,7) | | 0 | 0.026667 | 0.033333 | 0 | | 0.033333 |
| g(5,7) | | 0.010309 | 0.013333 | 0 | 0.011905 | | 0.013333 |
| g(5,8) | | 0 | 0.013333 | 0.033333 | 0.035714 | | 0.035714 |
| g(5.9) | | 0 | 0 | 0 | 0.011905 | | 0.011905 |
| g(5,10) | | 0 | 0 | 0 | 0.011905 | | 0.011905 |
| | | | | | | | |
| g(6.1) | | 0.56701 | 0.533333 | 0.5 | 0.607143 | | 0.107143 |
| g(6.2) | | 0.134021 | 0.08 | 0.266667 | 0.119048 | | 0.186667 |
| g(6.3) | | 0.113402 | 0.186667 | 0.1 | 0.119048 | | 0.086667 |
| g(6.4) | | 0.164948 | 0.133333 | 0.2 | 0.095238 | | 0.104762 |
| g(6.5) | | 0.010309 | 0.026667 | 0 | 0.011905 | | 0.026667 |
| g(6.6) | | 0 | 0.026667 | 0 | 0.011905 | | 0.026667 |
| g(6.7) | | 0.010309 | 0.013333 | 0 | 0 | | 0.013333 |
| | | | | | | | |
| g(7.1) | | 0.030928 | 0.12 | 0.133333 | 0.130952 | | 0.102405 |
| g(7.2) | | 0.309278 | 0.253333 | 0.166667 | 0.321429 | | 0.154762 |
| g(7.3) | | 0.247423 | 0.266667 | 0.133333 | 0.214286 | | 0.133333 |
| g(7,4) | | 0.010309 | 0 | 0.066667 | 0 | | 0.066667 |
| g(7.5) | | 0.247423 | 0.16 | 0.233333 | 0.095238 | | 0.152185 |
| g(7.6) | | 0030928 | 0.013333 | 0.1 | 0.011905 | | 0.088095 |
| g(7,7) | | 0010309 | 0.026667 | 0033333 | 0.011905 | | 0.023024 |
| g(7,8) | | 0.103093 | 0.12 | 0133333 | 0.142857 | | 0.039764 |
| g(7,9) | | 0010309 | 0.026667 | 0 | 0.02381 | | 0026667 |
| g(7.10) | | 0 | 0 | 0.066667 | 0.011905 | | 0.066667 |
| | | | | | | | |
| g(8.1) | | 0.402062 | 0.293333 | 0.3 | 0.321429 | | 0.108729 |
| g(8,2) | | 0463918 | 0.466667 | 0.566667 | 0.357143 | | 0.209524 |
| g(8,3) | | 0.041237 | 0.053333 | 0.033333 | 0.059524 | | 0.02619 |
| g(8,4) | | 0010309 | 0.013333 | 0 | 0 | | 0.013333 |
| g(8,5) | | 0.041237 | 0.106687 | 0.166667 | 0.119048 | | 0.12543 |
| | | | | | | | |
| g(9,1) | | 0.278351 | 0.213333 | 0.266667 | 0.261905 | | 0.065017 |
| g(9,2) | | 0.319588 | 0.306667 | 0.233333 | 0.202381 | | 0.117207 |
| g(9,3) | | 0.051546 | 0.053333 | 0 | 0.02381 | | 0.053333 |
| g(9,4) | | 0.041237 | 0.026667 | 0.066667 | 0.059524 | | 0.04 |
| g(9,5) | | 0.154639 | 0.16 | 0.166667 | 0.142857 | | 0.02381 |
| g(9,6) | | 0 | 0 | 0.033333 | 0 | | 0.033333 |

(continued)

| genotype | | Blue | green | hazel | brown | | range |
|---|---|---|---|---|---|---|---|
| g(9,7) | | 0.051546 | 0.066667 | 0.133333 | 0.071429 | | 0.081787 |
| g(9,8) | | 0.010309 | 0.066667 | 0 | 0.059524 | | 0.066667 |
| g(9,9) | | 0.061856 | 0.066667 | 0.166667 | 0.071429 | | 0.104811 |
| g(9,10) | | 0 | 0.013333 | 0 | 0.02381 | | 0.02381 |
| g(9,11) | | 0.030928 | 0.013333 | 0 | 0.047619 | | 0.047619 |
| | | | | | | | |
| g(10,1) | | 0.412371 | 0.373333 | 0.433333 | 0.369048 | | 0.064286 |
| g(10.2) | | 0.206186 | 0.24 | 0.266667 | 0.25 | | 0.060481 |
| g(10,3) | | 0.195876 | 0.24 | 0.166667 | 0.059524 | | 0.180476 |
| g(10,4) | | 0.030928 | 0.013333 | 0 | 0.011905 | | 0.030928 |
| g(10,5) | | 0 | 0.013333 | 0.033333 | 0.047619 | | 0.047619 |
| g(10,6) | | 0.051546 | 0.026667 | 0.1 | 0.059524 | | 0.073333 |
| g(10,7) | | 0.020619 | 0.013333 | 0 | 0.02381 | | 0.02381 |
| g(10.8) | | 0.010309 | 0.053333 | 0.066667 | 0.107143 | | 0.096834 |
| g(10.9) | | 0.010309 | 0 | 0 | 0 | | 0.010309 |
| g(10.10) | | 0.010309 | 0 | 0 | 0 | | 0.010309 |
| g(10.11) | | 0.041237 | 0.013333 | 0 | 0.047619 | | 0.047619 |
| g(10,12) | | 0.010309 | 0 | 0 | 0 | | 0.010309 |

**TABLE 16-2**

| genotype | | blue | green | hazel | brown | | range |
|---|---|---|---|---|---|---|---|
| | | | | | | | |
| g(1.1) | | 0.56701 | 0.386667 | 0.366667 | 0.511905 | | 0.200344 |
| g(1,2) | | 0.14433 | 0.226667 | 0.233333 | 0.166667 | | 0.089003 |
| g(1,3) | | 0.041237 | 0.013333 | 0 | 0 | | 0.041237 |
| g(1,4) | | 0.051546 | 0.013333 | 0.033333 | 0.02381 | | 0.038213 |
| g(1,5) | | 0.103093 | 0.12 | 0.166667 | 0.166667 | | 0.063574 |
| g(1,6) | | 0 | 0.026667 | | 0 | | 0.026667 |
| g(1,7) | | 0.010309 | 0 | 0 | 0 | | 0.010309 |
| g(1.8) | | 0.010309 | 0.026667 | 0 | 0 | | 0.026667 |
| g(1,9) | | 0.020619 | 0.066667 | 0.133333 | 0 | | 0.133333 |
| g(1,10) | | 0.010309 | 0.053333 | 0 | 0.02381 | | 0.053333 |
| g(1,11) | | | 0 | 0 | 0.011905 | | 0.011905 |
| g(1,12) | | 0 0.010309 | 0.026667 | 0.1 | 0.059524 | | 0.089691 |
| g(1,13) | | | 0 | 0.033333 | 0 | | 0.033333 |
| g(1,14) | | 0.010309 0 | 0.013333 | 0 | 0 | | 0.013333 |
| g(1,15) | | 0.010309 | 0 | 0 | 0 | | 0.010309 |
| g(1.16) | | 0 | 0.013333 | 0 | 0 | | 0.013333 |

(continued)

| genotype | | blue | green | hazel | brown | | range |
|---|---|---|---|---|---|---|---|
| | | | | | | | |
| g(1,17) | | 0 | 0 | 0 | 0.011905 | | 0.011905 |
| g(1,18) | | 0.010309 | 0 | 0 | 0 | | 0.010309 |
| | | | | | | | |
| g(2,1) | | 0.371134 | 0.306667 | 0.3 | 0.416667 | | 0.116667 |
| g(2,2) | | 0.164948 | 0.186667 | 0.133333 | 0.154762 | | 0.053333 |
| g(2,3) | | 0.319588 | 0.226667 | 0.233333 | 0.154762 | | 0.164826 |
| g(2.4) | | 0.030928 | 0.026667 | 0.033333 | 0.071429 | | 0.044762 |
| g(2,5) | | 0 | 0 | 0 | 0.02381 | | 0.02381 |
| g(2,6) | | 0.051546 | 0.106667 | 0.166667 | 0.047619 | | 0.119048 |
| g(2,7) | | 0.010309 | 0.04 | 0 | 0.02381 | | 0.04 |
| g(2.8) | | 0 | 0 | 0.033333 | 0 | | 0.033333 |
| g(2.9) | | 0.041237 | 0.026667 | 0.066667 | 0.035714 | | 0.04 |
| g(2,10) | | 0.010309 | 0.04 | 0.033333 | 0 | | 0.04 |
| g(2.11) | | 0 | 0.04 | 0.033333 | 0.011905 | | 0.04 |
| g(2,12) | | 0 | 0 | 0.033333 | 0.011905 | | 0.033333 |
| | | | | | | | |
| g(3,1) | | 0.701031 | 0.6 | 0.4 | 0.52381 | | 0.301031 |
| g(3,2) | | 0.154639 | 0.12 | 0.266667 | 0.202381 | | 0.146667 |
| g(3,3) | | 0.041237 | 0.053333 | 0.033333 | 0.011905 | | 0.041429 |
| g(3,4) | | 0.092784 | 0.106667 | 0.166667 | 0.059524 | | 0.107143 |
| g(3,5) | | 0 | 0 | 0.033333 | 0 | | 0.033333 |
| g(3,6) | | 0 | 0.066667 | 0.033333 | 0.083333 | | 0.083333 |
| g(3,7) | | 0 | 0.026667 | 0 | 0.02381 | | 0.026667 |
| g(3,8) | | 0 | 0 | 0.066667 | 0.02381 | | 0.066667 |
| g(3,9) | | 0 | 0.026667 | 0 | 0 | | 0.026667 |
| g(3,10) | | 0 | 0 | 0.033333 | 0 | | 0.033333 |
| g(3,11) | | 0 | 0 | 0 | 0.035714 | | 0.035714 |
| g(3,12) | | 0 | 0 | 0 | 0.011905 | | 0.011905 |
| g(3,13) | | 0.010309 | 0 | 0 | 0 | | 0.010309 |
| | | | | | | | |
| g(4,1) | | 0.371134 | 0.253333 | 0.433333 | 0.404762 | | 0.18 |
| g(4,2) | | 0.453608 | 0.44 | 0.366667 | 0.369048 | | 0.086942 |
| g(4,3) | | 0.010309 | 0.013333 | 0.033333 | 0.035714 | | 0.025405 |
| g(4,4) | | 0.010309 | 0.013333 | 0 | 0 | | 0.013333 |
| g(4.5) | | 0.113402 | 0.226667 | 0.166667 | 0.095238 | | 0.131429 |
| g(4,6) | | 0.041237 | 0.026667 | 0.033333 | 0.059524 | | 0.032857 |
| g(4.7) | | 0 | 0.013333 | 0 | 0 | | 0.013333 |

(continued)

| genotype | | blue | green | hazel | brown | | range |
|---|---|---|---|---|---|---|---|
| | | | | | | | |
| g(4.8) | | 0 | 0 | 0 | 0.011905 | | 0.011905 |
| | | | | | | | |
| g(5.1) | | 0.608247 | 0.453333 | 0.533333 | 0.547619 | | 0.154914 |
| g(5.2) | | 0.092784 | 0.186667 | 0.166667 | 0.107143 | | 0.093883 |
| g(5.3) | | 0.134021 | 0.173333 | 0.066667 | 0.142857 | | 0.106667 |
| g(5.4) | | 0.14433 | 0.12 | 0.2 | 0.083333 | | 0.116667 |
| g(5.5) | | 0.010309 | 0 | 0.033333 | 0.011905 | | 0.033333 |
| g(5.6) | | 0 | 0.013333 | 0 | 0 | | 0.013333 |
| g(5.7) | | 0 | 0 026667 | 0.033333 | 0 | | 0.033333 |
| g(5 7) | | 0.010309 | 0.013333 | 0 | 0.011905 | | 0.013333 |
| g(5.8) | | 0 | 0.013333 | 0.033333 | 0.035714 | | 0.035714 |
| g(5.9) | | 0 | C | C | 0.011905 | | 0.011905 |
| g(5.10) | | C | C | 0 | 0.011905 | | 0.011905 |
| | | | | | | | |
| g(6,1) | | 0.56701 | 0.533333 | 0.5 | 0.607143 | | 0.107143 |
| g(6,2) | | 0.134021 | 0.08 | 0.266667 | 0.119048 | | 0.186667 |
| g(6,3) | | 0.113403 | 0.186667 | 0.1 | 0.119048 | | 0.086667 |
| g(6,4) | | 0.164948 | 0.133333 | 0.2 | 0.095238 | | 0.104762 |
| g(6,5) | | 0.010309 | 0.026667 | 0 | 0.011905 | | 0.026667 |
| g(6,6) | | 0 | 0.026667 | 0 | 0.011905 | | 0.026667 |
| g(6,7) | | 0.010309 | 0.013333 | 0 | 0 | | 0.013333 |
| | | | | | | | |
| g(7,1) | | 0.030928 | 0.12 | 0.133333 | 0.130952 | | 0.102405 |
| g (7,2) | | 0.309278 | 0.253333 | 0.166667 | 0.321429 | | 0.154762 |
| g(7,3) | | 0.247423 | 0.266667 | 0.133333 | 0.214286 | | 0.133333 |
| g(7,4) | | 0.010309 | 0 | 0.066667 | 0 | | 0.066667 |
| g(7,5) | | 0.247423 | 0.16 | 0.233333 | 0.095238 | | 0.152185 |
| g(7,6) | | 0.030928 | 0.013333 | 0.1 | 0.011905 | | 0.088095 |
| g(7,7) | | 0.010309 | 0.026667 | 0.033333 | 0.011905 | | 0.023024 |
| g(7,8) | | 0.103093 | 0.12 | 0.133333 | 0.142857 | | 0.039764 |
| g(7,9) | | 0.010309 | 0.026667 | 0 | 0.02381 | | 0.026667 |
| g(7,10) | | 0 | 0 | 0.066667 | 0.011905 | | 0.066667 |
| | | | | | | | |
| g(8,1) | | 0.402062 | 0.293333 | 0.3 | 0.321429 | | 0.108729 |
| g(8,2) | | 0.463918 | 0.466667 | 0.566667 | 0.357143 | | 0.209524 |
| g(8,3) | | 0.041237 | 0.053333 | 0.033333 | 0.059524 | | 0.02619 |
| g(8,4) | | 0.010309 | 0.013333 | 0 | 0 | | 0.013333 |

(continued)

| genotype | | blue | green | hazel | brown | | range |
|---|---|---|---|---|---|---|---|
| | | | | | | | |
| g(8,5) | | 0.041237 | 0.106667 | 0.166667 | 0.119048 | | 0.12543 |
| | | | | | | | |
| g(9,1) | | 0.278351 | 0.213333 | 0.266667 | 0.261905 | | 0.065017 |
| g(9,2) | | 0.319588 | 0.306667 | 0.233333 | 0.202381 | | 0.117207 |
| g(9,3) | | 0.051546 | 0.053333 | 0 | 0.02381 | | 0.053333 |
| g(9,4) | | 0.041237 | 0.026667 | 0.066667 | 0.059524 | | 0.04 |
| g(9,5) | | 0.154639 | 0.16 | 0.166667 | 0.142857 | | 0.02381 |
| g(9.6) | | 0 | 0 | 0.033333 | 0 | | 0.033333 |
| g(9,7) | | 0.051546 | 0.066667 | 0.133333 | 0.071429 | | 0.081787 |
| g(9,8) | | 0.010309 | 0.066667 | 0 | 0.059524 | | 0.066667 |
| g(9,9) | | 0.061856 | 0.066667 | 0.166667 | 0.071429 | | 0.104811 |
| g(9.10) | | | 0 0.013333 | 0 | 0.02381 | | 0.02381 |
| g(9,11) | | 0.030928 | 0.013333 | C | 0.047619 | | 0.047619 |
| g(10.1) | | 0.412371 | 0.373333 | 0.433333 | 0.369048 | | 0.064286 |
| g(10,2) | | 0.206186 | 0.24 | 0.266667 | 0.25 | | 0.060481 |
| g(10.3) | | 0.195876 | 0.24 | 0.166667 | 0.059524 | | 0.180476 |
| g(10,4) | | 0.030928 | 0.013333 | C | 0.011905 | | 0.030928 |
| g(10,5) | | | 0 0.013333 | 0.033333 | 0.047619 | | 0.047619 |
| g(10.6) | | 0.051546 | 0.026667 | 0.1 | 0.059524 | | 0.073333 |
| g(10,7) | | 0.020619 | 0.013333 | 0 | 0.02381 | | 0.02381 |
| g(10,8) | | 0.010309 | 0.053333 | 0.066667 | 0.107143 | | 0096834 |
| g(10.9) | | 0.010309 | 0 | 0 | 0 | | 0.010309 |
| g(10,10) | | 0.010309 | 0 | 0 | 0 | | 0.010309 |
| g(10,11) | | 0.041237 | 0.013333 | 0 | 0.047619 | | 0.047619 |
| g(10,12) | | 0.010309 | 0 | 0 | 0 | | 0.010309 |

Table 16-3 Coding Key

OCA2-A

g(1,1) TTAA/TTAA

g(1,2) CCAG/TTAA

OCA2-B

g(2,1) CAA/CAA

g(2,3) CGA/CAA

OCA2-C

g(1,3) GGAA/GGAA

g(2,3) GGAA/TGAA

OCA2-D

g(4,1) AGG/AGG

g(4,2) GGG/AGG

OCA2-E

(continued)

| | |
|---|---|
| g(5,1) | ACG/ACG |
| g(5,4) | GCT/ACG |
| MC1R-A | |
| g(6,2) | CCC/CTC |
| g(61) | CCC/CCC |
| TYR-A | |
| g(7,2) | CGG/CAG |
| g(7,5) | AGG/CAG |
| TYRP-A | |
| g(8,2) | CC/TC |
| g(8,1) | TC/TC |
| TYRP-B | |
| g(9,2) | TTG/GAG |
| g(9,1) | TTG/TTG |
| DCT-B | |
| g(10,3) | CTG/GCA |

## EXAMPLE 17

## IDENTIFICATION OF PENETRANT AND LATENT HAPLOTYPE ALLELES AND CONSTRUCTION OF AN ACCURATE COMPLEX CLASSIFIER MODEL FOR EYE COLOR INFERENCE

This example provides the identification of a preferred combination of penetrant and latent haplotype alleles (also called genetic features herein) that are used in a complex classifier model to infer eye color. These results reveal that the identification of predictive markers for complex traits such as iris pigmentation is best accomplished in a manner that is respectful of intergenic complexity and that accurate classification models incorporating genetic features are best developed in a manner that is respectful of intragenic complexity. The combination of penetrant and latent haplotypes of this Example when used to infer eye color using the classification model disclosed in this Example, inferred eye shade for a group of 225 Caucasians with 99% accuracy for the inference of iris color shade, and 97% accuracy for the inference of actual eye colors.

Iris pigmentation is a complex genetic trait that has long interested geneticists and anthropologists but is yet to be completely understood. A novel population genetics approach was applied to identify the penetrant "genetic features" of variable human iris pigmentation. As described in this example, latent genetic features were identified through inference, and both types of features were modeled using a weighted quadratic discrimination method to develop a complex genetics classifier for the accurate inference of iris colors. The results provided in this Example show that of thousands of possible allele combinations in several human pigmentation genes, only 12 within eight of these genes are necessary for the accurate and sensitive inference of human iris color.

### A. METHODS

### Specimens

Specimens for re-sequencing were obtained from the Coriell Institute in Camden, New Jersey. Specimens for SNP scoring were collected from individuals of various ages, sex, hair, iris and skin shades using informed consent guidelines under IRB guidance. Anonymous unique identifiers were assigned to specimens from which DNA was prepared using standard DNA isolation techniques (Qiagen Inc.).

### SNP discovery

Vertical resequencing for the various genes was performed by amplifying the proximal promoter, each exon and 3' UTR sequences from a multiethnic panel of 670 individuals. PCR amplification was accomplished using *pfu* Turbo polymerase according to the manufacture's guidelines (Stratagene). We developed a program (unpublished) to design re-sequencing primers in a manner respectful of homologous sequences in the genome to insure that we did not co-amplify pseudo genes or amplify from within repeats. BLAST searches confirmed the specificity of all primers used Amplification products were subcloned into the pTOPO (Invitrogen) sequencing vector and 96 insert positive colonies were grown for plasmid DNA isolation. We sequenced with an ABI3700 with PE Applied Biosystems BDT chemistry and we deposited the sequences into a commercial relational database system (iFINCH, Geospiza, Seattle, WA). PHRED qualified sequences were aligned and analyzed using second program we developed (unpublished) to identify quality-validated discrepancies between sequences.

## Genotyping

A first round of PCR was performed on these samples using the high-fidelity DNA polymerase *pfu* turbo and cognate re-sequencing primers. Representatives of the resulting PCR products were checked on an agarose gel, and firs round PCR product was diluted and then used as template for a second round of PCR incorporating phosphothionated primers. Genotyping was performed for individual DNA specimens using an Orchid single base primer extension protocol and an SNP stream 25K/Ultra High Throughput (UHT) instrument (Orchid Biosystems, Princeton. NJ) using primers as described in Table 17-8.

## Data Analysis

Haplotype frequencies were calculated for haplotype i using the function $p_i=(x_i/n)$, where $x_i$ is the number of times that haplotype i was observed and n is the number of patients in the group. For contingency analysis we used a Pearson's test to test the null hypothesis that there was no association between genotypes and eye colors. We also determined and quantified the associations between specific genotypes and eye colors by computing the Adjusted Residuals which we assumed to follow an N(0,1) distribution as per large sample theory. We defined the 95% confidence intervals by carrying out Multiple Logistic Regression Analysis; it may be noted that estimates of conditional probabilities and their 95% confidence intervals obtained using this approach would be more stable compared to sample proportions, in the sense that the standard error and confidence intervals would be smaller being based on total sample size (n), rather than cell frequencies ($n_{ij}$). Individual haplotypes were inferred from phase unknown genotypes using a computational haplotype reconstruction method (Stephens and Donnelly, 2001).

## Genetic Feature Extraction

To identify useful genetic features of variable iris color, an iterative, empirical approach was used to test haplotype alleles of all possible SNP combinations within each gene for the ability to statistically resolve individuals of various trait values. The goal of the screen was to identify whether alleles of a gene were associated with variable iris color and if so, which SNP combinations had alleles most strongly associated with iris color. We designate the predictive phase-known alleles of these SNP combinations as "*genetic features*" of variable iris color. We designate the SNP combinations themselves as "*feature SNP combinations*".

For each gene, a list of all possible n-locus SNP combinations was created. The system iteratively

a) selected an n-locus SNP combination at random,
b) inferred haplotype phase for each individual with respect to this n-SNP combination (if n>2, using the algorithm described by Stephens and Donnelly, 2001),
c) counted the inferred haplotype pairs for the light and dark group,
d) calculated a pair-wise F-statistic, and Fishers Exact test statistic on haplotype pairs ("multilocus genotypes") and a Chi-square adjusted residual statistic on individual haplotypes, in order to determine whether there were significant allele differences between individuals of light (blue+green+hazel irises) and dark (black+brown) iris shade and
e) repeated the process for the next n-locus SNP combination until all possible combinations within a gene were tested.

The process was repeated for each gene. SNPs or SNP combinations with alleles that were statistically associated with iris color shade (p-value <0.05) were identified as "feature SNP combinations" and/or their alleles with significant adjusted residuals as "genetic features" of variable iris color. To avoid having to test all possible n-SNP combinations (which is computationally intensive), we first tested all possible 2-SNP haplotypes and used these results to guide subsequent tests of higher order SNP combinations. When more than one "genetic feature" was identified within a gene (i.e., in the case of overlapping SNP sets), the set of non-overlapping SNP combinations with the lowest (and significant) p-values within the gene was selected. In the case of multiple non-overlapping features identified within a gene, it was often observed that genotype trait class sample sizes and allelic complexity rendered the alleles of a single (n+m + ...)-locus SNP combination less robustly associated with trait value than the component (n-locus, m-locus ...) combinations on their own. In these cases, each of the (n, m, ...) combinations was selected as a "genetic feature" over the single (n+m+...) feature.

**Nested contingency analysis.**

To verify and validate the genetic features that were identified, a nested contingency analysis of haplotype cladograms was performed. To do this, an assumption was made that both detected and non-detected mutations were potential contributors for phenotypic effects at some point in the evolutionary history of a population, and that these mutations were embedded within the historical structure represented by the haplotype cladogram. Clades were obtained by using PAUP Ver. 4.0b8 software (Outgroup method or Neighbor Joining (NJ) method). We obtained nested cladograms based on each of the following four methods: (I) Maximum Parsimony, (ii) Neighbor joining, (iii) Maximum Likelihood and (iv) Bayes Method. In general, we used the tree for which nested statistical analysis gave the best results. Nested contingency analysis was carried out as described by others (Templeton et al., *supra,* 1997).

**Genetic Feature Modeling - Quadratic classification:**

To use the haplotype alleles for the inference of iris colors, we wrote a software program for using a parametric, multivariate Quadratic classification technique with modifications for genomics data. Under the assumption that the samples have been taken from multivariate normal distributions with different mean vectors, with a common variance covariance matrix, we applied classification procedures introduced previously by Fisher (1936), Rao (Nature 1947: 159: 30-31; Rao, C.R., Nature 1948a; 160:835-836; Rao, C.R., JRSS(B) 10:159-203) and Smith (1947). The pooled within-population variance-covariance matrix can be computed from

$$S = \Sigma^{p}{}_{i=1} \Sigma^{Ni}{}_{j=1} (Y_{ij}-\mu_i)(Y_{ij}-\mu_i)' / \Sigma(N_i-1) \qquad (1)$$

where $Y_{ij}$ is the vector of character measurements for the j'th individual in the i'th group and $\mu_i$ and $N_i$ are the vector of means and sample size for the i'th group. The components for these vectors are encodings for entities such as SNP alleles, haplotypes (genetic features) or in the preferred case, diploid pairs of haplotypes (multilocus genotypes of genetic features), each dimension of the vector representing a score for the different entity observed in the sample. Because the total number of genotypes observed for the genetic problem described herein exceed the total number of individuals in any one iris color group, we do not use Fisher's quadratic discriminate analysis directly because of variance-covariance matrix singularity. Instead, we form a contingency table $K=(k_{ij})$ of order Ni x Nj, where rows i represents multilocus genotypes and columns j represent iris colors (i={1,2,...,Ni} and j={1,2,...,Nj}). We computed the marginal column, $k(i) = \Sigma\{k(i,j)|j \in J$, the marginal row, $k(j)=\Sigma\{k(i,j)| i \in I$ and grand total of $k= \Sigma\{k(i,j)|i \in I$ and $j \in J$. After computing the mass of the $i^{th}$ row, $f_i=k(i)/k$, and the mass of $j^{th}$ column, $f_j=k(j)/k$, we computed the $i^{th}$ row and $j^{th}$ column profile of the correspondence matrix $(f_{ij}) =(k_{ij}/k)$ using the functions $f^i{}_J=\{f^i{}_j=k_{ij}/k(i)\ |j \in J\}$ and $f^j{}_I=\{f^j{}_i=k_{ij}/k(j)\ |i \in I\}$, respectively. We then computed the difference of observed and expected frequencies of the $(i,j)^{th}$ cell, $d_{ij}=(f_{ij}-f_i f_j)$. The principal inertia (Eigenvalue) was computed as follows: Let the scaled matrix be defined as $S=(s_{ij})$, where $s_{ij}=d_{ij}/(\sqrt{f_i f_j})$. $S=(s_{ij})$ is submitted to singular value decomposition (SVD) by breaking the matrix into the product of three matrices:

$$S=U\Lambda V^T \qquad (1)$$

where A is a diagonal matrix, and its diagonal elements arc referred to as the singular values of S, or factors, and U is the left eigenvector which represents eigengenotypes by rows and $V^T$ is the right eigenvector which represents eigentraits by columns. Thus, all of the eigentraits are decoupled from all of the eigengenotypes. Principal Coordinates were computed for the $i^{th}$ row coordinate of $k^{th}$ factor using the function $F_\kappa(i)=\lambda_\kappa\text{-}u_{i\kappa}/\sqrt{f_i}$ for k=1,2,..., NF, where $u_{i\kappa}$ is the left eigengenotype of the $i^{th}$ row coordinate of the $k^{th}$ factor. Similarly, principal components were computed for the $j^{th}$ column coordinate of $k^{th}$ factor using $G_\kappa(j)= \lambda_\kappa v_{j\kappa}/\sqrt{f_j}$, for $\kappa$=1,2, ..., NF=Min(r-1, c-1), where $v_{j\kappa}$ is the right eigentrait of the $j^{th}$ column coordinate of $\kappa^{th}$ factor. The $i^{th}$ row score of the $k^{th}$ factor is obtained by $s_k(i)=\Sigma\{G_k(j)k_{ij}|j \in 1J$. Similarly, the $j^{th}$ column score is computed by $c_k(j)=\Sigma\ \{F_k(i)k_{ij}|i \in I$. The Z-score of the $i^{th}$ genotype of the $k^{th}$ factor is given by $Z_{ik}=\{s_k(i) - E(s_k)\}/SD\ \{s_k(i)\}$, where $E(s_k)$ is the mean score of genotypes of the $k^{th}$ factor and $SD[s_k(i)]$ is the standard deviation of the genotype score of the $k^{th}$ factor. Finally, individual sample scores are obtained for each genetic feature for all factors as M=XZ, where $X=(x_{ij})$={1 if the $i^{th}$ individual has the $j^{th}$ genotype and 0 otherwise. The correspondence analysis in this case serves as an effective dimension reduction tool; it is with these sample scores on each genetic feature for each factor that we encode multilocus genotypes for quadratic discriminate analysis. An individual vector $Y=(i,j,...n)_m$, where n=number of multi locus genotypes for m genetic features before correspondence analysis now becomes a simpler $Y=\{(x)_m,(y)_m,(z)_m\}$ vector by encoding the individuals on m genetic features for factors x,y and z. It is these vectors that we use with quadratic discriminate analysis. Assuming that the iris color populations present different variance-covariance matrices with these encodings, as they did in this case, the estimate of the quadratic discriminate score for the $i^{th}$ group is:

$$D_i^Q = -(1/2)\ ln|S_i| - (Y-\mu_i)' S^{-1}{}_i(Y-\mu_i) + ln p_i \qquad for\ i=1,2,...g(groups) \qquad (2)$$

Where $\mu_1$ is the sample mean of the $i^{th}$ group and $S_i$ is the new sample variance-covariance matrix of the $i^{th}$ group calculated as in (1) but using sample scores, and $p_i$=1/g. Large between class distances, relative to within class differences, provide justification for using the mean vector values for each class as a basis for classification. Classification is accomplished by allocating the individual to that group for which (2) is largest, where the probability p(j|x) of $j^{th}$ membership in each iris color class is calculated as:

$$P(j|x) = exp[-0.5\ D^2{}_j(y)] / \Sigma_i\ exp[-0.5\ D^2{}_i(y)] \qquad for\ i=1,2,...g(groups) \qquad (3)$$

where,

$$D_j^2(Y)=(Y-\mu_j)'S_j^{-1}(Y-\mu_j). \qquad (4).$$

The P(j|x) applies to the classification of individuals used for the construction of S, but generalize S derived from one group by blindly classifying individuals of a second group to construct a classification probability table of individuals of known iris color by classified iris color groups.

Under the assumption of normality, the sample mean vector and the sample covariance matrix constitute minimally sufficient statistics, in the sense that any inference based of them carries with it all the information available in the sample. Thus, any classification rule based on these summary statistics ought to be optimal from the point of view of sample information used for their analysis. However, with complex systems, the data often provide additional information not reflected by these statistics, and this additional information can often be used for improving the results based on these statistics. With genetics, sequences may contribute towards phenotype variation through dominance or additivity, wherein their associations with trait values from independent analyses are of varying degrees of strength, but statistically significant. Alternatively, sequences may contribute through epistasis, wherein their association with trait values from independent analyses are weak or non-existent. To produce a quadratic classifier sensitive for the epistatic contributions, we devised a weighting scheme for producing unequal variance-covariance matrices for each of the iris color groups used in quadratic analysis. First the most strongly associated genotypes were identified. Next, genotypes of weaker association were randomly selected. Normally when constructing the covariance matrix, M for each factor was calculated using the Z-scores and binary values; a value of 0 within the individual vector if the genotype was absent in an individual, and a 1 if present. Using the weighting scheme, instead of using a binary x when calculating M for each factor, 1+x was used for randomly selected weakly/non-associated sequences, where x is the number of strongly associated genotypes also present in that individual. By successively selecting random combinations of weakly/non-associated pigmentation gene features for weighting and testing how well the model derived from these combinations generalizes to the test sample for iris color classification, an optimal weighting strategy can be obtained. Recoding in this manner generally increases the variability of the scores of weakly/non-associated sequences and hence it improves the discriminating power of the model. Although the coding procedure may seem arbitrary, it is important from a practical point of view. For example, there are instances in the areas of statistical forecasting of time series or economics, wherein a data supported methods are recommended, as long as they lead to relatively more accurate inferences. In this case, once the optimal model has been identified, the weighting used for its generation can provide clues on the non-linear relationships between genotypes of different genes towards complex trait variation (i.e., epistasis).

**Quadratic Classifier Simulation**

Monte Carlo simulation study was used to generate the distribution and summary statistics for the probabilities of correct and incorrect classifications using the linear/quadratic classification method. A program was written to use a random number generator to select 200 individuals on the basis of observed allele frequencies from both light and dark iris color shade goups, and used these individuals to calculate a multivariate linear classification probability matrix. This experiment was repeated 10,000 times to get the summary statistics of Classification and misclassification rates and their Confidence Intervals.

**B. RESULTS**

The public databases (NCBI: Unigene, dbSNP, LocusLink) and literature were mined and re-sequencing was performed to identify 181 candidate SNP loci in 8 pigmentation genes (an average of 23 candidate SNPs per gene) (column 2, Table 1). Genotypes were scored for each of these candidate SNP loci in a group of 335 Caucasians of self-reported iris color (97 brown, 117 blue, 36 green, 85 hazel) as well as in 230 additional individuals of varying racial backgrounds (100 Caucasian, 100 African American and 30 Asian individuals). A software system was developed to screen the phase known alleles of all possible n-SNP combinations for association with trait value (if any, where n = [1,2,...x] and x= the number of SNP loci). The screen was carried out in case control format, encoding iris color shade as light or dark (where light = blue, green or hazel and dark = black and brown). In all, we screened alleles of 411 n-locus SNP combinations and of these, alleles of 8 optimally discriminate combinations in 4 of the genes were identified as strongly associated with variable Caucasian iris color (Column 5, Table 17-1). The combinations were unequally distributed among the *OCA2* (n=5), *TYRP* (n=1), *DCT* (n=1) and *MC1R* (n=1) genes. Because their association with iris colors was strong enough to be detected with simple genetics approaches, we term haplotype alleles of these SNP combinations "penetrant genetic features," and the SNP combinations themselves "penetrant feature SNP combinations" of variable iris color. No penetrant genetic features or penetrant SNP combinations were identified in the TYR, SILV, ASIP or AP3B1 genes (Column 5, Table 17-1). The 8 penetrant genetic features were comprised of 25 SNPs, of an average minor allele frequency 0.21 (range 0.07 - 0.47). Four of these were coding changes, 17 were located in introns and 4 were silent changes (Column 6, Table 17-2). Ten of the SNPs were identified from resequencing (not present in the NCBI:dbSNP database or the literature) though alleles of two of these (217439 and 217441, Table 2) turned out to have been identified before as related to human pigmentation in the literature (specifically red hair and blue eyes, Valverde, P. et al., Nature Genet. 11: 328-330, 1995). 11 of the SNPs were selected from the NCBI dbSNP database (Column 7, Table 17-2).

Validation of the Penetrant Genetic Features:

Having identified several penetrant feature SNP combinations of variable iris color shade, the analysis was extended to more completely investigate the associations of their penetrant genetic features with specific eye colors. From a contingency analysis of haplotypes and multilocus genotypes versus iris colors (blue, green, hazel, brown and black), numerous significantly associated alleles and allele combinations were associated (Table 17-3). Chi-square adjusted residuals showed that many of the associations were quite strong at the haplotype level. For example, the OCA2-A TTAA was strongly associated with blue (p=0.0079, row 3, column 3, Table 17-3), but the OCA2-A CCAG and OCA2-B CGA alleles were strongly associated with brown (p=0.0008, row 4, column 3, Table 3; p=0.0024, row 11, column 3, Table 3, respectively). Analysis at the level of the multilocus genotypes showed that each of the penetrant genetic feature SNP combinations were also statistically associated with eye colors (i.e., none of the 8 SNP combination is missing an entry in column 8, Table 17-3). Though their alleles were associated with iris color shades, the chi-square statistic of contingency analysis for haplotype or multilocus alleles of the DCT-B, TYR-A, OCA2-D and OCA2-E features were not significant. For example, the DCT-B total p-value was insignificant at the haplotype (row 21, column 3, 8 Table 17-3) and multilocus genotype levels (row 21, column 8, Table 17-3). Nonetheless, adjusted residuals for 2 of the DCT-B haplotypes show that these particular alleles were strongly associated with eye colors even though the total chi-square statistic was not significant (CTG with brown, p=0.0133, row 17, column 3, Table 3 and GTG with hazel, p=0.0249, row 18, column 3, Table 17-3). The same was observed for other feature SNP combinations that were not associated with specific iris colors but were associated with iris color shade; the OCA2-D AGG genetic feature with Hazel irises (p=0.0468, row 27, column 3, Table 17-3), the OCA2-D GGG genetic feature with brown irises (p=0.0222, row 28, column 3, Table 17-3) and the OCA2-E GCA genetic feature with brown irises (p=0.0004, row 31, column 3, Table 17-3). Given sample size and association strength, the most important genetic features for predicting brown irises were found in the OCA2-D, OCA2-E and DCT-B feature SNP combinations, and the most important for blue or green iris colors were found in the MC1R-B and TYRP-B feature SNP combinations (columns 5 and 6, Table 17-3). Even though there were twice as many genetic features of blue irises counted as for brown (1474 vs. 664, counting down columns 6 and 11 for each color, Table 17-3), there were half as many types of genetic features of brown as for blue irises (4 versus 8, counting down column 4 for each color, Table 17-3). This suggests that the diversity of haplotypes associated with brown irises was significantly greater than that of the haplotypcs associated with blue irises. Most of the haplotypes and multilocus genotypes for the feature combinations were even more dramatically associated with eye colors in a multi-racial sample (data not shown), presumably because the variants associated with darker irises were enriched in those racial groups of the world that are of darker average iris color than Caucasians.

The associations at the level of the multilocus genotypes for these penetrant genetic features suggest that some of the haplotype alleles contribute towards the dominance component of iris color variance. For example, though the OCA2-A TTAA haplotype is strongly associated with blue irises (p=0.0079, row 3, column 3, Table 17-3) and the OCA2-A TTAG haplotype is strongly associated with brown irises (p=0.0045, row 5, column 3, Table 17-3), the OCA2-A TTAA/TTAG multilocus genotype was strongly associated with brown irises, not blue (p=0.0006, row 5, column 8, Table 17-3). Not all of the dominance component contributions were towards darker eye colors. For example, OCA2-B CAA was strongly associated with blue irises (p=0.0269, row 10, column 3, Table 17-3) and OCA2-B CGA with brown irises (p=0.0024, row 11, column 3, Table 17-3) but the OCA2-B CAA/CGA multilocus genotype was associated with blue, not brown irises (p=0.0.0314, row 11, column 8, Table 17-3).

A contingency table was constructed and the multilocus genotypes were plotted in Correspondence Analysis space to visualize the lower-dimensional interrelationships and between multilocus genotypes of the penetrant genetic features and iris colors, as well as to encode individuals as complex genetics vectors. From this analysis, it was clear that genotypes of penetrant genetic features of Blue, Green and Hazel irises share more profile similarity to one another than to those of brown irises. A plot of genotypes and trait values that are truly related to one another would produce a plot pattern that makes intuitive biological sense. In the COA plot, blue, green, hazel and brown irises plotted as profile functions of genetic feature genotypes arc found along a clockwise progression around the centroid. This is the order in which the concentrations of brown pigment (cumelanin) increases. Because the genes measured, in this analysis are involved in the production of this pigment, this pattern makes intuitive sense since. Further, the multilocus genotypes of the penetrant feature SNP combinations were more distantly removed from the centroid than genotypes of combinations that were not as significantly associated (Table 3). This was to be expected since the distance from the centroid is proportional to the contribution of a genotype towards the overall chi-square statistic in the original contingency table.

To confirm our results and determine the role of specific mutations in the determination of eye color variation we performed a nested contingency analysis on haplotype cladograms of the penetrant feature SNP combinations (Templeton et al., 1987). Haplotype cladograms of all genetic features are inlaid with variants that are functionally interconnected through evolutionary time. The evolutionary framework will often ascribe patterns to present day trait associations that are derived from the evolutionary history of the alleles and in so doing, may suggest a biological, not merely statistical relevance for a genetic association. However, failure to find a cladogram based pattern to the allele associations is not necessarily an indication that the allele associations are not real, since functionally relevant alleles may have been recently and independently derived. We identified significant cladogram based pattern for the associations of OCA2-A, OCA2-B, OCA2-C OCA2-D and TYRP-A alleles (Table 4), suggesting that mutations relevant for iris color occurred relatively early in the evolution of these gene sequences. Two of the feature SNP combinations (OCA2-B and OCA2-C) had more than one functionally relevant mutation with a discernable evolutionary history, but for most of the others, the largest amount (though not all) of the variability in iris colors could be traced back to branchings created by change at a single locus of the feature combination. No significant cladogram based pattern was detected for the MC1R-A, OCA2-E or DCT-B feature SNP combinations. For these, it appears that the alleles associated with iris color have independently evolved at a time later in the evolutionary history of their gene sequences than for the OCA2-A, OCA2-B, OCA2-C OCA2-D and TYRP-A alleles.

## Latent genetic features

Because the prevalence of each iris color trait was relatively high in our sample group as well as in the general population, and because the allele frequencies of most of the SNPs we studied was also relatively high, the habitability of iris colors would be expected to be reasonable for the detection of SNP associations within the context of a case-control study design (Culverhouse et al., Am. J. Hum. Genet. 70:461-471, 2002). Nonetheless, a major drawback of the genome based case control study design (given the analytical methods that we have so far employed) is the lack of power to detect alleles that exclusively or substantially contribute towards genetic variance through the epistatic component (Culverhouse et al., Am. J. Hum. Genet. 70:461-471, 2002). SNPs that were not part of the penetrant feature SNP combinations described in Table 1 may either not contribute towards iris color variance, or may contribute through epistatic means. Though undetectable with the case-control design, epistatic components can more easily be detected in linkage studies than in case control studies because purely (or largely) epistatic models give rise to excess allele sharing among affected sibs in linkage analysis. We reasoned that a racial comparison of pigmentation allele frequencies between Caucasians and Africans/Asians represent an extreme case of a very simple linkage study, where the racial groups are equivalent to sibs of a family pedigree. In this case, the linkage is considered within the context of an evolutionary, rather than familial scale, because individuals of the latter two races exhibit darker average iris color than Caucasians. Thus, to identify those SNPs that may contribute towards the epistatic component of iris color variance, we screened the SNPs that were not part of the penetrant feature SNP combinations described in Table 1 for alleles that were enriched in either Caucasians (n=100 new individuals, not yet analyzed) or the African/Asian combined (n=130 new individuals, not yet analyzed) groups. Though most alleles in non-pigmentation genes do not show dramatic minor allele frequency differences between the two racial groups (Frudakis et al., In Review, Human Heredity (2002); for example, Table 5B), alleles of many of the SNPs not part of the penetrant feature SNP combinations of Table 1 show unusual minor allele frequency differences between the two racial groups (Table 5A). We inferred that these differently shared SNP alleles may contribute towards the epistatic component of iris color variance. Though haplotype alleles are generally more predictive for trait value than individual SNP alleles, it is not possible to determine which alleles of which of these SNP combinations contribute most towards this variance. Thus, we combined them into arbitrary SNP combinations, the components of which were in linkage disequilibrium, and we call these "latent feature SNP combinations" of variable iris colors and their haplotype (and multilocus genotype) alleles "latent genetic features" of variable iris color.

## FEATURE MODELING AND CLASSIFIER CONSTRUCTION

Using the penetrant genetic features as independent classifiers, Bayesian posterior probabilities of correct classification approached 50% for some, but fell within the 30%-40% range for most (columns 5 and 10, Table 3). These results imply that the determination of variable iris colors is complex and suggest that though the alleles of the penetrant feature SNP combinations are associated with iris color variance, any one component on its own explains but a minor fraction of this variance and it's predictive power as an independent classifier is too low for field use.

### Weighted quadratic classification using only the penetrant genetic features

To generate a complex model by which to explain more iris color variance, to an extent that accurate inferences could be made, a weighted quadratic classification algorithm was developed based on standard coordinates from a correspondence analysis (see methods). We first used the penetrant genetic features to compute and weight a variance-covariance matrix (see methods) from 330 Caucasian individuals. This matrix was applied for a blind, quadratic discriminate classification of iris colors in 286 other Caucasians of known but concealed iris color. For the first analysis two groups were defined; a light iris shade group defined as individuals of blue, green or hazel irises, and the dark iris shade group defined as individuals of brown or black irises. On the level of the multilocus genotypes (gene-wise genotypes), an overall accuracy of 98% was obtained for this discrimination. The sensitivity for dark iris color shades was 100% and the sensitivity for light eye color shades was 97% (reading along the rows, Table 6a). The light iris classification was 100% accurate and the dark iris classification was 94% accurate (reading down the columns, Table 6b). Using this method at the level of individual SNP alleles, SNP genotypes or individual haplotype alleles produced lower accuracies (with accuracies in increasing order), suggesting that the highest level of intra-genic allele complexity is required for accurate inference of eye color shade and that increasing levels of complexity offer successively greater predictive power. Using the method with multilocus genotypes to infer actual eye colors rather than just eye color shade, 100% sensitivity was obtained for blue iris classification, 69% sensitivity of brown iris classification, 100% sensitivity of green iris classification and 84% sensitivity of hazel iris classification (reading along rows, Table 6B). The accuracy of blue iris classification was 67%, of brown iris classification 100%, of green iris classification 100% and of hazel iris classification 74% (reading down the columns, Table 6B). Using simulation to estimate the inference power of the quadratic classifier we obtained a log likelihood of r=1.96 (not shown). In effect, the classifier was remarkably accurate and sensitive, with good inference power, but its deficiency was apparent in the misclassification of brown and hazel iris individuals into the blue iris group.

By adding the latent genetic features to this analysis (latent + penetrant genetic features), the optimal weighting strategy produced a covariance matrix that blindly generalized to the same 286 Caucasians with 100% accuracy and sensitivity for discrimination of light versus dark iris color shades. The optimal model also generalized to this sample with 100% accuracy for the inference of actual iris colors (286/286 correctly classified; along diagonal of Table 7A). Using simulation to estimate inference power of the quadratic classifier, we obtained a log likelihood of r=3.22 for classification into the proper iris color group. Though it is true that markers over-represented in racial groups of average darker iris colors would help the classifier artificially infer eye color in a multi racial sample, it is not true that any such markers would help with the inference of iris colors in Caucasians unless they were functionally relevant for human iris coloration. That these markers contributed towards the classifications within Caucasians suggests that they are functionally related to, or linked to markers functionally related to iris color determination.

## C. DISCUSSION

A complex classifier is presented in this Example for the inference of human iris color from DNA. To our knowledge this is the first such classifier described. Though the pigmentation genes are well documented, until this work, merely a handful of SNP alleles were known to be weakly associated with natural distributions of iris colors in the healthy Caucasian population. The reason for this is that most work attempting to describe natural variation in iris colors has focused on simple genetics approaches, such as single SNP analysis in single genes including the TYR (Sturm et al., Gene 277:49-62, 2001), MC I R (Valverde et al., 1997) and ASIP (Sturm et al., Gene 277:49-62, 2001) genes. By developing new complex genetics methodologies and adopting a systematic approach for identifying and modeling genetic features of variable iris color, we looked at the problem through more of a complex genetics lens than others previously. Nevertheless, most of our results agree with the previous literature. Though the TYR expression product is the rate-limiting step in the catalytic chain leading to the synthesis of eumelanin from tyrosine, previous studies by others have belied the more simple hypothesis that TYR polymorphism is a principle (i.e., penetrant) component underlying normal variation of human pigmentation (Sturm et al., Gene 277:49-62, 2001). The present study also failed to identify penetrant genetic features of variable iris color in the TYR gene. In addition, our systematic approach for identifying penetrant genetic features independently confirmed that the "red hair" SNP alleles described by Valverde et al., Nature Genet. 11:328-330, 1995 and Koppula et al., Hum. Mutat. 9:30-36, 1997 are indeed associated with iris colors. However, our work has extended even these simple gene-wise analyses. While there are no SNPs or haplotypes within the TYR gene associated with iris color, TYR alleles are important within a complex genetics context for the inference of iris colors. While the "red hair" SNPs are indeed associated with natural iris colors (in Irish individuals), they seem to be most strongly associated with Caucasian iris colors within the multilocus context of another coding change in the MC1R gene, and even then, they represent merely one stroke of a larger portrait.

In fact, one of the most important points to be taken from the work presented herein is that speaking of variable iris color on the level of individual genes is illogical due to the complexity of the trait. The fact of the matter is, neither TYR nor MCIR. nor for that matter any of the other genes we surveyed, are very important for predicting iris colors on their own. This was indicated by the Bayesian conditional probabilities we obtained, which for even the most strongly associated alleles (the penetrant genetic features), were too low for their use as independent classifiers. Since the variance of any complex phenotype is a function of additive, dominance and epistatic genetic variance (in addition to environmental variance) any good complex genetics classifier must capture each of these three components when making inferences, and the classifier we have developed seems to be able to this. The additive component is captured most efficiently through the analysis of multilocus alleles (haplotypes) and the dominance component is captured by expressing individuals as vectors whose components are encodings of multilocus genotypes for each important region. The most innovative advance we have made here is algorithmically capturing the epistatic component. Our work showed that there is a minimal set of 25 penetrant SNPs, of 8 multilocus contexts in 4 genes that are required for minimal inference accuracy. However, a complete set of 57 SNPs, of 19 multilocus contexts (both penetrant and latent), in 7 of the 8 genes is needed for accurate inference. That latent genetic are needed for accurate inference suggests that there is a significant epistatic component to iris color variance in the Caucasian population. The agouti signaling protein (ASIP) harbored four and the silver locus (SILV) harbored three such polymorphisms, each of which was arbitrarily combined into a single latent feature SNP combination. DCT and TYR harbored five and six such polymorphisms, respectively. That no penetrant genetic features were identified in ASLP, SILV or TYR suggests that these genes contribute towards iris color variance largely through epistatic means. The latent features are not equivalently predictive, and to capture the epistatic component during classification, we randomly ascribed weights to different alleles in different contexts and selected the combination that allowed for the most optimal quadratic discrimination. Our results suggest that there is much to be learned about the genetics of iris color from a detailed inspection of this optimal weighting scheme. At present, we do not understand the mechanism by which the features fit together the way they do in the optimal COA-derived quadratic classifier model (we intend to present these data elsewhere), only that they do and that the fit is of maximal practical utility for the inference of iris colors. The results we have obtained suggest that iris color is indeed a complex genetic trait, the "whole" of which was empirically determined to be greater than the sum of it's "parts". On a more general level, our results illustrate a seemingly obvious but interesting concept: simple genetics approaches are useful for ascribing trait associations for individual genes and haplotypes within them, but because most human traits arc complex, complex genetics tools are required for their use in the development of accurate classification tests. Given the sources of error for this work, including genotyping errors, errors in self-reported iris color and statistical haplotype inference, it is quite remarkable that perfect classification accuracy was achieved with a combined sample size of 550 for such a complex trait. In terms of feature modeling, almost identical results were obtained using a classification tree (CART-based) method (unpublished data), even though the cost function of the method we used herein relates genotypes (haplotype pairs) to trait values in a more direct way than CART. Thus, it appears that the methods we employed herein are substantiated by other analytical methodologies and may be promising for the generation of other complex genetics classifiers, for example pharmacogenomics or complex disease genetics classifiers.

Though there are a number of processes, developmental and cellular, that could explain iris color variance, our results suggest that polymorphisms in merely seven genes explain all of the variation in iris colors in the population. This result is surprising. Studies in *Drosophila* have implicated over 85 genes in iris pigmentation (Ooi et al., EMBO J. 16(15):4508-4518, 1997; Lloyd et al., Trends Cell Biol. 8(7):257-259, 1998) and far more than 8 genes have been implicated in oculocutaneous albinism in model vertebrates. That almost all of iris color variance in human beings can be explained by polymorphisms in 7 of 8 carefully selected genes, given the biological complexity of pigmentation, illustrates that just because a gene is crucial for a process (i.e., its mutation causes loss of function) does not necessarily mean that natural distributions of this process among individuals is related to natural polymorphisms in this gene. By way of analogy, there are many ways to break an automobile engine - removing a water hose for example - but virtually none of the variability in engine performance is caused by variability in hose characteristics. Certain parts of the complex genetics "engine" seem to have become sinks for accumulating functionally relevant polymorphisms during the evolutionary branching of our ancestors.

In fact, one of the surprising findings of our work was that of all of the genes we tested, the OCA2 gene explained by far the most iris color variance. Five of the 8 feature SNP combinations were from the OCA2 gene and 17 of the 25 SNPs part of these penetrant feature SNP combinations were OCA2 SNPs. To date, no polymorphism screens within OCA2 have yet been described (though they had been called for - see Sturm et al., Gene 277:49-62, 2001) and this work is the first indication of the importance this gene has for natural iris color pigmentation. The OCA2 gene product localizes to the melanosomal membrane and resembles an E. coli Na+/H+ anti-porter. Though TYR activity correlates perfectly with eumelanin content in melanosomes (Iozumi et al., J. Invest. Dermatol. 100:806-811, 1993), its activity is thought to be manipulated by the OCA2 gene product through the control of intramelanosomal pH (Ancans et al., J. Invest. Dermatol. 117:158-159, 2001). Tyrosinase taken from dark and light skin functions identically in-vitro, but is highly pH dependent and melanocytes from white skin are more acidic than those from black (Fuller et al., Exp. Cell. Res. 262: 97-208, 2001, Ancans et al., Exp. Cell. Res. 268:26-35, 2001). Given these observations, it seems that OCA2 is the primary modifier of TYR activity, which is consistent with our statistical results. It is interesting to note that at the level of the cladogram analysis, four of the five allele associations were obtained for OCA2 feature SNP combinations. It is also interesting to note that the diversity of alleles associated with darker iris colors is significantly greater than that of alleles associated with lighter iris colors. These observations combined suggest that lighter colored irises branched from darker colored irises relatively long ago in human evolutionary time, and that modifications to the OCA2 gene may have been instrumental in this branching. The generally accepted anthropological and molecular view of the origin of modem humans from Africa states that Northern Europeans branched from African founders. Our results suggest that the reason lighter colored irises arc almost exclusive to individuals of Northern European ancestry is in large part due to relatively ancient (and numerous) modifications of the OCA2 expression product. The fact that brown classifications were far more accurate relative to blue before, but not after, the addition of the latent genetic features to the classifier model may indicate that blue irises are subject to more epistasis than dark, and that dark eyes tend to be relatively (though not strictly speaking) dominant.

When applied to a multi-racial sample, the penetrant feature (as well as the combined penetrant + latent feature) classifier performed with substantially better accuracy than when applied only to Caucasians. Since most non-Caucasian ethnic groups exhibit low variability in iris colors (on average of darker shade than Caucasians) this improvement may not seem surprising. However, though an incorrect solution would not necessarily be more accurate when applied to individuals of the world's various populations, notwithstanding genetic heterogeneity, a correct solution would be. The reason for this is that if alleles associated with darker iris color in Caucasians are deterministic, or linked to deterministic alleles for melanin production and iris color, and if we assume the between race component of iris color variance is low, the frequencies of these alleles should be greater in populations of average darker iris color. Because the accuracy of both our models increases when applied pan-ethnically, our results suggest that the penetrant and latent associations we have described are functionally relevant. Since most of the SNPs are intron or silent changes, we infer that the alleles we have described are statistically linked with other unidentified alleles, or are functional in ways other than through amino acid changes (such as RNA transcription, degradation, localization etc.). It is interesting that those that were amino acid changes tend to be changes in polarity, three of four involving an Arginine. Interestingly, the classifier we have generated for iris color does not accurately extend for classification of hair color or skin shade within Caucasians. In fact, this is what one would expect from a good complex genetic model for variable Caucasian iris color, since iris, skin and hair color are known to be independently inherited (and distributed) within this racial group. We have conducted a study similar to the one described herein for hair color and though there is about 33% overlap between the SNP marker sets, the sets are distinct (data be presented elsewhere). We assume that the classifier generated here would be, at least in part, extendable to other racial groups, such as for the discrimination between green, hazel and brown irises in individuals of African descent. Whether or not this is true is a subject for further study.

As the first genetic solution capable of ascribing qualitative characteristics from anonymously donated DNA, our results represent an important achievement. First, they illustrate one method for modeling complex human traits from high-density gcnomics data sets. Second, as a forensics tool, our solution could be used to guide criminal or other forensics investigations (in this case, multilocus genotype combinations that arc relatively ambiguous could be classified with regard to iris color shade and conditional probability statements offered for specific iris color classifications). Third, as a research tool, the common haplotypes we have identified may help researchers more accurately define the complex genetics risks for pigmentation related diseases such as cataracts and melanoma.

**Table 17-1. Genetic feature extraction results for human eye color.**

| GENE | CANDIDATE | □(n-SNP) | No. GENETIC | SELECTED HAPLOTYPE FEATURE | P-value |
|---|---|---|---|---|---|
| | SNPs[1] | TESTED[3] | FEATURES[4] | NAME, FEATURE Ids[5] | |
| AP3B1 | 6 | 1 | 0 | none | - |
| ASIP | 18 | 14 | 0 | none | - |
| DCT | 20 | 15 | 1 | DCT-B, (702\|650\|675) | <0.001 |
| 1R | MC16 | 8 | 1 | MC1R-A, (217438\|217439\|217441) | Insig* |
| OCA2 | 36 | 189 | 5 | OCA2-A, (217458\|886894\|886895\|88689 6) | <0.001 |
| | | | | OCA2-B, (217452\|712052\|886994) | <0.001 |
| | | | | OCA2-C, (712057\|712058\|712060\|71206 4) | 0.001 |
| | | | | OCA2-D, (712054\|712056\|886892) | 0.002 |
| | | | | OCA2-E, (217455\|712061\|886892) | 0.003 |
| SILV | 14 | 105 | 0 | None | - |
| TYR | 46 | 13 | 0 | None | - |
| TYRP1 | 28 | 66 | 1 | TYRP1-A, (886938\|886943) | <0.020 |
| | | | | | |
| TOTAL | 181 | 411 | 8 | 25 SNPs in 4 genes | |

1- Total number of SNPs in each gene tested for allelic association with iris color.
2- Total number of n-locus SNP combinations whose haploid alleles were tested for association with iris color using the genetic feature extraction algorithm described in the text. The number was dependent on the number of validated SNPs found from the total in column 2 (data not shown), and the results from lower order (i.e., 1, 2-locus combination) screens as described in the methods.
3- Number of non-overlapping SNP combinations, alleles of which were identified as genetic features for variable iris color as described in the text.
4- Name and SNP composition for each of the identified genetic features.
5- F-statistic P-value for the HAPLOTYPE FEATURE allele sequence composition between individuals of light and dark iris shade as described in the text.
*one haplotype for this combination was found to be strongly associated with iris color shade, but the other observed haplotypes were not significant.* this p-value was obtained within certain TYR genetic backgrounds. The p-value over a

**Table 17-2. Description of SNP loci incorporated into the haplotype features and classifier model for the inference of variable eye color described in the text.**

| GENE | HAPLOID FEATURE | POS. | MARKER | FCA (minor) | TYPE[1] | SOURCE[2] | Pigment HISTORY | SEQ ID NO: |
|---|---|---|---|---|---|---|---|---|
| | | | | | | | | |
| DCT | DCT-A | 2 | 702 | 0.15 | intron | dbsnp | none | I |
| DCT | DCT-A | 3 | 650 | 0.31 | intron | dbsnp | None | 2 |

(continued)

| GENE | HAPLOID FEATURE | POS. | MARKER | FCA (minor) | TYPE[1] | SOURCE[2] | Pigment HISTORY | SEQ ID NO: |
|---|---|---|---|---|---|---|---|---|
| DCT | DCT-A | 4 | 675 | 0.21 | intron | dbsnp | None | 3 |
| MCIR | MCIR-A | 1 | 217438 | 0.07 | VAL_MET | resequencing | Red hair/ blue eyes weak association[3] | 4 |
| MCIR | MC1R-A | 2 | 217439 | 0.07 | ARG_CYS | dbSNP, resequencing | Red hair/ blue eyes weak association[4] | 5 |
| MCIR | MC1R-A | 3 | 217441 | 0.07 | ARG_TRP | resequencing | Red hair/ blue eyes weak association[5] | 6 |
| OCA2 | OCA2-A | 1 | 217458 | 0.29 | Silent | dbSNP | None | 7 |
| OCA2 | OCA2-A | 2 | 886894 | 0.32 | intron | resequencing | None | 8 |
| OCA2 | OCA2-A | 3 | 886895 | 0.13 | intron | resequencing | None | 9 |
| OCA2 | OCA2-A | 1 | 886896 | 0.34 | intron | resequencing | None | 10 |
| OCA2 | OCA2-B | 2 | 217452 | 0.04 | ARG_TRP | dbSNP | None | 11 |
| OCA2 | OCA2-B | 3 | 712052 | 0.23 | intron | dbSNP | None | 12 |
| OCA2 | OCA2-B | 4 | 886994 | 0.19 | intron | resequencing | none | 13 |
| OCA2 | OCA2C | 1 | 712057 | 0.18 | intron | dbSNP | None | 14 |
| OCA2 | OCA2C | 2 | 712058 | 0.11 | intron | dbSNP | None | 15 |
| OCA2 | OCA2C | 3 | 712060 | 0.06 | intron | dbSNP | None | 16 |
| OCA2 | OCA2C | 4 | 712064 | 0.01 | Silent | dbSNP | None | 17 |
| OCA2 | OCA2D | 1 | 712054 | 0.37 | intron | dbSNP | None | 18 |
| OCA2 | OCA2D | 2 | 712056 | 0.02 | intron | dbSNP | None | 19 |
| OCA2 | OCA2D | 3 | 886892 | 0.03 | intron | dbSNP | None | 20 |
| OCA2 | OCA2E |  | 217455 | 0.42 | Silent | dbSNP | None | 21 |
| OCA2 | OCA2E |  | 712061 | 0.02 | Silent | dbSNP | None | 22 |
| OCA2 | OCA2E |  | 886892 | 0.19 | intron | resequencing | None | 23 |
| TYRP | TYRP-A | 1 | 886938 | 0.47 | intron | resequencing | None | 24 |
| TYRP | TYRP-A | 2 | 886943 | 0.47 | intron | resequencing | None | 25 |

1- SILENT - no amino acid change, INTRON - SNP was found in non-coding sequence.
2- DbSNP - candidate gene sequence is present in the NCBI:dbSNP as of 02/15/02. resequencing - SNP sequence was discovered from the re-sequencing effort described in the text.
3- Valverde et al., Nature Genet. 11:325-330, 1995 and Schioth et al., 1999.
4- Frandberg et al. Biochem. Biophys. Res. Commun. 245: 490-492, 1998; Smith et al., J. Invest. Derm. 111: 119-122, 1998.

The Gene and haplotype feature name are shown in columns 1 and 2. The position within the SNP combination discussed throughout the text is shown in column 3. The "marker" or unique identifier for the locus in column 4 and the frequency of the minor allele in the Caucasian population in column 5 ($f_{CA}$(minor)). The type of SNP (intron, silent and coding, where the two amino acid variants are separated by an underscore) is shown in column 6. The source of the SNP locus (from where we derived the sequence when designing our experiments) is shown in column 7 and the history of the SNP locus (whether there is any description of the SNP locus in the literature or otherwise common knowledge as relevant for the natural distribution of human pigmentation shades in any tissue).

**Table 17-3. Effect statistics for the association of genetic feature alleles with iris colors in the Caucasian population.**

| | Gene | Allele | p-value [1] | association | Posterior Probability[2] | (N)[1] | Genotypes: | p-value [1] | Association | Posterior Probability[2] | (N)[1] |
|---|---|---|---|---|---|---|---|---|---|---|---|
| 1 | MCIR- | ACCC | 0.0458 | Hazel | 0.369 | 499 | CCC/CCC | 0.0327 | Hazel | 0.344 | 186 |
| 2 | | Total | Insig. | | | 648 | Total | Insig. | | | 324 |
| 3 | OCA2-A | TTAA | 0.0079 | Blue | 0.382 | 423 | TTAA/TTAA | 0.0194 | Blue | 0.415 | 147 |
| 4 | | CCAG | 0.0008 | Brown | 0.447 | 85 | TTAA/CCAG | 0.0613 | Brown | 0.386 | 56 |
| 5 | | TTAG | 0.0045 | Brown | 0.627 | 13 | TTAA/TTAG | 0.0006 | Brown | 0.735 | 11 |
| 6 | | | | | | | TTAA/CTAG | 0.0167 | Blue | 0.795 | 5 |
| 7 | | | | | | | CCAG/CCAG | 0.0488 | Brown | 0.584 | 7 |
| 8 | | | | | | | CCAG/CCGG | 0.0050 | Brown | 0.649 | 11 |
| 9 | | Total | 0.0453 | | | 606 | Total | 0.0053 | | | 303 |
| 10 | OCA2-B | CAA | 0.0269 | Blue | 0.381 | 354 | CAA/CAA | 0.0255 | Hazel | 0.375 | 112 |
| 11 | | CGA | 0.0024 | Brown | 0.389 | 131 | CAA/CGA | 0.0314 | Blue | 0.443 | 70 |
| 12 | | CAC | 0.0200 | Brown | 0.386 | 83 | CGA/CAC | 0.0024 | Brown | 0.542 | 24 |
| 13 | | CGC | 0.0441 | Green | 0.417 | 12 | CGA/CGC | 0.0006 | Green | 0.500 | 6 |
| 14 | | Total | 0.0058 | | | 606 | Total | 0.02148 | | | 303 |
| 15 | TYRP- | BTC | 0.001 | Blue | 0.403 | 234 | none | --- | --- | --- | |
| 16 | | Total | 0.0451 | | | 660 | Total | Insig. | | | 330 |
| 17 | DCT-B | CTG | 0.0133 | Brown | 0.362 | 94 | GCA/CTG | 0.0006 | Hazel | 0.100 | 53 |
| 18 | | GTG | 0.0249 | Hazel | 0.571 | 7 | GCA/GTA | 0.0527 | Blue | 0.625 | 8 |
| 19 | | | | | | | GCA/GTG | 0.0090 | Hazel | 0.667 | 6 |
| 20 | | | | | | | CCA/CTG | 0.0044 | Blue | 0.412 | 17 |
| 21 | | Total | Insig. | | | 660 | Total | Insig. | | | 330 |
| 12 | OCA2-C | GGAA | 0.0013 | Blue | 0.382 | 463 | GGAA/GGAA | 0.0096 | Blue | 0.4045 | 178 |
| 23 | | TGAA | 0.0125 | Brown | 0.4058 | 69 | GGAA/TAAA | 0.0089 | Hazel | 0.5385 | 13 |
| 24 | | TAAA | 0.0475 | Hazel | 0.4375 | 16 | TGAA/TAAA | 0.0033 | Brown | 1.0000 | 3 |
| 25 | | | | | | | GGGA/GGGA | 0.0500 | Brown | 0.3333 | 3 |
| 26 | | Total | 0.0189 | | | 606 | Total | 0.0547 | | | 303 |
| 27 | OCA2-D | AGG | 0.0468 | Hazel | 0.2832 | 346 | AGG/AGG | 0.0445 | Hazel | 0.3148 | 108 |
| 28 | | GGG | 0.0222 | Brown | 0.3377 | 231 | AGG/AGC | 0.0202 | Brown | 0.6667 | 6 |
| 29 | | | | | | | GGG/GGG | 0.0509 | Brown | 0.3913 | 46 |
| 30 | | Total | Insig. | | | 606 | Total | Insig. | | | 303 |

(continued)

| | Gene | Allele | p-value [1] | association | Posterior Probability[2] | (N)[1] | Genotypes: | p-value [1] | Association | Posterior Probability[2] | (N)[1] |
|---|---|---|---|---|---|---|---|---|---|---|---|
| 31 | OCA2-E | GCA | 0.0004 | Brown | 0.4828 | 58 | ACG/GCA | 0.0436 | Brown | 0.4048 | 42 |
| | | | | | | | GCA/GCA | 0.0034 | Brown | 1.0000 | 3 |
| 32 | | | | | | | GCA/GCG | 0.0060 | Brown | 0.8000 | 5 |
| 33 | | Total | Insig. | | | 614 | Total | Insig. | | | 307 |

1- Pearsons Chi-square statistic p-value. Only alleles and allele combinations that were significantly associated with an iris color are shown (n>2).
2- Bayesian posterior probability of correct eye color classification using allele frequency in the eye color group as the class conditional probability.

**Table 17-4. Nested contingency analysis of haplotype cladograms for the identified genetic features of variable eye color.**

| Feature | Contingency Significance | Allele partition | p-value[1] | Site(s).[2] |
|---|---|---|---|---|
| MC1R-A | none found | ------ | --- | --- |
| OCA2-A | Between 3-Step Clades | (CCAG+CCGG+TCAG+TCGG+TCAA) vs. (TTGG+TTAG+CTAG+CTAA+TTAA) | 0.0011 | 2 |
| OCA2-B | Within 1-Step Clades | CGA vs. CAA | 0.0012 | 2 |
| | Between 2-Step Clades | (TAC + CAC + CGC) vs. (CGA + CAA + TAA + TGA) | 0.0246 | 3 |
| OCA2-C | Between 3-Step Clades | (TGAA+TAAA+TAAG) vs. (GGAA+GAAA+GGGA+GAGA+GGAG) | 0.0014 | 1 |
| | Within 1-Step Clades | TGAA vs. TAAA | 0.0263 | 2 |
| OCA2-D | Between 3-Step Clades[3] | (AGC+GGC) vs. (AGG+GGG+AAG+GAG) | 0.0052 | 3 |
| OCA2-E | none found | --- | --- | --- |
| TYRP-A | Between 2-step Clades | (CC+CT+TT) vs. TC | 0.0136 | 1 |
| DCT-B | none found | --- | --- | --- |

1- Chi-square statistic p-value, degrees of freedom were 3 for each analysis.

2- Locus within the SNP combination that the nested contingency analysis shows significant variations in eye colors can be traced back to. This information is also present in the Allele partition column.

3- This is a good example of the value this nested cladogram analysis afforded. Though two alleles of the OCA2-D SNP combination (AGG and GGG, rows 27 and 28, column 3, Table 3) were associated with iris colors from Chi-square adjusted residual analysis, the Chi-square statistic for contingency analysis of all of the alleles together was not significant (row 30, column 3, Table 3). The nested cladogram analysis showed that these two sequences are evolutionary neighbors and suggested that the GG 3' end of the OCA2-D combination is strongly associated with darker iris colors (p=0.0014, Table 4). Evidently, this significance was lost in the noise produced at the pan-allele level by the lack of association between the other four haplotype alleles and iris colors.

**Table 17-5A Allele frequency difference for alleles of latent haploid genetic features among racial groups.**

| GENE | MARKER | $F_{ca}$[1] | $F_{aa}$[2] | $F_{as}$[3] | $F_{light}$[4] | $F_{dark}$[5] | SEQ ID NO: |
|---|---|---|---|---|---|---|---|
| ASIP | 560 | 0.01 | 0 | 0.10 | 0.01 | 0.03 | 26 |
| ASIP | 552 | 0.19 | 0.58 | 0.23 | 0.19 | 0.49 | 27 |
| ASIP | 559 | 0.07 | 0.28 | 0 | 0.07 | 0.21 | 28 |
| ASIP | 468 | 0.20 | 0.80 | 0.40 | 0.20 | 0.70 | 29 |
| | | | | | | | |
| DCT | 657 | 0.28 | 0.29 | 0.90 | 0.28 | 0.44 | 30 |
| DCT | 674 | 0.36 | 0.56 | 0.63 | 0.36 | 0.58 | 31 |
| DCT | 632 | 0.01 | 0 | 0 | 0.01 | 0 | 32 |

(continued)

| GENE | MARKER | $F_{ca}$[1] | $F_{aa}$[2] | $F_{as}$[3] | $F_{light}$[4] | $F_{dark}$[5] | SEQ ID NO: |
|---|---|---|---|---|---|---|---|
| DCT | 701 | 0.21 | 0.32 | 0.10 | 0.21 | 0.27 | 33 |
| DCT | 710 | 0.53 | 0.37 | 0.57 | 0.53 | 0.42 | 34 |
| | | | | | | | |
| OCA2. | 217456 | 0.17 | 0.03 | 0.03 | 0.17 | 0.03 | 35 |
| | | | | | | | |
| SILV | 656 | 0.17 | 0.49 | 0.20 | 0.17 | 0.42 | 36 |
| SILV | 662 | 0.46 | 0.22 | 0.60 | 0.46 | 0.32 | 37 |
| SILV | 637 | 0.03 | 0 | 0.03 | 0.03 | 0.01 | 38 |
| | | | | | | | |
| TYR | 278 | 0.73 | 0.42 | 0.53 | 0.73 | 0.45 | 39 |
| TYR | 386 | 0.72 | 0.46 | 0.50 | 0.72 | 0.46 | 40 |
| TYR | 217480 | 0.17 | 0.03 | 0.03 | 0.17 | 0.03 | 41 |
| TYR | 951497 | 0.24 | 0.48 | 0.37 | 0.24 | 0.45 | 42 |
| TYR | 217468 | 0.64 | 0.10 | 0 | 0.64 | 0.08 | 43 |
| TYR | 217473 | 0.29 | 0.09 | 0.02 | 0.29 | 0.07 | 44 |
| | | | | | | | |
| TYRP1 | 217485 | 0.40 | 0.10 | 0.07 | 0.40 | 0.10 | 45 |
| TYRP1 | 217486 | 0.86 | 0.27 | 0.03 | 0.86 | 0.22 | 46 |
| TYRP1 | 869787 | 0 | 0.07 | 0 | 0 | 0.05 | 47 |
| TYRP1 | 869745 | 0 | 0.07 | 0 | 0 | 0.05 | 48 |
| TYRP1 | 886933 | 0.15 | 0.41 | 0.23 | 0.15 | 0.37 | 49 |
| TYRP1 | 886937 | 0.16 | 0.10 | 0 | 0.16 | 0.08 | 50 |
| TYRP1 | 886942 | 0 | 0.06 | 0 | 0 | 0.04 | 51 |

**Table 17-5B.**

| GENE | MARKER | $F_{ca}$ | $F_{aa}$ | $F_{as}$ | $F_{light}$ | $F_{dark}$ |
|---|---|---|---|---|---|---|
| SILV | 704 | 0.66 | 0.59 | 0.77 | 0.66 | 0.63 |
| | 699 | 0.30 | 0.11 | 0.87 | 0.30 | 0.30 |

1- Frequency in the Caucasian racial group (N=100).
2- Frequency in the African racial group (N=100).
3- Frequency in the Asian racial group (N=30).
4- The Caucasian group (N=100) is designated the light (blue, green or hazel) iris colored race since the frequency of these iris colors is greatest in this group.
5- The African and Asian groups (N=130) is designated the dark (black and brown) iris colored race since the frequency of blue, green and hazel irises are lowest in this group.

**Table 17-5C. Allele frequency difference for alleles of latent haploid genetic features among racial groups.**

| COMBO. NAME | GENE | POS.[1] | SNP | $F_{ca}$[2] | $F_{aa}$[3] | $F_{as}$[4] | $F_{light}$[5] | $F_{dark}$[6] |
|---|---|---|---|---|---|---|---|---|
| | | | | | | | | |
| ASIP-A(L) | ASIP | 1 | 552 | 0.19 | 0.58 | 0.23 | 0.19 | 0.49 |
| ASIP-A(L) | ASIP | 2 | 468 | 0.2 | 0.8 | 0.4 | 0.2 | 0.7 |
| | | | | | | | | |
| DCT-B (L) | DCT | 1 | 657 | 0.28 | 0.29 | 0.9 | 0.28 | 0.44 |
| DCT-B (L) | DCT | 2 | 701 | 0.21 | 0.32 | 0.1 | 0.21 | 0.27 |
| | | | | | | | | |
| SILV-A (L) | SILV | 1 | 656 | 0.17 | 0.49 | 0.2 | 0.17 | 0.42 |
| SILV-A (L) | SILV | 2 | 662 | 0.46 | 0.22 | 0.6 | 0.46 | 0.32 |
| | | | | | | | | |
| TYR-A (L) | TYR | 1 | 278 | 0.73 | 0.42 | 0.53 | 0.73 | 0.45 |
| TYR-A (L) | TYR | 2 | 386 | 0.72 | 0.46 | 0.5 | 0.72 | 0.46 |
| | | | | | | | | |
| TYRP-B (L) | TYRP1 | 1 | 217485 | 0.4 | 0.1 | 0.07 | 0.4 | 0.1 |
| TYRP-B (L) | TYRP1 | 2 | 886933 | 0.15 | 0.41 | 0.23 | 0.15 | 0.37 |
| TYRP-B (L) | TYRP1 | 3 | 886937 | 0.16 | 0.1 | 0 | 0.16 | 0.08 |

**Table 17-6. Correspondence analysis assisted quadratic discriminate-based classification of iris colors using the penetrant genetic features of variable iris color.** A

| | Light Iris Classification[1] | Dark Iris Classification[1] |
|---|---|---|
| Individuals of Light Irises | 97.5% (197) | 2.5% (5) |
| Individuals of Dark Irises | 0 | 100 (84) |

B

**Table 17-7. Correspondence analysis assisted quadratic discriminate-based classification of iris colors using both penetrant and latent genetic features of variable iris color.** A

| | Blue Iris Classification[1] | Brown Iris Classification[1] | Green Iris Classification[1] | Hazel Iris Classification[1] |
|---|---|---|---|---|
| Individuals of Blue Irises | 100% (97) | 0 | 0 | 0 |
| Individuals of Brown Irises | 19% (40) | 69% (141) | 0 | 12% (24) |
| Individuals of Green Irises | 0 | 0 | 100% (32) | 0 |
| Individuals of Hazel Irises | 14% (12) | 0 | 1% (1) | 84% (69) |

1. Percent classified is shown, with the number of individuals classified shown in parentheses.
A) Probability table for classification between dark (black and brown) versus light (blue, green and hazel) iris colors.
B) Probability table for classification among the various iris colors.

| | Blue Iris Classification[1] | Brown Iris Classification[1] | Green Iris Classification[1] | Hazel Iris Classification[1] | Total |
|---|---|---|---|---|---|
| Individuals of Blue Irises | **100% (97)** | 0 | 0 | 0 | 97 |
| Individuals of Brown Irises | 0 | **100% (84)** | 0 | 0 | 84 |
| Individuals of Green Irises | 0 | 0 | **100%(30)** | 0 | 30 |
| Individuals of Hazel Irises | 0 | 0 | 0 | **100%(75)** | 75 |
| Total | 97 | 84 | 31 | 59 | 286 |

B

| | Light Iris Classification[1] | Dark Iris Classification[1] |
|---|---|---|
| Individuals of Light Irises | 100% (197) | 0 |
| Individuals of Dark Irises | 0 | 100% (84) |

1. Percent classified is shown, with the number of individuals classified shown in parentheses.

Table 27-7. A) Probability table for classification between dark (black and brown) versus light (blue, green and hazel) iris colors. B) Probability table for classification among the various iris colors.

Table 17-8 Primers for Nucleotide Occurrence Determination of SNPs

| SEQ ID NO | Marker Name | Marker No. | Primer Use | Sequence |
|---|---|---|---|---|
| | | | | |
| 100 | TYRP 4 | 217486 | PCR | GAGTATGTGAAGATATAAGTAAGTGAACTACCAT |
| 101 | | | PCR | ACTGTGGTTTTCTTTAAATCTGTTGAC |
| 102 | | | Primer ext | AGCGATCTGCGAGACCGTATATTTCTAAAATGTTAAAACATAAAC |
| | | | | |
| 103 | DCT2892681 | 650 | PCR | AAGGAGAAGGCAAGATCCTAAG |
| 104 | | | PCR | GCCCTCCTGAGAGCTACAATTT |
| 105 | | | Primer ext | GGCTATGATTCGCAATGCTTCAATTAGTAATCTGGAGAGATAAAA |
| 106 | | | Primer ext | GGATGGCGTTCCGTCCTATTCAATTAGTAATCTGGAGAGATAAAA |
| | | | | |
| 107 | OCA2E16 300 | 886892 | PCR | TGGCATTCATCTTGATCTTGG |
| 108 | | | PCR | CTGTGGGCAAAGTCAGTGTCT |
| 109 | | | Primer ext | ACGCACGTCCACGGTGATITGGTTCATAGGCTTTGTCACATTCTG |
| | | | | |

(continued)

| SEQ ID NO | Marker Name | Marker No. | Primer Use | Sequence |
|---|---|---|---|---|
| 110 | OCA2E10 549 | 886994 | PCR | AGCCATTAGCTTCTGATTACTTTGC |
| 111 | | | PCR | GGCCAGAGCTGGCTGGTG |
| 112 | | | Primer ext | ACGCACGTCCACGGTGATTTTTTTGGTGAAA TAATTTCCATGATT |
| | | | | |
| 113 | TYRP 3 | 217485 | PCR | GTGGTCTAACAAATGCCCTACTCTC |
| 114 | | | PCR | AAAGGGTCTTCCCAGCTTTG |
| 115 | | | Primer ext | AGGGTCTCTACGCTGACGATTCTTTCTAATA CAAGCATATGTTAG |
| | | | | |
| 116 | TYR 3 | 217468 | PCR | TAACGACATCAATATTTATGACCTCTTTG |
| 117 | | | PCR | GCAGAAAAGCTGGTGCTTCA |
| 118 | | | Primer ext | CGTGCCGCTCGTGATAGAATTCAATGGATG CACTGCTTGGGGGAT |
| | | | | |
| 119 | ASIP2424984 | 468 | PCR | AGTGGCCCAAGCTCACTTA |
| 120 | | | PCR | AAGGCAAATGGGAAATCCAA |
| 121 | | | Primer ext | AGATAGAGTCGATGCCAGCTGTCGAGGGAC CAGGCCCCACAAGAG |
| | | | | |
| 122 | DCT2031527 | 675 | PCR | CCCTGGGGCAACCTTACTAA |
| 123 | | | PCR | CAGCATTTTGTTCACTCAGTTCTC |
| 124 | | | Primer ext | GGATGGCGTTCCGTCCTATTAAACATATCAC CTACTATGACAGTA |
| | | | | |
| 125 | DCT1325611 | 657 | PCR | GCATCTAAGGCCCTCTGTACCT |
| 126 | | | PCR | TAGAAAGCAATCAAGATGATTTCAGAG |
| 127 | | | Primer ext | GCGGTAGGTTCCCGACATATCTCTTTCATAA ATTTGAACTTAATT |
| | | | | |
| 128 | OCA2 2 | 217452 | PCR | TAAGGTCGTTGTTTCGTTCT |
| 129 | | | PCR | ATGAGCCATCAAAAGAGGG |
| 130 | | | Primer ext | AGAGCGAGTGACGCATACTACAGAGAGACG GTGTCCATCAGCATC |
| | | | | |
| 131 | OCA2 8 | 217458 | PCR | GCCTGGACTTTGCCGGAT |
| 132 | | | PCR | CTTTCTGTTCCAGTAAAGGAGTCTGA |
| 133 | | | Primer ext | GTGATTCTGTACGTGTCGCCCTGCACACAT GTTCATTGGGATTTG |
| | | | | |

(continued)

| SEQ ID NO | Marker Name | Marker No. | Primer Use | Sequence |
|---|---|---|---|---|
| 134 | OCA2DBSNP_252 | 712056 | PCR | GACACGAATTTTTATTGGACATGTTTA |
| 135 | | | PCR | AGGGTTATGCTCAAGGCCAT |
| 136 | | | Primer ext | AGCGATCTGCGAGACCGTATTTATTGTAGTAGATGTTCATGATTC |
| | | | | |
| 137 | SILV1052206 | 656 | PCR | GCTGCGTCTACCCCGCAT |
| 138 | | | PCR | AAATATAGGTGTTTCTGTCAACTCCAG |
| 139 | | | Primer ext | AGAGCGAGTGACGCATACTATCTGCTCTTGTCCCATTGGTGAGAA |
| | | | | |
| 140 | SIL V1052165 | 662 | PCR | TCCTGAGAAATCAGCCTCTG |
| 141 | | | PCR | AGTCCCAGGTGTAGGAGAGGTC |
| 142 | | | Primer ext | GTGATTCTGTACGTGTCGCCCCTTTGCCCTGCAGCTCCATGACCC |
| | | | | |
| 143 | TYRP1E4 32 | 886933 | PCR | GCCCCTCAGACACCGTTG |
| 144 | | | PCR | ATTATTCATTTCTGTTTGGTCTACTCTCTG |
| 145 | | | NPCR | CCTCAGACACCGTTGATATAC |
| 146 | | | NPCR | GTGTAGGCACTTTCTGTTTCC |
| 147 | | | Primer | GGATGGCGTTCCGTCCTATTTACCTTATTGT |
| | | | ext | CTGAAGAGAGCTAA |
| | | | | |
| 148 | TYRP1 E7 420 | 886943 | PCR | TCCAAAARCAAATGTGTTATCTTTCA |
| 149 | | | PCR | AGGGTGCTGTACAATAAGATCAATATC |
| 150 | | | Primer ext | GGCTATGATTCGCAATGCTTTTGGACTTGGAAACTTTCATTTGTA |
| | | | | |
| 151 | MC1R 5 | 217439 | PCR | ATCGCCGTGGACCGCTAC |
| 152 | | | PCR | GGGTCACGRTGCTGTGGTA |
| 153 | | | Primer ext | ACGCACGTCCACGGTGATTTCTACATCTCCATCTTCTACGCACTG |
| | | | | |
| 154 | MC1R 7 | 217441 | PCR | TACATCTCCATCTTCTACGCACTG |
| 155 | | | PCR | GATGAAGAGCGTGCTGAAGAC |
| 156 | | | Primer ext | CGTGCCGCTCGTGATAGAATCTACCACAGCATCGTGACCCTGCCG |
| | | | | |
| 157 | OCA2_RS1800405 | 712061 | PCR | CATGCTGGGTTCCCTTGC |

(continued)

| SEQ ID NO | Marker Name | Marker No. | Primer Use | Sequence |
|---|---|---|---|---|
| 158 | | | PCR | CACTGAGTGGTAAGCCAGGG |
| 159 | | | Primer ext | AGGGTCTCTACGCTGACGATCACTGGCAGC ACTGGCTGTGATTGG |
| | | | 1 | |
| 160 | ASIP819135 | 552 | PCR | AAGGGGCCACTTACCTCTTCA |
| 161 | | | PCR | GGCAGAGTTGTTGAAAGGCC |
| 162 | | | Primer ext | GACCTGGGTGTCGATACCTAACTTAATTTAT TAGCCTTATTCTGT |
| | | | | |
| 163 | DCT2296498 | 701 | PCR | ATCAACTCATATAGAGTGACTATGATGG |
| 164 | | | PCR | CCTGCTTGGAGAGAGAGATTCA |
| 165 | | | Primer ext | GGCTATGATTCGCAATGCTTGAGGATCAAGA TTTCGGGAAGAAAA |
| | | | | |
| 166 | DCT1028806 | 702 | PCR | TTAGTCCTAATGCAGTATTTATGTAACC |
| 167 | | | PCR | TCTCAGCGAACATGCTTGT |
| 168 | | | Primer ext | CGTGCCGCTCGTGATAGAATAACTTTCGCGT ATTTTGCCTCACCC |
| 169 | | | Primer ext | AGCGATCTGCGAGACCGTATAACTTTCGCG TATTTTGCCTCACCC |
| | | | | |
| 170 | OCA2 5 | 217455 | PCR | CGGTAATTTCCTGTGCTTCT |
| 171 | | | PCR | AACTTACATCGCCAATCACAG |
| 172 | | | Primer ext | AGAGCGAGTGACGCATACTATCCAGATCGT GCACAGAACTCTGGC |
| | | | | |
| 173 | OCA2DBSNP_ 52401 | 712052 | PCR | TTTCTTCTAATGGCATTGCATTTT |
| 174 | | | PCR | CTAATAGACTAATATAACCCAAACAGAAGTC CT |
| 175 | | | Primer ext | GTGATTCTGTACGTGTCGCCGAATAGACCA GACACCTAGACTTTA |
| | | | | |
| 176 | OCA2DBSNP_ 146405 | 712054 | PCR | AAACATCTTTATAGAGCCTTTCCCTG |
| 177 | | | PCR | GCCTTCAGGGCCAGGAGC |
| 178 | | | Primer ext | ACGCACGTCCACGGTGATTTTGCACGTTGC AGGGCCCGCCCTCTG |
| | | | | |
| 179 | OCA2DBSNP_ 98488 | 712058 | PCR | AAACATCTTTATAGAGCCTTTCCCTG |

(continued)

| SEQ ID NO | Marker Name | Marker No. | Primer Use | Sequence |
|---|---|---|---|---|
| 180 | | | PCR | GCCTTCAGGGCCAGGAGC |
| 181 | | | Primer ext | ACGCACGTCCACGGTGATTTTGCACGTTGC AGGGCCCGCCCTCTG |
| | | | | |
| 182 | OCA2DBSNP_ 165011 | 712060 | PCR | CTCTTGGAACAAGTGAAAAATGA |
| 183 | | | PCR | TGCTCTTAGGATGTTTTCAGATTGA |
| 184 | | | Primer ext | GGCTATGATTCGCAATGCTTTCATTTCCATTT GGTTCTTTTTTCT |
| | | | | |
| 185 | OCA2RS18004 14 | 712064 | PCR | TCAGAAGGTTGTGCAGAGTAA |
| 186 | | | PCR | AACACTGTCAGGCATTTGG |
| 187 | | | Primer ext | ACGCACGTCCACGGTGATTTTGAGCTGTGG TTTCTCTCTTACAGC |
| | | | | |
| 188 | OCA2E14 447 | 886894 | PCR | TAATACRTGATATTTAGGTGACGCACA |
| 189 | | | PCR | GTGTTGTTTCTTTGGTCCTTAAACTC |
| 190 | | | Primer ext | GGATGGCGTTCCGTCCTATTTAAACTCGGCT GTGTACCCCCTGCA |
| 191 | | | Primer ext | CGTGCCGCTCGTGATAGAATCATTTTATCTA ACCCTCACTGAGCT |
| | | | | |
| 192 | OCA2E11 263 | 886895 | PCR | ATGCTCCTCTTCACGCCTG |
| 193 | | | PCR | CTTTTCATGCACCTGAGAATGG |
| 194 | | | Primer ext | AGATAGAGTCGATGCCAGCTGTACGCAAAG CACCTCTGCCGTGGG |
| | | | | |
| 195 | OCA2E11 350 | 886896 | PCR | TGCCTGGCTCCAGGTTCC |
| 196 | | | PCR | CAGACACGAGCTGGACTGG |
| 197 | | | Primer ext | CGACTGTAGGTGCGTAACTCCTCAGGTGCA TGAAAAGGTGGGGGC |
| 198 | | | Primer ext | AGGGTCTCTACGCTGACGATCTCAGGTGCA TGAAAAGGTGGGGGC |
| | | | | |
| 199 | OCA2E10 102 | 886993 | PCR | GTTTTAATATGGTGTCCTGCTAAAA |
| 200 | | | PCR | TTTACAGCACAATAATCGAAAAATC |
| 201 | | | Primer ext | AGCGATCTGCGAGACCGTATTTATCCTTGTC TTCTTCTTTTCCCC |
| 202 | | | Primer ext | GCGGTAGGTTCCCGACATATTTATCCTTGTC TTCTTCTTTTCCCC |

(continued)

| SEQ ID NO | Marker Name | Marker No. | Primer Use | Sequence |
|---|---|---|---|---|
| | | | | |
| 203 | TYR_RS1851992 | 278 | PCR | TATTGAGTAGCTCACAAAATCATGGA |
| 204 | | | PCR | TGCCCTGTGTTCTATAGCATGG |
| 205 | | | Primer ext | GCGGTAGGTTCCCGACATATAAACAGGTGAGAATAAGCAAGAAGG |
| | | | | |
| 206 | TYR_RS1827430 | 386 | PCR | GAAAAAAAAAGGTTTTGAGACATGAGT |
| 207 | | | PCR | GGTCCCAGTATTTCAGGTGAATAAA |
| 208 | | | Primer ext | GGCTATGATTCGCAATGCTTGACTGTAAGGTGACCTGGGAAATTC |
| 209 | | | Primer ext | AGCGATCTGCGAGACCGTATGACTGTAAGGTGACCTGGGAAATTC |
| | | | | |
| 210 | TYRP1E6 354 | 886938 | PCR | ATGAATGGCTGAGGAGATAC |
| 211 | | | PCR | AACTGATAACTATGCCATCTAAACAAT |
| 212 | | | Primer ext | AGGGTCTCTACGCTGACGATAATCYGCCCAGCTGAGCATGCAAAA |
| | | | | |
| 213 | MC1R 4 | 217438 | PCR | ACTCACCCATGTACTGCTTCA |
| 214 | | | PCR | TCAATGACATTGTCCAGCTG |
| 215 | | | Primer ext | CGTGCCGCTCGTGATAGAATGGASCTGCTGGTGAGCGGGASSAAC |
| | | | | |
| 216 | OCA2DBSNP_8321 | 712057 | PCR | TGTGCCTGCTCTATGTCTGTGT |
| 217 | | | PCR | GGTGCACACACAGAGACATACAG |
| 218 | | | Primer ext | ACGCACGTCCACGGTGATTTTGCACCAGTGTGAACTGTGTAGGTT |
| 219 | | | Primer ext | AGCGATCTGCGAGACCGTATTGCACCAGTGTGAACTGTGTAGGTT |
| | | | | |
| 220 | TYRP1E4 499 | 886937 | PCR | CCTCAGACACCGTTGATATAC |
| 221 | | | PCR | GTGTAGGCACTTTCTGTTTCC |
| 222 | | | NPCR | CCTCAGACACCGTTGATATAC |
| 223 | | | NPCR | GTGTAGGCACTTTCTGTTTCC |
| 224 | | | Primer ext | ACGCACGTCCACGGTGATTTCACCTAGAATGTTCAAGGTACTCTA |

Table 17-8. PCR indicates that the primer was used in a PCR reaction to amplify a target polynucleotide surrounding the SNP. NPCR indicates that the primer was used for a nested PCR which amplified a sequence within the amplified product of the first PCR reaction. Primer ext indicates the primer that was used in a primer extension reaction using the amplified product as a template.

**EXAMPLE 18**

**IDENTIFICATION OF PENETRANT HAPLOTYPES FOR INFERING HAIR COLOR**

This example provides the identification of penetrant SNP marker and marker sets that are associated with hair color. Penetrant SNP marker sets were identified that were associated with variable hair color in precisely the same way we have just described for eye color, except of course during genetic feature extraction step where we partitioned individuals by hair color shade rather than eye color shade. Table 18-1 lists some of the markers that were identified and provides data on the frequency of alleles of those SNPs for individuals of different hair color, and a justification for considering the SNP preferentially segregated in either light or dark hair. The results of feature extraction are shown in Table 18-2. Table 18-3, lists some of the individual SNPs that were identified and provide further information on these SNPs which are also included in Table 1.
The SNP markers with penetrant alleles associated with Caucasian hair color were:

1. OCA2 gene: Markers 886896, 886894, 217458, 712060, 886895, 712057, 712054, 886892, 217455, and 712056.
2. TYRP gene: Markers 217486, 886937.
3. MC1R gene: Markers 217438, 217439, 217441.
4. ASIP gene: Markers 559, 560.

Those in bold print arc markers were discovered by re-sequencing efforts and were not found in the literature or in any public database, and were useful in developing certain preferred hair color classifiers described herein.
It is interesting to note with respect to penetrant hair color SNPs and haplotypes, versus penetrant eye color SNPs and haplotypes, the following:

1) Penetrant SNPs were identified within the ASIP gene to be predictive of human hair color, but none were identified as predictive for Caucasian eye color.
2) No penetrant SNPs or penetrant SNP sets were identified within the TYR or DCT genes as associated with Caucasian hair color, though 3 in DCT were identified as associated with Caucasian eye color.
3) The penetrant TYRP SNPs identified as associated with Caucasian hair color are different from those identified as associated with Caucasian eye color.
4) The penetrant MC1R and OCA2 SNPs identified as associated with Caucasian hair color are the same SNPs identified as associated with Caucasian eye color, though not all of the OCA2 SNPs identified as associated with Caucasian eye color were included in the set associated with hair color.

These observations are interesting because it is known that hair and eye color are independently inherited, but individuals of darker hair have a darker average eye color shade. That the OCA2 and MC1R SNP sets we identified as associated with Caucasian hair color were a subset of those identified as associated with Caucasian eye color may indicate why it is that eye and hair color shades tend to co-occur in the Caucasian (in fact, the world) population. That the TYRP, ASIP, TYR and DCT SNPs identified as associated with hair or eye color (as the case may be) were distinct makes sense in terms of what is known about the genetics of eye and hair color inheritance; namely that the two traits are independently inherited. For example, there exist brown or even black hair individuals with blue or green eyes, and there exist blond haired individuals with brown eyes. Knowing the eye color of parents imparts no ability to predict the eye and hair color of their offspring. Knowing the hair color of parents imparts some ability to predict the hair color of their offspring, but not all. Obviously the inherited factors imparting eumelanin content in human hair and eyes are distinct, and our findings that the human polymorphism sets related to eye and hair color are distinct, yet overlapping, seem to make perfect sense in light of the biology of these two traits and validate the invention.

**Table 18-1. Genetic feature extraction table for variable HAIR color shade.** SNPS WITH ALLELES THAT SEGREGATE PREFERENTIALLY IN EITHER DARK OR LIGHT HAIR COLORED CAUCASIANS:

| 1. GENE | SNPNAME | MARKER | LOCATION | GENBANK | INTEGRITY |
|---|---|---|---|---|---|
| OCA2 | OCA2_5 | 217455 | 21103 | 13651545 | POLY |
| | 217455 | OCA2_5 | | | |

(continued)

| | AA | GA | GG |
|---|---|---|---|
| BLACK | 1 | 2 | 0 |
| BROWN | 38 | 21 | 0 |
| AUB/RED | 6 | 0 | 0 |
| BLOND | 9 | 2 | 0 |

JUSTIFICATION: This SNP is part of the OCA3LOC109 and OCA3LOC920 haplotype systems, the utility of which has been demonstrated in the text elsewhere in this patent. As can be seen from this distribution, the ratio of AA:GA: GG alleles in dark (BLACK + BROWN) haired individuals is 39:23:0 but 15:2:0 in light haired persons, which is significantly different. Thus, the G allele is enriched for individuals of darker (brown and hazel) hair color.

| 2. GENE | SNPNAME | MARKER | LOCATION | GENBANK | INTEGRITY |
|---|---|---|---|---|---|
| OCA2 | OCA2_6 | 217456 | 26558 | 13651545 | POLY |

| 217456 | OCA2_6. | | |
|---|---|---|---|
| | AA | GA | GG |
| BLACK | 0 | 1 | 1 |
| BROWN | 0 | 5 | 41 |
| AUB/RED | 0 | 0 | 5 |
| BLOND | 0 | 0 | 12 |

JUSTIFICATION: As can be seen from this distribution, the A allele was only observed in individuals of dark (BLACK or BROWN) hair color.

| 3. GENE | SNPNAME | MARKER | LOCATION | GENBANK | INTEGRITY |
|---|---|---|---|---|---|
| OCA2 | OCA2_8 | 217458 | 86326 | 13651545 | POLY |

| 217458 | OCA2_8 | | |
|---|---|---|---|
| | CC | CT | TT |
| BLACK | 0 | 2 | 2 |
| BROWN | 5 | 26 | 34 |
| AUB/RED | 0 | 1 | 4 |
| BLOND | 1 | 5 | 8 |

JUSTIFICATION: The C allele is enriched in individuals of darker (BLACK or BROW) hair color relative to light. The ratio of CC:CT:TT genotypes in the former group is 5:28:36 but only 1:6:12 in the latter group, which is significantly different.

| 4. GENE | SNPNAME | MARKER | LOCATION | GENBANK | INTEGRITY |
|---|---|---|---|---|---|
| OCA2 | OCA2DBSNP 52401 | 712052 | 52401 | 13651545 | POLY |

| 712052 | OCA2DBSNP_52401 | | |
|---|---|---|---|
| | AA | GA | GG |
| BLACK | 2 | 1 | 1 |
| BROWN | 43 | 24 | 2 |
| AUB/RED | 4 | 3 | 0 |
| BLOND | 8 | 6 | 0 |

JUSTIFICATION: The ratio of GG:GA:AA alleles for dark haired individuals is 3:25:45 and for light haired individuals (BLOND, AUB/RED) is 0:9:12. It appears from this that the G allele is more frequently found in individuals of light hair color.

| 5. GENE | SNPNAME | MARKER | LOCATION | GENBANK | INTEGRITY |
|---|---|---|---|---|---|
| OCA2 | OCA2DBSNP_ 98488 | 712058 | 98488 | 13651545 | POLY |

| 712058 | OCA2DBSNP_98488 | | |
|---|---|---|---|
| | AA | GA | GG |
| BLACK | 0 | 0 | 4 |
| BROWN | 1 | 10 | 38 |
| AUB/RED | 0 | 0 | 6 |
| BLOND | 0 | 4 | 14 |

JUSTIFICATION: The ratio of AA:GA:GG genotypes in dark hair (BLACK + BROWN) individuals is 1:10:42, but 0:4: 20 in lights which is not significantly different. Nonetheless, this SNP is part of the OCA3LOC109 haplotype system which is a reasonable genetic feature for human hair color as described in the text.

| 6. GENE | SNPNAME | MARKER | LOCATION | GENBANK | INTEGRITY |
|---|---|---|---|---|---|
| OCA2 | OCA2DBSNP_ 146405 | 712054 | 146405 | 13651545 | POLY |

| 712054 | OCA2DBSNP_146405 | | |
|---|---|---|---|
| | AA | GA | GG |
| BLACK | 1 | 2 | 1 |
| BROWN | 30 | 28 | 10 |
| AUB/RED | 4 2 | | 0 |
| BLOND | 0 6 | | 6 |

JUSTIFICATION: The ratio of AA:GA:GG genotypes in the dark (BROWN and BLACK) group is 31:30:11 but is 4:8: 6 in the light group and 0:6:6 in the blond group, showing that the G allele is more frequently found in the light hair group.

| 7. GENE | SNPNAME | MARKER | LOCATION | GENBANK | INTEGRITY |
|---|---|---|---|---|---|
| OCA2 | OCA2DBSNP_ 83 | 712057 | 8321 | 13651545 | POLY |

| 712057 | OCA2DBSNP_8321 | | |
|---|---|---|---|
| | GG | GT | TT |
| BLACK | 4 | 0 | 0 |
| BROWN | 45 | 22 | 2 |
| AUB/RED | 6 | 1 | 0 |
| BLOND | 8 | 6 | 0 |

JUSTIFICATION: The GG:GT:TT genotype ratio in the blond group is 8:6:0, but 55:23:2 which is not significantly different. Nonetheless, this SNP is part of a good genetic feature (the OCA3LOC109 haplotype system) for predicting human hair color as described in the text of the application.

| 8. GENE | SNPNAME | MARKER | LOCATION | GENBANK | INTREGRITY |
|---|---|---|---|---|---|
| OCA2 | OCA2E11_263 | 886895 | 26692 | 1365145 | POLY |

| 886895 | OCA2E11_263 | | |
|---|---|---|---|
| | AA | AG | GG |
| BLACK | 5 | 0 | 0 |
| BROWN | 46 | 13 | 2 |
| AUB/RED | 7 | 0 | 0 |
| BLOND | 14 | 5 | 0 |

(continued)

| | AA | AG | GG |
|---|---|---|---|

JUSTIFICATION: The ratio of AA:AG:GG genotypes is not significantly different between the shade of hair color groups, but this SNP is part of the OCA3LOC109 haplotype system, the utility of which was described in the text.

| 9. GENE | SNPNAME | MARKER | LOCATION | GENBANK | INTEGRITY |
|---|---|---|---|---|---|
| OCA2 | OCA2E11_350 | 886896 | 26779 | 1365145 | POLY |

| 886896 | OCA2E11_350. | | |
|---|---|---|---|
| | AA | AG | GG |
| BLACK | 2 | 3 | 0 |
| BROWN | 30 | 26 | 6 |
| AUB/RED | 5 | 1 | 1 |
| BLOND | 12 | 7 | 0 |

JUSTIFICATION: The ratio of AA:AG:GG genotypes is 32:29:6 for dark hair individuals but only 17:8:1 for the light group. The frequency of the G allele is therefore greater in the dark hair group. This SNP is part of the OCA3LOC109 haplotype system, the utility of which was demonstrated in the text.

| 10. GENE | SNPNAME | MARKER | LOCATION | GENBANK | INTEGRITY |
|---|---|---|---|---|---|
| OCA2 | OCA2E14_447 | 886894 | 95957 | 1365145 | POLY |

| 886894 | OCA2E14_447 | | |
|---|---|---|---|
| | CC | CT | TT |
| BLACK | 0 | 3 | 2 |
| BROWN | 3 | 23 | 36 |
| AUB/RED | 1 | 1 | 5 |
| BLOND | 0 | 6 | 13 |

JUSTIFICATION: The ratio of CC:CT:TT genotypes in dark hair individuals (brown and black) is 3:26:38 but only 1: 7:18 in light hair individuals. The frequency of the C allele is therefore greater in the dark hair group (more heterozygotes relative to TT homozygotes).

| 11. GENE | SNPNAME | MARKER | LOCATION | GENBANK | INTEGRITY |
|---|---|---|---|---|---|
| OCA2 | OCA2E10_102 | 886993 | 25083 | 1365145 | POLY |

| 886993 | OCA2E10_102 | | |
|---|---|---|---|
| | AA | AG | GG |
| BLACK | 0 | 2 | 0 |
| BROWN | 1 | 10 | 42 |
| AUB/RED | 0 | 1 | 4 |
| BLOND | 0 | 1 | 14 |

JUSTIFICATON: The ratio of AA:AG:GG genotypes in individuals of dark hair color is 1: 12:42, but only 0:2:18 in persons of light hair color. Therefore the frequency of the A allele is greater in persons of darker hair color.

| 12. GENE | SNPNAME | MARKER | LOCATION | GENBANK | INTEGRITY |
|---|---|---|---|---|---|
| OCA2 | OCA2E10_549 | 886994 | 25519 | 1365145 | POLY |

| 886994 | OCA2E10_549 | | |
|---|---|---|---|
| | CC | CA | AA |
| BLACK | 0 | 2 | 1 |
| BROWN | 1 | 14 | 47 |

(continued)

| | CC | CA | AA |
|---|---|---|---|
| AUB/RED | 0 | 1 | 5 |
| BLOND | 0 | 1 | 16 |

JUSTIFICATION: The ratio of CC:CA:AA genotypes in persons of darker hair color is 1:16:48 but only 0:2:21 in persons of lighter hair color. Therefore, the C allele is more frequently found in persons of darker hair color.

| 13. GENE | SNPNAME | MARKER | LOCATION | GENBANK | INTEGRITY |
|---|---|---|---|---|---|
| TYR | TYR_3 | 217468 | 656 | APO00720 | POLY |

| 217468 | TYR_3 | | |
|---|---|---|---|
| | CC | CA | AA |
| BLACK | 2 | 2 | 0 |
| BROWN | 26 | 35 | 6 |
| AUB/RED | 1 | 4 | 0 |
| BLOND | 3 | 5 | 3 |

JUSTIFICATION: The ratio of CC:CA:AA genotypes is 28:37:6 in persons of darker hair color, but 4:9:3 in persons of lighter hair color. Therefore, the frequency of the A allele is greater in persons of lighter hair color.

| 14. GENE | SNPNAME | MARKER | LOCATION | GENBANK | INTEGRITY |
|---|---|---|---|---|---|
| TYR | TYRSNP_7 | 217472 | 37266 | APOO0720 | POLY |

**[NO TABLE OR JUSTIFICATION]**

| 15. GENE | SNPNAME | MARKER | LOCATION | GENBANK | INTEGRITY |
|---|---|---|---|---|---|
| TYR | TYRSNP_8 | 217473 | 77771 | APO00720 | POLY |

| 217473 | TYRSNP_8 | | |
|---|---|---|---|
| | AA | GA | GG |
| BLACK | 0 | 5 | 2 |
| BROWN | 0 | 47 | 41 |
| AUB/RED | 0 6 4 | | |
| BLOND | 0 | 11 | 14 |

JUSTIFICATION: The frequency of AA:GA:GG genotypes in persons of blond hair color is 0:11:14, but 0:58:47 in persons of dark or red/auburn hair color. Thus, the frequency of the A allele is slightly higher in persons of non-blond hair color.

| 16. GENE | SNPNAME | MARKER | LOCATION | GENBANK | INTEGRITY |
|---|---|---|---|---|---|
| TYR | TYRE3_358 | 951497 | 37434 | APO00720 | POLY |

| 951497 | TYRE3_358 | | |
|---|---|---|---|
| | AA | GA | GG |
| BLACK | 0 | 1 | 4 |
| BROWN | 1 | 8 | 51 |
| AUB/RED | 0 | 1 | 6 |
| BLOND | 1 | 3 | 15 |

JUSTIFICATION: The ratio of AA:GA:GG genotypes in persons of darker hair color (brown and black) is not significantly different from that of light hair color, but this SNP is part of a good haplotype based feature for hair color (the TYR2LOC920 haplotype system described in the text).

| 17. GENE | SNPNAME | MARKER | LOCATION | GENBANK | INTEGRITY |
|---|---|---|---|---|---|
| MCIR | MCIR_4 | 217438 | 442 | X67594 | POLY |
| | 217438 | MC1R_4 | | | |
| | | CC | CT | TT | |
| | BLACK | 3 | 1 | 0 | |
| | BROWN | 64 | 5 | 0 | |
| | AUB/RED | 6 | 0 | 0 | |
| | BLOND | 13 | 1 | 0 | |

JUSTIFICATION: The ratio of CC:CT:TT genotypes in persons of darker hair color is 67:6:0 and 19:1:0 in persons of lighter hair color, which is slightly different. However, this SNP is part of the MCR3LOC105 haplotype system, the utility of which was discussed in the text.

| 18. GENE | SNPNAME | MARKER | LOCATION | GENBANK | INTEGRITY |
|---|---|---|---|---|---|
| MC1R | MC1R_5 | 217439 | 619 | x67594 | POLY |
| | 217439 | MC1R_5 | | | |
| | | CC | CT | TT | |
| | BLACK | 4 | 0 | 0 | |
| | BROWN | 59 | 7 | 0 | |
| | AUB/RED | 5 | 0 | 0 | |
| | BLOND | 10 | 4 | 0 | |

JUSTIFICATION: This SNP is part of the MCR3LOC105 haplotype system, the utility of which was discussed in the text. The frequency of the T allele is higher in individuals of light hair color.

| 19. GENE | SNPNAME | MARKER | LOCATION | GENBANK | INTEGRITY |
|---|---|---|---|---|---|
| MC1R | MC1R_6 | 217440 | 632 | x67594 | POLY |

JUSTIFICATION: This SNP is only found to be a variant in African Americans, and absent in Caucasians, and the former have darker mean hair color than the latter.

| 20. GENE | SNPNAME | MARKER | LOCATION | GENBANK | INTEGRITY |
|---|---|---|---|---|---|
| MC1R | MC1R_7 | 217441 | 646 | X67594 | POLY |
| | 217441 | MC1R_5 | | | |
| | | CC | CT | TT | |
| | BLACK | 4 | 0 | 0 | |
| | BROWN | 53 | 12 | 0 | |
| | AUB/RED | 4 | 3 | 0 | |
| | BLOND | 12 | 2 | 0 | |

JUSTIFICATION: This SNP is part of the MCR3LOC105 haplotype system, the utility of which was described in the text. In particular, the frequency of the T allele is dramatically higher in the RED/AUBURN class than in the others.

| 21. GENE | SNPNAME | MARKER | LOCATION | GENBANK | INTEGRITY |
|---|---|---|---|---|---|
| MC1R | MC1R_14 | NULL | 1048 | X67594 | POLY |

JUSTIFICATION: This SNP is only found to be a variant in African Americans, and absent in Caucasians, and the former have darker mean hair color than the latter.

| 22. GENE | SNPNAME | MARKER | LOCATION | GENBANK | INTEGRITY |
|---|---|---|---|---|---|
| MC1R | MC1R_15 | 217450 | 1272 | X67594 | POLY |

JUSTIFICATION: This SNP is only found to be a variant in African Americans, and absent in Caucasians, and the former have darker mean hair color than the latter.

| 23. GENE | SNPNAME | MARKER | LOCATION | GENBANK | INTEGRITY |
|---|---|---|---|---|---|
| TYRP | TYRP_3 | 217485 | 21693 | AFO01295 | POLY |

| 217485 | TYRP_3 | | |
|---|---|---|---|
| | GG | GT | TT |
| BLACK | 0 | 1 | 2 |
| BROWN | 7 | 18 | 18 |
| AUB/RED | 0 | 2 | 1 |
| BLOND | 2 | 2 | 2 |

JUSTIFICATION: The ratio of GG:GT:TT genotypes is 7:19:20 in persons of darker hair color (brown and black) but 2:4:3 in persons of lighter hair color. The G allele is therefore more frequently found in persons of darker hair color.

| 24. GENE | SNPNAME | MARKER | LOCATION | GENBANK | INTEGRITY |
|---|---|---|---|---|---|
| TYRP | TYRP_4 | 217486 | 21970 | AFO01295 | POLY |

| 217486 | TYRP_4 | | |
|---|---|---|---|
| | AA | AT | TT |
| BLACK | 0 | 2 | 2 |
| BROWN | 6 | 33 | 23 |
| AUB/RED | 0 | 2 | 2 |
| BLOND | 1 | 5 | 2 |

JUSTIFICATION: The ratio of AA:AT:TT genotypes is 6:35:25 in persons of darker hair color (brown and black) but 1:7:4 in person of lighter hair color. Thus, the frequency of the A allele is greater in persons of lighter hair color.

| 25. GENE | SNPNAME | MARKER | LOCATION | GENBANK | INTEGRITY |
|---|---|---|---|---|---|
| TYRP | TYRP1E1E2_357 | 869787 | 6824 | AFO01295 | POLY |

JUSTIFICATION: This SNP is only found to be a variant in African Americans, and absent in Caucasians, and the former have darker mean hair color than the latter.

| 26. GENE | SNPNAME | MARKER | LOCATION | GENBANK | INTEGRITY |
|---|---|---|---|---|---|
| TYRP | TYRPIE1E2-5_38 | 869743 | 5695 | AFO01295 | POLY |

JUSTIFICATION: This SNP is only found to be a variant in African Americans, and absent in Caucasians, and the former have darker mean hair color than the latter.

| 27. GENE | SNPNAME | MARKER | LOCATION | GENBANK | INTEGRITY |
|---|---|---|---|---|---|
| TYRP | TYRPIEIE2-5_307 | 869745 | 5964 | AFO012 | POLY |

JUSTIFICATION: This SNP is only found to be a variant in African Americans, and absent in Caucasians, and the former have darker mean hair color than the latter.

| 28. GENE | SNPNAME | MARKER | LOCATION | GENBANK | INTEGRITY |
|---|---|---|---|---|---|
| TYRP | TYRP1E4_499 | 886937 | 11204 | AF001295 | POLY |

| 886937 | TYRP1E4_499 | | |
|---|---|---|---|
| | GG | GT | TT |
| BLACK | 3 | 2 | 0 |
| BROWN | 56 | 6 | 0 |
| AUB/RED | 7 | 0 | 0 |
| BLOND | 15 | 4 | 0 |

JUSTIFICATION: The ratio of GG:GT:TT genotypes in persons of darker hair color is 59:8:0 but 22:4:0 in lighter hair persons. Though not significantly different, this SNP is part of the TYR3L105 haplotype system, the utility of which was described in the text.

| 29. GENE | SNPNAME | MARKER | LOCATION | GENBANK | INTEGRITY |
|---|---|---|---|---|---|
| TYRP | TYRP1E6_354 | 886938 | 17112 | AFO01295 | POLY |

**[NO TABLE OR JUSTIFICATION]**

| 30. GENE | SNPNAME | MARKER | LOCATION | GENBANK | INTEGRITY |
|---|---|---|---|---|---|
| OCA2 | OCA2DBSNP_ 16501 | 165011 | 13651545 | DBSNP | POLY |

| 712055 | | | |
|---|---|---|---|
| | AA | GA | GG |
| BLACK | 4 | 0 | 0 |
| BROWN | 55 | 11 | 1 |
| AUB/RED | 6 | 1 0 | 0 |
| BLOND | 8 | 4 | 0 |

JUSTIFICATION: The ratio of AA:GA:GG genotypes in persons of darker hair color is 59:11:1 but 14:5:0 in lighter hair persons. The G allele is therefore more frequently found in individuals of light hair.

| 31. GENE | SNPNAME | MARKER | LOCATION | GENBANK | INTEGRITY |
|---|---|---|---|---|---|
| TYR | TYRSNP_16 | 87780 | APO00720 | MULTIPLE | POLY CYS_ TYR |

| 217480 | | | |
|---|---|---|---|
| | GG | GA | AA |
| BLACK | 4 | 1 | 0 |
| BROWN | 60 | 3 | 0 |
| AUB/RED | 6 | 1 | 0 |
| BLOND | 17 | 2 | 0 |

JUSTIFICATION: The ratio of GG:GA:AA genotypes in persons of darker hair color is 64:4:0 but 23:3:0 in lighter hair persons. Though the frequency of the A allele is slightly higher in the light hair group, this SNP is part of the TYR3L105 haplotype system, the utility of which was described in the text.

**MARKER 712060**

| SNPNAME | MARKER | LOCATION | GENBANK | INTEGRITY | SOURCE |
|---|---|---|---|---|---|
| OCA2DBSNP 165011 | 712060 | 165011 | 13651545 | POLY | DBSNP |

(continued)

| **MARKER 712056** | | | | | |
|---|---|---|---|---|---|
| SNPNAME | MARKER | LOCATION | GENBANK | INTEGRITY | SOURCE |
| OCA2DBSNP_252 | 712056 | 252 | 13651545 | POLY | DBSNP |
| **MARKER 217480** | | | | | |
| SNPNAME | MARKER | LOCATION | GENBANK | INTEGRITY | SOURCE |
| TYRSNP_16 | 217480 | 87780 | AP000720 | POLY_FL | DBSNP_SNIPDOC_RESEQ |
| **MARKER 886943** | | | | | |
| SNPNAME | MARKER | LOCATION | GENBANK | INTEGRITY | SOURCE |
| TYRP1E7_420 | 886943 | 20656 | AF001295 | POLY | RESEQ |

**MARKER 710**

DCT1028805 at position 3146161 in public DCT sequence NT 009952

**MARKER 702**

DCT1028806 at position 3146003 in public DCT sequence NT 009952

**MARKER 650**

DCT2892681 at position 3165290 in public DCT sequence NT 009952

**MARKER 675**

DCT2031527 at sequence 3141513 in public DCT sequence NT 009952

| **MARKER 217486** | | | | | |
|---|---|---|---|---|---|
| GENE | MARKER | SNPNAME | LOCATION | GENBANK | SOURCE |
| TYRP | 217486 | TYRP_4 | 21970 | AF001295 | RESEQ |
| **MARKER 886937** | | | | | |
| GENE | MARKER | SNPNAME | LOCATION | GENBANK | SOURCE |
| TYRP | 886937 | TYRP1E4_499 | 11204 | AF001295 | RESEQ |

**Table 18-2. Genetic feature extraction table for variable HAIR color shade.**

| GENE | CANDIDATE SNPs[1] | SNP FEATURES[2] | SNP COMBOS TESTED[3] | # HAPLO TYPE FEATU RES[4] | SELECTED HAPLOTYPE FEATURE NAME, FEATURE Ids[5] | P-value |
|---|---|---|---|---|---|---|
| AP3BI | 6 | | | | | - |
| ASIP | 18 | 2 | 5 | 1 | ASIP-A(5591560) | 0.027 |
| DCT | 20 | 0 | 15 | 0 | N/A MC1R-A | - |
| MCIR | 16 | 3 | 4 | 1 | (217438121743912174 41) | 0.018 |
| OCA2 | 36 | 10 | 152 | 4 | OCA2-A, (712060\|886892\|886896) | 0.012 |
| | | | | | OCA2-B, (217455\|712057\|886894) | 0.022 |
| | | | | | OCA2-C, (217458\|712056) | 0.001 |
| | | | | | OCA2-D, (712054\|886895) | 0.016 |
| SILV | 14 | 0 | 0 | 0 | - | - |
| TYR | 43 | 0 | 20 | 0 | - | - |
| TYRP1 | 28 | 11 | 55 | 1 | TYRP-A, (217486\|886937) | 0.090* |

(continued)

| GENE | CANDIDATE SNPs[1] | SNP FEATURES[2] | SNP COMBOS TESTED[3] | # HAPLO TYPE FEATU RES[4] | SELECTED HAPLOTYPE FEATURE NAME, FEATURE Ids[5] | P-value |
|---|---|---|---|---|---|---|
| TOTAL | 181 | 40 | 233 | 7 | 17 SNPs in 4 genes | |

1. OCA2 gene: Markers 217455, 712057, 712060, 886896, 712054, 712056, 217458, 886895, 886892, and 886894
2. TYR gene: none found.
3. TYRP gene: Markers 217485, 217486, 886943
4. DCT gene: Markers

**Table 18-3**

| GENE | HAPLOID FEATURE | POS. | MARKER | FCA(minor) | TYPE[1] | SOURCE[2] | HISTORY |
|---|---|---|---|---|---|---|---|
| ASIP | ASIP-A | 1 | 559 | 0.02 | exon* | resequencing | None |
| ASIP | ASIP-A | 2 | 560 | 0.12 | exon* | resequencing | None |
| MCIR | MCIR-A | 1 | 217438 | 0.07 | VAL_MET | literature | None |
| MCIR | MCIR-A | 2 | 217439 | 0.07 | ARG_CYS | dbSNP, literature | Hair color[3] |
| MCIR | MCIR-A | 3 | 217441 | 0.07 | ARG_TRP | literature | Hair color[3] |
| OCA2 | OCA2-A | 1 | 712060 | 0.06 | intron | dbSNP | None |
| OCA2 | OCA2-A | 2 | 886892 | 0.03 | intron | dbSNP | None |
| OCA2 | OCA2-A | 3 | 886896 | 0.34 | Intron | resequencing | None |
| OCA2 | OCA2-B | 1 | 217455 | 0.42 | Silent | dbSNP | None |
| OCA2 | OCA2-B | 2 | 712057 | 0.18 | intron | dbSNP | None |
| OCA2 | OCA2-B | 3 | 886894 | 0.32 | Intron | resequencing | None |
| OCA2 | OCA2-C | 1 | 217458 | 0.29 | Silent | dbSNP | None |
| OCA2 | OCA2-C | 2 | 712056 | 0.02 | intron | dbSNP | None |
| OCA2 | OCA2-D | 1 | 712054 | 0.37 | intron | dbSNP | None |
| OCA2 | OCA2-D | 2 | 886895 | 0.13 | Intron | resequencing | None |
| TYRP | TYRP-A | 1 | 217486 | 0.45 | SCR THR | resequencing | None |
| TYRP | TYRP-A | 2 | 886937 | 0.05 | intron | resequencing | None |

1- SILENT - no amino acid change, INTRON- SNP was found in non-coding sequence.
2- DbSNP - candidate gene sequence is present in the NCBI:dbSNP as of 02/15/02. resequencing - SNP sequence was discovered from the re-sequencing effort described in the text.
3- Valverde et al., 1995; Frandberg et al., 1995 and Schioth et al., 1999. coding change status not yet known as of the time of patent submission.

## Claims

1. A method for inferring a genetic pigmentation trait of a human subject from a nucleic acid sample of the subject, the method comprising identifying in the nucleic acid sample at least one pigmentation-related haplotype allele of at least one pigmentation gene, wherein the pigmentation gene is oculocutaneous albinism II (OCA2), tyrosinase-related protein 1(TYRP1), tyrosinase (TYR), adaptor-related protein complex 3, beta 1 subunit (AP3B1), adaptin B1 protein (ADP1), adaptin 3 D subunit 1 (AP3D1), dopachrome tautomerase (DCT), silver homolog (SILV), AIM-1 protein (LOC51151), proopiomelanocortin (POMC), ocular albinism 1(OA1), microphthalmia-associated transcription factor (MITF), myosin VA (MYO5A), RAB27A, or coagulation factor II (thrombin) receptor-like 1 (F2RL1), whereby the haplotype allele is associated with the pigmentation trait, thereby inferring the genetic pigmentation trait of the subject.

2. The method of claim 1, wherein the pigmentation gene includes at least one of OCA2, ASIP, TYRP1, TYR, SILV AP3B1, AP3D1, or DCT, and wherein the pigmentation-related haplotype allele is a penetrant pigmentation-related haplotype allele.

3. The method of claim 2, further comprising identifying in the nucleic acid sample at least one pigmentation-related haplotype allele of at least a second pigmentation gene.

4. The method of claim 3, wherein the at least second pigmentation gene is OCA2, ASIP, TYRP1, TYR, AP3B1, AP3D1, DCT, SILV, LOC51151, POMC, OA1, MITF, MY05A, RAB27A, F2RL1, agouti signaling protein (ASIP), or melanocortin-1 receptor (MC1R).

5. The method of claim 1, wherein the genetic pigmentation trait is hair color, hair shade, eye color, or eye shade.

6. The method of claim 1, wherein the subject is a human, the genetic pigmentation trait is eye color or eye shade and the penetrant pigmentation-related haplotype allele occurs in at least one of the following:

a) nucleotides of the DCT gene corresponding to a DCT-A haplotype, which comprises:

nucleotide 609 of SEQ ID NO:1,
nucleotide 501 of SEQ ID NO:2, and
nucleotide 256 of SEQ ID NO:3;

b) nucleotides of the OCA2 gene, corresponding to an OCA2-A haplotype, which comprises:

nucleotide 135 of SEQ ID NO:7,
nucleotide 193 of SEQ ID NO:8,
nucleotide 228 of SEQ ID NO:9, and
nucleotide 245 of SEQ ID NO: 10;

c) nucleotides of the OCA2 gene, corresponding to an OCA2-B haplotype, which comprises:

nucleotide 189 of SEQ ID NO:11,
nucleotide 573 of SEQ ID NO: 12, and
nucleotide 245 of SEQ ID NO: 13;

d) nucleotides of the OCA2 gene, corresponding to an OCA2-C haplotype, which comprises:

nucleotide 643 of SEQ ID NO: 14,
nucleotide 539 of SEQ ID NO: 15,
nucleotide 418 of SEQ ID NO: 16, and
nucleotide 795 of SEQ ID NO: 17,

e) nucleotides of the OCA2 gene, corresponding to an OCA2-D haplotype, which comprises:

nucleotide 535 of SEQ ID NO: 18,
nucleotide 554 of SEQ ID NO: 19, and

nucleotide 210 of SEQ ID NO:20;

f) nucleotides of the OCA2 gene, corresponding to an OCA2-E haplotype, which comprises:

nucleotide 225 of SEQ ID NO:21,
nucleotide 170 of SEQ ID NO:22, and
nucleotide 210 of SEQ ID NO:20; or

g) nucleotides of the TYRP 1 gene corresponding to a TYRP1-B haplotype which comprises:

nucleotide 172 of SEQ ID NO:23, and
nucleotide 216 of SEQ ID NO:24;
or any combination of a) through g).

**7.** The method of claim 4, wherein the subject is a human, the genetic pigmentation trait is eye color or eye shade and the penetrant pigmentation-related haplotype allele occurs in at least one of the following:

a) nucleotides of the DCT gene corresponding to a DCT-A haplotype, which comprises:

nucleotide 609 of SEQ ID NO:1,
nucleotide 501 of SEQ ID NO:2, and
nucleotide 256 of SEQ ID NO:3;

b) nucleotides of the MC1R gene corresponding to a melanocortin-1 receptor (MC1R)-A haplotype, which comprises:

nucleotide 442 of SEQ ID NO:4,
nucleotide 619 of SEQ ID NO: 5, and
nucleotide 646 of SEQ ID NO:6;

c) nucleotides of the OCA2 gene, corresponding to an OCA2-A haplotype, which comprises:

nucleotide 135 of SEQ ID NO:7,
nucleotide 193 of SEQ ID NO:8,
nucleotide 228 of SEQ ID NO:9, and
nucleotide 245 of SEQ ID NO: 10;

d) nucleotides of the OCA2 gene, corresponding to an OCA2-B haplotype, which comprises:

nucleotide 189 of SEQ ID NO:11,
nucleotide 573 of SEQ ID NO: 12, and
nucleotide 245 of SEQ ID NO: 13;

e) nucleotides of the OCA2 gene, corresponding to an OCA2-C haplotype, which comprises:

nucleotide 643 of SEQ ID NO: 14,
nucleotide 539 of SEQ ID NO: 15,
nucleotide 418 of SEQ ID NO:16, and
nucleotide 795 of SEQ ID NO:17,

f) nucleotides of the OCA2 gene, corresponding to an OCA2-D haplotype, which comprises:

nucleotide 535 of SEQ ID NO: 18,
nucleotide 554 of SEQ ID NO: 19, and
nucleotide 210 of SEQ ID NO:20;

g) nucleotides of the OCA2 gene, corresponding to an OCA2-E haplotype, which comprises:

nucleotide 225 of SEQ ID NO:21,
nucleotide 170 of SEQ ID NO:22, and
nucleotide 210 of SEQ ID NO:20; or

h) nucleotides of the TYRP1 gene corresponding to a TYRP1-B haplotype which comprises:

nucleotide 172 of SEQ ID NO:23, and
nucleotide 216 of SEQ ID NO:24;
or any combination of a) through h).

**8.** The method of claim 7, further comprising identifying in the nucleic acid sample at least one nucleotide occurrence of a latent pigmentation-related SNP of a pigmentation gene, wherein the latent pigmentation-related SNP is nucleotide 61 of SEQ ID NO:25, nucleotide 201 of SEQ ID NO:26, nucleotide 201 of SEQ ID NO:27, nucleotide 201 of SEQ ID NO:28, nucleotide 657 of SEQ ID NO:29, nucleotide 599 of SEQ ID NO:30, nucleotide 267 of SEQ ID NO: 31, nucleotide 61 of SEQ ID NO:32, nucleotide 451 of SEQ ID NO:33; nucleotide 326 of SEQ ID NO:34, nucleotide 61 of SEQ ID NO:35, nucleotide 61 of SEQ ID NO:36, nucleotide 61 of SEQ ID NO:37, nucleotide 93 of SEQ ID NO:38, nucleotide 114 of SEQ ID NO:39, nucleotide 558 of SEQ ID NO:40, nucleotide 221 of SEQ ID NO:41, nucleotide 660 of SEQ ID NO:42, nucleotide 163 of SEQ ID NO:43, nucleotide 364 of SEQ ID NO:44, nucleotide 473 of SEQ ID NO:45, nucleotide 314 of SEQ ID NO:46, nucleotide 224 of SEQ ID NO:47, nucleotide 169 of SEQ ID NO:48, nucleotide 214 of SEQ ID NO:49, or nucleotide 903 of SEQ ID NO:50; or any combination thereof.

**9.** The method of claim 7, further comprising identifying in the nucleic acid sample at least one latent pigmentation-related haplotype allele of a pigmentation gene, wherein the latent pigmentation-related haplotype allele is:

i) nucleotides of the ASIP gene corresponding to anASIP-A haplotype, which comprises:

nucleotide 201 of SEQ ID NO:26, and
nucleotide 201 of SEQ ID NO: 28;

j) nucleotides of the DCT gene corresponding to a DCT-B haplotype, which comprises:

nucleotide 451 of SEQ ID NO : 33, and
nucleotide 657 of SEQ ID NO : 29;

k) nucleotides of the SILV gene corresponding to a SILV-A haplotype, which comprises:

nucleotide 61 of SEQ ID NO : 35, and
nucleotide 61 of SEQ ID NO : 36;

l) nucleotides of the TYR gene corresponding to a TYR-A haplotype, which comprises:

nucleotide 93 of SEQ ID NO : 38, and
nucleotide 114 of SEQ ID NO : 39; or

m) nucleotides of the TYRP1 gene corresponding to a TYRP1-A haplotype, which comprises:

nucleotide 364 of SEQ ID NO : 44,
nucleotide 169 of SEQ ID NO : 48, and
nucleotide 214 of SEQ ID NO : 49, or any combination of i) through m).

**10.** The method of claim 7, wherein the pigmentation-related haplotype allele of MC1R-A is CCC.

**11.** The method of claim 7, wherein the pigmentation-related haplotype allele of OCA2-A is TTA, CCAG, or TTAG.

**12.** The method of claim 7, wherein the pigmentation-related haplotype allele of OCA2-B is CAA, CGA, CAC, or CGC.

**13.** The method of claim 7, wherein the pigmentation-related haplotype allele of OCA2-C is GGAA, TGAA, or TAAA

**14.** The method of claim 7, wherein the pigmentation-related haplotype allele of OCA2-D is AGG or GGG.

**15.** The method of claim 7, wherein the pigmentation-related haplotype allele of OCA2-E is GCA.

**16.** The method of claim 7, wherein the pigmentation-related haplotype allele of TYRP1-B is TC.

**17.** The method of claim 7, wherein the pigmentation-related haplotype allele of DCT-A is CTG or GTG.

**18.** The method of claim 9, wherein the pigmentation-related haplotype allele of ASIP-A is GT or AT.

**19.** The method of claim 9, wherein the pigmentation-related haplotype allele of DCT-B is TA or TG.

**20.** The method of claim 19, wherein the pigmentation-related haplotype allele of SILV-A is TC, TT, or CC.

**21.** The method of claim 9, wherein the pigmentation-related haplotype allele of TYR-A is GA, AA or GG.

**22.** The method of claim 9, wherein the pigmentation-related haplotype allele of TYRP1-B is GTG, TTG, or GTT.

**23.** The method of claim 7, wherein the at least one penetrant pigmentation-related haplotype allele identified comprises the MC1R-A haplotype, the OCA2-A haplotype, the OCA2-B haplotype, the OCA2-C haplotype, the OCA2-D haplotype, the OCA2-E haplotype, the TYRP1-B haplotype, and the DCT-B haplotype.

**24.** The method of claim 23, wherein the subject is a Caucasian, the genetic pigmentation trait is eye shade or eye color, and the penetrant pigmentation-related haplotype allele is:

a) the MC1R-A haplotype allele CCC;
b) the OCA2-A haplotype allele TTAA, CCAG, or TTAG;
c) the OCA2-B haplotype allele CAA, CGA, CAC, or CGC;
d) the OCA2-C haplotype allele GGAA, TGAA, or TAAA ;
e) the OCA2-D haplotype allele AGG or GGG;
f) the OCA2-E haplotype allele GCA;
g) the TYRP1-B haplotype allele TC; and
h) the DCT-B haplotype allele CTG, or GTG.

**25.** The method of claim 7, further comprising applying the pigment-related haplotype alleles to a matrix or contingency table created using a feature modeling algorithm.

**26.** The method of claim 25, wherein the feature modeling algorithm is a quadratic classifier, performs correspondence analysis, or is a quadratic classifier and performs correspondence analysis.

**27.** The method of claim 1, wherein the identifying is performed using an amplification reaction.

**28.** The method of claim 1, wherein the identifying is performed using a primer extension reaction.

**29.** The method of claim 1, wherein the identifying is performed using an immunoassay.

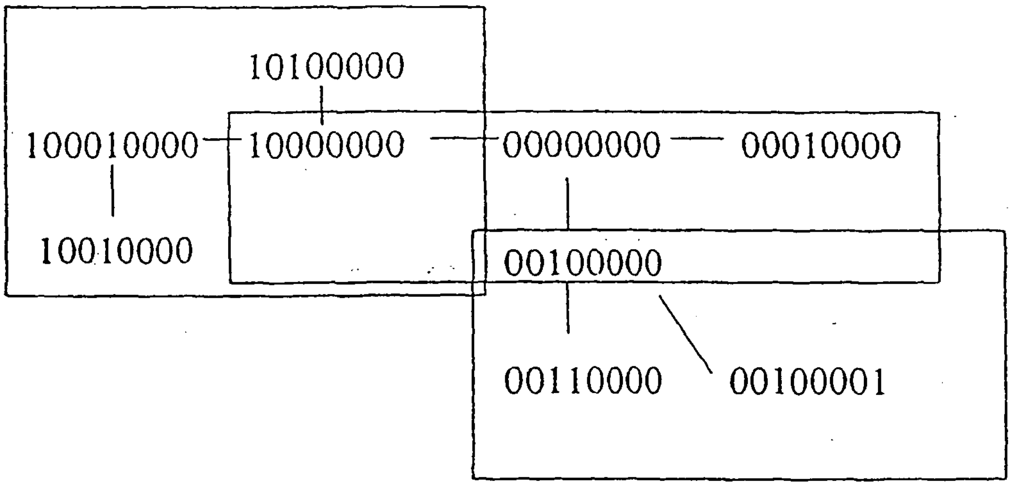
10100000
100010000
10000000
00000000
00010000
10010000
00100000
00110000
00100001

Fig. 1

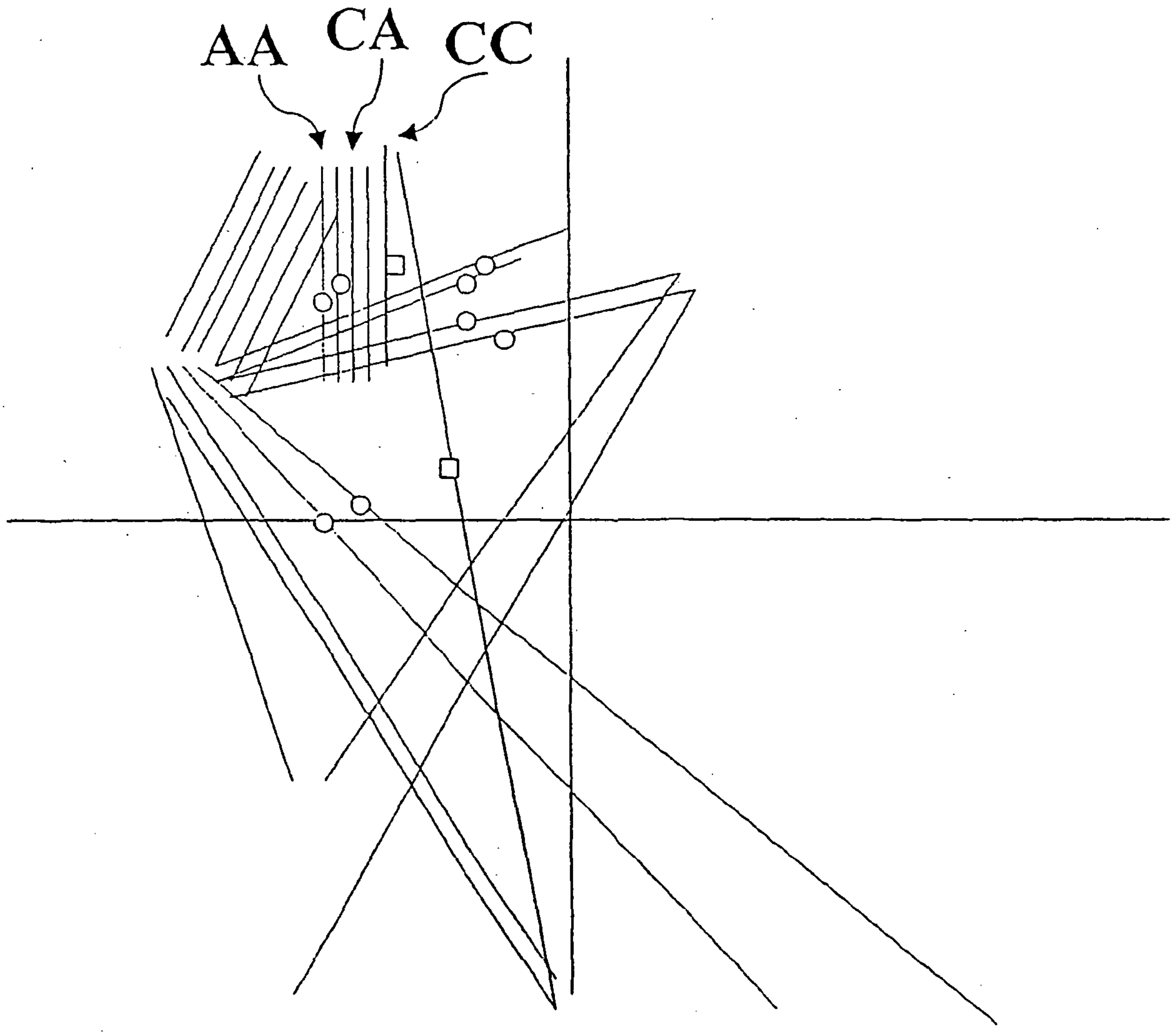


Fig. 2

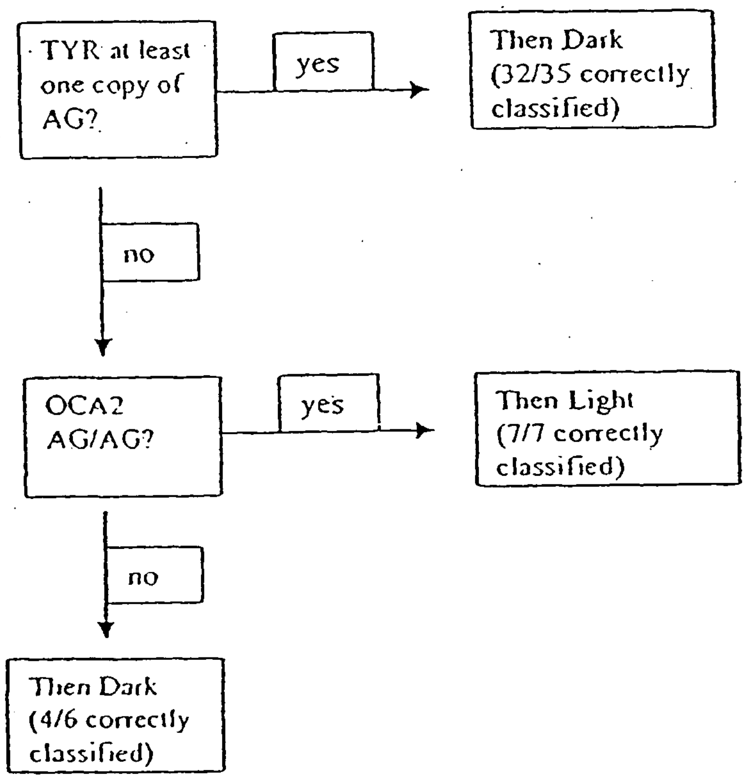
TYR at least one copy of AG?
yes
Then Dark (32/35 correctly classified)
no
OCA2 AG/AG?
yes
Then Light (7/7 correctly classified)
no
Then Dark (4/6 correctly classified)

Fig. 3

```
      Clade: II-1                          Clade: II-2
------------------------------    ----------------------------------------

 {H2}   ← 2 →  {H3}   ← 1 →  {H5}  ← 2 →  {H7}   ← 3 →   {H6}
 {ATG} T←→C {ACG}   A←→G  {GCG} C←→T {GTG} G←→A {GTA}
   /     G                      /  G
 {H1}←3 →                   {H4}←3→
{ATA} A                    {GCA} A
```

Fig. 4

Clade: II-1 Clade: II-2

{H1} ← 2 → {H2} ← 1 → {H3}
{CAC} A←→G {CGC} C←→T {TGC}
/ C
{H4}
{CGT} T

Fig. 5

```
2-step: Clade-1                                    Clade-2
________________________________           ______________________________
                                                      /<-- 1 --> {H4}TGC
{H5}<-- 1 --> {H1}<-- 3 -->  {H2}<-- 2 -->     {H3}   C<-->T
TAT  T<-->C  CAT  T<-->C   CAC  A<-->G      CGC
                                                      \<-- 3 --> {H6}CGT
```

Fig. 6

```
2-step: Clade-1                                     Clade-2
------------------------------------     ------------------------------------------
 {H5}<-- 3 -->  {H8}<-- 2 --> {H1}<-- 3 -->  {H3} <-- 1 -->{H4} <-- 2 --> {H2}
AAC  C<-->T  AAT  A<-->G  AGT  T<-->C  AGC  A<-->G  GGC  G<-->A  GAC
                           /                                      /
                {H7} <-- 1 -->/                       {H6} <-- 3 -->/
                GGT  G<-->A                           GAT  T<-->C]
```

Fig. 7

```
        Clade: II-1                         Clade: II-2
______________________________      ______________________________
{H5}← 3 → {H8} ← 2 → {H1} ← 3 →{H3} ← 1 → {H4}← 2 → {H2}
AAC C←→T AAT A←→G AGT T←→C AGC A←→G GGC G←→AGAC
                    /                                   /
          {H7}← 1 →/                          {H6}← 3→/
          GGT G← →A                           GAT T← →C
______________________________      ______________________________
```

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 120804 A
- US 6294336 B
- WO 9215712 A, Goelet, P.
- WO 8910414 A, Wallace
- US 4656127 A, Mundy, C.R.
- FR 2650840, Cohen, D.
- WO 9102087 A
- US 5002867 A
- US 6391589 B
- US 96405901 A
- US 60377164 B
- WO 60338734 A, Frudakis


### Non-patent literature cited in the description


- **STEPHENS ; DONNELLY.** *Am. J. Hum. Genet.,* 2001, vol. 68, 978-989
- **STEPHENS ; DONNELLY.** *Am, J. Hum. Genet.,* 2001, vol. 68, 978-989
- **EXCOFFIER ; SLATKIN.** *Mol Biol Evol.,* September 1995, vol. 12 (5), 921-7
- **CUMMING et al.** *Am. J. Opthalmol.,* 2000, vol. 130, 237-238
- **BROGELLI et al.** *Br. J. Dermatol.,* 1991, vol. 125, 349-52
- **PALMER et al.** *Am. J. Hum. Genet.,* 2000, vol. 66, 176-86
- **RIEDER et al.** *Mamm Genome.,* 2001, vol. 12 (6), 450-5
- **OETTING ; KING.** *Hum. Mutat.,* 1999, vol. 13, 99-115
- **AKEY et al.** *Hum. Genet.,* 2001, vol. 103, 516-520
- **BRAUER ; CHOPRA.** *Anthropol. Anz.,* 1978, vol. 36 (2), 109-120
- **BITO et al.** *Arch Ophthalmol.,* 1997, vol. 115 (5), 659-663
- **STURM et al.** *Gene,* 2001, vol. 277, 49-62
- **BOX et al.** *Hum. Mole. Genet.,* 1997, vol. 6, 1891-1897
- **BOX et al.** *Am. J. Hum. Genet.,* 2001, vol. 69, 765-773
- **IMESCH et al.** *Surv. Ophthalmol.,* 1997, vol. 41, S117-S123
- **BRUES.** *Am. J. Phys. Anthropol.,* 1975, vol. 43 (3), 387-391
- **OETTING ; KING.** *Hum. Mutat.,* 1993, vol. 2, 1-6
- **OETTING ; KING.** *Hum. Genet.,* 1992, vol. 90, 258-262
- **OETTING ; KING.** *Clin. Res.,* 1991, vol. 39, 267A
- **LINDSEY et al.** *Arch. Opthalmol.,* 2001, vol. 119 (6), 853-860
- **LEE et al.** *Hum. Molec. Genet.,* 1994, vol. 3, 2047-2051
- **SCHMIDT ; BEERMANN.** *Proc. Natl. Acad. Sci.,* 1994, vol. 91 (11), 4756-4760
- **DURHAM-PIERRE et al.** *Nature Genet.,* 1994, vol. 7, 176-179
- **DURHAM-PIERRE et al.** *Hum. Mutat.,* 1996, vol. 7, 370-373
- **GARDNER et al.** *Science,* 1992, vol. 257, 1121-1124
- **HAMABE et al.** *Am. J. Med. Genet.,* 1991, vol. 4.1, 54-63
- **CHINTAMANENI et al.** *Biochem. Biophys. Res. Commun.,* 1991, vol. 178, 227-235
- **ABBOTT et al.** *Genomics,* 1991, vol. 11, 471-473
- **BOISSY et al.** *Am J. Hum. Genet.,* 1996, vol. 58, 1145-1156
- **ROBBINS et al.** *Cell,* 1993, vol. 72, 827-834
- **SMITH et al.** *J. Invest. Derm.,* 1998, vol. 111, 119-122
- **FLANAGAN et al.** *Hum. Molec. Genet.,* 2000, vol. 9, 2531-2537
- **OOI et al.** *EMBO J.,* 1997, vol. 16 (15), 4508-451
- **OOI.** *EMBOJ.,* 1997, vol. 16 (15), 4548-4518
- **LLOYD et al.** *Trends Cell Biol.,* 1998, vol. 8 (7), 257-259
- **STURM.** *Gene,* 2001, vol. 277, 49-62
- **EIBERG ; MOHR.** *Eur. J. Hum. Genet,* 1996, vol. 4 (4), 237-241
- **VALVERDE et al.** *Nature Genet.,* 1995, vol. 11, 328-330
- **KOPPULA et al.** *Hum. Mutat.,* 1997, vol. 9, 30-36

- **KANETSKY et al.** *Am J. Hum. Gen.,* 2002, vol. 70, 770-775
- **SPRITZ.** *Nature Cenet.,* 1995, vol. 11, 225-226
- **RAYMOND et al.** *GenePop. Ver 3.0.,* 1994
- **SAMBROOK et al.** Molecular Cloning: A laboratory manual. Cold Spring Harbor Laboratory Press, 1989
- **LIN et al.** *Nucl. Acids Res.,* 1994, vol. 22, 5220-5234
- **JELLINEK et al.** *Biochemistry,* 1995, vol. 34, 11363-11372
- **PAGRATIS et al.** *Nature Biotechnol.,* 1997, vol. 15, 68-73
- **TAM et al.** *Nucl. Acids Res.,* 1994, vol. 22, 977-986
- **ECKER ; CROOKE.** *BioTechnology,* 1995, vol. 13, 351360
- **SANGER, F. et al.** *J. Molec. Biol.,* 1975, vol. 94, 441
- **PROBER et al.** *Science,* 1987, vol. 238, 336-340
- **MAXAM, A. M. et al.** *Proc. Natl. Acad. Sci.,* 1977, vol. 74, 564
- **KOMHER, J. S. et al.** *Nucl. Acids. Res.,* 1989, vol. 17, 7779-7784
- **SOKOLOV, B. P.** *Nucl. Acids Res.,* 1990, vol. 18, 3671
- **SYVANEN, A. -C. et al.** *Genomics,* 1990, vol. 8, 684-692
- **KUPPUSWAMY, M. N. et al.** *Proc. Natl. Acad. Sci.,* 1991, vol. 88, 1143-1147
- **PREZANT, T. R. et al.** *Hum. Mutat.,* 1992, vol. 1, 159-164
- **UGOZZOLI, L. et al.** *GATA,* 1992, vol. 9, 107-112
- **NYREN, P. et al.** *Anal. Biochem.,* 1993, vol. 208, 171-175
- **SYVANEN, A. -C. et al.** *Amer. J. Hum. Genet.,* 1993, vol. 52, 46-59
- **HARLOW et al.** Antibodies: A Laboratory Manual. Cold Spring Harbor Laboratory Press, 1988
- **HAMMERLING.** Monoclonal Antibodies and T-Cell Hybridomas. Elsevier, 1981, 563-681
- **KENETSKY et al.** *Am. J. Hum. Genet.,* 2002, vol. 70, 770
- **BUDOWLE et al.** *J. Forensic Sci.,* 2001, vol. 46 (3), 453-489
- **LEVADOKOU et al.** *J. Forensic Sci.,* 2001, vol. 46 (3), 736-761
- **BUDOWLE et al.** *Clin. Chim. Acta,* 1994, vol. 228 (1), 3-18
- **KERSTING et al.** *Croat Med. J.,* 2001, vol. 42 (3), 310-314
- **MEYER et al.** *Int. Int. J. Legal Med.,* 1995, vol. 107 (6), 314-322
- **MONSON et al.** *J. Forensic Sci.,* 1998, vol. 43 (3), 483-488
- **BRENNER.** *Am. J. Hum. Genet.,* 1998, vol. 62 (6), 1558-1560
- **LOWE et al.** *Forensic Sci. Int.,* 2001, vol. 119 (1), 17-22
- **BRENNER.** *Proceedings 7th Intl. Symposium on Hum. Identification,* 1997, 4892
- **SHRIVER et al.** *Am. J. Hum. Genet.,* 1997, vol. 60, 957-964
- **GOODMAN.** *Am. J. Public Health,* 2000, vol. 90 (11), 1699-1702
- **ERICKSON ; SVENSMARK.** *Int. J. Legal Med.,* 1994, vol. 106, 254-257
- **EVETT et al.** *J. Forensic Sci. Soc.,* 1992, vol. 32, 301-306
- **LEO BRIEMAN ; CHARLES J. STONE ; RICHARD A. ; OLSHEN JEROME ; H. FRIEDMAN.** Classification and Regression Trees. Wadsworth International Group, 1984
- **LOH ; SHIH.** *QUEST,* 1997
- **BREIMAN.** *C&RT,* 1954
- **LOH ; VANICHESTALCUL.** *FACT,* 1988
- **MORGAN ; MESSENGER.** *THAID,* 1973
- **FISHER, R.A.** *Annals of Eugenics,* 1936, vol. 7, 179-188
- **SMITH, C.A.B. et al.** *Annals of Eugenics,* 1948, vol. 13, 272-282
- **KISHINO ; WADDEL.** *Genome Informatics,* 2000, vol. 11, 83-95
- **BENZECRI.** Correspondence Analysis Handbook. Dekker, 1992
- L'Analyse des Correspondence. **BENZECRI.** L'Analyse des donnees. Dunod, 1973, vol. 2
- **GREENACRE.** Theory and Application of Correspondence Analyses. Academic Press, 1984
- **ERIKSON ; SVENSMARK.** *Int. J. Legal Med.,* 1994, vol. 106, 254-257
- **BALAKRISHNAN et al.** *Handbook of Statistics,* 1991, vol. 8, 145-202
- **ANDERSON, T.W.** Introductin to Multivariate Statistical Analysis. Wiley, 1958
- **SRIVASTAVA et al.** *Mykosen,* September 1979, vol. 22 (9), 311-3
- **SRIVASTAVA, M.S.** An introduction to multivariate statistics. 1979
- **SMOUSE, P.E. et al.** *Genetics,* 1977, vol. 85, 733-752
- **SPIELMAN, R.S. et al.** *Am. J Hum Genet.,* 1976, vol. 28, 317-331
- **RAO.** *Nature,* 1947, vol. 159, 30-31
- **RAO, C.R.** *Nature,* 1948, vol. 160, 835-836
- **RAO, C.R.** *JRSS(B,* vol. 10, 159-203
- **VALVERDE, P. et al.** *Nature Genet.,* 1995, vol. 11, 328-330
- **CULVERHOUSE et al.** *Am. J. Hum. Genet.,* 2002, vol. 70, 461-471
- **OOI et al.** *EMBO J.,* 1997, vol. 16 (15), 4508-4518

- **IOZUMI et al.** *J. Invest. Dermatol.,* 1993, vol. 100, 806-811
- **ANCANS et al.** *J. Invest. Dermatol.,* 2001, vol. 117, 158-159
- **FULLER et al.** *Exp. Cell. Res.,* 2001, vol. 262, 97-208
- **ANCANS et al.** *Exp. Cell. Res.,* 2001, vol. 268, 26-35
- **VALVERDE et al.** *Nature Genet.,* 1995, vol. 11, 325-330
- **FRANDBERG et al.** *Biochem. Biophys. Res. Commun.,* 1998, vol. 245, 490-492

SEQUENCE LISTING

<110> DNAPrint Genomics, Inc.
FRUDAKIS, Tony N.

<120> COMPOSITIONS AND METHODS FOR THE INFERENCE OF PIGMENTATION TRAITS

<130> DNA1140-7EP

<140> EP 02739467.5
<141> 2002-05-28

<150> PCT/US 02/16789
<151> 2002-05-28

<150> US 60/346,303
<151> 2002-01-02

<150> US 60/334,674
<151> 2001-11-15

<150> US 60/344,418
<151> 2001-10-26

<150> US 60/323,662
<151> 2001-09-17

<150> US 60/310,781
<151> 2001-08-07

<150> US 60/300,187
<151> 2001-06-21

<150> US 60/293,560
<151> 2001-05-25

<160> 224

<170> PatentIn version 3.1

<210> 1
<211> 1292
<212> DNA
<213> Homo sapiens 702

<220>
<221> misc_feature
<222> (609)..(609)
<223> n = a or g

<400> 1
tctctttcca gacacaacaa atggtaccgg tgccaggtaa caaatgcagg tccttgatgt 60

gagaaatcta tggccctgta ggggcgtcct ggtcctgaaa caattgggaa acatattaga 120

gatgacagaa tagaattttt taaaatgtta aatcttacct gggctgtatc tatagagcca 180

ataagtccat gatgttttat agtagtaaga aaaaaaagtt ttgttgttat ttatatctcc 240

aaaagcaata gtaatgacat gaatttcaga agtaactagt acttttagga taaaccctcc 300

tttatctctt aaaaggttga tttctagcag agatccagga aattcaggtg actttcagga 360

ttgggagccc ctgatacata tttaatttgt aagcaacaat ccattcaggt gaaatagact 420

atccaaaaga atagatcatt aagacctaaa ggaaatatat tttttaaagg catttgctaa 480

cctatgcttc acaaagatat aaatttaaaa gggagggatt aattaataat attgtaactt 540

caactgcact tagtcctaat gcagtattta tgtaaccaaa aagaactttc gcgtattttg 600

cctcacccnt caatctgtta taactactag ctgacaagca tgttcgctga gaccagaaga 660

gagaaaaata cattcaagcc atttaggctg gacactggaa ctgccaattt tagtgaaaag 720

ctctggacga tttaatgacc tccttactgc ctggtccatc cttcttccct ttttcataac 780

agctttgatg gttatgtgga aactttagct aactttagtt cttgtccaaa atacctataa 840

caacttcaga gagctgaaca gacgtgaata taaatttaat tcgtagctat gaccagaaac 900

taggactttc ttttcctctt gagagagtac tctctcataa agcaataatt cctcagagtg 960

ataacaggaa aactataaag gtttatagcc aaaagagcct ccttatctat cgataaagac 1020

agacagatgc ttacctgtaa ggcaagtttt aaaagactca cactgagaaa agcactgctg 1080

tccctgatgt tggctacacg gcccttttc atgttcaatt cagagcaagg tttcaaattc 1140

tcagaaccca acaacgagag ggtatcaatt gaaggggaac aagtgatgag cagaaggatg 1200

aacctgtcca aatcagaatc ttgctcttca aagtgagctt gatgtcttca ttcatcctga 1260

ccccaatgaa ggaccccaaa aatggggaga gg 1292

<210> 2
<211> 1001
<212> DNA
<213> Homo sapiens 650

<220>
<221> misc_feature
<222> (501)..(501)
<223> n = c or g

<400> 2
gggtttgcac tcttatgaga atctaatgct gctgatctga caggaggcgg agctcaagtg 60

ataatgctca attgcttacc atccacctgc tgtgtggccc agttcctaag aggccacaga 120

ccagcatggg cccatggccc aggggttggg gacccctgct ttacaccacc acactgtagt 180

acaaccttca ggaccaacta gaataagaaa aattgatttt gaaggcatat tctccaatct 240

taacaatgca gaaagaggat actttgacct atgagtccta aaccaatcat gaacttttag 300

atactgtctg agttcaagtc agggttcaaa ttgtgatgac agaaagagac aagagactat 360

caggacacaa ggaagatgca gaatcagtgc tgtaagaggc aaataactta tagaaagtat 420

cagaattcac caagggaaaa atgtaaataa taaaggagaa ggcaagatcc taaggcaatt 480

agtaatctgg agagataaaa naatagggga aaataataag gaggaattga aattgtagct 540

ctcaggaggg ctataaaaca tacaataaat gctctagtaa ggctgcacac agtggctcat 600

gcctgtaatc ccagcacttt gggaagctga ggcaagagga tcacatgagc ccaggagttc 660

gagaccagcc ccagcaacag agtgagaccc catttctaca aaaatttaaa aattagctag 720

gtgtgatagt gtgtgcctgt ggtcccagct acttgaggct gagatgcgag gatcatttga 780

gccctggaag tcaaggctgc agtgagctat gattgtgcca ctgcactcca gcctgagtga 840

cagagcgaga tactgtctca acaacaacaa caacaacaac aacaaagctc tagtaagaac 900

tatatacaga gtacccaaat tatagaatgt aagttttttt gaaaagactg gtacatgctg 960

agaaaatgca aagcatccaa tgaacaaaat gctacaagca a 1001

<210> 3
<211> 630
<212> DNA
<213> Homo sapiens 675

<220>
<221> misc_feature
<222> (256)..(256)
<223> n = c or t

<400> 3
ttctactctt cccaagacta acaatgtttt tgaatagttt aagttaaaca ttaaaatgta 60

cattgattcc tggagataat taaatacaaa ctatttattt aacaaatatt tatgaagcgg 120

ctactatctc atcagatgct atgcaagatg cttcgtttta tggaccttga agtctatttt 180

ccctggggca accttactaa aactgtatgt attaaaaaca gatttgatta aaacatatca 240

cctactatga cagtanagga atggaacact gttttgagaa ctgagtgaac aaaatgctgt 300

gtctaaagaa gagtagctac ccagattaaa attcagtcaa atttccagct gcctatctat 360

agctgattgg aggtaaatgt ctagtcactt ctctgcagta gaagagtaat tccttgcttc 420

ataatgctga gaaatttgga tgataataat gtcggtagag gttcattgat tgtaaccctg 480

gtttgcggat gttgctagtg gaagattgca gagtgtgggg gagaaggtag atgggaattt 540

tttgtacttt ctccccaatt tcaatgtgaa tccaaaactg atctaaaata ataaagtctc 600

aatatttaaa aatattaata aataacacac 630

<210> 4
<211> 1270
<212> DNA
<213> Homo sapiens 217438

<220>
<221> misc_feature
<222> (442)..(442)
<223> n = g or a

<400> 4
ggagagggtg tgagggcaga tctgggggtg cccagatgga aggaggcagg catgggggac 60

acccaaggcc ccctggcagc accatgaact aagcaggaca cctggagggg aagaactgtg 120

gggacctgga ggcctccaac gactccttcc tgcttcctgg acaggactat ggctgtgcag 180

ggatcccaga gaagacttct gggctccctc aactccaccc ccacagccat cccccagctg 240

gggctggctg ccaaccagac aggagcccgg tgcctggagg tgtccatctc tgacgggctc 300

ttcctcagcc tggggctggt gagcttggtg gagaacgcgc tgktggtggc caccatcgcc 360

aagaaccrga acctgcactc acccatgtac tgcttcatct gctgcctggc cttgtcggas 420

ctgctggtga gcgggassaa cntgctggag acggccgtca tcctcctgct ggaggccggt 480

gcactggtgg cccgggctgc ggtgctgcag cagctggaca atgtcattga cgtgatcacc 540

```
tgcagctcca tgctgtccag cctctgcttc ctgggcgcca tcgccgtgga ccgctacatc     600
tccatcttct acgcactgyg ctaccacagc aycgtgaccc tgccgygggc gcsgcrassc     660
gttgcggcca tctgggtggc cagtgtcgtc ttcagcacgc tcttcatcgs ctactacgac     720
cacgtggccg tcctgctgtg cstcgtggtc ttcttcctgg ctatgctggt gctcatggcc     780
gtgctgkacg tccacatgct ggcccgggcc tgccagcacg cccagggcat cgcccggctc     840
cacaagaggc agcgcccggt ccaccagggc tttggcctta aaggcgctgt caccctcacc     900
atcctgctgg gcattttctt cctctgctgg ggccccttct tcctgcatct cacactcatc     960
gtcctctgcc ccgagcaccc cacgtgcggc tgcatcttca agaacttcaa cctctttctc    1020
gccctcatca tctgcaatgc catcatcsac cccctcatct acgccttcca cagccaggag    1080
ctccgcagga cgctcaagga ggtgctgaca tgctcctggt gagcgcggtg cacgcgcttt    1140
aagtgtgctg ggcagaggga ggtggtgata ttgtgtggtc tggttcctgt gtgaccctgg    1200
gcagttcctt acctccctgg tccccgtttg tcaaagagga tggactaaat gatctctgaa    1260
agtgttgaag                                                           1270
```

```
<210>  5
<211>  1270
<212>  DNA
<213>  Homo sapiens 217439

<220>
<221>  misc_feature
<222>  (619)..(619)
<223>  n = t or c

<400>  5
ggagagggtg tgagggcaga tctgggggtg cccagatgga aggaggcagg catgggggac      60
acccaaggcc ccctggcagc accatgaact aagcaggaca cctggagggg aagaactgtg     120
gggacctgga ggcctccaac gactccttcc tgcttcctgg acaggactat ggctgtgcag     180
ggatcccaga gaagacttct gggctccctc aactccaccc ccacagccat cccccagctg     240
gggctggctg ccaaccagac aggagcccgg tgcctggagg tgtccatctc tgacgggctc     300
ttcctcagcc tggggctggt gagcttggtg gagaacgcgc tgktggtggc caccatcgcc     360
aagaaccrga acctgcactc acccatgtac tgcttcatct gctgcctggc cttgtcggas     420
ctgctggtga gcgggassaa crtgctggag acggccgtca tcctcctgct ggaggccggt     480
gcactggtgg cccgggctgc ggtgctgcag cagctggaca atgtcattga cgtgatcacc     540
tgcagctcca tgctgtccag cctctgcttc ctgggcgcca tcgccgtgga ccgctacatc     600
tccatcttct acgcactgng ctaccacagc aycgtgaccc tgccgygggc gcsgcrassc     660
gttgcggcca tctgggtggc cagtgtcgtc ttcagcacgc tcttcatcgs ctactacgac     720
cacgtggccg tcctgctgtg cstcgtggtc ttcttcctgg ctatgctggt gctcatggcc     780
gtgctgkacg tccacatgct ggcccgggcc tgccagcacg cccagggcat cgcccggctc     840
cacaagaggc agcgcccggt ccaccagggc tttggcctta aaggcgctgt caccctcacc     900
```

atcctgctgg gcattttctt cctctgctgg ggccccttct tcctgcatct cacactcatc 960

gtcctctgcc ccgagcaccc cacgtgcggc tgcatcttca agaacttcaa cctctttctc 1020

gccctcatca tctgcaatgc catcatcsac cccctcatct acgccttcca cagccaggag 1080

ctccgcagga cgctcaagga ggtgctgaca tgctcctggt gagcgcggtg cacgcgcttt 1140

aagtgtgctg ggcagaggga ggtggtgata ttgtgtggtc tggttcctgt gtgaccctgg 1200

gcagttcctt acctccctgg tccccgtttg tcaaagagga tggactaaat gatctctgaa 1260

agtgttgaag 1270

<210> 6
<211> 1270
<212> DNA
<213> Homo sapiens 217441

<220>
<221> misc_feature
<222> (646)..(646)
<223> n = t or c

<400> 6
ggagagggtg tgagggcaga tctgggggtg cccagatgga aggaggcagg catgggggac 60

acccaaggcc ccctggcagc accatgaact aagcaggaca cctggagggg aagaactgtg 120

gggacctgga ggcctccaac gactccttcc tgcttcctgg acaggactat ggctgtgcag 180

ggatcccaga gaagacttct gggctccctc aactccaccc ccacagccat cccccagctg 240

gggctggctg ccaaccagac aggagcccgg tgcctggagg tgtccatctc tgacgggctc 300

ttcctcagcc tggggctggt gagcttggtg gagaacgcgc tgktggtggc caccatcgcc 360

aagaaccrga acctgcactc acccatgtac tgcttcatct gctgcctggc cttgtcggas 420

ctgctggtga gcgggassaa crtgctggag acggccgtca tcctcctgct ggaggccggt 480

gcactggtgg cccgggctgc ggtgctgcag cagctggaca atgtcattga cgtgatcacc 540

tgcagctcca tgctgtccag cctctgcttc ctgggcgcca tcgccgtgga ccgctacatc 600

tccatcttct acgcactgyg ctaccacagc aycgtgaccc tgccgngggc gcsgcrassc 660

gttgcggcca tctgggtggc cagtgtcgtc ttcagcacgc tcttcatcgs ctactacgac 720

cacgtggccg tcctgctgtg cstcgtggtc ttcttcctgg ctatgctggt gctcatggcc 780

gtgctgkacg tccacatgct ggcccgggcc tgccagcacg cccagggcat cgcccggctc 840

cacaagaggc agcgcccggt ccaccagggc tttggcctta aaggcgctgt caccctcacc 900

atcctgctgg gcattttctt cctctgctgg ggccccttct tcctgcatct cacactcatc 960

gtcctctgcc ccgagcaccc cacgtgcggc tgcatcttca agaacttcaa cctctttctc 1020

gccctcatca tctgcaatgc catcatcsac cccctcatct acgccttcca cagccaggag 1080

ctccgcagga cgctcaagga ggtgctgaca tgctcctggt gagcgcggtg cacgcgcttt 1140

aagtgtgctg ggcagaggga ggtggtgata ttgtgtggtc tggttcctgt gtgaccctgg 1200

gcagttcctt acctccctgg tccccgtttg tcaaagagga tggactaaat gatctctgaa 1260

agtgttgaag 1270

<210> 7
<211> 435
<212> DNA
<213> Homo sapiens 217458

<220>
<221> misc_feature
<222> (135)..(135)
<223> n = t or c

<400> 7
gatcgaccca cctcggaaag tgctgggatt acaggcgtga gccaccatgc ctgggctgcc 60

atttcatttc cccttgttta tttccagggc ctggactttg ccggattcac tgcacacatg 120

ttcattggga tttgncttgt tctcctggtc tgctttccgc tcctcagact cctttactgg 180

aacagaaagc tttataacaa ggaacccagt gagattgttg gtgagtacaa gtgcaacctc 240

atgtaggctc agatttcatg accataatat tgtttgttta ccaggagaag ttcttattag 300

gaagtatctg ttgatgggtt gctggatgct caataccagt gactctccac gtccaccttc 360

tagtatacac tgttttcagg gctgctatca tgagctgtgc ctctttagtt ttcgtgaagt 420

gtactgtccc taaaa 435

<210> 8
<211> 350
<212> DNA
<213> Homo sapiens 886894

<220>
<221> misc_feature
<222> (193)..(193)
<223> n = t or c

<400> 8
tgcgtcgccc ggaggctgca caccttccac aggtaccggg cggggtcctg ctcagactgt 60

gcttggtgtg cagcagaaca ttccatgggc ctacaaaata gcgacattag ctgtatacta 120

atacrtgata tttaggtgac gcacactgtg ctaagcctct tatagtacat tttatctaac 180

cctcactgag ctntgcaggg ggtacacagc cgagtttaag gaccaaagaa acaacacaaa 240

accagaggct cagagaattt gagcggcgtg cccagggttg tgcagctcgg aaggagtggc 300

actggggatg ggctctcac tgtcaaccgc tgggctgtcc catctctcta 350

<210> 9
<211> 420
<212> DNA
<213> Homo sapiens 886895

<220>
<221> misc_feature
<222> (228)..(228)
<223> n = g or a

<400> 9
gtcactaatg aaaggctgcc tctgttctac gagcctgctc actctggctt gtactctctc 60

tgtgtgtgtg tggccaggca taccggctct cccggggacg ggtgtgggcc atgatcatca 120
tgctctgtct catcgcggcc gtcctctctg ccttcttgga caacgtcacc accatgctcc 180
tcttcacgcc tgtgaccata aggtacgcaa agcacctctg ccgtgggngt tgcggccagg 240
ttctggcagg caggggctct gcctgcactg cctggctcca ggttccattc tcaggtgcat 300
gaaaaggtgg gggcrgttga gcccacagct cactgcattc cagtccagct cgtgtctgct 360
ttgtgtgact gcagtacatg ctacaagcag tggggcctca gaagctggtg gcagaaatgc 420

<210> 10
<211> 420
<212> DNA
<213> Homo sapiens 886896

<220>
<221> misc_feature
<222> (245)..(245)
<223> n = g or a

<400> 10
tggccaggca taccggctct cccggggacg ggtgtgggcc atgatcatca tgctctgtct 60
catcgcggcc gtcctctctg ccttcttgga caacgtcacc accatgctcc tcttcacgcc 120
tgtgaccata aggtacgcaa agcacctctg ccgtgggrgt tgcggccagg ttctggcagg 180
caggggctct gcctgcactg cctggctcca ggttccattc tcaggtgcat gaaaaggtgg 240
gggcngttga gcccacagct cactgcattc cagtccagct cgtgtctgct ttgtgtgact 300
gcagtacatg ctacaagcag tggggcctca gaagctggtg gcagaaatgc ctgcaggagg 360
tggaagacat aggccttgct ttcctggaga ttgtggtctc atggggagac atgtggacaa 420

<210> 11
<211> 512
<212> DNA
<213> Homo sapiens 217452

<220>
<221> misc_feature
<222> (189)..(189)
<223> n = t or c

<400> 11
cctatgtctc acgcctgctg cctgtgctca ctgctcttcc agctgtgata ttgggcgttg 60
ggctgaattg ttccatttgg actctggtta attccatggc tgatacagag ggaggtcccc 120
taactgttga ccttgtgaac agtaaggtcg ttgtttcgtt ctgcagagag acggtgtcca 180
tcagcatcng ggcctccytg cagcagaccc aggctgtccc tcttttgatg gctcatcagt 240
acctccgcgg aagtgtagaa acccaggtga ccatcgcgac ggccatcctc gcgggcgtct 300
aygcgctgat catatttgag gtaactttca cacctgctcc cccgatctgt ctgggcccac 360
agtcagggag gcttgagatc cgtgagacac tctggatggg ctcagtcctg actccttaat 420
caaactggac tagtgtcatc attcctaaag attagcgtgt ccctctctct aggtagaaag 480
ggaaccatac aggaatattt gctgaatctt gg 512

<210> 12
<211> 1283
<212> DNA
<213> Homo sapiens 712052

<220>
<221> misc_feature
<222> (573)..(573)
<223> n = g or a

<400> 12
tctacccgcc cggccaaaac agcccctact gccccctggc ggcaagcctg tgtacgaggt 60

gtggggaggg gaagccacaa caggtagcag ttgggtcagg gcacctacaa gtcattttta 120

ttcttaaagc tcatttaaag catttatggt gttcattaaa atatattttg acagcctggg 180

caacatagcg agacttcatc tctaataaaa ataaaaaaaa ttaactcgat gtggtggtgc 240

acgcctatag tcccagctgc tcaggaggct gaggaaggag gatcacttga gcctggggat 300

ttgaagcttc agtgaactat gatcaggcca ctgcgctcca gcctgggtaa cagagcaaga 360

tgctctcacc ccaccacctc tctctctctc ttctctttat atatatatgt gtgtatatat 420

atatatgtgt gtgtgtgtgt gtgtatgtat gtgtatatat atatatatat atatataggc 480

tcaaattgga gaagaaagtg ctagaaatcc acaccacttt cttctaatgg cattgcattt 540

tgagagagaa tagaccagac acctagactt tancaacatt ctcaaagaac aagccatgga 600

aaggctgtgt gcggaggaga aaggacttct gtttgggtta tattagtcta ttagaggtct 660

gagcactgaa cttataaaaa cctgactttt gaactgaaaa aaagggagct tttatccatt 720

cacttccaca aaagtattgc ctagggtaat ctatgcaatt cctgcaggat aaagattagc 780

ctgtaggccc aattatcata aattcctatt accccaacag aaatgtgttg agtgcctatt 840

ttgcaggcaa aacgtcttcc cttctctccc tggtgctgcg ttcagcacag aacaaccgca 900

tttggcagct tcactccgac agaggccagt ggctccttag agagctgagg tgttccggag 960

gcgcgtgcac caagcttctg ctattcacgc tccttggttc ctcggaaaga gagcgttcgg 1020

ttggaggaat cagtgtgctt tttgcttatt gacagccttt cttctcttct gaatcactat 1080

ttgaactgaa ggtcattgga agacatgctt ttgggaggga tatttgtttg gaaaaacaga 1140

catagctcaa accctgcagg ctctctgaat cttttggtct ttgatgagcg ggtggcctgc 1200

caggcagcgg gtgttctggc tgctgtgttt gtgccatttg tatttgatca gctgctgggg 1260

cacttctccc tctgactgtg tgt 1283

<210> 13
<211> 420
<212> DNA
<213> Homo sapiens 886994

<220>
<221> misc_feature
<222> (245)..(245)
<223> n = a or c

<400> 13

tctgcagggc agggtatact tgctatgtta agttgtatgg ctctgagcag cactttcagc 60

tgctcagtaa ataaatgaag aaggaggtca aggaaaaggg tactcaggtt gaatcgttgt 120

gtattattta aattgtctgg tgagagctac acatcaaaaa ttgttttaca tattagagta 180

tcccaatatt tcaagccatt agcttctgat tactttgctt tttggtgaaa taatttccat 240

gattncttcc taaatattga atatatacac atttacattt ttaactggaa ccctggggag 300

cttcaccagc cagctctggc ctccaggatt tgtacctgtc ctgtcattca gggttggcaa 360

gaggagagct caacatgtac catgccctgc taatgcagtc tagtgctgtg cttgaatata 420

<210> 14
<211> 1400
<212> DNA
<213> Homo sapiens 712057

<220>
<221> misc_feature
<222> (643)..(643)
<223> n = g or t

<400> 14
gagatcgtgg atagcccaga gtgtctcagc accccttga gattgtgccc tgggcctctg 60

cccagcggta agtgttgaag ctctgaggtt tgcccctct ggggaggtgt ctctgatttt 120

atttgcccag acatctccat cctttcaaat acatgaacat tcacaacaga ctcattctgg 180

agccaaacca acgtgagatg cccttgtagg gaacagagtg tgggaacgag gggagggcga 240

gggctgctct ggtgagccca tggcagtggg cggtgctgtt cttcattgga ggggtcctag 300

cagatggacc catcaaggga aaggcacagg agcccgagca gggagccagg gctgcagaga 360

cacctctgtg ccctccgagg cgacagtcac aggtcccact gccagccttg gtgtgctttg 420

tggcctgatg tagcctgagc cagggtcaca tagcggcact caaaatacat ttgcccaatg 480

agtgggtgtg tggtctcctg tctggttgtg tgtctgtgtg tgtgcaggtg agtatatggt 540

ctcctgtctg tggctgtgtg tcttcgtgtc tgcacgtggg tgtatggtcc cctgtgcctg 600

ctctatgtct gtgtgtgtgc accagtgtga actgtgtagg ttntgtgtgg tcccctgtgc 660

ctgctgtatg tctctgtgtg tgcacctgtg tgagctgtgt aggttgtggt cagtgaccat 720

gatgggttgt gcctgtgctt tgcggagtta ttttgggtga gtgccacttt tgagtgttgc 780

tccaagatgg tgtgcatggg cagttgctgg gagaccacag tgggtggtag gtgctgctgt 840

gatgggggtg tgactgtggt tgtgggcaag ggtgggtctg agctgtgttt catgatgtgc 900

accagcttag gggtggtgt gctctcccac gcagcctgcc ctggtgacag ggtcccctgt 960

gcatgagatg tgtggctgtt gctggctgtt tcgtgacctg cacgcgtggc atgtgtgttg 1020

tggctctgcg tgcgtgcagg aatgtctgtg gccctgggtc cgctctcagc tgtgacatgt 1080

ggacagcagg gtggtgtacg aagtcagtgc tgattgcatt gagctgagta cagtcaactt 1140

cttaataacc aacccatgag ggaggagatc tgtgacccag agaaaggaaa cctatgctag 1200

aaatggaaaa ccaattacat tgagctctga aagcatcacg atttgatatt tttggctacc 1260

aatttgcttc cccacttcct actcacatga atgtgtgttt ctgaagccct gctgctcagc 1320

agggcctggc aggtgctctg agatttcaaa ggaaccgggc agggtgggcc aggtctcccc 1380

tggtccccaa gagctgacct 1400

<210> 15
<211> 1107
<212> DNA
<213> Homo sapiens 712058

<220>
<221> misc_feature
<222> (539)..(539)
<223> n = g or a

<400> 15
atcattcagg tcattatatg tattttttttg ggaaaataga gagtgagcac cttttccagc 60

caacaaatga agccccaccg gccccccatg actagtcctg ccagccaggc tccaagtcac 120

agaccgcgtc caggcacgaa gcgctgggga ctgctgctcc gcgatctcac cacgcagcgt 180

gaccagggaa gtaatgagtc tcttcttttc tcttttagac agatctcaca ggaggacaaa 240

aattgggaga ccaatatcca agaactccaa aaaaaggtac ccagctttct ttcctcaggg 300

atttctgatt cacttctcca agaagagagt gagtgatgcc ttttccttcc ctcacgtgac 360

tgaatgccgt ttctcttttt atttctgtgg ttatacacaa gacgttgagg tttatgtgtg 420

tgagtggatg gggaaaaatg ttttctggat acagcaggct gattttgtga ggatgtggaa 480

agcaaacatc tttatagagc ctttccctgt ccctgcacgt tgcagggccc gccctctgnc 540

gggtgtccct gaccaccagc ccgctcctgg ccctgaaggc aggcccaagg ttcacacttt 600

gcggaggggga gaccggcaag gcatgctgca tgaagtggag cagctttagg ggcagacata 660

ggatgcgtga gtgtgctgcc gatggctgtg acttcctggg gcagagcttg ttttcttttg 720

ttttgttttt tttggctcat tcttccatgg gggtggactt tctcagccca tttatgaaca 780

cagaggaccc cttcccagtc gagagagctc tgctcaagat ctgctaggag tcatttgcat 840

ctcagtgaca tttcagatcc atgcagtttg ttttctaggg agagattgaa tacccactct 900

aattttgatg ggcacactct ccatgcgagt caggtgtttc ctcaaagggc ttcagaacac 960

ctcacatcta tcgtgcttat tttccataaa gatgttggat gcaatctgat gaggtgcctc 1020

agtgccttca cactctgtcc catgtggatg gccagggtta gaaaagaaag gtatagctgt 1080

gatactcttg caggccccaa gttcata 1107

<210> 16
<211> 915
<212> DNA
<213> Homo sapiens 712060

<220>
<221> misc_feature
<222> (418)..(418)
<223> n = g or a

<400> 16

tccaattcta cattaattcc tccactatga gcttccacag taacctaatc ttaccctgag 60
atgtctatat caaactgctt cctcacatga gggaaggcac caggtctcgt ttacattttt 120
gctctgtatc actacaatac aagagagaat gtgataaagg ttgtaacaga cccggaaaaa 180
ccactctggg agctctaaga agggtagttc atgtaaatac acacacatat acatatagtt 240
catgtaaata tatatatgta tacacacaca cacggccttc ttcaaggaag agattgctct 300
taggatgttt tcagattgaa gatgctgtaa aatttgtatt gatgatataa aattaaaaaa 360
aagaaattct gttattgtat attttagatc tatcatttcc atttggttct tttttctnta 420
tcttttgttt cttcccatag tttttcattt ttcacttgtt ccaagagaag ttgttaactg 480
attgttgaga catttttagg aaggctgctt taaaatcctt ttaagataat ccagcatccg 540
atatatctca gtgttggcat caggtgtttg tcctttccca ttcaagttgt gattttctca 600
gtttctgata tgacaggtga cttttgattg tatcctggat attttgtcta ttattttagg 660
agactctgag tcataaataa ctgttttatt tcagcaggca gtcaacctgt ttaagtttag 720
cacacaggtt atagactatt tacatagcct gttgttcaaa tgaagattta attttcagag 780
atcttgcagt gctactttga tctgtttggt ttctccagtg ctgctgggtg ctgccttggg 840
ggctggaagg gatatcccca ggctgggctg cccagatgtc tcttcctgtg gagaggagtt 900
tcaggtctgc agaag 915

<210> 17
<211> 1750
<212> DNA
<213> Homo sapiens 712064

<220>
<221> misc_feature
<222> (795)..(795)
<223> n = g or a

<400> 17
atgaaaataa aaataaaaat aaataaataa ataaatgaaa gaaagaaaga aagagaaagg 60
acctggtgca gttatccttt cacacagtgg gccggcattc acacggggac cttgttaaca 120
gggcaggcct accaggtgcc catctgtgcg tgcttccctg ccctggcccc acgcagccat 180
ggcctgtgga gcacagtacc cttgggcctc atagggagag ccccgtctcg tgccgtctcc 240
taggtactcc taggttgatg gaggccttgc ggaggatgag gtgcctcggg gcctgccctc 300
cccctatgac caggtggatt ctcgcgggtg tcattccagc gctaagagtg cacccccctgc 360
attccagggg cctcatgcat gtcgtgtaaa gaacagtgcc agagccttat ctgggagtca 420
cagttccgtg agaaggctca gctcatggcc ccacccgcat gcttggcgtg gtagagaaag 480
gaacagtgaa gacagcatag gcccctgtag aaacgccccg tcatcctctg atacctgccg 540
gccaggtgtt tcataacagg gctgtgctac tcttgacatc tgtgtttatc tttcataaag 600
attttgaatg cagtaatcct gaatctgtac gggtttcctt gtaacacagt actttgccat 660
tttctttcaa gttcgagagg ttacattttt catcctcgtg aaatctgtcg tgattccagt 720

tgcgtaggtt atgacacgct gcaggagtca gaaggttgtg cagagtaaat gagctgtggt 780

ttctctctta cagcntagga tatctgacgg gattctgctc gccaaatgcc tgacagtgtt 840

gggatttgtt atcttcatgt ttttcctcaa ttcgtttgtc cctggcattc atcttgatct 900

tggtgagtct aatttagctt tggttcatag gctttgtcac attctggatg ggaaggtttc 960

agagcctgtt cccagacact gactttgccc acaggcagcc gggctggtgg aaggccagag 1020

agggctgaga tggagggtgg gcagcctgcc ctgggaagaa gggcgccttt ccttttggtt 1080

tcctgggcag gagggaggga gagagagatg catctctggc cccttagact ctgtgccatg 1140

ggtcctcagc ccctccaggg atgaccatga ggaggaatat agagtgggca ctgtcctgtc 1200

tattgtagtt aataaccaca tctttacatg gttcccagaa gagatggagc cacatgggca 1260

aggccagcgc tgccatctgt gccgcctacc atgccagtta ggtgacagtc tgttcgggag 1320

agccctggcg aatggccggt gctctgcagg gcccacttgc cttgtctgag ggtgcatctg 1380

gcgcatgaaa ctgttctccc accgtccacc attggtttct cttctcccac gttcaccaca 1440

cccatggctc tcagacctct ccactttctc tagcctgtgt tgtggccagg acctatcccc 1500

acctgagatg tggctctctc agggggagct caccacagag cttgtcaacc cctggcctcc 1560

tccaccctcc ataaacgttc tccactctcc caggcgtttc ttatcaattt cacagttatc 1620

tccattggta tccttattga aaacaaaaca aaacccaccc cacatgaaag tgtaggttta 1680

taagaagcat aaatttgagg tggtgtcaca gtcttttctt ttaccaaagc tttacccata 1740

gttttccttc 1750

<210> 18
<211> 1032
<212> DNA
<213> Homo sapiens 712054

<220>
<221> misc_feature
<222> (535)..(535)
<223> n = g or a

<400> 18
cattgactta tttttaaaaa tattgctcca ttgtcgtttt gtttatatct tgattttgga 60

agacctgatg tcagtctgat tgttttgcgt gcggccttga tgatttttat cttcttcctt 120

gaaatcttat agttttacta gaacatgtaa cagagatttt agttttaaat attagcttca 180

ttctactatt tgtttttttc ccttaaggac tccaataaac aaatattatt ccttcattgc 240

ccgggttcca tttccactac tatctctgcc cttttaattt atctatttac ttattcattt 300

ttattctctt acttgctttg atatctttat ttagtgaccc ttgttatatt ttcatttttg 360

tctattgtct tttgggcatc ttttaattta tttctcattt cttttgtaaa gtgatttctc 420

tgagtacata atagttgttg catatttatg ggggacatgt gatattttga tacaataata 480

caatatgtaa tgatgaaatc agggtaatta ggatatccat aacctcaaac atttnttgtt 540

acttgttttg ggaacattcc aagtcctttc ttccagttat tttaaaatat acaataagtt 600

attgttaatt atagtggccc tatcatgcta tcaaacacta gaacttatta cttctaacta 660

accctatttt ttgtacccat taaacaaccc cttatttctg agaaaacttg gttacctcat 720

ccttgagttc aatcaacttt ttatttctcc ctgttatttg cccatttctg ttttcaaatc 780

tctgatttaa ggtgggtttg tatttttgat gcttgcttga ggcgtgggca tggcgaattc 840

attttgaagt gtgggcttgt agttttcttc tacatgcttc atggttattt tcagagggga 900

ttttcctcag ctgatacatg tgacatttcc gctcctgata gcgtttgcac tagctctgta 960

ggtgtgactt cattttttctc ttgttcattt aatgccgttg ggcttgtttg tgttttgtag 1020

gattcctggc gc 1032

<210> 19
<211> 910
<212> DNA
<213> Homo sapiens 712056

<220>
<221> misc_feature
<222> (554)..(554)
<223> n = t or c

<400> 19
aatagcaaag gtggcctgaa tctcctgcta atgcaaaagt gagcctaaaa gtgtcatccc 60

atagtatttg gtctcctgtg cgagtttctg ccatgcattt tcaattgagt tggggagaag 120

aggtctttct tatggtgtct tctaaaactt cagcctttta caattcagac agacccttag 180

gcaaatttcc ttgtaagatt tatcactgaa tcttgggcac attcttgaat ctagcacctg 240

agtgctggga acacatacat tatttctgtg ttggtgattc tgtttcctac cctgtctctt 300

tcaggtctca ctcttctttg ccccagatgt ccagctccag gtctaaagat tcctgcttta 360

cagaaaacac tcctttgctg aggaattcct tacaggagaa agggtgagat attttccccc 420

tcatatgaaa gtaagagttt ctgagcattg cacctggcat gtatgctgga gaacttgaga 480

cacgaatttt tattggacat gtttaacctc tgccagatcc ttgacaattt attgtagtag 540

atgttcatga ttcnggtggt tatattctgt ggatattaat ttccagatgg ccttgagcat 600

aaccctgcag caactgcaca gcacacacgc acactcatgc atgcacaaag ctctgatggg 660

ctgtcttacc aggctggggt ttctcagcta ggggtgaggt tggcattgtc tggagacact 720

tttggttgtc acactgaggg ctgagatgct tctggcatct aagggtagag gcaagggatg 780

ctccaaacat tctgcaatgc acaggacagc ccccaccaca aagaattatc cagcacaaat 840

gtcagtagtg acgaagttga gaaaccctgt atatgtgttt cacaagaaaa ctcaatttcc 900

tgcaaacttg 910

<210> 20
<211> 420
<212> DNA
<213> Homo sapiens 886892

<220>
<221> misc_feature

<222> (210)..(210)
<223> n = g or c

<400> 20
gctgcaggag tcagaaggtt gtgcagagta aatgagctgt ggtttctctc ttacagcata 60

ggatatctga cgggattctg ctcgccaaat gcctgacagt gttgggattt gttatcttca 120

tgtttttcct caattcgttt gtccctggca ttcatcttga tcttggtgag tctaatttag 180

ctttggttca taggctttgt cacattctgn atgggaaggt ttcagagcct gttcccagac 240

actgactttg cccacaggca gccgggctgg tggaaggcca gagagggctg agatggaggg 300

tgggcagcct gccctgggaa gaagggcgcc tttccttttg gtttcctggg caggagggag 360

ggagagagag atgcatctct ggccccttag actctgtgcc atgggtcctc agcccctcca 420

<210> 21
<211> 453
<212> DNA
<213> Homo sapiens 217455

<220>
<221> misc_feature
<222> (225)..(225)
<223> n = g or a

<400> 21
caagcagctt cccttagatg gcacgttggt ggtagctgta tgtgtctgtg gggtgtccag 60

gcctgaaaca tcaagaccca tgacttatca tttgaataga tgtggtacag tggcagatat 120

agaccccctc atgaccacac agctttcgtg tgtgctaact ccctcgtgca ctggaacgcg 180

gtaatttcct gtgcttcttt ccagatcgtg cacagaactc tggcngccat gctgggttcc 240

cttgcagcac tggcagcact ggctgtgatt ggcgatgtaa gttgtcacag tcccaatccc 300

tggcttacca ctcagtggga tgtcagctca aagatgttcc aggattcagg ctttcgtggt 360

tttttcacta ttttatatgc cacgtccatg tttttgccca agaaccatgc tagaggtatg 420

aactaacaag ctacagcatt gaagagtact ttt 453

<210> 22
<211> 870
<212> DNA
<213> Homo sapiens 712061

<220>
<221> misc_feature
<222> (170)..(170)
<223> n = t or c

<400> 22
acacagtggc agatatagac cccctcatgt ccacacaggc tttcgtgtgt gctaactccc 60

tcgtgcactg gaacgcggta atttcctgtg cttctttcca gatcgtgcac agaactctgg 120

cggccatgct gggttccctt gcagcactgg cagcactggc tgtgattggn agtgggatgt 180

cagctcaaag atgttccagg attcaggctt tcgctggttt tttcactatt ttatatgcca 240

cgtccatgtt tttgcccaag aaccatgcta gaggtatgaa ctaacaagct acagcattga 300

agagtacttt tcattaggtt ttgtcacaca ctcacatccc agtggtgtga ttcctcatcg 360

tggtggagga aaggctcctc atgggcatgt ttgcctaggg ctgtggagct gggttgtgat 420

ggggctggat ctgggtgttg gaactagagg ggaccgtcct agctggtgca gaaaggtggg 480

agtcagttgg gccagggtct gtcctgaaga gatcaggagg cccctggaga ggcgtgtttg 540

gggatgaggg tgtcctgttt gggtctgagc agggcctctc tggcagggac atgggaaaca 600

aatgtaggga aacaacagag accagtggcc actggggatg gaggcccaga gttgtctgag 660

aggcagtgct ggaacccagc tgagagtggg acagctgcca tgccagttcc tgtcatctgg 720

tctcaggcac ggcactgcag agagcacata cagactccac tcggaatgta accaggggcc 780

agtcccgcca tgtgggagct gccagaggca ggggctagaa aaaactttat tactaaatgc 840

atagatttga cattatagat gccctgggtc 870

<210> 23
<211> 350
<212> DNA
<213> Homo sapiens 886938

<220>
<221> misc_feature
<222> (172)..(172)
<223> n = t or c

<400> 23
ccctgctgtt cgaagtcttc acaatttggc tcatctattc ctgaatggaa cagggggaca 60

aacccatttg tctccaaatg atcctatttt tgtcctcctg cacaccttca cagatgcagt 120

ctttgatgaa tggctgagga gatacaatgc tggtaagaca ttttcatatg cnttttgcat 180

gctcagctgg gcrgattgtt tagatggcat agttatcagt tcaagctgag cactcagcgc 240

ataaaaacac tttcaaaata aggatagcat agctgtaata tcaagtcact tccagacatt 300

caattctact ttgaaaatgc aggcaagaag tctctccaaa tagttattat 350

<210> 24
<211> 420
<212> DNA
<213> Homo sapiens 886943

<220>
<221> misc_feature
<222> (216)..(216)
<223> n = t or c

<400> 24
tggtgccatt ctggccccca gtcaccaaca cagaaatgtt tgttaytgct ccagacaacc 60

tgggatacac ttatgaaatt caatggccaa gtgagtgttg aaagtgtatt tttactgtga 120

taatttccaa aarcaaatgt gttatctttc aagtagagta atcacggtat tctgaagcta 180

tgttttccat ttggacttgg aaactttcat ttgtantttt atttgaggat aagggaagga 240

atttgatatt tgttgagagt ccacactaag ctgatattga tcttattgta cagcacccta 300

tctcatttaa tcctcacaat gctttggggt gagtatgaaa atcttcattt cacaaataag 360

gaagctgagg cttaaatagg ttaactgtta cagattcaca tttctaatga gggaagagaa 420

<210> 25
<211> 121
<212> DNA
<213> Homo sapiens 560

<220>
<221> misc_feature
<222> (61)..(61)
<223> n = a or g

<400> 25
tcatctcgga gcttctcctc aggtggcagg tggctgttgg cagtgaagaa gcagaggaag 60

nccagcaggg tggccaggag taagcgggtg acatccatcc caggaggcct gagtgggaca 120

g 121

<210> 26
<211> 401
<212> DNA
<213> Homo sapiens 552

<220>
<221> misc_feature
<222> (201)..(201)
<223> n = a or g

<400> 26
aatccagcta gcaaataaat aaatacataa atccaggggg cctatgtcaa cagctccatc 60

ctcctgacac aaactatacc tgcattcgtt ctctctgggt ttgggaatct aggatatgag 120

ccaacatata tgcttgtctg aaggggccac ttacctcttc atctttctca ctgagactta 180

atttattagc cttattctgt ncatcttaat aaaatctctg ctactgtgag gccctactgg 240

gggcctttca acaactctgc ccaggcctgg ggtctcctgg ggccacagac attaaaaaaa 300

cacacagaag cagccaacac accaccctct ggacagcaga gaccagtgca atgcattgct 360

ttttctcttt gctccaatct ccttctgttc cttgtctttc t 401

<210> 27
<211> 401
<212> DNA
<213> Homo sapiens 559

<220>
<221> misc_feature
<222> (201)..(201)
<223> n = g or a

<400> 27
tgccaccagt ctaataagca gcttagcata tggtagaggc tctgaaaggc ctgaagttaa 60

gacacttggt gaactttgtt taatttagca tttctgaaac ttaatgaatc acagaactcc 120

tgtcaacagt aacaaacttc aggaaatgct ccagaacata tgcaagtctg ggatggacca 180

gtcctgtcat gtcagggttg ngaatgaagg ctcaggggaa aatatgaggg gcactggagc 240

ctggcattgg agatctggtt tgacttcacc tgataataat catagacata ctgtgtggta 300

ggcactgtga ggtgagtatg gtctttattc atatttcaca gttgaggaac ttgaggctta 360

ggagaattaa gtaactagca cggatcacac agtttttaac t 401

<210> 28
<211> 401
<212> DNA
<213> Homo sapiens 468

<220>
<221> misc_feature
<222> (201)..(201)
<223> n = t or c

<400> 28
caaatcaggt cctcctggct ccagagctct caggaatgga aaataggaaa cacaggtgca 60

tctgtgtaca agacaggagt ccattttctg ggtatcacaa tgttcctgtg cctgccacaa 120

tggagatgaa ggaaggggca cctggggtgg ctctgagtgg cccaagctca cttaggtcga 180

gggaccaggc cccacaagag ngtcacaggc agatcccagt gcctgcttgg atttcccatt 240

tgccttccct cagaggacac gttgctatca gtgcctggct ccaggtcagt agccgggcta 300

acaagaacct actggcttga gtcctacagt gtgactcatc cagcacgctt cttctcctct 360

ccctgacctt gtgacttccc aagccccctc tgcccctctc a 401

<210> 29
<211> 540
<212> DNA
<213> Homo sapiens 657

<220>
<221> misc_feature
<222> (356)..(356)
<223> n = t or c

<400> 29
ttggctattg taagtaatgc tgctatgaac atgggtgtgc aaatatctct gctggacctt 60

gctttcagtt cttttaggta taccagaagt ataattgctg ggtcatatgg taattatttg 120

ttccattttt ttgaggaatc cccatactgt tttccatagt ggctgcacca ttttacattc 180

ccaccagcaa tgcacaagga ttccaatttc tctacaccct cagcaacact tactattttc 240

tatttttttt tgatagcagt catcccaatg ggtatgaggt ggtatcttat tggggtttct 300

gcctggcatc taaggccctc tgtacctagg ctctttcata aatttgaact taattngagg 360

taattctctg cccaagcgtc ccactacagc caggcttgaa agactcaggt caaagagaga 420

gagactgagc tctgaaatca tcttgattgc tttctaggct gagactttgg gtaaataggc 480

tgtgtgattt ttcaccttct tgattaagat tttttaaatt gttttgtttt tgttttttttg 540

<210> 30
<211> 636
<212> DNA
<213> Homo sapiens 674

<220>
<221> misc_feature

<222> (599)..(599)
<223> n = c or t

<400> 30
ggagaaagaa ccaaggtgat gctagaagag attctagaca gagactaagc tacctctcag 60

gccattcttg actaaacaat catgaaaact ctaggagaga gttgctcaac tcaatgctag 120

aaccatctta gatttgtatg taagttgtgg tttgttatta tattcatatt ttatcagaat 180

gaattggatg taattcatag gtttagttct tctcaatata gtatgcattt atccttataa 240

attctagagt tgaagagaat ccattcaggt gacatttagc acctgtgaaa ttaaagaaaa 300

caagccagcc cccagcctag tccatagaaa cactgccacc ctggggaacc agagaggggt 360

ccagccaccc tctctgattc ctcagctctt ataaaactca tcaagatgtt atgccactta 420

ggaggtagta actgtgtacc tgctatttaa aaactagtat tgaataagta aatgtgacat 480

ttaaaaagca taaatacatg ctcacaatga aagcaatgac tatcatttca aaagctgtgc 540

aaaattagtc agatctgccc ttcaccaatt agtgttaatt cctattaata tgatctaang 600

ggacttaatt tcctcagcta tagtgaatgc aattgt 636

<210> 31
<211> 681
<212> DNA
<213> Homo sapiens 632

<220>
<221> misc_feature
<222> (45)..(45)
<223> n is any nucleotide

<220>
<221> misc_feature
<222> (267)..(267)
<223> n = a or c

<400> 31
gaattcgcct ccacctcacc aactcacatc tttgatgatt aacangcttc acagaagaaa 60

agtttacact ttgaaccaag aacatacggt agaggagaga acatttaaag tgtctgcatc 120

caatgcacag gaagaaactg cttccttcta actccaggcg gtatttgata attctacgac 180

tttcataaac ctaaggctgc cttgtggttg ctctcttaat taacttgcat gaaattactt 240

cccactgcca taccctcaac ccaatcncaa acctgtaata atataccttc agccaaggaa 300

aaaacccacc taataatgta tctctaacag aataataatg gagccacaca aaaaaatcat 360

aaacactgca gttggcaaac tgcggctggg ttccattggg cccaagcagg cccagccagt 420

gttgtgtggt gatcacgtag tcggggtgta ctctcttctt cgcgagatct aaggcgccca 480

agaactgctc tctttcctga ggactcaagg aatggatgtt ctgccgaatc actggtggtt 540

tcttccgctc gcagttggga ccggtccagc caaacttgca gtctccacaa ttatagccgg 600

caaagtttcc tagttcacaa aacagaaaga tggaaaggaa gggggtttat gtcgtttgga 660

agaaaattct gattctatca t 681

<210> 32
<211> 121
<212> DNA
<213> Homo sapiens 701

<220>
<221> misc_feature
<222> (61)..(61)
<223> n = a or g

<400> 32
gcccaaatca actcatatag agtgactatg atggcgagga tcaagatttc gggaagaaaa 60

ncagttaagt tttcaacgat gtatgaatct ctctctccaa gcaggactat aaaccccttt 120

g 121

<210> 33
<211> 1292
<212> DNA
<213> Homo sapiens 710

<220>
<221> misc_feature
<222> (451)..(451)
<223> n = g or t

<400> 33
tctctttcca gacacaacaa atggtaccgg tgccaggtaa caaatgcagg tccttgatgt 60

gagaaatcta tggccctgta ggggcgtcct ggtcctgaaa caattgggaa acatattaga 120

gatgacagaa tagaattttt taaaatgtta aatcttacct gggctgtatc tatagagcca 180

ataagtccat gatgttttat agtagtaaga aaaaaaagtt ttgttgttat ttatatctcc 240

aaaagcaata gtaatgacat gaatttcaga agtaactagt acttttagga taaaccctcc 300

tttatctctt aaaaggttga tttctagcag agatccagga aattcaggtg actttcagga 360

ttgggagccc ctgatacata tttaatttgt aagcaacaat ccattcaggt gaaatagact 420

atccaaaaga atagatcatt aagacctaaa ngaaatatat tttttaaagg catttgctaa 480

cctatgcttc acaaagatat aaatttaaaa gggagggatt aattaataat attgtaactt 540

caactgcact tagtcctaat gcagtattta tgtaaccaaa aagaactttc gcgtattttg 600

cctcacccat caatctgtta taactactag ctgacaagca tgttcgctga gaccagaaga 660

gagaaaaata cattcaagcc atttaggctg gacactggaa ctgccaattt tagtgaaaag 720

ctctggacga tttaatgacc tccttactgc ctggtccatc cttcttccct ttttcataac 780

agctttgatg gttatgtgga aactttagct aactttagtt cttgtccaaa atacctataa 840

caacttcaga gagctgaaca gacgtgaata taaatttaat tcgtagctat gaccagaaac 900

taggactttc ttttcctctt gagagagtac tctctcataa agcaataatt cctcagagtg 960

ataacaggaa aactataaag gtttatagcc aaaagagcct ccttatctat cgataaagac 1020

agacagatgc ttacctgtaa ggcaagtttt aaaagactca cactgagaaa agcactgctg 1080

tccctgatgt tggctacacg gccctttttc atgttcaatt cagagcaagg tttcaaattc 1140

tcagaaccca acaacgagag ggtatcaatt gaaggggaac aagtgatgag cagaaggatg 1200

aacctgtcca aatcagaatc ttgctcttca aagtgagctt gatgtcttca ttcatcctga 1260

ccccaatgaa ggaccccaaa aatggggaga gg 1292

<210> 34
<211> 627
<212> DNA
<213> Homo sapiens 217456

<220>
<221> misc_feature
<222> (326)..(326)
<223> n = g or a

<400> 34
ccagaatacc gatggcatta cgggactgag ggtcatcacc ttgtgacaaa ttaaccatca 60

caggggctct gtgaaggaag aggatcagag gggtgacagt gctggctagg gaggatttag 120

aatgtctagg aacttcgatg gccagcactg tctctatctc ggcccccta ggactccgtg 180

ggtctatgtc ttaacccatg gggtaatgtt agtttggctc cctgttctta aagtcactaa 240

tgaaaggctg cctctgttct acgagcctgc tcactctggc ttgtactctc tctgtgtgtg 300

tgtggccagg cataccggct ctcccnggga cgggtgtggg ccatgatcat catgctctgt 360

ctcatcgcgg ccgtcctctc tgccttctts gacaacgtca ccaccatgct cctcttcacg 420

cctgtgacca taaggtacgc aaagcacctc tgccgtggga gttgcggcca ggttctggca 480

ggcaggggct ctgcctgcac tgcctggctc caggttccat tctcaggtgc atgaaaagga 540

gggggcagtt gagcccacag ctcactgcat tccagtccag ctcgtgtctg ctttgtgtga 600

ctgcagtaca tgctacaagc agtgggg 627

<210> 35
<211> 121
<212> DNA
<213> Homo sapiens 656

<220>
<221> misc_feature
<222> (61)..(61)
<223> n = t or c

<400> 35
tagcagcagt cactggctgc gtctaccccg catcttctgc tcttgtccca ttggtgagaa 60

nagccccctc ctcagtgggc agcaggtctg agtactctca tatgatgctg tgattttcct 120

g 121

<210> 36
<211> 121
<212> DNA
<213> Homo sapiens 662

<220>
<221> misc_feature
<222> (61)..(61)
<223> n = c or t

<400> 36
agggaacaag cacttcctga gaaatcagcc tctgaccttt gccctccagc tccatgaccc 60

nagtggctat ctggctgaag ctgacctctc ctacacctgg gactttggag acagtagtgg 120

a 121

<210> 37
<211> 121
<212> DNA
<213> Homo sapiens 637

<220>
<221> misc_feature
<222> (61)..(61)
<223> n = c or a

<400> 37
cctgtggctc ctccccagtt ccaggcacca cagatgggca caggccaact gcagaggccc 60

ntaacaccac agctggccaa gtgcctacta cagaagttgt gggtactaca cctggtcagg 120

c 121

<210> 38
<211> 425
<212> DNA
<213> Homo sapiens 278

<220>
<221> misc_feature
<222> (93)..(93)
<223> n = a or g

<400> 38
taagtaggaa aagaatttgc tgagaggcta ttgagtagct cacaaaatca tggagcagca 60

ggctcagaaa caggtgagaa taagcaagaa ggncatcagc taagacagct gccaaaacca 120

tgctatagaa cacagggcac ttgctgggca atggattcct ttgctggtac atctggcttt 180

gctgaccctg aaaactgaat attgttatac caactgccac tgcccatttc taggatggtt 240

tctgattatc cctgcttctt tgtgtcacta tctcctgttt cgaagtcatg aatgagtatg 300

tcagattggc agaatattta tcatatggtc atactctaac tttagaaaaa gccgagaaac 360

aaagtttaag tatctaaacc attgtcattg gaggtaagct ctgtctccca tcaagactca 420

ttaag 425

<210> 39
<211> 361
<212> DNA
<213> Homo sapiens 386

<220>
<221> misc_feature
<222> (114)..(114)
<223> n = a or g

<400> 39
ataggccatt ttgtacatgg caaccatgtg aagagcagta gaatcagaag aagaaaaaaa 60

aaggttttga gacatgactc tatcaactga ctgtaaggtg acctgggaaa ttcnctctac 120

atccctgaat ctcagtttat tcacctgaaa tactgggacc agaacacatt aaagaattat 180

ttagaatgat acattaatga gcctagtaca gtgtaacaca gggtaaacat ccagcagttt 240

tggaatcatt tttggaagtt tcttgctagg gttaccaaga aaatttgtag aaatcttgaa 300

cttaagtgta gttaataata atagctatta taatgtttat tgctctatga tgacgatagt 360

a 361

<210> 40
<211> 906
<212> DNA
<213> Homo sapiens 217480

<220>
<221> misc_feature
<222> (558)..(558)
<223> n = g or a

<220>
<221> misc_feature
<222> (328)..(330)
<223> nt. represent ctt insertion variant

<220>
<221> misc_feature
<222> (333)..(335)
<223> nt. represent ttc insertion variant

<400> 40
tcccattttt ctgatgaaga aactgaggct ttggagtatt aggtgtaact ttcccaagct 60

cttacagtta ataagtagta gagctggcct tcaaacccag gtgtctactc caaaggactg 120

tgaaaggatg aagatgatgg tgatcgtaac aatggtggta acaataaaaa caatgggatg 180

tctttttatt tcagacccag actcttttca agactacatt aagtcctatt tggaacaagc 240

ragtcggatc tggtcatggc tccttggggc ggcsatggta ggggccgtcc tcactgccct 300

gctggcaggg cytgtgagct tgctgtgnnn tcnnngtcac aagagaaagc agcttcctga 360

agaaaagcag ccactcctca tggagaaaga ggattaccac agcttgtatc agagccattt 420

ataaaaggct taggcaatag agtagggcca aaaagcctga cctcactcta actcaaagta 480

atgtccaggt tcccagagaa tatctgctgr tatttttctg taaagaccat ttgcaaaatt 540

gtaacctaat acaaagtnta gccttcttcc aactcaggta gaacacacct gtctttgtct 600

tgctgttttc actcagccct tttaacattt tcccctaagc ccatatgtct aaggaaagga 660

ygctatttgg taatgaggaa ctgttayttg tatgtgaatt aaagtgctct tattttaaaa 720

aattgaaata attttgattt ttgccttctg attatttaaa gatctatata tgttttattg 780

gccccttctt tattttaata aaacagtgag aaatctacat taactgactc ctttaggctt 840

cagaaacaca tttttattct cttcagaaag gatgatattc ccctttattt tacatttctg 900

ctccaa 906

<210> 41
<211> 420

```
<212>  DNA
<213>  Homo sapiens 951497

<220>
<221>  misc_feature
<222>  (221)..(221)
<223>  n = g or a

<400>  41
tttcattttt ttttaatgaa caggatttgc tagtccactt actgggatag cggatgcctc      60

tcaaagcagc atgcacaatg ccttgcacat ctatatgaat ggaacaatgt cccaggtaca     120

gggatctgcc aacgatccta tcttccttct tcaccatgca tttgttgaca ggttggttaa     180

tatttcttta taaataacgt gctcattgga tttaaataga nggtgcctat caaatgtgat     240

ttaagttatt aaataaaagc taagaagtta tggtagtcta ttgtctgtga tcaggttgtc     300

accaaaacag accttaggct aagaatttgc atgcaaatgt ataataaaga aagtgtttat     360

aaagataaat taaaagaagg tggattaggc aggatacaaa agaaagaaaa gtaaaataag     420


<210>  42
<211>  906
<212>  DNA
<213>  Homo sapiens 217468

<220>
<221>  misc_feature
<222>  (660)..(660)
<223>  n = a or c

<220>
<221>  misc_feature
<222>  (623)..(625)
<223>  nt. represent ata insertion variant

<220>
<221>  misc_feature
<222>  (686)..(688)
<223>  nt. represent tct insertion variant

<400>  42
atcactgtag tagtagctgg aaagagaaat ctgtgactcc aattagccag ttcctgcaga      60

ccttgtgagg actagaggaa gaatgctcct ggctgttttg tactgcctgc tgtggagttt     120

ccagacctcc gctggccatt tccctagagc ctgtgtctcc tctaagaacc tgatggagaa     180

ggaatgctgt ccaccgtgga gcggggacag gagtccctgt ggccagcttt caggcagagg     240

ttcctgtcag aatatccttc tgtccaatgc accacttggg cctcaatttc ccttcacagg     300

ggtggatgac cgggagtcgt ggccttccgt cttttataat aggacctgcc agtgctctgg     360

caacttcatg ggattcaact gtggaaactg caagtttggc ttttggggac caaactgcac     420

agagagacga ctcttggtga gaagaaacat cttcgatttg agtgccccag agaaggacaa     480

attttttgcc tacctcactt tagcaaagca taccatcagc tcagactatg tcatccccat     540

agggacctat ggccaaatga aaaatggatc aacacccatg tttaacgaca tcaatattta     600

tgacctcttt gtctggatsc atnnntatta tgtgtcaatg gatgcactgc ttgggggatn     660

tgaaatctgg agagacattg attttnnngc ccatgaagca ccagcttttc tgccttggca     720
```

tagactcttc ttgttgcggt gggaacaaga aatccagaag ctgacaggag atgaaaactt 780

cactattcca tattgggact ggcgggatgc agaaaagtgt gacatttgca cagatgagta 840

catgggaggt cagcacccca caaatcctaa cttactcagc ccagcatcat tcttctcctc 900

ttggca 906

<210> 43
<211> 483
<212> DNA
<213> Homo sapiens 217473

<220>
<221> misc_feature
<222> (163)..(163)
<223> n = g or a

<220>
<221> misc_feature
<222> (219)..(221)
<223> nt. represent cga insertion variant

<400> 43
tatttttgaa gtataaagaa tatattcaac atctttccat gtctccagat tttaatatat 60

gccttatttt actttaaaaa ttttcaaatg tttcttttat acacaatatg tttcttagtc 120

tgaataacct tttcctctgc agtatttttg agcagtggct ccnaaggcac cgtcctcttc 180

aagaagttta tccagaagcc aatgcaccca ttggacatnn naaccgggaa tcctacatgg 240

ttccttttat accactgtac agaaatggtg atttctttat ttcatccaaa gatctgggct 300

atgactatag ctatctacaa gattcaggta aagtttactt tctttcagag gaattgctga 360

atctagtgtt accaatttat tttgagataa cacaaaactt tatgcttcga caatgttatt 420

cctgaacact ttaaatcctg aaagtgcatt ataatcctta atttattacc agtttattat 480

cac 483

<210> 44
<211> 811
<212> DNA
<213> Homo sapiens 217485

<220>
<221> misc_feature
<222> (364)..(364)
<223> n = a or c

<220>
<221> misc_feature
<222> (198)..(201)
<223> nt. represent aaga insertion variant

<220>
<221> misc_feature
<222> (708)..(711)
<223> nt. represent aatt insertion variant

<400> 44
tttgtctttt tatttttatc ttcctttcca aataggtcgg gagtttagtg tacctgagat 60

aattgccata gcagtagttg gcgctttgtt actggttgca ctcatttttg ggactgcttc 120

ttatctgatt cgtgccagac gcagtatgga tgaagctaac cagcctctcc tcactgatca 180

gtatcaatgc tatgctgnnn natatgaaaa actccagaat cctaatcagt ctgtggtcta 240

acaaatgccc tactctctta tgcattagta tcacaaaacc acctggttga atataataga 300

ttgagttatt aactgtattt tctttcactt tattaccttc tttctaatac aagcatatgt 360

tagnattaaa gttctaggca tacttttcaa agctgggaag accctttcag aatcttttca 420

atgggtttta attttcagtt ctatttaaaa tggtgaatga cactaaactc catgatattt 480

aaggatagtg tgaagatctt tggcatgatt taaaggttga gtatgtgaag atataagtaa 540

gtgaactacc atgctttgtt tacgtgtaaa ggaaaataat gtttgatagt aaatgtccac 600

ttaaaataca tgaatgggca tttctaaaat gttaaaacat aaacwcattt ccattcatgg 660

atatttgtca acagatttaa agaaaaccac agttattaat taaagaannn naattaatta 720

tgtgtagtta taaaccaatg aaattttgat taacctttc aaattaatgt tccagtttga 780

agaccaatca aatatattat ttagtcaaca t 811

<210> 45
<211> 996
<212> DNA
<213> Homo sapiens 217486

<220>
<221> misc_feature
<222> (473)..(473)
<223> n = a or t

<220>
<221> misc_feature
<222> (26)..(29)
<223> nt. represent aaga insertion variant

<220>
<221> misc_feature
<222> (536)..(539)
<223> nt. represent aatt insertion variant

<400> 45
actgatcagt atcaatgcta tgctgnnnna tatgaaaaac tccagaatcc taatcagtct 60

gtggtctaac aaatgcccta ctctcttatg cattagtatc acaaaaccac ctggttgaat 120

ataatagatt gagttattaa ctgtattttc tttcacttta ttaccttctt tctaatacaa 180

gcatatgtta gmattaaagt tctaggcata cttttcaaag ctgggaagac cctttcagaa 240

tcttttcaat gggttttaat tttcagttct atttaaaatg gtgaatgaca ctaaactcca 300

tgatatttaa ggatagtgtg aagatctttg gcatgattta aaggttgagt atgtgaagat 360

ataagtaagt gaactaccat gctttgttta cgtgtaaagg aaaataatgt ttgatagtaa 420

atgtccactt aaaatacatg aatgggcatt tctaaaatgt taaaacataa acncatttcc 480

attcatggat atttgtcaac agatttaaag aaaaccacag ttattaatta aagaannnna 540

attaattatg tgtagttata aaccaatgaa attttgatta accttttcaa attaatgttc 600

cagtttgaag accaatcaaa tatattattt agtcaacata tactatttag tctcaggttc 660

aaggctacaa caaaaatcac catctttgtc aaactttgga gagggaaaat cttcactttc 720

ttaagcaaca atggatattg cctgtgtttg ccactgtgtt tccctgcctc tcaattcgct 780

gaaaaaggaa ctacctatcc ttacatttca cctactaatg tctcttctaa catcttagag 840

gtccatggag aaggcatatg gagaacatgt tttatactgc tctataaata gtattccaat 900

cactgtgctt aatttaaata gcattmtctt atcatttatc agccttttat gtattttcca 960

agtaaaatat taacatatta yttcattggt cttctt 996

<210> 46
<211> 560
<212> DNA
<213> Homo sapiens 869787

<220>
<221> misc_feature
<222> (314)..(314)
<223> n = g or t

<400> 46
ctgtgtctgg gcctgggaca gaccgctgtg gctcatcatc agggaggggc agatgtgagg 60

cagtgactgc agactccygg ccccacagcc ctcagtatcc ccatgatggc agagatgatc 120

gggaggtctg gcccttgcgc ttcttcaata ggacatgtca ctgcaacggc aatttctcag 180

gacacaactg tgggacgtgc cgtcctggct ggagaggagc tgcctgtgac cagagggttc 240

tcataggtaa gtggagatat gaatgagttc ataagtcctg catgagactc aaggctctta 300

ataaaatctt aaancatttg agctggagga atacctggaa atcatatagc tcaaccctct 360

cttttcatag ttgaggaaac tgaggcttag aaaggttaag aaacttgttt aatgtaaagg 420

gttggagttg aagctcagaa cttctgatca atattttatt ctgaaacatt tattgagcaa 480

ccactatatc ctaggaactg tgttaggtat tatgactagt caattcagta actccttcag 540

gtaaacatgt taattgtcat 560

<210> 47
<211> 490
<212> DNA
<213> Homo sapiens 869745

<220>
<221> misc_feature
<222> (224)..(224)
<223> n = t or c

<400> 47
attaattatc aggcagcaat ccacatgcac ttaacagttc tgacgtgaga ggacaagaaa 60

cacaagcaaa tataaaacat tcaattctaa gagaagttca tcagagacat ccttcaggat 120

tgtgaggtac tggaaagaag tcctatgggg agtgggtgga cacgtgccaa aactccatta 180

gtgtaaggga ctttaaatca cagaaattaa cttgctggaa atcngttccc aattcttcct 240

tcagctccaa ggttaaatta aatgtaatta atgatggtga cctgctaatt catgcttttg 300

ataactgata tctagtatgt atatatatat aaacaaaatg acgaggacag ggaatttaat 360

tatttgggta tcacacatgy aggtgttata tatgccaaat tttaaaggta aawtactact 420

tttattattt gtgtgaaatg tcattttaca tatgggttcc attttgaaag tggtttggga 480

agggggcata 490

<210> 48
<211> 350
<212> DNA
<213> Homo sapiens 886933

<220>
<221> misc_feature
<222> (169)..(169)
<223> n = t or c

<400> 48
aatcattttc agaaatgtct gcataatgag ttgagtttca ttccctctaa tgcctaaatg 60

acaccttgta ataaattacc agctttgtta aataaggttt taactcctct gggcccctca 120

gacaccgttg atatactaac cagtacctta ttgtctgaag agagctaana gaaatagact 180

gtcagagagt agaccaaaca gaaatgaata attgtaaaca gaagcagaga gtattaatgt 240

ggtttctgtg atctaggaaa tgttgcaaga gccttcyttc tcccttcctt actggaattt 300

tgcaacgggg aaaaatgtct gtgatatctg cayggatgac ttgatgggat 350

<210> 49
<211> 420
<212> DNA
<213> Homo sapiens 886937

<220>
<221> misc_feature
<222> (214)..(214)
<223> n = g or t

<400> 49
ctccttggaa gattatgata ccctgggaac actttgtaac agtaagttcc aaatgatagc 60

ttggagtcag aatttctttt tagataawga gattaaatat gttgcctgaa aggccttcat 120

tctactagag aattcagact aaaatctact tttattatag agtaacagtg taccaggcat 180

tcattaaaca cctagaatgt tcaaggtact ctanaagttg ctccagggga aacagaaagt 240

gcctacacat ttttacactg cctttcttga gtagtttggt caatatcttg ctaactttct 300

tattttggaa atgtctagtt gtataaacta atcctcttag ttttcttagc actacttaga 360

agtcatgtgt cttgtgttgg aatttcacag aaaatgtttc ctaagaaaat gtgaaaaata 420

<210> 50
<211> 1680
<212> DNA
<213> Homo sapiens 886942

<220>
<221> misc_feature
<222> (903)..(903)
<223> n = a or g

<400> 50
atatatatta ttttcaaatc tactatttcc tggtcagtat tcaaggtacc agaaatacga 60
tgctatacaa aattcaccaa caaattttct tcctgaaact tttcttcctg ttgataaaat 120
tgacaataac catatgtaaa tacatataca gcatgttaga tggtggaagt ctctatagac 180
aaataaaatc aggaaatagg ataggcagta ctgtgtagat atatggaaga agaatgtcct 240
aagacaaagg aacagggagc caactgtggc tggagtagag tggggtctgg ggagagagtg 300
gtgagagata gtaaggtcaa agggataaca ggaggcagag ctgtatgcca cagcaaatta 360
caggtttatg ttttctttgg ctttgaaaaa tacatttaat gaattttatt caaattgctt 420
tatgtaattt ttaaaaatta ttcaagatca ccataggtga tgaaatacta aaactcccct 480
gcttttaaac tctctttttt attaagtggt atattggtac tgtattcaaa gcattttctg 540
ttttatgtaa tttctcatcc tgctgtagtg aaacttcata tctttattca gtgtaaaata 600
agaataaaat tttttcaggt tctccttgaa tattggatgc ctttagaact caataatgat 660
aggaatatta attwtattat gtttattaat acgttgtctt tggaataatt tagatatatc 720
cacatttcca ttggaaaatg cccctattgg acataataga caatacaaca tggtgccatt 780
ctggccccca gtcaccaaca cagaaatgtt tgttaytgct ccagacaacc tgggatacac 840
ttatgaaatt caatggccaa gtgagtgttg aaagtgtatt tttactgtga taatttccaa 900
aancaaatgt gttatctttc aagtagagta atcacggtat tctgaagcta tgttttccat 960
ttggacttgg aaactttcat ttgtaytttt atttgaggat aagggaagga atttgatatt 1020
tgttgagagt ccacactaag ctgatattga tcttattgta cagcacccta tctcatttaa 1080
tcctcacaat gctttggggt gagtatgaaa atcttcattt cacaaataag gaagctgagg 1140
cttaaatagg ttaactgtta cagattcaca tttctaatga gggaagagaa tgagtttgag 1200
cttaggccca tggaatatgc cccaattttt ctactatacc atattgcctg catttatcta 1260
tcttaaagga aaagggagtg agatactctt aggtattttt ctgagatttt gaagttcaaa 1320
agttttttgt ttaaatcttt tccccaacaa aggcagtagg gcatagtgga aaagtacaag 1380
acttatattc taaaatatcc gggctccaaa gccaacttta ttgcttacca accaagtgac 1440
ctgggtaagt gactcagact cattgagtca tcattctctc actttcacaa aatgaaatgg 1500
aaataacaat gactatccca atagggtcca ctcattaaaa gaaatcagga agtggcttca 1560
aggccatgtg gccaatgtaa attaaatatg aggatttctg ttaaaataga catttctaaa 1620
tttcatgtgt ccactttttg gtgataacta ttttaatatt tgtcttttta tttttaatct 1680

<210> 51
<211> 464
<212> DNA
<213> Homo sapiens 217459

<220>
<221> misc_feature
<222> (207)..(207)
<223> n = g or t

<400> 51
ggtttccttg taacacagta ctttgccatt ttctttcaag ttcgagaggt tacatttttc 60

atcctcgtga aatctgtcgt gattccagtt gcgtaggtta tgacacgctg caggagtcag 120

aaggttgtgc agagtaaatg agctgtggtt tctctcttac agcataggat atctgacggg 180

attctgctcg ccaaatgcct gacagtnttg ggatttgtta tcttcatgtt tttcctcaat 240

tcgtttgtcc ctggcattca tcttgatctt ggtgagtcta atttagcttt ggttcatagg 300

ctttgtcaca ttctggatgg gaaggtttca gagcctgttc ccagacactg actttgccca 360

caggcagccg ggctggtggg aggccagaga gggctgagat ggagggtggg cagcctgccc 420

tgggaagaag ggcgcctttc cttttggttt cctgggcagg aggg 464

<210> 52
<211> 659
<212> DNA
<213> Homo sapiens 217460

<220>
<221> misc_feature
<222> (428)..(428)
<223> n = a or c

<400> 52
aaaattaagc caatctatag tgaaagaaaa gagatgaatg gtttactggg agtgtggggg 60

ttgacaagag gagctagagg agggtacaag aacataggca tgaataaact gtagggtcaa 120

tgggcatgtt cattatcttg attacagtgt tggtttcatg agtatacaca taaccaaata 180

gaaattatat gcattttaca tatatgcggt ttgtagtatg acaattattc ttgataaaga 240

aaaaggcaac cagaggttaa agaaatgaat cggtgtgtta acagtggaac tatatctcta 300

tgtctattta cttattttca ggatggattg ctattctggg tgccatctgg ttgctaattt 360

tagctgatat tcatgatttt gagataattc tacacagagt ggaatgggca acccttctgt 420

tttttgcngc gctctttgtt ctgatggagg taagatttta gaacttttgc catatggcat 480

tttacctgat ttttgtattt catgttttat ttggtgaatg aagaaagcct acatctatta 540

atctttcctt atattctcta agtggaaaac aatggaggtt gtaattggac tattttaagt 600

taaccagctt taccttagcc actgagagat ttctgacagc actgcgtatt tgttttttt 659

<210> 53
<211> 940
<212> DNA
<213> Homo sapiens 217487

<220>
<221> misc_feature
<222> (422)..(422)
<223> n represents aatt sequence or none

<400> 53
ttgaatataa tagattgagt tattaactgt attttctttc actttattac cttctttcta 60

atacaagcat atgttagmat taaagttcta ggcatacttt tcaaagctgg gaagaccctt 120

tcagaatctt ttcaatgggt tttaattttc agttctattt aaaatggtga atgacactaa 180

actccatgat atttaaggat agtgtgaaga tctttggcat gatttaaagg ttgagtatgt 240

gaagatataa gtaagtgaac taccatgctt tgtttacgtg taaaggaaaa taatgtttga 300

tagtaaatgt ccacttaaaa tacatgaatg ggcatttcta aaatgttaaa acataaacwc 360

atttccattc atggatattt gtcaacagat ttaaagaaaa ccacagttat taattaaaga 420

anaattaatt atgtgtagtt ataaaccaat gaaattttga ttaacctttt caaattaatg 480

ttccagtttg aagaccaatc aaatatatta tttagtcaac atatactatt tagtctcagg 540

ttcaaggcta caacaaaaat caccatcttt gtcaaacttt ggagagggaa aatcttcact 600

ttcttaagca acaatggata ttgcctgtgt ttgccactgt gtttccctgc ctctcaattc 660

gctgaaaaag gaactaccta tccttacatt tcacctacta atgtctcttc taacatctta 720

gaggtccatg gagaaggcat atggagaaca tgttttatac tgctctataa atagtattcc 780

aatcactgtg cttaatttaa atagcattmt cttatcattt atcagccttt tatgtatttt 840

ccaagtaaaa tattaacata ttayttcatt ggtcttcttt tttatctggt tctatatgaa 900

tgctattttt tcccttctct tctaacatga aatatatttt 940

<210> 54
<211> 751
<212> DNA
<213> Homo sapiens 217489

<220>
<221> misc_feature
<222> (459)..(459)
<223> n = t or c

<220>
<221> misc_feature
<222> (14)..(17)
<223> nt. represent aatt insertion variant

<220>
<221> misc_feature
<222> (568)..(568)
<223> n represents nucleotide "a" insertion variant

<400> 54
attaattaaa gaannnnaat taattatgtg tagttataaa ccaatgaaat tttgattaac 60

cttttcaaat taatgttcca gtttgaagac caatcaaata tattatttag tcaacatata 120

ctatttagtc tcaggttcaa ggctacaaca aaaatcacca tctttgtcaa actttggaga 180

gggaaaatct tcactttctt aagcaacaat ggatattgcc tgtgtttgcc actgtgtttc 240

cctgcctctc aattcgctga aaaaggaact acctatcctt acatttcacc tactaatgtc 300

tcttctaaca tcttagaggt ccatggagaa ggcatatgga gaacatgttt tatactgctc 360

tataaatagt attccaatca ctgtgcttaa tttaaatagc attmtcttat catttatcag 420

ccttttatgt attttccaag taaaatatta acatattant tcattggtct tcttttttat 480

ctggttctat atgaatgcta tttttccct tctcttctaa catgaaatat attttctctt 540

tttgatcttg tgctatgaaa caatcttncc aaagaactgt ataaggtggt cataagtgaa 600

tattttaatt aaaattggta aaaataaata ataacagtaa taatcatgca ctatagaaaa 660

tggctaaact gagattctaa attctacaaa cagaaacaag tttaagttat gtatccctga 720

ttggttactg ggttttccta tattcaaaaa t 751

<210> 55
<211> 2940
<212> DNA
<213> Homo sapiens 554353

<220>
<221> misc_feature
<222> (1528)..(1528)
<223> n = g or a

<400> 55
gtatcatata taattgtatg tgctatactt tttatatgac tggcaacaca ggtttgcttc 60

catcagcatc accatcaaca tgtgagcaat gctatgacat cattaagtga tagggatttt 120

tcggctccat tatgatctca tgggactacc atcatataca tggtctgtta ttgaccaaag 180

catcattatg caggacatga ctctacatac taattgttca tgagcaataa cactgggaaa 240

actgtctcaa agcataattc taattctggg tcagcaaaat cctcttcaga aataaacaca 300

taaacaaaaa tcattgcttg gtaatgttaa tttaagattt gattatctgt ttcaaattcc 360

ttgttattca caaaaagaat atacaatata ctttgtattt tcttctcctg tacttggtaa 420

catgagctaa ggatacaatg agataacaga caagtccact ctgaggaatc ctaagctgtt 480

ccctacagtc aactcctatg caggtgttca gactttgtaa caagaaaaca gcatctccta 540

tcaaatgatg attccacaat catgaatata caatcgtttt tctatgtggc agttatttga 600

gacatatgga aaggccataa tttctctgtc tagcaggcat tcaatcccaa gatggtaagc 660

atcctcctat taaaaaaggg ctacataatt ctctcatacc ttaaaaatcc taaaatagtc 720

aaaatacaag gcttcgtgtt atttcactca atttttgtta ctatatccta aagaacagtt 780

ttctgagtta tagaagatag tcaaaatcag aaataattac tttaaaatgt tactttcctt 840

tatcaaatta tttggtagca gatttttaaa gctgaaatca aagataacca aagaaaattg 900

gcttgttttt tctataactt aatgttaaac taaatttggg ctgaagatgc ctctgctcct 960

taagtcctta ccaaggaatt gcaacttcat ttactatata agctaacgga aagcctaaca 1020

gtagaattaa acttttgtaa ccaaatagct gagtctcagt cagtcacagg tggccaacta 1080

atcagatcat cttcaaataa ggcaaatccc aagctgtaat caatcaagcc atttctgtac 1140

ctcacttgca ttttctgttc ataaatgctc cagcccatgt ttttaagtga gctctctgaa 1200

gctctctggt tctgagggtt gcctgatttg ggaatagttc tctgctgaat tatattttgc 1260

taaattaaat ttgcctcaag cttttattgt aacaatagta aaagccagta tcataaaatt 1320

ttgtacctta ccagaaaacc agatgaatta tccagcagac accttagacg acatatgaag 1380

cacctctcca ttaaaggaga gttttctgga ggaatctggt ctgggttata acagactact 1440

gtctggggga gaccagtggc ttctgtagaa ataaaggcaa aacaaaattt actttccatt 1500

ttgctgtaaa attaagcact tgaatagnta aaatttgyat tagtttctaa actggagtct 1560

gtgttctgaa tacaaaaaaa tcattttcaa aagagctaat gacaagaaac taaaagttta 1620

ttaaatatca cttttatcag atagaacttg cataattttt taaattttta ttgagctatc 1680

actgtcaaca attttcatat tttctaatgc atttttgttt tcttaacact ttaacaaatt 1740

tccaaaaaca ttttttgcat tgagaaaagc atttttatac ttgcatatgc tagcttactt 1800

ttttctttgg tgcattttaa acacaataat ttacatgcac tcaggtatca accttgtacc 1860

ttccttccat ctttcatgaa ggtactactt atttttataa aatacattac tgactatggc 1920

aatactctgt tgcctgaaac tcattaaatg gctctcattt gccaaaatat tccttagctc 1980

atcaggctct ttgtgaccta gctgtggcct atgattgcag actttacatc tttccactac 2040

ttccctatca tggagttcta tgaccctaca ggttcccagc ctcctttcac agactcatat 2100

ttaaaaacta ccaacactat tttttgtacc atcaattctt accttcaatt ccttgtccag 2160

actctgtaca ctgagaagga tttaatgccc agtgtagctg acgctgaaat tcagctcggt 2220

cttcggtatg gataagttca tatacactct gatgtatgac atcagactaa agaaaaaaga 2280

tacaaggaat acaacaaaag gactgattaa aaaatatggc taaattaatt ttaaagccta 2340

tttagattag tgggtgcttg ctagtggtga tgaatacgaa ttgaatgatt cctaaaattc 2400

cctagtcacc taagatagaa cctaaggtag aaaaagctaa agagactggc actacaacag 2460

ataagccaaa agttacttgt catcagttcc caacatgaat gctcttgctc aaataagtca 2520

catccccatt ccttaaatgc aacatattaa aatctgttta tgttccatat tcatttgtta 2580

gtttttagat tcttatctct tccatgcagc ttttcctaac caccccaggc ctctttgact 2640

tctctaagct taatggtatt ctttatgtat ttccttcact gttctctaaa attttcttca 2700

tataaaattt tcactccaag tagactccac aagagcaagg gattgtgcct ttaccatatt 2760

aaacccactt tccttctctt gcctctcacc atttactacg gtgctgagtc cttagtgctt 2820

aaaccagagt tttccttttt ttttaactgc aagttatgat aaaaaataca tttccatctg 2880

tcacaggtgt gtgcacagac ataaacacat ggaaaagttt cacaaaacac ttaccattat 2940

<210> 56
<211> 2170
<212> DNA
<213> Homo sapiens 554363

<220>
<221> misc_feature
<222> (1093)..(1093)
<223> n = t or c

<400> 56
gacatctcag acatggtcgt gggagaggtg tgcccgggtc agggggcacc aggagaggcc 60

aaggactctg tacctcctat ccacgtcaga gatttcgatt ttaggtttct cctctgggca 120

aggagagagg gtggaggctg gcacttgggg agggacttgg tgaggtcagt ggtaaggaca 180

```
ggcaggccct gggtctacct ggagatggct ggggcctgag acttgtccag gtgaacgcag      240
agcacaggag ggattgagac cccgttctgt ctggtgtagg tgctgaatgc tgtccccgtc      300
ctcctgcata tcccagcgct ggctggcaag gtcctacgct tccaaaaggc tttcctgacc      360
cagctggatg agctgctaac tgagcacagg atgacctggg acccagccca gcccccccga      420
gacctgactg aggccttcct ggcagagatg gagaaggtga gagtggctgc cacggtgggg      480
ggcaagggtg gtgggttgag cgtcccagga ggaatgaggg gaggctgggc aaaaggttgg      540
accagtgcat cacccggcga gccgcatctg ggctgacagg tgcagaattg gaggtcattt      600
gggggctacc ccgttctgtc ccgagtatgc tctcggccct gctcaggcca aggggaaccc      660
tgagagcagc ttcaatgatg agaacctgcg catagtggtg gctgacctgt tctctgccgg      720
gatggtgacc acctcgacca cgctggcctg gggcctcctg ctcatgatcc tacatccgga      780
tgtgcagcgt gagcccatct gggaaacagt gcaggggccg agggaggaag ggtacaggcg      840
ggggcccatg aactttgctg ggacacccgg ggctccaagc acaggcttga ccaggatcct      900
gtaagcctga cctcctccaa cataggaggc aagaaggagt gtcagggccg gaccccctgg      960
gtgctgaccc attgtgggga cgcrtgtctg tccaggccgt gtccaacagg agatcgacra     1020
cgtgataggg caggtgyggy gaccagagat gggtgaccwg gctcrcatgc cctrcaycac     1080
tgccgtgatt cangaggtgc agcgctttgg ggacatcgtc cccctgggtg tgacccatat     1140
gacatcccgt gacatcgaag tacagggctt ccgcatccct aaggtaggcc tggcrccctc     1200
ctcaccccag ctcagcacca gcmcctggtg atagccccag catggcyact gccaggtggg     1260
cccastctag gaamcctggc caccyagtcc tcaatgccac cacactgact gtccccactt     1320
gggtgggggg tccagagtat aggcagggct ggcctgtcca tccagagccc ccgtctagtg     1380
gggagacaaa ccaggacctg ccagaatgtt ggaggaccca acgcctgcag ggagaggggg     1440
cagtgtgggt gcctctgaga ggtgtgactg cgccctgctg tggggtcgga gagggtactg     1500
tggagcttct cgggcgcagg actagttgac agagtccagc tgtgtgccag gcagtgtgtg     1560
tcccccgtgt gtttggtggc aggggtccca gcatcctaga gtccagtccc cactctcacc     1620
ctgcatctcc tgcccaggga acgacactca tcaccaacct gtcatcggtg ctgaaggatg     1680
aggccgtctg ggagaagccc ttccgcttcc accccgaaca cttcctggat gcccagggcc     1740
actttgtgaa gccggaggcc ttcctgcctt tctcagcagg tgcctgtggg gagcccggct     1800
ccctgtcccc ttccgtggag tcttgcaggg gtatcaccca ggagccaggc tcactgacgc     1860
ccctcccctc cccacaggcc gccgtgcatg cctcggggag cccctggccc gcatggagct     1920
cttcctcttc ttcacctccc tgctgcagca cttcagcttc tcggtgccca ctggacagcc     1980
ccggcccagc caccatggtg tctttgcttt cctggtgagc ccatccccct atgagctttg     2040
tgctgtgccc cgctagaatg gggtacctag tccccagcct gctccctagc cagaggctct     2100
aatgtacaat aaagcaatgt ggtagttcca actcgggtcc cctgctcacg ccctcgttgg     2160
gatcatcctc                                                            2170
```

<210> 57
<211> 2170
<212> DNA
<213> Homo sapiens 554368

<220>
<221> misc_feature
<222> (1274)..(1274)
<223> n = a or c

<400> 57
gacatctcag acatggtcgt gggagaggtg tgcccgggtc agggggcacc aggagaggcc 60

aaggactctg tacctcctat ccacgtcaga gatttcgatt ttaggtttct cctctgggca 120

aggagagagg gtggaggctg gcacttgggg agggacttgg tgaggtcagt ggtaaggaca 180

ggcaggccct gggtctacct ggagatggct ggggcctgag acttgtccag gtgaacgcag 240

agcacaggag ggattgagac cccgttctgt ctggtgtagg tgctgaatgc tgtccccgtc 300

ctcctgcata tcccagcgct ggctggcaag gtcctacgct tccaaaaggc tttcctgacc 360

cagctggatg agctgctaac tgagcacagg atgacctggg acccagccca gcccccccga 420

gacctgactg aggccttcct ggcagagatg gagaaggtga gagtggctgc cacggtgggg 480

ggcaagggtg gtgggttgag cgtcccagga ggaatgaggg gaggctgggc aaaaggttgg 540

accagtgcat cacccggcga gccgcatctg ggctgacagg tgcagaattg gaggtcattt 600

gggggctacc ccgttctgtc ccgagtatgc tctcggccct gctcaggcca aggggaaccc 660

tgagagcagc ttcaatgatg agaacctgcg catagtggtg gctgacctgt tctctgccgg 720

gatggtgacc acctcgacca cgctggcctg ggcctcctg ctcatgatcc tacatccgga 780

tgtgcagcgt gagcccatct gggaaacagt gcaggggccg agggaggaag ggtacaggcg 840

ggggcccatg aactttgctg ggacacccgg ggctccaagc acaggcttga ccaggatcct 900

gtaagcctga cctcctccaa cataggaggc aagaaggagt gtcagggccg gaccccctgg 960

gtgctgaccc attgtgggga cgcrtgtctg tccaggccgt gtccaacagg agatcgacra 1020

cgtgataggg caggtgyggy gaccagagat gggtgaccwg gctcrcatgc cctrcaycac 1080

tgccgtgatt caygaggtgc agcgctttgg ggacatcgtc cccctgggtg tgacccatat 1140

gacatcccgt gacatcgaag tacagggctt ccgcatccct aaggtaggcc tggcrccctc 1200

ctcaccccag ctcagcacca gcmcctggtg atagccccag catggcyact gccaggtggg 1260

cccastctag gaancctggc caccyagtcc tcaatgccac cacactgact gtccccactt 1320

gggtgggggg tccagagtat aggcagggct ggcctgtcca tccagagccc ccgtctagtg 1380

gggagacaaa ccaggacctg ccagaatgtt ggaggaccca acgcctgcag ggagaggggg 1440

cagtgtgggt gcctctgaga ggtgtgactg cgccctgctg tggggtcgga gagggtactg 1500

tggagcttct cgggcgcagg actagttgac agagtccagc tgtgtgccag gcagtgtgtg 1560

tcccccgtgt gtttggtggc aggggtccca gcatcctaga gtccagtccc cactctcacc 1620

ctgcatctcc tgcccaggga acgacactca tcaccaacct gtcatcggtg ctgaaggatg 1680

aggccgtctg ggagaagccc ttccgcttcc accccgaaca cttcctggat gcccagggcc 1740
actttgtgaa gccggaggcc ttcctgcctt tctcagcagg tgcctgtggg gagcccggct 1800
ccctgtcccc ttccgtggag tcttgcaggg gtatcaccca ggagccaggc tcactgacgc 1860
ccctcccctc cccacaggcc gccgtgcatg cctcggggag cccctggccc gcatggagct 1920
cttcctcttc ttcacctccc tgctgcagca cttcagcttc tcggtgccca ctggacagcc 1980
ccggcccagc caccatggtg tctttgcttt cctggtgagc ccatccccct atgagctttg 2040
tgctgtgccc cgctagaatg gggtacctag tccccagcct gctccctagc cagaggctct 2100
aatgtacaat aaagcaatgt ggtagttcca actcgggtcc cctgctcacg ccctcgttgg 2160
gatcatcctc 2170

<210> 58
<211> 2170
<212> DNA
<213> Homo sapiens 554370

<220>
<221> misc_feature
<222> (1024)..(1024)
<223> n = g or a

<400> 58
gggaggcagg gggtccactt gatgtcgaga ctgcagtgag ccatgatcct gccactgcac 60
tccggcctgg gcaacagagt gagaccctgt ctaaagaaaa aaaaaataaa gcaacatatc 120
ctgaacaaag gatcctccat aacgttccca ccagatttct aatcagaaac atggaggcca 180
gaaagcagtg gaggaggacg accctcaggc agcccgggag gatgttgtca caggctgggg 240
caagggcctt ccggctacca actgggagct ctgggaacag ccctgttgca aacaagaagc 300
catagcccgg ccagagccca ggaatgtggg ctgggctggg agcagcctct ggacaggagt 360
ggtcccatcc aggaaacctc cggcatggct gggaagtggg gtacttggtg ccgggtctgt 420
atgtgtgtgt gactggtgtg tgtgagagag aatgtgtgcc ctaagtgtca gtgtgagtct 480
gtgtatgtgt gaatattgtc tttgtgtggg tgattttctg cgtgtgtaat cgtgtccctg 540
caagtgtgaa caagtggaca agtgtctggg agtggacaag agatctgtgc accatcaggt 600
gtgtgcatag cgtctgtgca tgtcaagagt gcaaggtgaa gtgaagggac caggcccatg 660
atgccactca tcatcaggag ctctaaggcc ccaggtaagt gccagtgaca gataagggtg 720
ctgaaggtca ctctggagtg ggcaggtggg ggtagggaaa gggcaaggcc atgttctgga 780
ggaggggttg tgactacatt agggtgtatg agcctagctg ggaggtggat ggccgggtcc 840
actgaaaccc tggttatccc agaaggcttt gcaggcttca ggagcttgga gtggggagag 900
ggggtgactt ctccgaccag gcccctccac cggcctaccc tgggtaaggg cctggagcag 960
gaagcagggg caagaacctc tggagcagcc catacccgcc ctggcctgac tctgccactg 1020
gcancacagt caacacagca ggttcactca cagcagaggg caaaggccat catcagctcc 1080
ctttataagg gaagggtcac gcgctcggtg tgctgagagt gtcctgcctg gtcctctgtg 1140

cctggtgggg tgggggtgcc aggtgtgtcc agaggagccc atttggtagt gaggcaggta 1200

tggggctaga agcactggtg cccctggccg tgatagtggc catcttcctg ctcctggtgg 1260

acctgatgca ccggcgccaa cgctgggctg cacgctaccc accaggcccc ctgccactgc 1320

ccgggctggg caacctgctg catgtggact tccagaacac accatactgc ttcgaccagg 1380

tgagggagga ggtcctggag ggcggcagag gtgctgaggc tcccctacca gaagcaaaca 1440

tggatggtgg gtgaaaccac aggctggacc agaagccagg ctgagaaggg gaagcaggtt 1500

tgggggacgt cctggagaag ggcatttata catggcatga aggactggat tttccaaagg 1560

ccaaggaaga gtagggcaag ggcctggagg tggagctgga cttggcagtg ggcatgcaag 1620

cccattgggc aacatatgtt atggagtaca aagtcccttc tgctgacacc agaaggaaag 1680

gccttgggaa tggaagatga gttagtcctg agtgccgttt aaatcacgaa atcgaggatg 1740

aagggggtgc agtgacccgg ttcaaacctt ttgcactgtg ggtcctcggg cctcactgcc 1800

tcaccggcat ggaccatcat ctgggaatgg gatgctaact ggggcctctc ggcaattttg 1860

gtgactcttg caaggtcata cctgggtgac gcatccaaac tgagttcctc catcacagaa 1920

ggtgtgaccc ccaccccccgc cccacgatca ggaggctggg tctcctcctt ccacctgctc 1980

actcctggta gccccggggg tcgtccaagg ttcaaatagg actaggacct gtagtctggg 2040

gtgatcctgg cttgacaaga ggccctgacc ctccctctgc agttgcggcg ccgcttcggg 2100

gacgtgttca gcctgcagct ggcctggacg ccggtggtcg tgctcaatgg gctggcggcc 2160

gtgcgcgagg 2170

<210> 59
<211> 2240
<212> DNA
<213> Homo sapiens 554371

<220>
<221> misc_feature
<222> (1159)..(1159)
<223> n = t or c

<400> 59
aacgttccca ccagatttct aatcagaaac atggaggcca gaaagcagtg gaggaggacg 60

accctcaggc agcccgggag gatgttgtca caggctgggg caagggcctt ccggctacca 120

actgggagct ctgggaacag ccctgttgca aacaagaagc catagcccgg ccagagccca 180

ggaatgtggg ctgggctggg agcagcctct ggacaggagt ggtcccatcc aggaaacctc 240

cggcatggct gggaagtggg gtacttggtg ccgggtctgt atgtgtgtgt gactggtgtg 300

tgtgagagag aatgtgtgcc ctaagtgtca gtgtgagtct gtgtatgtgt gaatattgtc 360

tttgtgtggg tgattttctg cgtgtgtaat cgtgtccctg caagtgtgaa caagtggaca 420

agtgtctggg agtggacaag agatctgtgc accatcaggt gtgtgcatag cgtctgtgca 480

tgtcaagagt gcaaggtgaa gtgaagggac caggcccatg atgccactca tcatcaggag 540

ctctaaggcc ccaggtaagt gccagtgaca gataagggtg ctgaaggtca ctctggagtg 600

ggcaggtggg ggtagggaaa gggcaaggcc atgttctgga ggaggggttg tgactacatt 660

agggtgtatg agcctagctg ggaggtggat ggccgggtcc actgaaaccc tggttatccc 720

agaaggcttt gcaggcttca ggagcttgga gtggggagag ggggtgactt ctccgaccag 780

gcccctccac cggcctaccc tgggtaaggg cctggagcag gaagcagggg caagaacctc 840

tggagcagcc catacccgcc ctggcctgac tctgccactg gcagcacagt caacacagca 900

ggttcactca cagcagaggg caaaggccat catcagctcc ctttataagg gaagggtcac 960

gcgctcggtg tgctgagagt gtcctgcctg gtcctctgtg cctggtgggg tgggggtgcc 1020

aggtgtgtcc agaggagccc atttggtagt gaggcaggta tggggctaga agcactggtg 1080

cccctggccg tgatagtggc catcttcctg ctcctggtgg acctgatgca ccggcgccaa 1140

cgctgggctg cacgctacnc accaggcccc ctgccactgc ccgggctggg caacctgctg 1200

catgtggact tccagaacac accatactgc ttcgaccagg tgagggagga ggtcctggag 1260

ggcggcagag gtgctgaggc tcccctacca gaagcaaaca tggatggtgg gtgaaaccac 1320

aggctggacc agaagccagg ctgagaaggg gaagcaggtt tgggggacgt cctggagaag 1380

ggcatttata catggcatga aggactggat tttccaaagg ccaaggaaga gtagggcaag 1440

ggcctggagg tggagctgga cttggcagtg ggcatgcaag cccattgggc aacatatgtt 1500

atggagtaca aagtcccttc tgctgacacc agaaggaaag gccttgggaa tggaagatga 1560

gttagtcctg agtgccgttt aaatcacgaa atcgaggatg aagggggtgc agtgacccgg 1620

ttcaaacctt ttgcactgtg ggtcctcggg cctcactgcc tcaccggcat ggaccatcat 1680

ctgggaatgg gatgctaact ggggcctctc ggcaattttg gtgactcttg caaggtcata 1740

cctgggtgac gcatccaaac tgagttcctc catcacagaa ggtgtgaccc ccaccccccgc 1800

cccacgatca ggaggctggg tctcctcctt ccacctgctc actcctggta gccccggggg 1860

tcgtccaagg ttcaaatagg actaggacct gtagtctggg gtgatcctgg cttgacaaga 1920

ggccctgacc ctccctctgc agttgcggcg ccgcttcggg gacgtgttca gcctgcagct 1980

ggcctggacg ccggtggtcg tgctcaatgg gctggcggcc gtgcgcgagg cgctggtgac 2040

ccacggcgag gacaccgccg accgcccgcc tgtgcccatc acccagatcc tgggtttcgg 2100

gccgcgttcc caaggcaagc agcggtgggg acagagacag atttccgtgg gacccgggtg 2160

ggtgatgacc gtagtccgag ctgggcagag agggcgcggg gtcgtggaca tgaaacaggc 2220

cagcgagtgg ggacagcggg 2240

<210> 60
<211> 1050
<212> DNA
<213> Homo sapiens 615921

<220>
<221> misc_feature
<222> (484)..(484)
<223> n = a or c

<400> 60

tttgcataga tgggtttggg aaaggacatt ccaggagacc ccactgtaag aagggcctgg 60
aggaggaggg gacatctcag acatggtcgt gggagaggtg tgcccgggtc agggggcacc 120
aggagaggcc aaggactctg tacctcctat ccacgtcaga gatttcgatt ttaggtttct 180
cctctgggca aggagagagg gtggaggctg gcacttgggg agggacttgg tgaggtcagt 240
ggtaaggaca ggcaggccct gggtctacct ggagatggct ggggcctgag acttgtccag 300
gtgaacgcag agcacaggag ggattgagac cccgttctgt ctggtgtagg tgctgaatgc 360
tgtccccgtc ctcctgcaya tcccagcgct ggctggcaag gtcctacgct tccaaaaggc 420
tttcctgacc cagctggatg agctgctaac tgagcacagg atgacctggg acccagccca 480
gcmnccccga gacctgactg aggccttcct ggcagagatg gagaaggtga gagtggctgc 540
cacggtgggg ggcaagggtg gtgggttgar cgtcccagga ggaatgaggg gaggctgggc 600
aaaaggttgg accagtgcat cacccggcga gccgcatctg ggctgacagg tgcaraattg 660
gaggtcaytt gggggctacc ccgttctgtc ccgagtatgc tctcggccct gctcaggcca 720
aggggaaccc tgagagcagc ttcaatgatg agaacctgyg catagtggtg gctgacctgt 780
tctctgccgg gatggtgacc acctcgacca cgctggcctg gggcctcctg ytcatgatcc 840
tacatccgga tgtgcagcgt gagcccatct gggaaacagt scasgggccg agggaggaag 900
ggtacaggcg ggggcccatg aactttgctg ggacacccgg ggctccaagc acaggcttga 960
ccaggatcct gtaagcctga cctcctccaa cataggaggc aagaaggagt gtcagggccg 1020
gaccccctgg gtgctgaccc attgtgggga 1050

<210> 61
<211> 1820
<212> DNA
<213> Homo sapiens 615925

<220>
<221> misc_feature
<222> (619)..(619)
<223> n = c or t

<400> 61
tacctcctat ccacgtcaga gatttcgatt ttaggtttct cctctgggca aggagagagg 60
gtggaggctg gcacttgggg agggacttgg tgaggtcagt ggtaaggaca ggcaggccct 120
gggtctacct ggagatggct ggggcctgag acttgtccag gtgaacgcag agcacaggag 180
ggattgagac cccgttctgt ctggtgtagg tgctgaatgc tgtccccgtc ctcctgcaya 240
tcccagcgct ggctggcaag gtcctacgct tccaaaaggc tttcctgacc cagctggatg 300
agctgctaac tgagcacagg atgacctggg acccagccca gcmmccccga gacctgactg 360
aggccttcct ggcagagatg gagaaggtga gagtggctgc cacggtgggg ggcaagggtg 420
gtgggttgar cgtcccagga ggaatgaggg gaggctgggc aaaaggttgg accagtgcat 480
cacccggcga gccgcatctg ggctgacagg tgcaraattg gaggtcaytt gggggctacc 540
ccgttctgtc ccgagtatgc tctcggccct gctcaggcca aggggaaccc tgagagcagc 600

ttcaatgatg agaacctgng catagtggtg gctgacctgt tctctgccgg gatggtgacc 660

acctcgacca cgctggcctg gggcctcctg ytcatgatcc tacatccgga tgtgcagcgt 720

gagcccatct gggaaacagt scasgggccg agggaggaag ggtacaggcg ggggcccatg 780

aactttgctg ggacacccgg ggctccaagc acaggcttga ccaggatcct gtaagcctga 840

cctcctccaa cataggaggc aagaaggagt gtcagggccg gaccccctgg gtgctgaccc 900

attgtgggga cgcatgtctg tccaggccgt gtccaacagg agatcgacga cgtgataggg 960

caggtgcggc gaccagagat gggtgaccag gctcacatgc cctacaccac tgccgtgatt 1020

catgaggtgc agcgctttgg ggacatcgtc cccctgggtg tgacccatat gacatcccgt 1080

gacatcgaag tacagggctt ccgcatccct aaggtaggcc tggcgccctc ctcaccccag 1140

ctcagcacca gcacctggtg atagccccag catggctact gccaggtggg cccactctag 1200

gaaccctggc cacctagtcc tcaatgccac cacactgact gtccccactt gggtgggggg 1260

tccagagtat aggcagggct ggcctgtcca tccagagccc ccgtctagtg gggagacaaa 1320

ccaggacctg ccagaatgtt ggaggaccca acgcctgcag ggagaggggg cagtgtgggt 1380

gcctctgaga ggtgtgactg cgccctgctg tggggtcgga gagggtactg tggagcttct 1440

cgggcgcagg actagttgac agagtccagc tgtgtgccag gcagtgtgtg tcccccgtgt 1500

gtttggtggc aggggtccca gcatcctaga gtccagtccc cactctcacc ctgcatctcc 1560

tgcccaggga acgacactca tcaccaacct gtcatcggtg ctgaaggatg aggccgtctg 1620

ggagaagccc ttccgcttcc accccgaaca cttcctggat gcccagggcc actttgtgaa 1680

gccggaggcc ttcctgcctt tctcagcagg tgcctgtggg gagcccggct ccctgtcccc 1740

ttccgtggag tcttgcaggg gtatcaccca ggagccaggc tcactgacgc ccctcccctc 1800

cccacaggcc gccgtgcatg 1820

<210> 62
<211> 1050
<212> DNA
<213> Homo sapiens 615926

<220>
<221> misc_feature
<222> (551)..(551)
<223> n = t or c

<400> 62
ggggcctgag acttgtccag gtgaacgcag agcacaggag ggattgagac cccgttctgt 60

ctggtgtagg tgctgaatgc tgtccccgtc ctcctgcaya tcccagcgct ggctggcaag 120

gtcctacgct tccaaaaggc tttcctgacc cagctggatg agctgctaac tgagcacagg 180

atgacctggg acccagccca gcmmccccga gacctgactg aggccttcct ggcagagatg 240

gagaaggtga gagtggctgc cacggtgggg ggcaagggtg gtgggttgar cgtcccagga 300

ggaatgaggg gaggctgggc aaaaggttgg accagtgcat cacccggcga gccgcatctg 360

ggctgacagg tgcaraattg gaggtcaytt gggggctacc ccgttctgtc ccgagtatgc 420

tctcggccct gctcaggcca aggggaaccc tgagagcagc ttcaatgatg agaacctgyg 480

catagtggtg gctgacctgt tctctgccgg gatggtgacc acctcgacca cgctggcctg 540

gggcctcctg ntcatgatcc tacatccgga tgtgcagcgt gagcccatct gggaaacagt 600

scasgggccg agggaggaag ggtacaggcg ggggcccatg aactttgctg ggacacccgg 660

ggctccaagc acaggcttga ccaggatcct gtaagcctga cctcctccaa cataggaggc 720

aagaaggagt gtcagggccg gaccccctgg gtgctgaccc attgtgggga cgcatgtctg 780

tccaggccgt gtccaacagg agatcgacga cgtgataggg caggtgcggc gaccagagat 840

gggtgaccag gctcacatgc cctacaccac tgccgtgatt catgaggtgc agcgctttgg 900

ggacatcgtc cccctgggtg tgacccatat gacatcccgt gacatcgaag tacagggctt 960

ccgcatccct aaggtaggcc tggcgccctc ctcaccccag ctcagcacca gcacctggtg 1020

atagccccag catggctact gccaggtggg 1050

<210> 63
<211> 2170
<212> DNA
<213> Homo sapiens 664784

<220>
<221> misc_feature
<222> (1177)..(1177)
<223> n = g or a

<400> 63
cagcagaggg caaaggccat catcagctcc ctttataagg gaagggtcac gcgctcggtg 60

tgctgagagt gtcctgcctg gtcctctgtg cctggtgggg tgggggtgcc aggtgtgtcc 120

agaggagccc atttggtagt gaggcaggta tggggctaga agcactggtg cccctggccg 180

tgatagtggc catcttcctg ctcctggtgg acctgatgca ccggcgccaa cgctgggctg 240

cacgctaccc accaggcccc ctgccactgc ccgggctggg caacctgctg catgtggact 300

tccagaacac accatactgc ttcgaccagg tgagggagga ggtcctggag ggcggcagag 360

gtgctgaggc tcccctacca gaagcaaaca tggatggtgg gtgaaaccac aggctggacc 420

agaagccagg ctgagaaggg gaagcaggtt tgggggacgt cctggagaag ggcatttata 480

catggcatga aggactggat tttccaaagg ccaaggaaga gtagggcaag ggcctggagg 540

tggagctgga cttggcagtg ggcatgcaag cccattgggc aacatatgtt atggagtaca 600

aagtcccttc tgctgacacc agaaggaaag gccttgggaa tggaagatga gttagtcctg 660

agtgccgttt aaatcacgaa atcgaggatg aagggggtgc agtgacccgg ttcaaacctt 720

ttgcactgtg ggtcctcggg cctcactgcc tcaccggcat ggaccatcat ctgggaatgg 780

gatgctaact ggggcctctc ggcaattttg gtgactcttg caaggtcata cctgggtgac 840

gcatccaaac tgagttcctc catcacagaa ggtgtgaccc ccaccccgc cccacgatca 900

ggaggctggg tctcctcctt ccacctgctc actcctggta gccccggggg tcgtccaagg 960

ttcaaatagg actaggacct gtagtctggg gtgatcctgg cttgacaaga ggccctgacc 1020

ctccctctrc agytgcggcg ccgcttyggg gacgtgttca gcctgcagct ggcctggacg 1080
ccggtggtcg tgctcaatgg gctggcggcc gtgcgygagg cgctggtgac ccacggmgag 1140
gacaccgccg accgcccgcc tgygcccatc acccagntcc tgggyttcgg gccgcgytcc 1200
caaggcaagc rgcggtgggg acagagacag rtttccgtgg gaccygggtg gryrrtgacc 1260
gtagtccgag ctgggcagag agggcgyggg gtcgtggaca tgaaacaggc cagcgagtgg 1320
ggacagcggg ccaagaaacc acctgcacta gggaggtgtg agcatgggga cgagggcggg 1380
gcttgtgacg agtgggcggg gccactgccg agacctggca ggagcccaat gggtgagcgt 1440
ggcgcatttc ccagctggaa tccggtgtcg aagtgggggc ggggaccgca cctgtgctgt 1500
aagctcagtg tgggtggcgc ggggcccgcg gggtcttccc tgagtgcaaa ggcggtcagg 1560
gtgggcagag acgaggtggg gcaaagcctg ccccagccaa gggagcaagg tggatgcaca 1620
aagagtgggc cctgtgacca gctggacaga gccagggact gcgggagacc agggggagca 1680
tagggttgga gtgggtggtg gatggtgggg ctaatgcctt catggccacg cgcacgtgcc 1740
cgtcccaccc ccaggggtgt tcctggcgcg ctatgggccc gcgtggcgcg agcagaggcg 1800
cttctccgtg tccaccttgc gcaacttggg cctgggcaag aagtcgctgg agcagtgggt 1860
gaccgaggag gccgcctgcc tttgtgccgc cttcgccaac cactccggtg ggtgatgggc 1920
agaagggcac aaagcgggaa ctgggaaggc gggggacggg gaaggcgacc ccttacccgc 1980
atctcccacc cccaggacgc ccctttcgcc ccaacggtct cttggacaaa gccgtgagca 2040
acgtgatcgc ctccctcacc tgcgggcgcc gcttcgagta cgacgaccct cgcttcctca 2100
ggctgctgga cctagctcag gagggactga aggaggagtc gggctttctg cgcgaggtgc 2160
ggagcgagag 2170

<210> 64
<211> 2170
<212> DNA
<213> Homo sapiens 664785

<220>
<221> misc_feature
<222> (1185)..(1185)
<223> n = t or c

<400> 64
cagcagaggg caaaggccat catcagctcc ctttataagg gaagggtcac gcgctcggtg 60
tgctgagagt gtcctgcctg gtcctctgtg cctggtgggg tgggggtgcc aggtgtgtcc 120
agaggagccc atttggtagt gaggcaggta tggggctaga agcactggtg cccctggccg 180
tgatagtggc catcttcctg ctcctggtgg acctgatgca ccggcgccaa cgctgggctg 240
cacgctaccc accaggcccc ctgccactgc ccgggctggg caacctgctg catgtggact 300
tccagaacac accatactgc ttcgaccagg tgagggagga ggtcctggag ggcggcagag 360
gtgctgaggc tcccctacca gaagcaaaca tggatggtgg gtgaaaccac aggctggacc 420
agaagccagg ctgagaaggg gaagcaggtt tgggggacgt cctggagaag ggcatttata 480

catggcatga aggactggat tttccaaagg ccaaggaaga gtagggcaag ggcctggagg 540
tggagctgga cttggcagtg ggcatgcaag cccattgggc aacatatgtt atggagtaca 600
aagtcccttc tgctgacacc agaaggaaag gccttgggaa tggaagatga gttagtcctg 660
agtgccgttt aaatcacgaa atcgaggatg aagggggtgc agtgacccgg ttcaaacctt 720
ttgcactgtg ggtcctcggg cctcactgcc tcaccggcat ggaccatcat ctgggaatgg 780
gatgctaact ggggcctctc ggcaattttg gtgactcttg caaggtcata cctgggtgac 840
gcatccaaac tgagttcctc catcacagaa ggtgtgaccc ccacccccgc cccacgatca 900
ggaggctggg tctcctcctt ccacctgctc actcctggta gccccggggg tcgtccaagg 960
ttcaaatagg actaggacct gtagtctggg gtgatcctgg cttgacaaga ggccctgacc 1020
ctccctctrc agytgcggcg ccgcttyggg gacgtgttca gcctgcagct ggcctggacg 1080
ccggtggtcg tgctcaatgg gctggcggcc gtgcgygagg cgctggtgac ccacggmgag 1140
gacaccgccg accgcccgcc tgygcccatc acccagrtcc tgggnttcgg gccgcgytcc 1200
caaggcaagc rgcggtgggg acagagacag rtttccgtgg gaccygggtg gryrrtgacc 1260
gtagtccgag ctgggcagag agggcgyggg gtcgtggaca tgaaacaggc cagcgagtgg 1320
ggacagcggg ccaagaaacc acctgcacta gggaggtgtg agcatgggga cgagggcggg 1380
gcttgtgacg agtgggcggg gccactgccg agacctggca ggagcccaat gggtgagcgt 1440
ggcgcatttc ccagctggaa tccggtgtcg aagtgggggc ggggaccgca cctgtgctgt 1500
aagctcagtg tgggtggcgc ggggcccgcg gggtcttccc tgagtgcaaa ggcggtcagg 1560
gtgggcagag acgaggtggg gcaaagcctg ccccagccaa gggagcaagg tggatgcaca 1620
aagagtgggc cctgtgacca gctggacaga gccagggact gcgggagacc agggggagca 1680
tagggttgga gtgggtggtg gatggtgggg ctaatgcctt catggccacg cgcacgtgcc 1740
cgtcccaccc ccaggggtgt tcctggcgcg ctatgggccc gcgtggcgcg agcagaggcg 1800
cttctccgtg tccaccttgc gcaacttggg cctgggcaag aagtcgctgg agcagtgggt 1860
gaccgaggag gccgcctgcc tttgtgccgc cttcgccaac cactccggtg ggtgatgggc 1920
agaagggcac aaagcgggaa ctgggaaggc gggggacggg gaaggcgacc ccttacccgc 1980
atctcccacc cccaggacgc ccctttcgcc ccaacggtct cttggacaaa gccgtgagca 2040
acgtgatcgc ctccctcacc tgcgggcgcc gcttcgagta cgacgaccct cgcttcctca 2100
ggctgctgga cctagctcag gagggactga aggaggagtc gggctttctg cgcgaggtgc 2160
ggagcgagag 2170

<210> 65
<211> 2380
<212> DNA
<213> Homo sapiens 664793

<220>
<221> misc_feature
<222> (1421)..(1421)
<223> n = a or c

<400> 65
agccaatcca gacaaacatt tatatttaaa catttatatt taaacaaaag gcctctctga 60
acaaatagcc tgcggagata aatacagtga tttgttttcc tgatagaact atttagcatg 120
tttaacacat tattctgtag tttgggaata agagtgtttc ttcccttgaa gaaaacaggt 180
cccttctga agaataatgc tgattacccc ccaaaatcaa aatagaccag caccaaatga 240
agtattaatt tacaaacatg aacttagaac ttagctctta cttcttgaag ttctacatcc 300
cagacttaat aaattaacta caaaatcagg agtttcatca gctacagtat aatttaaaaa 360
tccattttca actggcagga gtgagggaga aggtcaattg cactgatcac catgaacttc 420
aagaatttca tcaaaacttt tttcccagct tatatttgcc ttcagaggtg agctgtagat 480
taccatctct gatgctttaa catacaatat tcttgttgaa atctcttcaa agagcacagc 540
atgtaaagca ctaaactgtg ttcagatctg aggagtctgc atggaaagaa cctgagacct 600
ctctgaaaga gccaaaaacc aagtggctgt ctcagtgatc acatctattc atcctccaca 660
agacaatgca ttgagctttt ttaattcaca gattttatgt tagtccttta gaacccaatg 720
cccatgttcc agttcagaac tgtcgggcta ttcaggctgt cttcttggtg caagctcctt 780
ggaggtcttg taaattgatc ttcgacctat ggtagaaaat gacaaagtag caatatataa 840
atatcaggag tgtagaattt taacttggaa ctacagtaga tgaatagtaa gtttttacac 900
tgcatatttt ttgaagtata gggggaacat gttaaatata tctttgagtc ttacctgttg 960
tgaatcatgt gacttttgac aagatgtcct gctgccaatg ctgccataag tgacaattcc 1020
ccagccatta cggtcccaca cacaattcgg gcaagctgcc gggcattttc cccaggatta 1080
tctttgcatg ctccttgaac acctagcatc tgtagccagg gagagacaca acaagattca 1140
cccttaaaat catgaccaat ttcttactaa atcaactaaa aacagggcaa ctgtaatggc 1200
atcagaatag aactagactc cactggaagc actaactttc caagacttga cagccacacc 1260
tgacagtgca taataccata gctaacataa tattcacagc ctgactggca gtacccttaa 1320
ctcagtagat gaacattcat ttgctctctt catctacttt cttatctaag cataagctta 1380
aacatgctta tttggacaca atggattagg ctgatatgac naaagagttt ggaaaagacc 1440
aattaaaata gaggtgagtg atacatartc tcagatagaa agagaaaccc agagagtcag 1500
aactaggctt gtggactcta tgcctgatac atcatacctg caaacaggct tgctgaggta 1560
gtaggttggt cccaccaccc accgttccta tctctataga tggcatggtg cagctgatat 1620
ataaatcttc atttgtggga ccacttgctt ccattaaagt aatacagttt gaactaccaa 1680
cattctgtgc tgcatcctgs aaacaagaaa agaaaaaata tacaatatac ttctttcact 1740
tagaaagacg tmacacaaga gaagtggagg ctggagagct cacctgtcca caggcaatgt 1800
agatggcggt gacaatgttt gctgcatggg cgttgtagcc tcctatgctc ccagscatgg 1860
cagagcccac taaattcttg ttaatgttga cctcaatcat agcctctgtg gtagtcttta 1920
atacctacaa aacagagctg tgtacattta gatgttcctc cagaaggttc aggggaatgt 1980

tacccaaatc tatctttctg aacctccaga aaacaaagtt tagatgtggc cccatttaag 2040

ccctgtcctc cattaaaaaa taaaaaaaat taaaaaaaat cagtaaagtt tgttcctatg 2100

gatgatacac acagacagat gggcaaggta caacagtcat ctttgatgga aaacactgtc 2160

ccatatattt aactttattt aaaatgttaa tactcctttc ccccattttt aaatacaatt 2220

aaagattaca aaataaaaaa gataaattat ccatccagtc actcacttct ctgacaacct 2280

tggctggaat gacagcttca caaacaacag attttcctct tccctctatc caatttatag 2340

cagcaggttt cttgtcagta caatagttac cactaacggc 2380

<210> 66
<211> 2730
<212> DNA
<213> Homo sapiens 664802

<220>
<221> misc_feature
<222> (1466)..(1466)
<223> n = t or c

<400> 66
ctgggactag agtctgcaca tttaactatg ggtggtgttg tgttttgtgc ttagatggtc 60

cctatcattg cccagtatgg agatgtgttg gtgagaaatc tgaggcggga agcagagaca 120

ggcaagcctg tcaccttgaa agagtaagta gaagcgcagc catggggttc tgagctgtca 180

tgaacccctc cagctgcctg ccatggagct gatattcctg ctgttgggtt attccagtga 240

ccagacaaaa ggagggctgt ggtaatgcaa cttcaatggg tctcccaaga tggggcagct 300

ccgatgagga ggtggggcag ctggaggaaa aggatcttct cccctgtgca caggggccag 360

ggtttacata tccattaaat tgtcaccttg gatattctag aagactaaat atatccttta 420

gggggaaaaa gtgtgattgt accaaagttt taagcatgga gtgtatggga tggtggaagg 480

ggaaggcact tggtatctgt tggttggcag tgagtaggtt gggagagtta taatggagaa 540

cttagaataa ctttgatcat ttcatgtttt tttctgagga tatcagtaga atactaaata 600

ttaaaattcc taccatttct ttttcctcca gtctcaaaga gagagggtgg taaaaacact 660

ataggtaggg caagcctatt atttgctatc tacacttatg cagtaaaaac aggtgtaatc 720

tgagtttgtc ctgggcagac cagggatatg tggtcactca ctatagaaat ttccaaatca 780

aattttgaga gatttttttt taaccaggac attattggtc attatatttt acaaaaataa 840

ttctgctgtc agggcaacct cagctcacca cagctgggga tagtggaatt ttccaaagct 900

tgagcaggga gtatagagaa taaggatgat atttctagga gctcagaaca gggtactgtt 960

gctttgtaaa gtgctgaaga ggaatcggct ctgggcatag agtctgcagt caggcaatat 1020

cacctgtctt gagcccctta ggaagagtta attattctac tcttgttctg ctgaagcaca 1080

gtgcttaccc atcttgtatc atccacaatc aatacatgct actgtagttg tctgatagtg 1140

ggtctctgtc ttcctatgat gggctccttg atctcagagg taggtctaat tcagttcagt 1200

gtctccatca cacccagcgt agggccagct gcatcactgg cacctgataa caccttctga 1260

tggagtgtga tagaaggtga tctagtagat ctgaaagtct gtggctgttt gtctgtcttg 1320

actggacatg tgggtttcct gttgcatgca tagaggaagg akggtaaaaa ggtgctgatt 1380

ttaattttcc acatctttct ccactcagcg tctttggggc ctacagcatg gatgtgatca 1440

ctagcacatc atttggagtg aacatngact ctctcaacaa tccacaagac ccctttgtgg 1500

aaaacaccaa gaagctttta agatttgatt ttttggatcc attctttctc tcaataagta 1560

tgtggactac tatttccttt aatttatctt kctctcttaa aaataactgc tttattgaga 1620

tataaatcac catgtaattc akccacttwa aatatacagt tcagtgattt gtagtacatt 1680

tgaagatatg tgtgaccatc atcattttaa actttaaaac ttttttgtc aatctagaga 1740

cctcatacat ttttagctat cagcccccctg tcacaaaccc tgtcatcata tgcaaccact 1800

aatcaacttt ctgcttctat ggatttgcct attctggaca cttcatagaa atgatattaa 1860

ttcatcaggg tttttattc tctagttcat gaatttgtac tttagtctgt atcattttct 1920

ttcttctgct ggcttcaggc ttagtttgcc cttcttcgtt tactatgttg tggcatgaac 1980

atagattact gatttgtgat ttttttgttc ctctaaattt agacattaca gctgtaactt 2040

tccctctgag cacttccttt gctaaatccc atgagattgt ggcctatcac atcttagttt 2100

tgttcacctc aaaacagttt ctatttgccc tttgggtttc tactttgact cattgggtac 2160

ttaaatgttt attatttaac ttccacatat gtgtgagttt ctcaattttc tttcccttat 2220

tgattttatc tttattccat gataggtgac agagatatgc tgtgttattt ctatcttgac 2280

tacctactat ttcttgaaca gcaagattaa ttttgagctt cagattatga tttgggttat 2340

tctaggagac tgtagtccaa tagataaagg caaagagatt agggcattga attttgttcc 2400

ttttatcctt caaaagatgc acaaggggct gctgatctca ctgctgtagc ggtgctcctt 2460

atgcatagac ctgcccttgc tcagccactg gcctgaaaga ggggcaaaag tcatagaagg 2520

aatggcttcc agttgagaac cttgatgtct tttactcttc tggttggtag agaaaactag 2580

aattgctcca ggtaaatttt gcacattcac aatgaatttc tttttctgtt tttgttttgt 2640

ttttcctaca gcagtctttc cattcctcat cccaattctt gaagtattaa atatctgtgt 2700

gtttccaaga gaagttacaa atttttaag 2730

<210> 67
<211> 2590
<212> DNA
<213> Homo sapiens 660843

<220>
<221> misc_feature
<222> (1311)..(1311)
<223> n = g or t

<400> 67
tctcccaaga tggggcagct ccgatgagga ggtggggcag ctggaggaaa aggatcttct 60

cccctgtgca caggggccag ggtttacata tccattaaat tgtcaccttg gatattctag 120

aagactaaat atatccttta gggggaaaaa gtgtgattgt accaaagttt taagcatgga 180

gtgtatggga tggtggaagg ggaaggcact tggtatctgt tggttggcag tgagtaggtt 240
gggagagtta taatggagaa cttagaataa ctttgatcat ttcatgtttt tttctgagga 300
tatcagtaga atactaaata ttaaaattcc taccatttct ttttcctcca gtctcaaaga 360
gagagggtgg taaaaacact ataggtaggg caagcctatt atttgctatc tacacttatg 420
cagtaaaaac aggtgtaatc tgagtttgtc ctgggcagac cagggatatg tggtcactca 480
ctatagaaat ttccaaatca aattttgaga gatttttttt taaccaggac attattggtc 540
attatatttt acaaaaataa ttctgctgtc agggcaacct cagctcacca cagctgggga 600
tagtggaatt ttccaaagct tgagcaggga gtatagagaa taaggatgat atttctagga 660
gctcagaaca gggtactgtt gctttgtaaa gtgctgaaga ggaatcggct ctgggcatag 720
agtctgcagt caggcaatat cacctgtctt gagcccctta ggaagagtta attattctac 780
tcttgttctg ctgaagcaca gtgcttaccc atcttgtatc atccacaatc aatacatgct 840
actgtagttg tctgatagtg ggtctctgtc ttcctatgat gggctccttg atctcagagg 900
taggtctaat tcagttcagt gtctccatca cacccagcgt agggccagct gcatcactgg 960
cacctgataa caccttctga tggagtgtga tagaaggtga tctagtagat ctgaaagtct 1020
gtggctgttt gtctgtcttg actggacatg tgggtttcct gttgcatgca tagaggaagg 1080
akggtaaaaa ggtgctgatt ttaattttcc acatctttct ccactcagcg tctttggggc 1140
ctacagcatg gatgtgatca ctagcacatc atttggagtg aacatygact ctctcaacaa 1200
tccacaagac ccctttgtgg aaaacaccaa gaagctttta agatttgatt ttttggatcc 1260
attctttctc tcaataagta tgtggactac tatttccttt aatttatctt nctctcttaa 1320
aaataactgc tttattgaga tataaatcac catgtaattc akccacttwa aatatacagt 1380
tcagtgattt gtagtacatt tgaagatatg tgtgaccatc atcattttaa actttaaaac 1440
tttttttgtc aatctagaga cctcatacat ttttagctat cagccccctg tcacaaaccc 1500
tgtcatcata tgcaaccact aatcaacttt ctgcttctat ggatttgcct attctggaca 1560
cttcatagaa atgatattaa ttcatcaggg ttttttattc tctagttcat gaatttgtac 1620
tttagtctgt atcattttct ttcttctgct ggcttcaggc ttagtttgcc cttcttcgtt 1680
tactatgttg tggcatgaac atagattact gatttgtgat ttttttgttc ctctaaattt 1740
agacattaca gctgtaactt tccctctgag cacttccttt gctaaatccc atgagattgt 1800
ggcctatcac atcttagttt tgttcacctc aaaacagttt ctatttgccc tttgggtttc 1860
tactttgact cattgggtac ttaaatgttt attatttaac ttccacatat gtgtgagttt 1920
ctcaattttc tttcccttat tgattttatc tttattccat gataggtgac agagatatgc 1980
tgtgttattt ctatcttgac tacctactat ttcttgaaca gcaagattaa ttttgagctt 2040
cagattatga tttgggttat tctaggagac tgtagtccaa tagataaagg caaagagatt 2100
agggcattga attttgttcc ttttatcctt caaaagatgc acaaggggct gctgatctca 2160
ctgctgtagc ggtgctcctt atgcatagac ctgcccttgc tcagccactg gcctgaaaga 2220

ggggcaaaag tcatagaagg aatggcttcc agttgagaac cttgatgtct tttactcttc 2280

tggttggtag agaaaactag aattgctcca ggtaaatttt gcacattcac aatgaatttc 2340

tttttctgtt tttgttttgt ttttcctaca gcagtctttc cattcctcat cccaattctt 2400

gaagtattaa atatctgtgt gtttccaaga gaagttacaa attttttaag aaaatctgta 2460

aaaaggatga aagaaagtcg cctcgaagat acacaaaagg taaaatgtgg tggtagttat 2520

aggaggatgt ttagtttttc ataatttttt agataatata catatgatca gtgcagttac 2580

ctgtatgttt 2590

<210> 68
<211> 1820
<212> DNA
<213> Homo sapiens 712037

<220>
<221> misc_feature
<222> (808)..(808)
<223> n = g or a

<400> 68
agattttgaa tcagtagttc aagggtgggg tttgagattt tgcatttcta aatgagctct 60

caagatgctt ctgacccatg gaccacactt tgaataccaa gaagtggtct gtagaccaat 120

attggtccct taagttccct caaacatatc ttcgggaaac gtcctttgat tttccctaca 180

tttaaccatt agtgttgcaa attctctcaa agtttgtcaa gatatattgt agctaaaata 240

aattacattt ttcttggggg agagtactac ctcatattaa cttacaataa agtactttta 300

ggatcattca aggaacacac ccataacact gagtatgtta tgcggaaatg ctctctctgg 360

aaattacaca gctgtgcagg tggcgggggt ggcatgagga ggagtggatg gcccacattc 420

tcgaagacct tggggaaaac tggattaaaa tgatttgcct tattctggtt ctgtaagata 480

cacatcagaa tgaaaccacc cccagtgtac ctctgaattg cttttctatt cttttccctt 540

agggatttga gggcttcact tagatttctc ttcatctaaa ctgtgatgcc ctacattgat 600

ctgatttacc taaaatgtct ttcctctcct ttcagctctg tccgatctgg agctcgtggc 660

ccaatcaatt atctttattt ttgctggcta tgaaaccacg agcagtgttc tctccttcat 720

tatgtatgaa ctggccactc accctgatgt ccagcagaaa ctgcaggagg aaattgatgc 780

agttttaccc aataaggtga gtggatgnta catggagaag gagggaggag gtgaaacctt 840

agcaaaaatg cctcctcacc acttcccagg araattttta taaaaagcat aatcactgat 900

tctttcactg actctatgta ggaaggctct gaaaagaaaa agaaagaaac atagcaaatg 960

gttgctactg gcagaagcgt aagatctttg taaaacgtgc tggctctggt tcatctgctt 1020

tctattacta caataatgct aagtaaaaaa cctccaaaaa cctcagtggc atctaacaat 1080

aagcatttgt tgctcacact catttcaatt ggttttggtt gtgaattaca tgtttgcagc 1140

aggcaccata gtggtgtgtg atgtcccctt agctgtatcc acatatggac acaggaattg 1200

gctcttttta tctcttttta ttttcttggt tacagacatg tgactttttt ttttgaaagg 1260

taacaatcac tttctcatat gttatttgat gctagtggtc atagcctata gtcacatttg 1320
tttcaatgag aaagaaaaac cagtacacgg ttatgctaag gatttcagtc cctggggtga 1380
gagccgtctc gaatgtctcc ccacttcata actcctccac acatcatagt tggatagtga 1440
gctctgctga tattggcagg acttgctctg gtctggctgt agtctgacgg agcctggccc 1500
tgggtgtgct gtgcaggctg actcagctct ccccacacct atctcatgtt ccagtcaggc 1560
agtaactggt gaagaagcca agctaggaac caggatatct ggctcctgag ctaaagtctt 1620
aaaacactat catattgcct tccaaatata acaccaaata ctaggtgcat atcaccctca 1680
ctgttttcag acctctgcca aaattgggat tctttgtggt atgaagagac acggctttgg 1740
ggctggcccg gctgtgacag tgaggtgaac acaaagggat gttcttcaga gattacagtc 1800
cagccctgaa gcaacaacta 1820

<210> 69
<211> 2240
<212> DNA
<213> Homo sapiens 712047

<220>
<221> misc_feature
<222> (1005)..(1005)
<223> n = t or c

<400> 69
gagtctcact ctgtggccca ggctggagtg cagtggcttg atcttggctc actgcaacat 60
ccacctcccg ggttcaagaa attctccagc ctcagcctcc cgagtagctg ggattacagg 120
cgcgggccac catgcccagc caatttttgt attttgagta gagacagggt ttcgccatgc 180
tggccaggct ggtctcgaac tcctggcctc aagtgatctg cccgcgttgg cctccccaaa 240
gtgttgggat tacaggcgtg agccactgcg cccagtcaca attatttctt aataaactta 300
cacagttcac ataaaaacaa atgtgttagc ttgaactata ctatggttat catttgtgtt 360
gattatgcta ctttattaat tttctttatt tgaagtaagt cttattatac taatatttct 420
ctcctatttg aaaaatcttt ttttctaaga cagttctctc ctaggcaaag taacatctaa 480
tcaaaattac tagctcacac tttttttttt ttcttactaa tttacctctg tggagctatt 540
catttgaatc aacattcttt ttttcccccc aaccaagcat aaatattact cattttaaat 600
gaatggcttt aaagttgata ttctgttatg tgctctttag caggtaatat gttaacaatt 660
atgtttggta atcacagaaa atgacactgg ttctaaaata aacaaataga tataactgta 720
catacaaatc cactcacaca cctgctagtg ctgtcaaatg cctcctttat cactgcgaac 780
ccttcagatg tttcgagcca ggctttcact tctgcagagt cacaagcacg tggaagacgc 840
acaactgggc cacgagtcat cccatctgca aggactcggc tgctggcacc tccaccaagc 900
tacacagtat atgttagaga agcaagcaca tgttacccaa aaatgctcat gcttgaccca 960
aaaggtatca ctaattgtcc ttaaaactct tctcattgcc ttacntatga tgtattttta 1020
aactggcaaa tatataaatg ccaacttaca cctattgctc tgcagcctct attggtgctg 1080

gccacaagac aaccttctgt tgttgccatt ggaacctgaa attctttttc atctaagcaa 1140

aggggtcctg ccactccaac agggatgggc atatatscaa taacattctc acaacaagct 1200

cccatcacct aaaaggtaaa gtcaggcacc aaatgaaaat ctatatagta aatgcacaaa 1260

attttatctc agcttgtcag tataactatc ttcaaactta atcctttagt atgtattctt 1320

tttaaacaaa atgtttaatt cacctttaaa aagtgtttaa aatactaaaa tctttgagta 1380

atgactataa aagcaggaaa tatattttta atttctatga cctacatcat aagcagaata 1440

aaaaattagg ataaaatgat ttaaaaggaa aatgttttat aaaactatgt acatatatga 1500

aactaatcaa gaaaaaagat taaaaacata caaagcaaat ctctcttaat cgagaaaata 1560

acatmccaag gagtaattat aatccctgta aggtaggtac tggagagaag aaggttctga 1620

aagcttcttg gaaagtaact gtcggcgaat agatacacca cgctcatgag tttccatcag 1680

agtttccaac ttgtaggctg ggatatgctt agcattgact aactggatga tctcagcatc 1740

actaaggaat tttgcacctt tctaaagaaa tggagaaaaa aaatgaaatt gtggtcagag 1800

agagagatga aacagaagac ttgaaggact gtatttcatg ttataaacca tttaatacaa 1860

acaatgctaa gctaaaataa tgtaattttt aaattaaatc actgtagttt ttaattaaaa 1920

gatacctctg caaaataatt catttaatgc aaaaaatgcc aaacaaaaat aatataaata 1980

ccgtatcatt agattttaca atacaaagac aggcttgcac taatacacca taaaaagaaa 2040

aataaatgtg aatttagcaa ggtttcaagt gtgtgtcaga tataacagct attgtttaaa 2100

ctctagtaaa cttgctattt ttgatatcct gaacaggcaa tatattcaca tggtttaaaa 2160

actaaatatt aaaagagata taacaaaaag tcttcatccc atccctgtct accatctgcc 2220

cagttctttg ctacctacac 2240

<210> 70
<211> 1382
<212> DNA
<213> Homo sapiens 712051

<220>
<221> misc_feature
<222> (743)..(743)
<223> n = a or t

<400> 70
gtgcttaggg atggaggacc agacaaggtt agagggactt tggttctgag gcagcttcta 60

aggcctctca gtgtcaaagc actggtcctc agggaatcac tttctcagcc caacactgcc 120

ttggtggata tcctagctct gcttctcagt aactttctag catctcacag tttatgaccg 180

gctatgagtg gcctctgaaa acacattgaa agtgcataga gaagcccctc aaagccacct 240

gcagtggacg ctccactgcg tgccagtctt aggtgtcgct gcagaaaagc acaagaactt 300

gagagccctg tacgctgaca agagtgattt tcctcctgga ataaaggatt tcttaccgag 360

agctagcagg gcgatgtgag aaagcactat ctgccaggtt tgacagctag gtaatttcaa 420

attggaattt cagatttggc cttcacttgt ctttcctttg aaatttccat tgcatttccc 480

agacatttat tgagtccact tgtcagcttg ggtgaaaaat actgcctacc catcactttc 540
tgatttttcc tttcagccaa gactgaatgc cactaatggg ccattacctg aaggtaccgt 600
gaagggcgag cttgtccttg ggaaataaac ctctggtgtg aattattcca cttgcatctg 660
accagcgtca acactgatgg cctgtggagt gggtaggttt gtatccactc aacagcctaa 720
ttacattcca taaatgttgg tanccttcc tttgctatct ccgcaaatgt aagtatctgt 780
agttcctgtt tctcttttca gcctacagaa tattattatt ttttcccttt ctttacaagc 840
aagagcacaa atgaatttac tagagctgac tggctccagt tcctttggaa caaatgctaa 900
gcataacagg atcagacata aggaaaaaaa tttaaaagga tgtttgatag aaatattttc 960
aaaattatgc tgtaaaaacc taggtggagt tcatagttat ggatataatt ttgtctgtag 1020
aggacaggat caagttttac agatgtgttg atgaaaagat taaaactcag taaaattgaa 1080
gagatgtatt ctaagccaaa tatgagtgac ctgtggcctg tgatacagcc ctcaggagat 1140
gttgagaaca cgtgcccaag gtggtcaggt tacagctcgg ttttatatgc tttaaagaga 1200
cataaagcat cagtcagtac atgcaaggtg tatatcgatt tggtctgaaa aggccggaaa 1260
accggaagtg gaggattcca ggtcataggt agattcaaag attttctgat tggcagttgg 1320
ttgaaacagt taaattattg tctaaagact tagaatcaat agaaaggaat gtctgggtca 1380
ag 1382

<210> 71
<211> 915
<212> DNA
<213> Homo sapiens 712055

<220>
<221> misc_feature
<222> (418)..(418)
<223> n = g or a

<400> 71
tccaattcta cattaattcc tccactatga gcttccacag taacctaatc ttaccctgag 60
atgtctatat caaactgctt cctcacatga gggaaggcac caggtctcgt ttacattttt 120
gctctgtatc actacaatac aagagagaat gtgataaagg ttgtaacaga cccggaaaaa 180
ccactctggg agctctaaga agggtagttc atgtaaatac acacacatat acatatagtt 240
catgtaaata tatatatgta tacacacaca cacggccttc ttcaaggaag agattgctct 300
taggatgttt tcagattgaa gatgctgtaa aatttgtatt gatgatataa aattaaaaaa 360
aagaaattct gttattgtat attttagatc tatcatttcc atttggttct tttttctnta 420
tcttttgttt cttcccatag tttttcattt ttcacttgtt ccaagagaag ttgttaactg 480
attgttgaga catttttagg aaggctgctt taaaatcctt ttaagataat ccagcatccg 540
atatatctca gtgttggcat caggtgtttg tcctttccca ttcaagttgt gattttctca 600
gtttctgata tgacaggtga cttttgattg tatcctggat attttgtcta ttattttagg 660
agactctgag tcataaataa ctgttttatt tcagcaggca gtcaacctgt ttaagtttag 720

cacacaggtt atagactatt tacatagcct gttgttcaaa tgaagattta attttcagag 780
atcttgcagt gctactttga tctgtttggt ttctccagtg ctgctgggtg ctgccttggg 840
ggctggaagg gatatcccca ggctgggctg cccagatgtc tcttcctgtg gagaggagtt 900
tcaggtctgc agaag 915

<210> 72
<211> 1629
<212> DNA
<213> Homo sapiens 712059

<220>
<221> misc_feature
<222> (884)..(884)
<223> n = c or t

<400> 72
aaccccaaaa gtgtaaaata tctgaactga aaagagaaaa gtgaaaatac caccaaaccc 60
ccagcctgct ctctctctca ctgctgtccc agccacaaag ctttacagca aatggacaga 120
aagccctgag ggaagacaga gggggtgctg aggtgatcag aaggtccacc agaaggggcc 180
agggggaact gggccctggg aggtgctgtg agaggagcag gagcacagaa agggcccatc 240
tgggggcatg cagccttggg gaggaaagag caaagaggaa gggaaagctc ctttgggcaa 300
ccaagtggtc aagtggaaaa gaaaaggagg taaaagcggg gttccgcaag gcaggagtcg 360
gaggactgtg ctctgcccgc agaagagcgc caggaatcct acaaaacaca aacaagccca 420
acggcattaa atgaacaaga gaaaaatgaa gtcacaccta cagagctagt gcaaacgcta 480
tcaggagcgg aaatgtcaca tgtatcagct gaggaaaatc ccctctgaaa ataaccatga 540
agcatgtaga agaaaactac aagcccacac ttcaaaatga attcgccatg cccatgcctc 600
aagcaagcat caaaaataca aacccacctt aaatcagaga tttgaaaaca gaaatgggca 660
aataacaggg agaaataaaa agttgattga actcaaggat gaggtaacca agttttctca 720
gaaataaggg gttgtttaat gggtacaaaa aatagggtta gttagaagta ataagttcta 780
gtgtttgata gcatgatagg gccactataa ttaacaataa cttattgtat attttaaaat 840
aactggaaga aaggacttgg aatgttccca aaacaagtaa caanaaatgt ttgaggttat 900
ggatatccta attaccctga tttcatcatt acatattgta ttattgtatc aaaatatcac 960
atgtccccca taaatatgca acaactatta tgtactcaga gaaatcactt tacaaaagaa 1020
atgagaaata aattaaaaga tgcccaaaag acaatagaca aaaatgaaaa tataacaagg 1080
gtcactaaat aaagatatca aagcaagtga gagaataaaa atgaataagt aaatagataa 1140
attaaaaggg cagagatagt agtggaaatg gaacccgggc aatgaaggaa taatatttgt 1200
ttattggagt ccttaaggga aaaaaacaaa tagtagaatg aagctaatat ttaaaactaa 1260
aatctctgtt acatgttcta gtaaaactat aagatttcaa ggaagaagat aaaaatcatc 1320
aaggccgcac gcaaaacaat cagactgaca tcaggtcttc caaaatcaag atataaacaa 1380
aacgacaatg gagcaatatt tttaaaaata agtcaatgaa aaaaaaaagt gtaacaaagg 1440

attccaatcc agccaactta caaagaacag tctatttggc tcatggttct gtagcctctg 1500

aaagtagagg cagacaccaa catccactca gcttctggtg agggcctcag gctgctccca 1560

cttgttgggg aaggtaaagg ggacccaggg catgctgaga tcacatggca aaagaggaag 1620

caagagagt 1629

<210> 73
<211> 1540
<212> DNA
<213> Homo sapiens 712043

<220>
<221> misc_feature
<222> (744)..(744)
<223> n = t or c

<400> 73
agccaatcca gacaaacatt tatatttaaa catttatatt taaacaaaag gcctctctga 60

acaaatagcc tgcggagata aatacagtga tttgttttcc tgatagaact atttagcatg 120

tttaacacat tattctgtag tttgggaata agagtgtttc ttcccttgaa gaaaacaggt 180

ccccttctga agaataatgc tgattacccc ccaaaatcaa aatagaccag caccaaatga 240

agtattaatt tacaaacatg aacttagaac ttagctctta cttcttgaag ttctacatcc 300

cagacttaat aaattaacta caaaatcagg agtttcatca gctacagtat aatttaaaaa 360

tccattttca actggcaggr gtgagggaga aggtcaattg cactgatcac catgaacttc 420

aagaatttca tcaaaacttt tttcccagct tatatttgcc ttcagaggtg agctgtagat 480

taccatctct gatgctttaa catacaatat tcttgttgaa atctcttcaa agagcacagc 540

atgtaaagca ctaaactgtg ttcagatctg aggagtctgc atggaaagaa cctgagacct 600

ctctgaaaga gccaaaaacc aagtggctgt ctcagtgatc acatctattc atcctccaca 660

agacaatgca ttgagctttt ttaattcaca gattttatgt tagtccttta gaacccaatg 720

cccatgttcc agttcagaac tgtngggcta ttcaggctgt cttcttggtg caagctcctt 780

ggaggtcttg taaattgatc ttcgacctat ggtagaaaat gacaaagtag caatatataa 840

atatcaggag tgtagaattt taacttggaa ctacagtaga tgaatagtaa gtttttacac 900

tgcatatttt ttgaagtata gggggaacat gttaaatata tctttgagtc ttacctgttg 960

tgaatcatgt gacttttgac aagatgtcct gctgccaatg ctgccataag tgacaattcc 1020

ccagccatta cggtcccaca cacaattcgg gcaagctgcc gggcattttc cccaggatta 1080

tctttgcatg ctccttgaac acctagcatc tgtagccagg gagagacaca acaagattca 1140

cccttaaaat catgaccaat ttcttactaa atcaactaaa aacagggcaa ctgtaatggc 1200

atcagaatag aactagactc cactggaagc actaactttc caagacttga cagccacacc 1260

tgacagtgca taataccata gctaacataa tattcacagc ctgactggca gtacccttaa 1320

ctcagtagat gaacattcat ttgctctctt catctacttt cttatctaag cataagctta 1380

aacatgctta tttggacaca atggattagg ctgatatgac aaaagagttt ggaaaagacc 1440

aattaaaata gaggtgagtg atacatagtc tcagatagaa agagaaaccc agagagtcag 1500

aactaggctt gtggactcta tgcctgatac atcatacctg 1540

<210> 74
<211> 840
<212> DNA
<213> Homo sapiens 756239

<220>
<221> misc_feature
<222> (360)..(360)
<223> n = g or a

<400> 74
acagaaaaac aagcaatcaa tctctagtct cggttcatac taagagccat caccccaaca 60

cctcaaccag gccatatata accacctccc tgtggcctgt ccccataccc actgctattt 120

tcctgcccac attaccttct gataccagca gatctgtccc cggcgggtaa gggatgaatc 180

catgatgtca tctgccacca ggaagaaagc ttgcagctag aaagagtgga ataagacctg 240

cagggctcct cattactgtt ccttctatca gcaacagagc tgctacttta tatctgtata 300

tagttttgct tttttttggt aggggacaga gtctcactat tatccagtgc agtggtgcan 360

tcacagctca ctgtagcctc taactcccag gctcaagtga tcctcccact tcagcttcct 420

gagttcctga gaccataggc acatacccca tgcctggcta tttttttttt ttaatttatt 480

ttttgtagag acagggtccc actatgttgc tcaggctggt tttgaacccc tgggttcaaa 540

tgatcctcct gcctcagcct cccaaattac tgggattaca ggcatgaggc atcacagccg 600

gccagagctg ctgcctttga cagtccctat gagctgggaa agtcaggatg gggagacaga 660

agacttctgt gctatggaga cttggaaagt gacataacat gtttggctca gactccccgc 720

ctataaaatg gaactaaaac actcttgttt taggttaaga aactagaaca gatctttgac 780

atctctaatg agccctagat tattcctggt gtcagggaga ttaggaaaca ccttcatata 840

<210> 75
<211> 1190
<212> DNA
<213> Homo sapiens 756251

<220>
<221> misc_feature
<222> (455)..(455)
<223> n = g or a

<400> 75
tgagtgcaaa ggcggtcagg gtgggcagag acgaggtggg gcaaagcctg ccccagccaa 60

gggagcaagg tggatgcaca aagagtgggc cctgtgacca gctggacaga gccagggact 120

gcgggagacc agggggagca tagggttgga gtgggtggtg gatggtgggg ctaatgcctt 180

catggccacg cgcacgtgcc cgtcccaccc ccaggggtgt tcctggcgcg ctatgggccc 240

gcgtggcgcg agcagaggcg cttctccstg tccaccttgc gcaacttggg cctgggcaag 300

aagtcgctgg agcagtgggt gaccgaggag gccgcctgcc tttgtgccgc cttcgccaac 360

cactccggtg ggtgatgggc agaagggcac aaagcgggaa ctgggaaggc gggggacggg 420

gaaggcgacc ccttacccgc atctcccacc cccangacgc ccctttcgcc ccaacggtct 480

cttggacaaa gccgtgagca acgtgatcgc ctccctcacc tgcgggcgcc gcttcgagta 540

cgacgaccct cgcttcctca ggctgctgga cctagctcag gagggactga aggaggagtc 600

gggctttctg cgcgaggtgc ggagcgagag accgaggagt ctctgcaggg cgagctcccg 660

agaggtgccg gggctggact ggggcctcgg aagagcagga tttgcataga tgggtttggg 720

aaaggacatt ccaggagacc ccactgtaag aagggcctgg aggaggaggg gacatctcag 780

acatggtcgt gggagaggtg tgcccgggtc agggggcacc aggagaggcc aaggactctg 840

tacctcctat ccacgtcaga gatttcgatt ttaggtttct cctctgggca aggagagagg 900

gtggaggctg gcacttgggg agggacttgg tgaggtcagt ggtaaggaca ggcaggccct 960

gggtctacct ggagatggct ggggcctgag acttgtccag gtgaacgcag agcacaggag 1020

ggattgagac cccgttctgt ctggtgtagg tgctgaatgc tgtccccgtc ctcctgcata 1080

tcccagcgct ggctggcaag gtcctacgct tccaaaaggc tttcctgacc cagctggatg 1140

agctgctaac tgagcacagg atgacctggg acccagccca gccccccga 1190

<210> 76
<211> 910
<212> DNA
<213> Homo sapiens 809125

<220>
<221> misc_feature
<222> (519)..(519)
<223> n = t or c

<400> 76
tcacttgagg tcagaaatta aagaccagtc tggccaacat ggcaaaactc cgtctctact 60

gaaaacacaa aaattagccg gggatggtgg tgcacatgtg taatcccagc tactcaggtg 120

gctgaggcag aagaatccct cgaaaccagg aggcgaaggt tgtggtgagc caagatctcg 180

ccactgcact ccagcctggg tgacagagtg agactacatc tcaaatcaat caatcaatca 240

atctaccctg ggtttctctt ccattagatc ttgttctgct ctctgatgtg tttcactagg 300

aaaatactct tatttaccca aaaattatta ttaccataag ttctgaaaac tttcaaaaag 360

aaaaatgggg gyaattccaa attccagtag ctacagaatc ataattgagt tgttagatac 420

aggggactgt tcctggggca cttatggaga ccagtcttgg gacttragaa ttaaacttaa 480

aactttgggc aattcttaaa tcttgtgcta tgaagaaang ctattaatcc ttcctattaa 540

tgtaaactga aaaaaggaat actattcaca ttcctatctt ataaataata cttacctgtg 600

agttggaact gagggcaaac tttgctaatg tgcttgctct ggaaaggtca atcaaaagta 660

ggaaaaaggg caaagcttca ctggaaaaga acaaaatgat cagataaatt taacgggaaa 720

aagtatgatt ttaaaaaaat tctttttaga acaaaacctt tccccctcca tactgtatga 780

tcctgtagta tgtgtacctt tctgcagaca aaaaagtata ccctatattt ctttggcatc 840

ctcaaagcta aacatagtag ttgctcaaaa tatttgttaa aaatattttt aatgttaaaa 900

tgtaagtata 910

<210> 77
<211> 557
<212> DNA
<213> Homo sapiens 869769

<220>
<221> misc_feature
<222> (277)..(277)
<223> n = a or c

<400> 77
accgaggttc ctctgtccac gcttggcacc agcagcrggc actgtgccag gccaggactg 60

ggtacccgtg caccagggag ggccgccgta gggaacccag ccacctctcc cggggtgccc 120

agtagggggc tggcagcagg aagacccccca cagatctcaa ccagcgggrc aggggcatcc 180

tgggagtggc atagggaggg ggcactaasc actccctgca ggggcaagca ccaaaggcag 240

aggcatggtg gcagctcccc agataactcc caccccntta gcccagagtg cccctccctc 300

ttgtggaatg tgcttgggga caattacagg aaggatacgc agggaacaaa aaagtatggc 360

tgggtgactg agacccaact aatcaccact tacaataaac aggctagaac tcagtgcctt 420

caagatggcg tgyacaaggc tgggtaagga ggcggtacag agtaaagtac cccaggatta 480

ggggctgaaa ggacccttaa gtcatcctat tttacacaag ccaaactgag gctctaggag 540

gtaggaagat agtagag 557

<210> 78
<211> 490
<212> DNA
<213> Homo sapiens 869772

<220>
<221> misc_feature
<222> (227)..(227)
<223> n = t or c

<400> 78
cactatttat ctcatctcaa caagactgaa agctcctata gtgtcaggag agtagaaagg 60

atctgtagct tacaattctc atagcaaaat aagcatagca ggatttcaat gaccagccca 120

caaaagtatc ctgtgtacta ctagttgagg ggtggcccck aagtaagaaa ccctaacatg 180

taactcttag ggtattatg tcattaactt tttaaaaatc taccaangtg gaaccagatt 240

crgcaagaag aacaaggaca acatagatcc ttacatatac acaccctttg gaagtggacc 300

cagaaactgc attggcatga ggtttgctct catgaacatg aaacttgctc taatcagagt 360

ccttcagaac ttctccttca aaccttgtaa agaaacacag gttagtcaat tttctataaa 420

aataatgttg tattaataat tcttttaact gagtggtctg tattttttaa aaagaatatg 480

cttgtttaat 490

<210> 79

<211> 490
<212> DNA
<213> Homo sapiens 869777

<220>
<221> misc_feature
<222> (270)..(270)
<223> n = g or c

<400> 79
tgagtgcaaa ggcggtcagg gtgggcagag acgaggtggg gcaaagcctg ccccagccaa 60

gggagcaagg tggatgcaca aagagtgggc cctgtgacca gctggacaga gccagggact 120

gcgggagacc agggggagca tagggttgga gtgggtggtg gatggtgggg ctaatgcctt 180

catggccacg cgcacgtgcc cgtcccaccc ccaggggtgw tyctggcgcg ctatgggccc 240

gcgtggcgcg agcagaggcg cttctccrtn tccaccttgc gcaacttggg cctgggcaag 300

aagtcgctgg agcagtgggt gaccgaggag gccgcctgcc tttgtgccgc cttcgccaac 360

cactccggtg ggtgatgggc agaagggcac aaagcgggaa ctgggaaggc gggggacggg 420

gaaggygacc ccttacccgc atctcccacc cccargacgc ccctttcgcc ccaacggtct 480

cttggacaaa 490

<210> 80
<211> 490
<212> DNA
<213> Homo sapiens 869784

<220>
<221> misc_feature
<222> (216)..(216)
<223> n = g or a

<400> 80
gccgtgagca acgtgatcgc ctccctcacc tgcgggcgcc gcttcgagta cgacgaccct 60

cgcttcctca ggctgctgga cctagctcag gagggactga agraggagtc gggctttctg 120

ygcgaggtgy ggagcgagag accgaggagt ctctgcwggg cgagctcccg agaggtgccg 180

gggctggact ggggcctcgg aagagcagga tttgcntaga tgggtttggg aaaggacatt 240

cyaggagacc ccactgtaag aagggcctgg aggaggaggg gacatctcag acatggtcgt 300

gggagaggtg tgcccgggtc agggggcacc aggagaggcc aaggactctg tacctcctat 360

ccacgtcaga gatttcgatt ttaggtttct cctctgggca aggagagagg gtggaggctg 420

gcacttgggg agggacttgg tgaggtcagt ggtaaggaca ggcaggccct gggtctacct 480

ggagatggct 490

<210> 81
<211> 420
<212> DNA
<213> Homo sapiens 869785

<220>
<221> misc_feature
<222> (172)..(172)
<223> n = t or c

<400> 81
ggctgctgga cctagctcag gagggactga agraggagtc gggctttctg ygcgaggtgy 60

ggagcgagag accgaggagt ctctgcwggg cgagctcccg agaggtgccg gggctggact 120

ggggcctcgg aagagcagga tttgcrtaga tgggtttggg aaaggacatt cnaggagacc 180

ccactgtaag aagggcctgg aggaggaggg gacatctcag acatggtcgt gggagaggtg 240

tgcccgggtc agggggcacc aggagaggcc aaggactctg tacctcctat ccacgtcaga 300

gatttcgatt ttaggtttct cctctgggca aggagagagg gtggaggctg gcacttgggg 360

agggacttgg tgaggtcagt ggtaaggaca ggcaggccct gggtctacct ggagatggct 420

<210> 82
<211> 350
<212> DNA
<213> Homo sapiens 869794

<220>
<221> misc_feature
<222> (176)..(176)
<223> n = g or c

<400> 82
tgaacatcac aggccatctg agtggcaagt ataatcatca tcatgtttct atttaaaatt 60

cagaaatatt tgaagcctgt gtggctgaat aaaagcatac aaatacaatg aaaatatcat 120

gctaaatcag gcttagcaaa tggacaaaat agtaacttcg tttgctgtta tctctntcta 180

ctttcctagc tctcaaaggt ctatggccct gtgttcactc tgtattttgg cctgaaaccc 240

atagtggtgc tgcatggata tgaagyagtg aaggaagccc tgattgatct tggagaggag 300

ttttctggaa gaggcatttt cccactggct gaaagagcta acagaggatt 350

<210> 83
<211> 350
<212> DNA
<213> Homo sapiens 869797

<220>
<221> misc_feature
<222> (145)..(145)
<223> n = t or c

<400> 83
tgattgatct tggagaggag ttttctggaa gaggcatttt cccactggct gaaagagcta 60

acagaggatt tggtaggtgt gcawgtgcct gtttcagcat ctgtcttggg gatggggagg 120

atggaaaaca gagacttaca gagcncctcg ggcagagctt ggcccatcca catggctgcc 180

cagtgtcagc ttcctctttc ttgcctggga tctccctcct agtttcgttt ctcwtcctgt 240

taggaattgt tttcagcaat ggaaagaaat ggaaggagat ccggcgtttc tccctcatga 300

cgctgcggaa ttttrggatg gggaagagga gcattgagga cmgtgttcaa 350

<210> 84
<211> 350
<212> DNA

<213> Homo sapiens 869798

<220>
<221> misc_feature
<222> (164)..(164)
<223> n = a or t

<400> 84
tggtaggtgt gcawgtgcct gtttcagcat ctgtcttggg gatggggagg atggaaaaca 60

gagacttaca gagcycctcg ggcagagctt ggcccatcca catggctgcc cagtgtcagc 120

ttcctctttc ttgcctggga tctccctcct agtttcgttt ctcntcctgt taggaattgt 180

tttcagcaat ggaaagaaat ggaaggagat ccggcgtttc tccctcatga cgctgcggaa 240

ttttrggatg gggaagagga gcattgagga cmgtgttcaa gaggaagccc gctgccttgt 300

ggaggagttg agaaaaacca agggtgggtg accmtactcc atatcactga 350

<210> 85
<211> 350
<212> DNA
<213> Homo sapiens 869802

<220>
<221> misc_feature
<222> (166)..(166)
<223> n = g or c

<400> 85
tgggaatgta aatttagcat ttgaacaacc attatttaac cagctaggtt gtaatggtca 60

actcaggatt aatgtaaaag tgaagtgttg attttatgca tgccgaactc ttttttgctg 120

ttaagggaat ttgtaggtaa gataatttct aaactactat tatctnttaa caaatacagt 180

gttttatatc taaagtttaa tagtatttta aattgtttct aattatttag cctcaccctg 240

tgatcccact ttcatcctgg gctgtgctcc ctgcaatgtg atctgctcca ttattttcca 300

kaaacgtttt gattataaag atcagcaatt tcttaactta atggaaaagt 350

<210> 86
<211> 420
<212> DNA
<213> Homo sapiens 869809

<220>
<221> misc_feature
<222> (213)..(213)
<223> n = t or c

<400> 86
tcctttattg aagagaattt tctccactta tatgtgtaca gatttttctt aatatctggt 60

ttatggcagt tacacatttg tgcatctgta accatcctct ctttaagttt gcatatactt 120

ccagcactat aatttaaatt tataatgatg tttggatacc ttcatgattc atatacccct 180

gaattgctac aacaaatgtg ccatttttct ccntttccat cagtttttac ttgtgtctta 240

tcagctaaag tccaggaaga gattgaacgt gtgattggca gaaaccggag cccctgcatg 300

caagacagga gccacatgcc ctacacagat gctgtggtgc acgaggtcca gagatacmtt 360

gaccttctcc ccaccagcct gccccatgca gtgacctgtg acattaaatt cagaaactat 420

<210> 87
<211> 420
<212> DNA
<213> Homo sapiens 869810

<220>
<221> misc_feature
<222> (218)..(218)
<223> n = a or c

<400> 87
tataatgatg tttggatacc ttcatgattc atatacccct gaattgctac aacaaatgtg 60

ccatttttct ccytttccat cagtttttac ttgtgtctta tcagctaaag tccaggaaga 120

gattgaacgt gtgattggca gaaaccggag cccctgcatg caagacagga gccacatgcc 180

ctacacagat gctgtggtgc acgaggtcca gagatacntt gaccttctcc ccaccagcct 240

gccccatgca gtgacctgtg acattaaatt cagaaactat ctcattccca aggtaagttt 300

gtttctccta cactgcaact ccatgttttc gaagtcccca aattcatagt atcattttta 360

aacctctacc atcaccgggt gagagaagtg cataactcat atgtatggca gtttaactgg 420

<210> 88
<211> 350
<212> DNA
<213> Homo sapiens 869813

<220>
<221> misc_feature
<222> (157)..(157)
<223> n = t or c

<400> 88
tctggatgaa ggtggcaatt ttaagaaaag taaatacttc atgcctttct cagcaggtaa 60

tataaattta tttccatttg tgtttcaggg tacaagataa ctttttgwt ccattggaac 120

ttacatgtgc ctcctctgca gtggtacaat tactctntgt acatgatcaa gagcactgtt 180

ctgaatgcct gtgtacaccc tgctcatgat acatcctaat tattgggcca gattagtgga 240

ctttggggag ttaatccaat tcttccaaat tgagaaagct gaaatatagg ttggttgaat 300

tctgcctcta ggtacaccag tgaggtaccc aagaactcct cctggaagat 350

<210> 89
<211> 1820
<212> DNA
<213> Homo sapiens 886934

<220>
<221> misc_feature
<222> (837)..(837)
<223> n = c or t

<400> 89
cttctcaagg caccaaagaa atgagaaata acaaggaaat gtatgtttta aagaaccgaa 60

tgaaataagc atgtgatctt gaggccagca tttttaaaaa tgtgagatca gctttgaatg 120

gaaactaggt ctctgatcta aaaaacaatg ggcagaaaga tttactctgc ttctgtttag 180
catttttatc agtataaatt taggcagaag cctgagtctt aaaagtttag attctaaggc 240
agggttccct aaataaaaca ccttcccgtg ctcagtgtga aagagtccat tggcctgttg 300
ccaaaccaga atctaaatgc ctagtcattc aaattaaatt taaaaacaga agcaaaacaa 360
aaattagcac tccacaaaac atattttaag gctggatctg gctccagact aagagttaat 420
gatgcttgaa ttaaagatag gaaaatggaa gaaggtggaa atgccaagaa gtggatgttg 480
ttattgataa ctttttgta taaccaatat aaatgtaatt atctgcctaa aaaagaaaaa 540
gaagaccctt tatcccttta aatcattttc agaaatgtct gcataatgag ttgagtttca 600
ttccctctaa tgcctaaatg acaccttgta ataaattacc agctttgtta aataaggttt 660
taactcctct gggcccctca gacaccgttg atatactaac cagtacctta ttgtctgaag 720
agagctaaya gaaatagact gtcagagagt agaccaaaca gaaatgaata attgtaaaca 780
gaagcagaga gtattaatgt ggtttctgtg atctaggaaa tgttgcaaga gccttcnttc 840
tcccttcctt actggaattt tgcaacgggg aaaaatgtct gtgatatctg cayggatgac 900
ttgatgggat ccagaagcaa ctttgattcc actctaataa gcccaaactc tgtcttttct 960
caatggcgag tggtctgtga ctccttggaa gattatgata ccctgggaac actttgtaac 1020
agtaagttcc aaatgatagc ttggagtcag aatttctttt tagataawga gattaaatat 1080
gttgcctgaa aggccttcat tctactagag aattcagact aaaatctact tttattatag 1140
agtaacagtg taccaggcat tcattaaaca cctagaatgt tcaaggtact ctakaagttg 1200
ctccagggga aacagaaagt gcctacacat ttttacactg cctttcttga gtagtttggt 1260
caatatcttg ctaactttct tattttggaa atgtctagtt gtataaacta atcctcttag 1320
ttttcttagc actacttaga agtcatgtgt cttgtgttgg aatttcacag aaaatgtttc 1380
ctaagaaaat gtgaaaaata ggcaaaaagt tggaaatgcc ctgggaagaa aaaaaagaaa 1440
agaagcaaac caaatgtatg cttgcagtta taaagttaga aaacaaaagc tgatatgggg 1500
gatagttttc agaaaaggag tatattgtac tgatgtctgc cccctagctg ctttccagct 1560
cttccaaagt gaacacagta agagtacgcc taatcagtgt cccagcatcc tttctccagt 1620
gatctgaatg ccaccactgt cacaggtcaa gtttctgcca catgtagatc tcttcctgag 1680
ctttctgttc tcctccttgg atcatattat tatttgtgcc tgtggtagta acacaaggtt 1740
taattattag acaccccct cctcatctta tttttcttct tcaggcatgt atggctcttt 1800
tgattgttct tccatataaa 1820

<210> 90
<211> 490
<212> DNA
<213> Homo sapiens 886993

<220>
<221> misc_feature
<222> (229)..(229)
<223> n = g or a

<400> 90
aaatccctgg acacacatat aggcacaaaa ctgctagcaa gaggctccat tcaaggagtg 60

agtgagtgta ctattccagg aagtgacggt ctttctgcat ctcagagtga ggagcttggt 120

gatgtggtgg ctttcagagg ccagagctca aatgtgtaag ggatcatgct gatgtcgttt 180

taatatggtg tcctgctaaa agattatcct tgtcttcttc ttttccccnt agatgatctt 240

agtagccata ttttcagaaa cgggattttt cgattattgt gctgtaaagg taggtatgat 300

gttgcattta ataattctat cctgattaat ttatatatgt atttttctga cattatatat 360

ttaggaaaca aacatttaaa acaaacattg aaaaattcca tccttctttt aaaggttgct 420

tctgcagggc agggtatact tgctatgtta agttgtatgg ctctgagcag cactttcagc 480

tgctcagtaa 490

<210> 91
<211> 350
<212> DNA
<213> Homo sapiens 951526

<220>
<221> misc_feature
<222> (160)..(160)
<223> n = g or a

<400> 91
acattaaaaa tagacatttt attacaagag tgtagagaag ggagaccaat agaaggtaat 60

tgaaataycm ccccctcact ccagccctag tcctggtgcc tggatatgtg cactccctgt 120

gcgctctgat ccccgcagac acaagtcccc agcccctccn ggacagcaat aagggtctta 180

caaggccaga aggcagccct gtttgttcct gcctgcagga agggcagagg aatgtgatgt 240

tcccaggaac tgtgtcctag acccataggg tcagattgct cagcctagtt caagcagtga 300

gactacctct gtgccagtat cctgggctgt ctcttccctt cactcttggc 350

<210> 92
<211> 488
<212> DNA
<213> Homo sapiens 217472

<220>
<221> misc_feature
<222> (219)..(219)
<223> n = g or a

<400> 92
atatttattg aatacacact gggtatccag aatgtaaaga gtctcaatac ggaatgaatt 60

ttatttttga ttttatattt tgaaacagtc ttcaagttat agttataaat caaatgggat 120

aatcacatag gttttcagtc attaaagtaa acatattttt ttcatttttt tttaatgaac 180

aggatttgct gatttgctag tccacttact gggatagcng atgcctctca aagcagcatg 240

cacaatgcct tgcacatcta tatgaatgga acaatgtccc aggtacaggg atctgccaac 300

gatcctatct tccttcttca ccatgcattt gttgacaggt tggttaatat ttctttataa 360

ataacgtgct cattggattt aaatagaggg tgcctatcaa atgtgattta agttattaaa 420

taaaagctaa gaagttatgg tagtctattg tctgtgatca ggttgtcacc aaaacagacc 480

ttaggcta 488

<210> 93
<211> 1270
<212> DNA
<213> Homo sapiens 217440

<220>
<221> misc_feature
<222> (632)..(632)
<223> n = t or c

<400> 93
ggagagggtg tgagggcaga tctgggggtg cccagatgga aggaggcagg catgggggac 60

acccaaggcc ccctggcagc accatgaact aagcaggaca cctggagggg aagaactgtg 120

gggacctgga ggcctccaac gactccttcc tgcttcctgg acaggactat ggctgtgcag 180

ggatcccaga gaagacttct gggctccctc aactccaccc ccacagccat cccccagctg 240

gggctggctg ccaaccagac aggagcccgg tgcctggagg tgtccatctc tgacgggctc 300

ttcctcagcc tggggctggt gagcttggtg gagaacgcgc tgktggtggc caccatcgcc 360

aagaaccrga acctgcactc acccatgtac tgcttcatct gctgcctggc cttgtcggas 420

ctgctggtga gcgggassaa crtgctggag acggccgtca tcctcctgct ggaggccggt 480

gcactggtgg cccgggctgc ggtgctgcag cagctggaca atgtcattga cgtgatcacc 540

tgcagctcca tgctgtccag cctctgcttc ctgggcgcca tcgccgtgga ccgctacatc 600

tccatcttct acgcactgyg ctaccacagc ancgtgaccc tgccgygggc gcsgcrassc 660

gttgcggcca tctgggtggc cagtgtcgtc ttcagcacgc tcttcatcgs ctactacgac 720

cacgtggccg tcctgctgtg cstcgtggtc ttcttcctgg ctatgctggt gctcatggcc 780

gtgctgkacg tccacatgct ggcccgggcc tgccagcacg cccagggcat cgcccggctc 840

cacaagaggc agcgcccggt ccaccagggc tttggcctta aaggcgctgt caccctcacc 900

atcctgctgg gcattttctt cctctgctgg ggccccttct tcctgcatct cacactcatc 960

gtcctctgcc ccgagcaccc cacgtgcggc tgcatcttca agaacttcaa cctctttctc 1020

gccctcatca tctgcaatgc catcatcsac cccctcatct acgccttcca cagccaggag 1080

ctccgcagga cgctcaagga ggtgctgaca tgctcctggt gagcgcggtg cacgcgcttt 1140

aagtgtgctg ggcagaggga ggtggtgata ttgtgtggtc tggttcctgt gtgaccctgg 1200

gcagttcctt acctccctgg tccccgtttg tcaaagagga tggactaaat gatctctgaa 1260

agtgttgaag 1270

<210> 94
<211> 1270
<212> DNA
<213> Homo sapiens null

<220>
<221> misc_feature
<222> (1048)..(1048)
<223> n = g or c

<400> 94
ggagagggtg tgagggcaga tctgggggtg cccagatgga aggaggcagg catgggggac 60
acccaaggcc ccctggcagc accatgaact aagcaggaca cctggagggg aagaactgtg 120
gggacctgga ggcctccaac gactccttcc tgcttcctgg acaggactat ggctgtgcag 180
ggatcccaga gaagacttct gggctccctc aactccaccc ccacagccat cccccagctg 240
gggctggctg ccaaccagac aggagcccgg tgcctggagg tgtccatctc tgacgggctc 300
ttcctcagcc tggggctggt gagcttggtg gagaacgcgc tgktggtggc caccatcgcc 360
aagaaccrga acctgcactc acccatgtac tgcttcatct gctgcctggc cttgtcggas 420
ctgctggtga gcgggassaa crtgctggag acggccgtca tcctcctgct ggaggccggt 480
gcactggtgg cccgggctgc ggtgctgcag cagctggaca atgtcattga cgtgatcacc 540
tgcagctcca tgctgtccag cctctgcttc ctgggcgcca tcgccgtgga ccgctacatc 600
tccatcttct acgcactgyg ctaccacagc aycgtgaccc tgccgygggc gcsgcrassc 660
gttgcggcca tctgggtggc cagtgtcgtc ttcagcacgc tcttcatcgs ctactacgac 720
cacgtggccg tcctgctgtg cstcgtggtc ttcttcctgg ctatgctggt gctcatggcc 780
gtgctgkacg tccacatgct ggcccgggcc tgccagcacg cccagggcat cgcccggctc 840
cacaagaggc agcgcccggt ccaccagggc tttggcctta aaggcgctgt caccctcacc 900
atcctgctgg gcattttctt cctctgctgg ggccccttct tcctgcatct cacactcatc 960
gtcctctgcc ccgagcaccc cacgtgcggc tgcatcttca agaacttcaa cctctttctc 1020
gccctcatca tctgcaatgc catcatcnac cccctcatct acgccttcca cagccaggag 1080
ctccgcagga cgctcaagga ggtgctgaca tgctcctggt gagcgcggtg cacgcgcttt 1140
aagtgtgctg ggcagaggga ggtggtgata ttgtgtggtc tggttcctgt gtgaccctgg 1200
gcagttcctt acctccctgg tccccgtttg tcaaagagga tggactaaat gatctctgaa 1260
agtgttgaag 1270

<210> 95
<211> 560
<212> DNA
<213> Homo sapiens 869743

<220>
<221> misc_feature
<222> (235)..(235)
<223> n = g or a

<400> 95
gttgctctag caaggtaata tgttgaataa cagttgaata acagaataaa aaaaaatctc 60
tgcaaagtaa acaaatctca ctagtttatc tgacttgtat tccaaattag tgcttctggc 120
cttttcttaa aactttaagc atcacaagga aatcagttgg aagggaatca tgtgctgatc 180

aagtccttaa agggcagaaa tattcactga agtgaaaagg attagtaaag ggtgnaaaaa 240

aagaccagcc ccccgcctag tttgggtgag cagatttgkg attaattatc aggcagcaat 300

ccacatgcac ttaacagttc tgacgtgaga ggacaagaaa cacaagcaaa tataaaacat 360

tcaattctaa gagaagttca tcagagacat ccttcaggat tgtgaggtac tggaaagaag 420

tcctatgggg agtgggtgga cacgtgccaa aactccatta gtgtaaggga ctttaaatca 480

cagaaattaa cttgctggaa atcygttccc aattcttcct tcagctccaa ggttaaatta 540

aatgtaatta atgatggtga 560

<210> 96
<211> 19
<212> DNA
<213> Artificial sequence

<220>
<223> OCA2_5 primer

<400> 96
caatcacagc cagtgctgc 19

<210> 97
<211> 21
<212> DNA
<213> Artificial sequence

<220>
<223> OCA2_5 primer

<400> 97
gcggtaattt cctgtgcttc t 21

<210> 98
<211> 20
<212> DNA
<213> Artificial sequence

<220>
<223> TYRP1_3 primer

<400> 98
aaagggtctt cccagctttg 20

<210> 99
<211> 25
<212> DNA
<213> Artificial sequence

<220>
<223> TYRP1_3 primer

<400> 99
gtggtctaac aaatgcccta ctctc 25

<210> 100
<211> 34
<212> DNA
<213> Artificial sequence

<220>
<223> PCR primer

<400> 100
gagtatgtga agatataagt aagtgaacta ccat 34

<210> 101
<211> 27
<212> DNA
<213> Artificial sequence

<220>
<223> PCR primer

<400> 101
actgtggttt tctttaaatc tgttgac 27

<210> 102
<211> 45
<212> DNA
<213> Artificial sequence

<220>
<223> Primer extension primer

<400> 102
agcgatctgc gagaccgtat atttctaaaa tgttaaaaca taaac 45

<210> 103
<211> 22
<212> DNA
<213> Artificial sequence

<220>
<223> PCR primer

<400> 103
aaggagaagg caagatccta ag 22

<210> 104
<211> 22
<212> DNA
<213> Artificial sequence

<220>
<223> PCR primer

<400> 104
gccctcctga gagctacaat tt 22

<210> 105
<211> 45
<212> DNA
<213> Artificial sequence

<220>
<223> Primer extension primer

<400> 105
ggctatgatt cgcaatgctt caattagtaa tctggagaga taaaa 45

<210> 106

<211> 45
<212> DNA
<213> Artificial sequence

<220>
<223> Primer extension primer

<400> 106
ggatggcgtt ccgtcctatt caattagtaa tctggagaga taaaa 45

<210> 107
<211> 21
<212> DNA
<213> Artificial sequence

<220>
<223> PCR primer

<400> 107
tggcattcat cttgatcttg g 21

<210> 108
<211> 21
<212> DNA
<213> Artificial sequence

<220>
<223> PCR primer

<400> 108
ctgtgggcaa agtcagtgtc t 21

<210> 109
<211> 45
<212> DNA
<213> Artificial sequence

<220>
<223> Primer extension primer

<400> 109
acgcacgtcc acggtgattt ggttcatagg ctttgtcaca ttctg 45

<210> 110
<211> 25
<212> DNA
<213> Artificial sequence

<220>
<223> PCR primer

<400> 110
agccattagc ttctgattac tttgc 25

<210> 111
<211> 18
<212> DNA
<213> Artificial sequence

<220>
<223> PCR primer

<400> 111

ggccagagct ggctggtg 18

<210> 112
<211> 45
<212> DNA
<213> Artificial sequence

<220>
<223> Primer extension primer

<400> 112
acgcacgtcc acggtgattt ttttggtgaa ataatttcca tgatt 45

<210> 113
<211> 25
<212> DNA
<213> Artificial sequence

<220>
<223> PCR primer

<400> 113
gtggtctaac aaatgcccta ctctc 25

<210> 114
<211> 20
<212> DNA
<213> Artificial sequence

<220>
<223> PCR primer

<400> 114
aaagggtctt cccagctttg 20

<210> 115
<211> 45
<212> DNA
<213> Artificial sequence

<220>
<223> Primer extension primer

<400> 115
agggtctcta cgctgacgat tctttctaat acaagcatat gttag 45

<210> 116
<211> 29
<212> DNA
<213> Artificial sequence

<220>
<223> PCR primer

<400> 116
taacgacatc aatatttatg acctctttg 29

<210> 117
<211> 20
<212> DNA
<213> Artificial sequence

<220>
<223> PCR primer

<400> 117
gcagaaaagc tggtgcttca 20

<210> 118
<211> 45
<212> DNA
<213> Artificial sequence

<220>
<223> Primer extension primer

<400> 118
cgtgccgctc gtgatagaat tcaatggatg cactgcttgg gggat 45

<210> 119
<211> 19
<212> DNA
<213> Artificial sequence

<220>
<223> PCR primer

<400> 119
agtggcccaa gctcactta 19

<210> 120
<211> 20
<212> DNA
<213> Artificial sequence

<220>
<223> PCR primer

<400> 120
aaggcaaatg ggaaatccaa 20

<210> 121
<211> 45
<212> DNA
<213> Artificial sequence

<220>
<223> Primer extension primer

<400> 121
agatagagtc gatgccagct gtcgagggac caggccccac aagag 45

<210> 122
<211> 20
<212> DNA
<213> Artificial sequence

<220>
<223> PCR primer

<400> 122
ccctggggca accttactaa 20

<210> 123

<211> 24
<212> DNA
<213> Artificial sequence

<220>
<223> PCR primer

<400> 123
cagcattttg ttcactcagt tctc 24

<210> 124
<211> 45
<212> DNA
<213> Artificial sequence

<220>
<223> Primer extension primer

<400> 124
ggatggcgtt ccgtcctatt aaacatatca cctactatga cagta 45

<210> 125
<211> 22
<212> DNA
<213> Artificial sequence

<220>
<223> PCR primer

<400> 125
gcatctaagg ccctctgtac ct 22

<210> 126
<211> 27
<212> DNA
<213> Artificial sequence

<220>
<223> PCR primer

<400> 126
tagaaagcaa tcaagatgat ttcagag 27

<210> 127
<211> 45
<212> DNA
<213> Artificial sequence

<220>
<223> Primer extension primer

<400> 127
gcggtaggtt cccgacatat ctctttcata aatttgaact taatt 45

<210> 128
<211> 20
<212> DNA
<213> Artificial sequence

<220>
<223> PCR primer

<400> 128

taaggtcgtt gtttcgttct 20

<210> 129
<211> 19
<212> DNA
<213> Artificial sequence

<220>
<223> PCR primer

<400> 129
atgagccatc aaaagaggg 19

<210> 130
<211> 45
<212> DNA
<213> Artificial sequence

<220>
<223> Primer extension primer

<400> 130
agagcgagtg acgcatacta cagagagacg gtgtccatca gcatc 45

<210> 131
<211> 18
<212> DNA
<213> Artificial sequence

<220>
<223> PCR primer

<400> 131
gcctggactt tgccggat 18

<210> 132
<211> 18
<212> DNA
<213> Artificial sequence

<220>
<223> PCR primer

<400> 132
gcctggactt tgccggat 18

<210> 133
<211> 45
<212> DNA
<213> Artificial sequence

<220>
<223> Primer extension primer

<400> 133
gtgattctgt acgtgtcgcc ctgcacacat gttcattggg atttg 45

<210> 134
<211> 27
<212> DNA
<213> Artificial sequence

<220>
<223> PCR primer

<400> 134
gacacgaatt tttattggac atgttta 27

<210> 135
<211> 20
<212> DNA
<213> Artificial sequence

<220>
<223> PCR primer

<400> 135
agggttatgc tcaaggccat 20

<210> 136
<211> 45
<212> DNA
<213> Artificial sequence

<220>
<223> Primer extension primer

<400> 136
agcgatctgc gagaccgtat ttattgtagt agatgttcat gattc 45

<210> 137
<211> 18
<212> DNA
<213> Artificial sequence

<220>
<223> PCR primer

<400> 137
gctgcgtcta ccccgcat 18

<210> 138
<211> 27
<212> DNA
<213> Artificial sequence

<220>
<223> PCR primer

<400> 138
aaatataggt gtttctgtca actccag 27

<210> 139
<211> 45
<212> DNA
<213> Artificial sequence

<220>
<223> Primer extension primer

<400> 139
agagcgagtg acgcatacta tctgctcttg tcccattggt gagaa 45

<210> 140

<211> 20
<212> DNA
<213> Artificial sequence

<220>
<223> PCR primer

<400> 140
tcctgagaaa tcagcctctg 20

<210> 141
<211> 22
<212> DNA
<213> Artificial sequence

<220>
<223> PCR primer

<400> 141
agtcccaggt gtaggagagg tc 22

<210> 142
<211> 45
<212> DNA
<213> Artificial sequence

<220>
<223> Primer extension primer

<400> 142
gtgattctgt acgtgtcgcc cctttgccct ccagctccat gaccc 45

<210> 143
<211> 18
<212> DNA
<213> Artificial sequence

<220>
<223> PCR primer

<400> 143
gcccctcaga caccgttg 18

<210> 144
<211> 30
<212> DNA
<213> Artificial sequence

<220>
<223> PCR primer

<400> 144
attattcatt tctgtttggt ctactctctg 30

<210> 145
<211> 21
<212> DNA
<213> Artificial sequence

<220>
<223> Primer extension primer

<400> 145

cctcagacac cgttgatata c 21

<210> 146
<211> 21
<212> DNA
<213> Artificial sequence

<220>
<223> Primer extension primer

<400> 146
gtgtaggcac tttctgtttc c 21

<210> 147
<211> 45
<212> DNA
<213> Artificial sequence

<220>
<223> Primer extension primer

<400> 147
ggatggcgtt ccgtcctatt taccttattg tctgaagaga gctaa 45

<210> 148
<211> 26
<212> DNA
<213> Artificial sequence

<220>
<223> PCR primer

<400> 148
tccaaaarca aatgtgttat ctttca 26

<210> 149
<211> 27
<212> DNA
<213> Artificial sequence

<220>
<223> PCR primer

<400> 149
agggtgctgt acaataagat caatatc 27

<210> 150
<211> 45
<212> DNA
<213> Artificial sequence

<220>
<223> Primer extension primer

<400> 150
ggctatgatt cgcaatgctt ttggacttgg aaactttcat ttgta 45

<210> 151
<211> 18
<212> DNA
<213> Artificial sequence

<220>
<223> PCR primer

<400> 151
atcgccgtgg accgctac 18

<210> 152
<211> 19
<212> DNA
<213> Artificial sequence

<220>
<223> PCR primer

<400> 152
gggtcacgrt gctgtggta 19

<210> 153
<211> 45
<212> DNA
<213> Artificial sequence

<220>
<223> Primer extension primer

<400> 153
acgcacgtcc acggtgattt ctacatctcc atcttctacg cactg 45

<210> 154
<211> 24
<212> DNA
<213> Artificial sequence

<220>
<223> PCR primer

<400> 154
tacatctcca tcttctacgc actg 24

<210> 155
<211> 21
<212> DNA
<213> Artificial sequence

<220>
<223> PCR primer

<400> 155
gatgaagagc gtgctgaaga c 21

<210> 156
<211> 45
<212> DNA
<213> Artificial sequence

<220>
<223> Primer extension primer

<400> 156
cgtgccgctc gtgatagaat ctaccacagc atcgtgaccc tgccg 45

<210> 157

<211> 18
<212> DNA
<213> Artificial sequence

<220>
<223> PCR primer

<400> 157
catgctgggt tcccttgc 18

<210> 158
<211> 20
<212> DNA
<213> Artificial sequence

<220>
<223> PCR primer

<400> 158
cactgagtgg taagccaggg 20

<210> 159
<211> 45
<212> DNA
<213> Artificial sequence

<220>
<223> Primer extension primer

<400> 159
agggtctcta cgctgacgat cactggcagc actggctgtg attgg 45

<210> 160
<211> 21
<212> DNA
<213> Artificial sequence

<220>
<223> PCR primer

<400> 160
aaggggccac ttacctcttc a 21

<210> 161
<211> 20
<212> DNA
<213> Artificial sequence

<220>
<223> PCR primer

<400> 161
ggcagagttg ttgaaaggcc 20

<210> 162
<211> 45
<212> DNA
<213> Artificial sequence

<220>
<223> Primer extension primer

<400> 162

gacctgggtg tcgataccta acttaattta ttagccttat tctgt 45

<210> 163
<211> 28
<212> DNA
<213> Artificial sequence

<220>
<223> PCR primer

<400> 163
atcaactcat atagagtgac tatgatgg 28

<210> 164
<211> 22
<212> DNA
<213> Artificial sequence

<220>
<223> PCR primer

<400> 164
cctgcttgga gagagagatt ca 22

<210> 165
<211> 45
<212> DNA
<213> Artificial sequence

<220>
<223> Primer extension primer

<400> 165
ggctatgatt cgcaatgctt gaggatcaag atttcgggaa gaaaa 45

<210> 166
<211> 28
<212> DNA
<213> Artificial sequence

<220>
<223> PCR primer

<400> 166
ttagtcctaa tgcagtattt atgtaacc 28

<210> 167
<211> 19
<212> DNA
<213> Artificial sequence

<220>
<223> PCR primer

<400> 167
tctcagcgaa catgcttgt 19

<210> 168
<211> 45
<212> DNA
<213> Artificial sequence

<220>
<223> Primer extension primer

<400> 168
cgtgccgctc gtgatagaat aactttcgcg tattttgcct caccc 45

<210> 169
<211> 45
<212> DNA
<213> Artificial sequence

<220>
<223> Primer extension primer

<400> 169
agcgatctgc gagaccgtat aactttcgcg tattttgcct caccc 45

<210> 170
<211> 20
<212> DNA
<213> Artificial sequence

<220>
<223> PCR primer

<400> 170
cggtaatttc ctgtgcttct 20

<210> 171
<211> 21
<212> DNA
<213> Artificial sequence

<220>
<223> PCR primer

<400> 171
aacttacatc gccaatcaca g 21

<210> 172
<211> 45
<212> DNA
<213> Artificial sequence

<220>
<223> Primer extension primer

<400> 172
agagcgagtg acgcatacta tccagatcgt gcacagaact ctggc 45

<210> 173
<211> 24
<212> DNA
<213> Artificial sequence

<220>
<223> PCR primer

<400> 173
tttcttctaa tggcattgca tttt 24

<210> 174

<211> 33
<212> DNA
<213> Artificial sequence

<220>
<223> PCR primer

<400> 174
ctaatagact aatataaccc aaacagaagt cct 33

<210> 175
<211> 45
<212> DNA
<213> Artificial sequence

<220>
<223> Primer extension primer

<400> 175
gtgattctgt acgtgtcgcc gaatagacca gacacctaga cttta 45

<210> 176
<211> 26
<212> DNA
<213> Artificial sequence

<220>
<223> PCR primer

<400> 176
aaacatcttt atagagcctt tccctg 26

<210> 177
<211> 18
<212> DNA
<213> Artificial sequence

<220>
<223> PCR primer

<400> 177
gccttcaggg ccaggagc 18

<210> 178
<211> 45
<212> DNA
<213> Artificial sequence

<220>
<223> Primer extension primer

<400> 178
acgcacgtcc acggtgattt tgcacgttgc agggcccgcc ctctg 45

<210> 179
<211> 26
<212> DNA
<213> Artificial sequence

<220>
<223> PCR primer

<400> 179

aaacatcttt atagagcctt tccctg 26

<210> 180
<211> 18
<212> DNA
<213> Artificial sequence

<220>
<223> PCR primer

<400> 180
gccttcaggg ccaggagc 18

<210> 181
<211> 45
<212> DNA
<213> Artificial sequence

<220>
<223> Primer extension primer

<400> 181
acgcacgtcc acggtgattt tgcacgttgc agggcccgcc ctctg 45

<210> 182
<211> 23
<212> DNA
<213> Artificial sequence

<220>
<223> PCR primer

<400> 182
ctcttggaac aagtgaaaaa tga 23

<210> 183
<211> 25
<212> DNA
<213> Artificial sequence

<220>
<223> PCR primer

<400> 183
tgctcttagg atgttttcag attga 25

<210> 184
<211> 45
<212> DNA
<213> Artificial sequence

<220>
<223> Primer extension primer

<400> 184
ggctatgatt cgcaatgctt tcatttccat ttggttcttt tttct 45

<210> 185
<211> 21
<212> DNA
<213> Artificial sequence

<220>
<223> PCR primer

<400> 185
tcagaaggtt gtgcagagta a 21

<210> 186
<211> 19
<212> DNA
<213> Artificial sequence

<220>
<223> PCR primer

<400> 186
aacactgtca ggcatttgg 19

<210> 187
<211> 45
<212> DNA
<213> Artificial sequence

<220>
<223> Primer extension primer

<400> 187
acgcacgtcc acggtgattt tgagctgtgg tttctctctt acagc 45

<210> 188
<211> 27
<212> DNA
<213> Artificial sequence

<220>
<223> PCR primer

<400> 188
taatacrtga tatttaggtg acgcaca 27

<210> 189
<211> 26
<212> DNA
<213> Artificial sequence

<220>
<223> PCR primer

<400> 189
gtgttgtttc tttggtcctt aaactc 26

<210> 190
<211> 45
<212> DNA
<213> Artificial sequence

<220>
<223> Primer extension primer

<400> 190
ggatggcgtt ccgtcctatt taaactcggc tgtgtacccc ctgca 45

<210> 191

<211> 45
<212> DNA
<213> Artificial sequence

<220>
<223> Primer extension primer

<400> 191
cgtgccgctc gtgatagaat cattttatct aaccctcact gagct 45

<210> 192
<211> 19
<212> DNA
<213> Artificial sequence

<220>
<223> PCR primer

<400> 192
atgctcctct tcacgcctg 19

<210> 193
<211> 22
<212> DNA
<213> Artificial sequence

<220>
<223> PCR primer

<400> 193
cttttcatgc acctgagaat gg 22

<210> 194
<211> 45
<212> DNA
<213> Artificial sequence

<220>
<223> Primer extension primer

<400> 194
agatagagtc gatgccagct gtacgcaaag cacctctgcc gtggg 45

<210> 195
<211> 18
<212> DNA
<213> Artificial sequence

<220>
<223> PCR primer

<400> 195
tgcctggctc caggttcc 18

<210> 196
<211> 19
<212> DNA
<213> Artificial sequence

<220>
<223> PCR primer

<400> 196

cagacacgag ctggactgg 19

<210> 197
<211> 45
<212> DNA
<213> Artificial sequence

<220>
<223> Primer extension primer

<400> 197
cgactgtagg tgcgtaactc ctcaggtgca tgaaaaggtg ggggc 45

<210> 198
<211> 45
<212> DNA
<213> Artificial sequence

<220>
<223> Primer extension primer

<400> 198
agggtctcta cgctgacgat ctcaggtgca tgaaaaggtg ggggc 45

<210> 199
<211> 25
<212> DNA
<213> Artificial sequence

<220>
<223> PCR primer

<400> 199
gttttaatat ggtgtcctgc taaaa 25

<210> 200
<211> 25
<212> DNA
<213> Artificial sequence

<220>
<223> PCR primer

<400> 200
tttacagcac aataatcgaa aaatc 25

<210> 201
<211> 45
<212> DNA
<213> Artificial sequence

<220>
<223> Primer extension primer

<400> 201
agcgatctgc gagaccgtat ttatccttgt cttcttcttt tcccc 45

<210> 202
<211> 45
<212> DNA
<213> Artificial sequence

<220>
<223> Primer extension primer

<400> 202
gcggtaggtt cccgacatat ttatccttgt cttcttcttt tcccc 45

<210> 203
<211> 26
<212> DNA
<213> Artificial sequence

<220>
<223> PCR primer

<400> 203
tattgagtag ctcacaaaat catgga 26

<210> 204
<211> 22
<212> DNA
<213> Artificial sequence

<220>
<223> PCR primer

<400> 204
tgccctgtgt tctatagcat gg 22

<210> 205
<211> 45
<212> DNA
<213> Artificial sequence

<220>
<223> Primer extension primer

<400> 205
gcggtaggtt cccgacatat aaacaggtga gaataagcaa gaagg 45

<210> 206
<211> 27
<212> DNA
<213> Artificial sequence

<220>
<223> PCR primer

<400> 206
gaaaaaaaaa ggttttgaga catgact 27

<210> 207
<211> 25
<212> DNA
<213> Artificial sequence

<220>
<223> PCR primer

<400> 207
ggtcccagta tttcaggtga ataaa 25

<210> 208

<211> 45
<212> DNA
<213> Artificial sequence

<220>
<223> Primer extension primer

<400> 208
ggctatgatt cgcaatgctt gactgtaagg tgacctggga aattc 45

<210> 209
<211> 45
<212> DNA
<213> Artificial sequence

<220>
<223> Primer extension primer

<400> 209
agcgatctgc gagaccgtat gactgtaagg tgacctggga aattc 45

<210> 210
<211> 20
<212> DNA
<213> Artificial sequence

<220>
<223> PCR primer

<400> 210
atgaatggct gaggagatac 20

<210> 211
<211> 27
<212> DNA
<213> Artificial sequence

<220>
<223> PCR primer

<400> 211
aactgataac tatgccatct aaacaat 27

<210> 212
<211> 45
<212> DNA
<213> Artificial sequence

<220>
<223> Primer extension primer

<400> 212
agggtctcta cgctgacgat aatcygccca gctgagcatg caaaa 45

<210> 213
<211> 21
<212> DNA
<213> Artificial sequence

<220>
<223> PCR primer

<400> 213

actcacccat gtactgcttc a 21

<210> 214
<211> 20
<212> DNA
<213> Artificial sequence

<220>
<223> PCR primer

<400> 214
tcaatgacat tgtccagctg 20

<210> 215
<211> 45
<212> DNA
<213> Artificial sequence

<220>
<223> Primer extension primer

<400> 215
cgtgccgctc gtgatagaat ggasctgctg gtgagcggga ssaac 45

<210> 216
<211> 22
<212> DNA
<213> Artificial sequence

<220>
<223> PCR primer

<400> 216
tgtgcctgct ctatgtctgt gt 22

<210> 217
<211> 23
<212> DNA
<213> Artificial sequence

<220>
<223> PCR primer

<400> 217
ggtgcacaca cagagacata cag 23

<210> 218
<211> 45
<212> DNA
<213> Artificial sequence

<220>
<223> Primer extension primer

<400> 218
acgcacgtcc acggtgattt tgcaccagtg tgaactgtgt aggtt 45

<210> 219
<211> 45
<212> DNA
<213> Artificial sequence

<220>
<223> Primer extension primer

<400> 219
agcgatctgc gagaccgtat tgcaccagtg tgaactgtgt aggtt 45

<210> 220
<211> 21
<212> DNA
<213> Artificial sequence

<220>
<223> PCR primer

<400> 220
cctcagacac cgttgatata c 21

<210> 221
<211> 21
<212> DNA
<213> Artificial sequence

<220>
<223> PCR primer

<400> 221
gtgtaggcac tttctgtttc c 21

<210> 222
<211> 21
<212> DNA
<213> Artificial sequence

<220>
<223> Primer extension primer

<400> 222
cctcagacac cgttgatata c 21

<210> 223
<211> 21
<212> DNA
<213> Artificial sequence

<220>
<223> Primer extension primer

<400> 223
gtgtaggcac tttctgtttc c 21

<210> 224
<211> 45
<212> DNA
<213> Artificial sequence

<220>
<223> Primer extension primer

<400> 224
acgcacgtcc acggtgattt cacctagaat gttcaaggta ctcta 45